(19) Europäisches Patentamt
European Patent Office
Office européen des brevets

(11) EP 1 383 864 B1

(12) # EUROPEAN PATENT SPECIFICATION

(45) Date of publication and mention of the grant of the patent:
**16.01.2008 Bulletin 2008/03**

(21) Application number: **01977223.5**

(22) Date of filing: **28.09.2001**

(51) Int Cl.:
*C12N 1/00* (2006.01)

(86) International application number:
**PCT/US2001/030328**

(87) International publication number:
**WO 2002/026933 (04.04.2002 Gazette 2002/12)**

(54) **ISOPRENOID PRODUCTION**

ISOPRENOIDPRODUKTION

PRODUCTION D'ISOPRENOIDES

(84) Designated Contracting States:
**AT BE CH CY DE DK ES FI FR GB GR IE IT LI LU MC NL PT SE TR**

(30) Priority: **29.09.2000 US 236580 P**

(43) Date of publication of application:
**28.01.2004 Bulletin 2004/05**

(73) Proprietor: **Cargill, Incorporated**
**Wayzata,**
**Minnesota 55391 (US)**

(72) Inventors:
- **GOKARN, Ravi**
**Plymouth, MN 55447 (US)**
- **JESSEN, Holly**
**Chanhassen, MN 55317 (US)**
- **ZIDWICK, Mary, Jo**
**Wayzata, MN 55391 (US)**

(74) Representative: **Wichmann, Hendrik**
**Patentanwälte**
**Isenbruck Bösl Hörschler Wichmann Huhn**
**Postfach 86 08 80**
**81635 München (DE)**

(56) References cited:

| | |
|---|---|
| **WO-A-01/27286** | **US-A- 6 103 488** |
| **US-B1- 6 190 895** | **US-B1- 6 225 097** |
| **US-B1- 6 232 530** | **US-B1- 6 461 842** |

- **ARMSTRONG G A: "Genetic and biochemical characterization of carotenoid biosynthesis mutants of Rhodobacter capsulatus" JOURNAL OF BIOLOGICAL CHEMISTRY, AMERICAN SOCIETY OF BIOLOGICAL CHEMISTS, BALTIMORE, MD, US, vol. 265, no. 14, 15 May 1990 (1990-05-15), pages 8329-8338, XP000881086 ISSN: 0021-9258**
- **PENFOLD R J ET AL: "Sequencing, chromosomal inactivation, and functional expression in Escherichia coli of ppsR, a gene which represses carotenoid and bacteriochlorophyll synthesis in Rhodobacter sphaeroides" JOURNAL OF BACTERIOLOGY, vol. 176, no. 10, 1994, pages 2869-2876, XP002307558 ISSN: 0021-9193**
- **OH J ET AL: "The cbb3 terminal oxidase of Rhodobacter sphaeroides 2.4.1: Structural and functional implications for the regulation of spectral complex formation" BIOCHEMISTRY, vol. 38, no. 9, 2 March 1999 (1999-03-02), pages 2688-2696, XP002307559 ISSN: 0006-2960**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

<110> Cargill Incorporated

<120> ISOPRENOID PRODUCTION

<130> 12904-002WO1

<140> PCT/US01/30328
<141> 2001-09-28

<150> US 60/236,580
<151> 2000-09-29

<160> 190

<170> FastSEQ for Windows Version 4.0

<210> 1
<211> 3626
<212> DNA
<213> Sphingomonas trueperi

<220>
<221> misc feature
<222> (1)..(3626)
<223> n = A,T,C or G

<400> 1

```
ctgcggccag accacgcata tcgacgacga ttcgatcacg aaaaacgtac ggtccgcagc      60
ccagcacgcc ggtttttcgc cggtccggcc ggtgatcgag gtgcgcggca agtgcggcaa     120
gtgtgactga cctgtccaac agaccgttcg acttgagact aacgttgcgc taacaaagcc     180
catggctgac ctacccaaga cgccgctgct cgacacggtc gacacgccgc aggacctccg     240
gaagctcgcc cccgcccagc tgcgccagct ggccgacgag cttcgtgccg aaaccatcag     300
tgcggtgggc tccaccggcg ggcatctagg ctccggcctg ggcgtcgtcg aactgacggt     360
ggcgatccac tatgtattca acaccccga cgaccggctg atctgggacg tcgggcacca     420
atgctatccg cacaagatcc tcaccggtcg gcgcgatcgg atccgcacga ttcgtcaggg     480
tggaggcctc tccggcttca ccaagcgcag cgagagcgag tatgatccgt tcggtgccgc     540
gcactcgtcg acctcgatct cggccgcact cggctttgcg atcgccaaca agctcaacga     600
ggcgccgggc aaggcgatcg cggtgatcgg cgacggcgcg atgagcgcgg gcatggccta     660
tgaggcgatg aacaacgccg aggccgccgg caaccggctg gtggtgatcc tcaacgacaa     720
cgacatgtcg atcgccccgc cggtgggcgg gctttcggcc tatcttgcgc gcctcatttc     780
ctcgtccgaa tatctcggcc tgcgcgagct cgccaagcgc ttcacccgca agctttcgcg     840
ccgcctcacc gcggcagccg gcaaggcgga ggaattcgcc cgcggcatgg cgaccggcgg     900
cacgctgttc gaggaacttg gcttctatta tgtcggcccg atcgacggcc acaatctcga     960
gcatctgatc ccggtgctgg agaatgtccg cgacagcgag cagggcccga tcctgatcca    1020
tgtcgtgacc aagaagggca agggctatgc ccggccgaa gcggcggcgg acaagtatca    1080
cggcgtccag aagttcgacg tgatcaccgg ggcacaggcc aaggcacccc cgggcccgcc    1140
cgcctatacc aaggtgttcg ccgatgcgct gctcgccgaa gcggagcgtg atgcgtcggt    1200
ctgcgcgatc accgcggcga tgccctcggg caccgggctc gacaagttcc aggcgacgtt    1260
ccccgatcgc accttcgacg tgggcattgc cgaacagcac gcggtcacct tcgcagcggg    1320
ccttgccgcg caggggatgc ggccgttctg cgcgatctac tcgaccttcc tgcagcgcgc    1380
ctacgaccag gtcgtccacg acgtcgcgat ccagaacctg ccggtccgct tcgcgatcga    1440
ccgcgcgggc ctggtcggtg ccgacggcgc gacccatgcc ggcagcttcg acgtgaccta    1500
tctcgccagc ctgcccaatt tcgtggtgat ggcggccgcg gacgaggtcg agctcgtcca    1560
catgacccac acggcggcga tgcacgacag cggcccgatc gcgctgcgct atccacgcgg    1620
caacggcgtc ggactggcgc tgcccaaggt tccggagcgg ctggaaatcg gcaagggtcg    1680
cgtggtccga gagggcaaga aggtagcgat cctgtcgctc ggcacgcgcc ttgcggaagc    1740
actaaaggcc gccgacacgc tcgaggccaa gggcctctcg accaccgtcg ccgacctgcg    1800
```

```
cttcgccaaa ccgctcgacg aggatctgat ccgccgcctg ctcaccaccc acgaagtggc    1860
ggtgacgatc gaggaaggcg cgatcggcgg ccccggtgcg catgtgctga cgctcgccag    1920
cgataccggc ctgatcgacg ccggcctcaa gctgcgcacc atgcgcctgc cggacatatt    1980
ccaggaccag gacaagcccg agaagcagta tgacgaagcg gggctgaacg ccgccaacat    2040
cgtcgacacg gtgctgaagg cgctccgcta caacgaggcc gagctggccg acggggtgcg    2100
ggcgtaaacg acgccagatc ctccccggaa cggggagggg aaccgccgcc gaaggcggtg    2160
gtggaggggc cgctgcggca cgcancggtt tcccaggctg agagcgatcc gcgccttgcg    2220
gcgcgccccc cccaccattc gctggcgcgg atggtccccc tccccgttcc ggggaggatc    2280
tgggtcctgc cccaccttga atctccaaca tgcacatgcc atgtacatgc acatggctac    2340
gcagcttccc cagactcgct ccagccgcgt tgtcgtgctg gtatcgcccg aggaaaaacg    2400
gcgcatttcc gccaatgcgg aagcggcgga catgacggtc agcgacttca tgcgcaccgc    2460
cgccgaacgc tataccgagc cgaccgacgc cgagatggcg ctgatgcgcg acctgctcgc    2520
ccagctcgaa caggccaatg cccgcacgga cgcggccttt gcccagctcg aagctgcgcg    2580
cgccgccgcc accgcgttcg acgaggaggc gtatcgcgcc gaggtccgcg aacagctgct    2640
gaccgatacc tcaatcgact gggatgcgct gtccactgcc ctttccggct gggcgcgcca    2700
gtgagcttct ggaccgatgc gctacgcgcg ctccagcagg tcgcgctgct ccagcacaag    2760
gtcgagcagg cgctgaccac cgccgaggaa gcccgccgcc attcaatcga gacgcgcgag    2820
cgggtgatcc ggcttgagac gctgatcgac atcgcgatga gacgccagcc cgcagcaccg    2880
cctacgccgc ctgcgcttcc cgaaagtcca caaaccggca gctagcgccc gcttccccga    2940
gcgcgtacat cgcggtacgt gctgaaaatg accatccttc ccctcaccgc ccgcccccgc    3000
gcgctcgcgc actggctgtt cgtcgtcgcc gcgatgatcg tcgcgatggt cgtggtcggg    3060
ggcattaccc ggctcaccga atcgggcctg tcgatcaccg aatggaagcc aatctccggc    3120
atcgtgcccc cgctcaacga cgcgcagtgg caggccgagt tcgaccacta caagcagatc    3180
ggccagtatg agcagctcaa ccagggcatg acgctcggcg ggttcaagag catcttcttc    3240
tgggaatata tccaccgcct gctcggccgg ctgatcggca tggtgttcgc gctgccgctg    3300
ctgtggttcg ccgtccgcaa gcagatcccg cagggctatg gctggcggct ggtcgcgctg    3360
ctcgcgctag gcgggctgca gggcgcgttc ggctggtgga tggtgaagtc ggggctcaac    3420
cacacccgca cctcggttag ccatttctgg ctggcgaccc acctgatgac cgcactgttc    3480
acgctgggcg gcatcgtctg gacgatgctc gacctgcgcg cgcttgccgc caaccatgcc    3540
gagcgccctg cccgactgac cgggctcggc gcgggcgtgc tggtactgct ggcggtccag    3600
ctcttctacg ggcgctggt agcagg                                          3626
```

<210> 2
<211> 1926
<212> DNA
<213> Sphingomonas trueperi

<400> 2

```
atggctgacc tacccaagac gccgctgctc gacacggtcg acacgccgca ggacctccgg      60
aagctcgccc ccgcccagct gcgccagctg gccgacgagc ttcgtgccga aaccatcagt     120
gcggtgggct ccaccggcgg gcatctaggc tccggcctgg gcgtcgtcga actgacggtg     180
gcgatccact atgtattcaa cacccccgac gaccggctga tctgggacgt cgggcaccaa     240
tgctatccgc acaagatcct caccggtcgg cgcgatcgga tccgcacgat tcgtcagggt     300
ggaggcctct ccggcttcac caagcgcagc gagagcgagt atgatccgtt cggtgccgcg     360
cactcgtcga cctcgatctc ggccgcactc ggctttgcga tcgccaacaa gctcaacgag     420
gcgccgggca aggcgatcgc ggtgatcggc gacggcgcga tgagcgcggg catggcctat     480
gaggcgatga acaacgccga ggccgccggc aaccggctgg tggtgatcct caacgacaac     540
gacatgtcga tcgcccccgcc ggtgggcggg ctttcggcct atcttgcgcg cctcatttcc    600
tcgtccgaat atctcggcct gcgcgagctc gccaagcgct tcacccgcaa gctttcgcgc     660
cgcctcaccg cggcagccgg caaggcggag gaattcgccc gcggcatggc gaccggcggc     720
acgctgttcg aggaacttgg cttctattat gtcggcccga tcgacggcca caatctcgag     780
catctgatcc cggtgctgga gaatgtccgc gacagcgagc agggcccgat cctgatccat     840
gtcgtgacca agaagggcaa gggctatgcc ccggccgaag cggcggcgga caagtatcac     900
ggcgtccaga agttcgacgt gatcaccggg gcacaggcca aggcaccccc gggcccgccc     960
gcctatacca aggtgttcgc cgatgcgctg ctcgccgaag cggagcgtga tgcgtcggtc    1020
tgcgcgatca ccgcggcgat gccctcgggc accgggctcg acaagttcca ggcgacgttc    1080
cccgatcgca ccttcgacgt gggcattgcc gaacagcacg cggtcacctt cgcagcgggc    1140
cttgccgcgc aggggatgcg gccgttctgc gcgatctact cgaccttcct gcagcgcgcc    1200
tacgaccagg tcgtccacga cgtcgcgatc cagaacctgc cggtccgctt cgcgatcgac    1260
cgcgcgggcc tggtcggtgc cgacggcgcg acccatgccg gcagcttcga cgtgacctat    1320
ctcgccagcc tgcccaattt cgtggtgatg gcggccgcgg acgaggtcga gctcgtccac    1380
```

```
atgacccaca cggcggcgat gcacgacagc ggcccgatcg cgctgcgcta tccacgcggc    1440
aacggcgtcg gactggcgct gcccaaggtt ccggagcggc tggaaatcgg caagggtcgc    1500
gtggtccgag agggcaagaa ggtagcgatc ctgtcgctcg gcacgcgcct tgcggaagca    1560
ctaaaggccg ccgacacgct cgaggccaag ggcctctcga ccaccgtcgc cgacctgcgc    1620
ttcgccaaac cgctcgacga ggatctgatc cgccgcctgc tcaccaccca cgaagtggcg    1680
gtgacgatcg aggaaggcgc gatcggcggc cccggtgcgc atgtgctgac gctcgccagc    1740
gataccggcc tgatcgacgc cggcctcaag ctgcgcacca tgcgcctgcc ggacatattc    1800
caggaccagg acaagcccga gaagcagtat gacgaagcgg ggctgaacgc cgccaacatc    1860
gtcgacacgg tgctgaaggc gctccgctac aacgaggccg agctggccga cggggtgcgg    1920
gcgtaa                                                                1926
```

<210> 3
<211> 641
<212> PRT
<213> Sphingomonas trueperi

<400> 3

```
Met Ala Asp Leu Pro Lys Thr Pro Leu Leu Asp Thr Val Asp Thr Pro
1               5                   10                  15
Gln Asp Leu Arg Lys Leu Ala Pro Ala Gln Leu Arg Gln Leu Ala Asp
            20                  25                  30
Glu Leu Arg Ala Glu Thr Ile Ser Ala Val Gly Ser Thr Gly Gly His
        35                  40                  45
Leu Gly Ser Gly Leu Gly Val Val Glu Leu Thr Val Ala Ile His Tyr
    50                  55                  60
Val Phe Asn Thr Pro Asp Asp Arg Leu Ile Trp Asp Val Gly His Gln
65                  70                  75                  80
Cys Tyr Pro His Lys Ile Leu Thr Gly Arg Arg Asp Arg Ile Arg Thr
                85                  90                  95
Ile Arg Gln Gly Gly Gly Leu Ser Gly Phe Thr Lys Arg Ser Glu Ser
            100                 105                 110
Glu Tyr Asp Pro Phe Gly Ala Ala His Ser Ser Thr Ser Ile Ser Ala
        115                 120                 125
Ala Leu Gly Phe Ala Ile Ala Asn Lys Leu Asn Glu Ala Pro Gly Lys
    130                 135                 140
Ala Ile Ala Val Ile Gly Asp Gly Ala Met Ser Ala Gly Met Ala Tyr
145                 150                 155                 160
Glu Ala Met Asn Asn Ala Glu Ala Ala Gly Asn Arg Leu Val Val Ile
                165                 170                 175
Leu Asn Asp Asn Asp Met Ser Ile Ala Pro Pro Val Gly Gly Leu Ser
            180                 185                 190
Ala Tyr Leu Ala Arg Leu Ile Ser Ser Ser Glu Tyr Leu Gly Leu Arg
        195                 200                 205
Glu Leu Ala Lys Arg Phe Thr Arg Lys Leu Ser Arg Arg Leu Thr Ala
    210                 215                 220
Ala Ala Gly Lys Ala Glu Glu Phe Ala Arg Gly Met Ala Thr Gly Gly
225                 230                 235                 240
Thr Leu Phe Glu Glu Leu Gly Phe Tyr Tyr Val Gly Pro Ile Asp Gly
                245                 250                 255
His Asn Leu Glu His Leu Ile Pro Val Leu Glu Asn Val Arg Asp Ser
            260                 265                 270
Glu Gln Gly Pro Ile Leu Ile His Val Val Thr Lys Lys Gly Lys Gly
        275                 280                 285
Tyr Ala Pro Ala Glu Ala Ala Ala Asp Lys Tyr His Gly Val Gln Lys
    290                 295                 300
Phe Asp Val Ile Thr Gly Ala Gln Ala Lys Ala Pro Pro Gly Pro Pro
305                 310                 315                 320
Ala Tyr Thr Lys Val Phe Ala Asp Ala Leu Leu Ala Glu Ala Glu Arg
                325                 330                 335
Asp Ala Ser Val Cys Ala Ile Thr Ala Ala Met Pro Ser Gly Thr Gly
            340                 345                 350
```

```
Leu Asp Lys Phe Gln Ala Thr Phe Pro Asp Arg Thr Phe Asp Val Gly
        355                 360                 365
Ile Ala Glu Gln His Ala Val Thr Phe Ala Ala Gly Leu Ala Ala Gln
    370                 375                 380
Gly Met Arg Pro Phe Cys Ala Ile Tyr Ser Thr Phe Leu Gln Arg Ala
385                 390                 395                 400
Tyr Asp Gln Val Val His Asp Val Ala Ile Gln Asn Leu Pro Val Arg
                405                 410                 415
Phe Ala Ile Asp Arg Ala Gly Leu Val Gly Ala Asp Gly Ala Thr His
            420                 425                 430
Ala Gly Ser Phe Asp Val Thr Tyr Leu Ala Ser Leu Pro Asn Phe Val
        435                 440                 445
Val Met Ala Ala Ala Asp Glu Val Glu Leu Val His Met Thr His Thr
    450                 455                 460
Ala Ala Met His Asp Ser Gly Pro Ile Ala Leu Arg Tyr Pro Arg Gly
465                 470                 475                 480
Asn Gly Val Gly Leu Ala Leu Pro Lys Val Pro Glu Arg Leu Glu Ile
                485                 490                 495
Gly Lys Gly Arg Val Val Arg Glu Gly Lys Lys Val Ala Ile Leu Ser
            500                 505                 510
Leu Gly Thr Arg Leu Ala Glu Ala Leu Lys Ala Ala Asp Thr Leu Glu
        515                 520                 525
Ala Lys Gly Leu Ser Thr Thr Val Ala Asp Leu Arg Phe Ala Lys Pro
    530                 535                 540
Leu Asp Glu Asp Leu Ile Arg Arg Leu Leu Thr Thr His Glu Val Ala
545                 550                 555                 560
Val Thr Ile Glu Glu Gly Ala Ile Gly Gly Pro Gly Ala His Val Leu
                565                 570                 575
Thr Leu Ala Ser Asp Thr Gly Leu Ile Asp Ala Gly Leu Lys Leu Arg
            580                 585                 590
Thr Met Arg Leu Pro Asp Ile Phe Gln Asp Gln Asp Lys Pro Glu Lys
        595                 600                 605
Gln Tyr Asp Glu Ala Gly Leu Asn Ala Ala Asn Ile Val Asp Thr Val
    610                 615                 620
Leu Lys Ala Leu Arg Tyr Asn Glu Ala Glu Leu Ala Asp Gly Val Arg
625                 630                 635                 640
Ala
```

<210> 4
<211> 2208
<212> DNA
<213> Chlamydomonas reinhardtii

<400> 4

```
atgctgcgtg gtgctgtttc tcacggccct gcggtcgccg accgggctgc cgctggcccc      60
gcccgctgcg ctgctcccgt cgcccgtggt gtgcgcagcg cagcgcccac gcgtcagcgt     120
cgcgcggagg cttcggtcaa tgccccgcgg gcgggcccgg ccggtagcta ctcgggcgag     180
tgggataagc tttcagtgga ggagattgat gagtggcgcg atgtgggccc gaagacgccc     240
ctgctggaca ctgtcaatta cccggtgcac ctgaagaact tcaacaatga gcagctgaag     300
cagctctgca aggagctgcg cagtgacatc gtgcacaccg tctctcgcac cggtggacac     360
cttagcagca gcctgggcgt ggtggagctg acggtggcta tgcactatgt attcaacacc     420
ccggaggaca agattatttg ggacgtgggc caccaggcgt atggccacaa gatcctgact     480
ggccgtcgca agggtatggc cacgattcgc cagaccaacg gcctttcggg cttcacgaag     540
cgcgacgaga gcgagtacga ccctttcggc gctggccaca gctccacctc gatttcggcg     600
gctctgggta tggcggtggg ccgcgacgtt aagggcaaga agaacagtgt gatcgctgtc     660
atcggcgacg gcgccatcac cgggggtatg gcctatgagg ccatgaacca tgcgggcttc     720
ctggacaaga acatgattgt gattctgaac gacaaccagc aggtgtcgct gcccacgcag     780
tacaacaaca agaaccagga ccccgtgggc gccctgtcca gcgccctggc gcgcctgcag     840
gccaaccggc ccctgcgcga gctgcgcgag attgccaagg gcgtgaccaa gcagctgcct     900
gacgttgtcc agaaggcaac tgctaagatt gacgagtatg ctcgcggcat gatcagcggc     960
```

```
actggctcca cgctgtttga ggagctgggc ctgtactaca tcggccctgt ggacggccac    1020
aacctggacg acctcatcgc cgtgctcagc gaggtgcgca gcgccgagac cgtgggcccg    1080
gtgctggtgc acgtggtaac ggagaagggc cgcggctacc tgcccgccga gacggcgcag    1140
gacaagatgc acggtgtggt caagttcgac ccccgcaccg gcaagcaggt gcaggccaag    1200
acgaaggcca tgtcgtacac gaactacttc gcggacgcgc tgacggcgga ggcggagcgc    1260
gacagccgca tcgtggcggt gcacgcggcc atggcgggcg gcaccggcct gtaccggttc    1320
gagaagaagt tcccggaccg cacctttgac gtgggcattg cggagcagca cgccgtgacc    1380
tttgctgccg gcctggcgtg cgagggcctg gtgcccttct gcaccatcta cagtaccttc    1440
atgcagcgcg gttacgacca gatcgtgcac gacgtgtccc tgcagaagct gcctgtgcgc    1500
ttcgctatgg accgcgctgg cctggtgggc gctgacggct ccacgcactg cggcgccttc    1560
gacgtgacgt tcatggcgtc gctgccgcac atgatcacca tggctccctc gaacgaggcg    1620
gagctcatca acatggtggc cacctgcgcc gccatcgacg acgcgccctc gtgcttccgc    1680
ttcccccgcg gcaacggcct gggcctggac ctggccgcct acggcatcag caaggacctg    1740
aagggtgtgc ccctcgaggt gggcaagggt gttgtccgcc gccagggcaa ggacgtgtgc    1800
ctggtggcgt acggcagcag tgtgaacgag gcgctggccg cggcggacat gctggagcgc    1860
gatggcgtgt ccaccaccgt cattgacgcg cgcttctgca agcctctgga caccaagctg    1920
atccgctcgg ctgccaagga gcaccctgtc atgatcacca tcgaggaggg ctccgtgggt    1980
ggcttcgctg cgcacgtgat gcagttcctc gcactggagg gcctgctgga cggcgggctc    2040
aagttccggc ccatgacgct gccggaccgc tacatcgacc acggcgacta ccgcgaccag    2100
ctggccatgg ccggcctcac cagccagcac atcgcctcca ccgcgctcac caccctgggg    2160
cgcgccaagg acgccgccaa gttctcactg tcagcgctgc aagcgtaa                 2208
```

<210> 5
<211> 1849
<212> DNA
<213> Campylobacter jejuni

<400> 5

```
atgagtaaaa aatttgccca tactcaagaa gagttagaaa agctaagttt aaaagaatta 60
gaaaatttag cagcatctat gcgtgaaaaa atcatacaag ttgtgagtaa aaatggtggg 120
catttaagtt caaatttggg tgctgtagaa cttagtatag caatgcattt ggtttttgat 180
gcaaaaaaag atccttttat ttttgatgtg tcgcatcagt cttatacaca caagctttta 240
agcggaaaag aagaaatatt tgatacttta agacaaatca atggtttaag tggttataca 300
aaacctagcg agggagatta ttttgtagca gggcattcta gtacctctat ttctttggca 360
gtaggtgctt gtaaggctat tgctttaaag ggtgaaaagc gtattcctgt tgctttgatt 420
ggagatggtg ctttaagtgc gggtatggcc tatgaggctt taaatgaatt gggtgattct 480
aaatttcctt gcgtaatact tttaaatgat aatgaaatga gtatttcaaa accaattgga 540
gcaatttcaa agtatctttc tcaggctatg gcaacgcagt tttatcaaag ttttaaaaag 600
cgtattgcta aaatgttgga tatattgcct gatagtgcta cttatatggc caagcgtttt 660
gaagagagtt ttaaacttat tacccctggg cttttgtttg aagaattagg gcttgaatat 720
atagggccta ttgatggaca taatttaggt gaaattattt ctgcattaaa acaagcaaaa 780
gctatgcaaa agccttgtgt gatacatgct caaaccataa agggtaaagg ctatgcttta 840
gctgaaggaa aacatgctaa atggcacggg gtgggagcct ttgatataga tagtggagag 900
agtgttaaaa aaagtgatac taaaaaatct gctactgaaa ttttttctaa gaatttgctt 960
gatttagcct caaaatatga aaatattgtt ggggttacgg cggctatgcc aagtggaaca 1020
ggtcttgata agcttataga aaaatatcca aatcgttttt gggatgtggc tattgcagaa 1080
cagcatgcag taacttctat ggccgctatg gcaaaagaag gatttaaacc ttttattgca 1140
atatatagca cctttttgca gcgtgcttat gatcaagtga tccatgattg tgcgattatg 1200
aaatttaaat gtggtttttg ctatggatag ggcagggata gtaggcgaag atggggagac 1260
gcatcaaggt gtttttgatc ttagtttttt agctcctttg ccaaatttca ctcttttagc 1320
cccaagagat gaacaaatga tgcaaaatat aatggagtat gcttatttac atcaaggacc 1380
tattgctttg cgttatccta gagggagttt tattttggat aaagaattta atccttgtga 1440
gataaaactt ggtaaggcac aatggcttgt aaaaaataat agtgaaattg ctttttttagg 1500
ttatggacaa ggtgtggcaa aagcgtggca agtcttaaga gccttgcaag aaatgaataa 1560
taatgctaat ttgattgatt taatttttgc taaaccttta gatgaagagc ttttgtgtga 1620
gcttgctaaa aaaagtaaaa tttggtttat ttttagtgaa aatgttaaaa ttggcggtat 1680
agaaagttta attaataatt ttttacaaaa atatgatttg catgtaaaag ttgttagctt 1740
tgaatatgaa gacaaattta ttgaacatgg aaaaacaagt gaggtggaaa aaaatctaga 1800
aaaagatgtc aatagtttgt tgacgaaagt tttaaaattt tatcattaa 1849
```

<210> 6

<211> 1884
<212> DNA
<213> Pseudomonas aeruginosa

<400> 6

```
atgcccaaga cgctccatga gattccccgc gagcgccccg ccacgcccct gctcgaccgc      60
gcctcttcgc cggccgaact gcgccggctg ggcgaggcgg acctggaaac cctggccgac     120
gagctgcgcc agtacctgct gtataccgtc ggccagaccg gcggtcattt cggcgccggc     180
ctcggcgtgg tcgagctgac cattgccctg cactacgtct tcgacactcc ggacgaccgc     240
ctggtctggg acgtcggcca ccaggcctat ccgcacaaga tcctcaccga gcgccgcgag     300
ctgatgggca ccctgcgcca gaagaacggc ctggcggcct tcccgcgccg cgcagagagc     360
gagtacgaca ccttcggcgt cggccactcc agcacctcca tcagcgccgc cctgggcatg     420
gccatcgccg cccgcctgca aggcaaggag cgtaagtcgg tggccgtgat cggcgacggt     480
gcgctgaccg ccggcatggc cttcgaggca ctcaaccacg cctcggaagt cgacgccgac     540
atgctggtga tcctcaacga caacgacatg tcgatctcgc acaacgtcgg cgggctctcc     600
aactacctgg cgaagatcct ctccagccgc acctatagca gcatgcgcga gggcagcaag     660
aaggtgctct cgcgcctgcc cggggcctgg gagatcgccc ggcgcaccga ggaatacgcc     720
aagggcatgc tggtccccgg caccctgttc gaggagctcg gctggaatta catcggcccg     780
atcgacggcc acgacctgcc gaccctggtg gctaccctgc gcaacatgcg cgacatgaag     840
ggcccgcagt tcctccatgt ggtgaccaag aaaggcaagg gcttcgcccc ggccgaactg     900
gatccgatcg gctaccacgc gatcaccaag ctggaagctc ccggcagtgc gccgaagaag     960
accggcggac ccaagtattc cagcgtcttc ggccagtggc tgtgcgacat ggccgcccag    1020
gacgcgcgcc tgctcggcat caccccggcg atgaaggaag gttccgacct ggtggccttc    1080
agcgaacgtt atccggaacg ctacttcgac gtcgccatcg ccgaacagca tgccgtgacc    1140
ctggccgccg gcatggcctg cgagggcatg aagccggtgg tagcgatcta ctcgaccttc    1200
ctccagcgcg cctacgacca gttgatccat gacgtcgccg tgcagcacct cgacgtgctg    1260
ttcgccatcg accgcgccgg cctggtcggc gaggacggcc cgacccacgc cggtagcttc    1320
gacatctcct acctgcgctg catccccggc atgctggtga tgacccccag cgacgaggac    1380
gagctgcgca agctgctcac caccggctac ctgttcgatg gcccggccgc ggtgcgctat    1440
ccgcgcggca gcggccccaa ccatccgatc gatccggacc tgcaaccggt ggagatcggc    1500
aagggcgtgg tccgtcggcg cggcggcagg gtcgcactgc tggtcttcgg cgtgcagttg    1560
gcggaggcga tgaaggtcgc cgaaagcctc gacgccacgg tcgtcgacat gcgtttcgtc    1620
aaacccctcg acgaagccct ggtacgcgaa ttggcgggca gccacgaact gctggtgacc    1680
atcgaggaaa acgccgtgat gggcggcgcc ggctcggcgg tcggcgagtt cctcgccagc    1740
gagggcctcg aagtcccgct gctgcaactg ggcctgcccg actactacgt cgaacacgcc    1800
aagcccagcg agatgctcgc cgaatgcggc ctggatgccg cgggcatcga aaaggcagta    1860
cgccagcgtc tcgaccggca gtag                                           1884
```

<210> 7
<211> 2160
<212> DNA
<213> Lycopersicon esculentum

<400> 7

```
atggctttgt gtgcttatgc atttcctggg attttgaaca ggactggtgt ggtttcagat      60
tcttctaagg caaccccttt gttctctgga tggattcatg gaacagatct gcagtttttg     120
ttccaacaca agcttactca tgaggtcaag aaaaggtcac gtgtggttca ggcttcctta     180
tcagaatctg gagaatacta cacacagaga ccgccaacgc ctattttgga cactgtgaac     240
tatcccattc atatgaaaaa tctgtctctg aaggaactta aacaactagc agatgaacta     300
aggtcagata caattttcaa tgtatcaaag actgggggtc accttggctc aagtcttggt     360
gttgttgagc tgactgttgc tcttcattat gtcttcaatg caccgcaaga taggattctc     420
tgggatgttg gtcatcagtc ttatcctcac aaaatcttga ctggtagaag ggacaagatg     480
tcgacattaa ggcagacaga tggtcttgca ggatttacta agcgatcgga gagtgaatat     540
gattgctttg gcaccggcca cagttccacc accatctcag caggcctagg gatggctgtt     600
ggtagagatc taaaaggaag aaacaacaat gttattgccg taataggtga tggtgccatg     660
acagcaggtc aagcttatga agccatgaat aatgctggtt acctggactc tgacatgatt     720
gttatcttaa acgacaatag acaagtttct ttacctactg ctactctgga tgggccagtt     780
gctcctgttg gagctctaag tagtgctttg agcaggttac agtctaatag gcctctcaga     840
gaactaagag aagtcgcaaa gggagttact aagcagattg gtggtcctat gcatgagctt     900
gctgcaaaag ttgatgaata tgctcgtggc atgattagtg gttctggatc aacattgttt     960
gaagaacttg gactttacta tattggtcct gtggatggtc acaacattga tgatctaatt    1020
```

gcgattctca aagaggttag aagtactaaa acaacaggtc cagtactgat ccatgttgtc 1080
actgagaaag gcagaggtta tccatatgct gagagagctg cagataagta tcatggagtt 1140
gccaagtttg atccagcaac aggaaagcaa ttcaaagcca gtgccaagac acagtcctat 1200
acaacatatt ttgccgaggc tttaattgca gaagcagaag cagataaaga cattgttgca 1260
atccatgctg ccatgggggg tgggaccgga atgaaccttt tccatcgtcg cttcccaaca 1320
aggtgttttg atgttggaat agcagaacaa catgcagtaa cctttgctgc tggattggct 1380
tgtgaaggca ttaaaccttt ctgtgcaatc tattcgtctt tcatgcagag ggcttatgac 1440
caggtagtgc atgacgttga tttgcaaaag ctgcccgtga ggtttgcaat ggacagagca 1500
ggtcttgttg gagcagatgg tccaacacat tgtggtgcat ttgatgttac ttacatggca 1560
tgtcttccta acatggttgt aatggctcct tctgatgaag cggagctatt tcacatggta 1620
gcaactgctg ccgccattga tgacagacca agttgtttta gatacccaag aggaaatggg 1680
atcggtgtag agcttccggc tggaaacaaa ggaattcctc ttgaggttgg taaaggtagg 1740
atattgattg agggggagag agtggctcta ttgggatatg gctcagcagt gcagaactgt 1800
ttggatgctg ctattgtgct agaatcccgc ggcttacaag taacagttgc agatgcacgt 1860
ttctgcaaac cactggacca tgccctcata aggagccttg caaaatcaca tgaagtgcta 1920
atcactgtcg aagaaggatc aattggaggt tttggatctc atgttgttca gttcatggcc 1980
ttagatgggc ttcttgatgg caagttgaag tggagaccaa tagttcttcc tgatcgatac 2040
attgaccatg gatctcctgt tgatcagttg gcggaagctg gcctaacacc atctcacatt 2100
gcagcaacag tatttaacat acttggacaa accagagagg ctctagaggt catgacataa 2160

<210> 8
<211> 1916
<212> DNA
<213> Mycobacterium tuberculosis

<400> 8

atgctgcaac agatccgcgg gcccgctgat ctgcagcacc tttcccaggc gcagcttcgg 60
gagctggccg ccgagatccg tgagttcctg atccacaagg ttgccgccac ggggggggcat 120
ctggggccga acctgggagt ggtggaactc accttggcgc tgcaccgggt attcgactcg 180
ccgcacgatc cgatcatctt cgacaccggt caccaggcct acgtccacaa gatgttgacc 240
ggacgcagcc aggacttcgc aaccctgcgt aagaagggcg ggttgtcggg gtatccgtct 300
cgtgccgaga gcgagcacga ctgggtggag tcgagccacg ccagcgcggc gctgtcgtac 360
gcggacgggt tggccaaggc gttcgagttg accggacacc gcaaccggca tgtggtcgcg 420
gtggtcggtg acggtgcgct caccggcggt atgtgctggg aggcgctgaa caatatcgcc 480
gcatcccgcc ggccggtgat tatcgtggtc aacgacaatg ggcgcagcta cgcgcccaca 540
atcgggggcg tcgccgacca tctggccacg ctgcggctgc agccggccta cgagcaggcg 600
ctggagacgg gccgcgacct ggtgcgcgcg gtgccgcttg tcggcggtct gtggtttcga 660
tcctgcacag cgtcaaggcc ggcatcaagg actcgctgtc gccgcagttg ctgttcaccg 720
acctcgggtt gaagtacgtc ggcccggtcg acggccatga cgagcgggcg gtggaggtcg 780
cgctgcgcag cgcgcggcgc ttcggtgcac cggtgatcgt gcacgtcgtc acccgcaagg 840
gcatgggcta cccgccggcc gaggccgacc aggccgagca gatgcattcc acggtcccga 900
tcgatccggc caccggacaa gccaccaagg tggccggccc aggctggacg gcgaccttct 960
ctgatgcact tatcggctac gcccagaaac gccgtgacat cgtggccatt accgcggcca 1020
tgccgggccc caccgggctg accgcgttcg ggcagcgctt cccggatcga ttgttcgacg 1080
tcgggatcgc cgagcaacac gcgatgacgt cggcggccgg gttggcgatg ggtgggctgc 1140
accccgtggt ggcgatctac tcgacgttcc tgaaccgggc gttcgaccag atcatgatgg 1200
atgtggcgct gcacaagctg ccggtcacca tggtgctgga ccgtgccggg atcaccggta 1260
gcgacggcgc cagccacaac ggaatgtggg acttgtcgat gctgggtatc gtgcccggca 1320
tccgggtggc agcgcccaga gacgccaccc ggttgcgtga agaactcggc gaggcgctcg 1380
acgtcgacga cggcccgacg gcgttacggt tccccaaagg tgatgtggga gaagatattt 1440
cggctttgga gcggcgtgga ggcgtggatg tgctggcggc gcccgccgat ggtttgaacc 1500
acgacgtcct gttggtggcc atcggcgcgt tcgcaccgat ggcgttggcg gtggccaagc 1560
ggctgcacaa ccagggggatc ggtgtgacgg tgatcgaccc gcgctgggtg ttgccggtgt 1620
ctgacggtgt gcgcgaactg gcggtgcagc acaagctgct cgtcacgcta gaggacaacg 1680
gggtcaacgg tggggcgggg tcagcggtgt cggccgcgct gcggcgcgcg gagatcgacg 1740
tgccctgccg cgatgtcggg ttgccgcagg agttctacga gcacgcgtct cgaagcgagg 1800
tgctggccga tctggggctt accgaccagg acgtggcccg gcggatcacc ggctgggtcg 1860
ccgcgctggg taccggggtg tgtgcgtccg acgcgattcc agaacatctc gactaa 1916

<210> 9

<211> 1914
<212> DNA
<213> Rhodobacter sphaeroides

<400> 9

```
atgaccgaca gaccctgcac gccgacgctc gaccgggtga cgctcccggt ggacataaag      60
ggcctcacgg accgtgagtt gcgctcgctg gccgacgagc tgcgggccga aacgatctcg     120
gccgtgtcgg tgacgggcgg gcatctgggc gcaggcctcg gcgtggtgga gttgacggtt     180
gcgctgcatg cgatcttcga tgcgccccgc gacaagatca tctgggacgt gggccaccag     240
tgctaccccc acaagatcct gaccgggcgg cgcgaccgca tccgcaccct gcggcagggc     300
gggggtctct cgggcttcac caagcgctcc gagagcccct atgactgttt cggcgcgggc     360
cattcctcga cctcgatctc ggccgcggtg ggctttgccg cggcacgcga gatgggcggc     420
gacacgggcg acgcggtggc ggtgatcggc gacggctcga tgtcggccgg catggccttc     480
gaggcgctga accacggcgg gcacctgaag aaccgggtga tcgtgatcct gaacgacaac     540
gagatgagca tcgcgccgcc ggtgggggcg ctgtcgtcct atctctcgcg gctctatgcg     600
ggcgcgccgt tccaggactt caaggcggcc gccaagggag cgctcgggct tctgcccgaa     660
ccgttccagg agggcgcgcg ccgcgccaag gagatgctga agagcgtcac cgtcggcggc     720
acgctcttcg aggagctggg tttctcctat gtcggcccga tcgacgggca cgatctcgac     780
cagcttctgc cggtgctgcg gaccgtcaag cagcgggcgc atgcgccggt gctgatccat     840
gtcatcacca agaagggcag ggctatgct ccggccgagg ccgcgcgcga ccgtggccat     900
gccacgaaca agttcaacgt cctgaccggc gcgcaggtga agccggtctc gaacgccccc     960
tcctatacca aggtcttcgc ccagagcctc atcaaggagg ccgaggtcga cgagcggatc    1020
tgcgcggtga cggccgccat gccggacggg acggggctca acctcttcgg cgagcggttt    1080
ccgaagcgca ccttcgatgt gggcatcgcg gaacagcatg cggtgacctt ctcggcggcg    1140
cttgcggcag gcggcatgcg gcccttctgc gccatctatt ccaccttcct ccagcgcggc    1200
tacgaccaga tcgtgcatga cgtggcaatc cagcgcctgc cggtgcgctt tgccatcgac    1260
cgcgccggcc tcgtgggggc ggacggcgcc acccatgcgg gctcgttcga tgtggccttc    1320
ctgtcgaacc tgcccggcat cgtggtgatg gccgccgccg acgaggccga gctcgtccat    1380
atggtagcca ccgccgccgc ccatgacgaa gggcccatcg ccttccgcta tccgcgcggc    1440
gacggcgtgg gggtcgaggt gccggtgaag ggcgtgccgc tccagatcgg ccgtggccgg    1500
gtggtgagcg agggcacgcg aatcgcgctc ctgtccttcg gcacccgtct ggccgaggtg    1560
caggtggccg ccgaggcgct ggctgcgcgc gggatctctc ccacggttgc ggatgcgcgc    1620
tttgcaaagc cgctcgaccg ggatctgatc ctgcagctcg cggcccatca cgaggcgctc    1680
attaccatcg aggagggcgc catcggcggc ttcggcagcc atgtggcgca gcttctggcc    1740
gaggccgggg tcttcgaccg cggcttccgg tatcgctcga tggtgctgcc cgacacgttc    1800
atcgaccaca acagcgccga agtgatgtat gccaccgccg ggctgaatgc ggccgacata    1860
gagcggaagg cgctggagac gctgggggtg gaggtcctcg cccgccgcgc ctga          1914
```

<210> 10
<211> 1947
<212> DNA
<213> Rhodobacter sphaeroides

<400> 10

```
atgaccaatc ccacccccgcg acccgaaacc ccgcttttgg atcgcgtctg ctgcccggcc      60
gacatgaagg cgctgagtga cgccgaactg gagcggctgg ccgacgaagt gcgttccgag     120
gtgatttcgg tcgttgccga gacgggagga catctggggt cctcgctggg ggtggttgag     180
ctgactgtcg cgctgcatgc ggtcttcaac acgcccaccg acaagctcgt ctgggacgtg     240
ggccaccagt gctacccccca caagatcctc accggccggc gcgagcagat gcgcaccctg     300
cgccagaagg gcggcctctc gggcttcacc aagcgctcgg aatccgccta cgacccgttc     360
ggcgcggctc attcctcgac ctcgatctcg gccgcgctcg gctttgccat gggtcgcgag     420
ctgggccagc ccgtgggcga cacgatcgcc gtgatcggcg acggctccat caccgcgggc     480
atggcctacg aggcactgaa ccacgcgggc catctgaaca agcgcctgtt cgtgatcctg     540
aacgacaatg acatgagcat cgcgccgccc gtgggggcgc ttgcgcgcta tctcgtgaat     600
ctctcctcga aggcgccctt cgccacgctg cgcgcggccg ccgacgggct cgaggcctcg     660
ctgccggggc cgctccgcga cggggcgcgc cgggcgcgcc agctcgtgac cgggatgccg     720
ggcgggggca cgctcttcga ggagctgggc ttcacctatg tcggccccat cgacggccac     780
gacatggagg cgctcctcca gacgctgcgc gcggcgcggg cccggaccac ggggccggtg     840
ctcatccatg tggtcacgaa gaagggcaag ggttacgccc ccgccgagaa tgcccccgac     900
aagtatcacg ggtgaacaa gttcgacccc gtcacgggcg agcagaagaa gtcggtggcc     960
aacgcgccga actacaccaa ggtcttcggc tccaccctga ccgaggaggc cgcgcgcgat    1020
ccgcgcatcg tggcgatcac cgccgctatg ccctcgggca ccggcgtcga catcatgcag    1080
```

```
aagcgtttcc cgaaccgcgt cttcgacgtg ggcatcgccg agcagcatgc cgtgaccttc    1140
gcggccggcc tcgccggggc cgggatgaag cccttctgcg cgatctattc ctcgttcctg    1200
caacggggtt acgaccagat cgcccatgac gtggcgctgc agaaccttcc cgtccgcttc    1260
gtgatcgacc gggcggggct cgtgggggcc gatggcgcga cccatgcggg ggccttcgac    1320
gttggcttca tcacttcgct gcccaacatg accgtgatgg ccgcggccga cgaggccgag    1380
ctcatccaca tgatcgccac cgccgtggcc ttcggcgagg gccccatcgc cttccgcttc    1440
ccgcggggcg aggggggtggg cgtcgagatg cccgagcgcg ggacggtgct ggagcccggc    1500
cggggccgcg tggtgcgcga agggacggat gtcgcgatcc tctccttcgg cgcgcatctg    1560
cacgaggcct tgcaggcggc gaaacttctc gaggccgagg gggtgagcgt gaccgtggcc    1620
gacgcccgct tctcgcgccc gctcgacacg gggcacatcg accagctcgt gcgccatcac    1680
gcggcgctgg taacggtgga gcagggggcc atgggcggct tcggcgccta tgtcatgcac    1740
tgtctcgcca attccggcgg cttcgacggg ggcctcgcgc tccgggtcat gacgctgccc    1800
gaccgcttca tcgagcaggc gagccccgag gacatgtatg ccgatgcggg gctgcgggcc    1860
gaggatatcg cggccaccgc gcggggcgcg ctcgcccggg ggcgcgtgat gccgctccgg    1920
cagacggcaa agccgcgggc ggtctga                                        1947
```

<210> 11
<211> 1911
<212> DNA
<213> Synechococcus sp. PC 6301

<400> 11

```
atgcatctca gcgaaattac ccatcccaac cagctccacg ggttgtcggt tgctcagctt      60
gagcaaattg gccaccagat tcgtgagaag cacctgcaga cggttgcagc gaccggtggg     120
cacctcgggc cgggcttggg cgtggtggaa ttgaccctag cgctttacca aacgctcgat     180
ctcgatcgcg acaaagtggt ttgggacgtt ggccaccaag cctatcccca caagctgctg     240
acagggcgct atcacaactt ccataccttg cggcaaaagg atggcattgc gggctacccg     300
aagcgcacgg aaaaccgctt cgatcatttc ggtgccggtc acgcttccac cagtatttct     360
gctggcctcg gtatggctct agcacgggat gcccagggcg aagactaccg atgtgtcgct     420
gtgattggtg atggatcgct caccggtggc atggccttgg aagccatcaa ccacgctggt     480
cacttgccca aaacacggct gttggtcgtg ctcaacgaca atgacatgtc gatctcgccc     540
aacgtgggtg cgctctctcg ctatctgaat aagattcggg ttagtgagcc gatgcagttg     600
ctcaccgatg gtttgaccca ggggatgcaa caaattccct tcgtcggcgg cgccattacc     660
caaggctttg agccggttaa ggaaggcatg aagcgcctct cctacagcaa gattggggcg     720
gtctttgaag agctgggctt cacctacatg ggccagtgg atggtcacaa ccttgaagaa     780
ctgatcgcca ccttccgcga agcgcacaaa cacaccggac cagtactcgt ccacgttgcc     840
acaaccaagg gtaagggcta tccctacgct gaagaagatc aggttggcta tcatgcccaa     900
aatccctttg atctggcgac agggaaggct aaaccagctt caaaaccgaa gccgcctagc     960
tattccaaag tgtttggcca aaccctgacg accttggcca agagcgatcg ccgcattgtc    1020
gggattacgg ctgcgatggc gacaggcacc ggcttggaca ttctccagaa ggcgctgccg    1080
aagcaataca tcgatgttgg cattgccgaa cagcacgccg tggtgctagc tgccggtatg    1140
gcctgcgatg gcatgcgtcc ggtggtggca atctattcca ccttcctgca gcgggccttt    1200
gatcaagtca tccacgacgt ttgtatccaa aagctgcccg tcttcttctg cctcgatcgc    1260
gcggggatag ttggcgcgga tggcccgact caccaaggca tgtacgacat tgcttacctg    1320
cggctgattc ccaacatggt gctgatggca ccgaaagatg aggccgaact gcagcggatg    1380
ctagtgacgg gtattgaata cgacggcccg atcgccatgc gtttcccgcg cgggaatggt    1440
attggcgtac ccctgccgga agaaggctgg gagtcgctcc cgattgggaa agcagagcaa    1500
ctgcgccaag gcgatgattt gctgatgttg gcttacggct cgatggtcta tccggccctg    1560
cagacggcag aactgctgaa tgagcacggc atctcagcta ctgtgatcaa tgcccgcttc    1620
gccaagccct tagatgagga actgattgtg ccgctggcgc gccagatcgg caaagtcgtc    1680
acctttgagg aaggctgcct acccggcggc tttggctccg cgattatgga gtccttgcag    1740
gcccatgatc tgcaggttcc ggtgttgccg atcggtgttc ccgatctctt ggtggaacat    1800
gccagccctg atgaatctaa acaggagttg ggcctgacgc cgcgtcagat ggccgatcgc    1860
atcctcgaaa agtttggaag ccgtcaacgg attggtgctg cttcggcttg a             1911
```

<210> 12
<211> 1863
<212> DNA
<213> Escherichia coli

<400> 12

```
atgagttttg atattgccaa atacccgacc ctggcactgg tcgactccac ccaggagtta     60
cgactgttgc cgaaagagag tttaccgaaa ctctgcgacg aactgcgccg ctatttactc    120
gacagcgtga gccgttccag cgggcacttc gcctccgggc tgggcacggt cgaactgacc    180
gtggcgctgc actatgtcta caacaccccg tttgaccaat tgatttggga tgtggggcat    240
caggcttatc cgcataaaat tttgaccgga cgccgcgaca aaatcggcac catccgtcag    300
aaaggcggtc tgcacccgtt cccgtggcgc ggcgaaagcg aatatgacgt attaagcgtc    360
gggcattcat caacctccat cagtgccgga attggtattg cggttgctgc cgaaaaagaa    420
ggcaaaaatc gccgcaccgt ctgtgtcatt ggcgatggcg cgattaccgc aggcatggcg    480
tttgaagcga tgaatcacgc gggcgatatc cgtcctgata tgctggtgat tctcaacgac    540
aatgaaatgt cgatttccga aaatgtcggc gcgctcaaca accatctggc acagctgctt    600
tccggtaagc tttactcttc actgcgcgaa ggcgggaaaa aagttttctc tggcgtgccg    660
ccaattaaag agctgctcaa acgcaccgaa gaacatatta aaggcatggt agtgcctggc    720
acgttgtttg aagagctggg ctttaactac atcggcccgg tggacggtca cgatgtgctg    780
gggcttatca ccacgctaaa gaacatgcgc gacctgaaag gcccgcagtt cctgcatatc    840
atgaccaaaa aaggtcgtgg ttatgaaccg gcagaaaaag acccgatcac tttccacgcc    900
gtgcctaaat ttgatccctc cagcggttgt ttgccgaaaa gtagcggcgg tttgccgagc    960
tattcaaaaa tctttggcga ctggttgtgc gaaacggcag cgaaagacaa caagctgatg   1020
gcgattactc cggcgatgcg tgaaggttcc ggcatggtcg agttttcacg taaattcccg   1080
gatcgctact tcgacgtggc aattgccgag caacacgcgg tgacctttgc tgcgggtctg   1140
gcgattggtg ggtacaaacc cattgtcgcg atttactcca ctttcctgca acgcgcctat   1200
gatcaggtgc tgcatgacgt ggcgattcaa aagcttccgg tcctgttcgc catcgaccgc   1260
gcgggcattg ttggtgctga cggtcaaacc catcagggtg cttttgatct ctcttacctg   1320
cgctgcatac cggaaatggt cattatgacc ccgagcgatg aaaacgaatg tcgccagatg   1380
ctctataccg gctatcacta taacgatggc ccgtcagcgg tgcgctaccc gcgtggcaac   1440
gcggtcggcg tggaactgac gccgctggaa aaactaccaa ttggcaaagg cattgtgaag   1500
cgtcgtggcg agaaactggc gatccttaac tttggtacgc tgatgccaga agcggcgaaa   1560
gtcgccgaat cgctgaacgc cacgctggtc gatatgcgtt ttgtgaaacc gcttgatgaa   1620
gcgttaattc tggaaatggc cgccagccat gaagcgctgg tcaccgtaga agaaaacgcc   1680
attatgggcg gcgcaggcag cggcgtgaac gaagtgctga tggcccatcg taaaccagta   1740
cccgtgctga acattggcct gccggacttc tttattccgc aaggaactca ggaagaaatg   1800
cgcgccgaac tcggcctcga tgccgctggt atggaagcca aaatcaaggc ctggctggca   1860
taa                                                                 1863
```

<210> 13
<211> 1914
<212> DNA
<213> Neisseria meningitidis

<400> 13

```
atgaacccaa gccccctact cgacctgatt gacagcccgc aagatttgcg ccgtctggac     60
aaaaaacagc tgccgcgcct tgccggcgag ttgcgcacct ttctgctgga atctgtcggg    120
cagaccggcg ggcatttcgc cagcaatttg ggcgcggtcg agctgacggt tgcgctgcac    180
tacgtttaca acacgcccga agacaagctg gtgtgggatg tcggacacca aagctatccg    240
cacaaaattc ttaccggacg taaaaaccag atgcacacca tgcgccaata tggcggtttg    300
gcgggttttc cgaaacgttg cgagtccgag tacgacgcgt tcggcgtggg gcattcctcc    360
acctccatcg gcgcggcgtt gggcatggcg gcggcggaca aacagttggg cagcgaccgc    420
cgcagcgtcg ccatcatcgg cgacggcgcg atgacggcgg gtcaggcgtt tgaagccttg    480
aactgcgcgg gcgatatgga tgtggatttg ctggtcgtcc tcaacgacaa cgaaatgtcg    540
atttccccca acgtcggtgc gttgcccaaa taccttgcca gcaacgtcgt gcgcgatatg    600
cacggactgt tgagtaccgt caaagcgcaa acgggcaagg tattagacaa aatacccggc    660
gcgatggagt ttgcccaaaa agtcgaacat aaaatcaaaa cccttgccga agaagccgaa    720
cacgccaaac agtcactgtc tttgtttgaa aacttcggct tccgctatac cggccccgtg    780
gacggacaca acgtcgaaaa tctggtcgat gtattggaag acctgcgcgg acgcaaaggc    840
ccgcagcttc tgcacgtcat caccaaaaag ggcaacggct acaaactcgc cgaaaacgat    900
cccgtcaaat accacgccgt cgccaacctg cctaaagaaa gcgcggcgca aatgccgtct    960
gaaaaagaac ccaagcccgc cgccaaaccg acctataccc aagtgttcgg caaatggctg   1020
tgcgaccggg cggcggcaga ttcccgactg gttgcgatta cccccgccat gcgcgagggc   1080
agcggcttgg ttgagtttga acaacgattc cccgaccgct atttcgatgt cggcatcgcc   1140
gagcagcacg ccgttacctt tgccggcggt ttggcttgcg aagggatgaa gcccgtcgtg   1200
gcgatttatt ccaccttttt acaacgcgcc tacgaccaac tggtgcacga catcgccctg   1260
caaaacctgc ccgttttgtt tgccgtcgac cgcgcgggca tcgtcggcgc ggacggcccg   1320
```

```
acccatgccg gtttgtacga tttaagcttt ttgcgctgca ttccgaatat gattgtcgcc   1380
gcgccgagcg atgaaaatga atgccgcctg ctgctttcga cctgctatca ggcagacgcg   1440
cccgccgccg tccgctatcc gcgcggcacg ggtacgggcg tgccggtttc agacggcatg   1500
gaaaccgtgg aaatcggcaa gggcattatc cgccgcgaag gtgagaaaac cgcattcatt   1560
gccttcggca gtatggtcgc ccctgcattg gcggtcgccg gaaaactgaa cgccaccgtc   1620
gccgatatgc gcttcgtcaa accgatagac gaagagttga ttgtccgcct tgcccgaagc   1680
cacgaccgca tcgttaccct tgaagaaaac gccgaacagg gcggcgcagg cagcgcggtg   1740
ctggaagtgt tggcgaaaca cggcatctgc aaacccgtct tgcttttggg cgttgccgat   1800
accgtaaccg gacacggcga tccgaaaaaa cttttagacg atttgggctt gagtgccgaa   1860
gcggtggaac ggcgtgtgcg cgcgtggctg tcggatcggg atgcggcaaa ttaa         1914
```

<210> 14
<211> 1878
<212> DNA
<213> Haemophilus influenzae

<400> 14

```
atgactaaca atatgaacaa ttatcctctt ttatctttaa ttaattctcc agaagatttg      60
cgtcttttaa ataaagatca gctaccacaa ctctgtcaag aattacgtgc ttatctttta     120
gaatctgtta gtcaaactag cggacattta gcgtcaggtt taggcactgt agagctaacc     180
gttgcgctgc attatgtata taagacgcca tttgatcagt taatttggga tgtgggacat     240
caagcttatc cacataaaat cctaacgggt cgccgagagc aaatgtccac aattcgccaa     300
aaagacggta ttcatccttt tccttggcgt gaagaaagtg aatttgatgt attaagtgtt     360
ggtcactcct ctacgtctat tagtgcggga ttaggcattg ccgttgccgc agaacgagaa     420
aatgcaggta gaaaaacagt atgcgtaatc ggtgatggcg caattactgc gggaatggca     480
tttgaggcat taaatcacgc gggggcattg catacagata tgttagttat tttaaatgat     540
aacgaaatgt ctatttcaga aaacgttggt gcattaaata atcatcttgc gcgtattttc     600
tctggctctc tttactctac gcttcgtgat ggcagtaaaa aaatccttga taaagttcct     660
ccaatcaaaa attttatgaa aaaaaccgaa gaacatatga aaggtgtaat gttttcgcca     720
gaaagtacat tatttgaaga actcggtttt aactatattg gcccagtgga tgggcataac     780
attgatgaat tagtggctac gcttacgaat atgcgtaatc tgaaaggccc acaatttttg     840
catataaaaa cgaaaaaagg taaaggatac gcacccgcag aaaaagatcc gattggtttc     900
cacggtgtac ctaaatttga tccaatcagt ggcgaattgc ccaaaaacaa tagtaaacca     960
acttattcga aaatttttgg cgattggcta tgtgaaatgg cagaaaaaga tgccaaaatt    1020
ataggtatca cacctgcaat gcgtgagggt tcaggtatgg tagaattttc ccaacgcttc    1080
ccaaaacaat attttgacgt agcgattgca gaacagcacg ctgtcacgtt tgccacagga    1140
cttgcaattg gcggatataa acctgtcgtc gcaatttact cgacattttt acaacgtgct    1200
tacgatcaat taattcacga tgttgccatt caaaatctcc ctgtgctatt tgcaattgat    1260
cgagcaggga tagttggtgc agatggggct acacatcaag gtgcattcga tattagcttt    1320
atgcgttgca ttccaaatat gatcattatg acgccgagtg atgaaaatga atgccgtcaa    1380
atgctctata caggttatca atgtggaaaa cctgcggcag tgcgctaccc tcgcggaaat    1440
gccgttggtg taaaacttac tcctttagaa atgcttccta ttggtaaatc acgtttaatt    1500
cgaaaaggtc aaaaaattgc gattttaaat tttggtactc tattaccatc cgctttagag    1560
ttatcagaaa aactcaatgc aacggttgtc gatatgcgtt ttgtgaaacc gattgatatt    1620
gaaatgatta atgtgcttgc acaaactcac gattatttgg tcacattgga agaaaatgca    1680
attcaaggtg gagcgggatc tgctgttgcg gaagtactaa attcatcagg aaaatcaacc    1740
gcacttttac aacttggctt gccagattat tttattccac aagcgacaca gcaagaagca    1800
ttggcagatt taggattgga tacaaaaggc attgaagaaa aaattctcaa ctttattgca    1860
aaacaaggta atttataa                                                  1878
```

<210> 15
<211> 627
<212> PRT
<213> Pseudomonas aeruginosa

<400> 15

```
Met Pro Lys Thr Leu His Glu Ile Pro Arg Glu Arg Pro Ala Thr Pro
1               5                   10                  15
Leu Leu Asp Arg Ala Ser Ser Pro Ala Glu Leu Arg Arg Leu Gly Glu
            20                  25                  30
Ala Asp Leu Glu Thr Leu Ala Asp Glu Leu Arg Gln Tyr Leu Leu Tyr
```

```
        35                  40                  45
Thr Val Gly Gln Thr Gly Gly His Phe Gly Ala Gly Leu Gly Val Val
    50                  55                  60
Glu Leu Thr Ile Ala Leu His Tyr Val Phe Asp Thr Pro Asp Asp Arg
65                  70                  75                  80
Leu Val Trp Asp Val Gly His Gln Ala Tyr Pro His Lys Ile Leu Thr
                85                  90                  95
Glu Arg Arg Glu Leu Met Gly Thr Leu Arg Gln Lys Asn Gly Leu Ala
            100                 105                 110
Ala Phe Pro Arg Arg Ala Glu Ser Glu Tyr Asp Thr Phe Gly Val Gly
        115                 120                 125
His Ser Ser Thr Ser Ile Ser Ala Ala Leu Gly Met Ala Ile Ala Ala
    130                 135                 140
Arg Leu Gln Gly Lys Glu Arg Lys Ser Val Ala Val Ile Gly Asp Gly
145                 150                 155                 160
Ala Leu Thr Ala Gly Met Ala Phe Glu Ala Leu Asn His Ala Ser Glu
                165                 170                 175
Val Asp Ala Asp Met Leu Val Ile Leu Asn Asp Asn Asp Met Ser Ile
            180                 185                 190
Ser His Asn Val Gly Gly Leu Ser Asn Tyr Leu Ala Lys Ile Leu Ser
        195                 200                 205
Ser Arg Thr Tyr Ser Ser Met Arg Glu Gly Ser Lys Lys Val Leu Ser
    210                 215                 220
Arg Leu Pro Gly Ala Trp Glu Ile Ala Arg Arg Thr Glu Glu Tyr Ala
225                 230                 235                 240
Lys Gly Met Leu Val Pro Gly Thr Leu Phe Glu Glu Leu Gly Trp Asn
                245                 250                 255
Tyr Ile Gly Pro Ile Asp Gly His Asp Leu Pro Thr Leu Val Ala Thr
            260                 265                 270
Leu Arg Asn Met Arg Asp Met Lys Gly Pro Gln Phe Leu His Val Val
        275                 280                 285
Thr Lys Lys Gly Lys Gly Phe Ala Pro Ala Glu Leu Asp Pro Ile Gly
    290                 295                 300
Tyr His Ala Ile Thr Lys Leu Glu Ala Pro Gly Ser Ala Pro Lys Lys
305                 310                 315                 320
Thr Gly Gly Pro Lys Tyr Ser Ser Val Phe Gly Gln Trp Leu Cys Asp
                325                 330                 335
Met Ala Ala Gln Asp Ala Arg Leu Leu Gly Ile Thr Pro Ala Met Lys
            340                 345                 350
Glu Gly Ser Asp Leu Val Ala Phe Ser Glu Arg Tyr Pro Glu Arg Tyr
        355                 360                 365
Phe Asp Val Ala Ile Ala Glu Gln His Ala Val Thr Leu Ala Ala Gly
    370                 375                 380
Met Ala Cys Glu Gly Met Lys Pro Val Val Ala Ile Tyr Ser Thr Phe
385                 390                 395                 400
Leu Gln Arg Ala Tyr Asp Gln Leu Ile His Asp Val Ala Val Gln His
                405                 410                 415
Leu Asp Val Leu Phe Ala Ile Asp Arg Ala Gly Leu Val Gly Glu Asp
            420                 425                 430
Gly Pro Thr His Ala Gly Ser Phe Asp Ile Ser Tyr Leu Arg Cys Ile
        435                 440                 445
Pro Gly Met Leu Val Met Thr Pro Ser Asp Glu Asp Glu Leu Arg Lys
    450                 455                 460
Leu Leu Thr Thr Gly Tyr Leu Phe Asp Gly Pro Ala Ala Val Arg Tyr
465                 470                 475                 480
Pro Arg Gly Ser Gly Pro Asn His Pro Ile Asp Pro Asp Leu Gln Pro
                485                 490                 495
Val Glu Ile Gly Lys Gly Val Val Arg Arg Arg Gly Gly Arg Val Ala
            500                 505                 510
Leu Leu Val Phe Gly Val Gln Leu Ala Glu Ala Met Lys Val Ala Glu
        515                 520                 525
```

```
Ser Leu Asp Ala Thr Val Val Asp Met Arg Phe Val Lys Pro Leu Asp
    530                 535                 540
Glu Ala Leu Val Arg Glu Leu Ala Gly Ser His Glu Leu Leu Val Thr
545                 550                 555                 560
Ile Glu Glu Asn Ala Val Met Gly Gly Ala Gly Ser Ala Val Gly Glu
                565                 570                 575
Phe Leu Ala Ser Glu Gly Leu Glu Val Pro Leu Leu Gln Leu Gly Leu
            580                 585                 590
Pro Asp Tyr Tyr Val Glu His Ala Lys Pro Ser Glu Met Leu Ala Glu
        595                 600                 605
Cys Gly Leu Asp Ala Ala Gly Ile Glu Lys Ala Val Arg Gln Arg Leu
    610                 615                 620
Asp Arg Gln
625
```

<210> 16
<211> 1896
<212> DNA
<213> Streptomyces sp. CL190

<400> 16

```
gtgacgattc tggagaacat ccggcaacca cgcgacctga aggcgctgcc cgaggagcag      60
ctgcacgaac tgtccgagga gatcaggcag ttcctggtgc acgcggtcac cagaaccggc     120
ggtcatctgg gacccaacct gggggtggtg gagctgacca tcgccctgca ccgggtcttc     180
gagtcgcccg tcgaccgcat cctgtgggac accggccacc agagctacgt acacaagctg     240
ctgacgggac gtcaggactt ctccaagctg cgcggcaagg gcggcctgtc cggctacccc     300
tcgcgcgagg agtccgagca cgacgtcatc gagaacagcc acgcctccac cgccctcggc     360
tgggccgacg gactcgccaa ggcccgccgg gtgcaggggg agaagggcca tgtcgtcgcc     420
gtcatcggcg gacgggcgct gaccggcggc atggcctggg aggccctgaa caacatcgcg     480
gccgccaagg accagccgct gatcatcgtc gtcaacgaca acgagcgctc ctacgcgccc     540
accatcggcg gcctcgccaa ccacctggcc accctgcgca ccaccgacgg ctacgagaag     600
gtcctcgcct ggggcaagga cgtcctgctg cgtaccccca tcgtcggcca ccccctctac     660
gaggccctgc acggcgccaa gaagggcttc aaggacgcct tcgccccgca gggcatgttc     720
gaggacctgg gcctgaagta cgtcggcccc atcgacgggc acgacatcgg cgcggtcgag     780
tccgcgctgc gccgcgccaa gcgcttccac gggccggtgc tggtgcactg cctcaccgtc     840
aagggccgcg gctacgaacc cgccctcgcc cacgaggagg accacttcca caccgtcggc     900
gtgatggacc cgctcacctg tgagcccctc tcgcccaccg acggcccgtc ctggacctcg     960
gtgttcggcg acgagatcgt acggatcggc gcggagcgcg aggacatcgt cgcgatcacc    1020
gccgcgatgc tccacccggt ggggctcgcc aggttcgccg accgcttccc ggaccgggtc    1080
tgggacgtcg gcatcgccga gcagcacgcg gccgtgtccg cggccgggct cgccaccggc    1140
ggactgcacc cggtcgtcgc cgtctacgcc accttcctca accgcgcctt cgaccagctc    1200
ctgatggacg tcgccctgca ccgctgcggt gtgaccttcg tcctggaccg ggccggcgtc    1260
acgggcgtcg acggcgcctc gcacaacggc atgtgggaca tgtccgtcct ccaggtcgtg    1320
cccggcctca ggatcgccgc cccgcgcgac gccgaccacg tgcgcgccca gctgcgggag    1380
gcggtcgccg tggacgacgc gccgacgctg atccgcttcc cgaaggagtc cgtcggcccg    1440
cggatcccgg ccctcgaccg ggtcggcggc ctcgatgtgc tgcaccgcga cgagcggccc    1500
gaggtgctgc tggtcgccgt gggcgtcatg gcacaggtct gcctccagac cgccgagctg    1560
ctccgggccc gcggcatcgg atgcacggtc gtcgacccgc gctgggtcaa gcccgtcgac    1620
cccgtgctgc ccccactcgc cgccgagcac cggctcgtcg ccgtcgtgga ggacaacagc    1680
cgggccgccg gggtcggttc ggcggtcgcc ctggcgctcg gggacgccga tgtcgacgta    1740
ccggtgcgcc gcttcggcat ccccgagcag ttcctcgcgc acgccaggcg cggtgaggtg    1800
ctcgccgaca tcgggctgac cccggtggag atcgccgggc ggatcggcgc gagcctgccc    1860
gtgcgggagg aaccggccga ggagcagccc gcatga                              1896
```

<210> 17
<211> 1857
<212> DNA
<213> Helicobacter pylori

<400> 17

```
gtgattttgc aaaataaaac ttttgattta aaccctaacg atattgcagg cttggagttg      60
gtgtgtcaaa cgctacggaa tcgtatttta gaagtggtga gcgctaatgg ggggcattta     120
agctcttctt taggggctgt ggagctgatt gtgggcatgc atgccttatt tgattgccaa     180
aaaaaccctt tcatttttga cacttcgcac caagcttacg cccacaagct tttaaccggg     240
cgctttgaaa gctttagcac tttaaggcaa ttcaagggtt tgagcggctt tactaaaccc     300
agcgagagcg catacgatta tttcatcgcc gggcatagtt ccacttcggt gtctataggc     360
gttggggtgg ctaaagcttt ttgtttgaaa caagcgctag gcatgcccat agctttatta     420
ggcgatggga gcattagtgc agggattttt tatgaagcct taaacgaact gggcgatagg     480
aaataccccca tgatcatgat tttaaacgat aatgaaatga gtatcagcac gcctattgga     540
gccttatcca aagcccttag ccagctgatg aaaggcccgt tttaccagtc tttccgctct     600
aaagttaaaa aaatcttaag caccttacct gaaagcgtga attacttagc gagtcgtttt     660
gaagaatctt tcaagctcat caccccgggc gtgtttttttg aagaattagg cattaactat     720
atagggccta ttaatgggca tgatttgagc gcgattattg aaaccttaaa attagccaaa     780
gagcttaaag agccggtgct aatccatgcg caaaccttaa agggcaaagg ctataagatc     840
gctgaagggc gctatgaaaa atggcatggg gtggggcctt ttgatttgga taccggcttg     900
tctaaaaaat ccaaaagcgc aatcttatcg cccactgaag cgtattctaa caccctttta     960
gaattagcta aaaaagatga aaaaatcgta ggcgtaaccg cggcgatgcc tagcggcaca    1020
ggattagaca aactcattga cgcttaccct ttgcgctttt ttgatgtcgc tatcgctgag    1080
caacacgctt taacttctag cagcgctatg gctaaagagg ggtttaaacc ttttgtgagc    1140
atctattcta cttttttgca gagggcttat gattctattg tgcatgacgc ttgtatttct    1200
agcttgccga ttaaattagc cattgacagg gctgggattg tgggcgaaga tggcgagacg    1260
caccaagggc ttttagacgt gtcgtatttg cgctctatcc ctaacatggt catttttgcc    1320
ccacgagaca atgagacttt aaaaaacgcc gtgcgttttg ccaatgaaca cgattcaagc    1380
ccttgcgcgt tccgataccc taggggggtcg tttgcgttaa aagagggggt ttttgagcct    1440
agcggttttg ttttaggcca aagcgaattg ttgaaaaaag agggcgaaat ttactcata     1500
ggctatggta atggcgtggg gcgggcgcat ttagtccaac tggctttaaa agaaaaaaac    1560
atagaatgcg ctctcttgga tctcaggttt ttaaagcctt tagatccaaa tttaagcgcg    1620
atcgttgccc cttatcaaaa gctctatgtt tttagcgata attacaagct tggagggggtg    1680
gctagcgcga ttttagagtt tttgagcgaa caaaatattt taaagcctgt taaaagcttt    1740
gaaatcattg atgaatttat catgcatggg aacaccgctt tagtggaaaa atccttagga    1800
ttagacacag agagtttgac tgacgctatt ttaaaagatt taggacaaga gagatga       1857
```

<210> 18
<211> 628
<212> PRT
<213> Aquifex aeolicus

<400> 18

```
Met Leu Glu Lys Tyr Glu Ile Leu Lys Asp Tyr Lys Gly Pro Phe Asp
 1              5                   10                  15
Ile Lys Asn Tyr Asp Tyr Glu Thr Leu Gln Lys Leu Ala Gln Glu Val
            20                  25                  30
Arg Asp Tyr Ile Ile Asn Val Thr Ser Lys Asn Gly Gly His Val Gly
        35                  40                  45
Pro Ser Leu Gly Val Val Glu Leu Thr Ile Ala Leu Leu Arg Val Phe
    50                  55                  60
Asn Pro Pro Glu Asp Val Ile Val Trp Asp Ile Gly His Gln Gly Tyr
65                  70                  75                  80
Pro Trp Lys Ile Leu Thr Asp Arg Lys Glu Gln Phe Pro Thr Leu Arg
                85                  90                  95
Gln Tyr Lys Gly Ile Ser Gly Phe Leu Arg Arg Glu Glu Ser Ile Tyr
            100                 105                 110
Asp Ala Phe Gly Ala Gly His Ser Ser Thr Ser Ile Ser Ala Ala Leu
        115                 120                 125
Gly Phe Arg Ile Gly Lys Asp Leu Lys Gly Glu Lys Glu Asp Tyr Val
    130                 135                 140
Ile Ala Val Ile Gly Asp Gly Ala Leu Thr Ala Gly Met Ala Tyr Glu
145                 150                 155                 160
Ala Leu Asn Asn Ala Gly His Ile Arg Pro Asp Arg Phe Ile Val Ile
                165                 170                 175
Leu Asn Asp Asn Glu Met Ser Ile Ser Pro Asn Val Gly Ala Ile Ser
            180                 185                 190
```

```
Thr Tyr Leu Asn Arg Ile Ile Ser Gly His Phe Val Gln Glu Thr Arg
        195                 200                 205
Gln Lys Ile Lys Asn Phe Leu Gln His Phe Gly Glu Thr Pro Leu Arg
    210                 215                 220
Ile Met Lys Leu Thr Glu Glu Phe Leu Lys Gly Leu Ile Ser Pro Gly
225                 230                 235                 240
Val Ile Phe Glu Glu Leu Gly Phe Asn Tyr Ile Gly Pro Ile Asp Gly
                245                 250                 255
His Asp Ile Lys Ala Leu Glu Asp Thr Leu Asn Asn Val Lys Asp Ile
            260                 265                 270
Lys Gly Pro Val Leu Leu His Val Tyr Thr Lys Lys Gly Lys Gly Tyr
        275                 280                 285
Lys Pro Ala Glu Glu Asn Pro Val Lys Trp His Gly Val Ala Pro Tyr
    290                 295                 300
Lys Val Glu Ser Gly Glu Ile Ile Lys Lys Ser Ser Pro Pro Thr Trp
305                 310                 315                 320
Thr Ser Val Phe Gly Lys Ala Leu Val Glu Leu Ala Glu Arg Asp Glu
                325                 330                 335
Lys Ile Val Ala Ile Thr Pro Ala Met Arg Glu Gly Ser Gly Leu Val
            340                 345                 350
Glu Phe Ala Lys Arg Phe Pro Asp Arg Phe Phe Asp Val Gly Ile Ala
        355                 360                 365
Glu Gln His Ala Cys Thr Phe Ala Ala Gly Leu Ala Ala Glu Gly Leu
    370                 375                 380
Arg Pro Val Ala Ala Tyr Tyr Ser Thr Phe Leu Gln Arg Ala Tyr Asp
385                 390                 395                 400
Gln Val Ile His Asp Val Ala Leu Gln Asn Leu Pro Val Thr Phe Ala
                405                 410                 415
Ile Asp Arg Ala Gly Leu Val Gly Asp Asp Gly Pro Thr His His Gly
            420                 425                 430
Val Phe Asp Leu Ser Tyr Leu Arg Cys Val Pro Asn Met Val Val Cys
        435                 440                 445
Ala Pro Lys Asp Glu Gln Glu Leu Arg Asp Leu Leu Tyr Thr Gly Ile
    450                 455                 460
Tyr Ser Gly Lys Pro Phe Ala Leu Arg Tyr Pro Arg Gly Ala Ala Tyr
465                 470                 475                 480
Gly Val Pro Thr Glu Gly Phe Lys Lys Ile Glu Ile Gly Thr Trp Glu
                485                 490                 495
Glu Leu Leu Glu Gly Glu Asp Cys Val Ile Leu Ala Val Gly Tyr Pro
            500                 505                 510
Val Tyr Gln Ala Leu Arg Ala Ala Glu Lys Leu Tyr Lys Glu Gly Ile
        515                 520                 525
Arg Val Gly Val Val Asn Ala Arg Phe Val Lys Pro Met Asp Glu Lys
    530                 535                 540
Met Leu Arg Asp Leu Ala Asn Arg Tyr Asp Thr Phe Ile Thr Val Glu
545                 550                 555                 560
Asp Asn Thr Val Val Gly Gly Phe Gly Ser Gly Val Leu Glu Phe Phe
                565                 570                 575
Ala Arg Glu Gly Ile Met Lys Arg Val Ile Asn Leu Gly Val Pro Asp
            580                 585                 590
Arg Phe Ile Glu His Gly Lys Gln Asp Ile Leu Arg Asn Leu Val Gly
        595                 600                 605
Ile Asp Ala Glu Gly Ile Glu Lys Ala Val Arg Asp Ala Leu Lys Gly
    610                 615                 620
Gly Arg Leu Ile
625
```

<210> 19
<211> 633
<212> PRT
<213> Bacillus subtilis

<400> 19

```
Met Asp Leu Leu Ser Ile Gln Asp Pro Ser Phe Leu Lys Asn Met Ser
1               5                   10                  15
Ile Asp Glu Leu Glu Lys Leu Ser Asp Glu Ile Arg Gln Phe Leu Ile
            20                  25                  30
Thr Ser Leu Ser Ala Ser Gly Gly His Ile Gly Pro Asn Leu Gly Val
        35                  40                  45
Val Glu Leu Thr Val Ala Leu His Lys Glu Phe Asn Ser Pro Lys Asp
    50                  55                  60
Lys Phe Leu Trp Asp Val Gly His Gln Ser Tyr Val His Lys Leu Leu
65                  70                  75                  80
Thr Gly Arg Gly Lys Glu Phe Ala Thr Leu Arg Gln Tyr Lys Gly Leu
                85                  90                  95
Cys Gly Phe Pro Lys Arg Ser Glu Ser Glu His Asp Val Trp Glu Thr
            100                 105                 110
Gly His Ser Ser Thr Ser Leu Ser Gly Ala Met Gly Met Ala Ala Ala
        115                 120                 125
Arg Asp Ile Lys Gly Thr Asp Glu Tyr Ile Ile Pro Ile Ile Gly Asp
    130                 135                 140
Gly Ala Leu Thr Gly Gly Met Ala Leu Glu Ala Leu Asn His Ile Gly
145                 150                 155                 160
Asp Glu Lys Lys Asp Met Ile Val Ile Leu Asn Asp Asn Glu Met Ser
                165                 170                 175
Ile Ala Pro Asn Val Gly Ala Ile His Ser Met Leu Gly Arg Leu Arg
            180                 185                 190
Thr Ala Gly Lys Tyr Gln Trp Val Lys Asp Glu Leu Glu Tyr Leu Phe
        195                 200                 205
Lys Lys Ile Pro Ala Val Gly Gly Lys Leu Ala Ala Thr Ala Glu Arg
    210                 215                 220
Val Lys Asp Ser Leu Lys Tyr Met Leu Val Ser Gly Met Phe Phe Glu
225                 230                 235                 240
Glu Leu Gly Phe Thr Tyr Leu Gly Pro Val Asp Gly His Ser Tyr His
                245                 250                 255
Glu Leu Ile Glu Asn Leu Gln Tyr Ala Lys Lys Thr Lys Gly Pro Val
            260                 265                 270
Leu Leu His Val Ile Thr Lys Lys Gly Lys Gly Tyr Lys Pro Ala Glu
        275                 280                 285
Thr Asp Thr Ile Gly Thr Trp His Gly Thr Gly Pro Tyr Lys Ile Asn
    290                 295                 300
Thr Gly Asp Phe Val Lys Pro Lys Ala Ala Ala Pro Ser Trp Ser Gly
305                 310                 315                 320
Leu Val Ser Gly Thr Val Gln Arg Met Ala Arg Glu Asp Gly Arg Ile
                325                 330                 335
Val Ala Ile Thr Pro Ala Met Pro Val Gly Ser Lys Leu Glu Gly Phe
            340                 345                 350
Ala Lys Glu Phe Pro Asp Arg Met Phe Asp Val Gly Ile Ala Glu Gln
        355                 360                 365
His Ala Ala Thr Met Ala Ala Ala Met Ala Met Gln Gly Met Lys Pro
    370                 375                 380
Phe Leu Ala Ile Tyr Ser Thr Phe Leu Gln Arg Ala Tyr Asp Gln Val
385                 390                 395                 400
Val His Asp Ile Cys Arg Gln Asn Ala Asn Val Phe Ile Gly Ile Asp
                405                 410                 415
Arg Ala Gly Leu Val Gly Ala Asp Gly Glu Thr His Gln Gly Val Phe
            420                 425                 430
Asp Ile Ala Phe Met Arg His Ile Pro Asn Met Val Leu Met Met Pro
        435                 440                 445
Lys Asp Glu Asn Glu Gly Gln His Met Val His Thr Ala Leu Ser Tyr
    450                 455                 460
Asp Glu Gly Pro Ile Ala Met Arg Phe Pro Arg Gly Asn Gly Leu Gly
```

```
465                 470                 475                 480
Val Lys Met Asp Glu Gln Leu Lys Thr Ile Pro Ile Gly Thr Trp Glu
                485                 490                 495
Val Leu Arg Pro Gly Asn Asp Ala Val Ile Leu Thr Phe Gly Thr Thr
            500                 505                 510
Ile Glu Met Ala Ile Glu Ala Ala Glu Glu Leu Gln Lys Glu Gly Leu
        515                 520                 525
Ser Val Arg Val Val Asn Ala Arg Phe Ile Lys Pro Ile Asp Glu Lys
    530                 535                 540
Met Met Lys Ser Ile Leu Lys Glu Gly Leu Pro Ile Leu Thr Ile Glu
545                 550                 555                 560
Glu Ala Val Leu Glu Gly Gly Phe Gly Ser Ser Ile Leu Glu Phe Ala
                565                 570                 575
His Asp Gln Gly Glu Tyr His Thr Pro Ile Asp Arg Met Gly Ile Pro
            580                 585                 590
Asp Arg Phe Ile Glu His Gly Ser Val Thr Ala Leu Leu Glu Glu Ile
        595                 600                 605
Gly Leu Thr Lys Gln Gln Val Ala Asn Arg Ile Arg Leu Leu Met Pro
    610                 615                 620
Pro Lys Thr His Lys Gly Ile Gly Ser
625                 630
```

<210> 20
<211> 735
<212> PRT
<213> Chlamydomonas reinhardtii

<400> 20

```
Met Leu Arg Gly Ala Val Ser His Gly Pro Ala Val Ala Asp Arg Ala
1               5                   10                  15
Ala Ala Gly Pro Ala Arg Cys Ala Ala Pro Val Ala Arg Gly Val Arg
            20                  25                  30
Ser Ala Ala Pro Thr Arg Gln Arg Arg Ala Glu Ala Ser Val Asn Ala
        35                  40                  45
Pro Arg Ala Gly Pro Ala Gly Ser Tyr Ser Gly Glu Trp Asp Lys Leu
    50                  55                  60
Ser Val Glu Glu Ile Asp Glu Trp Arg Asp Val Gly Pro Lys Thr Pro
65                  70                  75                  80
Leu Leu Asp Thr Val Asn Tyr Pro Val His Leu Lys Asn Phe Asn Asn
                85                  90                  95
Glu Gln Leu Lys Gln Leu Cys Lys Glu Leu Arg Ser Asp Ile Val His
            100                 105                 110
Thr Val Ser Arg Thr Gly Gly His Leu Ser Ser Ser Leu Gly Val Val
        115                 120                 125
Glu Leu Thr Val Ala Met His Tyr Val Phe Asn Thr Pro Glu Asp Lys
    130                 135                 140
Ile Ile Trp Asp Val Gly His Gln Ala Tyr Gly His Lys Ile Leu Thr
145                 150                 155                 160
Gly Arg Arg Lys Gly Met Ala Thr Ile Arg Gln Thr Asn Gly Leu Ser
                165                 170                 175
Gly Phe Thr Lys Arg Asp Glu Ser Glu Tyr Asp Pro Phe Gly Ala Gly
            180                 185                 190
His Ser Ser Thr Ser Ile Ser Ala Ala Leu Gly Met Ala Val Gly Arg
        195                 200                 205
Asp Val Lys Gly Lys Lys Asn Ser Val Ile Ala Val Ile Gly Asp Gly
    210                 215                 220
Ala Ile Thr Gly Gly Met Ala Tyr Glu Ala Met Asn His Ala Gly Phe
225                 230                 235                 240
Leu Asp Lys Asn Met Ile Val Ile Leu Asn Asp Asn Gln Gln Val Ser
                245                 250                 255
Leu Pro Thr Gln Tyr Asn Asn Lys Asn Gln Asp Pro Val Gly Ala Leu
```

```
                260                 265                 270
Ser Ser Ala Leu Ala Arg Leu Gln Ala Asn Arg Pro Leu Arg Glu Leu
            275                 280                 285
Arg Glu Ile Ala Lys Gly Val Thr Lys Gln Leu Pro Asp Val Val Gln
    290                 295                 300
Lys Ala Thr Ala Lys Ile Asp Glu Tyr Ala Arg Gly Met Ile Ser Gly
305                 310                 315                 320
Thr Gly Ser Thr Leu Phe Glu Glu Leu Gly Leu Tyr Tyr Ile Gly Pro
                325                 330                 335
Val Asp Gly His Asn Leu Asp Asp Leu Ile Ala Val Leu Ser Glu Val
            340                 345                 350
Arg Ser Ala Glu Thr Val Gly Pro Val Leu Val His Val Val Thr Glu
        355                 360                 365
Lys Gly Arg Gly Tyr Leu Pro Ala Glu Thr Ala Gln Asp Lys Met His
    370                 375                 380
Gly Val Val Lys Phe Asp Pro Arg Thr Gly Lys Gln Val Gln Ala Lys
385                 390                 395                 400
Thr Lys Ala Met Ser Tyr Thr Asn Tyr Phe Ala Asp Ala Leu Thr Ala
                405                 410                 415
Glu Ala Glu Arg Asp Ser Arg Ile Val Ala Val His Ala Ala Met Ala
            420                 425                 430
Gly Gly Thr Gly Leu Tyr Arg Phe Glu Lys Lys Phe Pro Asp Arg Thr
        435                 440                 445
Phe Asp Val Gly Ile Ala Glu Gln His Ala Val Thr Phe Ala Ala Gly
    450                 455                 460
Leu Ala Cys Glu Gly Leu Val Pro Phe Cys Thr Ile Tyr Ser Thr Phe
465                 470                 475                 480
Met Gln Arg Gly Tyr Asp Gln Ile Val His Asp Val Ser Leu Gln Lys
                485                 490                 495
Leu Pro Val Arg Phe Ala Met Asp Arg Ala Gly Leu Val Gly Ala Asp
            500                 505                 510
Gly Ser Thr His Cys Gly Ala Phe Asp Val Thr Phe Met Ala Ser Leu
        515                 520                 525
Pro His Met Ile Thr Met Ala Pro Ser Asn Glu Ala Glu Leu Ile Asn
    530                 535                 540
Met Val Ala Thr Cys Ala Ala Ile Asp Asp Ala Pro Ser Cys Phe Arg
545                 550                 555                 560
Phe Pro Arg Gly Asn Gly Leu Gly Leu Asp Leu Ala Ala Tyr Gly Ile
                565                 570                 575
Ser Lys Asp Leu Lys Gly Val Pro Leu Glu Val Gly Lys Gly Val Val
            580                 585                 590
Arg Arg Gln Gly Lys Asp Val Cys Leu Val Ala Tyr Gly Ser Ser Val
        595                 600                 605
Asn Glu Ala Leu Ala Ala Ala Asp Met Leu Glu Arg Asp Gly Val Ser
    610                 615                 620
Thr Thr Val Ile Asp Ala Arg Phe Cys Lys Pro Leu Asp Thr Lys Leu
625                 630                 635                 640
Ile Arg Ser Ala Ala Lys Glu His Pro Val Met Ile Thr Ile Glu Glu
                645                 650                 655
Gly Ser Val Gly Gly Phe Ala Ala His Val Met Gln Phe Leu Ala Leu
            660                 665                 670
Glu Gly Leu Leu Asp Gly Gly Leu Lys Phe Arg Pro Met Thr Leu Pro
        675                 680                 685
Asp Arg Tyr Ile Asp His Gly Asp Tyr Arg Asp Gln Leu Ala Met Ala
    690                 695                 700
Gly Leu Thr Ser Gln His Ile Ala Ser Thr Ala Leu Thr Thr Leu Gly
705                 710                 715                 720
Arg Ala Lys Asp Ala Ala Lys Phe Ser Leu Ser Ala Leu Gln Ala
                725                 730                 735
```

<210> 21
<211> 615
<212> PRT

<213> Campylobacter jejuni

<400> 21

```
Met Ser Lys Lys Phe Ala His Thr Gln Glu Glu Leu Glu Lys Leu Ser
 1               5                  10                  15
Leu Lys Glu Leu Glu Asn Leu Ala Ala Ser Met Arg Glu Lys Ile Ile
            20                  25                  30
Gln Val Val Ser Lys Asn Gly Gly His Leu Ser Ser Asn Leu Gly Ala
        35                  40                  45
Val Glu Leu Ser Ile Ala Met His Leu Val Phe Asp Ala Lys Lys Asp
    50                  55                  60
Pro Phe Ile Phe Asp Val Ser His Gln Ser Tyr Thr His Lys Leu Leu
65                  70                  75                  80
Ser Gly Lys Glu Glu Ile Phe Asp Thr Leu Arg Gln Ile Asn Gly Leu
                85                  90                  95
Ser Gly Tyr Thr Lys Pro Ser Glu Gly Asp Tyr Phe Val Ala Gly His
            100                 105                 110
Ser Ser Thr Ser Ile Ser Leu Ala Val Gly Ala Cys Lys Ala Ile Ala
        115                 120                 125
Leu Lys Gly Glu Lys Arg Ile Pro Val Ala Leu Ile Gly Asp Gly Ala
    130                 135                 140
Leu Ser Ala Gly Met Ala Tyr Glu Ala Leu Asn Glu Leu Gly Asp Ser
145                 150                 155                 160
Lys Phe Pro Cys Val Ile Leu Leu Asn Asp Asn Glu Met Ser Ile Ser
                165                 170                 175
Lys Pro Ile Gly Ala Ile Ser Lys Tyr Leu Ser Gln Ala Met Ala Thr
            180                 185                 190
Gln Phe Tyr Gln Ser Phe Lys Lys Arg Ile Ala Lys Met Leu Asp Ile
        195                 200                 205
Leu Pro Asp Ser Ala Thr Tyr Met Ala Lys Arg Phe Glu Glu Ser Phe
    210                 215                 220
Lys Leu Ile Thr Pro Gly Leu Leu Phe Glu Glu Leu Gly Leu Glu Tyr
225                 230                 235                 240
Ile Gly Pro Ile Asp Gly His Asn Leu Gly Glu Ile Ile Ser Ala Leu
                245                 250                 255
Lys Gln Ala Lys Ala Met Gln Lys Pro Cys Val Ile His Ala Gln Thr
            260                 265                 270
Ile Lys Gly Lys Gly Tyr Ala Leu Ala Glu Gly Lys His Ala Lys Trp
        275                 280                 285
His Gly Val Gly Ala Phe Asp Ile Asp Ser Gly Glu Ser Val Lys Lys
    290                 295                 300
Ser Asp Thr Lys Lys Ser Ala Thr Glu Ile Phe Ser Lys Asn Leu Leu
305                 310                 315                 320
Asp Leu Ala Ser Lys Tyr Glu Asn Ile Val Gly Val Thr Ala Ala Met
                325                 330                 335
Pro Ser Gly Thr Gly Leu Asp Lys Leu Ile Glu Lys Tyr Pro Asn Arg
            340                 345                 350
Phe Trp Asp Val Ala Ile Ala Glu Gln His Ala Val Thr Ser Met Ala
        355                 360                 365
Ala Met Ala Lys Glu Gly Phe Lys Pro Phe Ile Ala Ile Tyr Ser Thr
    370                 375                 380
Phe Leu Gln Arg Ala Tyr Asp Gln Val Ile His Asp Cys Ala Ile Met
385                 390                 395                 400
Asn Leu Asn Val Val Phe Ala Met Asp Arg Ala Gly Ile Val Gly Glu
                405                 410                 415
Asp Gly Glu Thr His Gln Gly Val Phe Asp Leu Ser Phe Leu Ala Pro
            420                 425                 430
Leu Pro Asn Phe Thr Leu Leu Ala Pro Arg Asp Glu Gln Met Met Gln
        435                 440                 445
```

Asn Ile Met Glu Tyr Ala Tyr Leu His Gln Gly Pro Ile Ala Leu Arg
450 455 460
Tyr Pro Arg Gly Ser Phe Ile Leu Asp Lys Glu Phe Asn Pro Cys Glu
465 470 475 480
Ile Lys Leu Gly Lys Ala Gln Trp Leu Val Lys Asn Asn Ser Glu Ile
485 490 495
Ala Phe Leu Gly Tyr Gly Gln Gly Val Ala Lys Ala Trp Gln Val Leu
500 505 510
Arg Ala Leu Gln Glu Met Asn Asn Asn Ala Asn Leu Ile Asp Leu Ile
515 520 525
Phe Ala Lys Pro Leu Asp Glu Glu Leu Leu Cys Glu Leu Ala Lys Lys
530 535 540
Ser Lys Ile Trp Phe Ile Phe Ser Glu Asn Val Lys Ile Gly Gly Ile
545 550 555 560
Glu Ser Leu Ile Asn Asn Phe Léu Gln Lys Tyr Asp Leu His Val Lys
565 570 575
Val Val Ser Phe Glu Tyr Glu Asp Lys Phe Ile Glu His Gly Lys Thr
580 585 590
Ser Glu Val Glu Lys Asn Leu Glu Lys Asp Val Asn Ser Leu Leu Thr
595 600 605
Lys Val Leu Lys Phe Tyr His
610 615

<210> 22
<211> 719
<212> PRT
<213> Lycopersicon esculentum

<400> 22

```
Met Ala Leu Cys Ala Tyr Ala Phe Pro Gly Ile Leu Asn Arg Thr Gly
 1               5                  10                  15
Val Val Ser Asp Ser Ser Lys Ala Thr Pro Leu Phe Ser Gly Trp Ile
            20                  25                  30
His Gly Thr Asp Leu Gln Phe Leu Phe Gln His Lys Leu Thr His Glu
        35                  40                  45
Val Lys Lys Arg Ser Arg Val Val Gln Ala Ser Leu Ser Glu Ser Gly
    50                  55                  60
Glu Tyr Tyr Thr Gln Arg Pro Pro Thr Pro Ile Leu Asp Thr Val Asn
65                  70                  75                  80
Tyr Pro Ile His Met Lys Asn Leu Ser Leu Lys Glu Leu Lys Gln Leu
                85                  90                  95
Ala Asp Glu Leu Arg Ser Asp Thr Ile Phe Asn Val Ser Lys Thr Gly
            100                 105                 110
Gly His Leu Gly Ser Ser Leu Gly Val Val Glu Leu Thr Val Ala Leu
        115                 120                 125
His Tyr Val Phe Asn Ala Pro Gln Asp Arg Ile Leu Trp Asp Val Gly
    130                 135                 140
His Gln Ser Tyr Pro His Lys Ile Leu Thr Gly Arg Arg Asp Lys Met
145                 150                 155                 160
Ser Thr Leu Arg Gln Thr Asp Gly Leu Ala Gly Phe Thr Lys Arg Ser
                165                 170                 175
Glu Ser Glu Tyr Asp Cys Phe Gly Thr Gly His Ser Ser Thr Thr Ile
            180                 185                 190
Ser Ala Gly Leu Gly Met Ala Val Gly Arg Asp Leu Lys Gly Arg Asn
        195                 200                 205
Asn Asn Val Ile Ala Val Ile Gly Asp Gly Ala Met Thr Ala Gly Gln
    210                 215                 220
Ala Tyr Glu Ala Met Asn Asn Ala Gly Tyr Leu Asp Ser Asp Met Ile
225                 230                 235                 240
Val Ile Leu Asn Asp Asn Arg Gln Val Ser Leu Pro Thr Ala Thr Leu
                245                 250                 255
```

```
Asp Gly Pro Val Ala Pro Val Gly Ala Leu Ser Ser Ala Leu Ser Arg
            260                 265                 270
Leu Gln Ser Asn Arg Pro Leu Arg Glu Leu Arg Glu Val Ala Lys Gly
        275                 280                 285
Val Thr Lys Gln Ile Gly Gly Pro Met His Glu Leu Ala Ala Lys Val
    290                 295                 300
Asp Glu Tyr Ala Arg Gly Met Ile Ser Gly Ser Gly Ser Thr Leu Phe
305                 310                 315                 320
Glu Glu Leu Gly Leu Tyr Tyr Ile Gly Pro Val Asp Gly His Asn Ile
                325                 330                 335
Asp Asp Leu Ile Ala Ile Leu Lys Glu Val Arg Ser Thr Lys Thr Thr
            340                 345                 350
Gly Pro Val Leu Ile His Val Val Thr Glu Lys Gly Arg Gly Tyr Pro
        355                 360                 365
Tyr Ala Glu Arg Ala Ala Asp Lys Tyr His Gly Val Ala Lys Phe Asp
    370                 375                 380
Pro Ala Thr Gly Lys Gln Phe Lys Ala Ser Ala Lys Thr Gln Ser Tyr
385                 390                 395                 400
Thr Thr Tyr Phe Ala Glu Ala Leu Ile Ala Glu Ala Glu Ala Asp Lys
                405                 410                 415
Asp Ile Val Ala Ile His Ala Ala Met Gly Gly Gly Thr Gly Met Asn
            420                 425                 430
Leu Phe His Arg Arg Phe Pro Thr Arg Cys Phe Asp Val Gly Ile Ala
        435                 440                 445
Glu Gln His Ala Val Thr Phe Ala Ala Gly Leu Ala Cys Glu Gly Ile
    450                 455                 460
Lys Pro Phe Cys Ala Ile Tyr Ser Ser Phe Met Gln Arg Ala Tyr Asp
465                 470                 475                 480
Gln Val Val His Asp Val Asp Leu Gln Lys Leu Pro Val Arg Phe Ala
                485                 490                 495
Met Asp Arg Ala Gly Leu Val Gly Ala Asp Gly Pro Thr His Cys Gly
            500                 505                 510
Ala Phe Asp Val Thr Tyr Met Ala Cys Leu Pro Asn Met Val Val Met
        515                 520                 525
Ala Pro Ser Asp Glu Ala Glu Leu Phe His Met Val Ala Thr Ala Ala
    530                 535                 540
Ala Ile Asp Asp Arg Pro Ser Cys Phe Arg Tyr Pro Arg Gly Asn Gly
545                 550                 555                 560
Ile Gly Val Glu Leu Pro Ala Gly Asn Lys Gly Ile Pro Leu Glu Val
                565                 570                 575
Gly Lys Gly Arg Ile Leu Ile Glu Gly Glu Arg Val Ala Leu Leu Gly
            580                 585                 590
Tyr Gly Ser Ala Val Gln Asn Cys Leu Asp Ala Ala Ile Val Leu Glu
        595                 600                 605
Ser Arg Gly Leu Gln Val Thr Val Ala Asp Ala Arg Phe Cys Lys Pro
    610                 615                 620
Leu Asp His Ala Leu Ile Arg Ser Leu Ala Lys Ser His Glu Val Leu
625                 630                 635                 640
Ile Thr Val Glu Glu Gly Ser Ile Gly Gly Phe Gly Ser His Val Val
                645                 650                 655
Gln Phe Met Ala Leu Asp Gly Leu Leu Asp Gly Lys Leu Lys Trp Arg
            660                 665                 670
Pro Ile Val Leu Pro Asp Arg Tyr Ile Asp His Gly Ser Pro Val Asp
        675                 680                 685
Gln Leu Ala Glu Ala Gly Leu Thr Pro Ser His Ile Ala Ala Thr Val
    690                 695                 700
Phe Asn Ile Leu Gly Gln Thr Arg Glu Ala Leu Glu Val Met Thr
705                 710                 715
```

<210> 23
<211> 643
<212> PRT

<213> Mycobacterium leprae

<400> 23

```
Met Leu Glu Gln Ile Arg Arg Pro Ala Asp Leu Gln His Leu Ser Gln
1               5                   10                  15
Gln Gln Leu Arg Asp Leu Ala Ala Glu Ile Arg Glu Leu Leu Val His
            20                  25                  30
Lys Val Ala Ala Thr Gly Gly His Leu Gly Pro Asn Leu Gly Val Val
        35                  40                  45
Glu Leu Thr Leu Ala Leu His Arg Val Phe Asp Ser Pro His Asp Pro
    50                  55                  60
Ile Ile Phe Asp Thr Gly His Gln Ala Tyr Val His Lys Met Leu Thr
65                  70                  75                  80
Gly Arg Cys Gln Asp Phe Asp Ser Leu Arg Lys Lys Ala Gly Leu Ser
                85                  90                  95
Gly Tyr Pro Ser Arg Ala Glu Ser Glu His Asp Trp Val Glu Ser Ser
            100                 105                 110
His Ala Ser Thr Ala Leu Ser Tyr Ala Asp Gly Leu Ala Lys Ala Phe
        115                 120                 125
Glu Leu Ala Gly Asn Arg Asn Arg His Val Val Ala Val Val Gly Asp
    130                 135                 140
Gly Ala Leu Thr Gly Gly Met Cys Trp Glu Ala Leu Asn Asn Ile Ala
145                 150                 155                 160
Ala Thr Pro Arg Pro Val Val Ile Val Val Asn Asp Asn Gly Arg Ser
                165                 170                 175
Tyr Ala Pro Thr Ile Gly Gly Val Ala Asp His Leu Ala Thr Leu Arg
            180                 185                 190
Leu Gln Pro Ala Tyr Glu Arg Leu Leu Glu Lys Gly Arg Asp Ala Leu
        195                 200                 205
His Ser Leu Pro Leu Ile Gly Gln Ile Ala Tyr Arg Phe Met His Ser
    210                 215                 220
Val Lys Ala Gly Ile Lys Asp Ser Leu Ser Pro Gln Leu Leu Phe Thr
225                 230                 235                 240
Asp Leu Gly Leu Lys Tyr Val Gly Pro Val Asp Gly His Asp Glu His
                245                 250                 255
Ala Val Glu Val Ala Leu Arg Lys Ala Arg Gly Phe Gly Gly Pro Val
            260                 265                 270
Ile Val His Val Val Thr Arg Lys Gly Met Gly Tyr Pro Pro Ala Glu
        275                 280                 285
Ala Asp Gln Ala Glu Gln Met His Thr Cys Gly Val Met Asp Pro Thr
    290                 295                 300
Thr Gly Gln Pro Thr Lys Ile Ala Ala Pro Asp Trp Thr Ala Ile Phe
305                 310                 315                 320
Ser Asp Ala Leu Ile Gly Tyr Ala Met Lys Arg Arg Asp Ile Val Ala
                325                 330                 335
Ile Thr Ala Ala Met Pro Gly Pro Thr Gly Leu Thr Ala Phe Gly Gln
            340                 345                 350
Cys Phe Pro Asp Arg Leu Phe Asp Val Gly Ile Ala Glu Gln His Ala
        355                 360                 365
Met Thr Ser Ala Ala Gly Leu Ala Met Gly Arg Met His Pro Val Val
    370                 375                 380
Ala Ile Tyr Ser Thr Phe Leu Asn Arg Ala Phe Asp Gln Ile Met Met
385                 390                 395                 400
Asp Val Ala Leu His Lys Leu Pro Val Thr Met Val Ile Asp Arg Ala
                405                 410                 415
Gly Ile Thr Gly Ser Asp Gly Pro Ser His Asn Gly Met Trp Asp Leu
            420                 425                 430
Ser Met Leu Gly Ile Val Pro Gly Met Arg Val Ala Ala Pro Arg Asp
        435                 440                 445
Ala Ile Arg Leu Arg Glu Glu Leu Gly Glu Ala Leu Asp Val Asp Asp
```

```
    450                 455                 460
Gly Pro Thr Ala Ile Arg Phe Pro Lys Gly Asp Val Cys Glu Asp Ile
465                 470                 475                 480
Pro Ala Leu Lys Arg Arg Ser Gly Val Asp Val Leu Ala Val Pro Ala
                485                 490                 495
Thr Gly Leu Ala Gln Asp Val Leu Leu Val Gly Val Gly Val Phe Ala
            500                 505                 510
Ser Met Ala Leu Ala Val Ala Lys Arg Leu His Asn Gln Gly Ile Gly
        515                 520                 525
Val Thr Val Ile Asp Pro Arg Trp Val Leu Pro Val Cys Asp Gly Val
    530                 535                 540
Leu Glu Leu Ala His Thr His Lys Leu Ile Val Thr Leu Glu Asp Asn
545                 550                 555                 560
Gly Val Asn Gly Gly Val Gly Ala Ala Val Ser Thr Ala Leu Arg Gln
                565                 570                 575
Val Glu Ile Asp Thr Pro Cys Arg Asp Val Gly Leu Pro Gln Glu Phe
            580                 585                 590
Tyr Asp His Ala Ser Arg Ser Glu Val Leu Ala Asp Leu Gly Leu Thr
        595                 600                 605
Asp Gln Asp Val Ala Arg Arg Ile Thr Gly Trp Val Val Ala Phe Gly
    610                 615                 620
His Cys Gly Ser Gly Asp Asp Ala Gly Gln Tyr Gly Pro Arg Ser Ser
625                 630                 635                 640
Gln Thr Met
```

<210> 24
<211> 638
<212> PRT
<213> Mycobacterium tuberculosis

<400> 24

```
Met Leu Gln Gln Ile Arg Gly Pro Ala Asp Leu Gln His Leu Ser Gln
1               5                   10                  15
Ala Gln Leu Arg Glu Leu Ala Ala Glu Ile Arg Glu Phe Leu Ile His
            20                  25                  30
Lys Val Ala Ala Thr Gly Gly His Leu Gly Pro Asn Leu Gly Val Val
        35                  40                  45
Glu Leu Thr Leu Ala Leu His Arg Val Phe Asp Ser Pro His Asp Pro
    50                  55                  60
Ile Ile Phe Asp Thr Gly His Gln Ala Tyr Val His Lys Met Leu Thr
65                  70                  75                  80
Gly Arg Ser Gln Asp Phe Ala Thr Leu Arg Lys Lys Gly Gly Leu Ser
                85                  90                  95
Gly Tyr Pro Ser Arg Ala Glu Ser Glu His Asp Trp Val Glu Ser Ser
            100                 105                 110
His Ala Ser Ala Ala Leu Ser Tyr Ala Asp Gly Leu Ala Lys Ala Phe
        115                 120                 125
Glu Leu Thr Gly His Arg Asn Arg His Val Val Ala Val Val Gly Asp
    130                 135                 140
Gly Ala Leu Thr Gly Gly Met Cys Trp Glu Ala Leu Asn Asn Ile Ala
145                 150                 155                 160
Ala Ser Arg Arg Pro Val Ile Ile Val Val Asn Asp Asn Gly Arg Ser
                165                 170                 175
Tyr Ala Pro Thr Ile Gly Gly Val Ala Asp His Leu Ala Thr Leu Arg
            180                 185                 190
Leu Gln Pro Ala Tyr Glu Gln Ala Leu Glu Thr Gly Arg Asp Leu Val
        195                 200                 205
Arg Ala Val Pro Leu Val Gly Gly Leu Trp Phe Arg Phe Leu His Ser
    210                 215                 220
Val Lys Ala Gly Ile Lys Asp Ser Leu Ser Pro Gln Leu Leu Phe Thr
```

```
225                 230                 235                 240
Asp Leu Gly Leu Lys Tyr Val Gly Pro Val Asp Gly His Asp Glu Arg
                245                 250                 255
Ala Val Glu Val Ala Leu Arg Ser Ala Arg Arg Phe Gly Ala Pro Val
            260                 265                 270
Ile Val His Val Val Thr Arg Lys Gly Met Gly Tyr Pro Pro Ala Glu
        275                 280                 285
Ala Asp Gln Ala Glu Gln Met His Ser Thr Val Pro Ile Asp Pro Ala
    290                 295                 300
Thr Gly Gln Ala Thr Lys Val Ala Gly Pro Gly Trp Thr Ala Thr Phe
305                 310                 315                 320
Ser Asp Ala Leu Ile Gly Tyr Ala Gln Lys Arg Arg Asp Ile Val Ala
                325                 330                 335
Ile Thr Ala Ala Met Pro Gly Pro Thr Gly Leu Thr Ala Phe Gly Gln
            340                 345                 350
Arg Phe Pro Asp Arg Leu Phe Asp Val Gly Ile Ala Glu Gln His Ala
        355                 360                 365
Met Thr Ser Ala Ala Gly Leu Ala Met Gly Gly Leu His Pro Val Val
    370                 375                 380
Ala Ile Tyr Ser Thr Phe Leu Asn Arg Ala Phe Asp Gln Ile Met Met
385                 390                 395                 400
Asp Val Ala Leu His Lys Leu Pro Val Thr Met Val Leu Asp Arg Ala
                405                 410                 415
Gly Ile Thr Gly Ser Asp Gly Ala Ser His Asn Gly Met Trp Asp Leu
            420                 425                 430
Ser Met Leu Gly Ile Val Pro Gly Ile Arg Val Ala Ala Pro Arg Asp
        435                 440                 445
Ala Thr Arg Leu Arg Glu Glu Leu Gly Glu Ala Leu Asp Val Asp Asp
    450                 455                 460
Gly Pro Thr Ala Leu Arg Phe Pro Lys Gly Asp Val Gly Glu Asp Ile
465                 470                 475                 480
Ser Ala Leu Glu Arg Arg Gly Gly Val Asp Val Leu Ala Ala Pro Ala
                485                 490                 495
Asp Gly Leu Asn His Asp Val Leu Leu Val Ala Ile Gly Ala Phe Ala
            500                 505                 510
Pro Met Ala Leu Ala Val Ala Lys Arg Leu His Asn Gln Gly Ile Gly
        515                 520                 525
Val Thr Val Ile Asp Pro Arg Trp Val Leu Pro Val Ser Asp Gly Val
    530                 535                 540
Arg Glu Leu Ala Val Gln His Lys Leu Leu Val Thr Leu Glu Asp Asn
545                 550                 555                 560
Gly Val Asn Gly Gly Ala Gly Ser Ala Val Ser Ala Ala Leu Arg Arg
                565                 570                 575
Ala Glu Ile Asp Val Pro Cys Arg Asp Val Gly Leu Pro Gln Glu Phe
            580                 585                 590
Tyr Glu His Ala Ser Arg Ser Glu Val Leu Ala Asp Leu Gly Leu Thr
        595                 600                 605
Asp Gln Asp Val Ala Arg Arg Ile Thr Gly Trp Val Ala Ala Leu Gly
    610                 615                 620
Thr Gly Val Cys Ala Ser Asp Ala Ile Pro Glu His Leu Asp
625                 630                 635
```

<210> 25
<211> 641
<212> PRT
<213> Rhodobacter capsulatus

<400> 25

```
Met Ser Ala Thr Pro Ser Arg Thr Pro His Leu Asp Arg Val Thr Gly
 1               5           .      10                  15
Pro Ala Asp Leu Lys Ala Met Ser Ile Ala Asp Leu Thr Ala Leu Ala
```

```
            20                  25                  30
Ser Glu Val Arg Arg Glu Ile Val Glu Val Val Ser Gln Thr Gly Gly
        35                  40                  45
His Leu Gly Ser Ser Leu Gly Val Val Glu Leu Thr Val Ala Leu His
    50                  55                  60
Ala Val Phe Asn Ser Pro Gly Asp Lys Leu Ile Trp Asp Val Gly His
65                  70                  75                  80
Gln Cys Tyr Pro His Lys Ile Leu Thr Gly Arg Arg Ser Arg Met Leu
                85                  90                  95
Thr Leu Arg Gln Ala Gly Gly Ile Ser Gly Phe Pro Lys Arg Ser Glu
            100                 105                 110
Ser Pro His Asp Ala Phe Gly Ala Gly His Ser Ser Thr Ser Ile Ser
        115                 120                 125
Ala Ala Leu Gly Phe Ala Val Gly Arg Glu Leu Gly Gln Pro Val Gly
    130                 135                 140
Asp Thr Ile Ala Ile Ile Gly Asp Gly Ser Ile Thr Ala Gly Met Ala
145                 150                 155                 160
Tyr Glu Ala Leu Asn His Ala Gly His Leu Lys Ser Arg Met Phe Val
                165                 170                 175
Ile Leu Asn Asp Asn Asp Met Ser Ile Ala Pro Pro Val Gly Ala Leu
            180                 185                 190
Gln His Tyr Leu Asn Thr Ile Ala Arg Gln Ala Pro Phe Ala Ala Leu
        195                 200                 205
Lys Ala Ala Ala Glu Gly Ile Glu Met His Leu Pro Gly Pro Val Arg
    210                 215                 220
Asp Gly Ala Arg Arg Ala Arg Gln Met Val Thr Ala Met Pro Gly Gly
225                 230                 235                 240
Ala Thr Leu Phe Glu Glu Leu Gly Phe Asp Tyr Ile Gly Pro Val Asp
                245                 250                 255
Gly His Asp Met Ala Glu Leu Val Glu Thr Leu Arg Val Thr Arg Ala
            260                 265                 270
Arg Ala Ser Gly Pro Val Leu Ile His Val Cys Thr Thr Lys Gly Lys
        275                 280                 285
Gly Tyr Ala Pro Ala Glu Gly Ala Glu Asp Lys Leu His Gly Val Ser
    290                 295                 300
Lys Phe Asp Ile Glu Thr Gly Lys Gln Lys Lys Ser Ile Pro Asn Ala
305                 310                 315                 320
Pro Asn Tyr Thr Ala Val Phe Gly Glu Arg Leu Thr Glu Glu Ala Ala
                325                 330                 335
Arg Asp Gln Ala Ile Val Ala Val Thr Ala Ala Met Pro Thr Gly Thr
            340                 345                 350
Gly Leu Asp Ile Met Gln Lys Arg Phe Pro Arg Arg Val Phe Asp Val
        355                 360                 365
Gly Ile Ala Glu Gln His Ala Val Thr Phe Ala Ala Gly Met Ala Ala
    370                 375                 380
Ala Gly Leu Lys Pro Phe Leu Ala Leu Tyr Ser Ser Phe Val Gln Arg
385                 390                 395                 400
Gly Tyr Asp Gln Leu Val His Asp Val Ala Leu Gln Asn Leu Pro Val
                405                 410                 415
Arg Leu Met Ile Asp Arg Ala Gly Leu Val Gly Gln Asp Gly Ala Thr
            420                 425                 430
His Ala Gly Ala Phe Asp Val Ser Met Leu Ala Asn Leu Pro Asn Phe
        435                 440                 445
Thr Val Met Ala Ala Ala Asp Glu Ala Glu Leu Cys His Met Val Val
    450                 455                 460
Thr Ala Ala Ala His Asp Ser Gly Pro Ile Ala Leu Arg Tyr Pro Arg
465                 470                 475                 480
Gly Glu Gly Arg Gly Val Glu Met Pro Glu Arg Gly Glu Val Leu Glu
                485                 490                 495
Ile Gly Lys Gly Arg Val Met Thr Glu Gly Thr Glu Val Ala Ile Leu
            500                 505                 510
```

```
Ser Phe Gly Ala His Leu Ala Gln Ala Leu Lys Ala Ala Glu Met Leu
        515                 520                 525
Glu Ala Glu Gly Val Ser Thr Thr Val Ala Asp Ala Arg Phe Cys Arg
    530                 535                 540
Pro Leu Asp Thr Asp Leu Ile Asp Arg Leu Ile Glu Gly His Ala Ala
545                 550                 555                 560
Leu Ile Thr Leu Glu Gln Gly Ala Met Gly Gly Phe Gly Ala Met Val
                565                 570                 575
Leu His Tyr Leu Ala Arg Thr Gly Gln Leu Glu Lys Gly Arg Ala Ile
            580                 585                 590
Arg Thr Met Thr Leu Pro Asp Cys Tyr Ile Asp His Gly Ser Pro Glu
        595                 600                 605
Glu Met Tyr Ala Trp Ala Gly Leu Thr Ala Asn Asp Ile Arg Asp Thr
    610                 615                 620
Ala Leu Ala Ala Ala Arg Pro Ser Lys Ser Val Arg Ile Val His Ser
625                 630                 635                 640
Ala
```

<210> 26
<211> 637
<212> PRT
<213> Rhodobacter sphaeroides

<400> 26

```
Met Thr Asp Arg Pro Cys Thr Pro Thr Leu Asp Arg Val Thr Leu Pro
 1               5                  10                  15
Val Asp Ile Lys Gly Leu Thr Asp Arg Glu Leu Arg Ser Leu Ala Asp
            20                  25                  30
Glu Leu Arg Ala Glu Thr Ile Ser Ala Val Ser Val Thr Gly Gly His
        35                  40                  45
Leu Gly Ala Gly Leu Gly Val Val Glu Leu Thr Val Ala Leu His Ala
    50                  55                  60
Ile Phe Asp Ala Pro Arg Asp Lys Ile Ile Trp Asp Val Gly His Gln
65                  70                  75                  80
Cys Tyr Pro His Lys Ile Leu Thr Gly Arg Arg Asp Arg Ile Arg Thr
                85                  90                  95
Leu Arg Gln Gly Gly Gly Leu Ser Gly Phe Thr Lys Arg Ser Glu Ser
            100                 105                 110
Pro Tyr Asp Cys Phe Gly Ala Gly His Ser Ser Thr Ser Ile Ser Ala
        115                 120                 125
Ala Val Gly Phe Ala Ala Ala Arg Glu Met Gly Gly Asp Thr Gly Asp
    130                 135                 140
Ala Val Ala Val Ile Gly Asp Gly Ser Met Ser Ala Gly Met Ala Phe
145                 150                 155                 160
Glu Ala Leu Asn His Gly Gly His Leu Lys Asn Arg Val Ile Val Ile
                165                 170                 175
Leu Asn Asp Asn Glu Met Ser Ile Ala Pro Pro Val Gly Ala Leu Ser
            180                 185                 190
Ser Tyr Leu Ser Arg Leu Tyr Ala Gly Ala Pro Phe Gln Asp Phe Lys
        195                 200                 205
Ala Ala Ala Lys Gly Ala Leu Gly Leu Leu Pro Glu Pro Phe Gln Glu
    210                 215                 220
Gly Ala Arg Arg Ala Lys Glu Met Leu Lys Ser Val Thr Val Gly Gly
225                 230                 235                 240
Thr Leu Phe Glu Glu Leu Gly Phe Ser Tyr Val Gly Pro Ile Asp Gly
                245                 250                 255
His Asp Leu Asp Gln Leu Leu Pro Val Leu Arg Thr Val Lys Gln Arg
            260                 265                 270
Ala His Ala Pro Val Leu Ile His Val Ile Thr Lys Lys Gly Arg Gly
        275                 280                 285
```

Tyr Ala Pro Ala Glu Ala Ala Arg Asp Arg Gly His Ala Thr Asn Lys
290 295 300
Phe Asn Val Leu Thr Gly Ala Gln Val Lys Pro Val Ser Asn Ala Pro
305 310 315 320
Ser Tyr Thr Lys Val Phe Ala Gln Ser Leu Ile Lys Glu Ala Glu Val
325 330 335
Asp Glu Arg Ile Cys Ala Val Thr Ala Ala Met Pro Asp Gly Thr Gly
340 345 350
Leu Asn Leu Phe Gly Glu Arg Phe Pro Lys Arg Thr Phe Asp Val Gly
355 360 365
Ile Ala Glu Gln His Ala Val Thr Phe Ser Ala Ala Leu Ala Ala Gly
370 375 380
Gly Met Arg Pro Phe Cys Ala Ile Tyr Ser Thr Phe Leu Gln Arg Gly
385 390 395 400
Tyr Asp Gln Ile Val His Asp Val Ala Ile Gln Arg Leu Pro Val Arg
405 410 415
Phe Ala Ile Asp Arg Ala Gly Leu Val Gly Ala Asp Gly Ala Thr His
420 425 430
Ala Gly Ser Phe Asp Val Ala Phe Leu Ser Asn Leu Pro Gly Ile Val
435 440 445
Val Met Ala Ala Ala Asp Glu Ala Glu Leu Val His Met Val Ala Thr
450 455 460
Ala Ala Ala His Asp Glu Gly Pro Ile Ala Phe Arg Tyr Pro Arg Gly
465 470 475 480
Asp Gly Val Gly Val Glu Val Pro Val Lys Gly Val Pro Leu Gln Ile
485 490 495
Gly Arg Gly Arg Val Val Ser Glu Gly Thr Arg Ile Ala Leu Leu Ser
500 505 510
Phe Gly Thr Arg Leu Ala Glu Val Gln Val Ala Ala Glu Ala Leu Ala
515 520 525
Ala Arg Gly Ile Ser Pro Thr Val Ala Asp Ala Arg Phe Ala Lys Pro
530 535 540
Leu Asp Arg Asp Leu Ile Leu Gln Leu Ala Ala His His Glu Ala Leu
545 550 555 560
Ile Thr Ile Glu Glu Gly Ala Ile Gly Gly Phe Gly Ser His Val Ala
565 570 575
Gln Leu Leu Ala Glu Ala Gly Val Phe Asp Arg Gly Phe Arg Tyr Arg
580 585 590
Ser Met Val Leu Pro Asp Thr Phe Ile Asp His Asn Ser Ala Glu Val
595 600 605
Met Tyr Ala Thr Ala Gly Leu Asn Ala Ala Asp Ile Glu Arg Lys Ala
610 615 620
Leu Glu Thr Leu Gly Val Glu Val Leu Ala Arg Arg Ala
625 630 635

<210> 27
<211> 648
<212> PRT
<213> Rhodobacter sphaeroides

<400> 27

```
Met Thr Asn Pro Thr Pro Arg Pro Glu Thr Pro Leu Leu Asp Arg Val
 1               5                  10                  15
Cys Cys Pro Ala Asp Met Lys Ala Leu Ser Asp Ala Glu Leu Glu Arg
            20                  25                  30
Leu Ala Asp Glu Val Arg Ser Glu Val Ile Ser Val Val Ala Glu Thr
        35                  40                  45
Gly Gly His Leu Gly Ser Ser Leu Gly Val Val Glu Leu Thr Val Ala
    50                  55                  60
Leu His Ala Val Phe Asn Thr Pro Thr Asp Lys Leu Val Trp Asp Val
65                  70                  75                  80
```

```
Gly His Gln Cys Tyr Pro His Lys Ile Leu Thr Gly Arg Arg Glu Gln
                85                  90                  95
Met Arg Thr Leu Arg Gln Lys Gly Gly Leu Ser Gly Phe Thr Lys Arg
            100                 105                 110
Ser Glu Ser Ala Tyr Asp Pro Phe Gly Ala Ala His Ser Ser Thr Ser
        115                 120                 125
Ile Ser Ala Ala Leu Gly Phe Ala Met Gly Arg Glu Leu Gly Gln Pro
    130                 135                 140
Val Gly Asp Thr Ile Ala Val Ile Gly Asp Gly Ser Ile Thr Ala Gly
145                 150                 155                 160
Met Ala Tyr Glu Ala Leu Asn His Ala Gly His Leu Asn Lys Arg Leu
                165                 170                 175
Phe Val Ile Leu Asn Asp Asn Asp Met Ser Ile Ala Pro Pro Val Gly
            180                 185                 190
Ala Leu Ala Arg Tyr Leu Val Asn Leu Ser Ser Lys Ala Pro Phe Ala
        195                 200                 205
Thr Leu Arg Ala Ala Ala Asp Gly Leu Glu Ala Ser Leu Pro Gly Pro
    210                 215                 220
Leu Arg Asp Gly Ala Arg Arg Ala Arg Gln Leu Val Thr Gly Met Pro
225                 230                 235                 240
Gly Gly Gly Thr Leu Phe Glu Glu Leu Gly Phe Thr Tyr Val Gly Pro
                245                 250                 255
Ile Asp Gly His Asp Met Glu Ala Leu Leu Gln Thr Leu Arg Ala Ala
            260                 265                 270
Arg Ala Arg Thr Thr Gly Pro Val Leu Ile His Val Val Thr Lys Lys
        275                 280                 285
Gly Lys Gly Tyr Ala Pro Ala Glu Asn Ala Pro Asp Lys Tyr His Gly
    290                 295                 300
Val Asn Lys Phe Asp Pro Val Thr Gly Glu Gln Lys Lys Ser Val Ala
305                 310                 315                 320
Asn Ala Pro Asn Tyr Thr Lys Val Phe Gly Ser Thr Leu Thr Glu Glu
                325                 330                 335
Ala Ala Arg Asp Pro Arg Ile Val Ala Ile Thr Ala Ala Met Pro Ser
            340                 345                 350
Gly Thr Gly Val Asp Ile Met Gln Lys Arg Phe Pro Asn Arg Val Phe
        355                 360                 365
Asp Val Gly Ile Ala Glu Gln His Ala Val Thr Phe Ala Ala Gly Leu
    370                 375                 380
Ala Gly Ala Gly Met Lys Pro Phe Cys Ala Ile Tyr Ser Ser Phe Leu
385                 390                 395                 400
Gln Arg Gly Tyr Asp Gln Ile Ala His Asp Val Ala Leu Gln Asn Leu
                405                 410                 415
Pro Val Arg Phe Val Ile Asp Arg Ala Gly Leu Val Gly Ala Asp Gly
            420                 425                 430
Ala Thr His Ala Gly Ala Phe Asp Val Gly Phe Ile Thr Ser Leu Pro
        435                 440                 445
Asn Met Thr Val Met Ala Ala Ala Asp Glu Ala Glu Leu Ile His Met
    450                 455                 460
Ile Ala Thr Ala Val Ala Phe Gly Glu Gly Pro Ile Ala Phe Arg Phe
465                 470                 475                 480
Pro Arg Gly Glu Gly Val Gly Val Glu Met Pro Glu Arg Gly Thr Val
                485                 490                 495
Leu Glu Pro Gly Arg Gly Arg Val Val Arg Glu Gly Thr Asp Val Ala
            500                 505                 510
Ile Leu Ser Phe Gly Ala His Leu His Glu Ala Leu Gln Ala Ala Lys
        515                 520                 525
Leu Leu Glu Ala Glu Gly Val Ser Val Thr Val Ala Asp Ala Arg Phe
    530                 535                 540
Ser Arg Pro Leu Asp Thr Gly His Ile Asp Gln Leu Val Arg His His
545                 550                 555                 560
Ala Ala Leu Val Thr Val Glu Gln Gly Ala Met Gly Gly Phe Gly Ala
```

```
                    565                     570                     575
Tyr Val Met His Cys Leu Ala Asn Ser Gly Gly Phe Asp Gly Gly Leu
                580                     585                     590
Ala Leu Arg Val Met Thr Leu Pro Asp Arg Phe Ile Glu Gln Ala Ser
            595                     600                     605
Pro Glu Asp Met Tyr Ala Asp Ala Gly Leu Arg Ala Glu Asp Ile Ala
    610                     615                     620
Ala Thr Ala Arg Gly Ala Leu Ala Arg Gly Arg Val Met Pro Leu Arg
625                     630                     635                     640
Gln Thr Ala Lys Pro Arg Ala Val
                645
```

<210> 28
<211> 636
<212> PRT
<213> Synechococcus sp. PCC 6301

<400> 28

Met His Leu Ser Glu Ile Thr His Pro Asn Gln Leu His Gly Leu Ser
1 5 10 15
Val Ala Gln Leu Glu Gln Ile Gly His Gln Ile Arg Glu Lys His Leu
20 25 30
Gln Thr Val Ala Ala Thr Gly Gly His Leu Gly Pro Gly Leu Gly Val
35 40 45
Val Glu Leu Thr Leu Ala Leu Tyr Gln Thr Leu Asp Leu Asp Arg Asp
50 55 60
Lys Val Val Trp Asp Val Gly His Gln Ala Tyr Pro His Lys Leu Leu
65 70 75 80
Thr Gly Arg Tyr His Asn Phe His Thr Leu Arg Gln Lys Asp Gly Ile
85 90 95
Ala Gly Tyr Pro Lys Arg Thr Glu Asn Arg Phe Asp His Phe Gly Ala
100 105 110
Gly His Ala Ser Thr Ser Ile Ser Ala Gly Leu Gly Met Ala Leu Ala
115 120 125
Arg Asp Ala Gln Gly Glu Asp Tyr Arg Cys Val Ala Val Ile Gly Asp
130 135 140
Gly Ser Leu Thr Gly Gly Met Ala Leu Glu Ala Ile Asn His Ala Gly
145 150 155 160
His Leu Pro Lys Thr Arg Leu Leu Val Val Leu Asn Asp Asn Asp Met
165 170 175
Ser Ile Ser Pro Asn Val Gly Ala Leu Ser Arg Tyr Leu Asn Lys Ile
180 185 190
Arg Val Ser Glu Pro Met Gln Leu Leu Thr Asp Gly Leu Thr Gln Gly
195 200 205
Met Gln Gln Ile Pro Phe Val Gly Gly Ala Ile Thr Gln Gly Phe Glu
210 215 220
Pro Val Lys Glu Gly Met Lys Arg Leu Ser Tyr Ser Lys Ile Gly Ala
225 230 235 240
Val Phe Glu Glu Leu Gly Phe Thr Tyr Met Gly Pro Val Asp Gly His
245 250 255
Asn Leu Glu Glu Leu Ile Ala Thr Phe Arg Glu Ala His Lys His Thr
260 265 270
Gly Pro Val Leu Val His Val Ala Thr Thr Lys Gly Lys Gly Tyr Pro
275 280 285
Tyr Ala Glu Glu Asp Gln Val Gly Tyr His Ala Gln Asn Pro Phe Asp
290 295 300
Leu Ala Thr Gly Lys Ala Lys Pro Ala Ser Lys Pro Lys Pro Pro Ser
305 310 315 320
Tyr Ser Lys Val Phe Gly Gln Thr Leu Thr Thr Leu Ala Lys Ser Asp
325 330 335
Arg Arg Ile Val Gly Ile Thr Ala Ala Met Ala Thr Gly Thr Gly Leu

```
            340                     345                     350
Asp Ile Leu Gln Lys Ala Leu Pro Lys Gln Tyr Ile Asp Val Gly Ile
        355                     360                     365
Ala Glu Gln His Ala Val Val Leu Ala Ala Gly Met Ala Cys Asp Gly
    370                     375                     380
Met Arg Pro Val Val Ala Ile Tyr Ser Thr Phe Leu Gln Arg Ala Phe
385                     390                     395                     400
Asp Gln Val Ile His Asp Val Cys Ile Gln Lys Leu Pro Val Phe Phe
                405                     410                     415
Cys Leu Asp Arg Ala Gly Ile Val Gly Ala Asp Gly Pro Thr His Gln
            420                     425                     430
Gly Met Tyr Asp Ile Ala Tyr Leu Arg Leu Ile Pro Asn Met Val Leu
        435                     440                     445
Met Ala Pro Lys Asp Glu Ala Glu Leu Gln Arg Met Leu Val Thr Gly
    450                     455                     460
Ile Glu Tyr Asp Gly Pro Ile Ala Met Arg Phe Pro Arg Gly Asn Gly
465                     470                     475                     480
Ile Gly Val Pro Leu Pro Glu Glu Gly Trp Glu Ser Leu Pro Ile Gly
                485                     490                     495
Lys Ala Glu Gln Leu Arg Gln Gly Asp Asp Leu Leu Met Leu Ala Tyr
            500                     505                     510
Gly Ser Met Val Tyr Pro Ala Leu Gln Thr Ala Glu Leu Leu Asn Glu
        515                     520                     525
His Gly Ile Ser Ala Thr Val Ile Asn Ala Arg Phe Ala Lys Pro Leu
    530                     535                     540
Asp Glu Glu Leu Ile Val Pro Leu Ala Arg Gln Ile Gly Lys Val Val
545                     550                     555                     560
Thr Phe Glu Glu Gly Cys Leu Pro Gly Gly Phe Gly Ser Ala Ile Met
                565                     570                     575
Glu Ser Leu Gln Ala His Asp Leu Gln Val Pro Val Leu Pro Ile Gly
            580                     585                     590
Val Pro Asp Leu Leu Val Glu His Ala Ser Pro Asp Glu Ser Lys Gln
        595                     600                     605
Glu Leu Gly Leu Thr Pro Arg Gln Met Ala Asp Arg Ile Leu Glu Lys
    610                     615                     620
Phe Gly Ser Arg Gln Arg Ile Gly Ala Ala Ser Ala
625                     630                     635
```

<210> 29
<211> 640
<212> PRT
<213> Synechocystis sp. PCC 6803

<400> 29

```
Met His Ile Ser Glu Leu Thr His Pro Asn Glu Leu Lys Gly Leu Ser
 1              5                   10                  15
Ile Arg Glu Leu Glu Glu Val Ser Arg Gln Ile Arg Glu Lys His Leu
            20                  25                  30
Gln Thr Val Ala Thr Ser Gly Gly His Leu Gly Pro Gly Leu Gly Val
        35                  40                  45
Val Glu Leu Thr Val Ala Leu Tyr Ser Thr Leu Asp Leu Asp Lys Asp
    50                  55                  60
Arg Val Ile Trp Asp Val Gly His Gln Ala Tyr Pro His Lys Met Leu
65                  70                  75                  80
Thr Gly Arg Tyr His Asp Phe His Thr Leu Arg Gln Lys Asp Gly Val
                85                  90                  95
Ala Gly Tyr Leu Lys Arg Ser Glu Ser Arg Phe Asp His Phe Gly Ala
            100                 105                 110
Gly His Ala Ser Thr Ser Ile Ser Ala Gly Leu Gly Met Ala Leu Ala
        115                 120                 125
Arg Asp Ala Lys Gly Glu Asp Phe Lys Val Val Ser Ile Ile Gly Asp
```

```
                130                 135                 140
            Gly Ala Leu Thr Gly Gly Met Ala Leu Glu Ala Ile Asn His Ala Gly
            145                 150                 155                 160
            His Leu Pro His Thr Arg Leu Met Val Ile Leu Asn Asp Asn Glu Met
                            165                 170                 175
            Ser Ile Ser Pro Asn Val Gly Ala Ile Ser Arg Tyr Leu Asn Lys Val
                        180                 185                 190
            Arg Leu Ser Ser Pro Met Gln Phe Leu Thr Asp Asn Leu Glu Glu Gln
                    195                 200                 205
            Ile Lys His Leu Pro Phe Val Gly Asp Ser Leu Thr Pro Glu Met Glu
                210                 215                 220
            Arg Val Lys Glu Gly Met Lys Arg Leu Val Val Pro Lys Val Gly Ala
            225                 230                 235                 240
            Val Ile Glu Glu Leu Gly Phe Lys Tyr Phe Gly Pro Ile Asp Gly His
                            245                 250                 255
            Ser Leu Gln Glu Leu Ile Asp Thr Phe Lys Gln Ala Glu Lys Val Pro
                        260                 265                 270
            Gly Pro Val Phe Val His Val Ser Thr Thr Lys Gly Lys Gly Tyr Asp
                    275                 280                 285
            Leu Ala Glu Lys Asp Gln Val Gly Tyr His Ala Gln Ser Pro Phe Asn
                290                 295                 300
            Leu Ser Thr Gly Lys Ala Tyr Pro Ser Ser Lys Pro Lys Pro Pro Ser
            305                 310                 315                 320
            Tyr Ser Lys Val Phe Ala His Thr Leu Thr Thr Leu Ala Lys Glu Asn
                            325                 330                 335
            Pro Asn Ile Val Gly Ile Thr Ala Ala Met Ala Thr Gly Thr Gly Leu
                        340                 345                 350
            Asp Lys Leu Gln Ala Lys Leu Pro Lys Gln Tyr Val Asp Val Gly Ile
                    355                 360                 365
            Ala Glu Gln His Ala Val Thr Leu Ala Ala Gly Met Ala Cys Glu Gly
                370                 375                 380
            Ile Arg Pro Val Val Ala Ile Tyr Ser Thr Phe Leu Gln Arg Gly Tyr
            385                 390                 395                 400
            Asp Gln Ile Ile His Asp Val Cys Ile Gln Lys Leu Pro Val Phe Phe
                            405                 410                 415
            Cys Leu Asp Arg Ala Gly Ile Val Gly Ala Asp Gly Pro Thr His Gln
                        420                 425                 430
            Gly Met Tyr Asp Ile Ala Tyr Leu Arg Cys Ile Pro Asn Leu Val Leu
                    435                 440                 445
            Met Ala Pro Lys Asp Glu Ala Glu Leu Gln Gln Met Leu Val Thr Gly
                450                 455                 460
            Val Asn Tyr Thr Gly Gly Ala Ile Ala Met Arg Tyr Pro Arg Gly Asn
            465                 470                 475                 480
            Gly Ile Gly Val Pro Leu Met Glu Glu Gly Trp Glu Pro Leu Glu Ile
                            485                 490                 495
            Gly Lys Ala Glu Ile Leu Arg Ser Gly Asp Asp Val Leu Leu Leu Gly
                        500                 505                 510
            Tyr Gly Ser Met Val Tyr Pro Ala Leu Gln Thr Ala Glu Leu Leu His
                    515                 520                 525
            Glu His Gly Ile Glu Ala Thr Val Val Asn Ala Arg Phe Val Lys Pro
                530                 535                 540
            Leu Asp Thr Glu Leu Ile Leu Pro Leu Ala Glu Arg Ile Gly Lys Val
            545                 550                 555                 560
            Val Thr Met Glu Glu Gly Cys Leu Met Gly Gly Phe Gly Ser Ala Val
                            565                 570                 575
            Ala Glu Ala Leu Met Asp Asn Asn Val Leu Val Pro Leu Lys Arg Leu
                        580                 585                 590
            Gly Val Pro Asp Ile Leu Val Asp His Ala Thr Pro Glu Gln Ser Thr
                    595                 600                 605
            Val Asp Leu Gly Leu Thr Pro Ala Gln Met Ala Gln Asn Ile Met Ala
                610                 615                 620
```

Ser Leu Phe Lys Thr Glu Thr Glu Ser Val Val Ala Pro Gly Val Ser
625 630 635 640

<210> 30
<211> 608
<212> PRT
<213> Thermotoga maritima

<400> 30

```
Met Leu Leu Asp Glu Ile Lys Arg Met Ser Tyr Asp Glu Leu Lys Arg
1               5                   10                  15
Leu Ala Glu Asp Ile Arg Lys Arg Ile Thr Glu Val Val Leu Lys Asn
            20                  25                  30
Gly Gly His Leu Ala Ser Asn Leu Gly Thr Ile Glu Leu Thr Leu Ala
        35                  40                  45
Leu Tyr Arg Val Phe Asp Pro Arg Glu Asp Ala Ile Ile Trp Asp Thr
    50                  55                  60
Gly His Gln Ala Tyr Thr His Lys Ile Leu Thr Gly Arg Asp Asp Leu
65                  70                  75                  80
Phe His Thr Ile Arg Thr Phe Gly Gly Leu Ser Gly Phe Val Thr Arg
                85                  90                  95
Arg Glu Ser Pro Leu Asp Trp Phe Gly Thr Gly His Ala Gly Thr Ser
            100                 105                 110
Ile Ala Ala Gly Leu Gly Phe Glu Lys Ala Phe Glu Leu Leu Gly Glu
        115                 120                 125
Lys Arg His Val Val Val Val Ile Gly Asp Gly Ala Leu Thr Ser Gly
    130                 135                 140
Met Ala Leu Glu Ala Leu Asn Gln Leu Lys Asn Leu Asn Ser Lys Met
145                 150                 155                 160
Lys Ile Ile Leu Asn Asp Asn Gly Met Ser Ile Ser Pro Asn Val Gly
                165                 170                 175
Gly Leu Ala Tyr His Leu Ser Lys Leu Arg Thr Ser Pro Ile Tyr Leu
            180                 185                 190
Lys Gly Lys Lys Val Leu Lys Lys Val Leu Glu Lys Thr Glu Ile Gly
        195                 200                 205
Phe Glu Val Glu Glu Glu Met Lys Tyr Leu Arg Asp Ser Leu Lys Gly
    210                 215                 220
Met Ile Gln Gly Thr Asn Phe Phe Glu Ser Leu Gly Leu Lys Tyr Phe
225                 230                 235                 240
Gly Pro Phe Asp Gly His Asn Ile Glu Leu Leu Glu Lys Val Phe Lys
                245                 250                 255
Arg Ile Arg Asp Tyr Asp Tyr Ser Ser Val Val His Val Val Thr Lys
            260                 265                 270
Lys Gly Lys Gly Phe Thr Ala Ala Glu Glu Asn Pro Thr Lys Tyr His
        275                 280                 285
Ser Ala Ser Pro Ser Gly Lys Pro Lys Met Leu Ser Tyr Ser Glu Leu
    290                 295                 300
Leu Gly His Thr Leu Ser Arg Val Ala Arg Glu Asp Lys Lys Ile Val
305                 310                 315                 320
Ala Ile Thr Ala Ala Met Ala Asp Gly Thr Gly Leu Ser Ile Phe Gln
                325                 330                 335
Lys Glu His Pro Asp Arg Phe Phe Asp Leu Gly Ile Thr Glu Gln Thr
            340                 345                 350
Cys Val Thr Phe Gly Ala Ala Leu Gly Leu His Gly Met Lys Pro Val
        355                 360                 365
Val Ala Ile Tyr Ser Thr Phe Leu Gln Arg Ala Tyr Asp Gln Ile Ile
    370                 375                 380
His Asp Val Ala Leu Gln Asn Ala Pro Val Leu Phe Ala Ile Asp Arg
385                 390                 395                 400
Ser Gly Val Val Gly Glu Asp Gly Pro Thr His His Gly Leu Phe Asp
                405                 410                 415
```

Ile Asn Tyr Leu Leu Pro Val Pro Asn Met Lys Ile Ile Ser Pro Ser
420 425 430
Ser Pro Glu Glu Phe Val Asn Ser Leu Tyr Thr Val Leu Lys His Leu
435 440 445
Asp Gly Pro Val Ala Ile Arg Tyr Pro Lys Glu Ser Phe Tyr Gly Glu
450 455 460
Val Glu Ser Leu Leu Glu Asn Met Lys Glu Ile Asp Leu Gly Trp Lys
465 470 475 480
Ile Leu Lys Arg Gly Arg Glu Ala Ala Ile Ile Ala Thr Gly Thr Ile
485 490 495
Leu Asn Glu Val Leu Lys Ile Pro Leu Asp Val Thr Val Val Asn Ala
500 505 510
Leu Thr Val Lys Pro Leu Asp Thr Ala Val Leu Lys Glu Ile Ala Arg
515 520 525
Asp His Asp Leu Ile Ile Thr Val Glu Glu Ala Met Lys Ile Gly Gly
530 535 540
Phe Gly Ser Phe Val Ala Gln Arg Leu Gln Glu Met Gly Trp Gln Gly
545 550 555 560
Lys Ile Val Asn Leu Gly Val Glu Asp Leu Phe Val Pro His Gly Gly
565 570 575
Arg Lys Glu Leu Leu Ser Met Leu Gly Leu Asp Ser Glu Gly Leu Thr
580 585 590
Lys Thr Val Leu Thr Tyr Ile Lys Ala Arg Ser Arg Glu Gly Lys Val
595 600 605

<210> 31
<211> 620
<212> PRT
<213> Escherichia coli

<400> 31

```
Met Ser Phe Asp Ile Ala Lys Tyr Pro Thr Leu Ala Leu Val Asp Ser
 1               5                  10                  15
Thr Gln Glu Leu Arg Leu Leu Pro Lys Glu Ser Leu Pro Lys Leu Cys
            20                  25                  30
Asp Glu Leu Arg Arg Tyr Leu Leu Asp Ser Val Ser Arg Ser Ser Gly
        35                  40                  45
His Phe Ala Ser Gly Leu Gly Thr Val Glu Leu Thr Val Ala Leu His
    50                  55                  60
Tyr Val Tyr Asn Thr Pro Phe Asp Gln Leu Ile Trp Asp Val Gly His
65                  70                  75                  80
Gln Ala Tyr Pro His Lys Ile Leu Thr Gly Arg Arg Asp Lys Ile Gly
                85                  90                  95
Thr Ile Arg Gln Lys Gly Gly Leu His Pro Phe Pro Trp Arg Gly Glu
            100                 105                 110
Ser Glu Tyr Asp Val Leu Ser Val Gly His Ser Ser Thr Ser Ile Ser
        115                 120                 125
Ala Gly Ile Gly Ile Ala Val Ala Ala Glu Lys Glu Gly Lys Asn Arg
    130                 135                 140
Arg Thr Val Cys Val Ile Gly Asp Gly Ala Ile Thr Ala Gly Met Ala
145                 150                 155                 160
Phe Glu Ala Met Asn His Ala Gly Asp Ile Arg Pro Asp Met Leu Val
                165                 170                 175
Ile Leu Asn Asp Asn Glu Met Ser Ile Ser Glu Asn Val Gly Ala Leu
            180                 185                 190
Asn Asn His Leu Ala Gln Leu Leu Ser Gly Lys Leu Tyr Ser Ser Leu
        195                 200                 205
Arg Glu Gly Gly Lys Lys Val Phe Ser Gly Val Pro Pro Ile Lys Glu
    210                 215                 220
Leu Leu Lys Arg Thr Glu Glu His Ile Lys Gly Met Val Val Pro Gly
225                 230                 235                 240
```

```
Thr Leu Phe Glu Glu Leu Gly Phe Asn Tyr Ile Gly Pro Val Asp Gly
                245                 250                 255
His Asp Val Leu Gly Leu Ile Thr Thr Leu Lys Asn Met Arg Asp Leu
            260                 265                 270
Lys Gly Pro Gln Phe Leu His Ile Met Thr Lys Lys Gly Arg Gly Tyr
        275                 280                 285
Glu Pro Ala Glu Lys Asp Pro Ile Thr Phe His Ala Val Pro Lys Phe
    290                 295                 300
Asp Pro Ser Ser Gly Cys Leu Pro Lys Ser Ser Gly Gly Leu Pro Ser
305                 310                 315                 320
Tyr Ser Lys Ile Phe Gly Asp Trp Leu Cys Glu Thr Ala Ala Lys Asp
                325                 330                 335
Asn Lys Leu Met Ala Ile Thr Pro Ala Met Arg Glu Gly Ser Gly Met
            340                 345                 350
Val Glu Phe Ser Arg Lys Phe Pro Asp Arg Tyr Phe Asp Val Ala Ile
        355                 360                 365
Ala Glu Gln His Ala Val Thr Phe Ala Ala Gly Leu Ala Ile Gly Gly
    370                 375                 380
Tyr Lys Pro Ile Val Ala Ile Tyr Ser Thr Phe Leu Gln Arg Ala Tyr
385                 390                 395                 400
Asp Gln Val Leu His Asp Val Ala Ile Gln Lys Leu Pro Val Leu Phe
                405                 410                 415
Ala Ile Asp Arg Ala Gly Ile Val Gly Ala Asp Gly Gln Thr His Gln
            420                 425                 430
Gly Ala Phe Asp Leu Ser Tyr Leu Arg Cys Ile Pro Glu Met Val Ile
        435                 440                 445
Met Thr Pro Ser Asp Glu Asn Glu Cys Arg Gln Met Leu Tyr Thr Gly
    450                 455                 460
Tyr His Tyr Asn Asp Gly Pro Ser Ala Val Arg Tyr Pro Arg Gly Asn
465                 470                 475                 480
Ala Val Gly Val Glu Leu Thr Pro Leu Glu Lys Leu Pro Ile Gly Lys
                485                 490                 495
Gly Ile Val Lys Arg Arg Gly Glu Lys Leu Ala Ile Leu Asn Phe Gly
            500                 505                 510
Thr Leu Met Pro Glu Ala Ala Lys Val Ala Glu Ser Leu Asn Ala Thr
        515                 520                 525
Leu Val Asp Met Arg Phe Val Lys Pro Leu Asp Glu Ala Leu Ile Leu
    530                 535                 540
Glu Met Ala Ala Ser His Glu Ala Leu Val Thr Val Glu Glu Asn Ala
545                 550                 555                 560
Ile Met Gly Gly Ala Gly Ser Gly Val Asn Glu Val Leu Met Ala His
                565                 570                 575
Arg Lys Pro Val Pro Val Leu Asn Ile Gly Leu Pro Asp Phe Phe Ile
            580                 585                 590
Pro Gln Gly Thr Gln Glu Glu Met Arg Ala Glu Leu Gly Leu Asp Ala
        595                 600                 605
Ala Gly Met Glu Ala Lys Ile Lys Ala Trp Leu Ala
    610                 615                 620
```

<210> 32
<211> 637
<212> PRT
<213> Neisseria meningitidis

<400> 32

```
Met Asn Pro Ser Pro Leu Leu Asp Leu Ile Asp Ser Pro Gln Asp Leu
 1               5                  10                  15
Arg Arg Leu Asp Lys Lys Gln Leu Pro Arg Leu Ala Gly Glu Leu Arg
            20                  25                  30
Thr Phe Leu Leu Glu Ser Val Gly Gln Thr Gly Gly His Phe Ala Ser
        35                  40                  45
```

```
Asn Leu Gly Ala Val Glu Leu Thr Val Ala Leu His Tyr Val Tyr Asn
    50                  55                  60
Thr Pro Glu Asp Lys Leu Val Trp Asp Val Gly His Gln Ser Tyr Pro
65                  70                  75                  80
His Lys Ile Leu Thr Gly Arg Lys Asn Gln Met His Thr Met Arg Gln
                85                  90                  95
Tyr Gly Gly Leu Ala Gly Phe Pro Lys Arg Cys Glu Ser Glu Tyr Asp
            100                 105                 110
Ala Phe Gly Val Gly His Ser Ser Thr Ser Ile Gly Ala Ala Leu Gly
        115                 120                 125
Met Ala Ala Ala Asp Lys Gln Leu Gly Ser Asp Arg Arg Ser Val Ala
    130                 135                 140
Ile Ile Gly Asp Gly Ala Met Thr Ala Gly Gln Ala Phe Glu Ala Leu
145                 150                 155                 160
Asn Cys Ala Gly Asp Met Asp Val Asp Leu Leu Val Val Leu Asn Asp
                165                 170                 175
Asn Glu Met Ser Ile Ser Pro Asn Val Gly Ala Leu Pro Lys Tyr Leu
            180                 185                 190
Ala Ser Asn Val Val Arg Asp Met His Gly Leu Leu Ser Thr Val Lys
        195                 200                 205
Ala Gln Thr Gly Lys Val Leu Asp Lys Ile Pro Gly Ala Met Glu Phe
    210                 215                 220
Ala Gln Lys Val Glu His Lys Ile Lys Thr Leu Ala Glu Glu Ala Glu
225                 230                 235                 240
His Ala Lys Gln Ser Leu Ser Leu Phe Glu Asn Phe Gly Phe Arg Tyr
                245                 250                 255
Thr Gly Pro Val Asp Gly His Asn Val Glu Asn Leu Val Asp Val Leu
            260                 265                 270
Glu Asp Leu Arg Gly Arg Lys Gly Pro Gln Leu Leu His Val Ile Thr
        275                 280                 285
Lys Lys Gly Asn Gly Tyr Lys Leu Ala Glu Asn Asp Pro Val Lys Tyr
    290                 295                 300
His Ala Val Ala Asn Leu Pro Lys Glu Ser Ala Ala Gln Met Pro Ser
305                 310                 315                 320
Glu Lys Glu Pro Lys Pro Ala Ala Lys Pro Thr Tyr Thr Gln Val Phe
                325                 330                 335
Gly Lys Trp Leu Cys Asp Arg Ala Ala Ala Asp Ser Arg Leu Val Ala
            340                 345                 350
Ile Thr Pro Ala Met Arg Glu Gly Ser Gly Leu Val Glu Phe Glu Gln
        355                 360                 365
Arg Phe Pro Asp Arg Tyr Phe Asp Val Gly Ile Ala Glu Gln His Ala
    370                 375                 380
Val Thr Phe Ala Gly Gly Leu Ala Cys Glu Gly Met Lys Pro Val Val
385                 390                 395                 400
Ala Ile Tyr Ser Thr Phe Leu Gln Arg Ala Tyr Asp Gln Leu Val His
                405                 410                 415
Asp Ile Ala Leu Gln Asn Leu Pro Val Leu Phe Ala Val Asp Arg Ala
            420                 425                 430
Gly Ile Val Gly Ala Asp Gly Pro Thr His Ala Gly Leu Tyr Asp Leu
        435                 440                 445
Ser Phe Leu Arg Cys Ile Pro Asn Met Ile Val Ala Ala Pro Ser Asp
    450                 455                 460
Glu Asn Glu Cys Arg Leu Leu Leu Ser Thr Cys Tyr Gln Ala Asp Ala
465                 470                 475                 480
Pro Ala Ala Val Arg Tyr Pro Arg Gly Thr Gly Thr Gly Val Pro Val
                485                 490                 495
Ser Asp Gly Met Glu Thr Val Glu Ile Gly Lys Gly Ile Ile Arg Arg
            500                 505                 510
Glu Gly Glu Lys Thr Ala Phe Ile Ala Phe Gly Ser Met Val Ala Pro
        515                 520                 525
Ala Leu Ala Val Ala Gly Lys Leu Asn Ala Thr Val Ala Asp Met Arg
```

```
    530                 535                 540
Phe Val Lys Pro Ile Asp Glu Glu Leu Ile Val Arg Leu Ala Arg Ser
545                 550                 555                 560
His Asp Arg Ile Val Thr Leu Glu Glu Asn Ala Glu Gln Gly Gly Ala
                565                 570                 575
Gly Ser Ala Val Leu Glu Val Leu Ala Lys His Gly Ile Cys Lys Pro
            580                 585                 590
Val Leu Leu Leu Gly Val Ala Asp Thr Val Thr Gly His Gly Asp Pro
        595                 600                 605
Lys Lys Leu Leu Asp Asp Leu Gly Leu Ser Ala Glu Ala Val Glu Arg
    610                 615                 620
Arg Val Arg Ala Trp Leu Ser Asp Arg Asp Ala Ala Asn
625                 630                 635
```

<210> 33
<211> 625
<212> PRT
<213> Haemophilus influenzae

<400> 33

```
Met Thr Asn Asn Met Asn Asn Tyr Pro Leu Leu Ser Leu Ile Asn Ser
 1               5                  10                  15
Pro Glu Asp Leu Arg Leu Leu Asn Lys Asp Gln Leu Pro Gln Leu Cys
            20                  25                  30
Gln Glu Leu Arg Ala Tyr Leu Leu Glu Ser Val Ser Gln Thr Ser Gly
        35                  40                  45
His Leu Ala Ser Gly Leu Gly Thr Val Glu Leu Thr Val Ala Leu His
    50                  55                  60
Tyr Val Tyr Lys Thr Pro Phe Asp Gln Leu Ile Trp Asp Val Gly His
65                  70                  75                  80
Gln Ala Tyr Pro His Lys Ile Leu Thr Gly Arg Arg Glu Gln Met Ser
                85                  90                  95
Thr Ile Arg Gln Lys Asp Gly Ile His Pro Phe Pro Trp Arg Glu Glu
            100                 105                 110
Ser Glu Phe Asp Val Leu Ser Val Gly His Ser Ser Thr Ser Ile Ser
        115                 120                 125
Ala Gly Leu Gly Ile Ala Val Ala Ala Glu Arg Glu Asn Ala Gly Arg
    130                 135                 140
Lys Thr Val Cys Val Ile Gly Asp Gly Ala Ile Thr Ala Gly Met Ala
145                 150                 155                 160
Phe Glu Ala Leu Asn His Ala Gly Ala Leu His Thr Asp Met Leu Val
                165                 170                 175
Ile Leu Asn Asp Asn Glu Met Ser Ile Ser Glu Asn Val Gly Ala Leu
            180                 185                 190
Asn Asn His Leu Ala Arg Ile Phe Ser Gly Ser Leu Tyr Ser Thr Leu
        195                 200                 205
Arg Asp Gly Ser Lys Lys Ile Leu Asp Lys Val Pro Pro Ile Lys Asn
    210                 215                 220
Phe Met Lys Lys Thr Glu Glu His Met Lys Gly Val Met Phe Ser Pro
225                 230                 235                 240
Glu Ser Thr Leu Phe Glu Glu Leu Gly Phe Asn Tyr Ile Gly Pro Val
                245                 250                 255
Asp Gly His Asn Ile Asp Glu Leu Val Ala Thr Leu Thr Asn Met Arg
            260                 265                 270
Asn Leu Lys Gly Pro Gln Phe Leu His Ile Lys Thr Lys Lys Gly Lys
        275                 280                 285
Gly Tyr Ala Pro Ala Glu Lys Asp Pro Ile Gly Phe His Gly Val Pro
    290                 295                 300
Lys Phe Asp Pro Ile Ser Gly Glu Leu Pro Lys Asn Asn Ser Lys Pro
305                 310                 315                 320
Thr Tyr Ser Lys Ile Phe Gly Asp Trp Leu Cys Glu Met Ala Glu Lys
```

```
                325                 330                 335
Asp Ala Lys Ile Ile Gly Ile Thr Pro Ala Met Arg Glu Gly Ser Gly
            340                 345                 350
Met Val Glu Phe Ser Gln Arg Phe Pro Lys Gln Tyr Phe Asp Val Ala
        355                 360                 365
Ile Ala Glu Gln His Ala Val Thr Phe Ala Thr Gly Leu Ala Ile Gly
    370                 375                 380
Gly Tyr Lys Pro Val Val Ala Ile Tyr Ser Thr Phe Leu Gln Arg Ala
385                 390                 395                 400
Tyr Asp Gln Leu Ile His Asp Val Ala Ile Gln Asn Leu Pro Val Leu
                405                 410                 415
Phe Ala Ile Asp Arg Ala Gly Ile Val Gly Ala Asp Gly Ala Thr His
            420                 425                 430
Gln Gly Ala Phe Asp Ile Ser Phe Met Arg Cys Ile Pro Asn Met Ile
        435                 440                 445
Ile Met Thr Pro Ser Asp Glu Asn Glu Cys Arg Gln Met Leu Tyr Thr
    450                 455                 460
Gly Tyr Gln Cys Gly Lys Pro Ala Ala Val Arg Tyr Pro Arg Gly Asn
465                 470                 475                 480
Ala Val Gly Val Lys Leu Thr Pro Leu Glu Met Leu Pro Ile Gly Lys
                485                 490                 495
Ser Arg Leu Ile Arg Lys Gly Gln Lys Ile Ala Ile Leu Asn Phe Gly
            500                 505                 510
Thr Leu Leu Pro Ser Ala Leu Glu Leu Ser Glu Lys Leu Asn Ala Thr
        515                 520                 525
Val Val Asp Met Arg Phe Val Lys Pro Ile Asp Ile Glu Met Ile Asn
    530                 535                 540
Val Leu Ala Gln Thr His Asp Tyr Leu Val Thr Leu Glu Glu Asn Ala
545                 550                 555                 560
Ile Gln Gly Gly Ala Gly Ser Ala Val Ala Glu Val Leu Asn Ser Ser
                565                 570                 575
Gly Lys Ser Thr Ala Leu Leu Gln Leu Gly Leu Pro Asp Tyr Phe Ile
            580                 585                 590
Pro Gln Ala Thr Gln Gln Glu Ala Leu Ala Asp Leu Gly Leu Asp Thr
        595                 600                 605
Lys Gly Ile Glu Glu Lys Ile Leu Asn Phe Ile Ala Lys Gln Gly Asn
    610                 615                 620
Leu
625
```

<210> 34
<211> 1205
<212> PRT
<213> Plasmodium falciparum

<400> 34

```
Met Ile Phe Asn Tyr Val Phe Phe Lys Asn Phe Val Pro Val Val Leu
 1              5                   10                  15
Tyr Ile Leu Leu Ile Ile Tyr Ile Asn Leu Asn Gly Met Asn Asn Lys
            20                  25                  30
Asn Gln Ile Lys Thr Glu Lys Ile Tyr Ile Lys Lys Leu Asn Arg Leu
        35                  40                  45
Ser Arg Lys Asn Ser Leu Cys Ser Ser Lys Asn Lys Ile Ala Cys Leu
    50                  55                  60
Phe Asp Ile Gly Asn Asp Asp Asn Arg Asn Thr Thr Tyr Gly Tyr Asn
65                  70                  75                  80
Val Asn Val Lys Asn Asp Asp Ile Asn Ser Leu Leu Lys Asn Asn Tyr
                85                  90                  95
Ser Asn Lys Leu Tyr Met Asp Lys Arg Lys Asn Ile Asn Asn Val Ile
            100                 105                 110
Ser Thr Asn Lys Ile Ser Gly Ser Ile Ser Asn Ile Cys Ser Arg Asn
```

```
            115                     120                     125
Gln Lys Glu Asn Glu Gln Lys Arg Asn Lys Gln Arg Cys Leu Thr Gln
        130                     135                     140
Cys His Thr Tyr Asn Met Ser His Glu Gln Asp Lys Leu Ala Asn Asp
145                     150                     155                 160
Asn Asn Arg Asn Asn Lys Lys Asn Phe Asn Leu Leu Phe Ile Asn Tyr
                    165                     170                     175
Phe Asn Leu Lys Arg Met Lys Asn Ser Leu Leu Asn Lys Asp Asn Phe
                180                     185                     190
Phe Tyr Cys Lys Glu Lys Lys Leu Ser Phe Leu His Lys Ala Tyr Lys
            195                     200                     205
Lys Lys Asn Cys Thr Phe Gln Asn Tyr Ser Leu Lys Arg Lys Ser Asn
        210                     215                     220
Arg Asp Ser His Lys Leu Phe Ser Gly Glu Phe Asp Asp Tyr Thr Asn
225                     230                     235                 240
Asn Asn Ala Leu Tyr Glu Ser Glu Lys Lys Glu Tyr Ile Thr Leu Asn
                    245                     250                     255
Asn Asn Asn Lys Asn Asn Asn Asn Lys Asn Asn Asp Asn Lys Asn Asn
                260                     265                     270
Asp Asn Asn Asp Tyr Asn Asn Asn Asn Ser Cys Asn Asn Leu Gly Glu
            275                     280                     285
Arg Ser Asn His Tyr Asp Asn Tyr Gly Gly Asp Asn Asn Asn Pro Cys
        290                     295                     300
Asn Asn Asn Asn Asp Lys Tyr Asp Ile Gly Lys Tyr Phe Lys Gln Ile
305                     310                     315                 320
Asn Thr Phe Ile Asn Ile Asp Glu Tyr Lys Thr Ile Tyr Gly Asp Glu
                    325                     330                     335
Ile Tyr Lys Glu Ile Tyr Glu Leu Tyr Val Glu Arg Asn Ile Pro Glu
                340                     345                     350
Tyr Tyr Glu Arg Lys Tyr Phe Ser Glu Asp Ile Lys Lys Ser Val Leu
            355                     360                     365
Phe Asp Ile Asp Lys Tyr Asn Asp Val Glu Phe Glu Lys Ala Ile Lys
        370                     375                     380
Glu Glu Phe Ile Asn Asn Gly Val Tyr Ile Asn Asn Ile Asp Asn Thr
385                     390                     395                 400
Tyr Tyr Lys Lys Glu Asn Ile Leu Ile Met Lys Lys Ile Leu His Tyr
                    405                     410                     415
Phe Pro Leu Leu Lys Leu Ile Asn Asn Pro Ser Asp Leu Lys Lys Leu
                420                     425                     430
Lys Lys Gln Tyr Leu Pro Leu Leu Ala His Glu Leu Lys Ile Phe Leu
            435                     440                     445
Phe Phe Ile Val Asn Ile Thr Gly Gly His Phe Ser Ser Val Leu Ser
        450                     455                     460
Ser Leu Glu Ile Gln Leu Leu Leu Leu Tyr Ile Phe Asn Gln Pro Tyr
465                     470                     475                 480
Asp Asn Val Ile Tyr Asp Ile Gly His Gln Ala Tyr Val His Lys Ile
                    485                     490                     495
Leu Thr Gly Arg Lys Leu Leu Phe Leu Ser Leu Arg Asn Lys Lys Gly
                500                     505                     510
Ile Ser Gly Phe Leu Asn Ile Phe Glu Ser Ile Tyr Asp Lys Phe Gly
            515                     520                     525
Ala Gly His Ser Ser Thr Ser Leu Ser Ala Ile Gln Gly Tyr Tyr Glu
        530                     535                     540
Ala Glu Trp Gln Val Lys Asn Lys Glu Lys Tyr Gly Asn Gly Asp Ile
545                     550                     555                 560
Glu Ile Ser Asp Asn Ala Asn Val Thr Asn Asn Glu Arg Ile Phe Gln
                    565                     570                     575
Lys Gly Ile His Asn Asp Asn Asn Ile Asn Asn Asn Ile Asn Asn Asn
                580                     585                     590
Asn Tyr Ile Asn Pro Ser Asp Val Val Gly Arg Glu Asn Thr Asn Val
            595                     600                     605
```

```
Pro Asn Val Arg Asn Asp Asn His Asn Val Asp Lys Val His Ile Ala
    610                 615                 620
Ile Ile Gly Asp Gly Gly Leu Thr Gly Gly Met Ala Leu Glu Ala Leu
625                 630                 635                 640
Asn Tyr Ile Ser Phe Leu Asn Ser Lys Ile Leu Ile Ile Tyr Asn Asp
                645                 650                 655
Asn Gly Gln Val Ser Leu Pro Thr Asn Ala Val Ser Ile Ser Gly Asn
            660                 665                 670
Arg Pro Ile Gly Ser Ile Ser Asp His Leu His Tyr Phe Val Ser Asn
        675                 680                 685
Ile Glu Ala Asn Ala Gly Asp Asn Lys Leu Ser Lys Asn Ala Lys Glu
    690                 695                 700
Asn Asn Ile Phe Glu Asn Leu Asn Tyr Asp Tyr Ile Gly Val Val Asn
705                 710                 715                 720
Gly Asn Asn Thr Glu Glu Leu Phe Lys Val Leu Asn Asn Ile Lys Glu
                725                 730                 735
Asn Lys Leu Lys Arg Ala Thr Val Leu His Val Arg Thr Lys Lys Ser
            740                 745                 750
Asn Asp Phe Ile Asn Ser Lys Ser Pro Ile Ser Ile Leu His Ser Ile
        755                 760                 765
Lys Lys Asn Glu Ile Phe Pro Phe Asp Thr Thr Ile Leu Asn Gly Asn
    770                 775                 780
Ile His Lys Glu Asn Lys Ile Glu Glu Glu Lys Asn Val Ser Ser Ser
785                 790                 795                 800
Thr Lys Tyr Asp Val Asn Asn Lys Asn Asn Lys Asn Asn Asp Asn Ser
                805                 810                 815
Glu Ile Ile Lys Tyr Glu Asp Met Phe Ser Lys Glu Thr Phe Thr Asp
            820                 825                 830
Ile Tyr Thr Asn Glu Met Leu Lys Tyr Leu Lys Lys Asp Arg Asn Ile
        835                 840                 845
Ile Phe Leu Ser Pro Ala Met Leu Gly Gly Ser Gly Leu Val Lys Ile
    850                 855                 860
Ser Glu Arg Tyr Pro Asn Asn Val Tyr Asp Val Gly Ile Ala Glu Gln
865                 870                 875                 880
His Ser Val Thr Phe Ala Ala Ala Met Ala Met Asn Lys Lys Leu Lys
                885                 890                 895
Ile Gln Leu Cys Ile Tyr Ser Thr Phe Leu Gln Arg Ala Tyr Asp Gln
            900                 905                 910
Ile Ile His Asp Leu Asn Leu Gln Asn Ile Pro Leu Lys Val Ile Ile
        915                 920                 925
Gly Arg Ser Gly Leu Val Gly Glu Asp Gly Ala Thr His Gln Gly Ile
    930                 935                 940
Tyr Asp Leu Ser Tyr Leu Gly Thr Leu Asn Asn Ala Tyr Ile Ile Ser
945                 950                 955                 960
Pro Ser Asn Gln Val Asp Leu Lys Arg Ala Leu Arg Phe Ala Tyr Leu
                965                 970                 975
Asp Lys Asp His Ser Val Tyr Ile Arg Ile Pro Arg Met Asn Ile Leu
            980                 985                 990
Ser Asp Lys Tyr Met Lys Gly Tyr Leu Asn Ile His Met Lys Asn Glu
        995                 1000                1005
Ser Lys Asn Ile Asp Val Asn Val Asp Ile Asn Asp Asp Val Asp Lys
    1010                1015                1020
Tyr Ser Glu Glu Tyr Met Asp Asp Asp Asn Phe Ile Lys Ser Phe Ile
1025                1030                1035                1040
Gly Lys Ser Arg Ile Ile Lys Met Asp Asn Glu Asn Asn Asn Thr Asn
                1045                1050                1055
Glu His Tyr Ser Ser Arg Gly Asp Thr Gln Thr Lys Lys Lys Lys Val
            1060                1065                1070
Cys Ile Phe Asn Met Gly Ser Met Leu Phe Asn Val Ile Asn Ala Ile
        1075                1080                1085
Lys Glu Ile Glu Lys Glu Gln Tyr Ile Ser His Asn Tyr Ser Phe Ser
```

```
    1090                1095                1100
Ile Val Asp Met Ile Phe Leu Asn Pro Leu Asp Lys Asn Met Ile Asp
1105                1110                1115                1120
His Val Ile Lys Gln Asn Lys His Gln Tyr Leu Ile Thr Tyr Glu Asp
                1125                1130                1135
Asn Thr Ile Gly Gly Phe Ser Thr His Phe Asn Asn Tyr Leu Ile Glu
            1140                1145                1150
Asn Asn Tyr Ile Thr Lys His Asn Leu Tyr Val His Asn Ile Tyr Leu
        1155                1160                1165
Ser Asn Glu Pro Ile Glu His Ala Ser Phe Lys Asp Gln Gln Glu Val
    1170                1175                1180
Val Lys Met Asp Lys Cys Ser Leu Val Asn Arg Ile Lys Asn Tyr Leu
1185                1190                1195                1200
Lys Asn Asn Pro Thr
                1205
```

<210> 35
<211> 631
<212> PRT
<213> Streptomyces sp. CL 190

<400> 35

```
Met Thr Ile Leu Glu Asn Ile Arg Gln Pro Arg Asp Leu Lys Ala Leu
 1               5                  10                  15
Pro Glu Glu Gln Leu His Glu Leu Ser Glu Glu Ile Arg Gln Phe Leu
            20                  25                  30
Val His Ala Val Thr Arg Thr Gly Gly His Leu Gly Pro Asn Leu Gly
        35                  40                  45
Val Val Glu Leu Thr Ile Ala Leu His Arg Val Phe Glu Ser Pro Val
    50                  55                  60
Asp Arg Ile Leu Trp Asp Thr Gly His Gln Ser Tyr Val His Lys Leu
65                  70                  75                  80
Leu Thr Gly Arg Gln Asp Phe Ser Lys Leu Arg Gly Lys Gly Gly Leu
                85                  90                  95
Ser Gly Tyr Pro Ser Arg Glu Glu Ser Glu His Asp Val Ile Glu Asn
            100                 105                 110
Ser His Ala Ser Thr Ala Leu Gly Trp Ala Asp Gly Leu Ala Lys Ala
        115                 120                 125
Arg Arg Val Gln Gly Glu Lys Gly His Val Val Ala Val Ile Gly Gly
    130                 135                 140
Arg Ala Leu Thr Gly Gly Met Ala Trp Glu Ala Leu Asn Asn Ile Ala
145                 150                 155                 160
Ala Ala Lys Asp Gln Pro Leu Ile Ile Val Val Asn Asp Asn Glu Arg
                165                 170                 175
Ser Tyr Ala Pro Thr Ile Gly Gly Leu Ala Asn His Leu Ala Thr Leu
            180                 185                 190
Arg Thr Thr Asp Gly Tyr Glu Lys Val Leu Ala Trp Gly Lys Asp Val
        195                 200                 205
Leu Leu Arg Thr Pro Ile Val Gly His Pro Leu Tyr Glu Ala Leu His
    210                 215                 220
Gly Ala Lys Lys Gly Phe Lys Asp Ala Phe Ala Pro Gln Gly Met Phe
225                 230                 235                 240
Glu Asp Leu Gly Leu Lys Tyr Val Gly Pro Ile Asp Gly His Asp Ile
                245                 250                 255
Gly Ala Val Glu Ser Ala Leu Arg Arg Ala Lys Arg Phe His Gly Pro
            260                 265                 270
Val Leu Val His Cys Leu Thr Val Lys Gly Arg Gly Tyr Glu Pro Ala
        275                 280                 285
Leu Ala His Glu Glu Asp His Phe His Thr Val Gly Val Met Asp Pro
    290                 295                 300
Leu Thr Cys Glu Pro Leu Ser Pro Thr Asp Gly Pro Ser Trp Thr Ser
```

```
305                 310                 315                 320
Val Phe Gly Asp Glu Ile Val Arg Ile Gly Ala Glu Arg Glu Asp Ile
                325                 330                 335
Val Ala Ile Thr Ala Ala Met Leu His Pro Val Gly Leu Ala Arg Phe
            340                 345                 350
Ala Asp Arg Phe Pro Asp Arg Val Trp Asp Val Gly Ile Ala Glu Gln
        355                 360                 365
His Ala Ala Val Ser Ala Ala Gly Leu Ala Thr Gly Gly Leu His Pro
    370                 375                 380
Val Val Ala Val Tyr Ala Thr Phe Leu Asn Arg Ala Phe Asp Gln Leu
385                 390                 395                 400
Leu Met Asp Val Ala Leu His Arg Cys Gly Val Thr Phe Val Leu Asp
                405                 410                 415
Arg Ala Gly Val Thr Gly Val Asp Gly Ala Ser His Asn Gly Met Trp
            420                 425                 430
Asp Met Ser Val Leu Gln Val Val Pro Gly Leu Arg Ile Ala Ala Pro
        435                 440                 445
Arg Asp Ala Asp His Val Arg Ala Gln Leu Arg Glu Ala Val Ala Val
    450                 455                 460
Asp Asp Ala Pro Thr Leu Ile Arg Phe Pro Lys Glu Ser Val Gly Pro
465                 470                 475                 480
Arg Ile Pro Ala Leu Asp Arg Val Gly Gly Leu Asp Val Leu His Arg
                485                 490                 495
Asp Glu Arg Pro Glu Val Leu Leu Val Ala Val Gly Val Met Ala Gln
            500                 505                 510
Val Cys Leu Gln Thr Ala Glu Leu Leu Arg Ala Arg Gly Ile Gly Cys
        515                 520                 525
Thr Val Val Asp Pro Arg Trp Val Lys Pro Val Asp Pro Val Leu Pro
    530                 535                 540
Pro Leu Ala Ala Glu His Arg Leu Val Ala Val Val Glu Asp Asn Ser
545                 550                 555                 560
Arg Ala Ala Gly Val Gly Ser Ala Val Ala Leu Ala Leu Gly Asp Ala
                565                 570                 575
Asp Val Asp Val Pro Val Arg Arg Phe Gly Ile Pro Glu Gln Phe Leu
            580                 585                 590
Ala His Ala Arg Arg Gly Glu Val Leu Ala Asp Ile Gly Leu Thr Pro
        595                 600                 605
Val Glu Ile Ala Gly Arg Ile Gly Ala Ser Leu Pro Val Arg Glu Glu
    610                 615                 620
Pro Ala Glu Glu Gln Pro Ala
625                 630
```

<210> 36
<211> 618
<212> PRT
<213> Helicobacter pylori

<400> 36

```
Met Ile Leu Gln Asn Lys Thr Phe Asp Leu Asn Pro Asn Asp Ile Ala
 1               5                  10                  15
Gly Leu Glu Leu Val Cys Gln Thr Leu Arg Asn Arg Ile Leu Glu Val
            20                  25                  30
Val Ser Ala Asn Gly Gly His Leu Ser Ser Ser Leu Gly Ala Val Glu
        35                  40                  45
Leu Ile Val Gly Met His Ala Leu Phe Asp Cys Gln Lys Asn Pro Phe
    50                  55                  60
Ile Phe Asp Thr Ser His Gln Ala Tyr Ala His Lys Leu Leu Thr Gly
65                  70                  75                  80
Arg Phe Glu Ser Phe Ser Thr Leu Arg Gln Phe Lys Gly Leu Ser Gly
                85                  90                  95
Phe Thr Lys Pro Ser Glu Ser Ala Tyr Asp Tyr Phe Ile Ala Gly His
```

```
                100                     105                     110
Ser Ser Thr Ser Val Ser Ile Gly Val Gly Val Ala Lys Ala Phe Cys
            115                     120                     125
Leu Lys Gln Ala Leu Gly Met Pro Ile Ala Leu Leu Gly Asp Gly Ser
    130                     135                     140
Ile Ser Ala Gly Ile Phe Tyr Glu Ala Leu Asn Glu Leu Gly Asp Arg
145                     150                     155                 160
Lys Tyr Pro Met Ile Met Ile Leu Asn Asp Asn Glu Met Ser Ile Ser
                165                     170                     175
Thr Pro Ile Gly Ala Leu Ser Lys Ala Leu Ser Gln Leu Met Lys Gly
            180                     185                     190
Pro Phe Tyr Gln Ser Phe Arg Ser Lys Val Lys Lys Ile Leu Ser Thr
        195                     200                     205
Leu Pro Glu Ser Val Asn Tyr Leu Ala Ser Arg Phe Glu Glu Ser Phe
    210                     215                     220
Lys Leu Ile Thr Pro Gly Val Phe Phe Glu Glu Leu Gly Ile Asn Tyr
225                     230                     235                 240
Ile Gly Pro Ile Asn Gly His Asp Leu Ser Ala Ile Ile Glu Thr Leu
                245                     250                     255
Lys Leu Ala Lys Glu Leu Lys Glu Pro Val Leu Ile His Ala Gln Thr
            260                     265                     270
Leu Lys Gly Lys Gly Tyr Lys Ile Ala Glu Gly Arg Tyr Glu Lys Trp
        275                     280                     285
His Gly Val Gly Pro Phe Asp Leu Asp Thr Gly Leu Ser Lys Lys Ser
    290                     295                     300
Lys Ser Ala Ile Leu Ser Pro Thr Glu Ala Tyr Ser Asn Thr Leu Leu
305                     310                     315                 320
Glu Leu Ala Lys Lys Asp Glu Lys Ile Val Gly Val Thr Ala Ala Met
                325                     330                     335
Pro Ser Gly Thr Gly Leu Asp Lys Leu Ile Asp Ala Tyr Pro Leu Arg
            340                     345                     350
Phe Phe Asp Val Ala Ile Ala Glu Gln His Ala Leu Thr Ser Ser Ser
        355                     360                     365
Ala Met Ala Lys Glu Gly Phe Lys Pro Phe Val Ser Ile Tyr Ser Thr
    370                     375                     380
Phe Leu Gln Arg Ala Tyr Asp Ser Ile Val His Asp Ala Cys Ile Ser
385                     390                     395                 400
Ser Leu Pro Ile Lys Leu Ala Ile Asp Arg Ala Gly Ile Val Gly Glu
                405                     410                     415
Asp Gly Glu Thr His Gln Gly Leu Leu Asp Val Ser Tyr Leu Arg Ser
            420                     425                     430
Ile Pro Asn Met Val Ile Phe Ala Pro Arg Asp Asn Glu Thr Leu Lys
        435                     440                     445
Asn Ala Val Arg Phe Ala Asn Glu His Asp Ser Ser Pro Cys Ala Phe
    450                     455                     460
Arg Tyr Pro Arg Gly Ser Phe Ala Leu Lys Glu Gly Val Phe Glu Pro
465                     470                     475                 480
Ser Gly Phe Val Leu Gly Gln Ser Glu Leu Leu Lys Lys Glu Gly Glu
                485                     490                     495
Ile Leu Leu Ile Gly Tyr Gly Asn Gly Val Gly Arg Ala His Leu Val
            500                     505                     510
Gln Leu Ala Leu Lys Glu Lys Asn Ile Glu Cys Ala Leu Leu Asp Leu
        515                     520                     525
Arg Phe Leu Lys Pro Leu Asp Pro Asn Leu Ser Ala Ile Val Ala Pro
    530                     535                     540
Tyr Gln Lys Leu Tyr Val Phe Ser Asp Asn Tyr Lys Leu Gly Gly Val
545                     550                     555                 560
Ala Ser Ala Ile Leu Glu Phe Leu Ser Glu Gln Asn Ile Leu Lys Pro
                565                     570                     575
Val Lys Ser Phe Glu Ile Ile Asp Glu Phe Ile Met His Gly Asn Thr
            580                     585                     590
```

```
Ala Leu Val Glu Lys Ser Leu Gly Leu Asp Thr Glu Ser Leu Thr Asp
        595                 600                 605
Ala Ile Leu Lys Asp Leu Gly Gln Glu Arg
    610                 615
```

<210> 37
<211> 1990
<212> DNA
<213> Rhodobacter sphaeroides

<400> 37

```
cgacggcccg gtagccccgg cgcggctgca gcaccgtcag acgtccgccg agaaagccgt      60
cggaagtcaa ttcgtccggg gcgaacatca gggggtcgtc gggatgccgt tgtcggacat     120
cacccggcag gcgcgatccc agtcttcttc cgggacaaac agacgccgcg gcaatatgcc     180
gatggagcct tcgaggacgc tcatgtggac gtccaccgga aaggcgtcta tatcctcgcc     240
ctgaaggagc gcggtggcga aggcgatgat cgtcgggtcg gtcgtgcgca acagttcctt     300
catgtcgggg acattgtcgg caacgcctcg gtttgtcgag gccggttcgt cgaccgggtg     360
gcaggatcgg gatgggattg gacgaggttt cgcaaaagcc gcatgaacgg ctcgccgcgt     420
ggctggccga ggacatggcc gccgtcaacg ggctgatccg cgagcggatg gcctcgaaac     480
acgcgccccg cattcccgag gtcacggcgc atctggtcga ggccggcggc aagcggctgc     540
ggccgctcct gacgctcgcc gcggcgcggc tgtgcggcta cgaggggccc tatcacatcc     600
atctggccgc gacggtggag ttcatccaca cggcgacgct gcttcacgac gatgtggtgg     660
acgaaagcca ccgccgccgc ggcaaaccca cggcgaacct gctgtgggac aacaaatcct     720
cggtgctggt gggcgactat ctcttcgccc gcagcttcca gctgatggtc gagaccggct     780
cgcttcgcgt gatggacatc ctcgccaatg cctcggccac catctccgag ggcgaggtgc     840
tgcagctgac cgcggcccag gatctgcgca cgaccgagga catccacctg caggtggtgc     900
gcggcaagac ggccgcgctc tttgccgcgg caaccgaggt gggcggcgtg gtcgcgggcg     960
tgcccgaggc gcaggtcgag gcgctccacg cctacgggga cgcgctgggg atcgccttcc    1020
agatcgtcga cgacctcctc gattatggcg gcgtggatgc ccagatcggc aagaacaccg    1080
gcgacgactt ccgcgaacgc aagctgacgc tgccggtcat caaggcggtg gcccaggccg    1140
atgccgagga gcgcgccttc tggcagcggg tgatcgagaa gggcgaccag cgcgagggtg    1200
acctcgagca agcccatgcg atcatgtccc gccacggcgc catggaggcc gcccggcagg    1260
atgcgctccg ctgggtcacg gtggcgcgcg aggcactcgg ccagctgccg gagcacccgc    1320
tgcgcgagat gctgcacgat ctggccgatt tcgtggtcga acgcatcgcc tgatcccttc    1380
cgggcgctct gccccggcgc agcgcaggat cccgcgctgc gccccttttcg gccttccgac    1440
agtccctctg ccgcgggagg ccggcctcgc ctgagaagcc gcactggccg ccggtcttcc    1500
cccgaaccgc tcccgggcct gctcggaagg cgtccgccgc aaaagccccc gcgggggggc    1560
cccaccggcg gccatcagga agagaccgtt gaagcggccc gctcgaatcc tgtcgcgccc    1620
ccccccgacc gggcggctct ccgatccgtg ttcgctcggc gatggacagc cgttccctgt    1680
ccgttcatga tggcgccatg cagaccctta ccgttcccga ttccggcctc gccccctcct    1740
gcccggccaa aggctcgccc gcggcgtctg ccgccatctg cgcagccatg atttcgtctc    1800
ggtggtcgaa ctcgtgcccg cgcccggcct cagggtcgac gtgatggcgc tggggcccaa    1860
gggcgagatc tgggtggtgg aatgcaaatc ctcgcgcgcg gactatcagt ccgaccgcaa    1920
gtggcagggc tatctcgact ggtgcgaccg cttcttcttc gcggtggacg aggaccagcc    1980
cgggccgtcg                                                           1990
```

<210> 38
<211> 1002
<212> DNA
<213> Rhodobacter sphaeroides

<400> 38

```
atgggattgg acgaggtttc gcaaaagccg catgaacggc tcgccgcgtg gctggccgag      60
gacatggccg ccgtcaacgg gctgatccgc gagcggatgg cctcgaaaca cgcgccccgc     120
attccccgagg tcacggcgca tctggtcgag gccggcggca agcggctgcg gccgctcctg    180
acgctcgccg cggcgcggct gtgcggctac gaggggccct atcacatcca tctggccgcg     240
acggtggagt tcatccacac ggcgacgctg cttcacgacg atgtggtgga cgaaagccac     300
cgccgccgcg gcaaacccac ggcgaacctg ctgtgggaca acaaatcctc ggtgctggtg     360
ggcgactatc tcttcgcccg cagcttccag ctgatggtcg agaccggctc gcttcgcgtg     420
atggacatcc tcgccaatgc ctcggccacc atctccgagg gcgaggtgct gcagctgacc     480
gcggcccagg atctgcgcac gaccgaggac atccacctgc aggtggtgcg cggcaagacg     540

gccgcgctct ttgccgcggc aaccgaggtg ggcggcgtgg tcgcgggcgt gcccgaggcg     600
caggtcgagg cgctccacgc ctacggggac gcgctgggga tcgccttcca gatcgtcgac     660
gacctcctcg attatggcgg cgtggatgcc cagatcggca agaacaccgg cgacgacttc     720
cgcgaacgca agctgacgct gccggtcatc aaggcggtgg cccaggccga tgccgaggag     780
cgcgccttct ggcagcgggt gatcgagaag ggcgaccagc gcgagggtga cctcgagcaa     840
gcccatgcga tcatgtcccg ccacggcgcc atggaggccg cccggcagga tgcgctccgc     900
tgggtcacgg tggcgcgcga ggcactcggc cagctgccgg agcacccgct gcgcgagatg     960
ctgcacgatc tggccgattt cgtggtcgaa cgcatcgcct ga                       1002
```

<210> 39
<211> 333
<212> PRT
<213> Rhodobacter sphaeroides

<400> 39

```
Met Gly Leu Asp Glu Val Ser Gln Lys Pro His Glu Arg Leu Ala Ala
 1               5                  10                  15
Trp Leu Ala Glu Asp Met Ala Ala Val Asn Gly Leu Ile Arg Glu Arg
            20                  25                  30
Met Ala Ser Lys His Ala Pro Arg Ile Pro Glu Val Thr Ala His Leu
        35                  40                  45
Val Glu Ala Gly Gly Lys Arg Leu Arg Pro Leu Leu Thr Leu Ala Ala
    50                  55                  60
Ala Arg Leu Cys Gly Tyr Glu Gly Pro Tyr His Ile His Leu Ala Ala
65                  70                  75                  80
Thr Val Glu Phe Ile His Thr Ala Thr Leu Leu His Asp Asp Val Val
                85                  90                  95
Asp Glu Ser His Arg Arg Arg Gly Lys Pro Thr Ala Asn Leu Leu Trp
            100                 105                 110
Asp Asn Lys Ser Ser Val Leu Val Gly Asp Tyr Leu Phe Ala Arg Ser
        115                 120                 125
Phe Gln Leu Met Val Glu Thr Gly Ser Leu Arg Val Met Asp Ile Leu
    130                 135                 140
Ala Asn Ala Ser Ala Thr Ile Ser Glu Gly Glu Val Leu Gln Leu Thr
145                 150                 155                 160
Ala Ala Gln Asp Leu Arg Thr Thr Glu Asp Ile His Leu Gln Val Val
                165                 170                 175
Arg Gly Lys Thr Ala Ala Leu Phe Ala Ala Ala Thr Glu Val Gly Gly
            180                 185                 190
Val Val Ala Gly Val Pro Glu Ala Gln Val Glu Ala Leu His Ala Tyr
        195                 200                 205
Gly Asp Ala Leu Gly Ile Ala Phe Gln Ile Val Asp Asp Leu Leu Asp
    210                 215                 220
Tyr Gly Gly Val Asp Ala Gln Ile Gly Lys Asn Thr Gly Asp Asp Phe
225                 230                 235                 240
Arg Glu Arg Lys Leu Thr Leu Pro Val Ile Lys Ala Val Ala Gln Ala
                245                 250                 255
Asp Ala Glu Glu Arg Ala Phe Trp Gln Arg Val Ile Glu Lys Gly Asp
            260                 265                 270
Gln Arg Glu Gly Asp Leu Glu Gln Ala His Ala Ile Met Ser Arg His
        275                 280                 285
Gly Ala Met Glu Ala Ala Arg Gln Asp Ala Leu Arg Trp Val Thr Val
    290                 295                 300
Ala Arg Glu Ala Leu Gly Gln Leu Pro Glu His Pro Leu Arg Glu Met
305                 310                 315                 320
Leu His Asp Leu Ala Asp Phe Val Val Glu Arg Ile Ala
                325                 330
```

<210> 40
<211> 1833
<212> DNA
<213> Sphingomonas trueperi

<400> 40

```
ggatcgcgca gcgcctcggc cacgcgcacc atcagcagca gattgccgtt cggcagccgc     60
gcgaagccgg ggttgaaggc gccaaggaca taggtcgcgt cgtccacccc ctcgcgcagc    120
ggtgagcggg tcaggtcgac attgtcgggc cggaagatca gataatcgtc gctcaagcgc    180
ttgccccctc gggtttcacg cccagcaacg gggtcaggcc ccgggggttc cggcttcagc    240
gccggcttcc tgggcctggc ggtggtgccg gatcacctcg tcgatgatga agcgcaggaa    300
tttctcggaa aattcggggt cgagatcggc atcctgcgcc agcgcgcgca gccgggcgat    360
ctgcgcctcc tcgcggccgg gatcggcggg cggcagcccg gattcggcct tgtagcgccc    420
caccgcctgg gtcaccttga accgctcggc gagcatgaag acgagcgccg catcgatatt    480
gtcgatgctc tggcgatagc gggtcagcgt cgcgtcggtc atgcgaatct cctttgccgc    540
tgcggcacgg ccatgcaagc acctcttgcc tttgcaatgc acaaaggcca gaggctcgtt    600
gcatatgagc gcaaccgtcc accgcctggg ctcgcgaacc cagccttcgc tcgatccgat    660
catggcgctg gtcgcccagg acatgaacct ggtgaacgcg gtgatcctcg atcgcatgca    720
gtccgagatc ccgctgatcc ccgaactcgc cggccatctg atcgctggcg gcggcaagcg    780
gatgcggccg atgctgacgc tcgccagcgc ccggctgctc ggctattcgg gcacgcgcca    840
ccacaagctg gcggcggcag tggagttcat ccacaccgcg acgctgctgc atgacgacgt    900
ggtcgacagc tcggacctgc gccgcggccg ccgcaccgcc aacatcatct ggggcaatcc    960
cgccagcgtg ctggtcggcg acttcctgtt cagccgctcg ttcgagctga tggtcgaggc   1020
cgaaagcctc aaggcgctgc acatcctgtc gaacgccagc gcggtgatcg ccgagggcga   1080
agtcaaccag ctgaccgcgg tgcgccggat cgacctgtcc gaggatcgct atctcgacat   1140
catcggcgcc aagactgcgg cgctgttcgc cgccgcctgc cgggtggcgg gcgtggtcgc   1200
cgagcgtccc gaggcggagg aactcgcgct cgacgcctat ggccgcaacc tcggcatcgc   1260
tttccagctg gtcgacgacg cgatcgacta tgtctcggac gcgtcgacga tgggcaagga   1320
tgccggcgac gatttccgcg aaggcaagat gacgctgccg gtggtcctgg cgtacgcgcg   1380
cggcgacgag gcggaacgcg gcttctggaa ggaagcgatt tcgggccgcc gcatctcgga   1440
cgaggatttc gccgaggcga tccggctggt gcagagctgc cgcgcggtgg acgacacgct   1500
cgcccgtgcc cgccattacg gccagctcgc gatcgatgcg ctgggcggct tccgcgcctg   1560
cgaggcgaag gacgcgatgg tcgaggcggt cgaattcgcg gtggcgcgcg cctactgacg   1620
cgcgccgacc ggagcatttc cgggtggatc gcttgcgatc caaggctcgg gaaatgcgac   1680
catcaaaaag cttccgggga ttacgcctcg gtcgactttt cttcgccctc gtcctcgtcg   1740
acttcgagcg cgtcttcctc gtccatgtcg agcactacct cgatgccctc gacgatcagg   1800
tcgagctgct cgtagctcgc cgtcatctcg atc                                1833
```

<210> 41
<211> 1014
<212> DNA
<213> Sphingomonas trueperi

<400> 41

```
atgagcgcaa ccgtccaccg cctgggctcg cgaacccagc cttcgctcga tccgatcatg     60
gcgctggtcg cccaggacat gaacctggtg aacgcggtga tcctcgatcg catgcagtcc    120
gagatcccgc tgatccccga actcgccggc catctgatcg ctggcggcgg caagcggatg    180
cggccgatgc tgacgctcgc cagcgcccgg ctgctcggct attcgggcac gcgccaccac    240
aagctggcgg cggcagtgga gttcatccac accgcgacgc tgctgcatga cgacgtggtc    300
gacagctcgg acctgcgccg cggccgccgc accgccaaca tcatctgggg caatcccgcc    360
agcgtgctgg tcggcgactt cctgttcagc cgctcgttcg agctgatggt cgaggccgaa    420
agcctcaagg cgctgcacat cctgtcgaac gccagcgcgg tgatcgccga gggcgaagtc    480
aaccagctga ccgcggtgcg ccggatcgac ctgtccgagg atcgctatct cgacatcatc    540
ggcgccaaga ctgcggcgct gttcgccgcc gcctgccggg tggcgggcgt ggtcgccgag    600
cgtcccgagg cggaggaact cgcgctcgac gcctatggcc gcaacctcgg catcgctttc    660
cagctggtcg acgacgcgat cgactatgtc tcggacgcgt cgacgatggg caaggatgcc    720
ggcgacgatt tccgcgaagg caagatgacg ctgccggtgg tcctggcgta cgcgcgcggc    780
gacgaggcgg aacgcggctt ctggaaggaa gcgatttcgg gccgccgcat ctcggacgag    840
gatttcgccg aggcgatccg gctggtgcag agctgccgcg cggtggacga cacgctcgcc    900
cgtgcccgcc attacggcca gctcgcgatc gatgcgctgg gcggcttccg cgcctgcgag    960
gcgaaggacg cgatggtcga ggcggtcgaa ttcgcggtgg cgcgcgccta ctga         1014
```

<210> 42
<211> 337
<212> PRT
<213> Sphingomonas trueperi

<400> 42

```
Met Ser Ala Thr Val His Arg Leu Gly Ser Arg Thr Gln Pro Ser Leu
1               5                   10                  15
Asp Pro Ile Met Ala Leu Val Ala Gln Asp Met Asn Leu Val Asn Ala
            20                  25                  30
Val Ile Leu Asp Arg Met Gln Ser Glu Ile Pro Leu Ile Pro Glu Leu
        35                  40                  45
Ala Gly His Leu Ile Ala Gly Gly Gly Lys Arg Met Arg Pro Met Leu
    50                  55                  60
Thr Leu Ala Ser Ala Arg Leu Leu Gly Tyr Ser Gly Thr Arg His His
65                  70                  75                  80
Lys Leu Ala Ala Ala Val Glu Phe Ile His Thr Ala Thr Leu Leu His
                85                  90                  95
Asp Asp Val Val Asp Ser Ser Asp Leu Arg Arg Gly Arg Arg Thr Ala
            100                 105                 110
Asn Ile Ile Trp Gly Asn Pro Ala Ser Val Leu Val Gly Asp Phe Leu
        115                 120                 125
Phe Ser Arg Ser Phe Glu Leu Met Val Glu Ala Glu Ser Leu Lys Ala
    130                 135                 140
Leu His Ile Leu Ser Asn Ala Ser Ala Val Ile Ala Glu Gly Glu Val
145                 150                 155                 160
Asn Gln Leu Thr Ala Val Arg Arg Ile Asp Leu Ser Glu Asp Arg Tyr
                165                 170                 175
Leu Asp Ile Ile Gly Ala Lys Thr Ala Ala Leu Phe Ala Ala Ala Cys
            180                 185                 190
Arg Val Ala Gly Val Val Ala Glu Arg Pro Glu Ala Glu Glu Leu Ala
        195                 200                 205
Leu Asp Ala Tyr Gly Arg Asn Leu Gly Ile Ala Phe Gln Leu Val Asp
    210                 215                 220
Asp Ala Ile Asp Tyr Val Ser Asp Ala Ser Thr Met Gly Lys Asp Ala
225                 230                 235                 240
Gly Asp Asp Phe Arg Glu Gly Lys Met Thr Leu Pro Val Val Leu Ala
                245                 250                 255
Tyr Ala Arg Gly Asp Glu Ala Glu Arg Gly Phe Trp Lys Glu Ala Ile
            260                 265                 270
Ser Gly Arg Arg Ile Ser Asp Glu Asp Phe Ala Glu Ala Ile Arg Leu
        275                 280                 285
Val Gln Ser Cys Arg Ala Val Asp Asp Thr Leu Ala Arg Ala Arg His
    290                 295                 300
Tyr Gly Gln Leu Ala Ile Asp Ala Leu Gly Gly Phe Arg Ala Cys Glu
305                 310                 315                 320
Ala Lys Asp Ala Met Val Glu Ala Val Glu Phe Ala Val Ala Arg Ala
                325                 330                 335
Tyr
```

<210> 43
<211> 1137
<212> DNA
<213> Schizosaccharomyces pombe

<400> 43

```
atgattcagt atgtatattt aaaacatatg aggaaattat ggagtcttgg aaaagtccgt      60
tcgactgttc ttcggttttc tactacgaac cgcaatgctt cacatttaat taaaaacgag     120
ttggaacaaa tctcaccagg gattcgtcaa atgctgaatt caaattcaga atttcttgaa     180
gagtgttcta aatattatac cattgctcaa ggaaaacaaa tgcgtccttc tcttgttttg     240
ctgatgtcca aagctacaag cttgtgccat ggtattgatc ggtccgtagt gggcgacaaa     300
tatattgatg atgatgattt aagatcattt tcgacgggtc aaattcttcc ttctcaattg     360
```

```
agattagcac aaataaccga gatgatccat atagcaagtt tgctgcatga cgatgtgatt     420
gatcacgcta atgtccgtag aggctcacct tcaagcaatg ttgctttcgg taatcgacgg     480
tcaatccttg cgggtaattt catccttgca cgggcttcga ctgctatggc ccgccttcga     540
aatccccaag ttacggagtt gttagctaca gtgatagcag acttggttcg aggtgagttt     600
ttgcagctaa aaaatactat ggatccttca tctttggaaa taaaacaatc aaattttgac     660
tattatattg aaaaaagttt tttgaaaaca gccagtttaa tttccaaaag ctgcaaggct     720
tctacaatcc tcggacaatg ttctcctact gtagcaacag ctgctggaga atacggtcga     780
tgcattggta ctgcttttca actaatggat gacgtgttgg actatacgtc gaaagatgat     840
actttaggaa aggcggctgg tgcagatttg aagctagggt tggctacagc tcccgtcctc     900
tttgcatgga aaaagtatcc agaacttggt gcaatgattg tgaatagatt caatcatcct     960
tctgatatcc aacgggctcg ttctttggtt gagtgcactg atgctatcga gcaaaccatc    1020
acttgggcaa aagaatatat caaaaaagcc aaagattccc ttctgtgtct ccctgattca    1080
cctgcaagga aggcactttt tgcgttggct gataaagtaa taacgagaaa gaagtga       1137
```

<210> 44
<211> 948
<212> DNA
<213> Gluconobacter suboxydans

<400> 44

```
atgctggcct gcaaccgggc gatcatcgcc cggatggaaa gtccggttcc cctgatcccg      60
cagcttggcg cccatcttgt cgcggcggga ggcaagcgcc ttcgcccgct gctgacgctg     120
gcctccgcac gtctgtgcgg ttatcagccg ggtccggacc atcagcgtca tgtcgggctc     180
gccgcctgcg ttgagttcat tcataccgcc acactgctgc atgatgatgt cgtggatgag     240
agcacgttgc gtcgggggct ggcttcggcc aatgccgtgt tcggcaacaa ggcgtccgtg     300
ctggtaggtg acttcctgtt cgcccgctcg ttccagctta tgacagcaga cggctccctg     360
aaggtcatgg cgatcctgtc ggatgcatcg gcgacaattg ctgaaggtga agtccttcag     420
atggtcgtgc agaacgacct tacgacgcct gtagaacgct atcttgaagt cattcacggc     480
aagacggctg cgctgtttgc ggctgcctgc cgtgtcggcg ctgtcgtggc cgagcgtccg     540
gaagcagaag aggaagctct ggagcggttt ggcaccaatc tgggtatggc gttccagctt     600
gttgatgatg ccctggatta tgccgcagac cagcaggttt tgggcaagac cgttggtgat     660
gacatgcgtg aaggcaagat caccctgccg gtcctggccg cctatgaggc tggctcgccg     720
gaagatcgta ttttctggga gcgcgtcatt ggagaagggg agcagactga ggacgatctg     780
cctcatgctc tgaacctgat tgcaaagacg ggtgcgatca atacgacgat cgcccgcgcg     840
caggtctatg ccgacgcagc tgttgaagcc ctgtccattt tcccggatag cgaactgcgc     900
cgccttctga tcgaaacggt tcagttcacg gtgaatcggg cccgctaa                  948
```

<210> 45
<211> 978
<212> DNA
<213> Rhodobacter capsulatus

<400> 45

```
atggccatcg atttcaagca agatattctc gctcctgttg ctcaagattt tgcagcgatg      60
gaccagttta ttaatgaagg aatcagctcc aaggtcgcac tggtcatgtc agtcagcaag     120
catgtcgttg aagcaggtgg aaagcgcatg cgtccgatta tgtgcttgct ggccgcttat     180
gcctgtggtg aaaccaattt aaagcatgca cagaagctgg cggccattat tgaaatgctg     240
catacggcga ctctggtaca tgatgatgat gtagatgagt ctggcttacg ccgtggcaga     300
ccaacagcaa atgcgacatg gaataaccag actgcggtac tggtggggga ttttctgatt     360
gcccgggcat ttgatctgct ggttgatctg gacaatatga tcctgttaaa ggacttctct     420
acaggaacct gtgagattgc tgagggtgaa gtattgcagt tgcaggcaca gcatcagcca     480
gatacaacag aagatattta tttacagatt attcacggta aaacctcacg gttgttcgaa     540
ctggcgaccg aaggcgctgc aatactggca ggcaaacctg aataccgtga acctttacgt     600
cgttttgccg gacactttgg caatgctttt cagattattg atgatattct ggattacact     660
tcagatgctg atacgctcgg caaaaatatt ggcgatgact tgatggaagg caaacccacc     720
ctgccgctga ttgcagcaat gcaaaatact caaggtgaac agcgcgacct gatccgtcgc     780
agcattgcca ctggcggtac ttcacagctt gaacaagtta ttgcgattgt acaaaattcg     840
ggagcgctgg attattgcca taagcgtgct actgaagaaa ccgagcgagc attacaggca     900
ctagaaatat tacctgagag tacttaccgg caggcgctgg ttaacttgac ccgcttagct     960
ttagaccgaa tccaataa                                                   978
```

<210> 46
<211> 315
<212> PRT
<213> Gluconobacter suboxydans

<400> 46

Met Leu Ala Cys Asn Arg Ala Ile Ile Ala Arg Met Glu Ser Pro Val
1               5                   10                  15
Pro Leu Ile Pro Gln Leu Gly Ala His Leu Val Ala Ala Gly Gly Lys
            20                  25                  30
Arg Leu Arg Pro Leu Leu Thr Leu Ala Ser Ala Arg Leu Cys Gly Tyr
        35                  40                  45
Gln Pro Gly Pro Asp His Gln Arg His Val Gly Leu Ala Ala Cys Val
    50                  55                  60
Glu Phe Ile His Thr Ala Thr Leu Leu His Asp Asp Val Val Asp Glu
65                  70                  75                  80
Ser Thr Leu Arg Arg Gly Leu Ala Ser Ala Asn Ala Val Phe Gly Asn
                85                  90                  95
Lys Ala Ser Val Leu Val Gly Asp Phe Leu Phe Ala Arg Ser Phe Gln
            100                 105                 110
Leu Met Thr Ala Asp Gly Ser Leu Lys Val Met Ala Ile Leu Ser Asp
        115                 120                 125
Ala Ser Ala Thr Ile Ala Glu Gly Glu Val Leu Gln Met Val Val Gln
    130                 135                 140
Asn Asp Leu Thr Thr Pro Val Glu Arg Tyr Leu Glu Val Ile His Gly
145                 150                 155                 160
Lys Thr Ala Ala Leu Phe Ala Ala Ala Cys Arg Val Gly Ala Val Val
                165                 170                 175
Ala Glu Arg Pro Glu Ala Glu Glu Glu Ala Leu Glu Arg Phe Gly Thr
            180                 185                 190
Asn Leu Gly Met Ala Phe Gln Leu Val Asp Asp Ala Leu Asp Tyr Ala
        195                 200                 205
Ala Asp Gln Gln Val Leu Gly Lys Thr Val Gly Asp Asp Met Arg Glu
    210                 215                 220
Gly Lys Ile Thr Leu Pro Val Leu Ala Ala Tyr Glu Ala Gly Ser Pro
225                 230                 235                 240
Glu Asp Arg Ile Phe Trp Glu Arg Val Ile Gly Glu Gly Glu Gln Thr
                245                 250                 255
Glu Asp Asp Leu Pro His Ala Leu Asn Leu Ile Ala Lys Thr Gly Ala
            260                 265                 270
Ile Asn Thr Thr Ile Ala Arg Ala Gln Val Tyr Ala Asp Ala Ala Val
        275                 280                 285
Glu Ala Leu Ser Ile Phe Pro Asp Ser Glu Leu Arg Arg Leu Leu Ile
    290                 295                 300
Glu Thr Val Gln Phe Thr Val Asn Arg Ala Arg
305                 310                 315

<210> 47
<211> 378
<212> PRT
<213> Schizosaccharomyces pombe

<400> 47

Met Ile Gln Tyr Val Tyr Leu Lys His Met Arg Lys Leu Trp Ser Leu
1               5                   10                  15
Gly Lys Val Arg Ser Thr Val Leu Arg Phe Ser Thr Thr Asn Arg Asn
            20                  25                  30
Ala Ser His Leu Ile Lys Asn Glu Leu Glu Gln Ile Ser Pro Gly Ile
        35                  40                  45
Arg Gln Met Leu Asn Ser Asn Ser Glu Phe Leu Glu Glu Cys Ser Lys
    50                  55                  60

```
Tyr Tyr Thr Ile Ala Gln Gly Lys Gln Met Arg Pro Ser Leu Val Leu
65                  70                  75                  80
Leu Met Ser Lys Ala Thr Ser Leu Cys His Gly Ile Asp Arg Ser Val
                85                  90                  95
Val Gly Asp Lys Tyr Ile Asp Asp Asp Asp Leu Arg Ser Phe Ser Thr
            100                 105                 110
Gly Gln Ile Leu Pro Ser Gln Leu Arg Leu Ala Gln Ile Thr Glu Met
        115                 120                 125
Ile His Ile Ala Ser Leu Leu His Asp Asp Val Ile Asp His Ala Asn
    130                 135                 140
Val Arg Arg Gly Ser Pro Ser Ser Asn Val Ala Phe Gly Asn Arg Arg
145                 150                 155                 160
Ser Ile Leu Ala Gly Asn Phe Ile Leu Ala Arg Ala Ser Thr Ala Met
                165                 170                 175
Ala Arg Leu Arg Asn Pro Gln Val Thr Glu Leu Leu Ala Thr Val Ile
            180                 185                 190
Ala Asp Leu Val Arg Gly Glu Phe Leu Gln Leu Lys Asn Thr Met Asp
        195                 200                 205
Pro Ser Ser Leu Glu Ile Lys Gln Ser Asn Phe Asp Tyr Tyr Ile Glu
    210                 215                 220
Lys Ser Phe Leu Lys Thr Ala Ser Leu Ile Ser Lys Ser Cys Lys Ala
225                 230                 235                 240
Ser Thr Ile Leu Gly Gln Cys Ser Pro Thr Val Ala Thr Ala Ala Gly
                245                 250                 255
Glu Tyr Gly Arg Cys Ile Gly Thr Ala Phe Gln Leu Met Asp Asp Val
            260                 265                 270
Leu Asp Tyr Thr Ser Lys Asp Asp Thr Leu Gly Lys Ala Ala Gly Ala
        275                 280                 285
Asp Leu Lys Leu Gly Leu Ala Thr Ala Pro Val Leu Phe Ala Trp Lys
    290                 295                 300
Lys Tyr Pro Glu Leu Gly Ala Met Ile Val Asn Arg Phe Asn His Pro
305                 310                 315                 320
Ser Asp Ile Gln Arg Ala Arg Ser Leu Val Glu Cys Thr Asp Ala Ile
                325                 330                 335
Glu Gln Thr Ile Thr Trp Ala Lys Glu Tyr Ile Lys Lys Ala Lys Asp
            340                 345                 350
Ser Leu Leu Cys Leu Pro Asp Ser Pro Ala Arg Lys Ala Leu Phe Ala
        355                 360                 365
Leu Ala Asp Lys Val Ile Thr Arg Lys Lys
    370                 375
```

<210> 48
<211> 325
<212> PRT
<213> Rhodobacter capsulatus

<400> 48

```
Met Ala Ile Asp Phe Lys Gln Asp Ile Leu Ala Pro Val Ala Gln Asp
1               5                   10                  15
Phe Ala Ala Met Asp Gln Phe Ile Asn Glu Gly Ile Ser Ser Lys Val
            20                  25                  30
Ala Leu Val Met Ser Val Ser Lys His Val Val Glu Ala Gly Gly Lys
        35                  40                  45
Arg Met Arg Pro Ile Met Cys Leu Leu Ala Ala Tyr Ala Cys Gly Glu
    50                  55                  60
Thr Asn Leu Lys His Ala Gln Lys Leu Ala Ala Ile Ile Glu Met Leu
65                  70                  75                  80
His Thr Ala Thr Leu Val His Asp Asp Asp Val Asp Glu Ser Gly Leu
                85                  90                  95
Arg Arg Gly Arg Pro Thr Ala Asn Ala Thr Trp Asn Asn Gln Thr Ala
            100                 105                 110

Val Leu Val Gly Asp Phe Leu Ile Ala Arg Ala Phe Asp Leu Leu Val
        115                 120                 125
Asp Leu Asp Asn Met Ile Leu Leu Lys Asp Phe Ser Thr Gly Thr Cys
    130                 135                 140
Glu Ile Ala Glu Gly Glu Val Leu Gln Leu Gln Ala Gln His Gln Pro
145                 150                 155                 160
Asp Thr Thr Glu Asp Ile Tyr Leu Gln Ile Ile His Gly Lys Thr Ser
                165                 170                 175
Arg Leu Phe Glu Leu Ala Thr Glu Gly Ala Ala Ile Leu Ala Gly Lys
            180                 185                 190
Pro Glu Tyr Arg Glu Pro Leu Arg Arg Phe Ala Gly His Phe Gly Asn
        195                 200                 205
Ala Phe Gln Ile Ile Asp Asp Ile Leu Asp Tyr Thr Ser Asp Ala Asp
    210                 215                 220
Thr Leu Gly Lys Asn Ile Gly Asp Asp Leu Met Glu Gly Lys Pro Thr
225                 230                 235                 240
Leu Pro Leu Ile Ala Ala Met Gln Asn Thr Gln Gly Glu Gln Arg Asp
                245                 250                 255
Leu Ile Arg Arg Ser Ile Ala Thr Gly Gly Thr Ser Gln Leu Glu Gln
            260                 265                 270
Val Ile Ala Ile Val Gln Asn Ser Gly Ala Leu Asp Tyr Cys His Lys
        275                 280                 285
Arg Ala Thr Glu Glu Thr Glu Arg Ala Leu Gln Ala Leu Glu Ile Leu
    290                 295                 300
Pro Glu Ser Thr Tyr Arg Gln Ala Leu Val Asn Leu Thr Arg Leu Ala
305                 310                 315                 320
Leu Asp Arg Ile Gln
                325
```

<210> 49
<211> 325
<212> PRT
<213> Rhodobacter capsulatus

<400> 49

Met Ala Ile Asp Phe Lys Gln Asp Ile Leu Ala Pro Val Ala Gln Asp
1 5 10 15
Phe Ala Ala Met Asp Gln Phe Ile Asn Glu Gly Ile Ser Ser Lys Val
20 25 30
Ala Leu Val Met Ser Val Ser Lys His Val Val Glu Ala Gly Gly Lys
35 40 45
Arg Met Arg Pro Ile Met Cys Leu Leu Ala Ala Tyr Ala Cys Gly Glu
50 55 60
Thr Asn Leu Lys His Ala Gln Lys Leu Ala Ala Ile Ile Glu Met Leu
65 70 75 80
His Thr Ala Thr Leu Val His Asp Asp Val Val Asp Glu Ser Gly Leu
85 90 95
Arg Arg Gly Arg Pro Thr Ala Asn Ala Thr Trp Asn Asn Gln Thr Ala
100 105 110
Val Leu Val Gly Asp Phe Leu Ile Ala Arg Ala Phe Asp Leu Leu Val
115 120 125
Asp Leu Asp Asn Met Ile Leu Leu Lys Asp Phe Ser Thr Gly Thr Cys
130 135 140
Glu Ile Ala Glu Gly Glu Val Leu Gln Leu Gln Ala Gln His Gln Pro
145 150 155 160
Asp Thr Thr Glu Asp Ile Tyr Leu Gln Ile Ile His Gly Lys Thr Ser
165 170 175
Arg Leu Phe Glu Leu Ala Thr Glu Gly Ala Ala Ile Leu Ala Gly Lys
180 185 190
Pro Glu Tyr Arg Glu Pro Leu Arg Arg Phe Ala Gly His Phe Gly Asn
195 200 205

Ala Phe Gln Ile Ile Asp Asp Ile Leu Asp Tyr Thr Ser Asp Ala Asp
210 215 220
Thr Leu Gly Lys Asn Ile Gly Asp Asp Leu Met Glu Gly Lys Pro Thr
225 230 235 240
Leu Pro Leu Ile Ala Ala Met Gln Asn Thr Gln Gly Glu Gln Arg Asp
245 250 255
Leu Ile Arg Arg Ser Ile Ala Thr Gly Gly Thr Ser Gln Leu Glu Gln
260 265 270
Val Ile Ala Ile Val Gln Asn Ser Gly Ala Leu Asp Tyr Cys His Lys
275 280 285
Arg Ala Thr Glu Glu Thr Glu Arg Ala Leu Gln Ala Leu Glu Ile Leu
290 295 300
Pro Glu Ser Thr Tyr Arg Gln Ala Leu Val Asn Leu Thr Arg Leu Ala
305 310 315 320
Leu Asp Arg Ile Gln
325

<210> 50
<211> 327
<212> PRT
<213> Rickettsia prowazeki

<400> 50

```
Met Asn Ile Ile Val Lys Ile Gln Gln Asn Leu Lys Asp Glu Val Thr
 1               5                  10                  15
Gln Leu Asn Asp Leu Ile Ile Ser Cys Leu Lys Ser Asp Ala Glu Leu
            20                  25                  30
Ile Glu Lys Val Gly Lys Tyr Leu Val Glu Ala Gly Gly Lys Arg Ile
        35                  40                  45
Arg Pro Leu Leu Thr Ile Ile Thr Ala Lys Met Phe Asp Tyr Lys Gly
    50                  55                  60
Asn Asn His Ile Lys Leu Ala Ser Ala Val Glu Phe Ile His Ala Ala
65                  70                  75                  80
Thr Leu Leu His Asp Asp Val Val Asp Asn Ser Thr Leu Arg Arg Phe
                85                  90                  95
Lys Pro Thr Ala Asn Val Ile Trp Gly Ser Lys Thr Ser Ile Leu Val
            100                 105                 110
Gly Asp Phe Leu Phe Ser Gln Ser Phe Lys Leu Met Val Ala Ser Gly
        115                 120                 125
Cys Ile Lys Ala Met Asn Val Leu Ala Lys Ala Ser Val Ile Ile Ser
    130                 135                 140
Glu Gly Glu Val Val Gln Leu Val Lys Leu Asn Glu Arg Arg Ile Ile
145                 150                 155                 160
Thr Ile Asp Glu Tyr Gln Gln Ile Val Lys Ser Lys Thr Ala Glu Leu
                165                 170                 175
Phe Gly Ala Ala Cys Glu Val Gly Ala Ile Ile Ala Glu Gln Val Asp
            180                 185                 190
Arg Val Ser Lys Asp Val Gln Asn Phe Gly Arg Leu Leu Gly Thr Ile
        195                 200                 205
Phe Gln Val Ile Asp Asp Leu Leu Asp Tyr Leu Gly Ser Asp Lys Gln
    210                 215                 220
Val Gly Lys Asn Ile Gly Asp Asp Phe Leu Glu Gly Lys Val Thr Leu
225                 230                 235                 240
Pro Leu Ile Phe Leu Tyr His Lys Leu Glu Gln Asp Lys Gln Leu Trp
                245                 250                 255
Leu Glu Asn Met Leu Lys Ser Asp Lys Arg Thr Lys Asp Asp Phe Val
            260                 265                 270
Lys Ile Arg Asp Leu Met Leu Lys His Ala Ile Tyr Asn Glu Thr Val
        275                 280                 285
Asn Tyr Leu Ser Ser Leu Glu Asn Glu Ala Asn Asn Leu Leu Asn Lys
    290                 295                 300

Ile Pro Val Gln Asn Ile Tyr Lys Tyr Tyr Leu Phe Ser Ile Ile Arg
305                 310                 315                 320
Phe Ile Leu Tyr Arg Ser Tyr
                325
```

<210> 51
<211> 323
<212> PRT
<213> Escherichia coli

<400> 51

```
Met Asn Leu Glu Lys Ile Asn Glu Leu Thr Ala Gln Asp Met Ala Gly
1               5                   10                  15
Val Asn Ala Ala Ile Leu Glu Gln Leu Asn Ser Asp Val Gln Leu Ile
            20                  25                  30
Asn Gln Leu Gly Tyr Tyr Ile Val Ser Gly Gly Gly Lys Arg Ile Arg
        35                  40                  45
Pro Met Ile Ala Val Leu Ala Ala Arg Ala Val Gly Tyr Glu Gly Asn
    50                  55                  60
Ala His Val Thr Ile Ala Ala Leu Ile Glu Phe Ile His Thr Ala Thr
65                  70                  75                  80
Leu Leu His Asp Asp Val Val Asp Glu Ser Asp Met Arg Arg Gly Lys
                85                  90                  95
Ala Thr Ala Asn Ala Ala Phe Gly Asn Ala Ala Ser Val Leu Val Gly
            100                 105                 110
Asp Phe Ile Tyr Thr Arg Ala Phe Gln Met Met Thr Ser Leu Gly Ser
        115                 120                 125
Leu Lys Val Leu Glu Val Met Ser Glu Ala Val Asn Val Ile Ala Glu
    130                 135                 140
Gly Glu Val Leu Gln Leu Met Asn Val Asn Asp Pro Asp Ile Thr Glu
145                 150                 155                 160
Glu Asn Tyr Met Arg Val Ile Tyr Ser Lys Thr Ala Arg Leu Phe Glu
                165                 170                 175
Ala Ala Ala Gln Cys Ser Gly Ile Leu Ala Gly Cys Thr Pro Glu Glu
            180                 185                 190
Glu Lys Gly Leu Gln Asp Tyr Gly Arg Tyr Leu Gly Thr Ala Phe Gln
        195                 200                 205
Leu Ile Asp Asp Leu Leu Asp Tyr Asn Ala Asp Gly Glu Gln Leu Gly
    210                 215                 220
Lys Asn Val Gly Asp Asp Leu Asn Glu Gly Lys Pro Thr Leu Pro Leu
225                 230                 235                 240
Leu His Ala Met His His Gly Thr Pro Glu Gln Ala Gln Met Ile Arg
                245                 250                 255
Thr Ala Ile Glu Gln Gly Asn Gly Arg His Leu Leu Glu Pro Val Leu
            260                 265                 270
Glu Ala Met Asn Ala Cys Gly Ser Leu Glu Trp Thr Arg Gln Arg Ala
        275                 280                 285
Glu Glu Glu Ala Asp Lys Ala Ile Ala Ala Leu Gln Val Leu Pro Asp
    290                 295                 300
Thr Pro Trp Arg Glu Ala Leu Ile Gly Leu Ala His Ile Ala Val Gln
305                 310                 315                 320
Arg Asp Arg
```

<210> 52
<211> 329
<212> PRT
<213> Haemophilus influenzae

<400> 52

```
Met Lys Lys Gln Asp Leu Met Ser Ile Asp Glu Ile Gln Lys Leu Ala
```

```
1               5                   10                  15
Asp Pro Asp Met Gln Lys Val Asn Gln Asn Ile Leu Ala Gln Leu Asn
            20                  25                  30
Ser Asp Val Pro Leu Ile Gly Gln Leu Gly Phe Tyr Ile Val Gln Gly
        35                  40                  45
Gly Gly Lys Arg Ile Arg Pro Leu Ile Ala Val Leu Ala Ala Arg Ser
    50                  55                  60
Leu Gly Phe Glu Gly Ser Asn Ser Ile Thr Cys Ala Thr Phe Val Glu
65                  70                  75                  80
Phe Ile His Thr Ala Ser Leu Leu His Asp Asp Val Val Asp Glu Ser
                85                  90                  95
Asp Met Arg Arg Gly Arg Ala Thr Ala Asn Ala Glu Phe Gly Asn Ala
            100                 105                 110
Ala Ser Val Leu Val Gly Asp Phe Ile Tyr Thr Arg Ala Phe Gln Leu
        115                 120                 125
Val Ala Gln Leu Glu Ser Leu Lys Ile Leu Ser Ile Met Ala Asp Ala
    130                 135                 140
Thr Asn Val Leu Ala Glu Gly Glu Val Gln Gln Leu Met Asn Val Asn
145                 150                 155                 160
Asp Pro Glu Thr Ser Glu Ala Asn Tyr Met Arg Val Ile Tyr Ser Lys
                165                 170                 175
Thr Ala Arg Leu Phe Glu Val Ala Gly Gln Ala Ala Ala Ile Val Ala
            180                 185                 190
Gly Gly Thr Glu Ala Gln Glu Lys Ala Leu Gln Asp Tyr Gly Arg Tyr
        195                 200                 205
Leu Gly Thr Ala Phe Gln Leu Val Asp Asp Val Leu Asp Tyr Ser Ala
    210                 215                 220
Asn Thr Gln Ala Leu Gly Lys Asn Val Gly Asp Asp Leu Ala Glu Gly
225                 230                 235                 240
Lys Pro Thr Leu Pro Leu Leu His Ala Met Arg His Gly Asn Ala Gln
                245                 250                 255
Gln Ala Ala Leu Ile Arg Glu Ala Ile Glu Gln Gly Gly Lys Arg Glu
            260                 265                 270
Ala Ile Asp Glu Val Leu Ala Ile Met Thr Glu His Lys Ser Leu Asp
        275                 280                 285
Tyr Ala Met Asn Arg Ala Lys Glu Glu Ala Gln Lys Ala Val Asp Ala
    290                 295                 300
Ile Glu Ile Leu Pro Glu Ser Glu Tyr Lys Gln Ala Leu Ile Ser Leu
305                 310                 315                 320
Ala Tyr Leu Ser Val Asp Arg Asn Tyr
                325
```

<210> 53
<211> 24
<212> DNA
<213> Artificial Sequence

<220>
<223> Primer

<400> 53
rtkattytma aygayaayga aatg 24

<210> 54
<211> 23
<212> DNA
<213> Artificial Sequence

<220>
<223> Primer

<400> 54
tttgaagary tvggywttaa cta 23

<210> 55
<211> 21
<212> DNA
<213> Artificial Sequence

<220>
<223> Primer

<400> 55
rcaycargct tayscvcaya a 21

<210> 56
<211> 22
<212> DNA
<213> Artificial Sequence

<220>
<223> Primer

<400> 56
cgtgytgytc dgcrathgcb ac 22

<210> 57
<211> 23
<212> DNA
<213> Artificial Sequence

<220>
<223> Primer

<400> 57
tgytcdgcra thgcbacrtc raa 23

<210> 58
<211> 20
<212> DNA
<213> Artificial Sequence

<220>
<223> Primer

<400> 58
ggsccdatrt agttaawrcc 20

<210> 59
<211> 27
<212> DNA
<213> Artificial Sequence

<220>
<223> Primer

<400> 59
tcgtgaccaa gaagggcaag ggctatg 27

<210> 60
<211> 27
<212> DNA
<213> Artificial Sequence

<220>
<223> Primer

<400> 60
gacaagtatc acggcgtcca gaagttc 27

<210> 61
<211> 27
<212> DNA
<213> Artificial Sequence

<220>
<223> Primer

<400> 61
atagcccttg cccttcttgg tcacgac 27

<210> 62
<211> 26
<212> DNA
<213> Artificial Sequence

<220>
<223> Primer

<400> 62
cgaacggatc atactcgctc tcgctg 26

<210> 63
<211> 28
<212> DNA
<213> Artificial Sequence

<220>
<223> Primer

<400> 63
tgaggatctt gtgcggatag cattggtg 28

<210> 64
<211> 26
<212> DNA
<213> Artificial Sequence

<220>
<223> Primer

<400> 64
agcggcgtct tgggtaggtc agccat 26

<210> 65
<211> 30
<212> DNA

<213> Artificial Sequence

<220>
<223> Primer

<400> 65
atatggtacc gtgtgactga cctgtccaac 30

<210> 66
<211> 30
<212> DNA
<213> Artificial Sequence

<220>
<223> Primer

<400> 66
agtctctaga atgttggaga ttcaaggtgg 30

<210> 67
<211> 20
<212> DNA
<213> Artificial Sequence

<220>
<223> Primer

<400> 67
ggwgghaarm gmmtkcgycc 20

<210> 68
<211> 20
<212> DNA
<213> Artificial Sequence

<220>
<223> Primer

<400> 68
acwytgstdc atgatgatgt 20

<210> 69
<211> 20
<212> DNA
<213> Artificial Sequence

<220>
<223> Primer
<221> misc_feature
<222> (1) ... (20)
<223> n = A,T,C or G

<400> 69
acnytnbtnc aygaygaygt 20

<210> 70
<211> 20
<212> DNA

<213> Artificial Sequence

<220>
<223> Primer

<400> 70
tyrtcyacsa catcatcatg 20

<210> 71
<211> 23
<212> DNA
<213> Artificial Sequence

<220>
<223> Primer

<400> 71 23
tghavkacyt caccytcrgm aat 23

<210> 72
<211> 20
<212> DNA
<213> Artificial Sequence

<220>
<223> Primer

<221> misc feature
<222> (1)...(20)
<223> n = A,T,C or G

<400> 72 20
tartcnarda trtcrtcdat 20

<210> 73
<211> 20
<212> DNA
<213> Artificial Sequence

<220>
<223> Primer
<221> misc_feature
<222> (1)...(20)
<223> n = A,T,C or G

<400> 73 20
tcrtcnccna ynktyttncc 20

<210> 74
<211> 26
<212> DNA
<213> Artificial Sequence

<220>
<223> Primer

<400> 74
tggaagctgc gggcgaagag atagtc 26

<210> 75
<211> 26
<212> DNA
<213> Artificial Sequence

<220>
<223> Primer

<400> 75
cccaccagca ccgaggattt gttgtc 26

<210> 76
<211> 27
<212> DNA
<213> Artificial Sequence

<220>
<223> Primer

<400> 76
gaacctgctg tgggacaaca aatcctc 27

<210> 77
<211> 27
<212> DNA
<213> Artificial Sequence

<220>
<223> Primer

<400> 77
tcggtgctgg tgggcgacta tctcttc 27

<210> 78
<211> 30
<212> DNA
<213> Artificial Sequence

<220>
<223> Primer

<400> 78
actagaattc cgcaacagtt ccttcatgtc 30

<210> 79
<211> 30
<212> DNA
<213> Artificial Sequence

<220>
<223> Primer

<400> 79
atagaagctt acttgcggtc ggactgatag 30

<210> 80
<211> 23
<212> DNA

<213> Artificial Sequence

<220>
<223> Primer

<400> 80
ctsstscayg aygaygtsgt sga 23

<210> 81
<211> 19
<212> DNA
<213> Artificial Sequence
<220>
<223> Primer

<400> 81
gtsgmvgssg gsggsaarc 19

<210> 82
<211> 18
<212> DNA
<213> Artificial Sequence

<220>
<223> Primer

<400> 82
ctsmtscayg aygaygts 18

<210> 83
<211> 20
<212> DNA
<213> Artificial Sequence

<220>
<223> Primer

<400> 83
dssrtbctsg tsggsgaytt 20

<210> 84
<211> 21
<212> DNA
<213> Artificial Sequence

<220>
<223> Primer

<400> 84
vakraartcs ccsacsagsa c 21

<210> 85
<211> 18
<212> DNA
<213> Artificial Sequence

<220>
<223> Primer

<400> 85 18
sacytcsccy tcsgcrat 18

<210> 86
<211> 21
<212> DNA
<213> Artificial Sequence

<220>
<223> Primer

<400> 86
rtcrtcsccv ayvktyttsc c 21

<210> 87
<211> 21
<212> DNA
<213> Artificial Sequence

<220>
<223> Primer

<400> 87
sggsagsgtv rbyttsccyt c 21

<210> 88
<211> 26
<212> DNA
<213> Artificial Sequence

<220>
<223> Primer

<400> 88
gtgctggtcg gcgacttcct gttcag 26

<210> 89
<211> 27
<212> DNA
<213> Artificial Sequence

<220>
<223> Primer

<400> 89
atcgacctgt ccgaggatcg ctatctc 27

<210> 90
<211> 26
<212> DNA
<213> Artificial Sequence

<220>
<223> Primer

<400> 90 26
tcgaacgagc ggctgaacag gaagtc 26

<210> 91
<211> 26
<212> DNA
<213> Artificial Sequence

<220>
<223> Primer

<400> 91
tggcgggatt gccccagatg atgttg 26

<210> 92
<211> 30
<212> DNA
<213> Artificial Sequence
<220>
<223> Primer

<400> 92
attaggtacc atcagataat cgtcgctcaa 30

<210> 93
<211> 30
<212> DNA
<213> Artificial Sequence

<220>
<223> Primer

<400> 93
tataggatcc gacatggacg aggaagacgc 30

<210> 94
<211> 20
<212> DNA
<213> Artificial Sequence

<220>
<223> Primer

<400> 94
cgatggtgac cacgaagaag 20

<210> 95
<211> 2017
<212> DNA
<213> Sphingomonas trueperi

<400> 95

```
ggcccgggct ggtggggttt ctggcgctgg ggctggtgtt cggcgcgttc ttcttcgtcg     60
cgatcgtgac gcggaacgcc aagctggcgg cggggcaggt ctatgtcggg ctgccggtgc    120
tcgcgctgct gctgctccgc gaccatccgc agggctttgc cgcgacgctg tggacgatgg    180
cgatcgtctg ggtgtgcgac agcggcgcct attttgccgg tcgcgcgatc ggtgggccca    240
agctcgcgcc ctcgatcagc ccgaacaaga cctgggcggg gctgatcggc gggttggttg    300
ccgcgatcct gttctccgcc ggctatgtcg cgctggcgcc ggggagcgcg atcggctggt    360
ggctggtcgc ggtgtcgccg ctggtagcct tcgcctcgca gatcggcgac ctgtacgaga    420
gccatctcaa gcgggtcgcg ggcgtgaagg attcgagcaa cctgctgccc ggccatggcg    480
gcattctcga ccggctcgac ggccttgtct tcgcagcccc ggttgcagct ttgtttttg     540
cgatccatca tcaggtggtc gtgggaggat actggtggtg aagcgcgtca cggtgttggg    600
ggcgaccggc tcggtcggca cctcgacgct ggatctgatc gaacgaaatc cgcacgcctt    660
cgaagtcgtg gcgctgaccg caaattgcga tgtcgagaag ctggctgccg cggcgatccg    720
cacgcgcgcg cgctgcgccg tggtcgccga cgagaaatgc ctgccggcgc tacaggagcg    780
gctggccggc agcggtgtcg aggcgatggg cggggcgcat tcggtgtgcg acgtggcgcg    840
gatgggtgct gactggacga tggctgcgat cgtcggcagc gcagggctca agccggtgat    900
ggccgcgctg gaggccggtg gcaccgtcgc gctcgcgaac aaggagtcgc tcgtctcggc    960
gggtgaggtg atgatggcgg cggcccgcgc gcatggcgcg acgctgctgc cggtcgattc   1020
ggagcacaat gcggtgttcc agtgcctcga tcgcaccgcg cccaggggcg tccgccggat   1080
catccttacc gccagcggtg gtccgttccg cgcgacgccg aaggaagcga tgcgcgacat   1140
caccccccgca caggcggtgg cgcatcccaa ctggtcgatg ggcgccaaga tctcggtcga   1200
ctccgcgacg atgatgaaca aggggctcga actgatcgaa gccttccacc tgttcccggt   1260
cgccgccgag caactggccg tgctggtcca tcgccaatcc gtcgtccatt cgatggtgga   1320
atatgtcgac ggatcggtgc tggcccagct cggcacgccc gacatgcgca cgccgatcgc   1380
ctatgcgctg gcttggcccg agcggatgga gacgctgtgc ccgccgctcg accttgccac   1440
ggtgggtaag ctcgagttcg aaaatcccga tctcgatcgc ttcccggcgc tcgcgctggc   1500
gatggaggca ttgaaggcgg gcggggcgcg tccggccatt ctcaatgccg ccaacgaagt   1560
cgccgtcgcg gcctttctcg ccgggcggat cggattcctt gaaattgccg caatctctgc   1620

cgatacgctg tctcgctatg acccggccgc gccggaaacg ctcgatgccg tgctggcgat   1680
cgacgcggag gcgcggcttt acgcggctga gcgagtgaag gactgcgtcg cttgatccaa   1740
tccccccggca tcctgctcac cattctggcg ttcgcgctgg tgatcggccc gctcgtgttc  1800
ctgcacgagc tgggacatta tctggcgggc cgcctcttcg ggtgaaggc cgaggaattc    1860
tcgatcggct tcggccgcga gatcgccggc accaccgatc gccgcggcac gcgctggaag   1920
ttcagcctgt tgccgctggg cggctatgtc cgcttcgccg gcgacatgaa cccggcgagc   1980
cagccttcgc ccgaatggct gcagaccagc ccgggcc                            2017
```

<210> 96
<211> 1161
<212> DNA
<213> Sphingomonas trueperi

<400> 96

```
gtggtgaagc gcgtcacggt gttgggggcg accggctcgg tcggcacctc gacgctggat      60
ctgatcgaac gaaatccgca cgccttcgaa gtcgtggcgc tgaccgcaaa ttgcgatgtc     120
gagaagctgg ctgccgcggc gatccgcacg cgcgcgcgct gcgccgtggt cgccgacgag     180
aaatgcctgc cggcgctaca ggagcggctg gccggcagcg gtgtcgaggc gatgggcggg     240
gcgcattcgg tgtgcgacgt ggcgcggatg ggtgctgact ggacgatggc tgcgatcgtc     300
ggcagcgcag ggctcaagcc ggtgatggcc gcgctggagg ccggtggcac cgtcgcgctc     360
gcgaacaagg agtcgctcgt ctcggcgggt gaggtgatga tggcggcggc ccgcgcgcat     420
ggcgcgacgc tgctgccggt cgattcggag cacaatgcgg tgttccagtg cctcgatcgc     480
accgcgccca ggggcgtccg ccggatcatc cttaccgcca gcggtggtcc gttccgcgcg     540
acgccgaagg aagcgatgcg cgacatcacc cccgcacagg cggtggcgca tcccaactgg     600
tcgatgggcg ccaagatctc ggtcgactcc gcgacgatga tgaacaaggg gctcgaactg     660
atcgaagcct tccacctgtt cccggtcgcc gccgagcaac tggccgtgct ggtccatcgc     720
caatccgtcg tccattcgat ggtggaatat gtcgacggat cggtgctggc ccagctcggc     780
acgcccgaca tgcgcacgcc gatcgcctat gcgctggctt ggcccgagcg gatggagacg     840
ctgtgcccgc cgctcgacct tgccacggtg ggtaagctcg agttcgaaaa tcccgatctc     900
gatcgcttcc cggcgctcgc gctggcgatg gaggcattga aggcgggcgg ggcgcgtccg     960
gccattctca atgccgccaa cgaagtcgcc gtcgcggcct ttctcgccgg gcggatcgga    1020
ttccttgaaa ttgccgcaat ctctgccgat acgctgtctc gctatgaccc ggccgcgccg    1080
gaaacgctcg atgccgtgct ggcgatcgac gcggaggcgc ggctttacgc ggctgagcga    1140
gtgaaggact gcgtcgcttg a                                               1161
```

<210> 97
<211> 386
<212> PRT
<213> Sphingomonas trueperi

<400> 97

```
Val Val Lys Arg Val Thr Val Leu Gly Ala Thr Gly Ser Val Gly Thr
1               5                   10                  15
Ser Thr Leu Asp Leu Ile Glu Arg Asn Pro His Ala Phe Glu Val Val
            20                  25                  30
Ala Leu Thr Ala Asn Cys Asp Val Glu Lys Leu Ala Ala Ala Ala Ile
        35                  40                  45
Arg Thr Arg Ala Arg Cys Ala Val Val Ala Asp Glu Lys Cys Leu Pro
    50                  55                  60
Ala Leu Gln Glu Arg Leu Ala Gly Ser Gly Val Glu Ala Met Gly Gly
65                  70                  75                  80
Ala His Ser Val Cys Asp Val Ala Arg Met Gly Ala Asp Trp Thr Met
                85                  90                  95
Ala Ala Ile Val Gly Ser Ala Gly Leu Lys Pro Val Met Ala Ala Leu
            100                 105                 110
Glu Ala Gly Gly Thr Val Ala Leu Ala Asn Lys Glu Ser Leu Val Ser
        115                 120                 125
Ala Gly Glu Val Met Met Ala Ala Ala Arg Ala His Gly Ala Thr Leu
    130                 135                 140
Leu Pro Val Asp Ser Glu His Asn Ala Val Phe Gln Cys Leu Asp Arg
145                 150                 155                 160
```

```
Thr Ala Pro Arg Gly Val Arg Arg Ile Ile Leu Thr Ala Ser Gly Gly
                165                 170                 175
Pro Phe Arg Ala Thr Pro Lys Glu Ala Met Arg Asp Ile Thr Pro Ala
            180                 185                 190
Gln Ala Val Ala His Pro Asn Trp Ser Met Gly Ala Lys Ile Ser Val
        195                 200                 205
Asp Ser Ala Thr Met Met Asn Lys Gly Leu Glu Leu Ile Glu Ala Phe
    210                 215                 220
His Leu Phe Pro Val Ala Ala Glu Gln Leu Ala Val Leu Val His Arg
225                 230                 235                 240
Gln Ser Val Val His Ser Met Val Glu Tyr Val Asp Gly Ser Val Leu
                245                 250                 255
Ala Gln Leu Gly Thr Pro Asp Met Arg Thr Pro Ile Ala Tyr Ala Leu
            260                 265                 270
Ala Trp Pro Glu Arg Met Glu Thr Leu Cys Pro Pro Leu Asp Leu Ala
        275                 280                 285
Thr Val Gly Lys Leu Glu Phe Glu Asn Pro Asp Leu Asp Arg Phe Pro
    290                 295                 300
Ala Leu Ala Leu Ala Met Glu Ala Leu Lys Ala Gly Gly Ala Arg Pro
305                 310                 315                 320
Ala Ile Leu Asn Ala Ala Asn Glu Val Ala Val Ala Ala Phe Leu Ala
                325                 330                 335
Gly Arg Ile Gly Phe Leu Glu Ile Ala Ala Ile Ser Ala Asp Thr Leu
            340                 345                 350
Ser Arg Tyr Asp Pro Ala Ala Pro Glu Thr Leu Asp Ala Val Leu Ala
        355                 360                 365
Ile Asp Ala Glu Ala Arg Leu Tyr Ala Ala Glu Arg Val Lys Asp Cys
    370                 375                 380
Val Ala
385
```

<210> 98
<211> 388
<212> PRT
<213> Bacillus subtilis

<400> 98

```
Met Lys Asn Ile Cys Leu Leu Gly Ala Thr Gly Ser Ile Gly Glu Gln
1               5                   10                  15
Thr Leu Asp Val Leu Arg Ala His Gln Asp Gln Phe Gln Leu Val Ser
            20                  25                  30
Met Ser Phe Gly Arg Asn Ile Asp Lys Ala Val Pro Met Ile Glu Val
        35                  40                  45
Phe Gln Pro Lys Phe Val Ser Val Gly Asp Leu Asp Thr Tyr His Lys
    50                  55                  60
Leu Lys Gln Met Ser Phe Ser Phe Glu Cys Gln Ile Gly Leu Gly Glu
65                  70                  75                  80
Glu Gly Leu Ile Glu Ala Ala Val Met Glu Glu Val Asp Ile Val Val
                85                  90                  95
Asn Ala Leu Leu Gly Ser Val Gly Leu Ile Pro Thr Leu Lys Ala Ile
            100                 105                 110
Glu Gln Lys Lys Thr Ile Ala Leu Ala Asn Lys Glu Thr Leu Val Thr
        115                 120                 125
Ala Gly His Ile Val Lys Glu His Ala Lys Lys Tyr Asp Val Pro Leu
    130                 135                 140
Leu Pro Val Asp Ser Glu His Ser Ala Ile Phe Gln Ala Leu Gln Gly
145                 150                 155                 160
Glu Gln Ala Lys Asn Ile Glu Arg Leu Ile Ile Thr Ala Ser Gly Gly
                165                 170                 175
Ser Phe Arg Asp Lys Thr Arg Glu Glu Leu Glu Ser Val Thr Val Glu
            180                 185                 190
```

```
Asp Ala Leu Lys His Pro Asn Trp Ser Met Gly Ala Lys Ile Thr Ile
        195                 200                 205
Asp Ser Ala Thr Met Met Asn Lys Gly Leu Glu Val Ile Glu Ala His
    210                 215                 220
Trp Leu Phe Asp Ile Pro Tyr Glu Gln Ile Asp Val Val Leu His Lys
225                 230                 235                 240
Glu Ser Ile Ile His Ser Met Val Glu Phe His Asp Lys Ser Val Ile
                245                 250                 255
Ala Gln Leu Gly Thr Pro Asp Met Arg Val Pro Ile Gln Tyr Ala Leu
            260                 265                 270
Thr Tyr Pro Asp Arg Leu Pro Leu Pro Asp Ala Lys Arg Leu Glu Leu
        275                 280                 285
Trp Glu Ile Gly Ser Leu His Phe Glu Lys Ala Asp Phe Asp Arg Phe
    290                 295                 300
Arg Cys Leu Gln Phe Ala Phe Glu Ser Gly Lys Ile Gly Gly Thr Met
305                 310                 315                 320
Pro Thr Val Leu Asn Ala Ala Asn Glu Val Ala Val Ala Ala Phe Leu
                325                 330                 335
Ala Gly Lys Ile Pro Phe Leu Ala Ile Glu Asp Cys Ile Glu Lys Ala
            340                 345                 350
Leu Thr Arg His Gln Leu Leu Lys Lys Pro Ser Trp Arg Thr Phe Lys
        355                 360                 365
Lys Trp Thr Lys Ile Pro Gly Asp Thr Ser Ile Gln Tyr Ser His Lys
    370                 375                 380
Val Val Cys Ser
385
```

<210> 99
<211> 397
<212> PRT
<213> Haemophilus influenzae

<400> 99

Met Gln Lys Gln Asn Ile Val Ile Leu Gly Ser Thr Gly Ser Ile Gly
1 5 10 15
Lys Ser Thr Leu Ser Val Ile Glu Asn Asn Pro Gln Lys Tyr His Ala
20 25 30
Phe Ala Leu Val Gly Gly Lys Asn Val Glu Ala Met Phe Glu Gln Cys
35 40 45
Ile Lys Phe Arg Pro His Phe Ala Ala Leu Asp Asp Val Asn Ala Ala
50 55 60
Lys Ile Leu Arg Glu Lys Leu Ile Ala His His Ile Pro Thr Glu Val
65 70 75 80
Leu Ala Gly Arg Arg Ala Ile Cys Glu Leu Ala Ala His Pro Asp Ala
85 90 95
Asp Gln Ile Met Ala Ser Ile Val Gly Ala Ala Gly Leu Leu Pro Thr
100 105 110
Leu Ser Ala Val Lys Ala Gly Lys Arg Val Leu Leu Ala Asn Lys Glu
115 120 125
Ser Leu Val Thr Cys Gly Gln Leu Phe Ile Asp Ala Val Lys Asn Tyr
130 135 140
Gly Ser Lys Leu Leu Pro Val Asp Ser Glu His Asn Ala Ile Phe Gln
145 150 155 160
Ser Leu Pro Pro Glu Ala Gln Glu Lys Ile Gly Phe Cys Pro Leu Ser
165 170 175
Glu Leu Gly Val Ser Lys Ile Ile Leu Thr Gly Ser Gly Gly Pro Phe
180 185 190
Arg Tyr Thr Pro Leu Glu Gln Phe Thr Asn Ile Thr Pro Glu Gln Ala
195 200 205
Val Ala His Pro Asn Trp Ser Met Gly Lys Lys Ile Ser Val Asp Ser
210 215 220

Ala Thr Met Met Asn Lys Gly Leu Glu Tyr Ile Glu Ala Arg Trp Leu
225 230 235 240
Phe Asn Ala Ser Ala Glu Glu Met Glu Val Ile Ile His Pro Gln Ser
245 250 255
Ile Ile His Ser Met Val Arg Tyr Val Asp Gly Ser Val Ile Thr Gln
260 265 270
Met Gly Asn Pro Asp Met Arg Thr Pro Ile Ala Glu Thr Met Ala Tyr
275 280 285
Pro His Arg Thr Phe Ala Gly Val Glu Pro Leu Asp Phe Phe Lys Ile
290 295 300
Lys Glu Leu Thr Phe Ile Glu Pro Asp Phe Asn Arg Tyr Pro Asn Leu
305 310 315 320
Lys Leu Ala Ile Asp Ala Phe Ala Ala Gly Gln Tyr Ala Thr Thr Ala
325 330 335
Met Asn Ala Ala Asn Glu Ile Ala Val Gln Ala Phe Leu Asp Arg Gln
340 345 350
Ile Gly Phe Met Asp Ile Ala Lys Ile Asn Ser Lys Thr Ile Glu Arg
355 360 365
Ile Ser Pro Tyr Thr Ile Gln Asn Ile Asp Asp Val Leu Glu Ile Asp
370 375 380
Ala Gln Ala Arg Glu Ile Ala Lys Thr Leu Leu Arg Glu
385 390 395

<210> 100
<211> 398
<212> PRT
<213> Escherichia coli

<400> 100

Met Lys Gln Leu Thr Ile Leu Gly Ser Thr Gly Ser Ile Gly Cys Ser
1 5 10 15
Thr Leu Asp Val Val Arg His Asn Pro Glu His Phe Arg Val Val Ala
20 25 30
Leu Val Ala Gly Lys Asn Val Thr Arg Met Val Glu Gln Cys Leu Glu
35 40 45
Phe Ser Pro Arg Tyr Ala Val Met Asp Asp Glu Ala Ser Ala Lys Leu
50 55 60
Leu Lys Thr Met Leu Gln Gln Gln Gly Ser Arg Thr Glu Val Leu Ser
65 70 75 80
Gly Gln Gln Ala Ala Cys Asp Met Ala Ala Leu Glu Asp Val Asp Gln
85 90 95
Val Met Ala Ala Ile Val Gly Ala Ala Gly Leu Leu Pro Thr Leu Ala
100 105 110
Ala Ile Arg Ala Gly Lys Thr Ile Leu Leu Ala Asn Lys Glu Ser Leu
115 120 125
Val Thr Cys Gly Arg Leu Phe Met Asp Ala Val Lys Gln Ser Lys Ala
130 135 140
Gln Leu Leu Pro Val Asp Ser Glu His Asn Ala Ile Phe Gln Ser Leu
145 150 155 160
Pro Gln Pro Ile Gln His Asn Leu Gly Tyr Ala Asp Leu Glu Gln Asn
165 170 175
Gly Val Val Ser Ile Leu Leu Thr Gly Ser Gly Gly Pro Phe Arg Glu
180 185 190
Thr Pro Leu Arg Asp Leu Ala Thr Met Thr Pro Asp Gln Ala Cys Arg
195 200 205
His Pro Asn Trp Ser Met Gly Arg Lys Ile Ser Val Asp Ser Ala Thr
210 215 220
Met Met Asn Lys Gly Leu Glu Tyr Ile Glu Ala Arg Trp Leu Phe Asn
225 230 235 240
Ala Ser Ala Ser Gln Met Glu Val Leu Ile His Pro Gln Ser Val Ile
245 250 255

His Ser Met Val Arg Tyr Gln Asp Gly Ser Val Leu Ala Gln Leu Gly
260 265 270
Glu Pro Asp Met Arg Thr Pro Ile Ala His Thr Met Ala Trp Pro Asn
275 280 285
Arg Val Asn Ser Gly Val Lys Pro Leu Asp Phe Cys Lys Leu Ser Ala
290 295 300
Leu Thr Phe Ala Ala Pro Asp Tyr Asp Arg Tyr Pro Cys Leu Lys Leu
305 310 315 320
Ala Met Glu Ala Phe Glu Gln Gly Gln Ala Ala Thr Thr Ala Leu Asn
325 330 335
Ala Ala Asn Glu Ile Thr Val Ala Ala Phe Leu Ala Gln Gln Ile Arg
340 345 350
Phe Thr Asp Ile Ala Ala Leu Asn Leu Ser Val Leu Glu Lys Met Asp
355 360 365
Met Arg Glu Pro Gln Cys Val Asp Asp Val Leu Ser Val Asp Ala Asn
370 375 380
Ala Arg Glu Val Ala Arg Lys Glu Val Met Arg Leu Ala Ser
385 390 395

<210> 101
<211> 388
<212> PRT
<213> Zymonas mobilis

<400> 101

```
Met Ser Gln Pro Arg Thr Val Thr Val Leu Gly Ala Thr Gly Ser Ile
 1               5                  10                  15
Gly His Ser Thr Leu Asp Leu Ile Glu Arg Asn Leu Asp Arg Tyr Gln
            20                  25                  30
Val Ile Ala Leu Thr Ala Asn Arg Asn Val Lys Asp Leu Ala Asp Ala
        35                  40                  45
Ala Lys Arg Thr Asn Ala Lys Arg Ala Val Ile Ala Asp Pro Ser Leu
    50                  55                  60
Tyr Asn Asp Leu Lys Glu Ala Leu Ala Gly Ser Ser Val Glu Ala Ala
65                  70                  75                  80
Ala Gly Ala Asp Ala Leu Val Glu Ala Ala Met Met Gly Ala Asp Trp
                85                  90                  95
Thr Met Ala Ala Ile Ile Gly Cys Ala Gly Leu Lys Ala Thr Leu Ala
            100                 105                 110
Ala Ile Arg Lys Gly Lys Thr Val Ala Leu Ala Asn Lys Glu Ser Leu
        115                 120                 125
Val Ser Ala Gly Gly Leu Met Ile Asp Ala Val Arg Glu His Gly Thr
    130                 135                 140
Thr Leu Leu Pro Val Asp Ser Glu His Asn Ala Ile Phe Gln Cys Phe
145                 150                 155                 160
Pro His His Asn Arg Asp Tyr Val Arg Arg Ile Ile Ile Thr Ala Ser
                165                 170                 175
Gly Gly Pro Phe Arg Thr Thr Ser Leu Ala Glu Met Ala Thr Val Thr
            180                 185                 190
Pro Glu Arg Ala Val Gln His Pro Asn Trp Ser Met Gly Ala Lys Ile
        195                 200                 205
Ser Ile Asp Ser Ala Thr Met Met Asn Lys Gly Leu Glu Leu Ile Glu
    210                 215                 220
Ala Tyr His Leu Phe Gln Ile Pro Leu Glu Lys Phe Glu Ile Leu Val
225                 230                 235                 240
His Pro Gln Ser Val Ile His Ser Met Val Glu Tyr Leu Asp Gly Ser
                245                 250                 255
Ile Leu Ala Gln Ile Gly Ser Pro Asp Met Arg Thr Pro Ile Gly His
            260                 265                 270
Thr Leu Ala Trp Pro Lys Arg Met Glu Thr Pro Ala Glu Ser Leu Asp
        275                 280                 285

Phe Thr Lys Leu Arg Gln Met Asp Phe Glu Ala Pro Asp Tyr Glu Arg
    290                 295                 300
Phe Pro Ala Leu Thr Leu Ala Met Glu Ser Ile Lys Ser Gly Gly Ala
305                 310                 315                 320
Arg Pro Ala Val Met Asn Ala Ala Asn Glu Ile Ala Val Ala Ala Phe
                325                 330                 335
Leu Asp Lys Lys Ile Gly Phe Leu Asp Ile Ala Lys Ile Val Glu Lys
            340                 345                 350
Thr Leu Asp His Tyr Thr Pro Ala Thr Pro Ser Ser Leu Glu Asp Val
        355                 360                 365
Phe Ala Ile Asp Asn Glu Ala Arg Ile Gln Ala Ala Ala Leu Met Glu
    370                 375                 380
Ser Leu Pro Ala
385
```

<210> 102
<211> 402
<212> PRT
<213> Synechococcus leopoliensis

<400> 102

Met Lys Ala Val Thr Leu Leu Gly Ser Thr Gly Ser Ile Gly Thr Gln
1 5 10 15
Thr Leu Asp Ile Leu Glu Gln Tyr Pro Asp Arg Phe Arg Leu Val Gly
20 25 30
Leu Ala Ala Gly Arg Asn Val Ala Leu Leu Ser Glu Gln Ile Arg Arg
35 40 45
His Arg Pro Glu Ile Val Ala Ile Gln Asp Ala Ala Gln Leu Ser Glu
50 55 60
Leu Gln Ala Ala Ile Ala Asp Leu Asp Asn Pro Pro Leu Ile Leu Thr
65 70 75 80
Gly Glu Ala Gly Val Thr Glu Val Ala Arg Tyr Gly Asp Ala Glu Ile
85 90 95
Val Val Thr Gly Ile Val Gly Cys Ala Gly Leu Leu Pro Thr Ile Ala
100 105 110
Ala Ile Glu Ala Gly Lys Asp Ile Ala Leu Ala Asn Lys Glu Thr Leu
115 120 125
Ile Ala Ala Gly Pro Val Val Leu Pro Leu Leu Gln Lys His Gly Val
130 135 140
Thr Ile Thr Pro Ala Asp Ser Glu His Ser Ala Ile Phe Gln Cys Ile
145 150 155 160
Gln Gly Leu Ser Thr His Ala Asp Phe Arg Pro Ala Gln Val Val Ala
165 170 175
Gly Leu Arg Arg Ile Leu Leu Thr Ala Ser Gly Gly Ala Phe Arg Asp
180 185 190
Trp Pro Val Glu Arg Leu Ser Gln Val Thr Val Ala Asp Ala Leu Lys
195 200 205
His Pro Asn Trp Ser Met Gly Arg Lys Ile Thr Val Asp Ser Ala Thr
210 215 220
Leu Met Asn Lys Gly Leu Glu Val Ile Glu Ala His Tyr Leu Phe Gly
225 230 235 240
Leu Asp Tyr Asp Tyr Ile Asp Ile Val Ile His Pro Gln Ser Ile Ile
245 250 255
His Ser Leu Ile Glu Leu Glu Asp Thr Ser Val Leu Ala Gln Leu Gly
260 265 270
Trp Pro Asp Met Arg Leu Pro Leu Leu Tyr Ala Leu Ser Trp Pro Asp
275 280 285
Arg Leu Ser Thr Gln Trp Ser Ala Leu Asp Leu Val Lys Ala Gly Ser
290 295 300
Leu Glu Phe Arg Glu Pro Asp His Ala Lys Tyr Pro Cys Met Asp Leu
305 310 315 320

Ala Tyr Ala Ala Gly Arg Lys Gly Gly Thr Met Pro Ala Val Leu Asn
325 330 335
Ala Ala Asn Glu Gln Ala Val Ala Leu Phe Leu Glu Glu Gln Ile His
340 345 350
Phe Ser Asp Ile Pro Arg Leu Ile Glu Arg Ala Cys Asp Arg His Gln
355 360 365
Thr Glu Trp Gln Gln Gln Pro Ser Leu Asp Asp Ile Leu Ala Tyr Asp
370 375 380
Ala Trp Ala Arg Gln Phe Val Gln Ala Ser Tyr Gln Ser Leu Glu Ser
385 390 395 400
Val Val

<210> 103
<211> 394
<212> PRT
<213> Synechocystis sp. PCC 6803

<400> 103

```
Met Val Lys Arg Ile Ser Ile Leu Gly Ser Thr Gly Ser Ile Gly Thr
1               5                   10                  15
Gln Thr Leu Asp Ile Val Thr His His Pro Asp Ala Phe Gln Val Val
            20                  25                  30
Gly Leu Ala Ala Gly Gly Asn Val Ala Leu Leu Ala Gln Gln Val Ala
        35                  40                  45
Glu Phe Arg Pro Glu Ile Val Ala Ile Arg Gln Ala Glu Lys Leu Glu
    50                  55                  60
Asp Leu Lys Ala Ala Val Ala Glu Leu Thr Asp Tyr Gln Pro Met Tyr
65                  70                  75                  80
Val Val Gly Glu Glu Gly Val Val Glu Val Ala Arg Tyr Gly Asp Ala
                85                  90                  95
Glu Ser Val Val Thr Gly Ile Val Gly Cys Ala Gly Leu Leu Pro Thr
            100                 105                 110
Met Ala Ala Ile Ala Ala Gly Lys Asp Ile Ala Leu Ala Asn Lys Glu
        115                 120                 125
Thr Leu Ile Ala Gly Ala Pro Val Val Leu Pro Leu Val Glu Lys Met
    130                 135                 140
Gly Val Lys Leu Leu Pro Ala Asp Ser Glu His Ser Ala Ile Phe Gln
145                 150                 155                 160
Cys Leu Gln Gly Val Pro Glu Gly Gly Leu Arg Arg Ile Ile Leu Thr
                165                 170                 175
Ala Ser Gly Gly Ala Phe Arg Asp Leu Pro Val Glu Arg Leu Pro Phe
            180                 185                 190
Val Thr Val Gln Asp Ala Leu Lys His Pro Asn Trp Ser Met Gly Gln
        195                 200                 205
Lys Ile Thr Ile Asp Ser Ala Thr Leu Met Asn Lys Gly Leu Glu Val
    210                 215                 220
Ile Glu Ala His Tyr Leu Phe Gly Leu Asp Tyr Asp His Ile Asp Ile
225                 230                 235                 240
Val Ile His Pro Gln Ser Ile Ile His Ser Leu Ile Glu Val Gln Asp
                245                 250                 255
Thr Ser Val Leu Ala Gln Leu Gly Trp Pro Asp Met Arg Leu Pro Leu
            260                 265                 270
Leu Tyr Ala Leu Ser Trp Pro Glu Arg Ile Tyr Thr Asp Trp Glu Pro
        275                 280                 285
Leu Asp Leu Val Lys Ala Gly Ser Leu Ser Phe Arg Glu Pro Asp His
    290                 295                 300
Asp Lys Tyr Pro Cys Met Gln Leu Ala Tyr Gly Ala Gly Arg Ala Gly
305                 310                 315                 320
Gly Ala Met Pro Ala Val Leu Asn Ala Ala Asn Glu Gln Ala Val Ala
                325                 330                 335

Leu Phe Leu Gln Glu Lys Ile Ser Phe Leu Asp Ile Pro Arg Leu Ile
            340                 345                 350
Glu Lys Thr Cys Asp Leu Tyr Val Gly Gln Asn Thr Ala Ser Pro Asp
        355                 360                 365
Leu Glu Thr Ile Leu Ala Ala Asp Gln Trp Ala Arg Arg Thr Val Leu
    370                 375                 380
Glu Asn Ser Ala Cys Val Ala Thr Arg Pro
385                 390
```

<210> 104
<211> 436
<212> PRT
<213> Mycobacterium tuberculosis

<400> 104

Met Ala Thr Gly Gly Arg Val Val Ile Arg Arg Arg Gly Asp Asn Glu
1               5                   10                  15
Val Val Ala His Asn Asp Glu Val Thr Asn Ser Thr Asp Gly Arg Ala
            20                  25                  30
Asp Gly Arg Leu Arg Val Val Val Leu Gly Ser Thr Gly Ser Ile Gly
        35                  40                  45
Thr Gln Ala Leu Gln Val Ile Ala Asp Asn Pro Asp Arg Phe Glu Val
    50                  55                  60
Val Gly Leu Ala Ala Gly Gly Ala His Leu Asp Thr Leu Leu Arg Gln
65                  70                  75                  80
Arg Ala Gln Thr Gly Val Thr Asn Ile Ala Val Ala Asp Glu His Ala
                85                  90                  95
Ala Gln Arg Val Gly Asp Ile Pro Tyr His Gly Ser Asp Ala Ala Thr
            100                 105                 110
Arg Leu Val Glu Gln Thr Glu Ala Asp Val Val Leu Asn Ala Leu Val
        115                 120                 125
Gly Ala Leu Gly Leu Arg Pro Thr Leu Ala Ala Leu Lys Thr Gly Ala
    130                 135                 140
Arg Leu Ala Leu Ala Asn Lys Glu Ser Leu Val Ala Gly Gly Ser Leu
145                 150                 155                 160
Val Leu Arg Ala Ala Arg Pro Gly Gln Ile Val Pro Val Asp Ser Glu
                165                 170                 175
His Ser Ala Leu Ala Gln Cys Leu Arg Gly Gly Thr Pro Asp Glu Val
            180                 185                 190
Ala Lys Leu Val Leu Thr Ala Ser Gly Gly Pro Phe Arg Gly Trp Ser
        195                 200                 205
Ala Ala Asp Leu Glu His Val Thr Pro Glu Gln Ala Gly Ala His Pro
    210                 215                 220
Thr Trp Ser Met Gly Pro Met Asn Thr Leu Asn Ser Ala Ser Leu Val
225                 230                 235                 240
Asn Lys Gly Leu Glu Val Ile Glu Thr His Leu Leu Phe Gly Ile Pro
                245                 250                 255
Tyr Asp Arg Ile Asp Val Val Val His Pro Gln Ser Ile Ile His Ser
            260                 265                 270
Met Val Thr Phe Ile Asp Gly Ser Thr Ile Ala Gln Ala Ser Pro Pro
        275                 280                 285
Asp Met Lys Leu Pro Ile Ser Leu Ala Leu Gly Trp Pro Arg Arg Val
    290                 295                 300
Ser Gly Ala Ala Ala Ala Cys Asp Phe His Thr Ala Ser Ser Trp Glu
305                 310                 315                 320
Phe Glu Pro Leu Asp Thr Asp Val Phe Pro Ala Val Glu Leu Ala Arg
                325                 330                 335
Gln Ala Gly Val Ala Gly Gly Cys Met Thr Ala Val Tyr Asn Ala Ala
            340                 345                 350
Asn Glu Glu Ala Ala Ala Ala Phe Leu Ala Gly Arg Ile Gly Phe Pro
        355                 360                 365

Ala Ile Val Gly Ile Ile Ala Asp Val Leu His Ala Ala Asp Gln Trp
    370                 375                 380
Ala Val Glu Pro Ala Thr Val Asp Asp Val Leu Asp Ala Gln Arg Trp
385                 390                 395                 400
Ala Arg Glu Arg Ala Gln Arg Ala Val Ser Gly Met Ala Ser Val Ala
                405                 410                 415
Ile Ala Ser Thr Ala Lys Pro Gly Ala Ala Gly Arg His Ala Ser Thr
            420                 425                 430
Leu Glu Arg Ser
        435

<210> 105
<211> 1191

<212> DNA
<213> Pseudomonas aeruginosa

<400> 105

```
atgagtcgac cgcagcggat cagcgtgctc ggcgcgaccg gctcgatcgg cctgagcacc     60
ctggacgtcg tccagcgtca tcccgatcgt tacgaagcct tcgccctgac tggcttcagc    120
cgcctggccg aactcgaggc gctgtgcctc aggcaccgcc ccgtctatgc ggtggtgccg    180
gagcaggccg cggcgattgc cttgcagggc tcgctcgccg cggcgggtat ccgcacccgg    240
gtgctgttcg gcgagcaggc gttgtgcgaa gtggccagcg cgcccgaagt ggacatggta    300
atggcggcca tcgtcggcgc cgccgggctg ccgtcgaccc tggcggccgt cgaggccggc    360
aagcgcgtac tgctggccaa caaggaggcg ctggtgatgt ccggcgcgct gttcatgcag    420
gcggtcaagc gcagcggcgc ggtgctcctg ccgatcgaca gcgagcacaa cgcgatcttc    480
cagtcgctgc cgcgcaatta tgccgatggc ctggagcggg tcggcgtgcg ccggatcctc    540
ttgaccgcct ccggcggccc gttccgcgag acgccgctgg agcaactcgc ttcggtgacg    600
ccggagcagg cttgtgcgca cccgaactgg tcgatggggc gtaagatttc cgtcgactcc    660
gccagcatga tgaacaaggg gctcgaactg atcgaggcgt gctggctgtt cgacgcccag    720
ccgagccagg tcgaggtggt gatccacccg cagagcgtga tccactcgat ggtggactac    780
gtcgacggtt cggtgatcgc ccagctcggc aatccggaca tgcgcacgcc gatttcctat    840
gccatggcct ggccggagcg aatcgattcc ggcgtttcgc cgctggatat gttcgccgtc    900
ggtcgcctgg atttccagcg ccccgacgag cagcgcttcc cctgcctgcg cctggcgagc    960
caggccgcgg aaaccggcgg cagcgccccg gccatgctga atgccgcgaa cgaggtggcc   1020
gtggccgcat ttctcgagcg gcacatccgc ttcagcgaca tcgcggttat catcgaggac   1080
gtgctgaacc gcgaggcggt gaccgcagtc gaatcgctcg atcaggtcct ggctgccgat   1140
cgccgcgcgc gttcggtcgc cgggcaatgg ttgacccggc acgccggcta g            1191
```

<210> 106
<211> 1167
<212> DNA
<213> Zygomonas mobilis

<400> 106

```
atgagtcagc caagaacagt cactgtttta ggggcgaccg gatccattgg tcattcaaca     60
ctggatttaa tcgaacggaa tttagatcgg tatcaggtca tcgctttgac cgccaaccgc    120
aatgtcaaag atctggccga tgcggcgaaa agaacgaatg ccaagcgggc ggttatcgct    180
gacccgtcgc tttataatga tctgaaagag gctttggccg gaagctctgt tgaggcagcc    240
gcgggtgctg atgccttggt cgaagccgcc atgatgggtg ccgattggac aatggcagcc    300
attatcggtt gcgccggtct aaaagcgacg cttgcagcta ttcgcaaggg caaaacggtc    360
gctttagcga ataaggaatc cttagtttca gctggcggat tgatgatcga tgccgtgcgg    420
gaacatggca cgacgcttct ccccgtcgat tccgagcata acgctatttt ccaatgcttc    480
ccgcatcata accgcgacta tgttcgccgg attattatta cggccagcgg aggtcccttc    540
agaacaacgt ctcttgccga aatggcaacg gtcacgccag aacgcgcggt tcagcatccc    600
aactggtcaa tgggtgccaa gatttctatc gattctgcta caatgatgaa taaggggctt    660
gaattgatag aagcctatca tctcttccag attccattag aaaaatttga aattttggtt    720
catcctcagt cagttattca ctccatggtg gaatatttgg atggttctat ccttgcccag    780
atcggtagtc ctgatatgag aacaccgatc ggtcatactt tggcttggcc aaagcggatg    840
gaaacaccag ccgaatcgtt ggattttacc aaattgcgcc agatggattt tgaagcacca    900
gattatgaac gttttccggc attaactttg gcaatggaat ccatcaaatc aggtggggct    960
cgtcctgctg taatgaatgc cgctaatgaa atagctgtgg cggccttcct tgataagaaa   1020

atcggttttc ttgatatcgc taaaattgtc gagaaaacat tagatcatta tacacccgca   1080
accccgtctt ctttggaaga tgtctttgcg atcgacaatg aagcgcggat acaagccgct   1140
gctttaatgg agagtttgcc cgcgtga                                       1167
```

<210> 107
<211> 1161

<212> DNA
<213> Streptomyces griseolosporeus

<400> 107

```
ttggtcattc tcggctcgac cggctcgatc ggcacccagg ccatcgacgt ggtgctccgc      60
aaccccggcc ggttcaaggt ggtcgcgctg tccgcggccg gcggcgcggt ggagctgctc     120
gccgagcagg ccgtcgcact gggcgtgcac accgtcgcgg tggccgaccc ggccgccgag     180
gaagccgctg cgcgaggccc tggcggccaa ggcgcagggc gcccgctgcc gcgggtgctg     240
gcgggcccgg acgcggcgac cgagctggcc gcggcggagt gccactcggt gctgaacggc     300
atcaccggtt cgatcggcct ggccccgacg ctggccgcgc tgcgggccgg ccgggtgctg     360
gtgctggcga acaaggagtc gctgatcgtc ggcggtccgc tggtgaaggc ggtggcgcag     420
cccggccaga tcgtgccggt ggactccgag cacgccgcgc tgttccaggc gctggccggc     480
ggcgcccgcg cggaggtccg caagctggtg gtgaccgcca gcggcggccc gttccgcaac     540
cgcacccgtg agcagctggc ggccgtcacg ccggccgacg cgctggcgca cccgacctgg     600
gcgatgggcc cggtggtgac gatcaactcg gcgaccctgg tgaacaaggg cctggaggtg     660
atcgaggcgc acctgctgta cgacgtgccg ttcgaccgga tcgaggtggt ggtccatccg     720
cagtcggtcg ttcattcgat ggtggaattc gtggacggtt cgacgatggc ccaggccagc     780
ccgccggaca tgcgcatgcc gatcgcgctg ggcctcggct ggccggaccg ggtgccggac     840
gccgcccccg gctgcgactg gaccaaggcc gcgacctggg agttcttccc gctggacaac     900
gaggcgttcc cggcggtcga gctggcccgc gaggtgggta cgctcggcgg gaccgccccg     960
gcggtcttca atgccgccaa cgaggaatgt gtggacgctt tcctgaaggg cgcactgccc    1020
ttcaccggaa tcgtggacac tgtggcgaag gtggtcgccg aacacggcac accgcaatcg    1080
ggaacttcgc tcacggtgga ggacgtactc cacgcggaga gctgggcccg ggcccgggcc    1140
cgcgagctgg cggccggctg a                                              1161
```

<210>108
<211> 1185
<212> DNA
<213> Neisseria meningitidis

<400> 108

```
atgacaccac aagtcctgac catattaggc agtaccggca gcataggcga aagcacgctg      60
gacgttgtct cccgccaccc cgaaaaattc cgcgtattcg cgctggcagg gcataagcag     120
gtcgagaaat tggcggctca atgtcaaacg ttccaccccg aatatgccgt cgttgccgat     180
gccgaacacg ccgcccggct tgaagccctg ttgaaacgcg acggcacggc gactcaggtt     240
ttacacggcg cgcaggcatt ggttgacgtt gcctctgccg acgaagtcag cggtgtcatg     300
tgcgccatcg tcggggcggt ggggctgcct tccgcgctcg cagcggcgca aaaaggcaaa     360
accatttatc tggcgaacaa agagacgctg gtggtttccg gcgcgttgtt tatggaaacc     420
gcccgtgcaa acggcgcggc agtgctgccc gtcgacagcg aacacaacgc cgttttccaa     480
gttttgccgc gcgattacac aggtcgcctg aacgaacacg gcatcgcttc gattatcctg     540
accgcttccg gcggcccgtt tctgaccgcc gatttaaaca cgttcgacag cattacgccc     600
gaccaagcgg tcaaacaccc caattggcgt atgggacgca aaatctccgt cgattccgcc     660
accatgatga acaaaggttt ggagctgatt gaagcgcatt ggctgttcaa ctgtccgccc     720
gacaaactcg aagtcgtcat ccatccgcaa tctgtgatac acagcatggt gcgctaccgc     780
gacggctccg tgttggcgca actgggcaat cccgatatgc gaacgcctat cgcttattgt     840
ttgggtttgc ccgagcgcat cgattcgggt gtcggcgacc tggatttcga cgcattgtcc     900
gcgctgacct tccaaaagcc cgactttgac cgcttcccct gcctgaagct cgcctatgaa     960
gccatgaacg caggcggagc cgcgccctgc gtattgaacg ccgccaacga agccgccgtc    1020
gccgcctttt tggacggaca gattaagttt accgacattg ccaaaaccgt cgcccattgt    1080
ctttcacaag acttttcaga cggcataggc gacatagggg ggctcttggc gcaagatgcc    1140
cggacacgcg cacaagcgcg ggcatttatc ggcacactgc gctga                    1185
```

<210> 109
<211> 1197
<212> DNA
<213> Escherichia coli

<400> 109

```
atgaagcaac tcaccattct gggctcgacc ggctcgattg gttgcagcac gctggacgtg      60
gtgcgccata atcccgaaca cttccgcgta gttgcgctgg tggcaggcaa aaatgtcact     120
cgcatggtag aacagtgcct ggaattctct ccccgctatg ccgtaatgga cgatgaagcg     180
agtgcgaaac ttcttaaaac gatgctacag caacagggta gccgcaccga agtcttaagt     240
gggcaacaag ccgcttgcga tatggcagcg cttgaggatg ttgatcaggt gatggcagcc     300
attgttggcg ctgctgggct gttacctacg cttgctgcga tccgcgcggg taaaaccatt     360
ttgctggcca ataaagaatc actggttacc tgcggacgtc tgtttatgga cgccgtaaag     420
cagagcaaag cgcaattgtt accggtcgat agcgaacata acgccatttt tcagagttta     480
ccgcaaccta tccagcataa tctgggatac gctgaccttg agcaaaatgg cgtggtgtcc     540
atttactta ccgggtctgg tggccctttc cgtgagacgc cattgcgcga tttggcaaca      600
atgacgccgg atcaagcctg ccgtcatccg aactggtcga tggggcgtaa aatttctgtc     660
gattcggcta ccatgatgaa caaaggtctg gaatacattg aagcgcgttg gctgtttaac     720
gccagcgcca gccagatgga agtgctgatt cacccgcagt cagtgattca ctcaatggtg     780
cgctatcagg acggcagtgt tctggcgcag ctgggggaac cggatatgcg tacgccaatt     840
gcccacacca tggcatggcc gaatcgcgtg aactctggcg tgaagccgct cgatttttgc     900
aaactaagtg cgttgacatt tgccgcaccg gattatgatc gttatccatg cctgaaactg     960
gcgatggagg cgttcgaaca aggccaggca gcgacgacag cattgaatgc cgcaaacgaa    1020
atcaccgttg ctgcttttct tgcgcaacaa atccgcttta cggatatcgc tgcgttgaat    1080
ttatccgtac tggaaaaaat ggatatgcgc gaaccacaat gtgtggacga tgtgttatct    1140
gttgatgcga acgcgcgtga agtcgccaga aaagaggtga tgcgtctcgc aagctga       1197
```

<210> 110
<211> 1209
<212> DNA
<213> Synechococcus leopoliensis

<400> 110

```
gtgaaagcag tgacactgct cggttcaacc ggctcgatcg ggacacaaac cctagacatt      60
cttgagcagt atcccgatcg ctttcgcctc gtagggctgg cggctggtcg taatgtggcg     120
ctgttgtcgg agcaaattcg gcggcaccga ccagagattg tggcgattca agatgcagct     180
cagctgtcgg aactgcaagc ggcgatcgca gaccttgata atccgccgct catcctgacc     240
ggtgaggcag gtgtcacgga agtggctcgc tacggtgatg ccgagattgt ggtcactggc     300
attgtcggtt gcgctggtct gctacccacg atcgccgcga tcgaagccgg caaggatatc     360
gcccttgcca acaaagaaac cctgattgca gcaggcccag tggtcctgcc actcctgcaa     420
aagcacggtg tcaccattac gcctgccgac tccgagcact ccgcgatctt tcagtgcatc     480
caagggcttt caacccatgc tgattttcgg cctgctcaag tcgtggcagg gctgcgacgg     540
attctcctga ctgccagtgg cggcgctttt cgggactggc cggtcgaacg gctgtcgcaa     600
gtaactgtcg cagatgcgct caagcatccc aactggtcga tggggcgcaa gattaccgtc     660
gactccgcca ccttgatgaa taaaggcctc gaggtgatcg aagcccacta tctcttcggc     720
ttggattacg actacatcga catcgtcatc catccccaga gcatcatcca ctcgctgatt     780
gagctagaag atacctccgt cttggcgcaa ttgggctggc cggatatgcg actgcccttg     840
ctctacgccc tctcctggcc cgatcgcctc tctactcaat ggtcggcgct cgatctggtc     900
aaagcgggca gcttggagtt ccgggaaccg gatcacgcca aataccccctg catggacttg    960
gcctacgccg ccggtcgcaa aggcggcaca atgccagccg tcttgaatgc ggcgaatgag    1020
caagccgtcg ccctcttcct agaggagcaa attcacttct cggatattcc gcgcctgatt    1080
gaacgtgcct gcgatcgcca ccaaacggag tggcaacagc aaccgagctt ggatgacatt    1140
ttggcctacg acgcttgggc acggcagttt gtgcaagcta gctatcaaag tctggaatcc    1200
gtcgtttag                                                            1209
```

<210> 111
<211> 1221
<212> DNA
<213> Mycobacterium leprae

<400> 111

gtgaacaatc cgatcgaggg gcacgctggc ggccgcctcc gcgtgctggt gttgggaagt 60
actggctcaa ttggcaccca ggcgctggaa gttatcgccg ccaatccgga ccgtttcgag 120
gtagtcgggc tggccgccgg gggcgcgcag ctggacacgc tgctgaggca gcgcgccgcg 180

accggcgtca ccaatatcgc catcgctgac gatcgcgcgg ctcagctggc cggcgacatc 240
ccttaccacg ggaccgatgc ggtcacccgg ctggttgagg agactgaggc tgacgttgtc 300
ctcaatgcgc tggtcggggc attgggtctg cgacccacac tggctgcact gcacacgggc 360
gcgcgattgg cgttggccaa caaggaatcg ctggtagctg gcggttcgct ggtgttggcc 420
gcggcgcagc caggccagat cgtgcccgta gactcggaac actccgcgct ggcgcaatgc 480
ctgcgcggtg gtacccccga cgaagttgct aagttagtgc taaccgcctc cggcgggccg 540
tttcgtggct ggaacgccgg cgacttggag cgcgttacac ccgagcaggc gggcgtccat 600
ccgacttggt caatggggac gatgaacacg ctgaactcag cgtctctggt taacaagggg 660
ctcgagctca tcgaagccaa cctgttgttc ggcattccct acgaccgcat tgaggtggtt 720
gtgcaccctc agtcaattgt tcattcgatg gtgacattca tcgacggctc gacgatcgcc 780
caagccagcc ctccggacat gaagctacct atttctttgg cgttgggctg gccacagcgg 840
gtgggtggcg ctgctcgagc ctgtgctttc actaccgcat ctacctggga attcgagccg 900
ctggacatcg atgtttttcc cgcagtcgag ctggcccggc acgctggaca gatcggcggc 960
tgtatgaccg ccatttacga tgctgctaat gaggaggctg cagaggcctt cctccaaggt 1020
cggatcggct tccccgccat cgtcgcaaca atcgcggatg tgttgcagcg tgccgaccaa 1080
tgggctcccc aatggggtga gggacccgct actgtggatg atgtactcga cgcgcagcgc 1140
tgggcccgtg agcgagcgtt gtgtgcggta gcaacagcga gttctggaaa ggtctctgac 1200
atggtcttag aaaggtccta a 1221

<210> 112
<211> 1218
<212> DNA
<213> Pasteurella multocida

<400> 112

atgagtatta gttattttat gaaaaagatc gttattttag gttcaactgg atcgattggt 60
accagtactt tatccgtgat tacacataat cctgataagt accaagtgtt tgcgttagtt 120
ggtggacgta atgtagagct aatgtttcaa caatgtttga cattccaacc gtcgtttgct 180
gcgttagatg acgatgtcgc agccaaaatg ttggcagaga aactgaaagc ccaccaaagc 240
caaacaacag tcttagcagg acagcaagcc atttgtgagt tagcggcaca tcctgaagca 300
gatatggtaa tggctgcgat tgtgggggcg gcgggattat tgcctacttt gtctgcggtg 360
aaagctggaa aacgtgtact attagcaaat aaagaagcct tggtaacttg cgggcaatta 420
tttattgatg cagtgcgtga atctcaagca caattgttac cagtagatag tgaacataat 480
gcgatttcc aatcccttcc gcctgaagcg caaagacaaa ttgggttttg cccgctttct 540
gaattaggga tcagtaagat tgtgttaacg ggatccggtg gtccattccg ttataccccct 600
ctggagcaat ttgaacagat caccccagca caagcagttg cgcatcctaa ttggtcaatg 660
gggaaaaaga tctctgtcga ttccgctacc atgatgaata aagggttgga atatattgaa 720
gcacgctggt tatttaatgc ctcggcagaa gaaatggaag ttattattca tcctcaatcc 780
attattcatt ctatggtacg ttacatcgat gggtccgtga ttgctcaaat ggggaatcct 840
gatatgcgta caccgattgc ggaaaccatg gcatatccaa gtcggaccgt tgctggcgtt 900
gagcccttgg atttttacca actgaatgga ttaaccttta ttgagccaga ctatcaacgt 960
tatccatcca tggatcttgc ttatgctgct ggacgagctg gaggcacaac cacgacagca 1020
atgaatgcag cgaatgaaat cgcggtagcg tctttcttag acaataagat taaattcaca 1080
gatattgcgc gactaaatca gttagtcgtg agcaaattgc aaccacaaaa aattcattgc 1140
atagaagatg tacttgaggt agataaaaag gcaagggaat tatctcagtc aatcatttta 1200
agtttttcac atccgtaa 1218

<210> 113
<211> 1434
<212> DNA
<213> Arabidopsis thaliana

<400> 113

```
atgatgacat taaactcact atctccagct gaatccaaag ctatttcttt cttggatacc    60
tccaggttca atccaatccc taaactctca ggtgggttta gtttgaggag gaggaatcaa   120
gggagaggtt ttggaaaagg tgttaagtgt tcagtgaaag tgcagcagca acaacaacct   180
cctccagcat ggcctgggag agctgtccct gaggcgcctc gtcaatcttg ggatggacca   240
aaacccatct ctatcgttgg atctactggt tctattggca ctcagacatt ggatattgtg   300
gctgagaatc ctgacaaatt cagagttgtg gctctagctg ctggttcgaa tgttactcta   360
cttgctgatc aggtaaggag atttaagcct gcattggttg ctgttagaaa cgagtcactg   420
attaatgagc ttaaagaggc tttagctgat ttggactata aactcgagat tattccagga   480
gagcaaggag tgattgaggt tgcccgacat cctgaagctg taaccgttgt taccggaata   540
gtaggttgtg cgggactaaa gcctacggtt gctgcaattg aagcaggaaa ggacattgct   600
cttgcaaaca aagagacatt aatcgcaggt ggtcctttcg tgcttccgct tgccaacaaa   660
cataatgtaa agattcttcc ggcagattca gaacattctg ccatatttca gtgtattcaa   720
ggtttgcctg aaggcgctct gcgcaagata atcttgactg catctggtgg agcttttagg   780
gattggcctg tcgaaaagct aaaggaagtt aaagtagcgg atgcgttgaa gcatccaaac   840
tggaacatgg gaaagaaaat cactgtggac tctgctacgc ttttcaacaa gggtcttgag   900
gtcattgaag cgcattattt gtttggagct gagtatgacg atatagagat tgtcattcat   960
ccgcaaagta tcatacattc catgattgaa acacaggatt catctgtgct tgctcaattg  1020
ggttggcctg atatgcgttt accgattctc tacaccatgt catggcccga tagagttcct  1080
tgttctgaag taacttggcc aagacttgac ctttgcaaac tcggttcatt gactttcaag  1140
aaaccagaca atgtgaaata cccatccatg gatcttgctt atgctgctgg acgagctgga  1200
ggcacaatga ctggagttct cagcgccgcc aatgagaaag ctgttgaaat gttcattgat  1260
gaaaagataa gctatttgga tatcttcaag gttgtggaat taacatgcga taaacatcga  1320
aacgagttgg taacatcacc gtctcttgaa gagattgttc actatgactt gtgggcacgt  1380
gaatatgccg cgaatgtgca gctttcttct ggtgctaggc cagttcatgc atga         1434
```

<210> 114
<211> 1071
<212> DNA
<213> Campylobacter jejuni

<400> 114

```
atgatacttt ttggaagtac gggcagtata ggagtaaatg ctcttaaact tgctgcttta    60
aaaaacattc ccatttctgc tttagcttgt ggggataaca tcgctctttt aaatgagcaa   120
atcgcaaggt ttaaacccaa atttgtttcc ataaaagatt caaaaaataa gcatttagtt   180
aaacacgata gagtttttat agggcaagaa ggtttagagc aaattttaac agaatgtcaa   240
gataagcttt tactcaatgc cattgtaggt tttgcaggac ttaaaagcac tttaaaggct   300
aaagagcttg gcaaaaacat agctttagct aacaaagaaa gtcttgtagt agctgggagt   360
tttttgaaag gggctaaatt tttacccgtt gatagtgagc atgcagcttt aaaattttta   420
ctcgaaggta aaaaaaatat agcaaaactt tatatcacag caagtggtgg agctttttat   480
aggtataaaa tcaaagattt aaatcaagtc agtgtcaaag atgctttaaa acatcctaat   540
tggaacatgg gagcaaagat cactatagat agtgcgacta tggcaaataa gctttttgag   600
attatagagg cttatcattt atatgatttt aaagaaattg atgctttaat agaaccaaga   660
tctttagtgc atgcaatgtg tgaatttaaa aatggagcta gcacggcgta tttttcaaaa   720
gcagatatga aactagctat ttcagatgct atatttgaaa aacaagatac gcctatttta   780
gaggctgttg attttagcaa aatgcctgct ttaaaatttc atccaatcag cacaaaaaaa   840
tatcctattt ttaagcttaa aaatacattt ttaaaagagc caaatttagg tgttatcatc   900
aatgctgcta atgaagttgg tgtttataat tttttagaaa ataaaagtgg atttttagac   960
attgctaaat gcatttttaa agcccttgat cattttggag tacctaaaat ttcaagcata  1020
gaagaagttt ttgagtatga ttttaaaaca agagagtatt taaggagtta a           1071
```

<210> 115
<211> 1467
<212> DNA

<213> Plasmodium falciparum

<400> 115

```
atgaagaaat atatttatat atattttttc ttcatcacaa taactattaa tgatttagta      60
ataaataata catcaaaatg tgtttccatt gaaagaagaa aaaataacgc atatataaat     120
tatggtatag gatataatgg accagataat aaaataacaa agagtagaag atgtaaaaga     180
ataaagttat gcaaaaagga tttaatagat attggtgcaa taaagaaacc aattaatgta     240
gcaatttttg gaagtactgg tagtataggt acgaatgctt taaatataat aagggagtgt     300
aataaaattg aaaatgtttt taatgttaaa gcattgtatg tgaataagag tgtgaatgaa     360
ttatatgaac aagctagaga atttttacca gaatatttgt gtatacatga taaaagtgta     420
tatgaagaat taaaagaact ggtaaaaaat ataaaagatt ataaacctat aatattgtgt     480
ggtgatgaag ggatgaaaga aatatgtagt agtaatagta tagataaaat agttattggt     540
attgattctt ttcaaggatt atattctact atgtatgcaa ttatgaataa taaaatagtt     600
gcgttagcta ataaagaatc cattgtctct gctggtttct ttttaaagaa attattaaat     660
attcataaaa atgcaaagat aatacctgtt gattcagaac atagtgctat atttcaatgt     720
ttagataata ataaggtatt aaaaacaaaa tgtttacaag acaatttttc taaaattaac     780

aatataaata aaatattttt atgttcatct ggaggtccat ttcaaaattt aactatggac     840
gaattaaaaa atgtaacatc agaaaatgct ttaaagcatc ctaaatggaa aatgggtaag     900
aaaataacta tagattctgc aactatgatg aataaaggtt tagaggttat agaaacccat     960
tttttatttg atgtagatta taatgatata gaagttatag tacataaaga atgcattata    1020
cattcttgtg ttgaatttat agacaaatca gtaataagtc aaatgtatta tccagatatg    1080
caaataccca tattatattc tttaacatgg cctgatagaa taaaaacaaa tttaaaacct    1140
ttagatttgg ctcaggtttc aactcttaca tttcataaac cttctttaga acatttcccg    1200
tgtattaaat tagcttatca agcaggtata aaaggaaact tttatccaac tgtactaaat    1260
gcgtcaaatg aaatagctaa caacttattt ttgaataata aaattaaata ttttgatatt    1320
tcctctataa tatcgcaagt tcttgaatct ttcaattctc aaaaggtttc ggaaaatagt    1380
gaagatttaa tgaagcaaat tctacaaata cattcttggg ccaaagataa agctaccgat    1440
atatacaaca aacataattc ttcatag                                        1467
```

<210> 116
<211> 388
<212> PRT
<213> Zymononas mobilis

<400> 116

Met Ser Gln Pro Arg Thr Val Thr Val Leu Gly Ala Thr Gly Ser Ile
1 5 10 15
Gly His Ser Thr Leu Asp Leu Ile Glu Arg Asn Leu Asp Arg Tyr Gln
20 25 30
Val Ile Ala Leu Thr Ala Asn Arg Asn Val Lys Asp Leu Ala Asp Ala
35 40 45
Ala Lys Arg Thr Asn Ala Lys Arg Ala Val Ile Ala Asp Pro Ser Leu
50 55 60
Tyr Asn Asp Leu Lys Glu Ala Leu Ala Gly Ser Ser Val Glu Ala Ala
65 70 75 80
Ala Gly Ala Asp Ala Leu Val Glu Ala Ala Met Met Gly Ala Asp Trp
85 90 95
Thr Met Ala Ala Ile Ile Gly Cys Ala Gly Leu Lys Ala Thr Leu Ala
100 105 110
Ala Ile Arg Lys Gly Lys Thr Val Ala Leu Ala Asn Lys Glu Ser Leu
115 120 125
Val Ser Ala Gly Gly Leu Met Ile Asp Ala Val Arg Glu His Gly Thr
130 135 140
Thr Leu Leu Pro Val Asp Ser Glu His Asn Ala Ile Phe Gln Cys Phe
145 150 155 160
Pro His His Asn Arg Asp Tyr Val Arg Arg Ile Ile Ile Thr Ala Ser
165 170 175
Gly Gly Pro Phe Arg Thr Thr Ser Leu Ala Glu Met Ala Thr Val Thr
180 185 190
Pro Glu Arg Ala Val Gln His Pro Asn Trp Ser Met Gly Ala Lys Ile
195 200 205
Ser Ile Asp Ser Ala Thr Met Met Asn Lys Gly Leu Glu Leu Ile Glu
210 215 220
Ala Tyr His Leu Phe Gln Ile Pro Leu Glu Lys Phe Glu Ile Leu Val
225 230 235 240
His Pro Gln Ser Val Ile His Ser Met Val Glu Tyr Leu Asp Gly Ser
245 250 255
Ile Leu Ala Gln Ile Gly Ser Pro Asp Met Arg Thr Pro Ile Gly His
260 265 270
Thr Leu Ala Trp Pro Lys Arg Met Glu Thr Pro Ala Glu Ser Leu Asp
275 280 285
Phe Thr Lys Leu Arg Gln Met Asp Phe Glu Ala Pro Asp Tyr Glu Arg
290 295 300
Phe Pro Ala Leu Thr Leu Ala Met Glu Ser Ile Lys Ser Gly Gly Ala
305 310 315 320
Arg Pro Ala Val Met Asn Ala Ala Asn Glu Ile Ala Val Ala Ala Phe
325 330 335

Leu Asp Lys Lys Ile Gly Phe Leu Asp Ile Ala Lys Ile Val Glu Lys
340 345 350
Thr Leu Asp His Tyr Thr Pro Ala Thr Pro Ser Ser Leu Glu Asp Val
355 360 365
Phe Ala Ile Asp Asn Glu Ala Arg Ile Gln Ala Ala Ala Leu Met Glu
370 375 380
Ser Leu Pro Ala
385

<210> 117
<211> 396
<212> PRT
<213> Pseudomonas aeruginosa

<400> 117

Met Ser Arg Pro Gln Arg Ile Ser Val Leu Gly Ala Thr Gly Ser Ile
1 5 10 15
Gly Leu Ser Thr Leu Asp Val Val Gln Arg His Pro Asp Arg Tyr Glu
20 25 30
Ala Phe Ala Leu Thr Gly Phe Ser Arg Leu Ala Glu Leu Glu Ala Leu
35 40 45
Cys Leu Arg His Arg Pro Val Tyr Ala Val Val Pro Glu Gln Ala Ala
50 55 60
Ala Ile Ala Leu Gln Gly Ser Leu Ala Ala Ala Gly Ile Arg Thr Arg
65 70 75 80
Val Leu Phe Gly Glu Gln Ala Leu Cys Glu Val Ala Ser Ala Pro Glu
85 90 95
Val Asp Met Val Met Ala Ala Ile Val Gly Ala Ala Gly Leu Pro Ser
100 105 110
Thr Leu Ala Ala Val Glu Ala Gly Lys Arg Val Leu Leu Ala Asn Lys
115 120 125
Glu Ala Leu Val Met Ser Gly Ala Leu Phe Met Gln Ala Val Lys Arg
130 135 140
Ser Gly Ala Val Leu Leu Pro Ile Asp Ser Glu His Asn Ala Ile Phe
145 150 155 160
Gln Ser Leu Pro Arg Asn Tyr Ala Asp Gly Leu Glu Arg Val Gly Val
165 170 175
Arg Arg Ile Leu Leu Thr Ala Ser Gly Gly Pro Phe Arg Glu Thr Pro
180 185 190
Leu Glu Gln Leu Ala Ser Val Thr Pro Glu Gln Ala Cys Ala His Pro
195 200 205
Asn Trp Ser Met Gly Arg Lys Ile Ser Val Asp Ser Ala Ser Met Met
210 215 220
Asn Lys Gly Leu Glu Leu Ile Glu Ala Cys Trp Leu Phe Asp Ala Gln
225 230 235 240
Pro Ser Gln Val Glu Val Val Ile His Pro Gln Ser Val Ile His Ser
245 250 255
Met Val Asp Tyr Val Asp Gly Ser Val Ile Ala Gln Leu Gly Asn Pro
260 265 270
Asp Met Arg Thr Pro Ile Ser Tyr Ala Met Ala Trp Pro Glu Arg Ile
275 280 285
Asp Ser Gly Val Ser Pro Leu Asp Met Phe Ala Val Gly Arg Leu Asp
290 295 300
Phe Gln Arg Pro Asp Glu Gln Arg Phe Pro Cys Leu Arg Leu Ala Ser
305 310 315 320
Gln Ala Ala Glu Thr Gly Gly Ser Ala Pro Ala Met Leu Asn Ala Ala
325 330 335
Asn Glu Val Ala Val Ala Ala Phe Leu Glu Arg His Ile Arg Phe Ser
340 345 350
Asp Ile Ala Val Ile Ile Glu Asp Val Leu Asn Arg Glu Ala Val Thr
355 360 365

Ala Val Glu Ser Leu Asp Gln Val Leu Ala Ala Asp Arg Arg Ala Arg
370 375 380
Ser Val Ala Gly Gln Trp Leu Thr Arg His Ala Gly
385 390 395

<210> 118
<211> 398
<212> PRT
<213> Escherichia coli

<400> 118

```
Met Lys Gln Leu Thr Ile Leu Gly Ser Thr Gly Ser Ile Gly Cys Ser
1               5                   10                  15
Thr Leu Asp Val Val Arg His Asn Pro Glu His Phe Arg Val Val Ala
            20                  25                  30
Leu Val Ala Gly Lys Asn Val Thr Arg Met Val Glu Gln Cys Leu Glu
        35                  40                  45
Phe Ser Pro Arg Tyr Ala Val Met Asp Asp Glu Ala Ser Ala Lys Leu
    50                  55                  60
Leu Lys Thr Met Leu Gln Gln Gln Gly Ser Arg Thr Glu Val Leu Ser
65                  70                  75                  80
Gly Gln Gln Ala Ala Cys Asp Met Ala Ala Leu Glu Asp Val Asp Gln
                85                  90                  95
Val Met Ala Ala Ile Val Gly Ala Ala Gly Leu Leu Pro Thr Leu Ala
            100                 105                 110
Ala Ile Arg Ala Gly Lys Thr Ile Leu Leu Ala Asn Lys Glu Ser Leu
        115                 120                 125
Val Thr Cys Gly Arg Leu Phe Met Asp Ala Val Lys Gln Ser Lys Ala
    130                 135                 140
Gln Leu Leu Pro Val Asp Ser Glu His Asn Ala Ile Phe Gln Ser Leu
145                 150                 155                 160
Pro Gln Pro Ile Gln His Asn Leu Gly Tyr Ala Asp Leu Glu Gln Asn
                165                 170                 175
Gly Val Val Ser Ile Leu Leu Thr Gly Ser Gly Gly Pro Phe Arg Glu
            180                 185                 190
Thr Pro Leu Arg Asp Leu Ala Thr Met Thr Pro Asp Gln Ala Cys Arg
        195                 200                 205
His Pro Asn Trp Ser Met Gly Arg Lys Ile Ser Val Asp Ser Ala Thr
    210                 215                 220
Met Met Asn Lys Gly Leu Glu Tyr Ile Glu Ala Arg Trp Leu Phe Asn
225                 230                 235                 240
Ala Ser Ala Ser Gln Met Glu Val Leu Ile His Pro Gln Ser Val Ile
                245                 250                 255
His Ser Met Val Arg Tyr Gln Asp Gly Ser Val Leu Ala Gln Leu Gly
            260                 265                 270
Glu Pro Asp Met Arg Thr Pro Ile Ala His Thr Met Ala Trp Pro Asn
        275                 280                 285
Arg Val Asn Ser Gly Val Lys Pro Leu Asp Phe Cys Lys Leu Ser Ala
    290                 295                 300
Leu Thr Phe Ala Ala Pro Asp Tyr Asp Arg Tyr Pro Cys Leu Lys Leu
305                 310                 315                 320
Ala Met Glu Ala Phe Glu Gln Gly Gln Ala Ala Thr Thr Ala Leu Asn
                325                 330                 335
Ala Ala Asn Glu Ile Thr Val Ala Ala Phe Leu Ala Gln Gln Ile Arg
            340                 345                 350
Phe Thr Asp Ile Ala Ala Leu Asn Leu Ser Val Leu Glu Lys Met Asp
        355                 360                 365
Met Arg Glu Pro Gln Cys Val Asp Asp Val Leu Ser Val Asp Ala Asn
    370                 375                 380
Ala Arg Glu Val Ala Arg Lys Glu Val Met Arg Leu Ala Ser
385                 390                 395
```

<210> 119
<211> 394
<212> PRT
<213> Neisseria meningitidis

<400> 119

Met Thr Pro Gln Val Leu Thr Ile Leu Gly Ser Thr Gly Ser Ile Gly
1               5                   10                  15
Glu Ser Thr Leu Asp Val Val Ser Arg His Pro Glu Lys Phe Arg Val
            20                  25                  30
Phe Ala Leu Ala Gly His Lys Gln Val Glu Lys Leu Ala Ala Gln Cys
        35                  40                  45
Gln Thr Phe His Pro Glu Tyr Ala Val Val Ala Asp Ala Glu His Ala
    50                  55                  60
Ala Arg Leu Glu Ala Leu Leu Lys Arg Asp Gly Thr Ala Thr Gln Val
65                  70                  75                  80
Leu His Gly Ala Gln Ala Leu Val Asp Val Ala Ser Ala Asp Glu Val
                85                  90                  95
Ser Gly Val Met Cys Ala Ile Val Gly Ala Val Gly Leu Pro Ser Ala
            100                 105                 110
Leu Ala Ala Ala Gln Lys Gly Lys Thr Ile Tyr Leu Ala Asn Lys Glu
        115                 120                 125
Thr Leu Val Val Ser Gly Ala Leu Phe Met Glu Thr Ala Arg Ala Asn
    130                 135                 140
Gly Ala Ala Val Leu Pro Val Asp Ser Glu His Asn Ala Val Phe Gln
145                 150                 155                 160
Val Leu Pro Arg Asp Tyr Thr Gly Arg Leu Asn Glu His Gly Ile Ala
                165                 170                 175
Ser Ile Ile Leu Thr Ala Ser Gly Gly Pro Phe Leu Thr Ala Asp Leu
            180                 185                 190
Asn Thr Phe Asp Ser Ile Thr Pro Asp Gln Ala Val Lys His Pro Asn
        195                 200                 205
Trp Arg Met Gly Arg Lys Ile Ser Val Asp Ser Ala Thr Met Met Asn
    210                 215                 220
Lys Gly Leu Glu Leu Ile Glu Ala His Trp Leu Phe Asn Cys Pro Pro
225                 230                 235                 240
Asp Lys Leu Glu Val Val Ile His Pro Gln Ser Val Ile His Ser Met
                245                 250                 255
Val Arg Tyr Arg Asp Gly Ser Val Leu Ala Gln Leu Gly Asn Pro Asp
            260                 265                 270
Met Arg Thr Pro Ile Ala Tyr Cys Leu Gly Leu Pro Glu Arg Ile Asp
        275                 280                 285
Ser Gly Val Gly Asp Leu Asp Phe Asp Ala Leu Ser Ala Leu Thr Phe
    290                 295                 300
Gln Lys Pro Asp Phe Asp Arg Phe Pro Cys Leu Lys Leu Ala Tyr Glu
305                 310                 315                 320
Ala Met Asn Ala Gly Gly Ala Ala Pro Cys Val Leu Asn Ala Ala Asn
                325                 330                 335
Glu Ala Ala Val Ala Ala Phe Leu Asp Gly Gln Ile Lys Phe Thr Asp
            340                 345                 350
Ile Ala Lys Thr Val Ala His Cys Leu Ser Gln Asp Phe Ser Asp Gly
        355                 360                 365
Ile Gly Asp Ile Gly Gly Leu Leu Ala Gln Asp Ala Arg Thr Arg Ala
    370                 375                 380
Gln Ala Arg Ala Phe Ile Gly Thr Leu Arg
385                 390

<210> 120
<211> 397
<212> PRT
<213> Haemophilus influenzae

<400> 120

```
Met Gln Lys Gln Asn Ile Val Ile Leu Gly Ser Thr Gly Ser Ile Gly
1               5                   10                  15
Lys Ser Thr Leu Ser Val Ile Glu Asn Asn Pro Gln Lys Tyr His Ala
            20                  25                  30
Phe Ala Leu Val Gly Gly Lys Asn Val Glu Ala Met Phe Glu Gln Cys
        35                  40                  45
Ile Lys Phe Arg Pro His Phe Ala Ala Leu Asp Asp Val Asn Ala Ala
    50                  55                  60
Lys Ile Leu Arg Glu Lys Leu Ile Ala His His Ile Pro Thr Glu Val
65                  70                  75                  80
Leu Ala Gly Arg Arg Ala Ile Cys Glu Leu Ala Ala His Pro Asp Ala
                85                  90                  95
Asp Gln Ile Met Ala Ser Ile Val Gly Ala Ala Gly Leu Leu Pro Thr
            100                 105                 110
Leu Ser Ala Val Lys Ala Gly Lys Arg Val Leu Leu Ala Asn Lys Glu
        115                 120                 125
Ser Leu Val Thr Cys Gly Gln Leu Phe Ile Asp Ala Val Lys Asn Tyr
    130                 135                 140
Gly Ser Lys Leu Leu Pro Val Asp Ser Glu His Asn Ala Ile Phe Gln
145                 150                 155                 160
Ser Leu Pro Pro Glu Ala Gln Glu Lys Ile Gly Phe Cys Pro Leu Ser
                165                 170                 175
Glu Leu Gly Val Ser Lys Ile Ile Leu Thr Gly Ser Gly Gly Pro Phe
            180                 185                 190
Arg Tyr Thr Pro Leu Glu Gln Phe Thr Asn Ile Thr Pro Glu Gln Ala
        195                 200                 205
Val Ala His Pro Asn Trp Ser Met Gly Lys Lys Ile Ser Val Asp Ser
    210                 215                 220
Ala Thr Met Met Asn Lys Gly Leu Glu Tyr Ile Glu Ala Arg Trp Leu
225                 230                 235                 240
Phe Asn Ala Ser Ala Glu Glu Met Glu Val Ile Ile His Pro Gln Ser
                245                 250                 255
Ile Ile His Ser Met Val Arg Tyr Val Asp Gly Ser Val Ile Thr Gln
            260                 265                 270
Met Gly Asn Pro Asp Met Arg Thr Pro Ile Ala Glu Thr Met Ala Tyr
        275                 280                 285
Pro His Arg Thr Phe Ala Gly Val Glu Pro Leu Asp Phe Phe Lys Ile
    290                 295                 300
Lys Glu Leu Thr Phe Ile Glu Pro Asp Phe Asn Arg Tyr Pro Asn Leu
305                 310                 315                 320
Lys Leu Ala Ile Asp Ala Phe Ala Ala Gly Gln Tyr Ala Thr Thr Ala
                325                 330                 335
Met Asn Ala Ala Asn Glu Ile Ala Val Gln Ala Phe Leu Asp Arg Gln
            340                 345                 350
Ile Gly Phe Met Asp Ile Ala Lys Ile Asn Ser Lys Thr Ile Glu Arg
        355                 360                 365
Ile Ser Pro Tyr Thr Ile Gln Asn Ile Asp Asp Val Leu Glu Ile Asp
    370                 375                 380
Ala Gln Ala Arg Glu Ile Ala Lys Thr Leu Leu Arg Glu
385                 390                 395
```

<210> 121
<211> 394
<212> PRT
<213> Synechocystis sp. PCC 6803

<400> 121

```
Met Val Lys Arg Ile Ser Ile Leu Gly Ser Thr Gly Ser Ile Gly Thr
```

```
1               5                   10                  15
Gln Thr Leu Asp Ile Val Thr His His Pro Asp Ala Phe Gln Val Val
            20                  25                  30
Gly Leu Ala Ala Gly Gly Asn Val Ala Leu Leu Ala Gln Gln Val Ala
        35                  40                  45
Glu Phe Arg Pro Glu Ile Val Ala Ile Arg Gln Ala Glu Lys Leu Glu
    50                  55                  60
Asp Leu Lys Ala Ala Val Ala Glu Leu Thr Asp Tyr Gln Pro Met Tyr
65                  70                  75                  80
Val Val Gly Glu Glu Gly Val Val Glu Val Ala Arg Tyr Gly Asp Ala
                85                  90                  95
Glu Ser Val Val Thr Gly Ile Val Gly Cys Ala Gly Leu Leu Pro Thr
            100                 105                 110
Met Ala Ala Ile Ala Ala Gly Lys Asp Ile Ala Leu Ala Asn Lys Glu
        115                 120                 125
Thr Leu Ile Ala Gly Ala Pro Val Val Leu Pro Leu Val Glu Lys Met
    130                 135                 140
Gly Val Lys Leu Leu Pro Ala Asp Ser Glu His Ser Ala Ile Phe Gln
145                 150                 155                 160
Cys Leu Gln Gly Val Pro Glu Gly Gly Leu Arg Arg Ile Ile Leu Thr
                165                 170                 175
Ala Ser Gly Gly Ala Phe Arg Asp Leu Pro Val Glu Arg Leu Pro Phe
            180                 185                 190
Val Thr Val Gln Asp Ala Leu Lys His Pro Asn Trp Ser Met Gly Gln
        195                 200                 205
Lys Ile Thr Ile Asp Ser Ala Thr Leu Met Asn Lys Gly Leu Glu Val
    210                 215                 220
Ile Glu Ala His Tyr Leu Phe Gly Leu Asp Tyr Asp His Ile Asp Ile
225                 230                 235                 240
Val Ile His Pro Gln Ser Ile Ile His Ser Leu Ile Glu Val Gln Asp
                245                 250                 255
Thr Ser Val Leu Ala Gln Leu Gly Trp Pro Asp Met Arg Leu Pro Leu
            260                 265                 270
Leu Tyr Ala Leu Ser Trp Pro Glu Arg Ile Tyr Thr Asp Trp Glu Pro
        275                 280                 285
Leu Asp Leu Val Lys Ala Gly Ser Leu Ser Phe Arg Glu Pro Asp His
    290                 295                 300
Asp Lys Tyr Pro Cys Met Gln Leu Ala Tyr Gly Ala Gly Arg Ala Gly
305                 310                 315                 320
Gly Ala Met Pro Ala Val Leu Asn Ala Ala Asn Glu Gln Ala Val Ala
                325                 330                 335
Leu Phe Leu Gln Glu Lys Ile Ser Phe Leu Asp Ile Pro Arg Leu Ile
            340                 345                 350
Glu Lys Thr Cys Asp Leu Tyr Val Gly Gln Asn Thr Ala Ser Pro Asp
        355                 360                 365
Leu Glu Thr Ile Leu Ala Ala Asp Gln Trp Ala Arg Arg Thr Val Leu
    370                 375                 380
Glu Asn Ser Ala Cys Val Ala Thr Arg Pro
385                 390
```

<210> 122
<211> 405
<212> PRT
<213> Pasteurella multocida

<400> 122

```
Met Ser Ile Ser Tyr Phe Met Lys Lys Ile Val Ile Leu Gly Ser Thr
 1               5                  10                  15
Gly Ser Ile Gly Thr Ser Thr Leu Ser Val Ile Thr His Asn Pro Asp
            20                  25                  30
Lys Tyr Gln Val Phe Ala Leu Val Gly Gly Arg Asn Val Glu Leu Met
        35                  40                  45
Phe Gln Gln Cys Leu Thr Phe Gln Pro Ser Phe Ala Ala Leu Asp Asp
    50                  55                  60
Asp Val Ala Ala Lys Met Leu Ala Glu Lys Leu Lys Ala His Gln Ser
65                  70                  75                  80
Gln Thr Thr Val Leu Ala Gly Gln Gln Ala Ile Cys Glu Leu Ala Ala
                85                  90                  95
His Pro Glu Ala Asp Met Val Met Ala Ala Ile Val Gly Ala Ala Gly
            100                 105                 110
Leu Leu Pro Thr Leu Ser Ala Val Lys Ala Gly Lys Arg Val Leu Leu
        115                 120                 125
Ala Asn Lys Glu Ala Leu Val Thr Cys Gly Gln Leu Phe Ile Asp Ala
    130                 135                 140
Val Arg Glu Ser Gln Ala Gln Leu Leu Pro Val Asp Ser Glu His Asn
145                 150                 155                 160
Ala Ile Phe Gln Ser Leu Pro Pro Glu Ala Gln Arg Gln Ile Gly Phe
                165                 170                 175
Cys Pro Leu Ser Glu Leu Gly Ile Ser Lys Ile Val Leu Thr Gly Ser
            180                 185                 190
Gly Gly Pro Phe Arg Tyr Thr Pro Leu Glu Gln Phe Glu Gln Ile Thr
        195                 200                 205
Pro Ala Gln Ala Val Ala His Pro Asn Trp Ser Met Gly Lys Lys Ile
    210                 215                 220
Ser Val Asp Ser Ala Thr Met Met Asn Lys Gly Leu Glu Tyr Ile Glu
225                 230                 235                 240
Ala Arg Trp Leu Phe Asn Ala Ser Ala Glu Glu Met Glu Val Ile Ile
                245                 250                 255
His Pro Gln Ser Ile Ile His Ser Met Val Arg Tyr Ile Asp Gly Ser
            260                 265                 270
Val Ile Ala Gln Met Gly Asn Pro Asp Met Arg Thr Pro Ile Ala Glu
        275                 280                 285
Thr Met Ala Tyr Pro Ser Arg Thr Val Ala Gly Val Glu Pro Leu Asp
    290                 295                 300
Phe Tyr Gln Leu Asn Gly Leu Thr Phe Ile Glu Pro Asp Tyr Gln Arg
305                 310                 315                 320
Tyr Pro Cys Leu Lys Leu Ala Ile Asp Ala Phe Ser Ala Gly Gln Tyr
                325                 330                 335
Ala Thr Thr Ala Met Asn Ala Ala Asn Glu Ile Ala Val Ala Ser Phe
            340                 345                 350
Leu Asp Asn Lys Ile Lys Phe Thr Asp Ile Ala Arg Leu Asn Gln Leu
        355                 360                 365
Val Val Ser Lys Leu Gln Pro Gln Lys Ile His Cys Ile Glu Asp Val
    370                 375                 380
Leu Glu Val Asp Lys Lys Ala Arg Glu Leu Ser Gln Ser Ile Ile Leu
385                 390                 395                 400
Ser Phe Ser His Pro
                405
```

<210> 123
<211> 402
<212> PRT
<213> Synechococcus leopoliensis

<400> 123

```
Met Lys Ala Val Thr Leu Leu Gly Ser Thr Gly Ser Ile Gly Thr Gln
 1               5                  10                  15
Thr Leu Asp Ile Leu Glu Gln Tyr Pro Asp Arg Phe Arg Leu Val Gly
            20                  25                  30
Leu Ala Ala Gly Arg Asn Val Ala Leu Leu Ser Glu Gln Ile Arg Arg
        35                  40                  45
His Arg Pro Glu Ile Val Ala Ile Gln Asp Ala Ala Gln Leu Ser Glu
    50                  55                  60
Leu Gln Ala Ala Ile Ala Asp Leu Asp Asn Pro Pro Leu Ile Leu Thr
65                  70                  75                  80
Gly Glu Ala Gly Val Thr Glu Val Ala Arg Tyr Gly Asp Ala Glu Ile
                85                  90                  95
Val Val Thr Gly Ile Val Gly Cys Ala Gly Leu Leu Pro Thr Ile Ala
            100                 105                 110
Ala Ile Glu Ala Gly Lys Asp Ile Ala Leu Ala Asn Lys Glu Thr Leu
        115                 120                 125
Ile Ala Ala Gly Pro Val Val Leu Pro Leu Leu Gln Lys His Gly Val
    130                 135                 140
Thr Ile Thr Pro Ala Asp Ser Glu His Ser Ala Ile Phe Gln Cys Ile
145                 150                 155                 160
Gln Gly Leu Ser Thr His Ala Asp Phe Arg Pro Ala Gln Val Val Ala
                165                 170                 175
Gly Leu Arg Arg Ile Leu Leu Thr Ala Ser Gly Gly Ala Phe Arg Asp
            180                 185                 190
Trp Pro Val Glu Arg Leu Ser Gln Val Thr Val Ala Asp Ala Leu Lys
        195                 200                 205
His Pro Asn Trp Ser Met Gly Arg Lys Ile Thr Val Asp Ser Ala Thr
    210                 215                 220
Leu Met Asn Lys Gly Leu Glu Val Ile Glu Ala His Tyr Leu Phe Gly
225                 230                 235                 240
Leu Asp Tyr Asp Tyr Ile Asp Ile Val Ile His Pro Gln Ser Ile Ile
                245                 250                 255
His Ser Leu Ile Glu Leu Glu Asp Thr Ser Val Leu Ala Gln Leu Gly
            260                 265                 270
Trp Pro Asp Met Arg Leu Pro Leu Leu Tyr Ala Leu Ser Trp Pro Asp
        275                 280                 285
Arg Leu Ser Thr Gln Trp Ser Ala Leu Asp Leu Val Lys Ala Gly Ser
    290                 295                 300
Leu Glu Phe Arg Glu Pro Asp His Ala Lys Tyr Pro Cys Met Asp Leu
305                 310                 315                 320
Ala Tyr Ala Ala Gly Arg Lys Gly Gly Thr Met Pro Ala Val Leu Asn
                325                 330                 335
Ala Ala Asn Glu Gln Ala Val Ala Leu Phe Leu Glu Glu Gln Ile His
            340                 345                 350
Phe Ser Asp Ile Pro Arg Leu Ile Glu Arg Ala Cys Asp Arg His Gln
        355                 360                 365
Thr Glu Trp Gln Gln Gln Pro Ser Leu Asp Asp Ile Leu Ala Tyr Asp
    370                 375                 380
Ala Trp Ala Arg Gln Phe Val Gln Ala Ser Tyr Gln Ser Leu Glu Ser
385                 390                 395                 400
Val Val
```

<210> 124
<211> 386
<212> PRT
<213> Streptomyces griseolosporeus

<400> 124

Met Val Ile Leu Gly Ser Thr Gly Ser Ile Gly Thr Gln Ala Ile Asp
1               5                   10                  15
Val Val Leu Arg Asn Pro Gly Arg Phe Lys Val Val Ala Leu Ser Ala
            20                  25                  30
Ala Gly Gly Ala Val Glu Leu Leu Ala Glu Gln Ala Val Ala Leu Gly
        35                  40                  45
Val His Thr Val Ala Val Ala Asp Pro Ala Ala Glu Glu Ala Ala Ala
    50                  55                  60
Arg Gly Pro Gly Gly Gln Gly Ala Gly Arg Pro Leu Pro Arg Val Leu
65                  70                  75                  80
Ala Gly Pro Asp Ala Ala Thr Glu Leu Ala Ala Ala Glu Cys His Ser
                85                  90                  95
Val Leu Asn Gly Ile Thr Gly Ser Ile Gly Leu Ala Pro Thr Leu Ala
            100                 105                 110
Ala Leu Arg Ala Gly Arg Val Leu Val Leu Ala Asn Lys Glu Ser Leu
        115                 120                 125
Ile Val Gly Gly Pro Leu Val Lys Ala Val Ala Gln Pro Gly Gln Ile
    130                 135                 140
Val Pro Val Asp Ser Glu His Ala Ala Leu Phe Gln Ala Leu Ala Gly
145                 150                 155                 160
Gly Ala Arg Ala Glu Val Arg Lys Leu Val Val Thr Ala Ser Gly Gly
                165                 170                 175
Pro Phe Arg Asn Arg Thr Arg Glu Gln Leu Ala Ala Val Thr Pro Ala
            180                 185                 190
Asp Ala Leu Ala His Pro Thr Trp Ala Met Gly Pro Val Val Thr Ile
        195                 200                 205
Asn Ser Ala Thr Leu Val Asn Lys Gly Leu Glu Val Ile Glu Ala His
    210                 215                 220
Leu Leu Tyr Asp Val Pro Phe Asp Arg Ile Glu Val Val Val His Pro
225                 230                 235                 240
Gln Ser Val Val His Ser Met Val Glu Phe Val Asp Gly Ser Thr Met
                245                 250                 255
Ala Gln Ala Ser Pro Pro Asp Met Arg Met Pro Ile Ala Leu Gly Leu
            260                 265                 270
Gly Trp Pro Asp Arg Val Pro Asp Ala Ala Pro Gly Cys Asp Trp Thr
        275                 280                 285
Lys Ala Ala Thr Trp Glu Phe Phe Pro Leu Asp Asn Glu Ala Phe Pro
    290                 295                 300
Ala Val Glu Leu Ala Arg Glu Val Gly Thr Leu Gly Gly Thr Ala Pro
305                 310                 315                 320
Ala Val Phe Asn Ala Ala Asn Glu Glu Cys Val Asp Ala Phe Leu Lys
                325                 330                 335
Gly Ala Leu Pro Phe Thr Gly Ile Val Asp Thr Val Ala Lys Val Val
            340                 345                 350
Ala Glu His Gly Thr Pro Gln Ser Gly Thr Ser Leu Thr Val Glu Asp
        355                 360                 365
Val Leu His Ala Glu Ser Trp Ala Arg Ala Arg Ala Arg Glu Leu Ala
    370                 375                 380
Ala Gly
385

<210> 125
<211> 388
<212> PRT
<213> Bacillus subtilis

<400> 125

Met Lys Asn Ile Cys Leu Leu Gly Ala Thr Gly Ser Ile Gly Glu Gln
1               5                   10                  15
Thr Leu Asp Val Leu Arg Ala His Gln Asp Gln Phe Gln Leu Val Ser
            20                  25                  30
Met Ser Phe Gly Arg Asn Ile Asp Lys Ala Val Pro Met Ile Glu Val
        35                  40                  45
Phe Gln Pro Lys Phe Val Ser Val Gly Asp Leu Asp Thr Tyr His Lys
    50                  55                  60
Leu Lys Gln Met Ser Phe Ser Phe Glu Cys Gln Ile Gly Leu Gly Glu
65                  70                  75                  80
Glu Gly Leu Ile Glu Ala Ala Val Met Glu Glu Val Asp Ile Val Val
                85                  90                  95
Asn Ala Leu Leu Gly Ser Val Gly Leu Ile Pro Thr Leu Lys Ala Ile
            100                 105                 110
Glu Gln Lys Lys Thr Ile Ala Leu Ala Asn Lys Glu Thr Leu Val Thr
        115                 120                 125
Ala Gly His Ile Val Lys Glu His Ala Lys Lys Tyr Asp Val Pro Leu
    130                 135                 140
Leu Pro Val Asp Ser Glu His Ser Ala Ile Phe Gln Ala Leu Gln Gly
145                 150                 155                 160
Glu Gln Ala Lys Asn Ile Glu Arg Leu Ile Ile Thr Ala Ser Gly Gly
                165                 170                 175
Ser Phe Arg Asp Lys Thr Arg Glu Glu Leu Glu Ser Val Thr Val Glu
            180                 185                 190
Asp Ala Leu Lys His Pro Asn Trp Ser Met Gly Ala Lys Ile Thr Ile
        195                 200                 205
Asp Ser Ala Thr Met Met Asn Lys Gly Leu Glu Val Ile Glu Ala His
    210                 215                 220
Trp Leu Phe Asp Ile Pro Tyr Glu Gln Ile Asp Val Val Leu His Lys
225                 230                 235                 240
Glu Ser Ile Ile His Ser Met Val Glu Phe His Asp Lys Ser Val Ile
                245                 250                 255
Ala Gln Leu Gly Thr Pro Asp Met Arg Val Pro Ile Gln Tyr Ala Leu
            260                 265                 270
Thr Tyr Pro Asp Arg Leu Pro Leu Pro Asp Ala Lys Arg Leu Glu Leu
        275                 280                 285
Trp Glu Ile Gly Ser Leu His Phe Glu Lys Ala Asp Phe Asp Arg Phe
    290                 295                 300
Arg Cys Leu Gln Phe Ala Phe Glu Ser Gly Lys Ile Gly Gly Thr Met
305                 310                 315                 320
Pro Thr Val Leu Asn Ala Ala Asn Glu Val Ala Val Ala Ala Phe Leu
                325                 330                 335
Ala Gly Lys Ile Pro Phe Leu Ala Ile Glu Asp Cys Ile Glu Lys Ala
            340                 345                 350
Leu Thr Arg His Gln Leu Leu Lys Lys Pro Ser Trp Arg Thr Phe Lys
        355                 360                 365
Lys Trp Thr Lys Ile Pro Gly Asp Thr Ser Ile Gln Tyr Ser His Lys
    370                 375                 380
Val Val Cys Ser
385

<210> 126
<211> 406
<212> PRT
<213> Mycobacterium leprae

<400> 126

Met Asn Asn Pro Ile Glu Gly His Ala Gly Gly Arg Leu Arg Val Leu
1               5                   10                  15
Val Leu Gly Ser Thr Gly Ser Ile Gly Thr Gln Ala Leu Glu Val Ile
            20                  25                  30
Ala Ala Asn Pro Asp Arg Phe Glu Val Val Gly Leu Ala Ala Gly Gly
        35                  40                  45
Ala Gln Leu Asp Thr Leu Leu Arg Gln Arg Ala Ala Thr Gly Val Thr
    50                  55                  60
Asn Ile Ala Ile Ala Asp Asp Arg Ala Ala Gln Leu Ala Gly Asp Ile
65                  70                  75                  80
Pro Tyr His Gly Thr Asp Ala Val Thr Arg Leu Val Glu Glu Thr Glu
                85                  90                  95
Ala Asp Val Val Leu Asn Ala Leu Val Gly Ala Leu Gly Leu Arg Pro
            100                 105                 110
Thr Leu Ala Ala Leu His Thr Gly Ala Arg Leu Ala Leu Ala Asn Lys
        115                 120                 125
Glu Ser Leu Val Ala Gly Gly Ser Leu Val Leu Ala Ala Ala Gln Pro
    130                 135                 140
Gly Gln Ile Val Pro Val Asp Ser Glu His Ser Ala Leu Ala Gln Cys
145                 150                 155                 160
Leu Arg Gly Gly Thr Pro Asp Glu Val Ala Lys Leu Val Leu Thr Ala
                165                 170                 175
Ser Gly Gly Pro Phe Arg Gly Trp Asn Ala Gly Asp Leu Glu Arg Val
            180                 185                 190
Thr Pro Glu Gln Ala Gly Val His Pro Thr Trp Ser Met Gly Thr Met
        195                 200                 205
Asn Thr Leu Asn Ser Ala Ser Leu Val Asn Lys Gly Leu Glu Leu Ile
    210                 215                 220
Glu Ala Asn Leu Leu Phe Gly Ile Pro Tyr Asp Arg Ile Glu Val Val
225                 230                 235                 240
Val His Pro Gln Ser Ile Val His Ser Met Val Thr Phe Ile Asp Gly
                245                 250                 255
Ser Thr Ile Ala Gln Ala Ser Pro Pro Asp Met Lys Leu Pro Ile Ser
            260                 265                 270
Leu Ala Leu Gly Trp Pro Gln Arg Val Gly Gly Ala Ala Arg Ala Cys
        275                 280                 285
Ala Phe Thr Thr Ala Ser Thr Trp Glu Phe Glu Pro Leu Asp Ile Asp
    290                 295                 300
Val Phe Pro Ala Val Glu Leu Ala Arg His Ala Gly Gln Ile Gly Gly
305                 310                 315                 320
Cys Met Thr Ala Ile Tyr Asp Ala Ala Asn Glu Glu Ala Ala Glu Ala
                325                 330                 335
Phe Leu Gln Gly Arg Ile Gly Phe Pro Ala Ile Val Ala Thr Ile Ala
            340                 345                 350
Asp Val Leu Gln Arg Ala Asp Gln Trp Ala Pro Gln Trp Gly Glu Gly
        355                 360                 365
Pro Ala Thr Val Asp Asp Val Leu Asp Ala Gln Arg Trp Ala Arg Glu
    370                 375                 380
Arg Ala Leu Cys Ala Val Ala Thr Ala Ser Ser Gly Lys Val Ser Asp
385                 390                 395                 400
Met Val Leu Glu Arg Ser
                405

<210> 127
<211> 436
<212> PRT
<213> Mycobacterium tuberculosis

<400> 127

```
Met Ala Thr Gly Gly Arg Val Val Ile Arg Arg Arg Gly Asp Asn Glu
 1               5                  10                  15
Val Val Ala His Asn Asp Glu Val Thr Asn Ser Thr Asp Gly Arg Ala
            20                  25                  30
Asp Gly Arg Leu Arg Val Val Val Leu Gly Ser Thr Gly Ser Ile Gly
        35                  40                  45
Thr Gln Ala Leu Gln Val Ile Ala Asp Asn Pro Asp Arg Phe Glu Val
    50                  55                  60
Val Gly Leu Ala Ala Gly Gly Ala His Leu Asp Thr Leu Leu Arg Gln
65                  70                  75                  80
Arg Ala Gln Thr Gly Val Thr Asn Ile Ala Val Ala Asp Glu His Ala
                85                  90                  95
Ala Gln Arg Val Gly Asp Ile Pro Tyr His Gly Ser Asp Ala Ala Thr
            100                 105                 110
Arg Leu Val Glu Gln Thr Glu Ala Asp Val Val Leu Asn Ala Leu Val
        115                 120                 125
Gly Ala Leu Gly Leu Arg Pro Thr Leu Ala Ala Leu Lys Thr Gly Ala
    130                 135                 140
Arg Leu Ala Leu Ala Asn Lys Glu Ser Leu Val Ala Gly Gly Ser Leu
145                 150                 155                 160
Val Leu Arg Ala Ala Arg Pro Gly Gln Ile Val Pro Val Asp Ser Glu
                165                 170                 175
His Ser Ala Leu Ala Gln Cys Leu Arg Gly Gly Thr Pro Asp Glu Val
            180                 185                 190
Ala Lys Leu Val Leu Thr Ala Ser Gly Gly Pro Phe Arg Gly Trp Ser
        195                 200                 205
Ala Ala Asp Leu Glu His Val Thr Pro Glu Gln Ala Gly Ala His Pro
    210                 215                 220
Thr Trp Ser Met Gly Pro Met Asn Thr Leu Asn Ser Ala Ser Leu Val
225                 230                 235                 240
Asn Lys Gly Leu Glu Val Ile Glu Thr His Leu Leu Phe Gly Ile Pro
                245                 250                 255
Tyr Asp Arg Ile Asp Val Val Val His Pro Gln Ser Ile Ile His Ser
            260                 265                 270
Met Val Thr Phe Ile Asp Gly Ser Thr Ile Ala Gln Ala Ser Pro Pro
        275                 280                 285
Asp Met Lys Leu Pro Ile Ser Leu Ala Leu Gly Trp Pro Arg Arg Val
    290                 295                 300
Ser Gly Ala Ala Ala Ala Cys Asp Phe His Thr Ala Ser Ser Trp Glu
305                 310                 315                 320
Phe Glu Pro Leu Asp Thr Asp Val Phe Pro Ala Val Glu Leu Ala Arg
                325                 330                 335
Gln Ala Gly Val Ala Gly Gly Cys Met Thr Ala Val Tyr Asn Ala Ala
            340                 345                 350
Asn Glu Glu Ala Ala Ala Ala Phe Leu Ala Gly Arg Ile Gly Phe Pro
        355                 360                 365
Ala Ile Val Gly Ile Ile Ala Asp Val Leu His Ala Ala Asp Gln Trp
    370                 375                 380
Ala Val Glu Pro Ala Thr Val Asp Asp Val Leu Asp Ala Gln Arg Trp
385                 390                 395                 400
Ala Arg Glu Arg Ala Gln Arg Ala Val Ser Gly Met Ala Ser Val Ala
                405                 410                 415
Ile Ala Ser Thr Ala Lys Pro Gly Ala Ala Gly Arg His Ala Ser Thr
            420                 425                 430
Leu Glu Arg Ser
        435
```

<210> 128
<211> 477

<212> PRT
<213> Arabidopsis thaliana

<400> 128

```
Met Met Thr Leu Asn Ser Leu Ser Pro Ala Glu Ser Lys Ala Ile Ser
 1              5                   10                  15
Phe Leu Asp Thr Ser Arg Phe Asn Pro Ile Pro Lys Leu Ser Gly Gly
            20                  25                  30
Phe Ser Leu Arg Arg Arg Asn Gln Gly Arg Gly Phe Gly Lys Gly Val
        35                  40                  45
Lys Cys Ser Val Lys Val Gln Gln Gln Gln Gln Pro Pro Pro Ala Trp
    50                  55                  60
Pro Gly Arg Ala Val Pro Glu Ala Pro Arg Gln Ser Trp Asp Gly Pro
65                  70                  75                  80
Lys Pro Ile Ser Ile Val Gly Ser Thr Gly Ser Ile Gly Thr Gln Thr
                85                  90                  95
Leu Asp Ile Val Ala Glu Asn Pro Asp Lys Phe Arg Val Val Ala Leu
            100                 105                 110
Ala Ala Gly Ser Asn Val Thr Leu Leu Ala Asp Gln Val Arg Arg Phe
        115                 120                 125
Lys Pro Ala Leu Val Ala Val Arg Asn Glu Ser Leu Ile Asn Glu Leu
```

```
            130                 135                 140
Lys Glu Ala Leu Ala Asp Leu Asp Tyr Lys Leu Glu Ile Ile Pro Gly
145                 150                 155                 160
Glu Gln Gly Val Ile Glu Val Ala Arg His Pro Glu Ala Val Thr Val
                165                 170                 175
Val Thr Gly Ile Val Gly Cys Ala Gly Leu Lys Pro Thr Val Ala Ala
            180                 185                 190
Ile Glu Ala Gly Lys Asp Ile Ala Leu Ala Asn Lys Glu Thr Leu Ile
        195                 200                 205
Ala Gly Gly Pro Phe Val Leu Pro Leu Ala Asn Lys His Asn Val Lys
    210                 215                 220
Ile Leu Pro Ala Asp Ser Glu His Ser Ala Ile Phe Gln Cys Ile Gln
225                 230                 235                 240
Gly Leu Pro Glu Gly Ala Leu Arg Lys Ile Ile Leu Thr Ala Ser Gly
                245                 250                 255
Gly Ala Phe Arg Asp Trp Pro Val Glu Lys Leu Lys Glu Val Lys Val
            260                 265                 270
Ala Asp Ala Leu Lys His Pro Asn Trp Asn Met Gly Lys Lys Ile Thr
        275                 280                 285
Val Asp Ser Ala Thr Leu Phe Asn Lys Gly Leu Glu Val Ile Glu Ala
    290                 295                 300
His Tyr Leu Phe Gly Ala Glu Tyr Asp Asp Ile Glu Ile Val Ile His
305                 310                 315                 320
Pro Gln Ser Ile Ile His Ser Met Ile Glu Thr Gln Asp Ser Ser Val
                325                 330                 335
Leu Ala Gln Leu Gly Trp Pro Asp Met Arg Leu Pro Ile Leu Tyr Thr
            340                 345                 350
Met Ser Trp Pro Asp Arg Val Pro Cys Ser Glu Val Thr Trp Pro Arg
        355                 360                 365
Leu Asp Leu Cys Lys Leu Gly Ser Leu Thr Phe Lys Lys Pro Asp Asn
    370                 375                 380
Val Lys Tyr Pro Ser Met Asp Leu Ala Tyr Ala Ala Gly Arg Ala Gly
385                 390                 395                 400
Gly Thr Met Thr Gly Val Leu Ser Ala Ala Asn Glu Lys Ala Val Glu
                405                 410                 415
Met Phe Ile Asp Glu Lys Ile Ser Tyr Leu Asp Ile Phe Lys Val Val
            420                 425                 430
Glu Leu Thr Cys Asp Lys His Arg Asn Glu Leu Val Thr Ser Pro Ser
        435                 440                 445
Leu Glu Glu Ile Val His Tyr Asp Leu Trp Ala Arg Glu Tyr Ala Ala
    450                 455                 460
Asn Val Gln Leu Ser Ser Gly Ala Arg Pro Val His Ala
465                 470                 475
```

<210> 129
<211> 19
<212> DNA
<213> Artificial Sequence

<220>
<223> Primer

<400> 129
cgtaacgctc ggtctcgtc 19

<210> 130
<211> 356
<212> PRT
<213> Campylobacter jejuni

<400> 130

Met Ile Leu Phe Gly Ser Thr Gly Ser Ile Gly Val Asn Ala Leu Lys
1               5                   10                  15
Leu Ala Ala Leu Lys Asn Ile Pro Ile Ser Ala Leu Ala Cys Gly Asp
            20                  25                  30
Asn Ile Ala Leu Leu Asn Glu Gln Ile Ala Arg Phe Lys Pro Lys Phe
        35                  40                  45
Val Ser Ile Lys Asp Ser Lys Asn Lys His Leu Val Lys His Asp Arg
    50                  55                  60
Val Phe Ile Gly Gln Glu Gly Leu Glu Gln Ile Leu Thr Glu Cys Gln
65                  70                  75                  80
Asp Lys Leu Leu Leu Asn Ala Ile Val Gly Phe Ala Gly Leu Lys Ser
                85                  90                  95
Thr Leu Lys Ala Lys Glu Leu Gly Lys Asn Ile Ala Leu Ala Asn Lys
            100                 105                 110
Glu Ser Leu Val Val Ala Gly Ser Phe Leu Lys Gly Ala Lys Phe Leu
        115                 120                 125
Pro Val Asp Ser Glu His Ala Ala Leu Lys Phe Leu Leu Glu Gly Lys
    130                 135                 140
Lys Asn Ile Ala Lys Leu Tyr Ile Thr Ala Ser Gly Gly Ala Phe Tyr
145                 150                 155                 160
Arg Tyr Lys Ile Lys Asp Leu Asn Gln Val Ser Val Lys Asp Ala Leu
                165                 170                 175
Lys His Pro Asn Trp Asn Met Gly Ala Lys Ile Thr Ile Asp Ser Ala
            180                 185                 190
Thr Met Ala Asn Lys Leu Phe Glu Ile Ile Glu Ala Tyr His Leu Tyr
        195                 200                 205
Asp Phe Lys Glu Ile Asp Ala Leu Ile Glu Pro Arg Ser Leu Val His
    210                 215                 220
Ala Met Cys Glu Phe Lys Asn Gly Ala Ser Thr Ala Tyr Phe Ser Lys
225                 230                 235                 240
Ala Asp Met Lys Leu Ala Ile Ser Asp Ala Ile Phe Glu Lys Gln Asp
                245                 250                 255
Thr Pro Ile Leu Glu Ala Val Asp Phe Ser Lys Met Pro Ala Leu Lys
            260                 265                 270
Phe His Pro Ile Ser Thr Lys Lys Tyr Pro Ile Phe Lys Leu Lys Asn
        275                 280                 285
Thr Phe Leu Lys Glu Pro Asn Leu Gly Val Ile Ile Asn Ala Ala Asn
    290                 295                 300
Glu Val Gly Val Tyr Asn Phe Leu Glu Asn Lys Ser Gly Phe Leu Asp
305                 310                 315                 320
Ile Ala Lys Cys Ile Phe Lys Ala Leu Asp His Phe Gly Val Pro Lys
                325                 330                 335
Ile Ser Ser Ile Glu Glu Val Phe Glu Tyr Asp Phe Lys Thr Arg Glu
            340                 345                 350
Tyr Leu Arg Ser
        355

<210> 131
<211> 486
<212> PRT
<213> Plasmodium falciparum

<400> 131

```
Met Lys Lys Tyr Ile Tyr Ile Tyr Phe Phe Phe Ile Thr Ile Thr Ile
 1               5                  10                  15
Asn Asp Leu Val Ile Asn Asn Thr Ser Lys Cys Val Ser Ile Glu Arg
            20                  25                  30
Arg Lys Asn Asn Ala Tyr Ile Asn Tyr Gly Ile Gly Tyr Asn Gly Pro
        35                  40                  45
Asp Asn Lys Ile Thr Lys Ser Arg Arg Cys Lys Arg Ile Lys Leu Cys
    50                  55                  60
```

```
Lys Lys Asp Leu Ile Asp Ile Gly Ala Ile Lys Lys Pro Ile Asn Val
65                  70                  75                  80
Ala Ile Phe Gly Ser Thr Gly Ser Ile Gly Thr Asn Ala Leu Asn Ile
                85                  90                  95
Ile Arg Glu Cys Asn Lys Ile Glu Asn Val Phe Asn Val Lys Ala Leu
            100                 105                 110
Tyr Val Asn Lys Ser Val Asn Glu Leu Tyr Glu Gln Ala Arg Glu Phe
        115                 120                 125
Leu Pro Glu Tyr Leu Cys Ile His Asp Lys Ser Val Tyr Glu Glu Leu
    130                 135                 140
Lys Glu Leu Val Lys Asn Ile Lys Asp Tyr Lys Pro Ile Ile Leu Cys
145                 150                 155                 160
Gly Asp Glu Gly Met Lys Glu Ile Cys Ser Ser Asn Ser Ile Asp Lys
                165                 170                 175
Ile Val Ile Gly Ile Asp Ser Phe Gln Gly Leu Tyr Ser Thr Met Tyr
            180                 185                 190
Ala Ile Met Asn Asn Lys Ile Val Ala Leu Ala Asn Lys Glu Ser Ile
        195                 200                 205
Val Ser Ala Gly Phe Phe Leu Lys Lys Leu Leu Asn Ile His Lys Asn
    210                 215                 220
Ala Lys Ile Ile Pro Val Asp Ser Glu His Ser Ala Ile Phe Gln Cys
225                 230                 235                 240
Leu Asp Asn Asn Lys Val Leu Lys Thr Lys Cys Leu Gln Asp Asn Phe
                245                 250                 255
Ser Lys Ile Asn Asn Ile Asn Lys Ile Phe Leu Cys Ser Ser Gly Gly
            260                 265                 270
Pro Phe Gln Asn Leu Thr Met Asp Glu Leu Lys Asn Val Thr Ser Glu
        275                 280                 285
Asn Ala Leu Lys His Pro Lys Trp Lys Met Gly Lys Lys Ile Thr Ile
    290                 295                 300
Asp Ser Ala Thr Met Met Asn Lys Gly Leu Glu Val Ile Glu Thr His
305                 310                 315                 320
Phe Leu Phe Asp Val Asp Tyr Asn Asp Ile Glu Val Ile Val His Lys
                325                 330                 335
Glu Cys Ile Ile His Ser Cys Val Glu Phe Ile Asp Lys Ser Val Ile
            340                 345                 350
Ser Gln Met Tyr Tyr Pro Asp Met Gln Ile Pro Ile Leu Tyr Ser Leu
        355                 360                 365
Thr Trp Pro Asp Arg Ile Lys Thr Asn Leu Lys Pro Leu Asp Leu Ala
    370                 375                 380
Gln Val Ser Thr Leu Thr Phe His Lys Pro Ser Leu Glu His Phe Pro
385                 390                 395                 400
Cys Ile Lys Leu Ala Tyr Gln Ala Gly Ile Lys Gly Asn Phe Tyr Pro
                405                 410                 415
Thr Val Leu Asn Ala Ser Asn Glu Ile Ala Asn Asn Leu Phe Leu Asn
            420                 425                 430
Asn Lys Ile Lys Tyr Phe Asp Ile Ser Ser Ile Ile Ser Gln Val Leu
        435                 440                 445
Glu Ser Phe Asn Ser Gln Lys Val Ser Glu Asn Ser Glu Asp Leu Met
    450                 455                 460
Lys Gln Ile Leu Gln Ile His Ser Trp Ala Lys Asp Lys Ala Thr Asp
465                 470                 475                 480
Ile Tyr Asn Lys His Asn
                485
```

<210> 132
<211> 24
<212> DNA
<213> Artificial Sequence

<220>

<223> Primer

<400> 132
ccsgtsgayw ssgarcayaa cgcs 24

<210> 133
<211> 21
<212> DNA
<213> Artificial Sequence

<220>
<223> Primer

<400> 133
atgatgaaca agggsctsga r 21

<210> 134
<211> 20
<212> DNA
<213> Artificial Sequence

<220>
<223> Primer

<400> 134
catccvaact ggwmvatggg 20

<210> 135
<211> 20
<212> DNA
<213> Artificial Sequence

<220>
<223> Primer

<400> 135
atyggyrwwc kcatatcmgg 20

<210> 136
<211> 27
<212> DNA
<213> Artificial Sequence

<220>
<223> Primer

<400> 136
cgaatggacg acggattggc gatggac 27

<210> 137
<211> 29
<212> DNA
<213> Artificial Sequence

<220>
<223> Primer

<400> 137
tcagttcgag ccccttgttc atcatcgtc 29

<210> 138

<211> 28
<212> DNA
<213> Artificial Sequence

<220>
<223> Primer

<400> 138
cgaactgatc gaagccttcc acctgttc 28

<210> 139
<211> 27
<212> DNA
<213> Artificial Sequence

<220>
<223> Primer

<400> 139
ggtccatcgc caatccgtcg tccattc 27

<210> 140
<211> 30
<212> DNA
<213> Artificial Sequence

<220>
<223> Primer

<400> 140
attatctaga atccgccccg cctccacctc 30

<210> 141
<211> 30
<212> DNA
<213> Artificial Sequence

<220>
<223> Primer

<400> 141
gatggatcct gggtagggtc gctgctgtcc 30

<210> 142
<211> 34
<212> DNA
<213> Artificial Sequence

<220>
<223> Primer

<400> 142
ttatctagaa ccgtctacgc cgacctcgtt caac 34

<210> 143
<211> 30

<212> DNA
<213> Artificial Sequence

<220>
<223> Primer

<400> 143
ttaggatccc ctccgctggt ccgattgaac 30

<210> 144
<211> 20
<212> DNA
<213> Artificial Sequence

<220>
<223> Primer

<400> 144
aggcgattaa gttgggtaac 20

<210> 145
<211> 18
<212> DNA
<213> Artificial Sequence

<220>
<223> Primer

<400> 145
gaccatgatt acgccaag 18

<210> 146
<211> 30
<212> DNA
<213> Artificial Sequence

<220>
<223> Primer

<400> 146
gataatcgat gtgtgactga cctgtccaac 30

<210> 147
<211> 30
<212> DNA
<213> Artificial Sequence

<220>
<223> Primer

<400> 147
cttaggtacc atgttggaga ttcaaggtgg 30

<210> 148
<211> 30
<212> DNA
<213> Artificial Sequence

<220>
<223> Primer

<400> 148
ctagatcgat acttgcggtc ggactgatag 30

<210> 149
<211> 30
<212> DNA
<213> Artificial Sequence

<220>
<223> Primer

<400> 149
gtcgctcgag atcagataat cgtcgctcaa 30

<210> 150
<211> 30
<212> DNA
<213> Artificial Sequence

<220>
<223> Primer

<400> 150
atatggtacc gacatggacg aggaagacgc 30

<210> 151
<211> 41
<212> DNA
<213> Artificial Sequence

<220>
<223> Primer

<400> 151
tagagaattc gaaggaagag catgggattg gacgaggttt c 41

<210> 152
<211> 41
<212> DNA
<213> Artificial Sequence

<220>
<223> Primer

<400> 152
tactacttgt atgtaggtac cacttgcggt cggactgata g 41

<210> 153
<211> 41
<212> DNA
<213> Artificial Sequence

<220>
<223> Primer

<400> 153
gcgtgatatc gaaggaagag catgagcgca accgtccacc g 41

<210> 154
<211> 41
<212> DNA
<213> Artificial Sequence

<220>
<223> Primer

<400> 154
actgctaggg tccgaggtac cgacatggac gaggaagacg c 41

<210> 155
<211> 42
<212> DNA
<213> Artificial Sequence

<220>
<223> Primer

<400> 155
gatgatatcg aaggaagagc atggtgaagc gcgtcacggt gt 42

<210> 156
<211> 40
<212> DNA
<213> Artificial Sequence

<220>
<223> Primer

<400> 156
caagagtcag aaggtacccg ccagaatggt gagcaggatg 40

<210> 157
<211> 40
<212> DNA
<213> Artificial Sequence

<220>
<223> Primer

<400> 157
caagagtcag aatctagacg ccagaatggt gagcaggatg 40

<210> 158
<211> 39
<212> DNA
<213> Artificial Sequence

<220>
<223> Primer

<400> 158
ctagatatcg gaaggaagag catgtcacac cccgcgtta 39

<210> 159
<211> 36
<212> DNA
<213> Artificial Sequence

<220>
<223> Primer

<400> 159
tcaggtaccg tgtcgccacc cacaacgccc ataatg 36

<210> 160
<211> 34
<212> DNA
<213> Artificial Sequence
<220>
<223> Primer

<400> 160
tagggtacca ccgtctacgc cgacctcgtt caac 34

<210> 161
<211> 34
<212> DNA
<213> Artificial Sequence

<220>
<223> Primer

<400> 161
tgtatgcatg tcgccaccca caacgcccat aatg 34

<210> 162
<211> 43
<212> DNA
<213> Artificial Sequence

<220>
<223> Primer

<400> 162
gacgaagctt gaaggaagag catgcctccc ctcaccctct atc 43

<210> 163
<211> 43
<212> DNA
<213> Artificial Sequence

<220>
<223> Primer

<400> 163
gtcactgaat gaatggtacc gcagccgaga accgccagaa gcc 43

<210> 164
<211> 34
<212> DNA
<213> Artificial Sequence

<220>
<223> Primer

<400> 164
tagggtacca ccgtctacgc cgacctcgtt gaac 34

<210> 165
<211> 34
<212> DNA
<213> Artificial Sequence

<220>
<223> Primer

<400> 165
aggcaatgca tgcagccgag aaccgccaga agcc 34

<210> 166
<211> 32
<212> DNA
<213> Artificial Sequence

<220>
<223> Primer

<400> 166
aagcatgcga aaaagttgac acctgtggag tc 32

<210> 167
<211> 29
<212> DNA
<213> Artificial Sequence

<220>
<223> Primer

<400> 167
actctagaag cacctgcgaa tggacgaag 29

<210> 168
<211> 31
<212> DNA
<213> Artificial Sequence

<220>
<223> Primer

<400> 168
ataaaggcct tacatggcga tagctagact g 31

<210> 169
<211> 31
<212> DNA
<213> Artificial Sequence

<220>
<223> Primer

<400> 169
aaggctcgag aaggatctta ccgctgttga g 31

<210> 170
<211> 32
<212> DNA
<213> Artificial Sequence

<220>
<223> Primer

<400> 170
atagtactga aaaagttgac acctgtggag tc 32

<210> 171
<211> 29
<212> DNA
<213> Artificial Sequence
<220>
<223> Primer

<400> 171
atagtactag cacctgcgaa tggacgaag 29

<210> 172
<211> 40
<212> DNA
<213> Artificial Sequence

<220>
<223> Primer

<400> 172
cgtggcatgc gtgtaagaaa aagttgacac ctgtggagtc 40

<210> 173
<211> 40
<212> DNA
<213> Artificial Sequence

<220>
<223> Primer

<400> 173
ctaagagctc agttcgggct cggtctcgcc tttcaggaag 40

<210> 174
<211> 40
<212> DNA
<213> Artificial Sequence

<220>
<223> Primer

<400> 174
gagagcgaga gccagatcaa gaagsggctg aaggacatcc 40

<210> 175

<211> 40
<212> DNA
<213> Artificial Sequence

<220>
<223> Primer

<400> 175
ggatgtcctt cagccscttc ttgatctggc tctcgctctc 40

<210> 176
<211> 30
<212> DNA
<213> Artificial Sequence

<220>
<223> Primer

<400> 176
agtcagtact aactggtgaa gacgctgaag 30

<210> 177
<211> 30
<212> DNA
<213> Artificial Sequence

<220>
<223> Primer

<400> 177
gatcagtact gtgaacgaat acgatacgca 30

<210> 178
<211> 40
<212> DNA
<213> Artificial Sequence

<220>
<223> Primer

<400> 178
gtcaaatgag ctccaaactg gtgaagacgc tgaaggacat 40

<210> 179
<211> 40
<212> DNA
<213> Artificial Sequence

<220>
<223> Primer

<400> 179
cagtcgggca tgcgtccatt tcagttgaca tacttctgtg 40

<210> 180
<211> 41
<212> DNA
<213> Artificial Sequence

<220>
<223> Primer

<400> 180
ctcttgctcg gcggcgtgcg gctctatcac gagggggtgg a 41

<210> 181
<211> 41
<212> DNA
<213> Artificial Sequence

<220>
<223> Primer

<400> 181
tccaccccct cgtgatagag ccgcacgccg ccgagcaaga g 41

<210> 182
<211> 20
<212> DNA
<213> Artificial Sequence
<220>
<223> Primer

<400> ’182
gagcagcaca ctctgggagc 20

<210> 183
<211> 21
<212> DNA
<213> Artificial Sequence

<220>
<223> Primer

<400> 183
ccacacaggt aggacaccca c 21

<210> 184
<211> 40
<212> DNA
<213> Artificial Sequence

<220>
<223> Primer

<400> 184
tcagagctcg tgtgatcgaa tggggctttg ttccttgatg 40

<210> 185
<211> 39
<212> DNA
<213> Artificial Sequence

<220>
<223> Primer

<400> 185

gaagcatgca ggtgatcgac gtgccactcg tccgaatag 39

<210> 186
<211> 20
<212> DNA
<213> Artificial Sequence

<220>
<223> Primer

<400> 186
ctcacaacct ccaaccgatg 20

<210> 187
<211> 20
<212> DNA
<213> Artificial Sequence

<220>
<223> Exemplary motif

<400> 187
aggtcgtgta ctgtcagtca 20

<210> 188
<211> 20
<212> DNA
<213> Artificial Sequence

<220>
<223> Exemplary motif

<400> 188
acgtggtgaa ctgccagtga 20

<210> 189
<211> 40
<212> DNA
<213> Artificial Sequence

<220>
<223> Primer

<400> 189
actatcgatg aaggaagagc atggctgacc tacccaagac 40

<210> 190
<211> 30
<212> DNA
<213> Artificial Sequence

<220>
<223> Primer

<400> 190
actagaattc cgcaacagtt ccttcatgtc 30

## Description

## BACKGROUND

*1. Technical Field*

The invention relates to methods and materials involved in the production of isoprenoids.

*2. Background Information*

Isoprenoids are compounds that have at least one five-carbon isoprenoid unit. Examples of isoprenoid compounds include, without limitation, carotenoids, isoprenes, sterols, terpenes, and ubiquinones. Various enzymatic pathways in plants, animals, and microorganisms result in the synthesis of isoprenoid compounds. Typically, isopentenyl diphosphate (IPP), dimethylallyl diphosphate (DMAPP), or combinations thereof are polymerized to form isoprenoid compounds.

Two pathways can be used to produce IPP. The first pathway, known as the mevalonate-dependent pathway, produces IPP from 3-hydroxymethyl-3-methylglutaryl Coenzyme A (HMGCoA) in a series of reactions. The second pathway, known as the mevalonate-independent pathway, produces IPP from 1-deoxyxylulose-5-phosphate (DXP) in a series of reactions. One of those reactions involves the use of DXP synthase (DXS) to catalyze the condensation of pyruvate and glyceraldehyde-3-phosphate to form DXP.

Once made, IPP can be used to make various isoprenoid compounds. Specifically, enzymes known as polyprenyl diphosphate synthases catalyze polymerization reactions that combine IPP and DMAPP to form compounds known as polyprenyl diphosphates. For example, decaprenyl diphosphate synthase (DDS) catalyzes the consecutive condensation of IPP with allylic diphosphates to produce decaprenyl diphosphate. Decaprenyl diphosphate is a polyprenyl diphosphate that can be used to form the side chain of a ubiquinone known as CoQ(10). Other polyprenyl diphosphate synthases include, without limitation, farnesyl-, geranyl-, and octapreneyl diphosphate synthases.

## SUMMARY

The invention relates to methods and materials involved in the production of isoprenoid compounds. Specifically, described herein are nucleic acid molecules, polypeptides, host cells, and methods that can be used to produce isoprenoid compounds. Isoprenoid compounds are both biologically and commercially important. For example, the nutritional industry uses isoprenoid compounds as nutritional supplements, while the perfume industry uses isoprenoid compounds as fragrances. The nucleic acid molecules described herein can be used to engineer host cells having the ability to produce particular isoprenoid compounds. The polypeptides described herein can be used in cell-free systems to make particular isoprenoid compounds. The host cells described herein can be used in culture systems to produce large quantities of particular isoprenoid compounds.

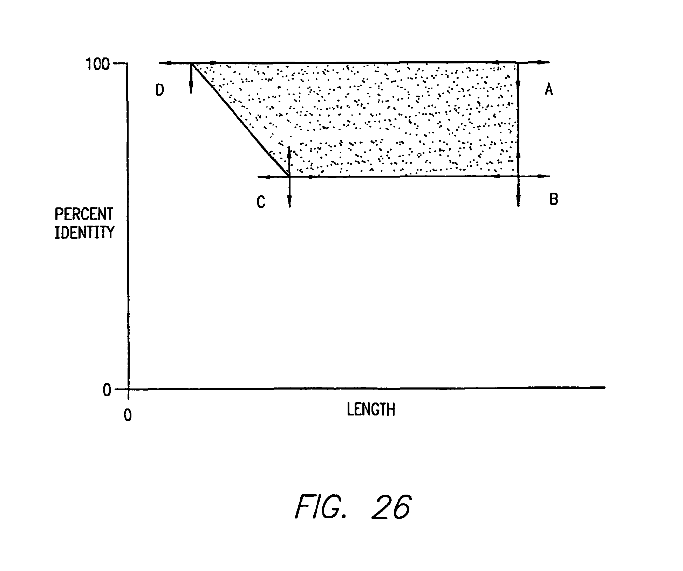

In general, an isolated nucleic acid is featured containing a nucleic acid sequence having a length and a percent identity to the sequence set forth in SEQ ID NO:1 over the length, wherein the point defined by the length and the percent identity is within the area defined by points A, B, C, and D of Figure 26, wherein point A has coordinates (3626, 100), point B has coordinates (3626, 65), point C has coordinates (50, 65), and point D has coordinates (12, 100). The point B can have coordinates (3626, 85). The point C can have coordinates (100, 65). The point C can have coordinates (50, 85). The point D can have coordinates (15, 100). The nucleic acid sequence can encode a polypeptide. The polypeptide can have DXS activity. The nucleic acid sequence can be as set forth in SEQ ID NO:1.

Further, an isolated nucleic acid is featured containing a nucleic acid sequence having a length and a percent identity to the sequence set forth in SEQ ID NO:2 over the length, wherein the point defined by the length and the percent identity is within the area defined by points A, B, C, and D of Figure 26, wherein point A has coordinates (1926, 100), point B has coordinates (1926, 65), point C has coordinates (50, 65), and point D has coordinates (12,100). The nucleic acid sequence can encode a polypeptide. The polypeptide can have DXS activity.

Further, an isolated nucleic acid is featured containing a nucleic acid sequence, wherein the nucleic acid sequence encodes a polypeptide containing an amino acid sequence, wherein the amino acid sequence has a length and a percent identity to the sequence set forth in SEQ ID NO:3 over the length, wherein the point defined by the length and the percent identity is within the area defined by points A, B, C, and D of Figure 26, wherein point A has coordinates (641, 100), point B has coordinates (641, 65), point C has coordinates (25, 65), and point D has coordinates (5, 100). The polypeptide can have DXS activity.

Further, an isolated nucleic acid is featured containing a nucleic acid sequence having a length and a percent identity to the sequence set forth in SEQ ID NO:37 over the length, wherein the point defined by the length and the percent identity is within the area defined by points A, B, C, and D of Figure 26, wherein point A has coordinates (1990, 100), point B has coordinates (1990, 65), point C has coordinates (50, 65), and point D has coordinates (16, 100). The point B can have coordinates (1990, 85). The point C can have coordinates (100, 55). The point C can have coordinates (50, 85). The point D can have coordinates (20, 100). The nucleic acid sequence can encode a polypeptide. The polypeptide can have DDS activity. The nucleic acid sequence can be as set forth in SEQ ID NO:37.

Further, an isolated nucleic acid is featured containing a nucleic acid sequence having a length and a percent identity to the sequence set forth in SEQ ID NO:38 over the length, wherein the point defined by the length and the percent identity is within the area defined by points A, B, C, and D of Figure 26, wherein point A has coordinates (1002, 100), point B has coordinates (1002, 65), point C has coordinates (50, 65), and point D has coordinates (16, 100). The nucleic acid sequence can encode a polypeptide. The polypeptide can have DDS activity.

Further, an isolated nucleic acid is featured containing a nucleic acid sequence, wherein the nucleic acid sequence encodes a polypeptide containing an amino acid sequence, wherein the amino acid sequence has a length and a percent identity to the sequence set forth in SEQ ID NO:39 over the length, wherein the point defined by the length and the percent identity is within the area defined by points A, B, C, and D of Figure 26, wherein point A has coordinates (333, 100), point B has coordinates (333, 65), point C has coordinates (25, 65), and point D has coordinates (5, 100). The polypeptide can have DDS activity.

Further, an isolated nucleic acid is featured containing a nucleic acid sequence having a length and a percent identity to the sequence set forth in SEQ ID NO:40 over the length, wherein the point defined by the length and the percent identity is within the area defined by points A, B, C, and D of Figure 26, wherein point A has coordinates (1833, 100), point B has coordinates (1833, 65), point C has coordinates (50, 65), and point D has coordinates (16,100). The point B can have coordinates (1833, 85). The point C can have coordinates (100, 65). The point C can have coordinates (50, 85). The point D can have coordinates (20, 100). The nucleic acid sequence can encode a polypeptide. The polypeptide can have DDS activity. The nucleic acid sequence can be as set forth in SEQ ID NO:40.

Further, an isolated nucleic acid is featured containing a nucleic acid sequence having a length and a percent identity to the sequence set forth in SEQ ID NO:41 over the length, wherein the point defined by the length and the percent identity is within the area defined by points A, B, C, and D of Figure 26, wherein point A has coordinates (1014, 100), point B has coordinates (1014, 65), point C has coordinates (50, 65), and point D has coordinates (16, 100). The nucleic acid sequence can encode a polypeptide. The polypeptide can have DDS activity.

Further, an isolated nucleic acid is featured containing a nucleic acid sequence, wherein the nucleic acid sequence encodes a polypeptide containing an amino acid sequence, wherein the amino acid sequence has a length and a percent identity to the sequence set forth in SEQ ID NO:42 over the length, wherein the point defined by the length and the percent identity is within the area defined by points A, B, C, and D of Figure 26, wherein point A has coordinates (337, 100), point B has coordinates (337, 65), point C has coordinates (25, 65), and point D has coordinates (5, 100). The polypeptide can have DDS activity.

Further, an isolated nucleic acid is featured containing a nucleic acid sequence having a length and a percent identity to the sequence set forth in SEQ ID NO:95 over the length, wherein the point defined by the length and the percent identity is within the area defined by points A, B, C, and D of Figure 26, wherein point A has coordinates (2017, 100), point B has coordinates (2017, 65), point C has coordinates (50, 65), and point D has coordinates (16, 100). The point B can have coordinates (2017, 85). The point C can have coordinates (100, 65). The point C can have coordinates (50, 85). The point D can have coordinates (20, 100). The nucleic acid sequence can encode a polypeptide. The polypeptide can have DXR activity. The nucleic acid sequence can be as set forth in SEQ ID NO:95.

Further, an isolated nucleic acid is featured containing a nucleic acid sequence having a length and a percent identity to the sequence set forth in SEQ ID NO:96 over the length, wherein the point defined by the length and the percent identity is within the area defined by points A, B, C, and D of Figure 26, wherein point A has coordinates (1161, 100), point B has coordinates (1161, 65), point C has coordinates (50, 65), and point D has coordinates (16, 100). The nucleic acid sequence can encode a polypeptide. The polypeptide can have DXR activity.

Further, an isolated nucleic acid is featured containing a nucleic acid sequence, wherein the nucleic acid sequence encodes a polypeptide containing an amino acid sequence, wherein the amino acid sequence has a length and a percent identity to the sequence set forth in SEQ ID NO:97 over the length, wherein the point defined by the length and the percent identity is within the area defined by points A, B, C, and D of Figure 26, wherein point A has coordinates (386, 100), point B has coordinates (386, 65), point C has coordinates (25, 65), and point D has coordinates (5, 100). The polypeptide can have DXR activity.

Further, an isolated nucleic acid is featured containing a nucleic acid sequence of at least 12 nucleotides, wherein the isolated nucleic acid hybridizes under hybridization conditions to the sense or antisense strand of a nucleic acid molecule, the sequence of the nucleic acid molecule being the sequence set forth in SEQ ID NO: 1, 2, 37, 3 8, 40, 41, 95, or 96. The nucleic acid sequence can be at least 50 nucleotides (e.g., at least 100, 200, 300, 400, 500, or more). The nucleic acid sequence can encode a polypeptide. The polypeptide can have DXS, DDS, or DXR activity.

Further a substantially pure polypeptide is featured containing an amino acid sequence, wherein the amino acid sequence has a length and a percent identity to the sequence set forth in SEQ ID NO:3 over the length, wherein the point defined by the length and the percent identity is within the area defined by points A, B, C, and D of Figure 26, wherein point A has coordinates (641,100), point B has coordinates (641, 65), point C has coordinates (25, 65), and point D has coordinates (5, 100). The polypeptide can have DXS activity.

Further, substantially pure polypeptide is featured containing an amino acid sequence, wherein the amino acid sequence has a length and a percent identity to the sequence set forth in SEQ ID NO:39 over the length, wherein the point defined by the length and the percent identity is within the area defined by points A, B, C, and D of Figure 26, wherein point A has coordinates (333, 100), point B has coordinates (333, 65), point C has coordinates (25, 65), and point D has coordinates (5, 100). The polypeptide can have DDS activity.

Further, a substantially pure polypeptide is featured containing an amino acid sequence, wherein the amino acid sequence has a length and a percent identity to the sequence set forth in SEQ ID NO:42 over the length, wherein the point defined by the length and the percent identity is within the area defined by points A, B, C, and D of Figure 26, wherein point A has coordinates (337, 100), point B has coordinates (337, 65), point C has coordinates (25, 65), and point D has coordinates (5, 100). The polypeptide can have DDS activity.

Further, a substantially pure polypeptide is featured containing an amino acid sequence, wherein the amino acid sequence has a length and a percent identity to the sequence set forth in SEQ ID NO:97 over the length, wherein the point defined by the length and the percent identity is within the area defined by points A, B, C, and D of Figure 26, wherein point A has coordinates (3 86, 100), point B has coordinates (386, 65), point C has coordinates (25, 65), and point D has coordinates (5, 100). The polypeptide can have DXR activity.

An aspect of the invention features a host cell comprising a nucleic acid as defined in the claims. The host cell can be prokaryotic. The host cell can be a *Rhodobacter, Sphingomonas,* or *Escherichia* cell. The host cell can contain an exogenous nucleic acid that encodes a polypeptide having DDS, DXS or chorismate lyase activity. The host cell can contain an exogenous nucleic acid containing an UbiC sequence or LytB sequence. The host cell can contain an exogenous nucleic acid containing an UbiC sequence and LytB sequence. The host cell can contain a non-functional crtE sequence and either a non-functional ppsR sequence, or a non-functional ccoN sequence. The host cell can contain a non-functional crtE sequence, ppsR sequence, and ccoN sequence.

Another embodiment of the invention features a host cell as defined in the claims. Further, a host cell is featured containing an exogenous nucleic acid and a non-functional crtE sequence, ppsR sequence, or ccoN sequence, wherein the exogenous nucleic acid is within a crtE, ppsR, or ccoN locus of the host cell.

Further, a host cell is featured containing a genomic deletion, wherein the deletion comprises at least a portion of a crtE sequence, ppsR sequence, or ccoN sequence, and wherein the host cell comprises a non-functional crtE sequence, ppsR sequence, or ccoN sequence.

Another aspect of the invention features a method for making CoQ (10), as defined in the claims. Further, a method for increasing production of CoQ(10) in a cell having endogenous DDS activity is featured. The method includes inserting a nucleic acid molecule containing a nucleic acid sequence that encodes a polypeptide having DDS activity into the cell such that production of CoQ(10) is increased. The nucleic acid molecule can contain an isolated nucleic acid as defined in the claims. The production of CoQ(10) can be increased at least about 5 percent as compared to a control cell lacking the inserted nucleic acid molecule. The cell can be a *Rhodobacter* or *Sphingomonas* cell. The cell can be a membraneous bacterium or highly membraneous bacterium. The method can also include inserting a second nucleic acid molecule containing a nucleotide sequence that encodes a polypeptide having DXS activity into the cell. The second nucleic acid molecule can contain an isolated nucleic acid as defined in the claims.

Further, a method is featured for increasing production of CoQ(10) in a cell having endogenous DDS activity. The method includes inserting a nucleic acid molecule containing a nucleic acid sequence that encodes a polypeptide having DXS activity into the cell such that production of CoQ(10) is increased. The production of CoQ(10) can be increased at least about 5 percent as compared to a control cell lacking the inserted nucleic acid molecule. The cell can be a *Rhodobacter* or *Sphingomonas* cell. The nucleic acid molecule can contain an isolated nucleic acid as defined in the claims. The cell can be a membraneous bacterium or highly membraneous bacterium. The method can also include inserting a second nucleic acid molecule containing a nucleotide sequence that encodes a polypeptide having DDS activity into the cell. The second nucleic acid molecule can contain an isolated nucleic acid as defined in the claims.

Further, a method is featured for increasing production of CoQ(10) in a membraneous bacterium. The method includes inserting a nucleic acid molecule containing a nucleic acid sequence that encodes a polypeptide having DDS activity into the bacterium such that production of CoQ(10) is increased.

Further, a method is featured for increasing production of CoQ(10) in a highly membraneous bacterium. The method includes inserting a nucleic acid molecule containing a nucleic acid sequence that encodes a polypeptide having DDS activity into the highly membraneous bacterium such that production of CoQ(10) is increased.

Further, a method for making an isoprenoid is featured. The method includes culturing a cell under conditions wherein the cell produces the isoprenoid, wherein the cell contains at least one exogenous nucleic acid that encodes at least one polypeptide, wherein the cell produces more of the isoprenoid than a comparable cell lacking the at least one exogenous nucleic acid. The cell can be a *Rhodobacter* or *Sphingomonas* cell. The isoprenoid can be CoQ(10).

The at least one polypeptide can have DDS, DXS, ODS, SDS, DXR, 4-diphosphocytidyl-2C-methyl-D-erythritol synthase, 4-diphosphocytidyl-2C-methyl-D-erythritol kinase, or chorismate lyase activity. The at least one polypeptide can be a UbiC polypeptide or a LytB polypeptide. The cell can contain a non-functional crtE sequence, ppsR sequence, or ccoN sequence. The cell can contain a non-functional crtE sequence, ppsR sequence, and ccoN sequence. The cell can contain a genomic deletion, wherein the deletion contains at least a portion of a crtE sequence, ppsR sequence, or ccoN sequence, and wherein the cell contains a non-functional crtE sequence, ppsR sequence, or ccoN sequence.

Further, a method for making an isoprenoid is featured. The method includes culturing a genetically modified cell under conditions wherein the cell produces the isoprenoid. The isoprenoid can be CoQ(10). The cell can contain an exogenous nucleic acid. The cell can contain a genomic deletion.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Other features and advantages of the invention will be apparent from the following detailed description, and from the claims.

## DESCRIPTION OF DRAWINGS

Figure 1 is a diagram of a pathway for producing CoQ(10).

Figure 2 is a listing of a nucleic acid sequence that encodes a *Sphingomonas trueperi* (ATCC 12417) polypeptide having DXS activity (SEQ ID NO:1). The start codon is the ATG at nucleotide number 182, and the stop codon is the TAA at nucleotide number 2107. The probable ribosome binding site is at nucleotide numbers 175-178. This sequence contains an open reading frame as well as 5' and 3' untranslated sequences.

Figure 3 is a listing of a nucleic acid sequence that encodes a *Sphingomonas trueperi* (ATCC 12417) polypeptide having DXS activity (SEQ ID NO:2). This sequence corresponds to the open reading frame.

Figure 4 is a listing of an amino acid sequence of a *Sphingomonas trueperi* (ATCC 12417) polypeptide having DXS activity (SEQ ID NO:3).

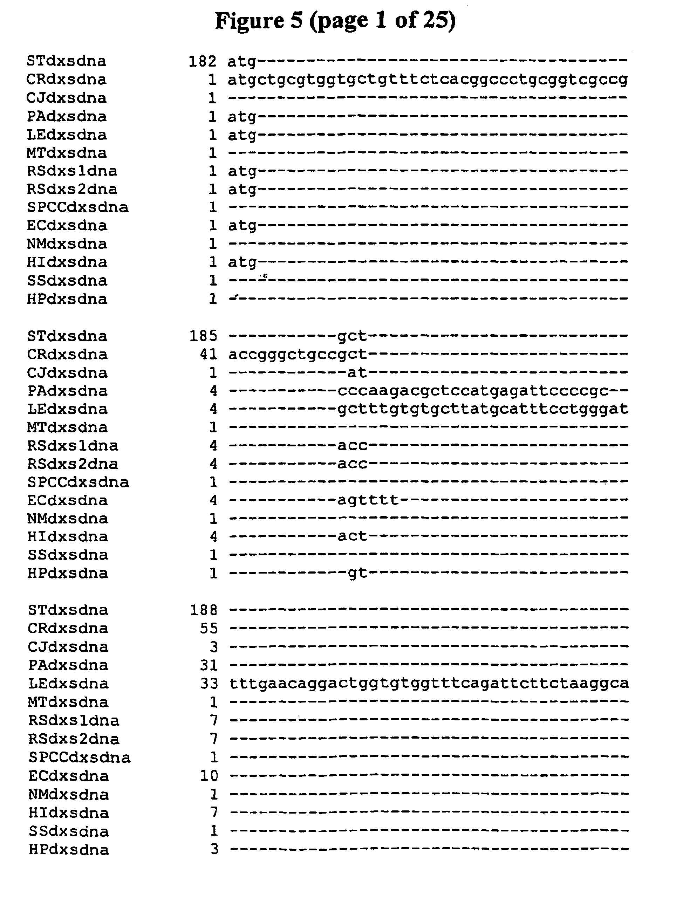

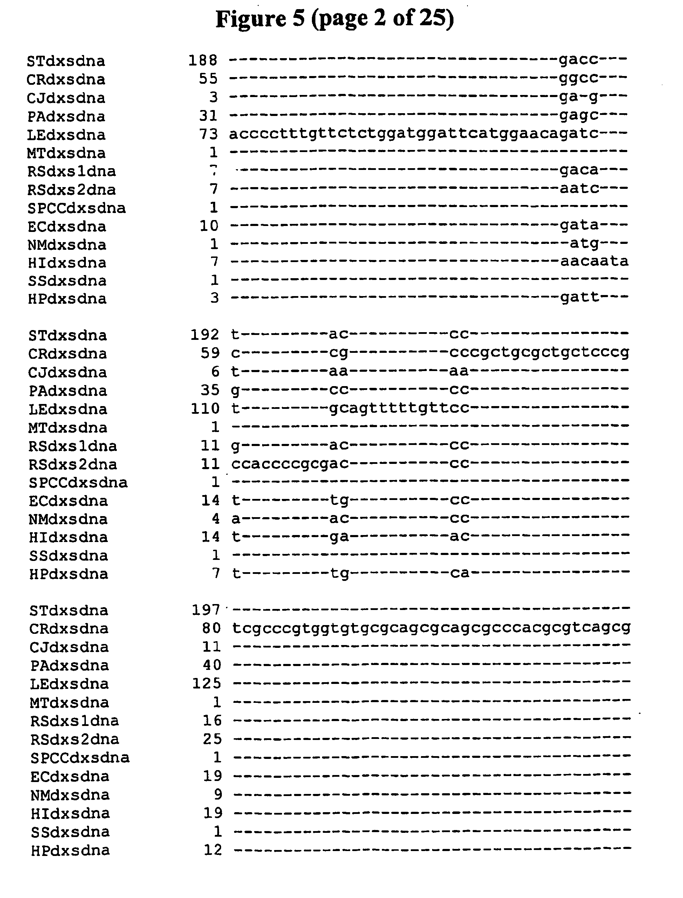

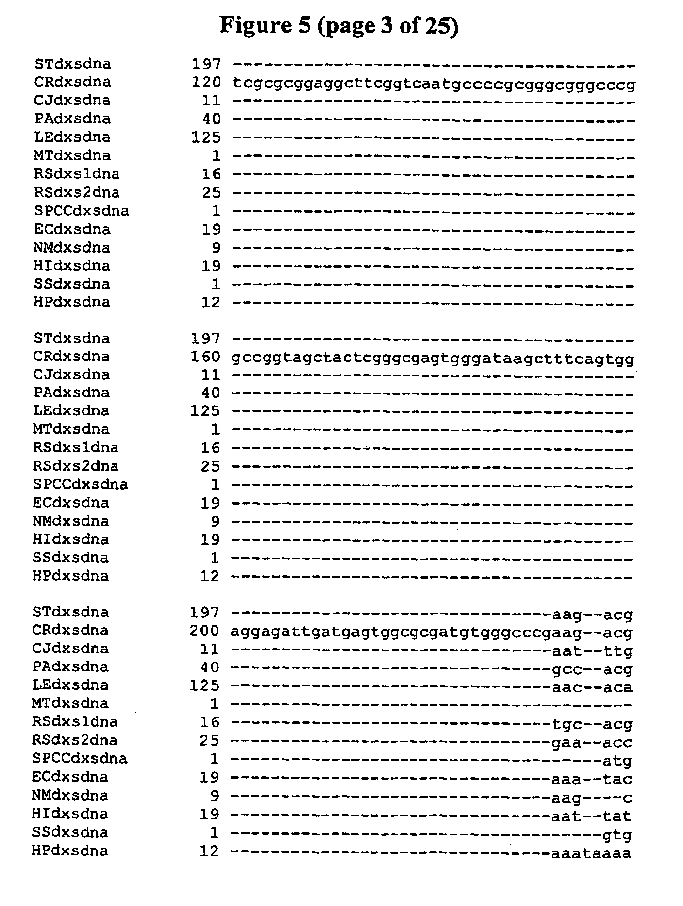

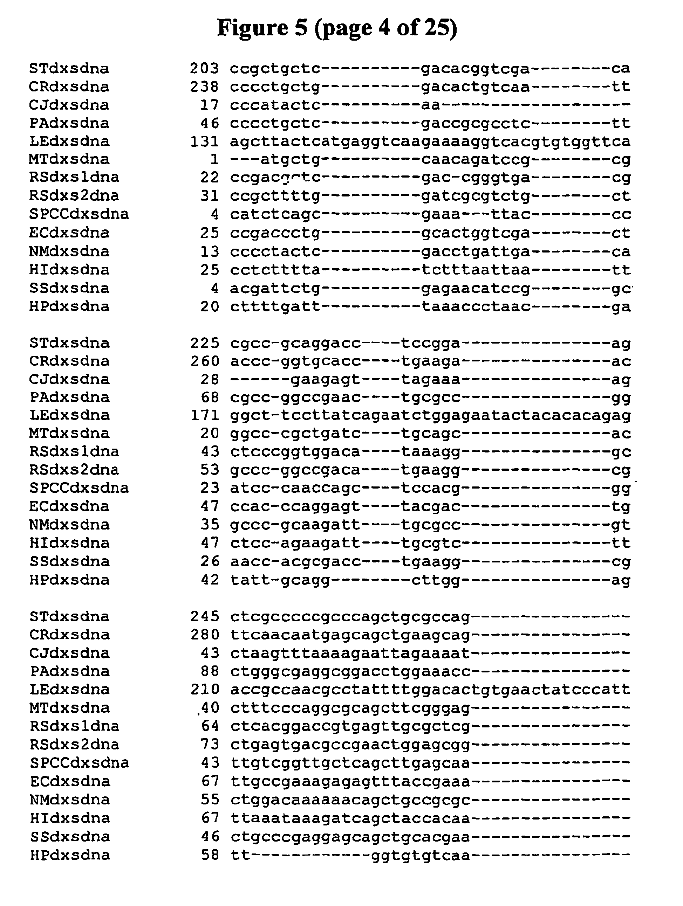

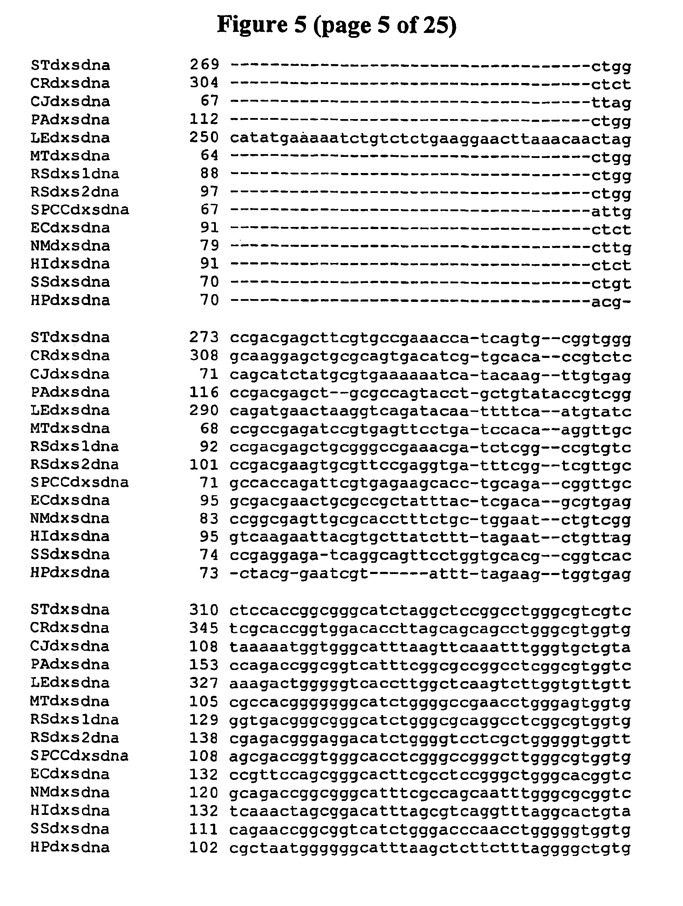

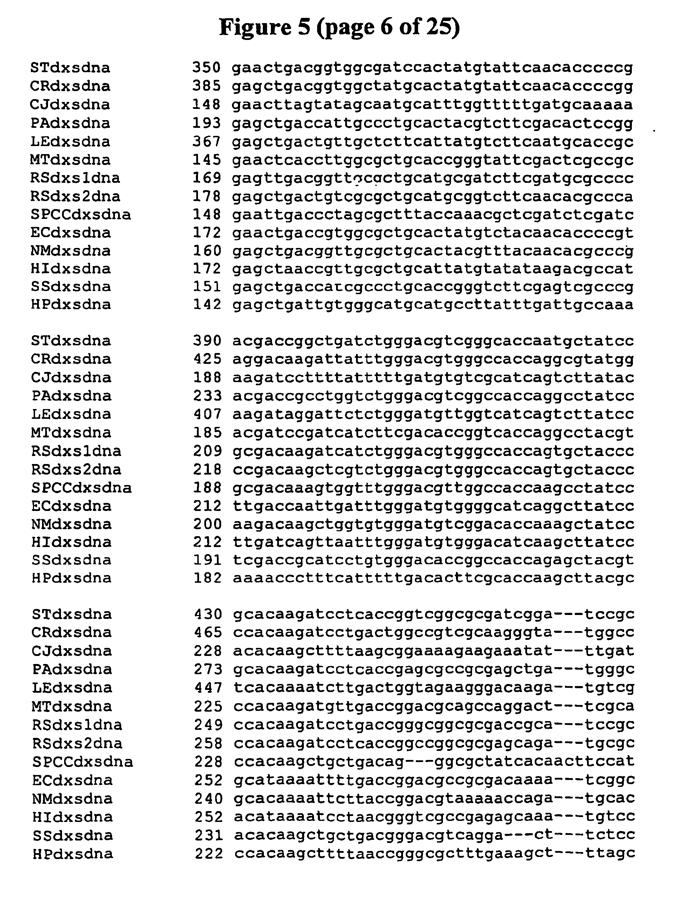

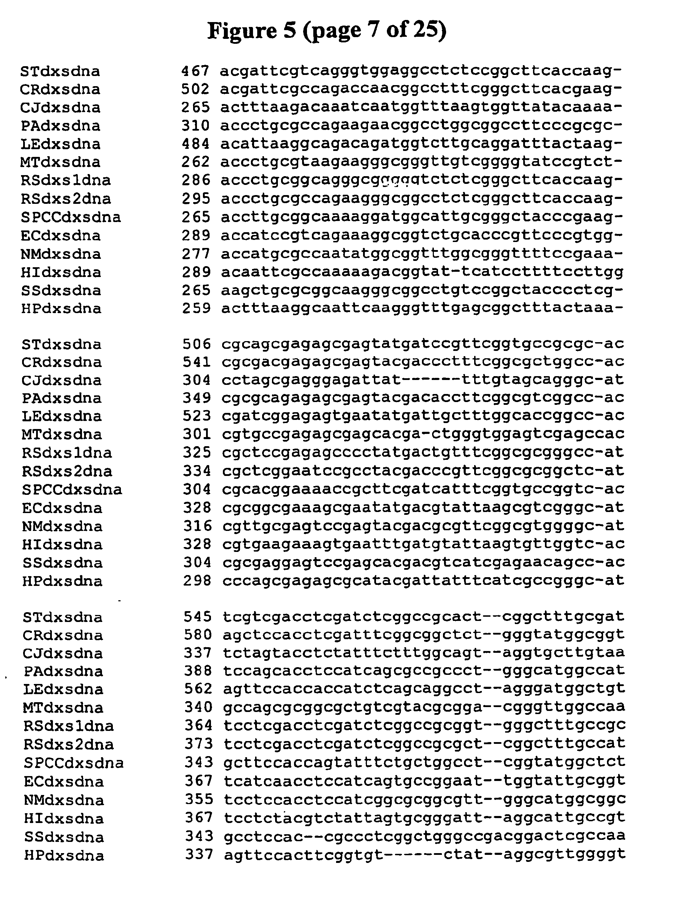

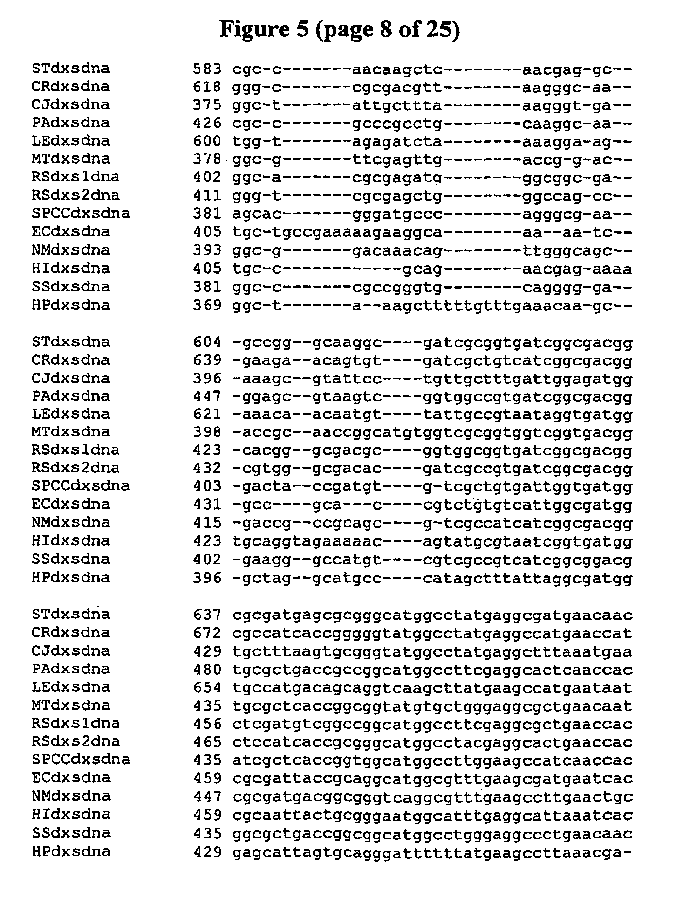

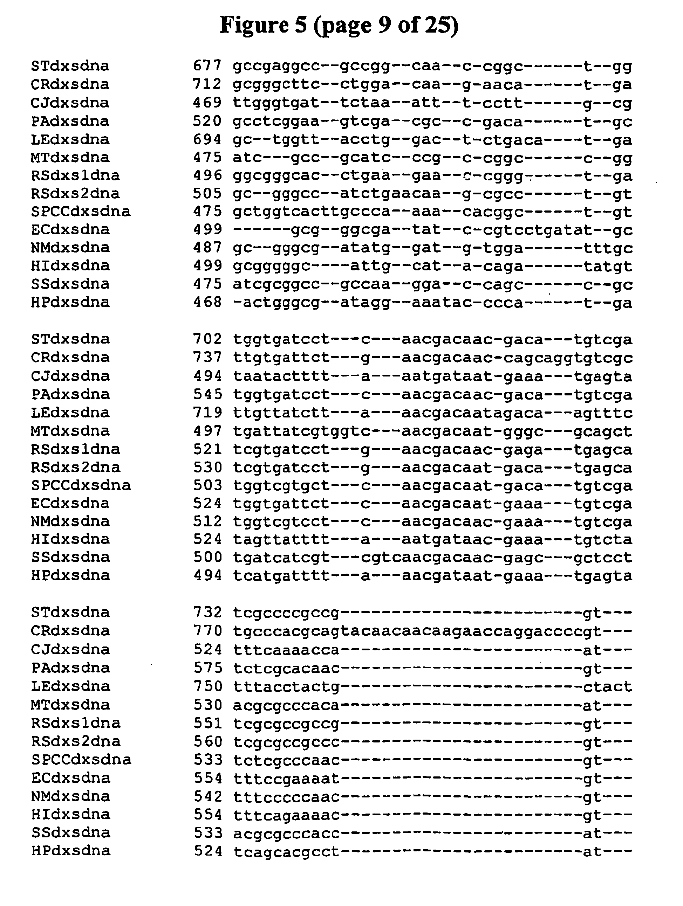

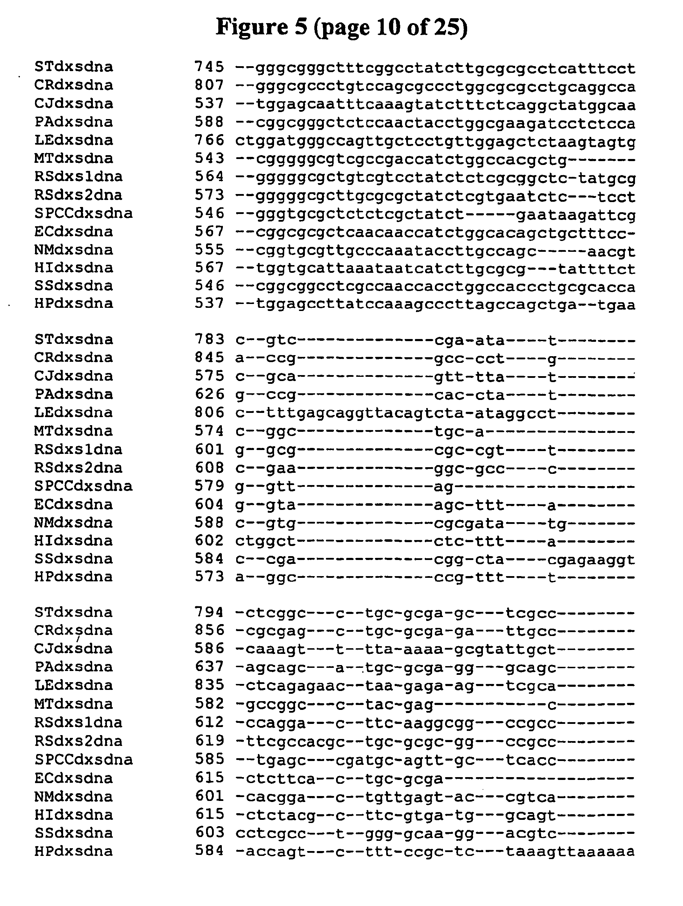

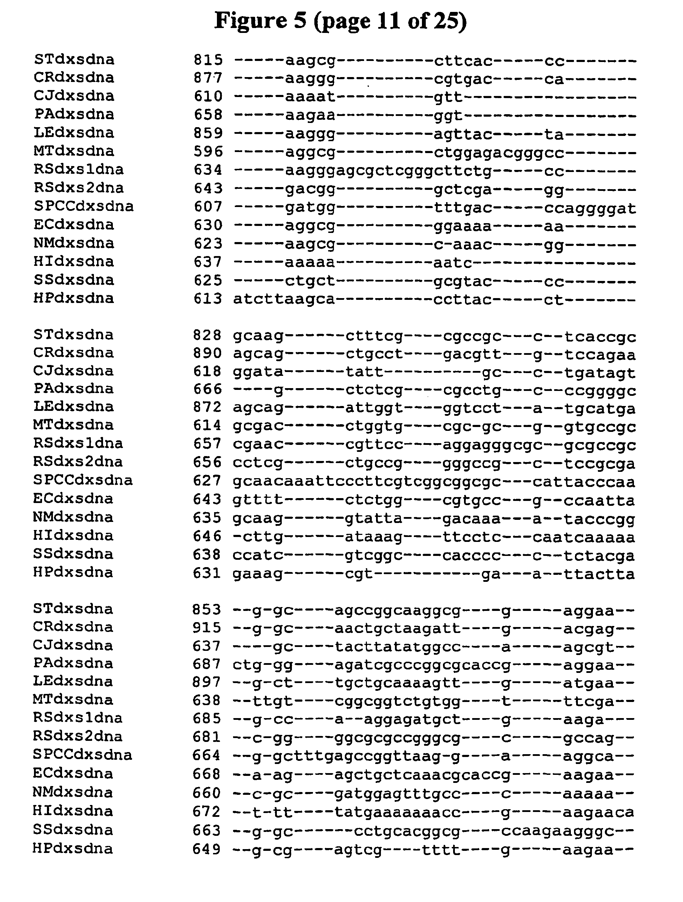

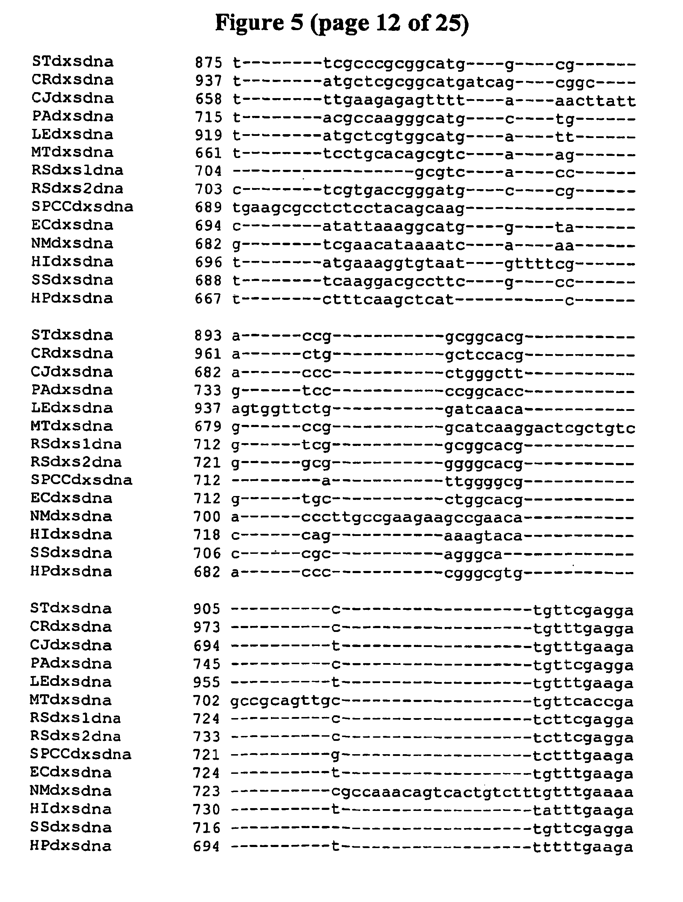

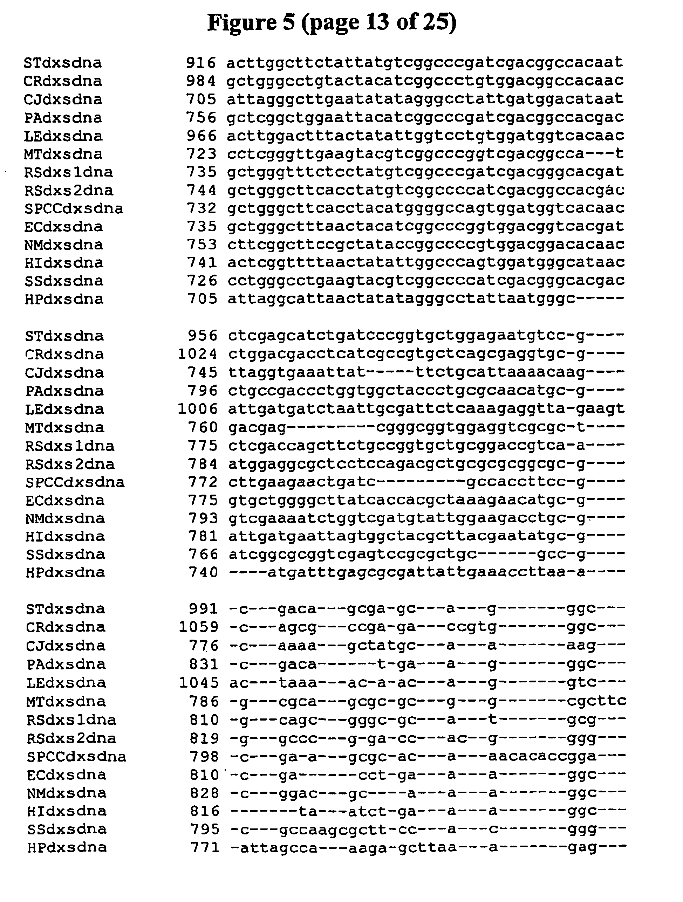

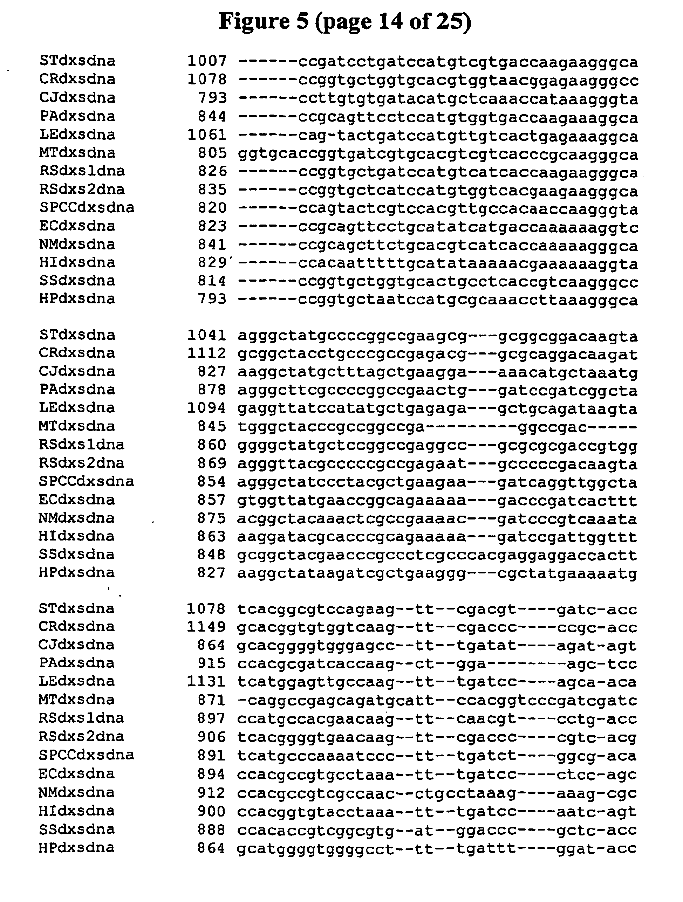

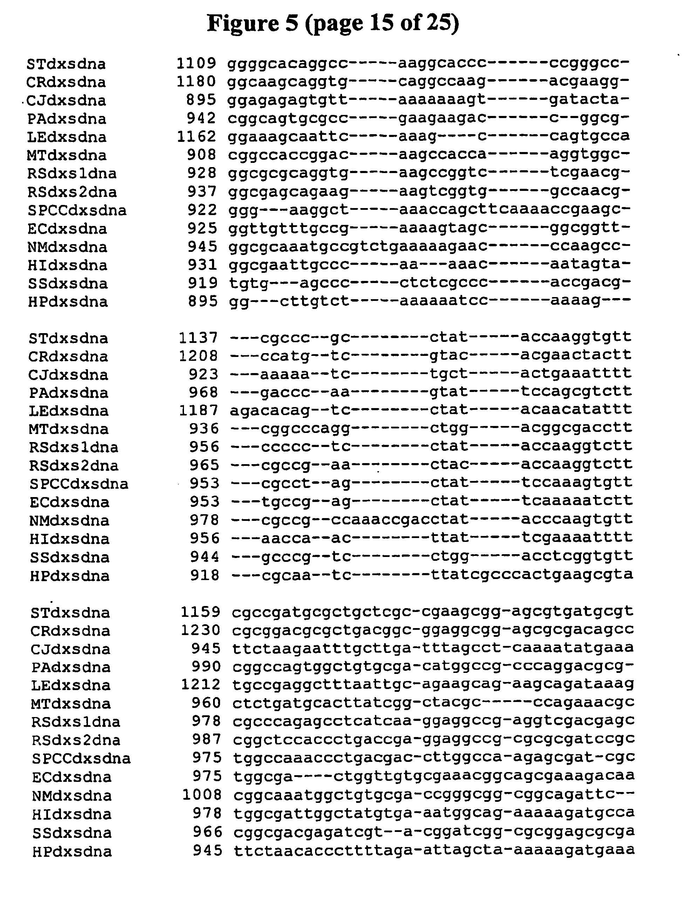

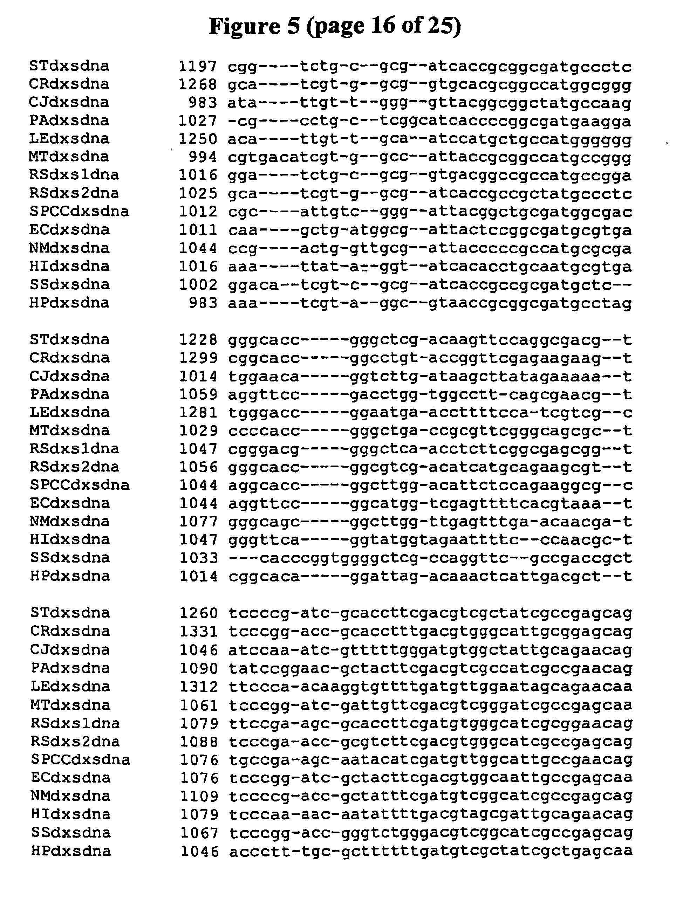

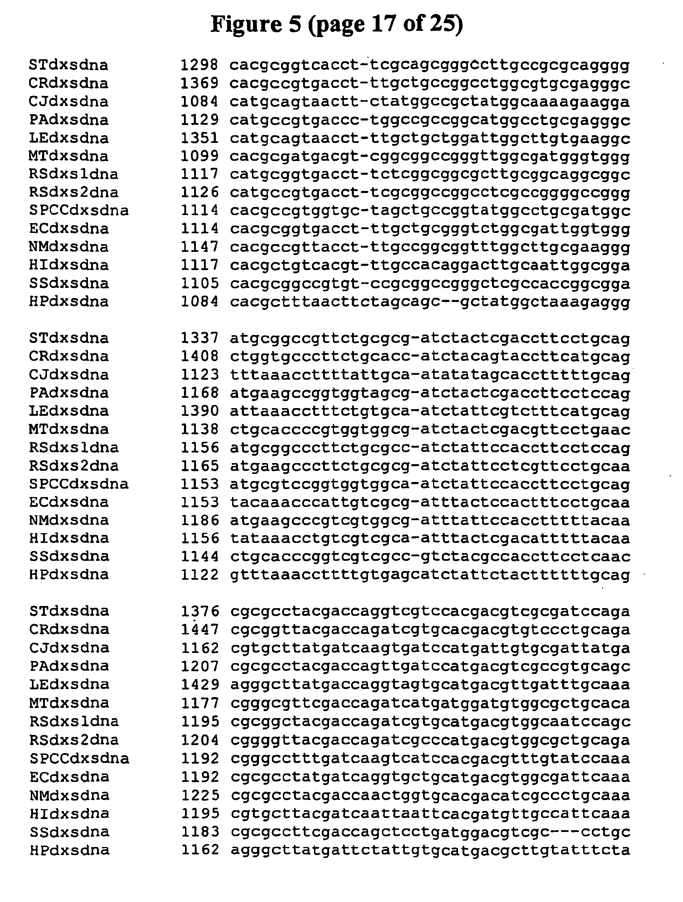

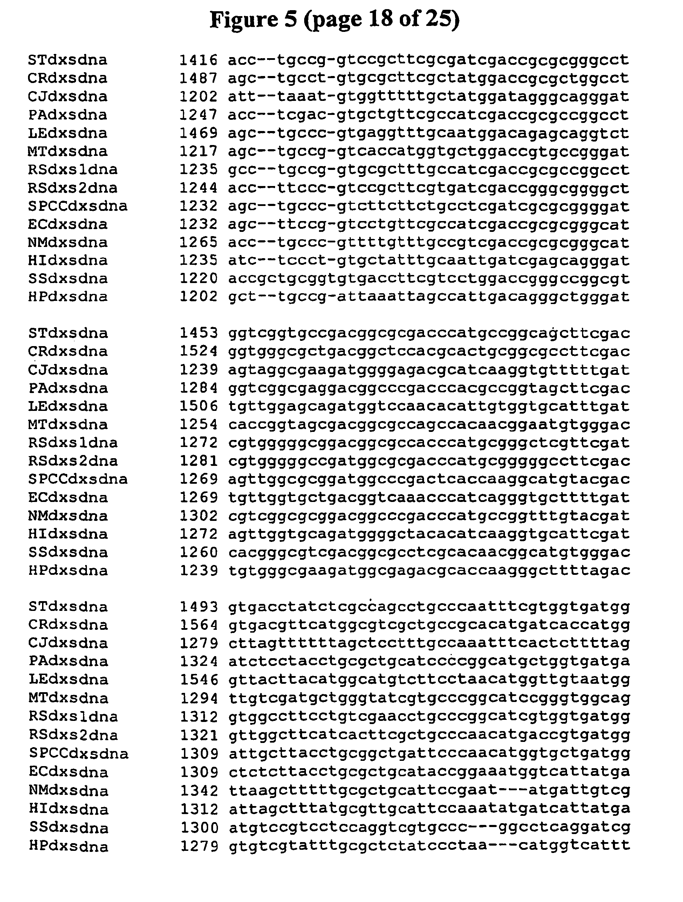

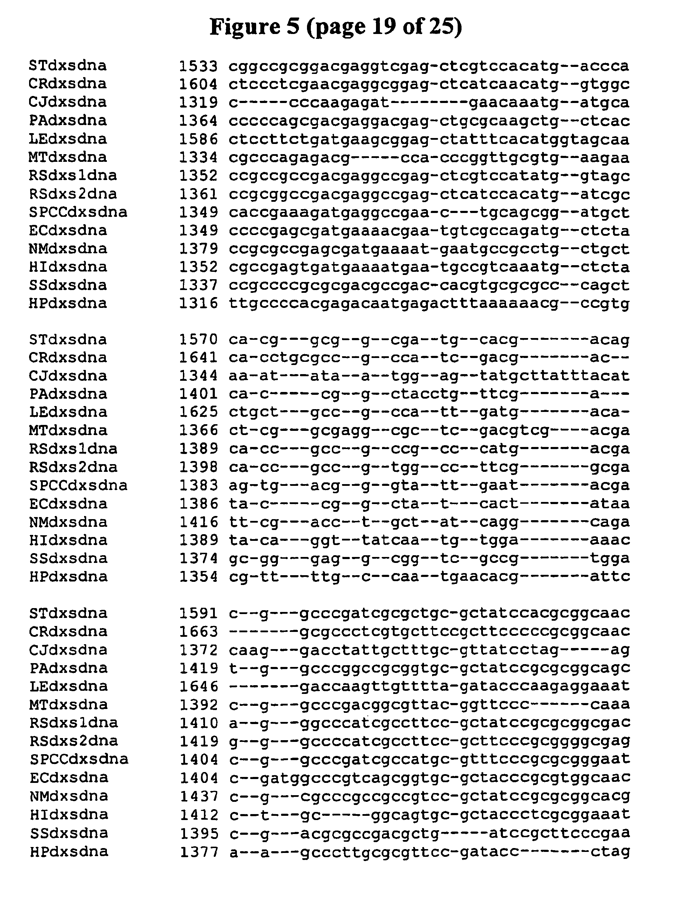

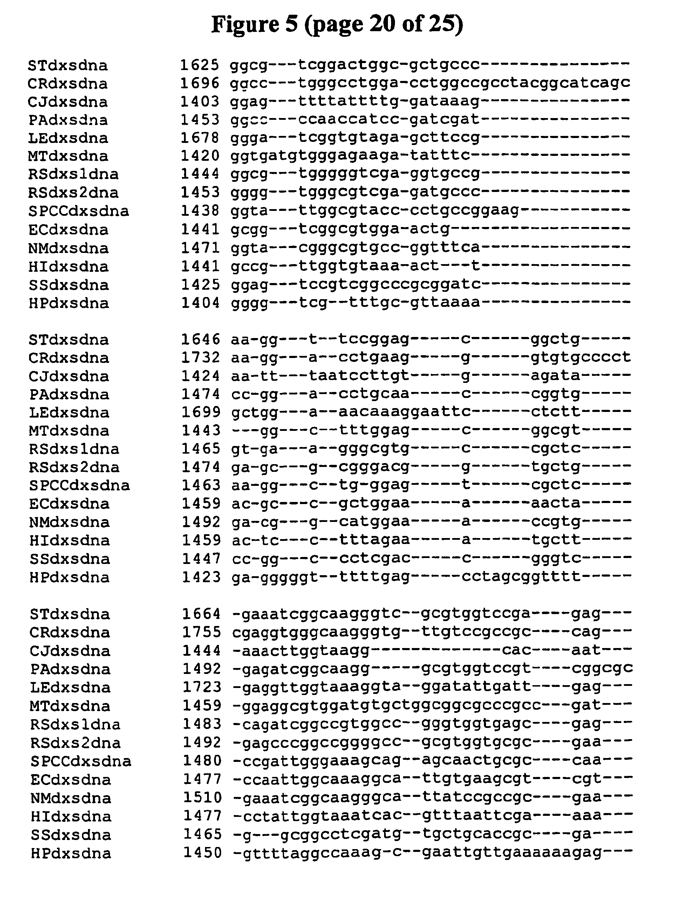

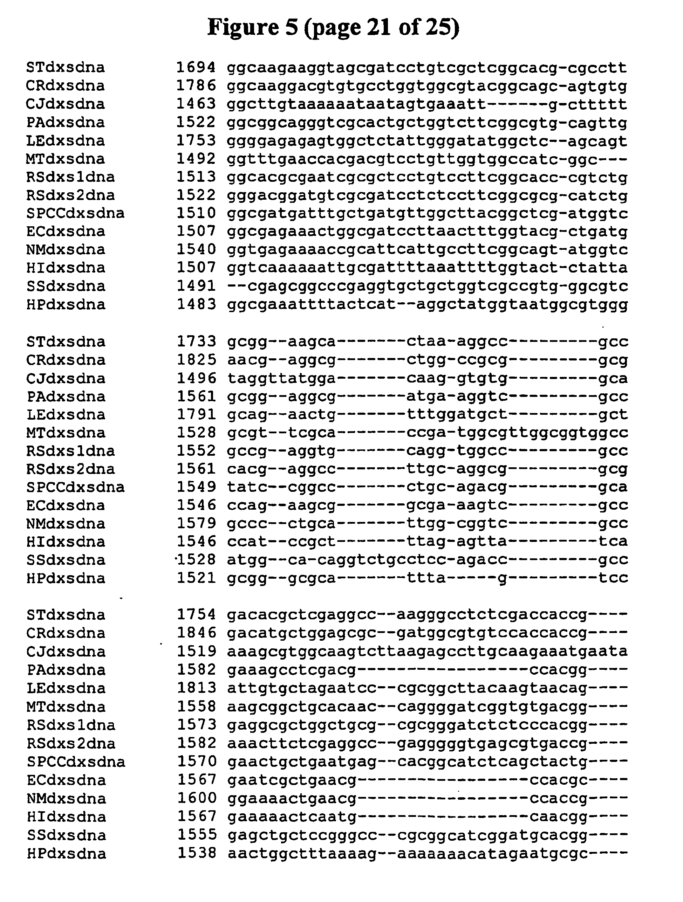

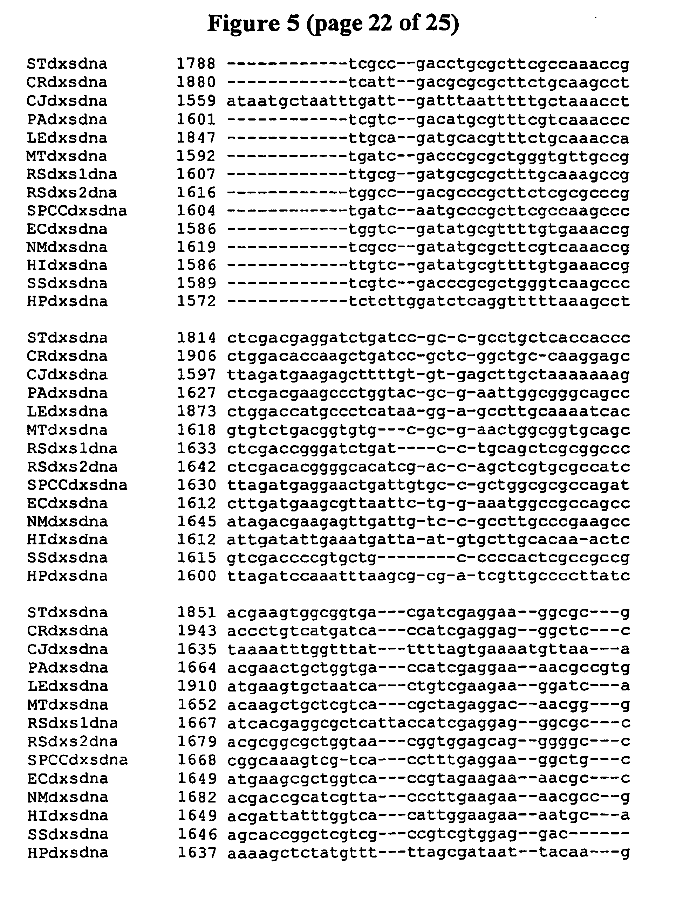

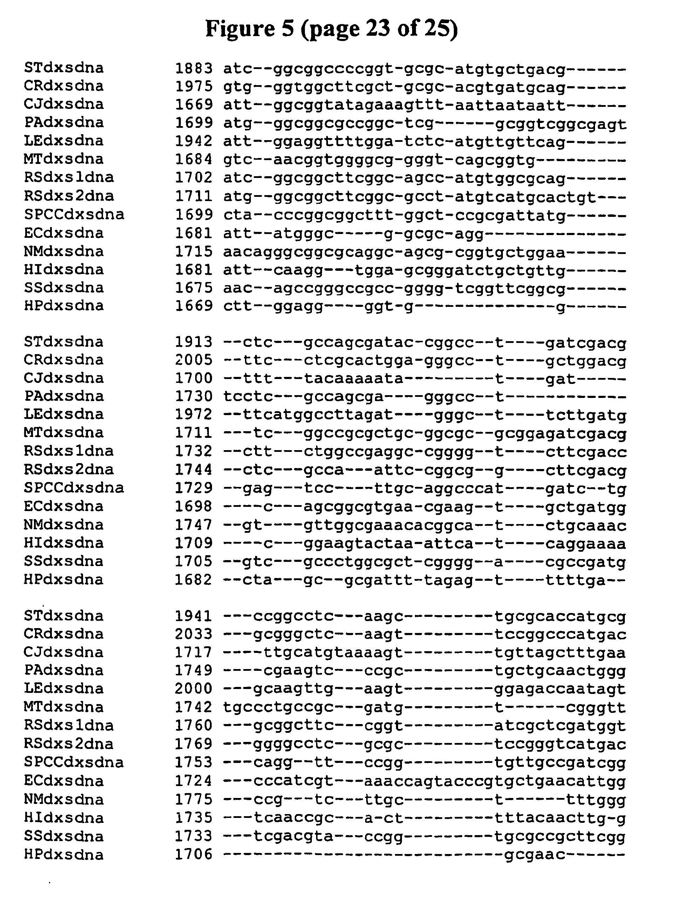

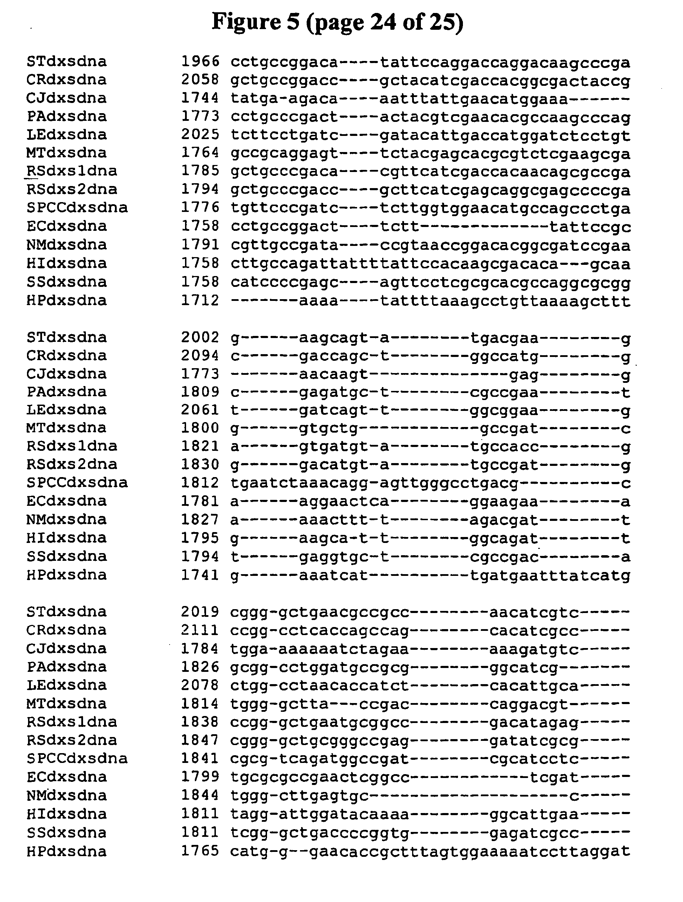

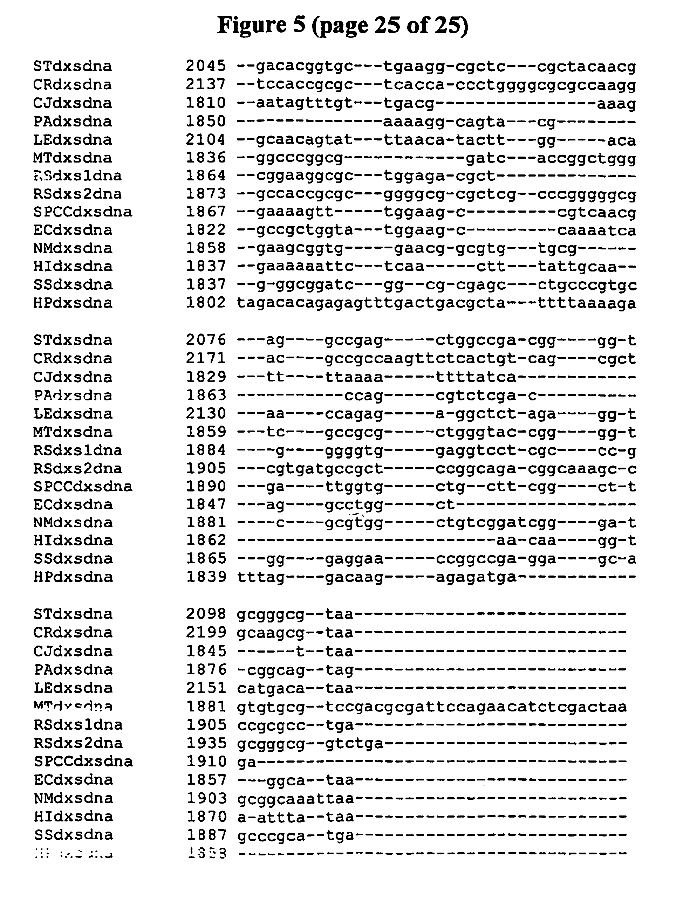

Figure 5 is a sequence pile-up of 14 nucleic acid sequences that encode polypeptides having DXS activity. STdxsdna represents the nucleic acid sequence set forth in SEQ ID NO:2; CRdxsdna represents a nucleic acid sequence from *Chlamydomonas reinhardtii* (GenBank accession number AJ007559; SEQ ID NO:4); CJdxsdna represents a nucleic acid sequence from *Campylobacter jejuni* (GenBank accession number AL139074; SEQ ID NO:5); PAdxsdna represents a nucleic acid sequence from *Pseudomonas aeruginosa* (GenBank accession number AE004821; SEQ ID NO:6); LEdxsdna represents a nucleic acid sequence from *Lycopersicon esculentum* (GenBank accession number AF143812; SEQ ID NO:7); MTdxsdna represents a nucleic acid sequence from *Mycobacterium tuberculosis* (GenBank accession number Z96072; ; SEQ ID NO:8); RSdxs1dna represents a nucleic acid sequence from a *Rhodobacter sphaeroides* dxs1 gene (SEQ ID NO:9); RSdxs2dna represents a nucleic acid sequence from a *Rhodobacter sphaeroides* dxs2 gene (SEQ ID NO:10); SPCCdxsdna represents a nucleic acid sequence from *Synechococcus* PCC6301 (GenBank accession number Y18874; SEQ ID NO:11); ECdxsdna represents a nucleic acid sequence from *Escherichia coli* (GenBank accession number AF035440; SEQ ID NO:12); NMdxsdna represents a nucleic acid sequence from *Neisseria meningitidis* (GenBank accession number AL162753; SEQ ID NO:13); HIdxsdna represents a nucleic acid sequence from *Haemophilus influenza* (GenBank accession number U32822; SEQ ID NO:14); SSdxsdna represents a nucleic acid sequence from *Streptomyces sp.* CL190 (GenBank accession number AB026631; SEQ ID NO:16); and HPdxsdna represents a nucleic acid sequence from *Helicobacter pylori* 26695 (GenBank accession number AE000552; SEQ ID NO:17).

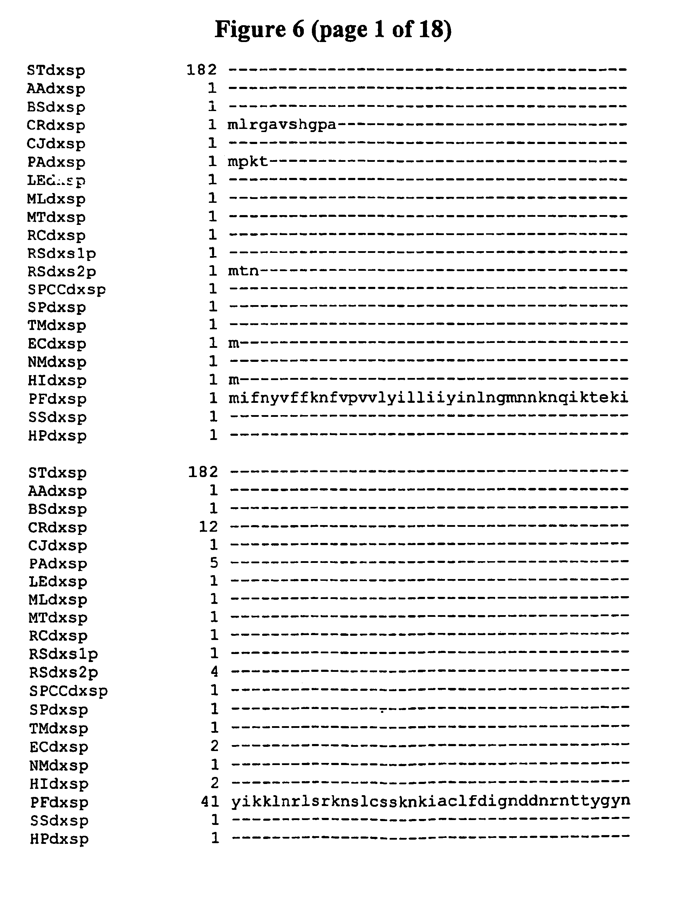

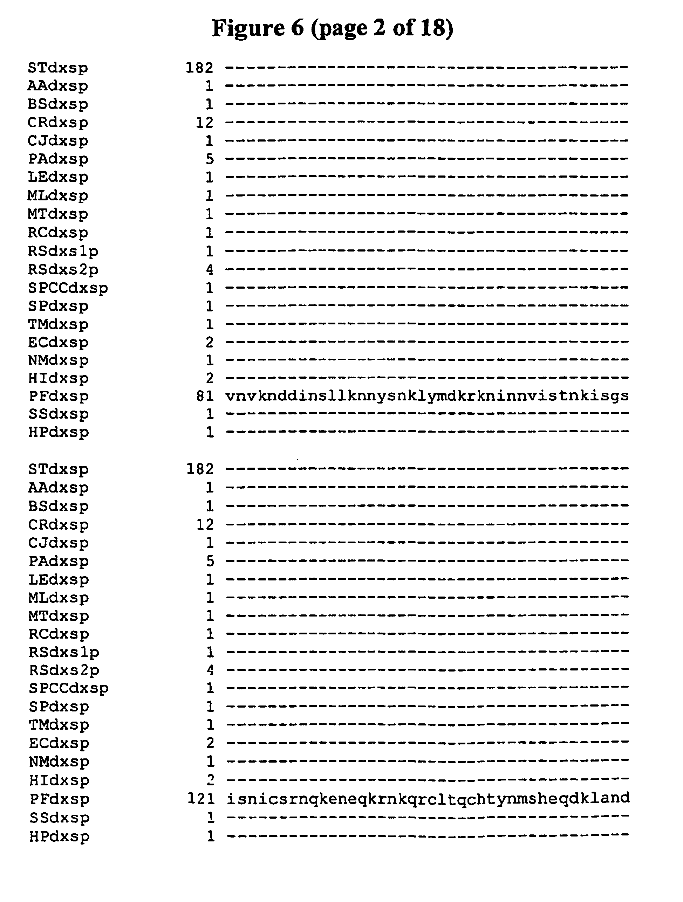

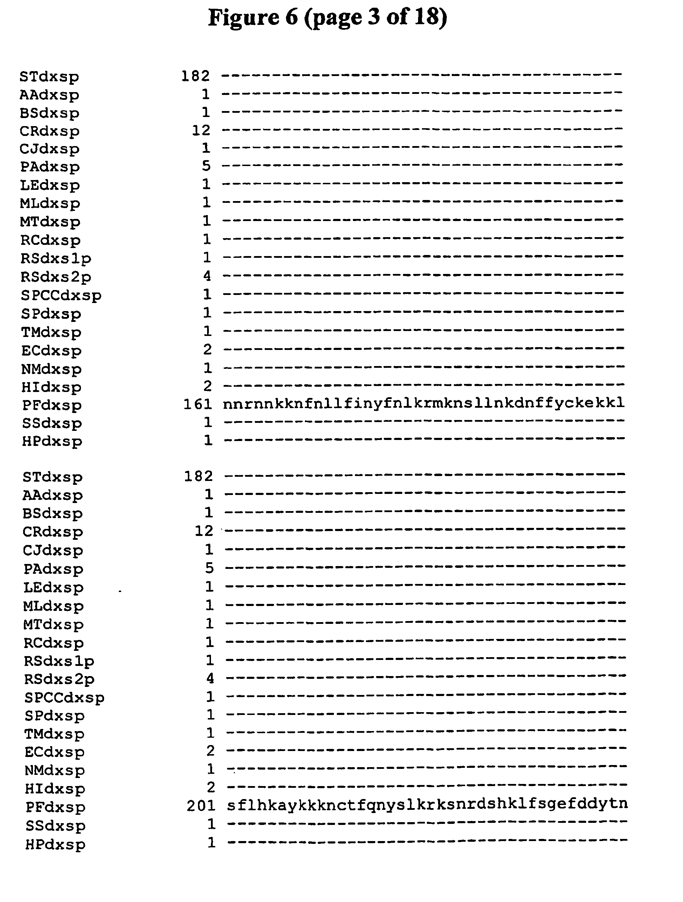

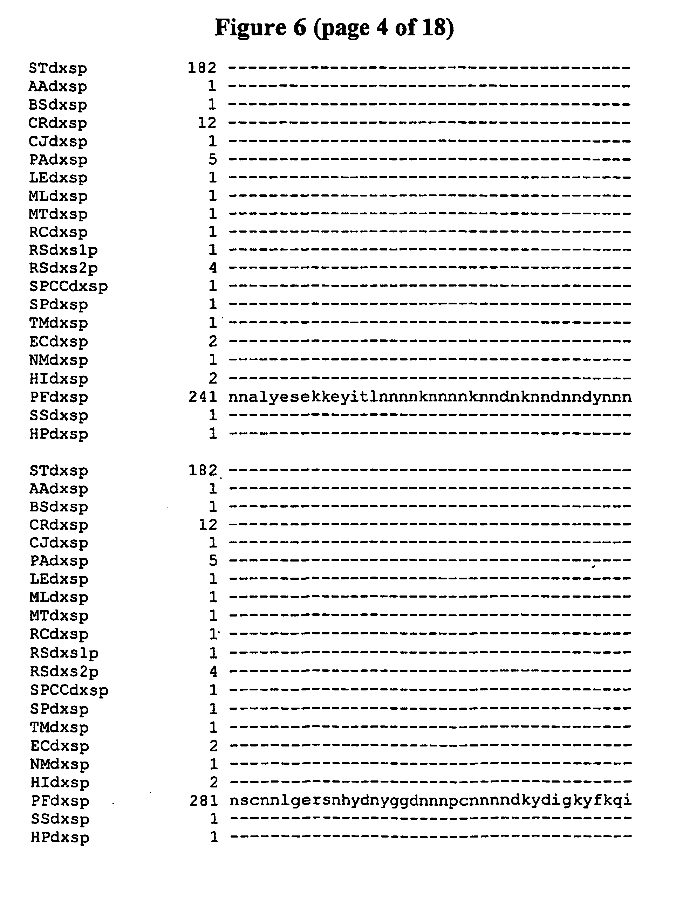

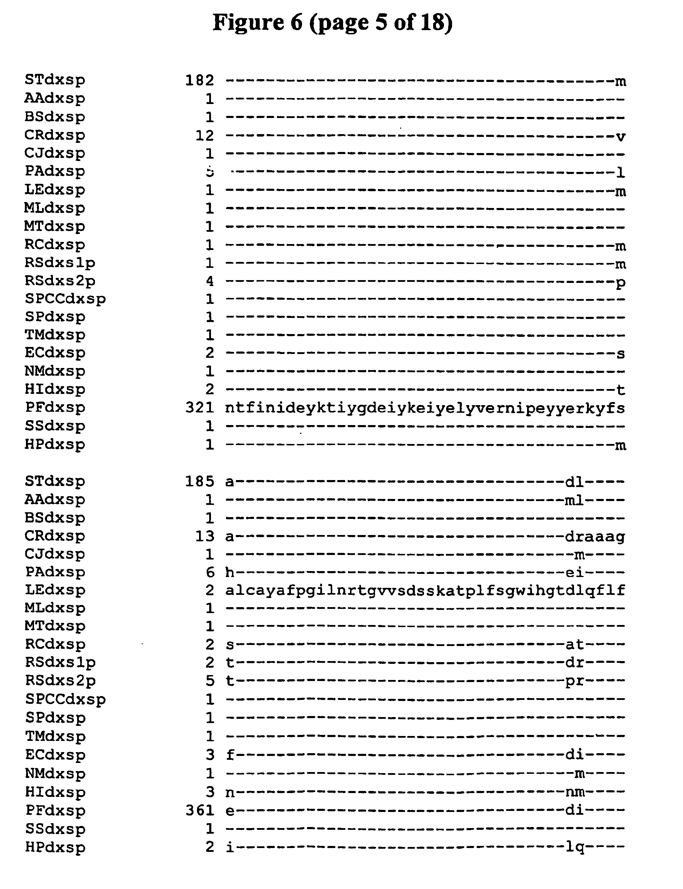

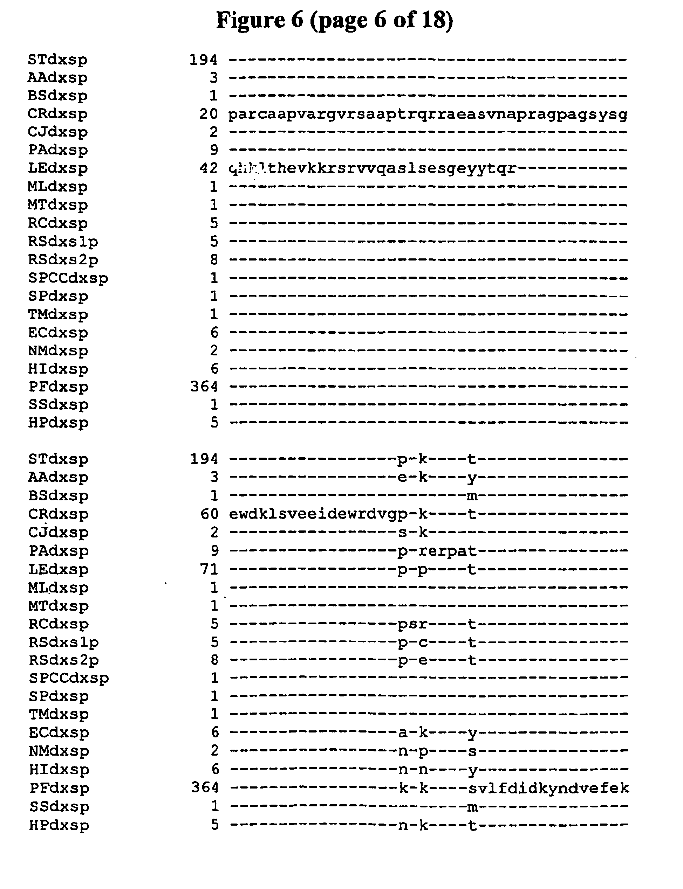

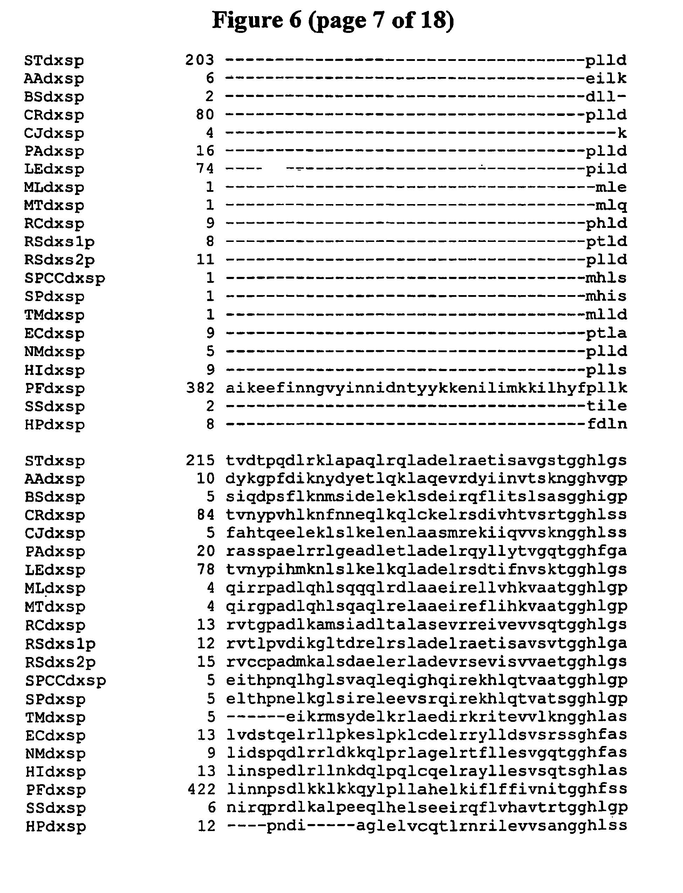

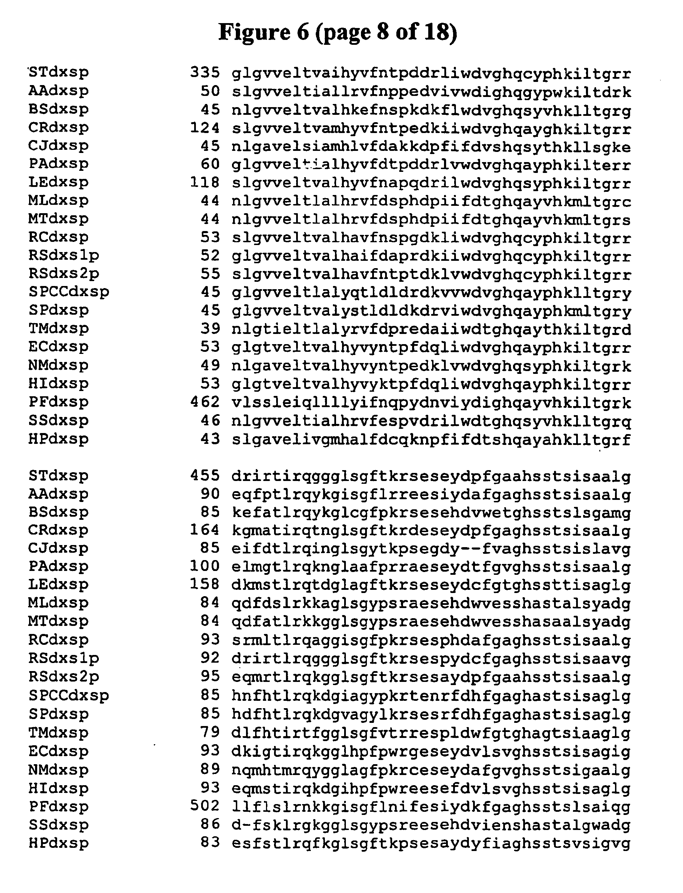

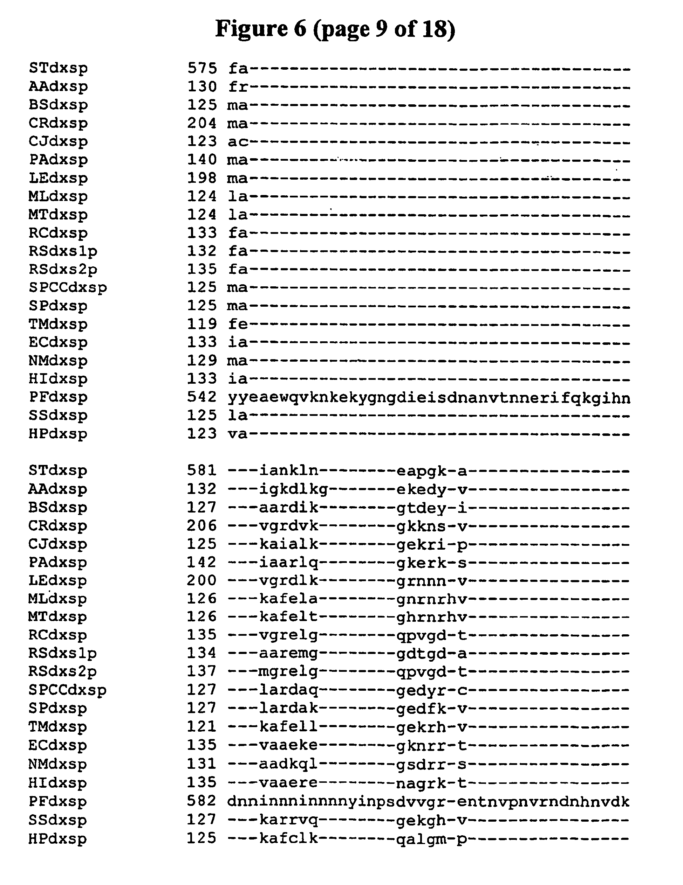

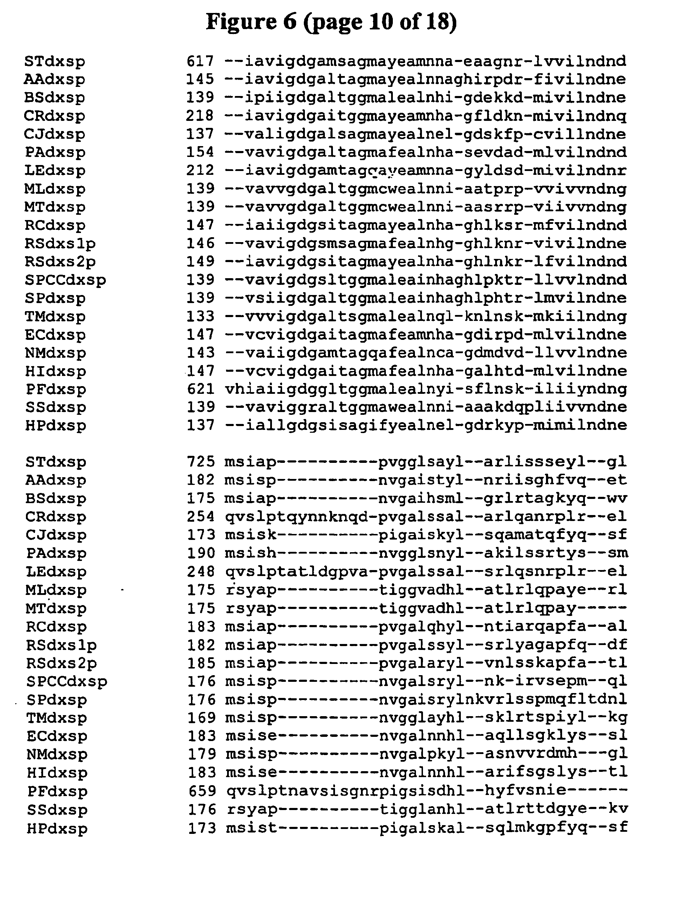

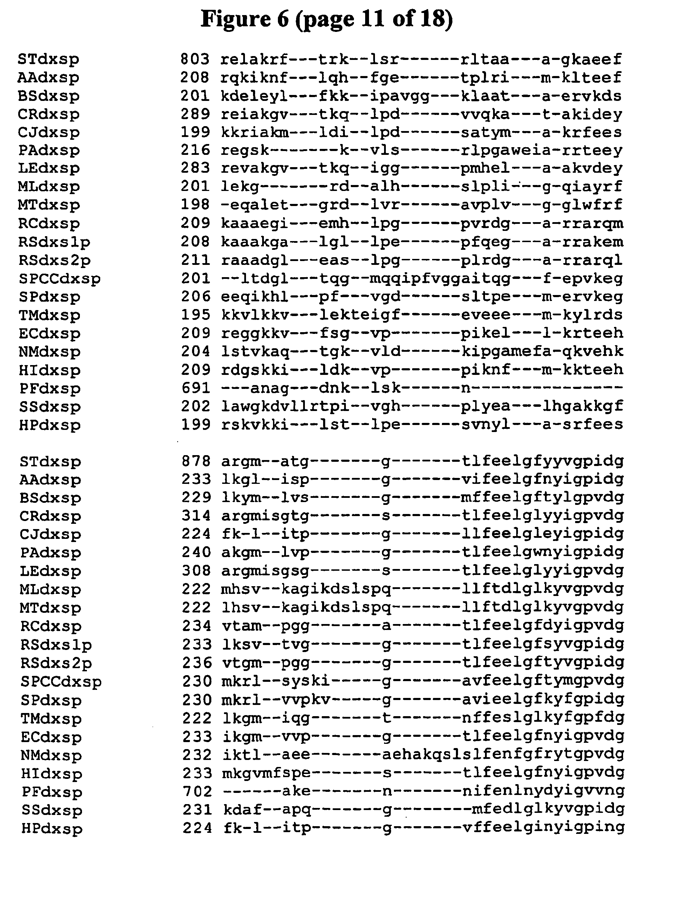

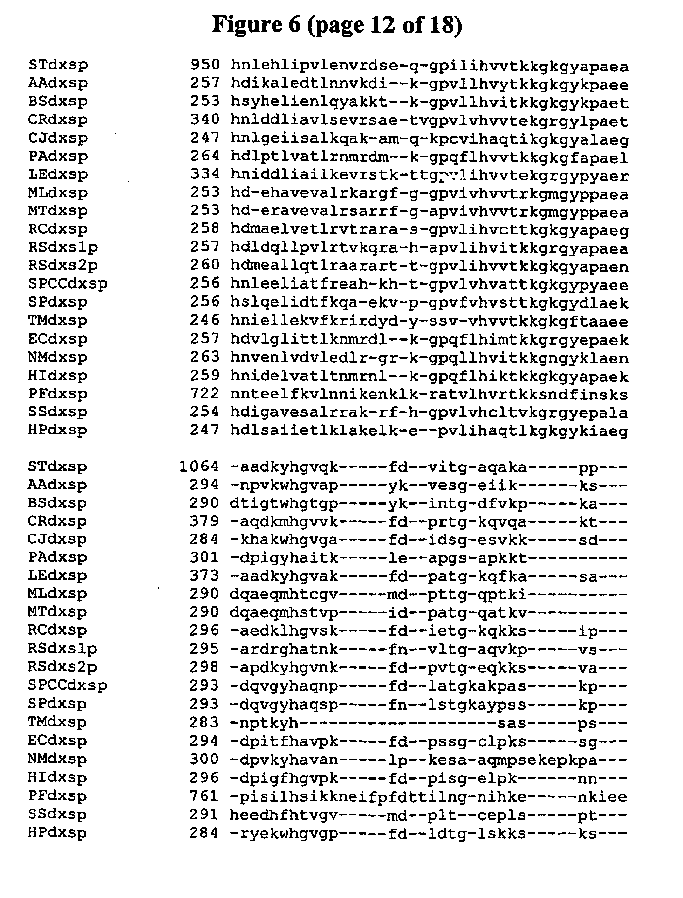

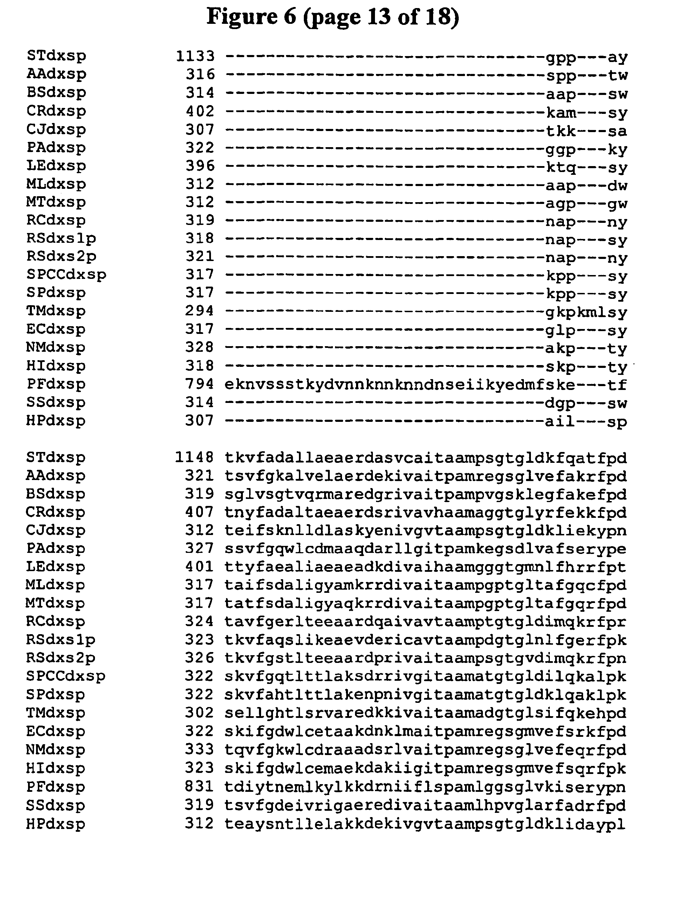

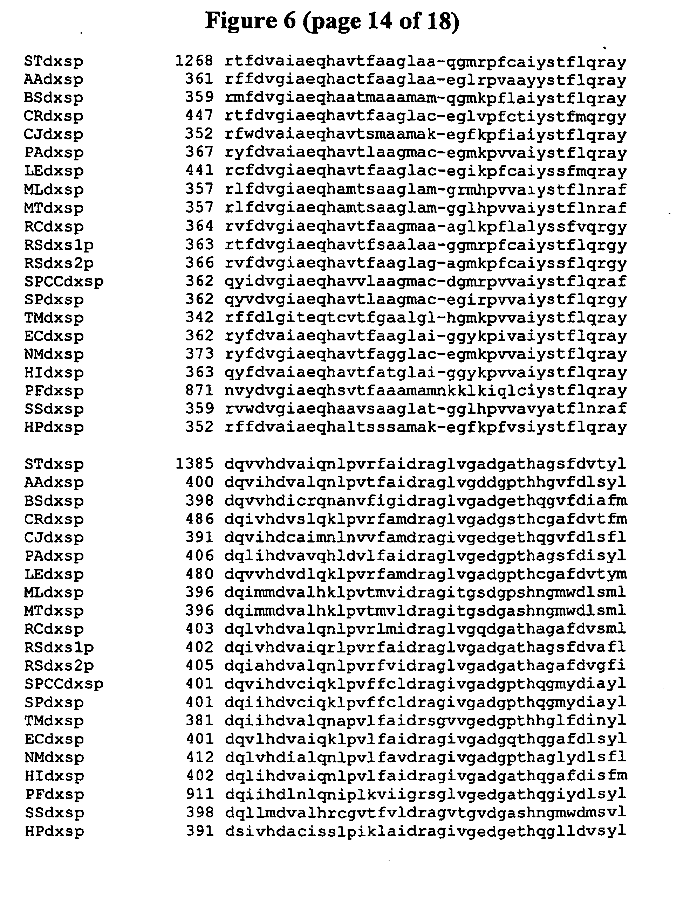

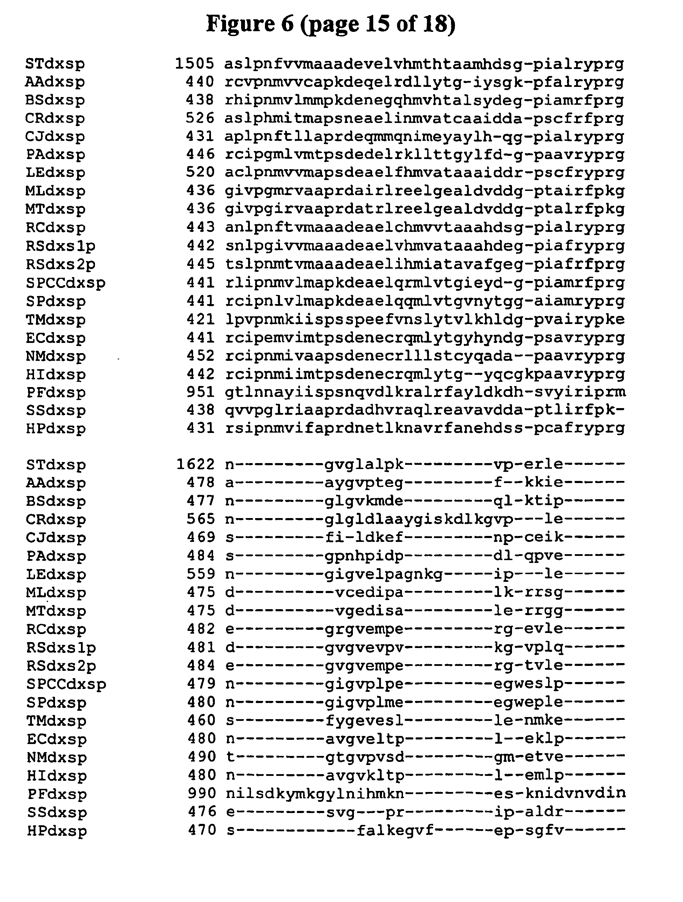

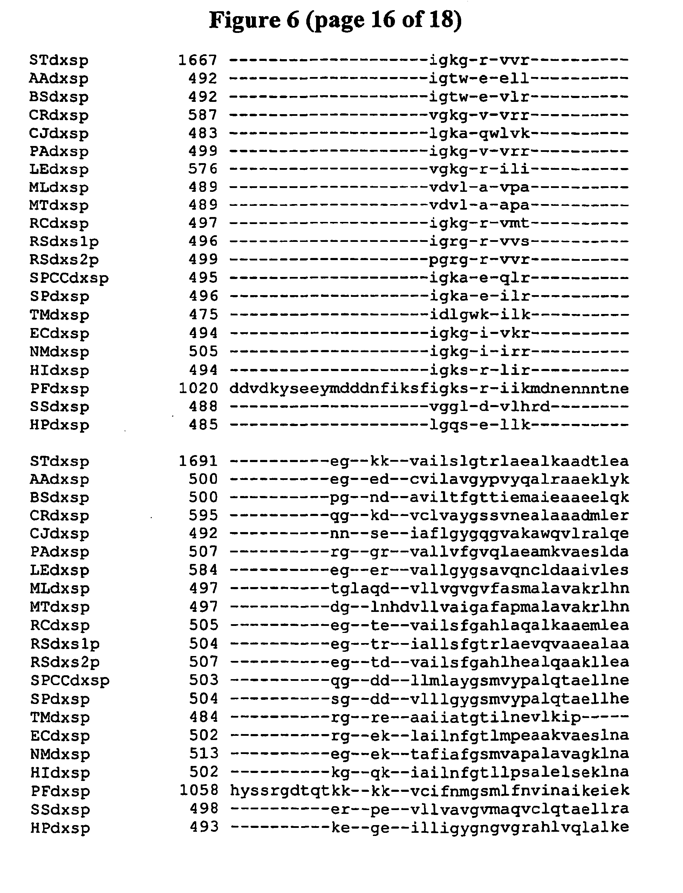

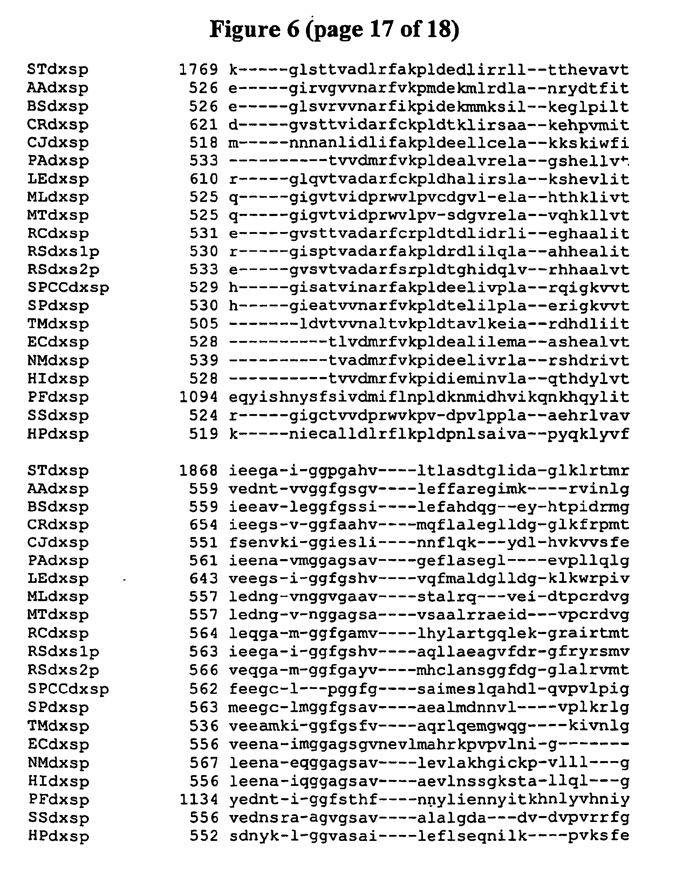

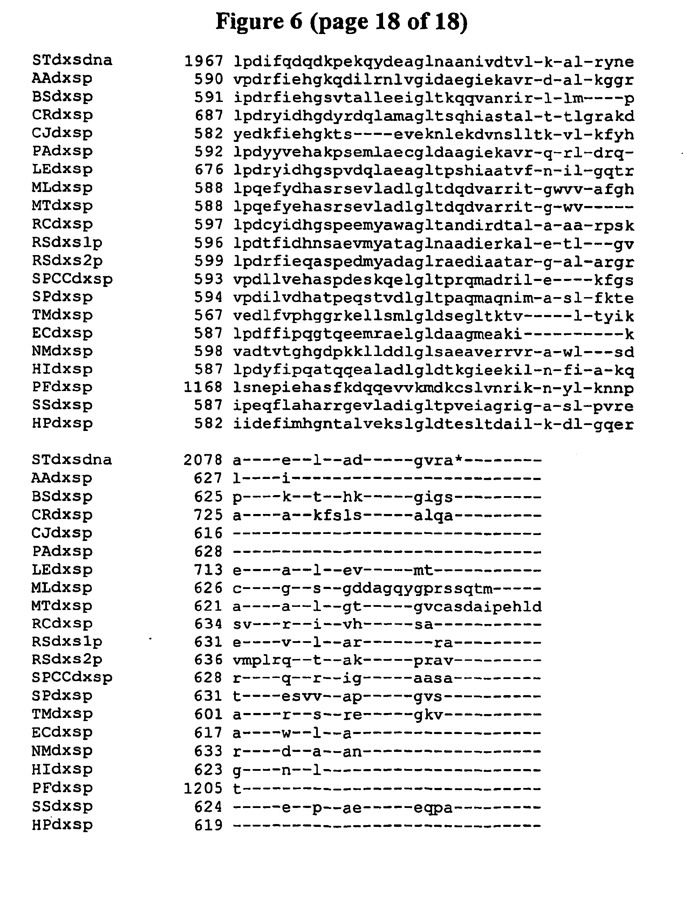

Figure 6 is a sequence pile-up of 21 amino acid sequences of polypeptides having DXS activity. STdxsp represents an amino acid sequence set forth in SEQ ID NO:3; AAdxsp represents an amino acid sequence from *Aquifex aeolicus* (GenBank accession number 067036; SEQ ID NO:18); BSdxsp represents an amino acid sequence from *Bacillus subtilis* (GenBank accession number P54523; SEQ ID NO:19); CRdxsp represents an amino acid sequence from *Chlamydomonas reinhardtii* (GenBank accession number CAA07554; SEQ ID NO:20); CJdxsp represents an amino acid sequence from *Campylobacter jejuni* (GenBank accession number CAB72788; SEQ ID NO:21); PAdxsp represents an amino acid sequence from *Pseudomonas aeruginosa* (GenBank accession number AAG07431; SEQ ID NO:15); LEdxsp represents an amino acid sequence from *Lycopersicon esculentum* (GenBank accession number AAD38941; SEQ ID NO:22); MLdxsp represents an amino acid sequence from *Mycobacterium leprae* (GenBank accession number Q50000; SEQ ID NO:23); MTdxsp represents an amino acid sequence from *Mycobacterium tuberculosis* (GenBank accession number CAB09493; SEQ ID NO:24); RCdxsp represents an amino acid sequence from *Rhodobacter capsulatus* (GenBank accession number P26242; SEQ ID NO:25); RSdxs1p represents an amino acid sequence encoded by a *Rhodobacter sphaeroides* dxs1 gene (SEQ ID NO:26); RSdxs2p represents an amino acid sequence encoded by a *Rhodobacter sphaeroides* dxs2 gene (SEQ ID NO:27); SPCCdxsp represents an amino acid sequence from *Synechococcus* PCC6301 (GenBank accession number CAB60078; SEQ ID NO:28); SPdxsp represents an amino acid sequence from *Synechocystis* PCC6803 (GenBank accession number P73067; SEQ ID NO:29); TMdxsp represents an amino acid sequence from *Thermotoga maritima* (GenBank accession number Q9X291; SEQ ID NO:30); ECdxsp represents an amino acid sequence from *Escherichia coli* (GenBank accession number D64771; SEQ ID NO:31); NMdxsp represents an amino acid sequence from *Neisseria meningitidis* (GenBank accession number CAB83880; SEQ ID NO:32); HIdxsp represents an amino acid sequence from *Haemophilus influenza* (GenBank accession number B64172; SEQ ID NO:33); PFdxsp represents an amino acid sequence from *Plasmodium falciparum* (GenBank accession number AAD03740; SEQ ED NO:34); SSdxsp represents an amino acid sequence from *Streptomyces sp.* CL190 (GenBank accession number BAA85847; SEQ ID NO:35); and HPdxsp represents an amino acid sequence from *Helicobacter pylori* 26695 (GenBank accession number AAD07422; SEQ ID NO:36).

Figure 7 is a listing of a nucleic acid sequence that encodes a *Rhodobacter sphaeroides* (ATCC 17023) polypeptide having DDS activity (SEQ ID NO:37). The start codon is the ATG at nucleotide number 372, and the stop codon is the TGA at nucleotide number 1373. The probable ribosome binding site is at nucleotide numbers 363-366. This sequence contains an open reading frame as well as 5' and 3' untranslated sequences.

Figure 8 is a listing of a nucleic acid sequence that encodes a *Rhodobacter sphaeroides* (ATCC 17023) polypeptide having DDS activity (SEQ ID NO:38). This sequence corresponds to the open reading frame.

Figure 9 is a listing of an amino acid sequence of a *Rhodobacter sphaeroides* (ATCC 17023) polypeptide having DDS activity (SEQ ID NO:39).

Figure 10 is a listing of a nucleic acid sequence that encodes a *Sphingomonas trueperi* (ATCC 12417) polypeptide having DDS activity (SEQ ID NO:40). The start - codon is the ATG at nucleotide number 605, and the stop codon is the TGA at nucleotide number 1618. The probable ribosome binding site is at nucleotide numbers 590-594. This sequence contains an open reading frame as well as 5' and 3' untranslated sequences.

Figure 11 is a listing of a nucleic acid sequence that encodes a *Sphingomonas trueperi* (ATCC 12417) polypeptide having DDS activity (SEQ ID NO:41). This sequence corresponds to the open reading frame.

Figure 12 is a listing of an amino acid sequence of a *Sphingomonas trueperi* (ATCC 12417) polypeptide having DDS activity (SEQ ID NO:42). This sequence corresponds to the open reading-frame.

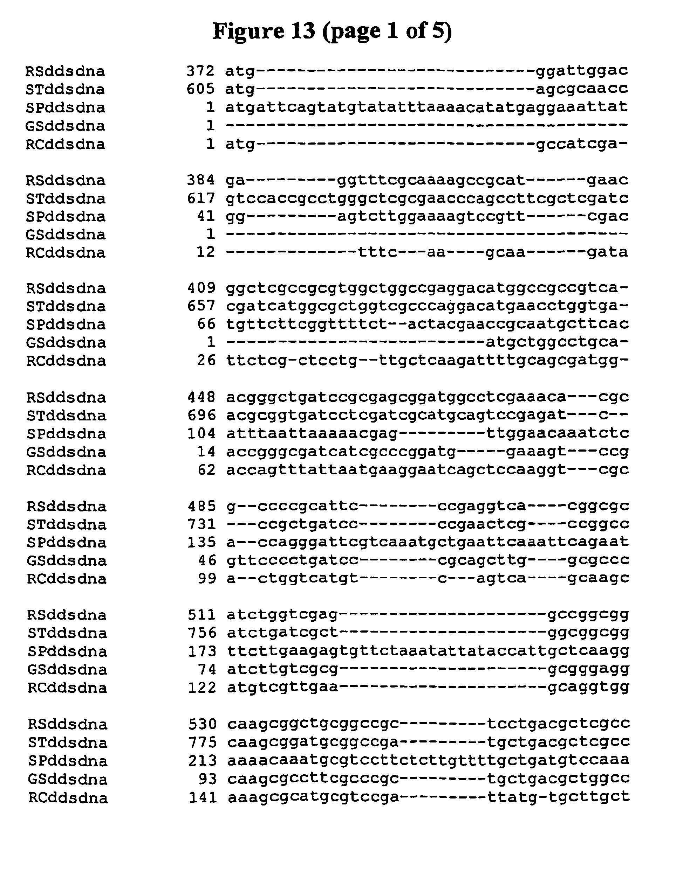

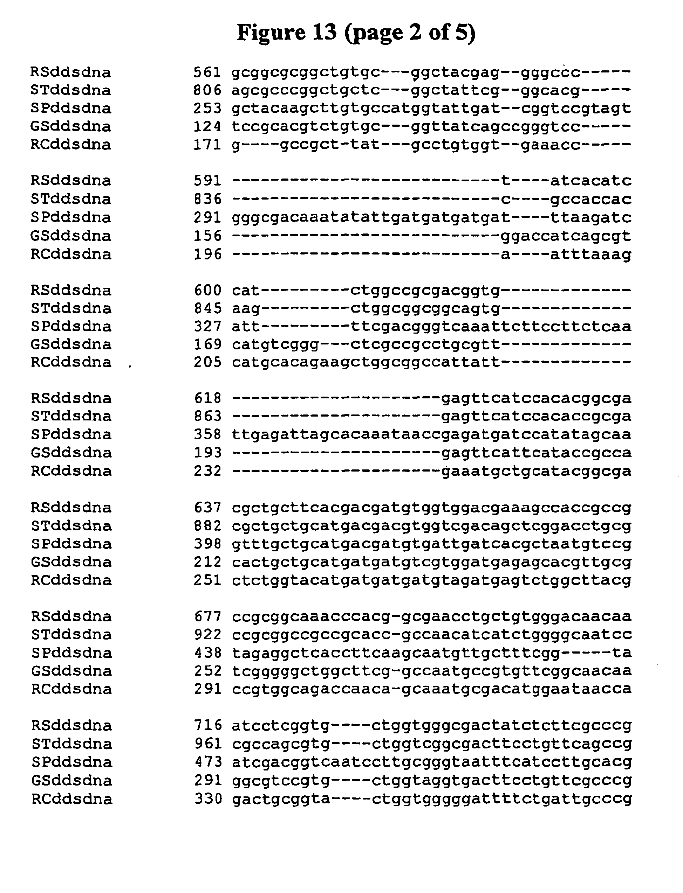

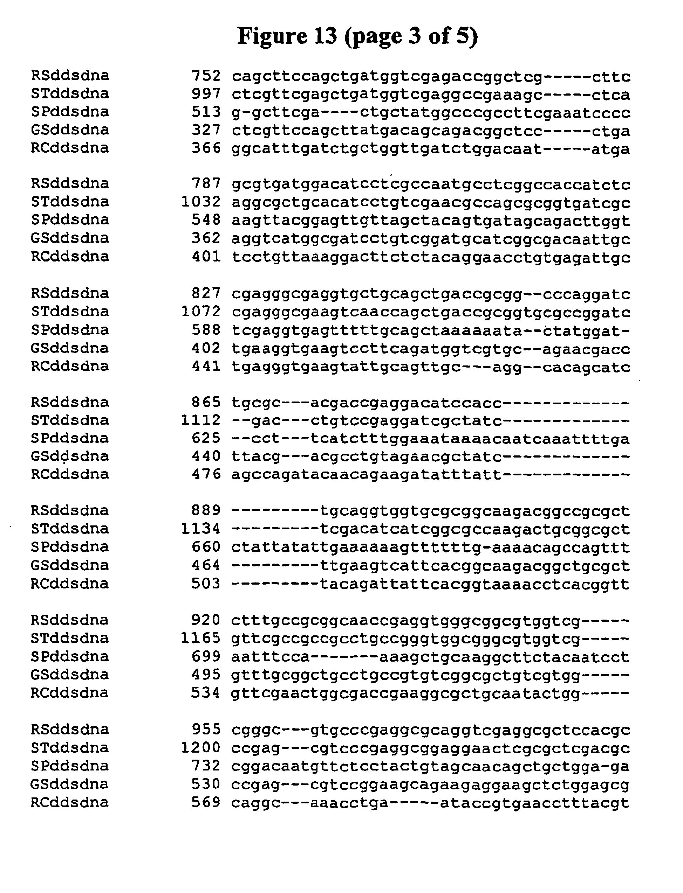

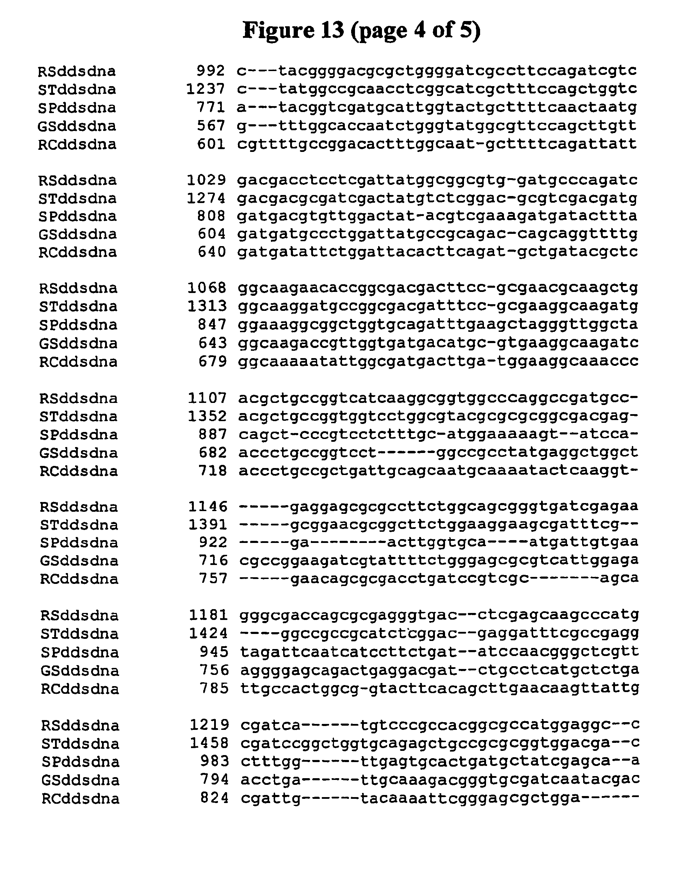

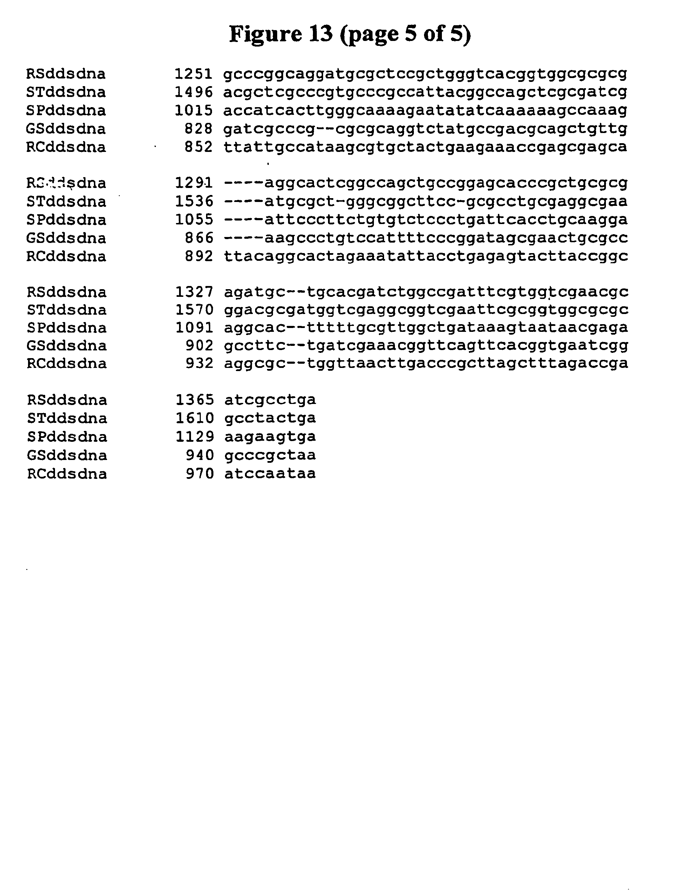

Figure 13 is a sequence pile-up of five nucleic acid sequences that encode polypeptides having DDS activity. RSddsdna represents the nucleic acid sequence set forth in SEQ ID NO:38; STddsdna represents the nucleic acid sequence set forth in SEQ ID NO:41; SPddsdna represents a nucleic acid sequence from *Schizosaccharomyces pombe* (GenBank accession number D84311; SEQ ID NO:43); GSddsdna represents a nucleic acid sequence from *Gluconobacter suboxydans* (GenBank accession number AB006850; SEQ ID NO:44); and RCddsdna represents a nucleic acid sequence from *Rhodobacter capsulatus* (U.S. Patent No. 6,103,488; SEQ ID NO:45).

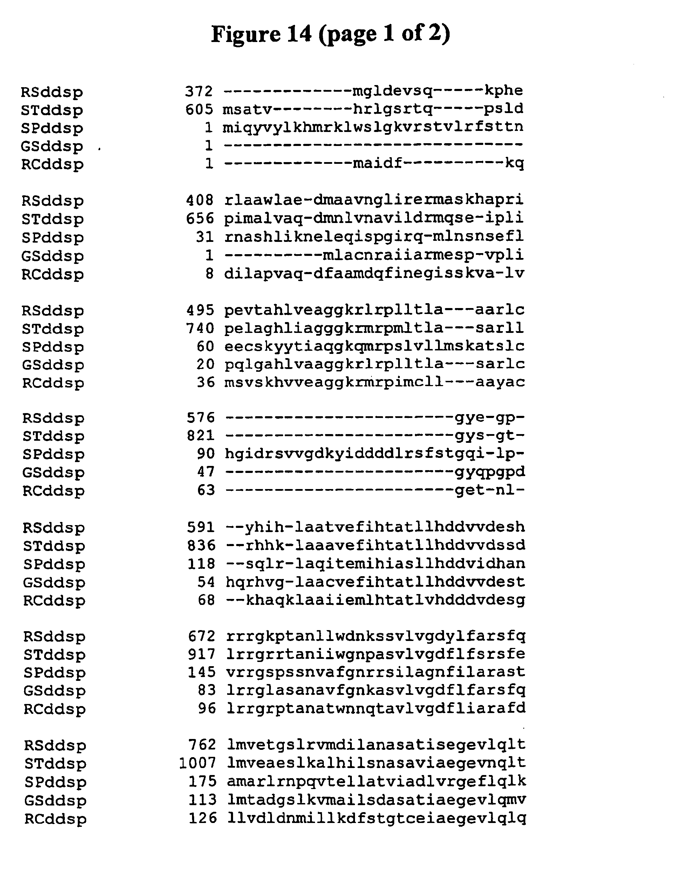

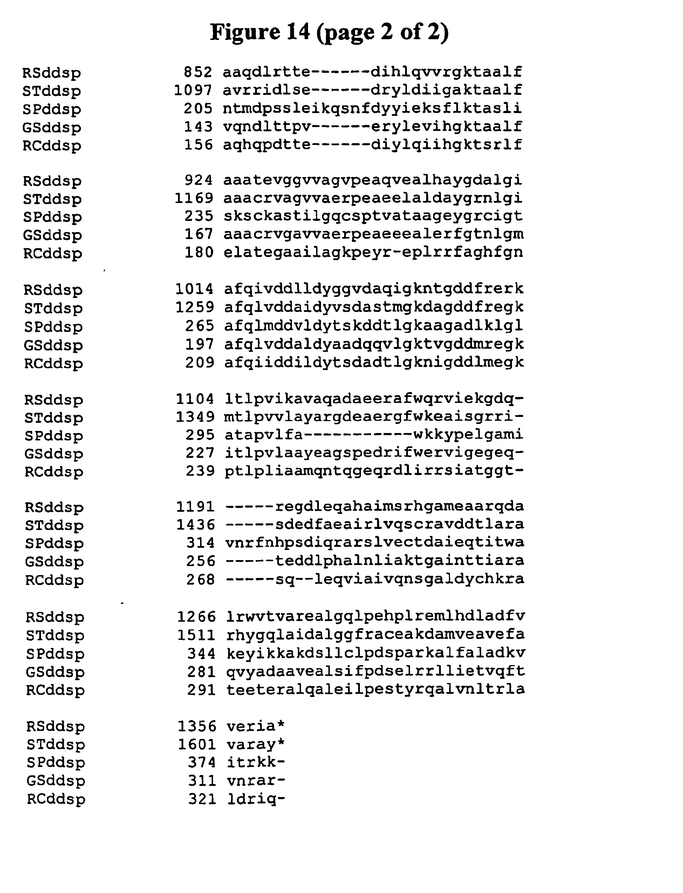

Figure 14 is a sequence pile-up of five amino acid sequences of polypeptides having DDS activity. RSddsp represents the amino acid sequence set forth in SEQ ID NO:39; STddsp represents the amino acid sequence set forth in SEQ ID NO:42; GSddsp represents an amino acid sequence from *Gluconobacter suboxydans* (GenBank accession number BAA32241; SEQ ID NO:46); SPddsp represents an amino acid sequence from *Schizosaccharomyces pombe* (GenBank accession number CAB66154; SEQ ID NO:47); and RCddsp represents an amino acid sequence from *Rhodobacter capsulatus* (U.S. Patent No. 6,103,488; SEQ ID NO:48).

Figure 15 is a sequence pile-up of three amino acid sequences of polypeptides having DXS activity. Hpdxsp represents the amino acid sequence set forth in SEQ ID NO:36; Ecdxsp represents the amino acid sequence set forth in SEQ ID NO:31; and Hidxsp represents the amino acid sequence set forth in SEQ ID NO:33.

Figure 16 is a sequence pile-up of four amino acid sequences of polypeptides having DDS, ODS (octaprenyl diphosphate synthase), or SDS (solanesyl diphosphate synthase) activity. Rcsdsp represents an amino acid sequence from *Rhodobacter capsulatus* having SDS activity (SEQ ID NO:49); Rpodsp represents an amino acid sequence from *Rickettsia prowazeki* having ODS activity (SEQ ID NO:50); Gsddsp represents the amino acid sequence set forth in SEQ ID NO:46; and Ecodsp represents an amino acid sequence from *Escherichia coli ispB* having ODS activity (SEQ ID NO:51).

Figure 17 is a sequence pile-up of five amino acid sequences of polypeptides having DDS, ODS, or SDS activity. Rpodsp represents the amino acid sequence set forth in SEQ ID NO:50; Gsddsp represents the amino acid sequence set forth in SEQ ID NO:46; Ecodsp represents the amino-acid sequence set forth in SEQ ID NO:51; Hiodsp represents an amino acid sequence from *Haemophilus influenze* having ODS activity (SEQ ID NO:52); and Rcsdsp represents the amino acid sequence set forth in SEQ ID NO:49.

Figure 18 is a diagram of a construct designated appUC18-SHDXS.

Figure 19 is a diagram of a construct designated appUC18-RSdds.

Figure 20 is a diagram of a construct designated appUC18-SHDDS.

Figure 21 is a mass chromatogram obtained from a MG1655 PUC18 specimen.

Figure 22 is a mass chromatogram obtained from a MG1655 PUC18-DDS specimen.

Figure 23 is a mass spectra obtained from a MG1655 PUC18 specimen.

Figure 24 is a mass spectra obtained from a MG1655 PUC18-DDS specimen.

Figure 25 is a mass spectra obtained from a MG1655 PUC18-DDS specimen.

Figure 26 is a graph plotting length and percent identity with points A, B, C, and D defining an area indicated by shading.

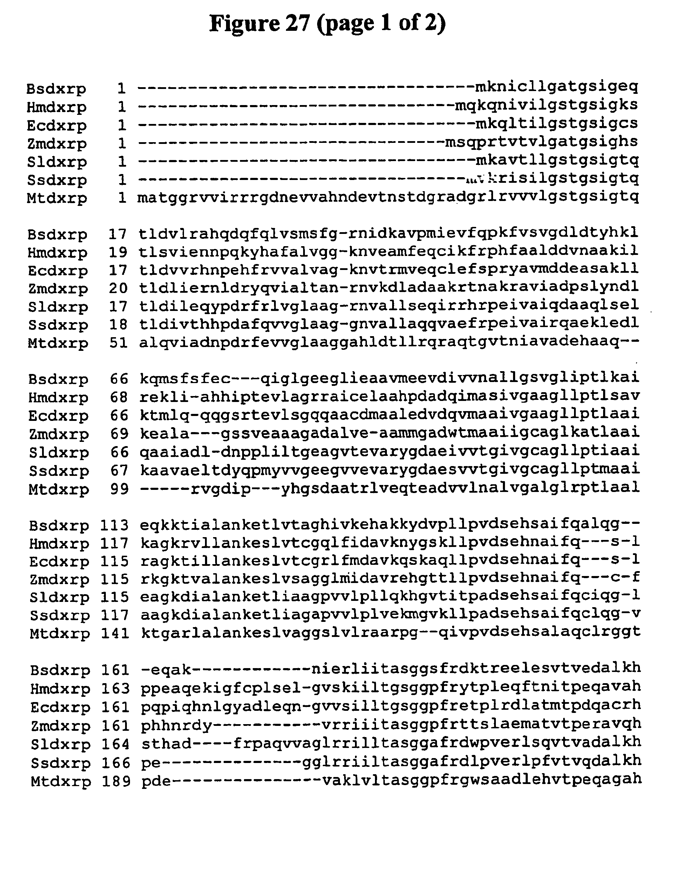

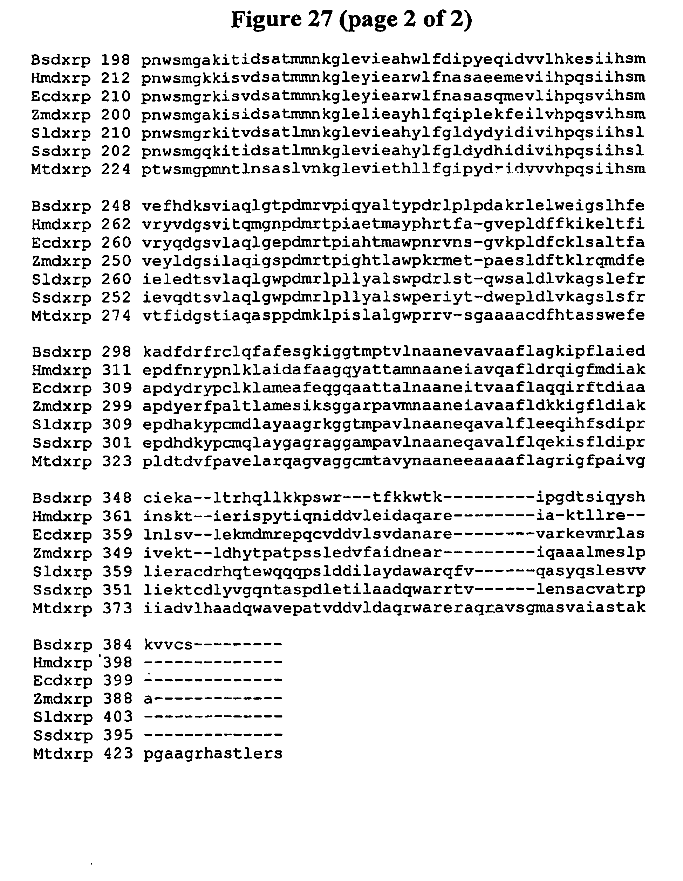

Figure 27 is a sequence pile-up of seven amino acid sequences of polypeptides having DXR activity. Bsdxrp represents an amino acid sequence from *Bacillus subtilis* (SEQ ID NO:98); Hmdxrp represents an amino acid sequence from *Haemophilus influenzae* (SEQ ID NO:99); Ecdxrp represents an amino acid sequence from *Escherishia coli* (SEQ ID NO: 100); Zmdxrp represents an amino acid sequence from *Zymonas mobilis* (SEQ ID NO:101); Sldxrp represents an amino acid sequence from *Synechococcus leopoliensis* (SEQ ID NO: 102); Ssdxrp represents an amino acid sequence from *Synechocystis sp.* PCC6803 (SEQ ID NO: 103); and Mtdxrp represents an amino acid sequence from *Mycobacterium tuberculosis* (SEQ ID NO: 104),

Figure 28 is a listing of a nucleic acid sequence that encodes a *Sphingomonas trueperi* polypeptide having DXR activity (SEQ ID NO:95). The start codon is the GTG at either nucleotide number 575 or 578, and the stop codon is the TGA at nucleotide number 1733. This sequence contains an open reading frame as well as 5' and 3' untranslated sequences.

Figure 29 is a listing of a nucleic acid sequence that encodes a *Sphingomonas trueperi* polypeptide having DXR activity (SEQ ID NO:96). This sequence corresponds to the open reading frame. -

Figure 30 is a listing of an amino acid sequence of a *Sphingomonas trueperi* polypeptide having DXR activity (SEQ ID NO:97).

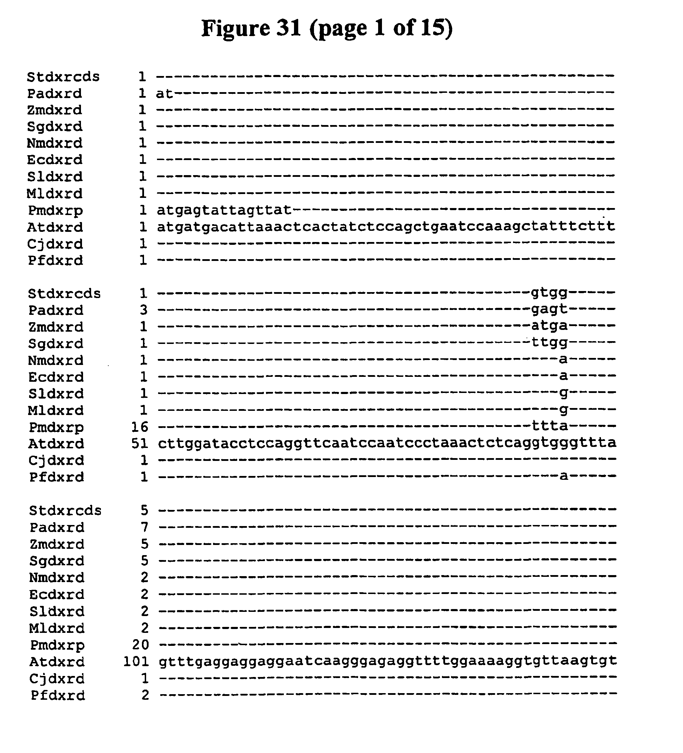

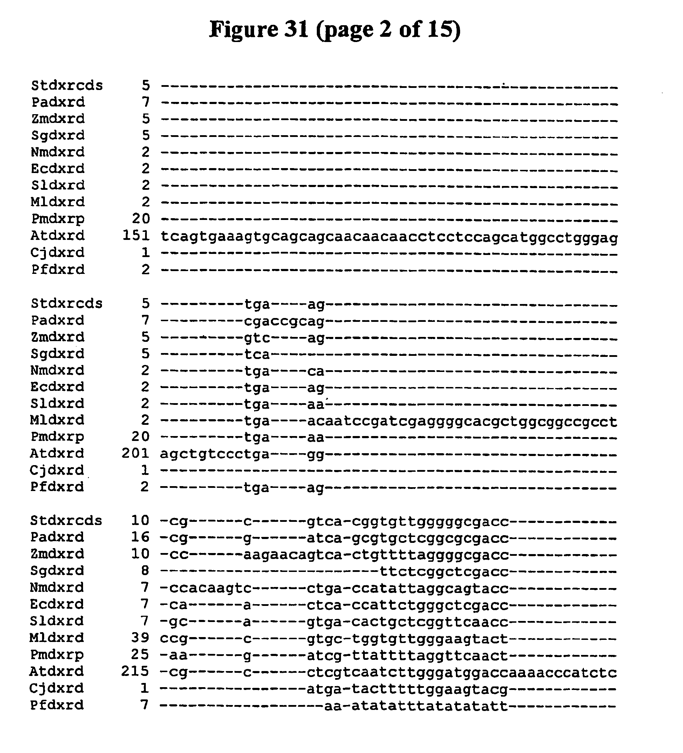

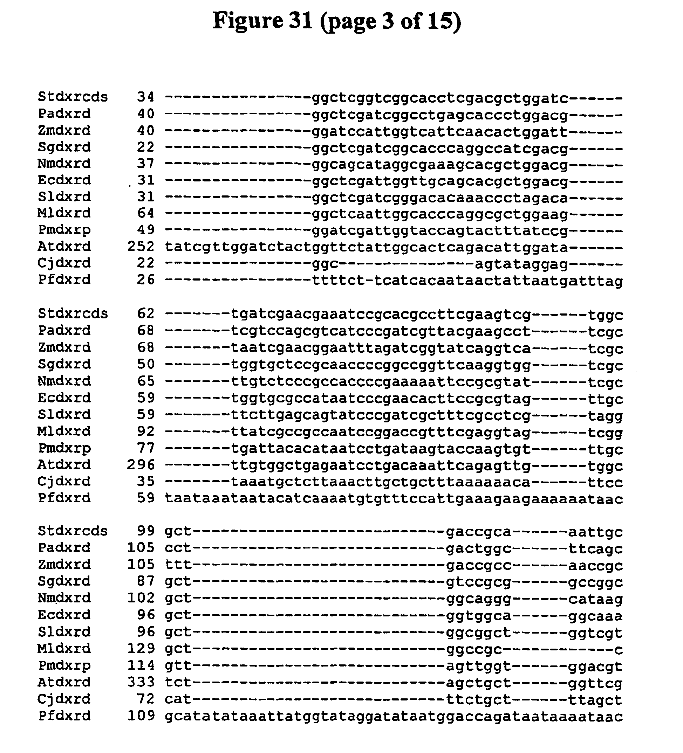

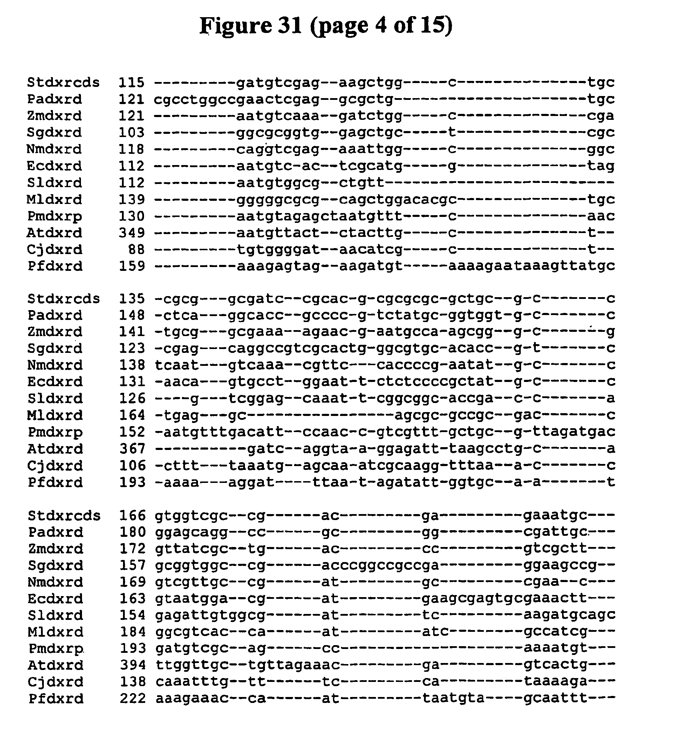

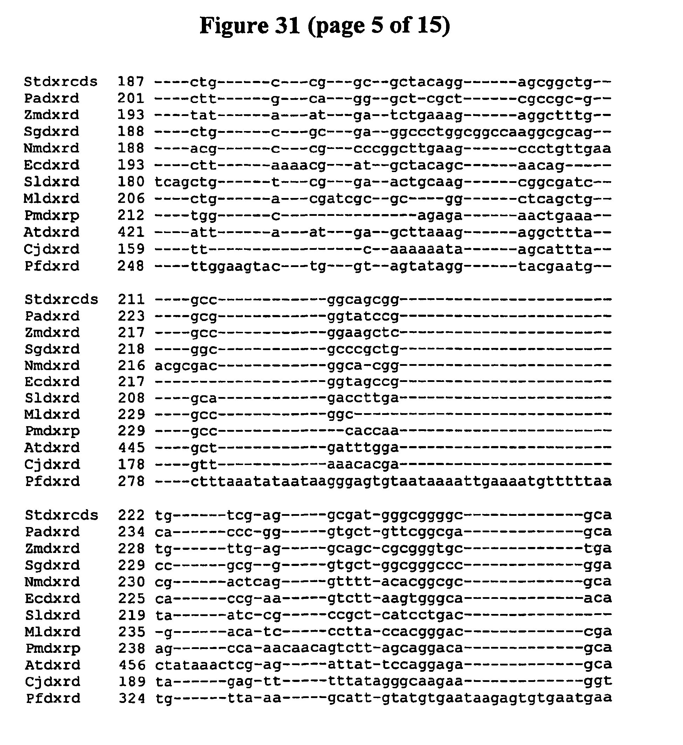

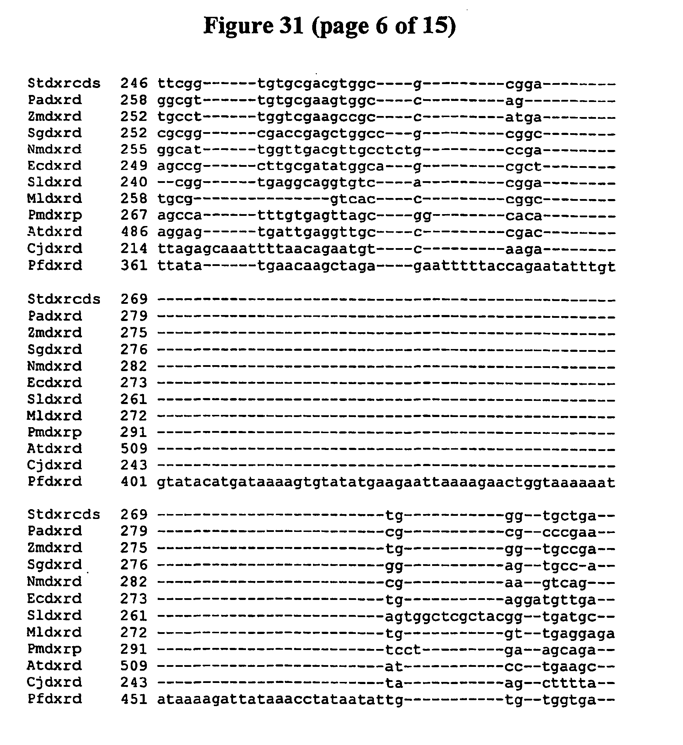

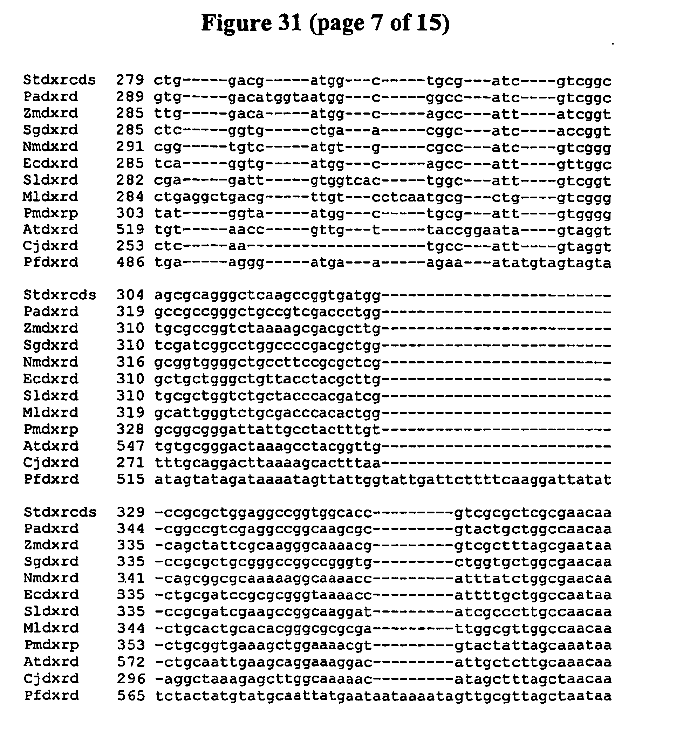

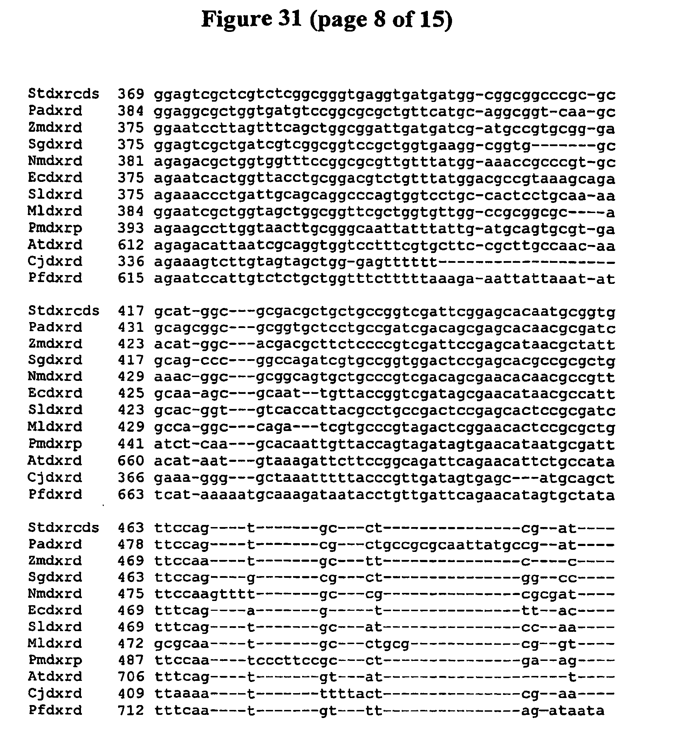

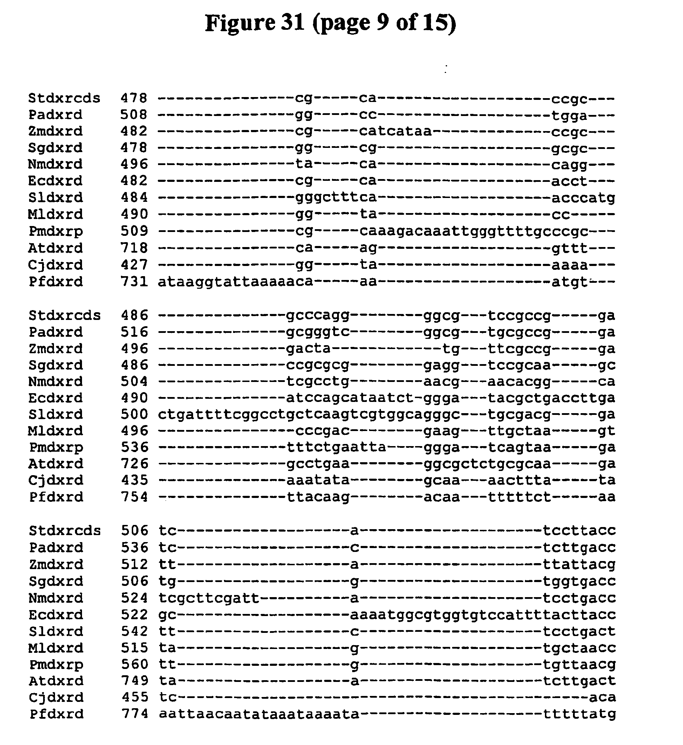

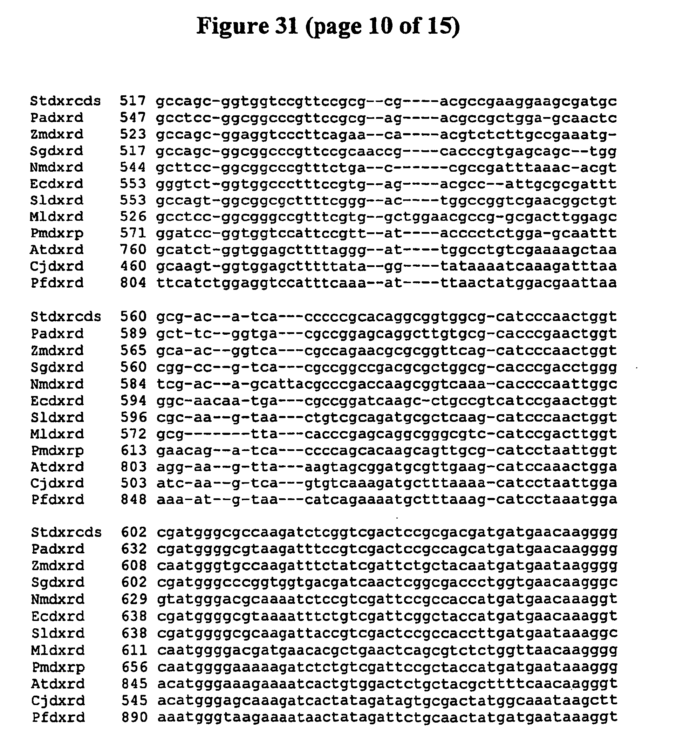

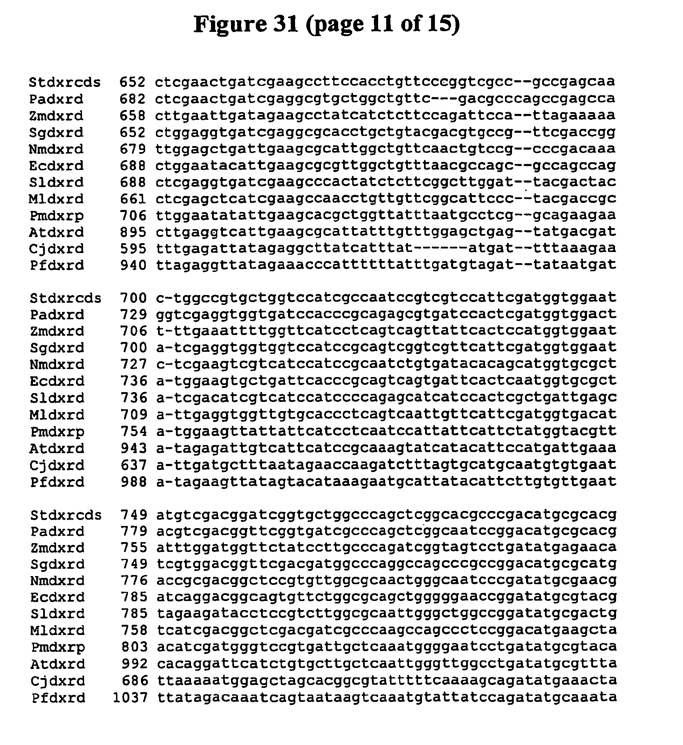

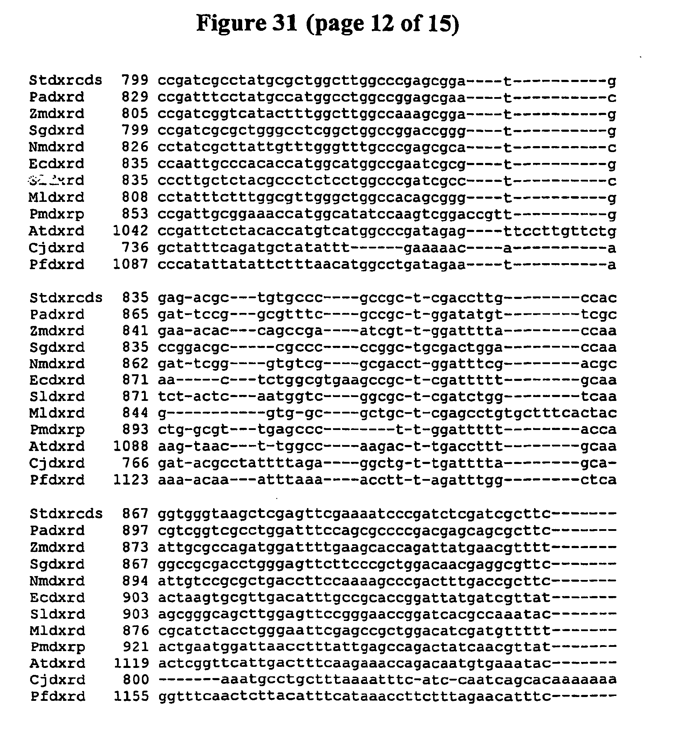

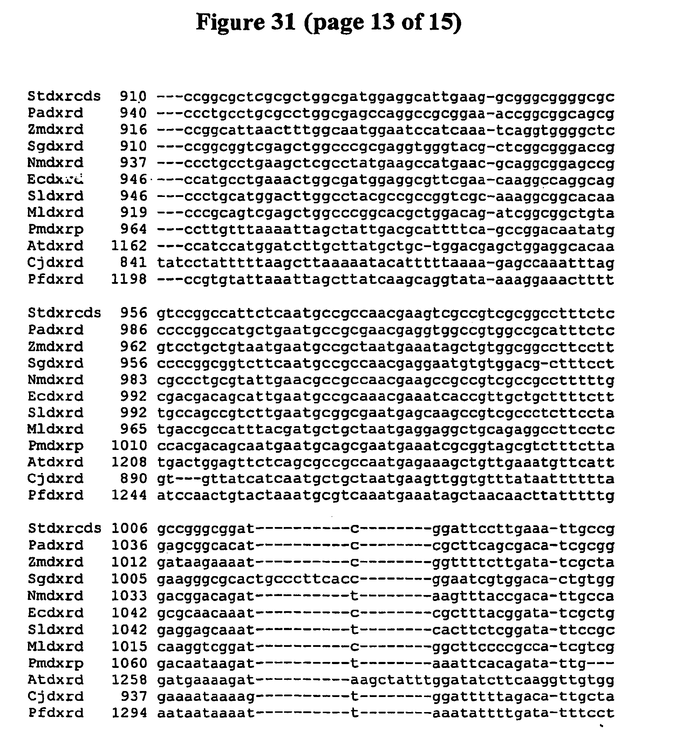

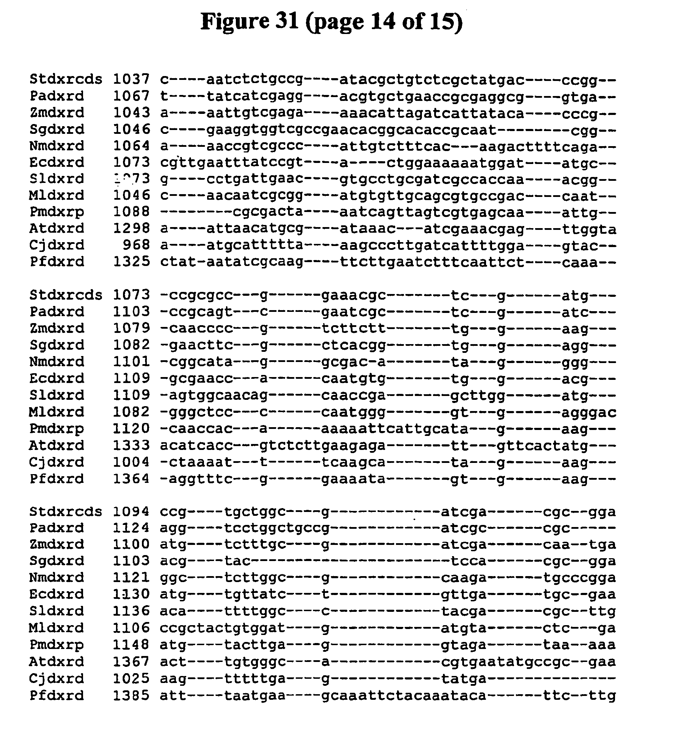

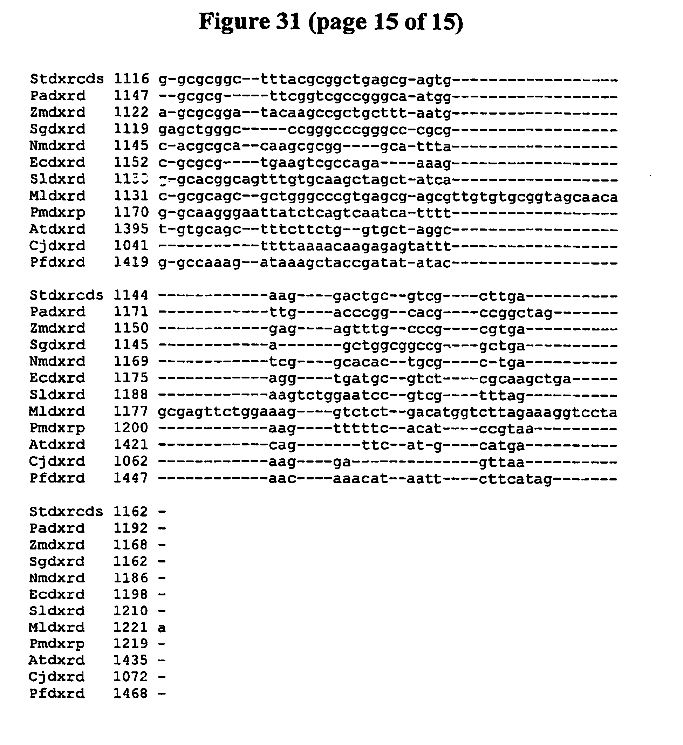

Figure 31 is a sequence pile-up of twelve nucleic acid sequences that encode polypeptides having DXR activity. Stdxrcds represents the nucleic acid sequence set forth in SEQ ID NO:96; Padxrd represents a nucleic acid sequence from *Pseudomonas aeruginosa* (SEQ ID NO:105); Zmdxrd represents a nucleic acid sequence from *Zygomonas mobilis* (SEQ ID NO:106); Sgdxrd represents a nucleic acid sequence from *Streptomyces griseolosporeus* (SEQ ID NO:107); Nmdxrd represents a nucleic acid sequence from *Neisseria meningitidis* (SEQ ID NO:108); Ecdxrd represents a nucleic acid sequence from *Escherishia coli* (SEQ ID NO:109); Sldxrd represents a nucleic acid sequence from *Synechococcus leopoliensis* (SEQ ID NO:110); Mldxrd represents a nucleic acid sequence from *Mycobacterium leprae* (SEQ ID NO:111); Pmdxrd represents a nucleic acid sequence from *Pasteurella multocida* (SEQ ID NO:112); Atdxrd represents a nucleic acid sequence from *Arabidopsis thaliana* (SEQ ID NO:113); Cjdxrd represents a nucleic acid sequence from *Campylobacter jejuni* (SEQ ID NO:114); and Pfdxrd represents a nucleic acid sequence from *Plasmodium falciparum* (SEQ ID NO:115).

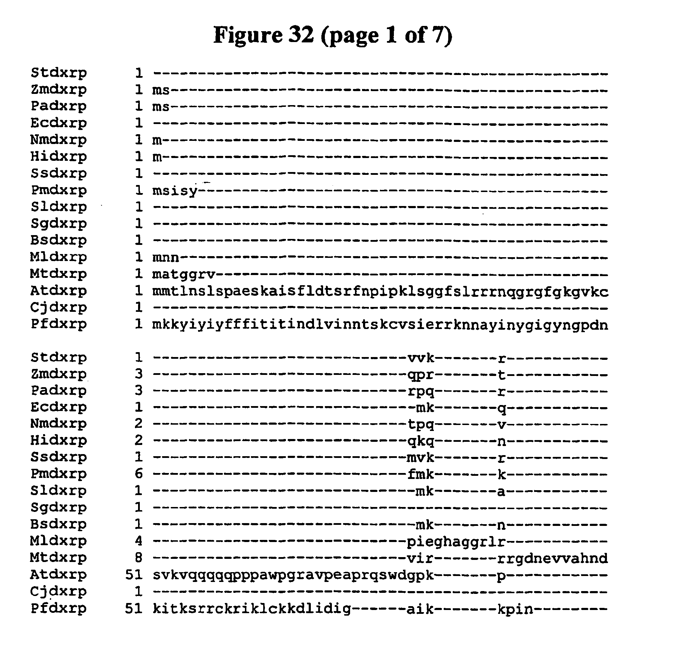

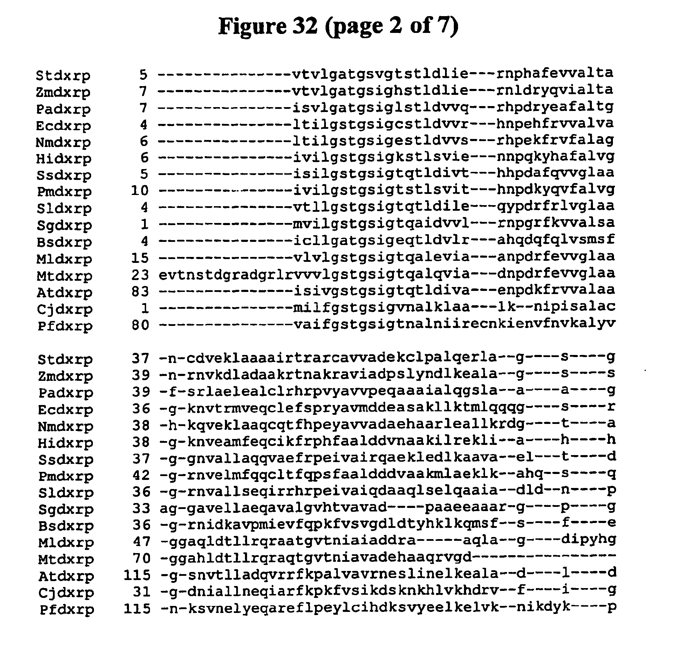

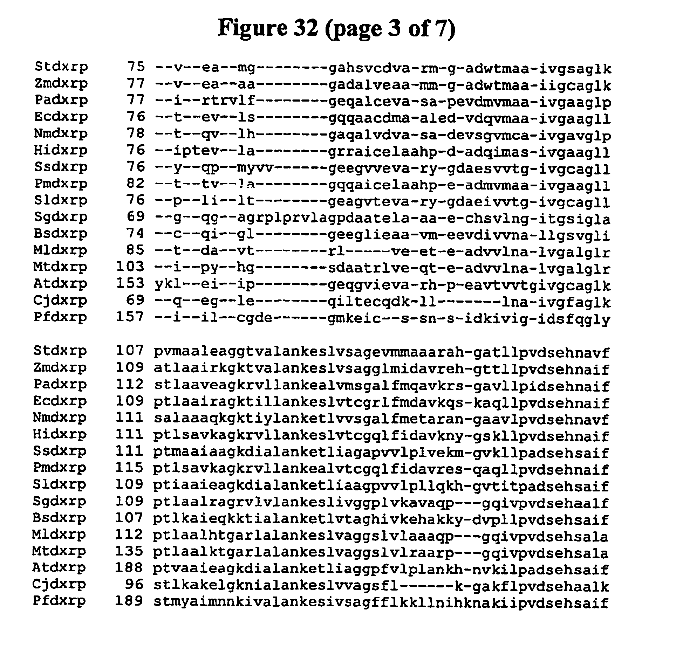

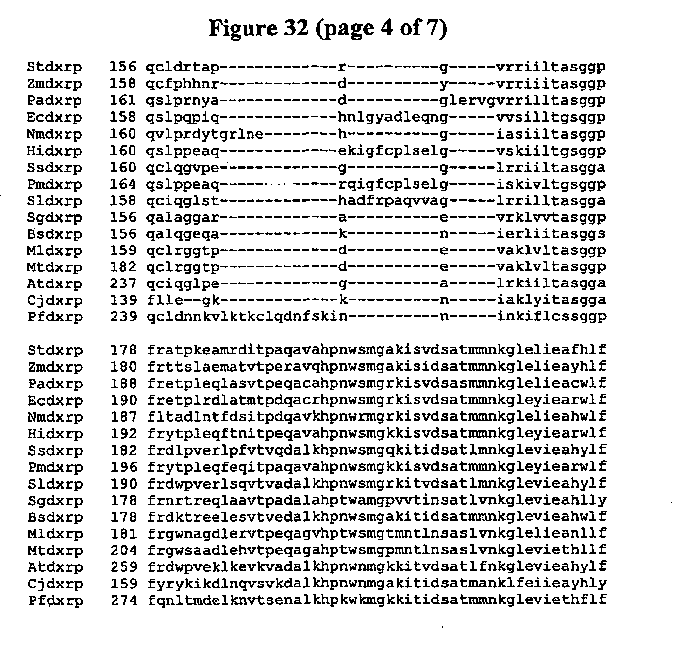

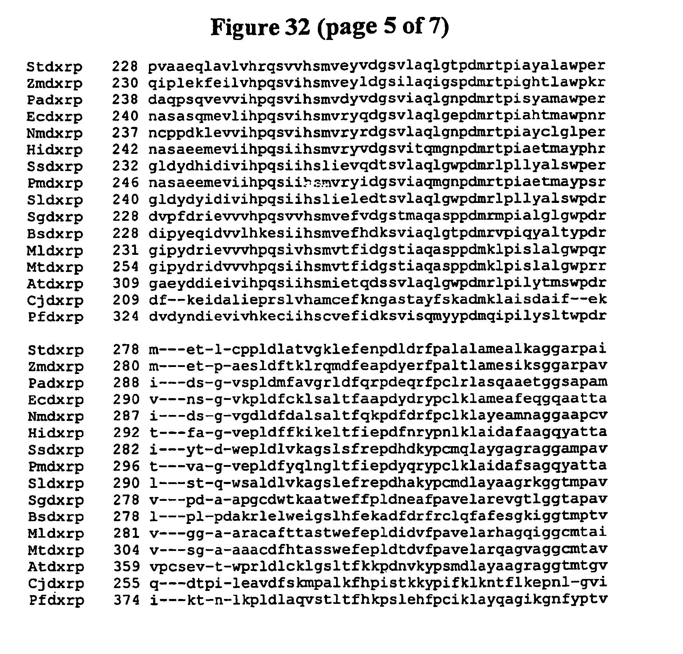

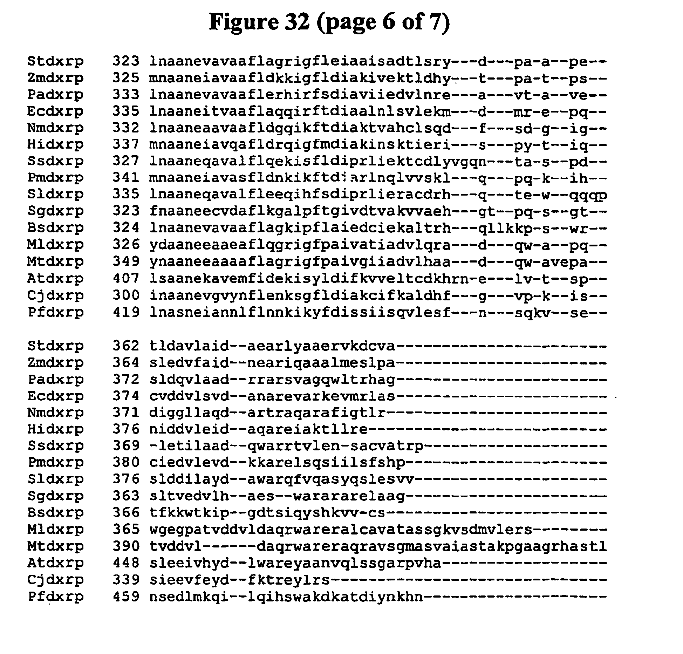

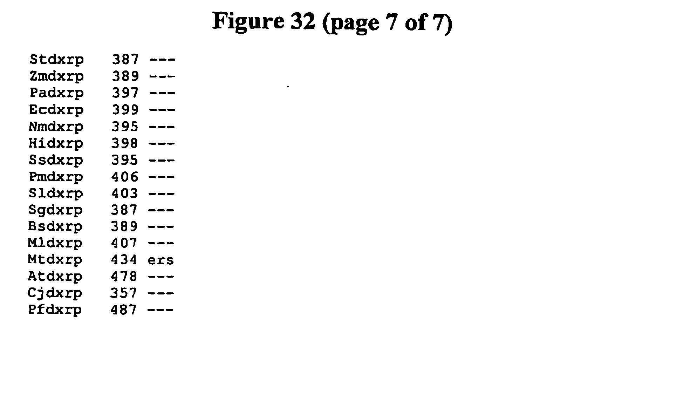

Figure 32 is a sequence pile-up of sixteen amino acid sequences of polypeptides having DXR activity. Stdxrp represents the amino acid sequence set forth in SEQ ID NO:97; Zmdxrp represents an amino acid sequence from *Zymononas mobilis* (SEQ ID NO:116); Padxrp represents an amino acid sequence from *Pseudomonas aeruginosa* (SEQ ID NO:117); Ecdxrp represents an amino acid sequence from *Escherishia coli* (SEQ ID NO:118); Nmdxrp represents an amino acid sequence from *Neisseria meningitidis* (SEQ ID NO:119); Hidxrp represents an amino acid sequence from *Haemophilus influenzae* (SEQ ID NO:120); Ssdxrp represents an amino acid sequence from *Synechocystis sp.* PCC6803 (SEQ ID NO:121); Pmdxrp represents an amino acid sequence from *Pasteurella multocida* (SEQ ID NO:122); Sldxrp represents an amino acid sequence from *Synechococcus leopoliensis* (SEQ ID NO:123); Sgdxrp represents an amino acid sequence from *Streptomyces griseolosporeus* (SEQ ID NO:124); Bsdxrp represents an amino acid sequence from *Bacillus subtilis* (SEQ ID NO:125); Mldxrp represents an amino acid sequence from *Mycobacterium leprae* (SEQ ID NO:126); Mtdxrp represents an amino acid sequence from *Mycobacterium tuberculosis* (SEQ ID NO:127); Atdxrp represents an amino acid sequence from *Arabidopsis thaliana* (SEQ ID NO:128); Cjdxrp represents an amino acid sequence from *Campylobacter jejuni* (SEQ ID NO:130); and Pfdxrp represents an amino acid sequence from *Plasmodium falciparum* (SEQ ID NO:131).

## DETAILED DESCRIPTION

Described herein are methods and materials related to the production of isoprenoids Specifically, described herein are isolated nucleic acids, substantially pure polypeptides, host cells, and methods and materials for producing various isoprenoid compounds. The invention provides methods and materials related to the production of CoQ(10), as defined in the claims. For the purpose of this invention, an isoprenoid compound is any compound containing a five-carbon isoprenoid unit. Examples of isoprenoid compounds include, without limitation, carotenoids, isoprenes, sterols, terpenes, and ubiquinones. Such isoprenoid compounds can be used in a wide range of applications. For example, isoprenoid compounds produced as described herein can be used in industrial, pharmaceutical, or cosmetic products.

In general terms, carotenoids are lipophilic pigments typically found in photosynthetic plants and bacteria.

Examples of carotenoids include, without limitation, carotenes, xanthophylls, hydrocarbon carotenoids, hydroxy carotenoid derivatives, epoxy carotenoid derivatives, furanoxy carotenoid derivatives, and oxy carotenoid derivatives. Isoprenes are oily hydrocarbons that can be obtained by distilling caoutchouc or guttaipercha. Examples of isoprenes include, without limitation, rubber, vitamin A, and vitamin K. Sterols are steroid-based alcohols typically having a hydrocarbon side-chain of eight to ten carbon atoms at the 17-beta position and a hydroxyl group at the 3-beta position. Examples of sterols include, without limitation, ergosterol, cholesterol, and stigmasterol. Terpenes are lipid species typically found in plants in great abundance. Examples of terpenes include, without limitation, dolichol, squalene, and limonene. Ubiquinones are 2,3-dimethoxy-5-methylbenzoquinone derivatives having a side chain containing at least one isoprenoid unit. Typically, ubiquinone is referred to as Coenzyme Q (CoQ). In addition, the number of isoprenoid units of a side chain of a particular ubiquinone is used to identify that particular ubiquinone. For example, a ubiquinone with six isoprenoid units is referred to as CoQ(6), while a ubiquinone with ten isoprenoid units is referred to as CoQ(10). It is noted that CoQ(10) also is referred to as ubidecarenone. Examples of ubiquinones include, without limitation, CoQ(6), CoQ(8), CoQ(10), and CoQ(12).

Isoprenoid compounds can be pyruvate-derived products. The term "pyruvate-derived product" as used herein refers to any compound that is synthesized from pyruvate within no more than 25 enzymatic steps. Thus, an isoprenoid compound is not a pyruvate-derived product if that isoprenoid compound is synthesized from pyruvate in more than 25 enzymatic steps. An enzymatic step is a single chemical reaction catalyzed by a polypeptide having enzymatic activity. The term "polypeptide having enzymatic activity" as used herein refers to any polypeptide that catalyzes a chemical reaction of other substances without itself being destroyed or altered upon completion of the reaction. Typically, a polypeptide having enzymatic activity catalyzes the formation of one or more products from one or more substrates. Such polypeptides can have any type of enzymatic activity including, without limitation, the enzymatic activity associated with an enzyme such as DXS, DDS, ODS, SDS, DXR (1-deoxy-D-xylulose 5-phosphate reductoisomerase), ispD (4-diphosphocytidyl-2C-methyl-D-erythritol synthase), and ispE (4-diphosphocytidyl-2C-methyl-D-erythritol kinase).

A polypeptide having a particular enzymatic activity can be a polypeptide that is either naturally-occurring or non-naturally-occurring. A naturally-occurring polypeptide is any polypeptide having an amino acid sequence as found in nature, including wild-type and polymorphic polypeptides. Such naturally-occurring polypeptides can be obtained from any species including, without limitation, animal (e.g., mammalian), plant, fungal, and bacterial species. A non-naturally-occurring polypeptide is any polypeptide having an amino acid sequence that is not found in nature. Thus, a non-naturally-occurring polypeptide can be a mutated version of a naturally-occurring polypeptide, or an engineered polypeptide. For example, a non-naturally-occurring polypeptide having DDS activity can be a mutated version of a naturally-occurring polypeptide having DDS activity that retains at least some DDS activity. A polypeptide can be mutated by, for example, sequence additions, deletions, substitutions, or combinations thereof.

Examples of isoprenoid compounds that are pyruvate-derived products include, without limitation, CoQ(6), CoQ(7), CoQ(8), CoQ(9), CoQ(10), astaxanthin, canthaxanthin, lutein, zeaxanthin, beta-carotene, lycopene, capsanthin, bixin, norbixin, crocetin, zeta-carotene, vitamin E, giberellins, abscisic acid, ergosterol, geraniol, and latex.

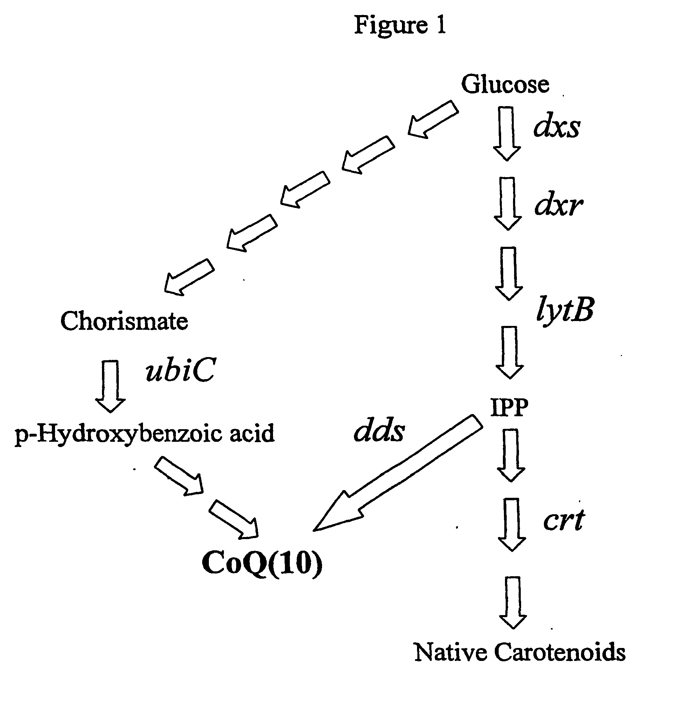

As depicted in Figure 1, multiple polypeptide can be used to convert glucose CoQ(10). For example, polypeptides having DXS, DXR, LytB, and DDS activity can be used to convert glucose CoQ(10). Such polypeptides can be obtained and used to make CoQ(10) as described herein.

*1. Nucleic acids*

The term "nucleic acid" as used herein encompasses both RNA and DNA, including cDNA, genomic DNA, and synthetic (e.g., chemically synthesized) DNA. The nucleic acid can be double-stranded or single-stranded. Where single-stranded, the nucleic acid can be the sense strand or the antisense strand. In addition, nucleic acid can be circular or linear.

The term "isolated" as used herein with reference to nucleic acid refers to a naturally-occurring nucleic acid that is not immediately contiguous with both of the sequences with which it is immediately contiguous (one on the 5' end and one on the 3' end) in the naturally-occurring genome of the organism from which it is derived. For example, an isolated nucleic acid can be, without limitation, a recombinant DNA molecule of any length, provided one of the nucleic acid sequences normally found immediately flanking that recombinant DNA molecule in a naturally-occurring genome is removed or absent. Thus, an isolated nucleic acid includes, without limitation, a recombinant DNA that exists as a separate molecule (e.g., a cDNA or a genomic DNA fragment produced by PCR or restriction endonuclease treatment) independent of other sequences as well as recombinant DNA that is incorporated into a vector, an autonomously replicating plasmid, a virus (e.g., a retrovirus, adenovirus, or herpes virus), or into the genomic DNA of a prokaryote or eukaryote. In addition, an isolated nucleic acid can include a recombinant DNA molecule that is part of a hybrid or fusion nucleic acid sequence.

The term "isolated" as used herein with reference to nucleic acid also includes any non-naturally-occurring nucleic acid since non-naturally occurring nucleic acid sequences are not found in nature and do not have immediately contiguous sequences in a naturally-occurring genome. For example, non-naturally-occurring nucleic acid such as an engineered nucleic acid is considered to be isolated nucleic acid. Engineered nucleic acid can be made using common molecular cloning or chemical nucleic acid synthesis techniques. Isolated non-naturally-occurring nucleic acid can be independent of other sequences, or incorporated into a vector, an autonomously replicating plasmid, a virus (e.g., a retrovirus, adenovirus, or herpes virus), or the genomic DNA of a prokaryote or eukaryote. In addition, a non-naturally-occurring nucleic acid can include a nucleic acid molecule that is part of a hybrid or fusion nucleic acid sequence.

It will be apparent to those of skill in the art that a nucleic acid existing among hundreds to millions of other nucleic acid molecules within, for example, cDNA or genomic libraries, or gel slices containing a genomic DNA restriction digest is not to be considered an isolated nucleic acid.

The term "exogenous" as used herein with reference to nucleic acid and a particular cell refers to any nucleic acid that does not originate from that particular cell as found in nature. Thus, all non-naturally-occurring nucleic acid is considered to be exogenous to a cell once introduced into the cell. It is important to note that non-naturally-occurring nucleic acid can contain nucleic acid sequences or fragments of nucleic acid sequences that are found in nature provided the nucleic acid as a whole does not exist in nature. For example, a nucleic acid molecule containing a genomic DNA sequence within an expression vector is non-naturally-occurring nucleic acid, and thus is exogenous to a cell once introduced into the cell, since that nucleic acid molecule as a whole (genomic DNA plus vector DNA) does not exist in nature. Thus, any vector, autonomously replicating plasmid, or virus (e.g., retrovirus, adenovirus, or herpes virus) that as a whole does not exist in nature is considered to be non-naturally-occurring nucleic acid. It follows that genomic DNA fragments produced by PCR or restriction endonuclease treatment as well as cDNAs are considered to be non-naturally-occurring nucleic acid since they exist as separate molecules not found in nature. It also follows that any nucleic acid containing a promoter sequence and polypeptide-encoding sequence (e.g., cDNA or genomic DNA) in an arrangement not found in nature is non-naturally-occurring nucleic acid.

Nucleic acid that is naturally-occurring can be exogenous to a particular cell. For example, an entire chromosome isolated from a cell of person X is an exogenous nucleic acid with respect to a cell of person Y once that chromosome is introduced into Y's cell.

Described herein is an isolated nucleic acid that contains a nucleic acid sequence having (1) a length, and (2) a percent identity to an identified nucleic acid sequence over that length. Further described herein is an isolated nucleic acid that contains a nucleic acid sequence encoding a polypeptide that contains an amino acid sequence having (1) a length, and (2) a percent identity to an identified amino acid sequence over that length. Typically, the identified nucleic acid or amino acid sequence is a sequence referenced by a particular sequence identification number, and the nucleic acid or amino acid sequence being compared to the identified sequence is referred to as the target sequence. For example, an identified sequence can be the sequence set forth in SEQ ID NO: 1.

A length and percent identity over that length for any nucleic acid or amino acid sequence is determined as follows. First, a nucleic acid or amino acid sequence is compared to the identified nucleic acid or amino acid sequence using the BLAST 2 Sequences (B12seq) program from the stand-alone version of BLASTZ containing BLASTN version 2.0.14 and BLASTP version 2.0.14. This stand-alone version of BLASTZ can be obtained from the University of Wisconsin library as well as at www.fr.com or www.ncbi.nlm.nih.gov. Instructions explaining how to use the B12seq program can be found in the readme file accompanying BLASTZ. B12seq performs a comparison between two sequences using either the BLASTN or BLASTP algorithm. BLASTN is used to compare nucleic acid sequences, while BLASTP is used to compare amino acid sequences. To compare two nucleic acid sequences, the options are set as follows: -i is set to a file containing the first nucleic acid sequence to be compared (e.g., C:\seq1.txt); -j is set to a file containing the second nucleic acid sequence to be compared (e.g., C:\seq2.txt); -p is set to blastn; -o is set to any desired file name (e.g., C:\output.txt); -q is set to -1; -r is set to 2; and all other options are left at their default setting. For example, the following command can be used to generate an output file containing a comparison between two sequences: C:\B12seq -i c:\seq 1.txt -j c:\seq2.txt -p blastn -o c:\output.txt -q -1-r 2. To compare two amino acid sequences, the options of Bl2seq are set as follows: -i is set to a file containing the first amino acid sequence to be compared (e.g., C:\seq1.txt); -j is set to a file containing the second amino acid sequence to be compared (e.g., C:\.seq2.tact); -p is set to blastp; -o is set to any desired file name (e.g., C:\output.txt); and all other options are left at their default setting. For example, the following command can be used to generate an output file containing a comparison between two amino acid sequences: C:\B12seq -i c:\seq1 .txt -j c:\seq2.txt -p blastp -o c:\output.txt. If the target sequence shares homology with any portion of the identified sequence, then the designated output file will present those regions of homology as aligned sequences. If the target sequence does not share homology with any portion of the identified sequence, then the designated output file will not present aligned sequences. Once aligned, a length is determined by counting the number of consecutive nucleotides or amino acid residues from the target sequence presented in alignment with sequence from the identified sequence starting with any matched position and ending with any other matched position. A matched position is any position where an identical nucleotide or amino acid residue is presented in both the target and identified sequence. Gaps presented in the target sequence are not counted since gaps are not nucleotides or amino acid residues. Likewise, gaps presented in the identified sequence are not counted since target sequence nucleotides or amino acid residues are counted, not nucleotides or amino acid residues from the identified sequence.

The percent identity over a determined length is determined by counting the number of matched positions over that length and dividing that number by the length followed by multiplying the resulting value by 100. For example, if (1) a 1000 nucleotide target sequence is compared to the sequence set forth in SEQ ID NO:1, (2) the B12seq program presents 200 nucleotides from the target sequence aligned with a region of the sequence set forth in SEQ ID NO: 1 where the first and last nucleotides of that 200 nucleotide region are matches, and (3) the number of matches over those 200 aligned nucleotides is 180, then the 1000 nucleotide target sequence contains a length of 200 and a percent identity over that length of 90 (i.e. 180 ÷ 200 * 100 = 90).

It will be appreciated that a single nucleic acid or amino acid target sequence that aligns with an identified sequence can have many different lengths with each length having its own percent identity. For example, a target sequence containing a 20 nucleotide region that aligns with an identified sequence as follows has many different lengths including those listed in Table 1.

```
                                 1                  20
Target Sequence:                 AGGTCGTGTACTGTCAGTCA
                                 | || ||| |||| |||| |
Identified Sequence:             ACGTGGTGAACTGCCAGTGA
```

Table I.

| Starting Position | Ending Position | Length | Matched Positions | Percent Identity |
|---|---|---|---|---|
| 1 | 20 | 20 | 15 | 75.0 |
| 1 | 18 | 18 | 14 | 77.8 |
| 1 | 15 | 15 | 11 | 73.3 |
| 6 | 20 | 15 | 12 | 80.0 |
| 6 | 17 | 12 | 10 | 83.3 |
| 6 | 15 | 10 | 8 | 80.0 |
| 8 | 20 | 13 | 10 | 76.9 |
| 8 | 16 | 9 | 7 | 77.8 |

It is noted that the percent identity value is rounded to the nearest tenth. For example, 78.11, 78.12, 78.13, and 78.14 is rounded down to 78.1, while 78.15, 78.16, 78.17, 78.18, and 78.19 is rounded up to 78.2. It is also noted that the length value will always be an integer.

Described herein is an isolated nucleic acid containing a nucleic acid sequence that has at least one length and percent identity over that length as determined above such that the point defined by that length and percent identity is within the area defined by points A, B, C, and D of Figure 26. In addition, described herein is an isolated nucleic acid containing a nucleic acid sequence that encodes a polypeptide containing an amino acid sequence that has at least one length and percent identity over that length as determined above such that the point defined by that length and percent identity is within the area defined by points A, B, C, and D of Figure 26. The point defined by a length and percent identity over that length is that point on the X/Y coordinate of Figure 26 where the X axis is the length and the Y axis is the percent identity. Thus, the point defined by a nucleic acid sequence with a length of 200 and a percent identity of 90 has coordinates (200, 90). For the purpose of this invention, any point that falls on point A, D, C, or D is considered within the area defined by points A, B, C, and D of Figure 26. Likewise, any point that falls on a line that defines the area defined by points A, B, C, and D is considered within the area defined by points A, B, C, and D of Figure 26.

It will be appreciated that the term "the area defined by points A, B, C, and D of Figure 26" as used herein refers to that area defined by the lines that connect point A with point B, point B with point C, point C with point D, and point D with point A. Points A, B, C, and D can define an area having any shape defined by four points (e.g., square, rectangle, or rhombus). In addition, two or more points can have the same coordinates. For example, points B and C can have identical coordinates. In this case, the area defined by points A, B, C, and D of Figure 26 is triangular. If three points have identical coordinates, then the area defined by points A, B, C, and D of Figure 26 is a line. In this case, any point that falls on that line would be considered within the area defined by points A, B, C, and D of Figure 26. If all four points have identical coordinates, then the area defined by points A, B, C, and D of Figure 26 is a point. In all cases, simple algebraic equations can be used to determine whether a point is within the area defined by points A, B, C, and D of Figure 26.

It is noted that Figure 26 is a graphical representation presenting possible positions of points A, B, C, and D. The shaded area illustrated in Figure 26 represents one possible example, while the arrows indicate that other positions for points A, B, C, and D are possible. In fact, points A, B, C, and D can have any X coordinate and any Y coordinate. For example, point A can have an X coordinate equal to the number of nucleotides or amino acid residues in an identified sequence, and a Y coordinate of 100. Point B can have an X coordinate equal to the number of nucleotides or amino acid residues in an identified sequence, and a Y coordinate less than or equal to 100 (e.g., 50, 55, 65, 70, 75, 80, 85, 90, 95, and 99). Point C can have an X coordinate equal to a percent (e.g., 1, 2, 5, 10, 15, or more percent) of the number of nucleotides or amino acid residues in an identified sequence, and a Y coordinate less than or equal to 100 (e.g., 50, 55, 65, 70, 75, 80, 85, 90, 95, and 99). Point D can have an X coordinate equal to the length of a typical PCR primer (e.g., 12, 13, 14, 15, 16, 17, or more) or antigenic polypeptide (e.g., 5, 6, 7, 8, 9, 10, 11, 12, or more), and a Y coordinate less than or equal to 100 (e.g., 50, 55, 65, 70, 75, 80, 85, 90, 95, and 99).

An isolated nucleic acid containing a nucleic acid sequence having a length and a percent identity to the sequence set forth in SEQ ID NO:1 over that length is contemplated provided the point defined by that length and percent identity is within the area defined by points A, B, C, and D of Figure 26; where point A has an X coordinate less than or equal to 3626, and a Y coordinate less than or equal to 100; where point B has an X coordinate less than or equal to 3626, and a Y coordinate greater than or equal to 65; where point C has an X coordinate greater than or equal to 50, and a Y coordinate greater than or equal to 65; and where point D has an X coordinate greater than or equal to 12, and a Y coordinate less than or equal to 100. For example, the X coordinate for point A can be 3626, 3600, 3500, 3000, 2500, or less; and the Y coordinate for point A can be 100, 99, 95, 90, 85, 80, 75, or less. The X coordinate for point B can be 3626, 3600, 3500, 3000, 2500, or less; and the Y coordinate for point B can be 65, 70, 75, 80, 85, 90, 95, 99 or more. The X coordinate for point C can be 50, 60, 70, 80, 90, 100, 150, 200, or more; and the Y coordinate for point C can be 65, 70, 75, 80, 85, 90, 95, 99 or more. The X coordinate for point D can be 12, 13, 14, 15, 16, 17, 18, 19, 20, 25, 30, 40, 50, 75, 100, or more; and the Y coordinate for point D can be 100, 99, 95, 90, 85, 80, 75, or less. In one example, point A can be (3626, 100), point B can be (3626, 95), point C can be (1900, 95), and point D can be (1900, 100).

An isolated nucleic acid containing a nucleic acid sequence having a length and a percent identity to the sequence set forth in SEQ ID NO:2 over that length is contemplated provided the point defined by that length and percent identity is within the area defined by points A, B, C, and D of Figure 26; where point A has an X coordinate less than or equal to 1926, and a Y coordinate less than or equal to 100; where point B has an X coordinate less than or equal to 1926, and a Y coordinate greater than or equal to 65; where point C has an X coordinate greater than or equal to 50, and a Y coordinate greater than or equal to 65; and where point D has an X coordinate greater than or equal to 12, and a Y coordinate less than or equal to 100. For example, the X coordinate for point A can be 1926, 1900, 1850, 1800, 1750, or less; and the Y coordinate for point A can be 100, 99, 95, 90, 85, 80, 75, or less. The X coordinate for point B can be 1926, 1900, 1850, 1800, 1750, or less; and the Y coordinate for point B can be 65, 70, 75, 80, 85, 90, 95, 99 or more. The X coordinate for point C can be 50, 60, 70, 80, 90, 100, 150, 200, or more; and the Y coordinate for point C can be 65, 70, 75, 80, 85, 90, 95, 99 or more. The X coordinate for point D can be 12, 13, 14, 15, 16, 17, 18, 19, 20, 25, 30, 40, 50, 75, 100, or more; and the Y coordinate for point D can be 100, 99, 95, 90, 85, 80, 75, or less. In one example, point A can be (1926, 100), point B can be (1926, 95), point C can be (1000, 95), and point D can be (1000, 100).

An isolated nucleic acid containing a nucleic acid sequence that encodes a polypeptide containing an amino acid sequence having a length and a percent identity to the sequence set forth in SEQ ID NO:3 over that length is contemplated provided the point defined by that length and percent identity is within the area defined by points A, B, C, and D of Figure 26; where point A has an X coordinate less than or equal to 641, and a Y coordinate less than or equal to 100; where point B has an X coordinate less than or equal to 641, and a Y coordinate greater than or equal to 50; where point C has an X coordinate greater than or equal to 25, and a Y coordinate greater than or equal to 50; and where point D has an X coordinate greater than or equal to 5, and a Y coordinate less than or equal to 100. For example, the X coordinate for point A can be 641, 635, 630, 625, 620, or less; and the Y coordinate for point A can be 100, 99, 95, 90, 85, 80, 75, or less. The X coordinate for point B can be 641, 635, 630, 625, 620, or less; and the Y coordinate for point B can be 50, 55, 60, 65, 70, 75, 80, 85, 90, 95, 99 or more. The X coordinate for point C can be 25, 30, 35, 40, 50, 60, 70, 80, 90, 100, 150, 200, or more; and the Y coordinate for point C can be 50, 55, 60, 65, 70, 75, 80, 85, 90, 95, 99 or more. The X coordinate for point D can be 5, 6, 7, 8, 9, 10, 15, 20, 25, 30, 40, 50, 75, 100, or more; and the Y coordinate for point D can be 100, 99,95,90, 85, 80, 75, or less. In one example, point A can be (641, 100), point B can be (641, 95), point C can be (400, 95), and point D can be (400, 100).

An isolated nucleic acid containing a nucleic acid sequence having a length and a percent identity to the sequence set forth in SEQ ID NO:37 over that length is contemplated provided the point defined by that length and percent identity is within the area defined by points A, B, C, and D of Figure 26; where point A has an X coordinate less than or equal to 1990, and a Y coordinate less than or equal to 100; where point B has an X coordinate less than or equal to 1990, and a Y coordinate greater than or equal to 65; where point C has an X coordinate greater than or equal to 50, and a Y coordinate greater than or equal to 65; and where point D has an X coordinate greater than or equal to 12, and a Y coordinate less than or equal to 100. For example, the X coordinate for point A can be 1990, 1950, 1900, 1850, 1800, 1750, or less; and the Y coordinate for point A can be 100, 99, 95, 90, 85, 80, 75, or less. The X coordinate for point B can be 1990, 1950, 1900, 1850, 1800, 1750, or less; and the Y coordinate for point B can be 65, 70, 75, 80, 85, 90, 95, 99 or more. The X coordinate for point C can be 50, 60, 70, 80, 90, 100, 150, 200, or more; and the Y coordinate for point C can be 65, 70, 75, 80, 85, 90, 95, 99 or more. The X coordinate for point D can be 12, 13, 14, 15, 16, 17, 18, 19, 20, 25, 30, 40, 50, 75, 100, or more; and the Y coordinate for point D can be 100, 99, 95, 90, 85, 80, 75, or less. In one example, point A can be (1990, 100), point B can be (1990, 95), point C can be (1000, 95), and point D can be (1000, 100).

An isolated nucleic acid containing a nucleic acid sequence having a length and a percent identity to the sequence set forth in SEQ ID NO:38 over that length is contemplated provided the point defined by that length and percent identity is within the area defined by points A, B, C, and D of Figure 26; where point A has an X coordinate less than or equal to 1002, and a Y coordinate less than or equal to 100; where point B has an X coordinate less than or equal to 1002, and a Y coordinate greater than or equal to 65; where point C has an X coordinate greater than or equal to 50, and a Y coordinate greater than or equal to 65; and where point D has an X coordinate greater than or equal to 12, and a Y coordinate less than or equal to 100. For example, the X coordinate for point A can be 1002, 950, 900, 850, 800, 750, or less; and the Y coordinate for point A can be 100, 99, 95, 90, 85, 80, 75, or less. The X coordinate for point B can be 1002, 950, 900, 850, 800, 750, or less; and the Y coordinate for point B can be 65, 70, 75, 80, 85, 90, 95, 99 or more. The X coordinate for point C can be 50, 60, 70, 80, 90, 100, 150, 200, or more; and the Y coordinate for point C can be 65, 70, 75, 80, 85, 90, 95, 99 or more. The X coordinate for point D can be 12, 13, 14, 15, 16, 17, 18, 19, 20, 25, 30, 40, 50, 75, 100, or more; and the Y coordinate for point D can be 100,99,95,90, 85, 80, 75, or less. In one example, point A can be (1002, 100), point B can be (1002, 95), point C can be (500, 95), and point D can be (500, 100).

An isolated nucleic acid containing a nucleic acid sequence that encodes a polypeptide containing an amino acid sequence having a length and a percent identity to the sequence set forth in SEQ ID NO:39 over that length is contemplated provided the point defined by that length and percent identity is within the area defined by points A, B, C, and D of Figure 26; where point A has an X coordinate less than or equal to 333, and a Y coordinate less than or equal to 100; where point B has an X coordinate less than or equal to 333, and a Y coordinate greater than or equal to 50; where point C has an X coordinate greater than or equal to 25, and a Y coordinate greater than or equal to 50; and where point D has an X coordinate greater than or equal to 5, and a Y coordinate less than or equal to 100. For example, the X coordinate for point A can be 333, 330, 325, 320, 315, or less; and the Y coordinate for point A can be 100, 99, 95, 90, 85, 80, 75, or less. The X coordinate for point B can be 333,330,325, 320, 315, or less; and the Y coordinate for point B can be 50, 55, 60, 65, 70, 75, 80, 85, 90, 95, 99 or more. The X coordinate for point C can be 25, 30, 35, 40, 50, 60, 70, 80, 90, 100, 150, 200, or more; and the Y coordinate for point C can be 50, 55, 60, 65, 70, 75, 80, 85, 90, 95, 99 or more. The X coordinate for point D can be 5, 6, 7, 8, 9, 10, 15, 20, 25, 30, 40, 50, 75, 100, or more; and the Y coordinate for point D can be 100, 99, 95, 90, 85, 80, 75, or less. In one example, point A can be (333, 100), point B can be (333, 95), point C can be (150, 95), and point D can be (150, 100).

An isolated nucleic acid containing a nucleic acid sequence having a length and a percent identity to the sequence set forth in SEQ ID NO:40 over that length is contemplated provided the point defined by that length and percent identity is within the area defined by points A, B, C, and D of Figure 26; where point A has an X coordinate less than or equal to 1833, and a Y coordinate less than or equal to 100; where point B has an X coordinate less than or equal to 1833, and a Y coordinate greater than or equal to 65; where point C has an X coordinate greater than or equal to 50, and a Y coordinate greater than or equal to 65; and where point D has an X coordinate greater than or equal to 12, and a Y coordinate less than or equal to 100. For example, the X coordinate for point A can be 1833, 1800, 1750, 1700, 1650, or less; and the Y coordinate for point A can be 100,99,95,90, 85, 80, 75, or less. The X coordinate for point B can be 1833, 1800, 1750, 1700, 1650, or less; and the Y coordinate for point B can be 65, 70, 75, 80, 85,90, 95, 99 or more. The X coordinate for point C can be 50, 60, 70, 80, 90, 100, 150, 200, or more; and the Y coordinate for point C can be 65, 70, 75, 80, 85, 90, 95, 99 or more. The X coordinate for point D can be 12, 13, 14, 15, 16, 17, 18, 19, 20, 25, 30, 40, 50, 75, 100, or more; and the Y coordinate for point D can be 100, 99, 95, 90, 85, 80, 75, or less. In one example, point A can be (1833, 100), point B can be (1833, 95), point C can be (900, 95), and point D can be (900,100).

An isolated nucleic acid containing a nucleic acid sequence having a length and a percent identity to the sequence set forth in SEQ ID NO:41 over that length is contemplated provided the point defined by that length and percent identity is within the area defined by points A, B, C, and D of Figure 26; where point A has an X coordinate less than or equal to 1014, and a Y coordinate less than or equal to 100; where point B has an X coordinate less than or equal to 1014, and a Y coordinate greater than or equal to 65; where point C has an X coordinate greater than or equal to 50, and a Y coordinate greater than or equal to 65; and where point D has an X coordinate greater than or equal to 12, and a Y coordinate less than or equal to 100. For example, the X coordinate for point A can be 1014, 950, 900, 800, 700, 600, or less; and the Y coordinate for point A can be 100, 99, 95, 90, 85, 80, 75, or less. The X coordinate for point B can be 1014, 950, 900, 800, 700, 600, or less; and the Y coordinate for point B can be 65, 70, 75, 80, 85, 90, 95, 99 or more. The X coordinate for point C can be 50, 60, 70, 80, 90, 100, 150,200, or more; and the Y coordinate for point C can be 65, 70, 75, 80, 85, 90, 95, 99 or more. The X coordinate for point D can be 12,13,14,15,16,17,18,19, 20,25, 30,40, 50,75,100, or more; and the Y coordinate for point D can be 100 99, 95, 90, 85, 80, 75, or less. In one example, point A can be (1014,100), point B can be (1014, 95), point C can be (500, 95), and point D can be (500, 100).

An isolated nucleic acid containing a nucleic acid sequence that encodes a polypeptide containing an amino acid sequence having a length and a percent identity to the sequence set forth in SEQ ID NO:42 over that length is contemplated provided the point defined by that length and percent identity is within the area defined by points A, B, C, and D of Figure 26; where point A has an X coordinate less than or equal to 337, and a Y coordinate less than or equal to 100; where point B has an X coordinate less than or equal to 337, and a Y coordinate greater than or equal to 50; where point C has an X coordinate greater than or equal to 25, and a Y coordinate greater than or equal to 50; and where point D has an X coordinate greater than or equal to 5, and a Y coordinate less than or equal to 100. For example, the X coordinate for point A can be 337, 335, 330, 325, 320, 315, or less; and the Y coordinate for point A can be 100, 99, 95, 90, 85, 80, 75, or less. The X coordinate for point B can be 337, 335, 330, 325, 320, 315, or less; and the Y coordinate for point B can be 50, 55, 60, 65, 70, 75, 80, 85, 90, 95, 99 or more. The X coordinate for point C can be 25, 30, 35, 40, 50, 60, 70, 80, 90, 100, 150. 200, or more; and the Y coordinate for point C can be 50, 55, 60, 65, 70, 75, 80, 85, 90, 95, 99 or more. The X coordinate for point D can be 5, 6, 7, 8, 9, 10, 15, 20, 25, 30, 40, 50, 75, 100, or more; and the Y coordinate for point D can be 100, 99, 95, 90, 85, 80, 75, or less. In one example, point A can be (337, 100), point B can be (337, 95), point C can be (150, 95), and point D can be (150, 100).

An isolated nucleic acid containing a nucleic acid sequence having a length and a percent identity to the sequence set forth in SEQ ID NO:95 over that length is contemplated provided the point defined by that length and percent identity is within the area defined by points A, B, C, and D of Figure 26; where point A has an X coordinate less than or equal to 2017, and a Y coordinate less than or equal to 100; where point B has an X coordinate less than or equal to 2017, and a Y coordinate greater than or equal to 65; where point C has an X coordinate greater than or equal to 50, and a Y coordinate greater than or equal to 65; and where point D has an X coordinate greater than or equal to 12, and a Y coordinate less than or equal to 100. For example, the X coordinate for point A can be 2017, 2000, 1900, 1950, 1800, 1700, 1600, or less; and the Y coordinate for point A can be 100, 99, 95, 90, 85, 80, 75, or less. The X coordinate for point B can be 2017, 2000, 1900, 1950, 1800, 1700, 1600, or less; and the Y coordinate for point B can be 65, 70, 75, 80, 85, 90, 95, 99 or more. The X coordinate for point C can be 50, 60, 70, 80, 90, 100,150, 200, 500,1000, 1500, or more; and the Y coordinate for point C can be 65, 70, 75, 80, 85, 90, 95, 99 or more. The X coordinate for point D can be 12,13,14,15,16,17,18,19,20,25, 30,40,50,75,100,250, 500,1000,1500, or more; and the Y coordinate for point D can be 100, 99, 95, 90, 85, 80, 75, or less. In one example, point A can be (2017,100), point B can be (2017, 95), point C can be (1800, 95), and point D can be (1800, 100).

An isolated nucleic acid containing a nucleic acid sequence having a length and a percent identity to the sequence set forth in SEQ ID NO:96 over that length is contemplated provided the point defined by that length and percent identity is within the area defined by points A, B, C, and D of Figure 26; where point A has an X coordinate less than or equal to 1161, and a Y coordinate less than or equal to 100; where point B has an X coordinate less than or equal to 1161, and a Y coordinate greater than or equal to 65; where point C has an X coordinate greater than or equal to 50, and a Y coordinate greater than or equal to 65; and where point D has an X coordinate greater than or equal to 12, and a Y coordinate less than or equal to 100. For example, the X coordinate for point A can be 1161, 1050, 1000, 950, 900, 800, 700, 600, or less; and the Y coordinate for point A can be 100, 99, 95, 90, 85, 80, 75, or less. The X coordinate for point B can be 1161, 1050, 1000, 950, 900, 800, 700, 600, or less; and the Y coordinate for point B can be 65, 70, 75, 80, 85, 90, 95, 99 or more. The X coordinate for point C can be 50, 60, 70, 80, 90, 100, 150, 200, 250, 500, 1000, or more; and the Y coordinate for point C can be 65, 70, 75, 80, 85, 90, 95, 99 or more. The X coordinate for point D can be 12, 13, 14, 15, 16, 17, 18, 19, 20, 25, 30, 40, 50, 75, 100, 250, 500, 1000, or more; and the Y coordinate for point D can be 100, 99, 95, 90, 85, 80, 75, or less. In one example point A can be (1161, 100), point B can be (1161, 95), point C can be (1000, 95), and point D can be (1000, 100).

An isolated nucleic acid containing a nucleic acid sequence that encodes a polypeptide containing an amino acid sequence having a length and a percent identity to the sequence set forth in SEQ ID NO:97 over that length is contemplated provided the point defined by that length and percent identity is within the area defined by points A, B, C, and D of Figure 26; where point A has an X coordinate less than or equal to 386, and a Y coordinate less than or equal to 100; where point B has an X coordinate less than or equal to 386, and a Y coordinate greater than or equal to 50; where point C has an X coordinate greater than or equal to 25, and a Y coordinate greater than or equal to 50; and where point D has an X coordinate greater than or equal to 5, and a Y coordinate less than or equal to 100. For example, the X coordinate for point A can be 386, 380, 375, 370, 375, 360, 365, 350, 325, 300, or less; and the Y coordinate for point A can be 100, 99, 95, 90, 85, 80, 75, or less. The X coordinate for point B can be 386, 380, 375, 370, 375, 360, 365, 350, 325, 300, or less; and the Y coordinate for point B can be 50, 55, 60, 65, 70, 75, 80, 85, 90, 95, 99 or more. The X coordinate for point C can be 25, 30, 35, 40, 50, 60, 70, 80, 90, 100, 150, 200, 300, 350, or more; and the Y coordinate for point C can be 50, 55, 60,65, 70, 75, 80,85, 90,95, 99 or more. The X coordinate for point D can be 5, 6, 7, 8, 9, 10, 15, 20, 25, 30, 40, 50, 75, 100, 200, 300, 350, or more; and the Y coordinate for point D can be 100, 99, 95, 90, 85, 80, 75, or less. In one example, point A can be (386, 100), point B can be (386, 95), point C can be (350, 95), and point D can be (350, 100).

An isolated nucleic acid is also provided that is at least about 12 bases in length (e.g., at least about 13, 14, 15, 16, 17, 18, 19, 20, 25, 30, 40, 50, 60, 100, 250, 500, 750, 1000, 1500, 2000, 3000, 4000, or 5000 bases in length) and hybridizes, under hybridization conditions, to the sense or antisense strand of a nucleic acid having the sequence set forth in SEQ ID NO:1; 2, 37, 38, 40, 41, 95, or 96. The hybridization conditions can be moderately or highly stringent hybridization conditions.

For the purpose of this invention, moderately stringent hybridization conditions mean the hybridization is performed at about 42°C in a hybridization solution containing 25 mM $KPO_4$ (pH 7.4), 5X SSC, 5X Denhart's solution, 50 μg/mL denatured, sonicated salmon sperm DNA, 50% formamide, 10% Dextran sulfate, and 1-15 ng/mL probe (about $5x10^7$ cpm/μg), while the washes are performed at about 50°C with a wash solution containing 2X SSC and 0.1 % sodium dodecyl sulfate.

Highly stringent hybridization conditions mean the hybridization is performed at about 42°C in a hybridization solution containing 25 mM $KPO_4$ (pH 7.4), 5X SSC, 5X Denhart's solution, 50 μg/mL denatured, sonicated salmon sperm DNA, 50% formamide, 10% Dextran sulfate, and 1-15 ng/mL probe (about $5x10^7$ cpm/μg), while the washes are performed at about 65°C with a wash solution containing 0.2X SSC and 0.1% sodium dodecyl sulfate.

Isolated nucleic acid described herein can be obtained using any method including, without limitation, common molecular cloning and chemical nucleic acid synthesis techniques. For example, PCR can be used to obtain an isolated nucleic acid containing a nucleic acid sequence sharing similarity to the sequence set forth in SEQ ID NO:1, 2, 37, 38, 40, 41, 95, or 96. PCR refers to a procedure or technique in which target nucleic acid is amplified in a manner similar to that described in U.S. Patent No. 4,683,195, and subsequent modifications of the procedure described therein. Generally, sequence information from the ends of the region of interest or beyond are used to design oligonucleotide primers that are identical or similar in sequence to opposite strands of a potential template to be amplified. Using PCR, a nucleic acid sequence can be amplified from RNA or DNA. For example, a nucleic acid sequence can be isolated by PCR amplification from total cellular RNA, total genomic DNA, and cDNA as well as from bacteriophage sequences, plasmid sequences, viral sequences, and the like. When using RNA as a source of template, reverse transcriptase can be used to synthesize complimentary DNA strands.

An isolated nucleic acid described herein also can be obtained by mutagenesis. For example, an isolated nucleic acid containing a sequence set forth in SEQ ID NO:1, 2, 37, 38,40,41,95, or 96 can be mutated using common molecular cloning techniques (e.g., site-directed mutagenesis). Possible mutations include, without limitation, deletions, insertions, and substitutions, as well as combinations of deletions, insertions, and substitutions.

In addition, nucleic acid and amino acid databases (e.g., GenBank®) can be used to obtain an isolated nucleic acid described herein. For example, any nucleic acid sequence having some homology to a sequence set forth in SEQ ID NO:1, 2, 37, 38, 40, 41, 95, or 96, or any amino acid sequence having some homology to a sequence set forth in SEQ ID NO:3, 39, 42, or 97 can be used as a query to search GenBank®.

Further, nucleic acid hybridization techniques can be used to obtain an isolated nucleic acid described herein. Briefly, any nucleic acid having some homology to a sequence set forth in SEQ ID NO:1, 2, 37, 38, 40, 41, 95, or 96 can be used as a probe to identify a similar nucleic acid by hybridization under conditions of moderate to high stringency. Once identified, the nucleic acid then can be purified, sequenced, and analyzed to determine whether it is as described herein.

Hybridization can be done by Southern or Northern analysis to identify a DNA or RNA sequence, respectively, that hybridizes to a probe. The probe can be labeled with a biotin, digoxygenin, an enzyme, or a radioisotope such as $^{32}P$. The DNA or RNA to be analyzed can be electrophoretically separated on an agarose or polyacrylamide gel, transferred to nitrocellulose, nylon, or other suitable membrane, and hybridized with the probe using standard techniques well known in the art such as those described in sections 7.39-7.52 of Sambrook et al., (1989) Molecular Cloning, second edition, Cold Spring harbor Laboratory, Plainview, NY. Typically, a probe is at least about 20 nucleotides in length. For example, a probe corresponding to a 20 nucleotide sequence set forth in SEQ ID NO: 1, 2, 37, 38, 40, 41, 95, or 96 can be used to identify an identical or similar nucleic acid. In addition, probes longer or shorter than 20 nucleotides can be used.

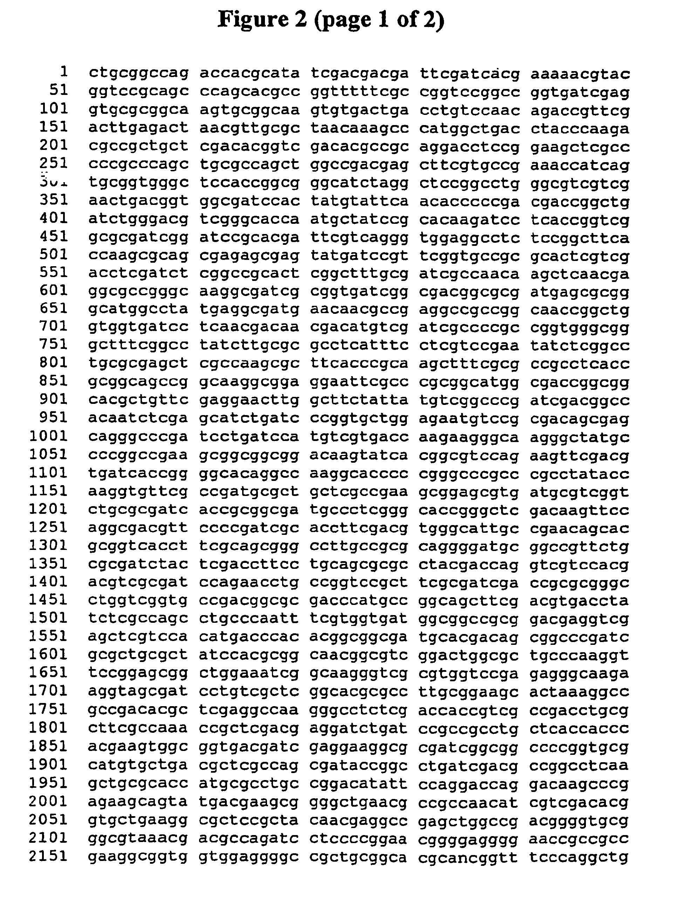

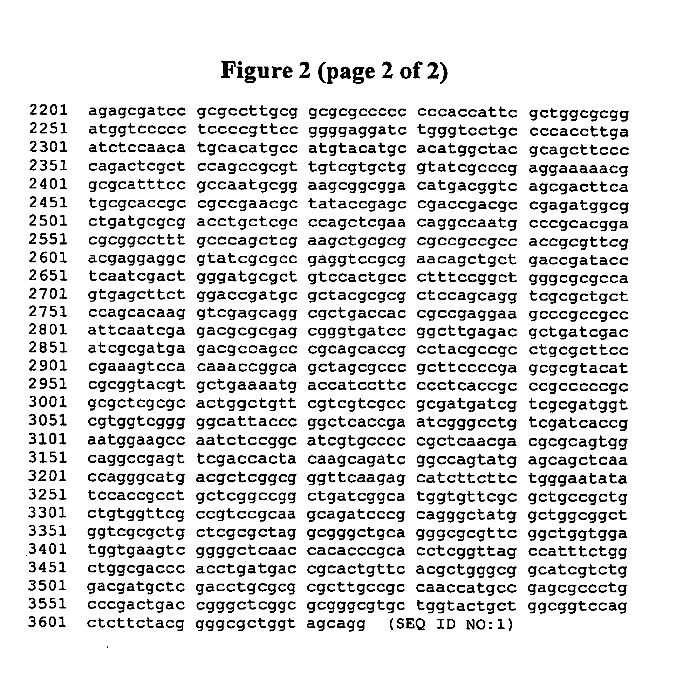

An isolated nucleic acid is provided that contains the entire nucleic acid sequence depicted in Figure 2, 3, 7, 8, 10, 11, 28, or 29. In addition, an isolated nucleic acid is provided that contains a portion of the nucleic acid sequence depicted in Figure 2, 3, 7, 8, 10, 11, 28, or 29. For example, an isolated nucleic acid that contains a 15 nucleotide sequence identical to any 15 nucleotide sequence depicted in Figure 2, 3, 7, 8,10, 11, 28, or 29 including, without limitation, the sequence starting at nucleotide number 1 and ending at nucleotide number 15, the sequence starting at nucleotide number 2 and ending at nucleotide number 16, the sequence starting at nucleotide number 3 and ending at nucleotide number 17, and so forth. It will be appreciated that an isolated nucleic acid is also provided that contains a nucleotide sequence that is greater than 15 nucleotides (e.g., 16, 17,18, 19, 20, 21, 22, 23, 24, 25, 26, 27, 28, 29, 30, or more nucleotides) in length and identical to any portion of the sequence depicted in Figure 2, 3, 7, 8, 10, 11, 28, or 29. For example, an isolated nucleic acid is provided that contains a 25 nucleotide sequence identical to any 25 nucleotide sequence depicted in Figure 2, 3, 7, 8, 10, 11, 28, or 29 including, without limitation, the sequence starting at nucleotide number 1 and ending at nucleotide number 25, the sequence starting at nucleotide number 2 and ending at nucleotide number 26, the sequence starting at nucleotide number 3 and ending at nucleotide number 27, and so forth. Additional examples include, without limitation, isolated nucleic acids that contain a nucleotide sequence that is 50 or more nucleotides (e.g., 100, 150,200. 250, 300, 350, or more nucleotides) in length and identical to any portion of the sequence depicted in Figure 2,3,7, 8, 10, 11, 28, or 29. Such isolated nucleic acids can include, without limitation, those isolated nucleic acids containing a nucleic acid sequence represented in a single line of sequence depicted in Figure 2, 3, 7, 8, 10, 11, 28, or 29 since each line of sequence depicted in these figures, with the exception of the last line, provides a 50 nucleotide sequence. In addition, an isolated nucleic acid is provided that contains a variation of the nucleic acid sequence depicted in Figure 2, 3, 7, 8, 10, 11, 28, or 29. For example, an isolated nucleic acid is provided containing a nucleic acid sequence depicted in Figure 2, 3, 7, 8, 10, I1, 28, or 29 that contains a single insertion, a single deletion, a single substitution, multiple insertions, multiple deletions, multiple substitutions, or any combination thereof (e.g., single deletion together with multiple insertions). Multiple examples of isolated nucleic acid are provided that contains a variation of a nucleic acid sequence depicted in Figure 2, 3, 7, 8, 10, 11, 28, or 29.

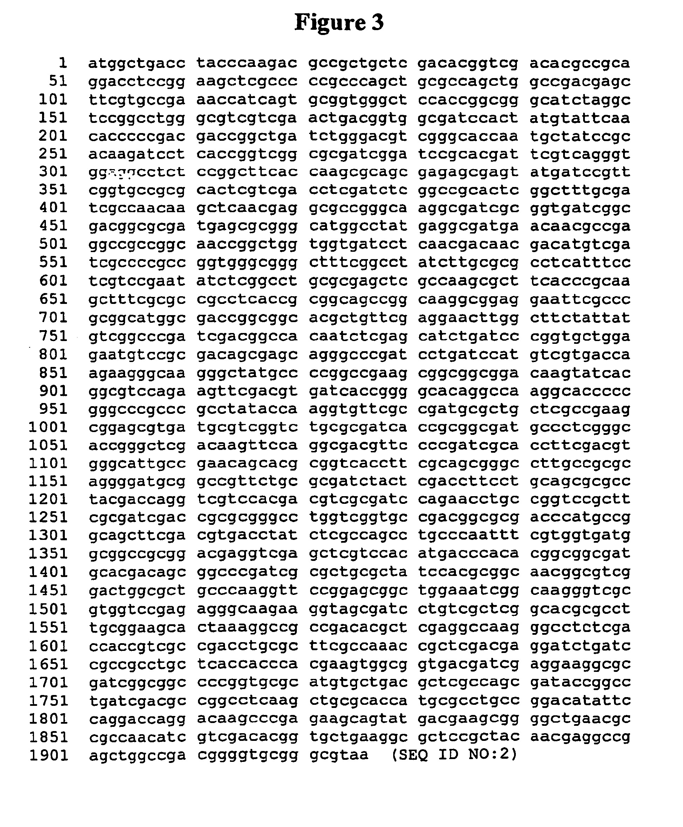

Figure 5 depicts the nucleic acid sequence depicted in Figure 3 (designated STdxsdna) aligned with 13 other nucleic acid sequences. Examples of variations of the STdxsdna sequence include, without limitation, any variation of the STdxsdna sequence provided in Figure 5. Such variations are provided in Figure 5 in that a comparison of the nucleotide (or lack thereof) at a particular position of the STdxsdna sequence with the nucleotide (or lack thereof) at the same position of any of the other 13 nucleic acid sequences depicted in Figure 5 provides a list of specific changes for the STdxsdna sequence. For example, the "t" at position 1260 of the STdxsdna sequence can be substituted with an "a" as indicated in Figure 5. As also indicated in Figure 5, the "a" at position 1851 of the STdxsdna sequence can be substituted with a "t" or "c"; a "t" can be inserted after the "a" at position 1865 of the STdxsdna sequence; or the "t" at position 1078 of the STdxsdna sequence can be deleted. It will be appreciated that the STdxsdna sequence can contain any number of variations as well as any combination of types of variations. For example, the STdxsdna sequence can contain one variation provided in Figure 5 or more than one (e.g., 2, 3, 4, 5, 6, 7, 8, 9, 10, 15, 20, 25, 50, 100, or more) of the variations provided in Figure 5. It is noted that the full-length nucleic acid sequences depicted in Figure 5 can encode polypeptides having DXS activity. It also is noted that the nucleic acid sequence depicted in Figure 2 contains the nucleic acid sequence depicted in Figure 3.

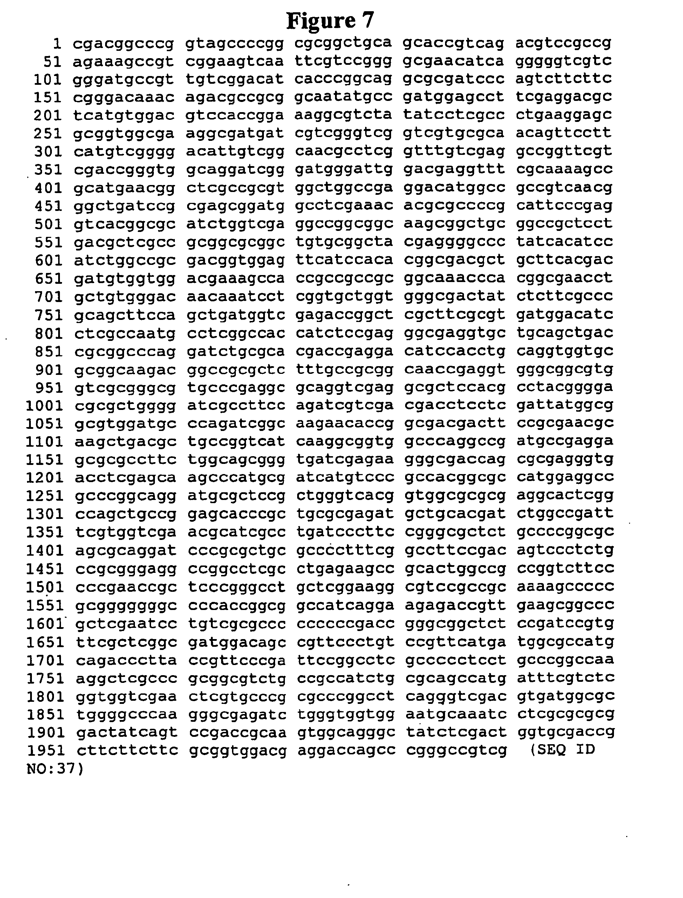

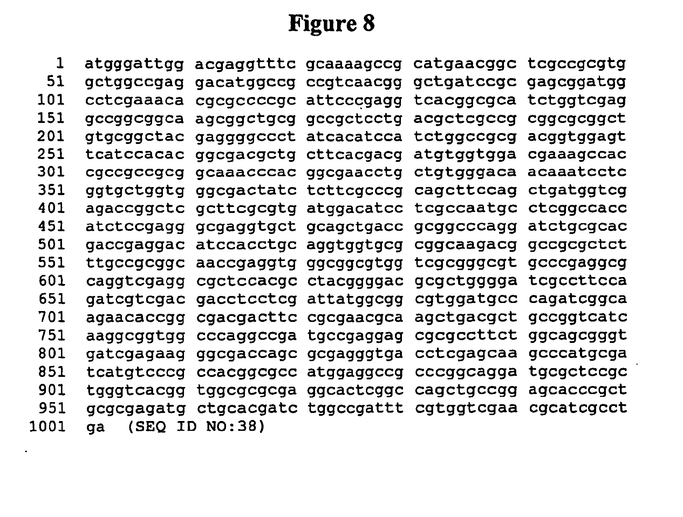

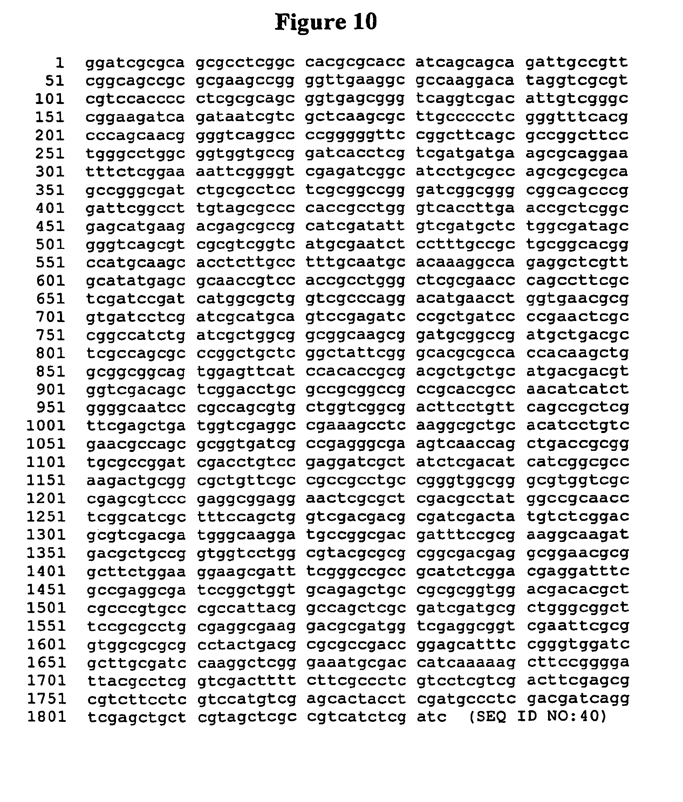

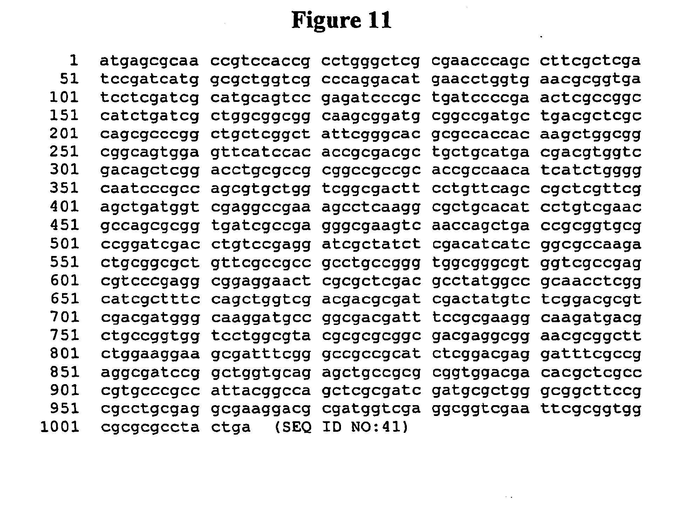

Figure 13 depicts the nucleic acid sequence depicted in Figure 8 (designated RSddsdna) and the nucleic acid sequence depicted in Figure 11 (designated STddsdna) aligned with each other as well as aligned with three other nucleic acid sequences. Examples of variations of the RSddsdna sequence include, without limitation, any variation of the RSddsdna sequence provided in Figure 13. Examples of variations of the STddsdna sequence include, without limitation, any variation of the STddsdna sequence provided in Figure 13. Such variations are provided in Figure 13 in that a comparison of the nucleotide (or lack thereof) at a particular position of the RSddsdna sequence or the STddsdna sequence with the nucleotide (or lack thereof) at the same position of any of the other nucleic acid sequences depicted in Figure 13 provides a list of specific changes for the RSddsdna sequence and the STddsdna sequence. For example, the "a" at position 511 of the RSddsdna sequence or the "a" at position 756 of the STddsdna sequence can be substituted with an "t" as indicated in Figure 13. Again, it will be appreciated that the RSddsdna sequence as well as the STddsdna sequence can contain any number of variations as well as any combination of types of variations. For example, the RSddsdna sequence can contain one variation provided in Figure 13 or more than one (e.g., 2, 3, 4, 5, 6, 7, 8, 9, 10, 15, 20, 25, 50, 100, or more) of the variations provided in Figure 13. Likewise, the STddsdna sequence can contain one variation provided in Figure 13 or more than one (e.g., 2, 3, 4, 5, 6, 7, 8, 9, 10, 15, 20, 25, 50, 100, or more) of the variations provided in Figure 13. It is noted that the full-length nucleic acid sequences depicted in Figure 13 can encode polypeptides having DDS activity. It also is noted that the nucleic acid sequence depicted in Figure 7 contains the nucleic acid sequence depicted in Figure 8 and that the nucleic acid sequence depicted in Figure 10 contains the nucleic acid sequence depicted in Figure 11.

The nucleic acid sequence depicted in Figure 7 contains a nucleic acid sequence that encodes a *R. sphaeroides* (ATCC 17023) polypeptide having DDS activity. Another variant of this nucleic acid sequence is the nucleic acid sequence of a clone isolated from *R. sphaeroides* (ATCC 35053). Briefly, a *R. sphaeroides* (ATCC 35053) clone was identified and found to contain a sequence identical to the nucleic acid sequence depicted in Figure 7 with the following three exceptions. The *R. sphaeroides* (ATCC 35053) clone has a "t" at position 885 rather than a "c", a "c" inserted after the "c" at position 1620, and a "c" inserted after the "c" at position 1733.

The nucleic acid depicted in Figure 8 also contains a nucleic acid sequence that encodes a *R. sphaeroides* (ATCC 17023) polypeptide having DDS activity. Another variant of this nucleic acid sequence is the nucleic acid sequence of a clone isolated from *R. sphaeroides* (ATCC 35053). Briefly, a *R. sphaeroides* (ATCC 35053) clone was identified and found to contain a sequence identical to the nucleic acid sequence depicted in Figure 8 with the following exception. The *R. sphaeroides* (ATCC 35053) clone has a "t" at position 514 rather than a "c".

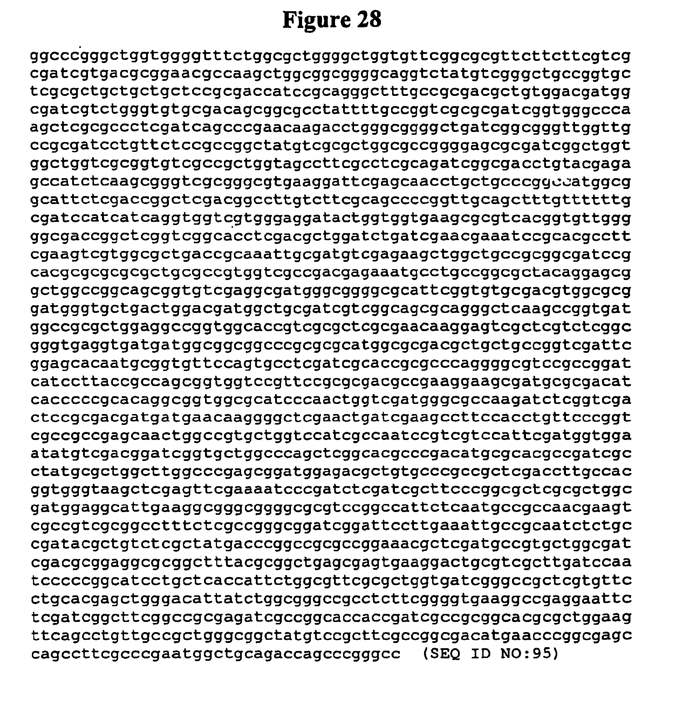

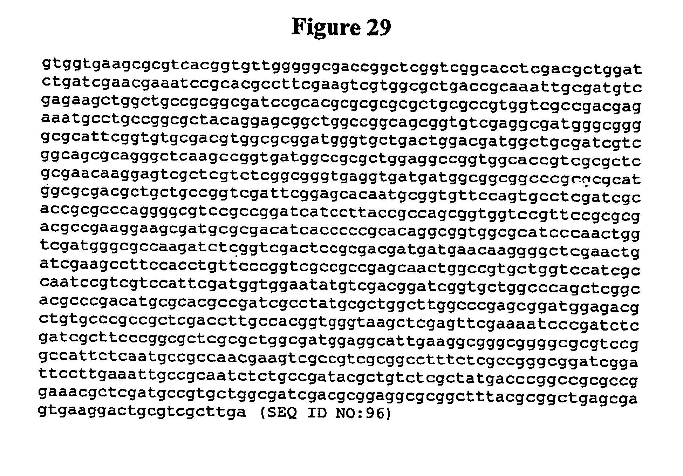

Figure 31 depicts the nucleic acid sequence depicted in Figure 29 (designated Stdxrcds) aligned with eleven other nucleic acid sequences. Examples of variations of the Stdxrcds sequence include, without limitation, any variation of the Stdxrcds sequence provided in Figure 31. Such variations are provided in Figure 31 in that a comparison of the nucleotide (or lack thereof) at a particular position of the Stdxrcds sequence with the nucleotide (or lack thereof) at the same position of any of the other nucleic acid sequences depicted in Figure 31 provides a list of specific changes for the Stdxrcds sequence. Again, it will be appreciated that the Stdxrcds sequence can contain any number of variations as well as any combination of types of variations. For example, the Stdxrcds sequence can contain one variation provided in Figure 31 or more than one (e.g., 2, 3, 4, 5, 6, 7, 8, 9, 10, 15, 20, 25, 50, 100, or more) of the variations provided in Figure 31. It is noted that the full-length nucleic acid sequences depicted in Figure 31 can encode polypeptides having DXR activity. It also is noted that the nucleic acid sequence depicted in Figure 29 contains the nucleic acid sequence depicted in Figure 28.

Also, an isolated nucleic acid is provided that contains a variant of a portion of the nucleic acid sequence depicted in Figure 2, 3, 7, 8, 10, 11, 28, or 29 as described herein.

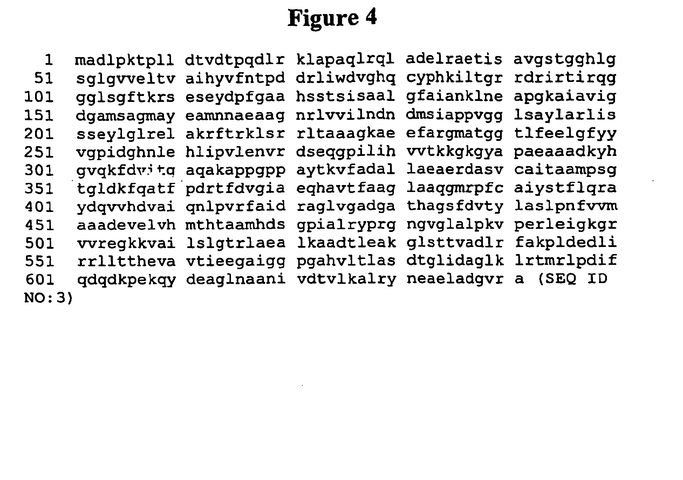

An isolated nucleic acid is provided that contains a nucleic acid sequence that encodes the entire amino acid sequence depicted in Figure 4, 9, 12, or 30. In addition an, isolated nucleic acid is provided that contains a nucleic acid sequence that encodes a portion of the amino acid sequence depicted in Figure 4, 9, 12, or 30. For example, an isolated nucleic acid is provided that contains a nucleic acid sequence that encodes a 15 amino acid sequence identical to any 15 amino acid sequence depicted in Figure 4, 9, 12, or 30 including, without limitation, the sequence starting at amino acid residue number 1 and ending at amino acid residue number 15, the sequence starting at amino acid residue number 2 and ending at amino acid residue number 16, the sequence starting at amino acid residue number 3 and ending at amino acid residue number 17, and so forth. It will be appreciated that also, an isolated nucleic acid is provided that contains a nucleic acid sequence that encodes an amino acid sequence that is greater than 15 amino acid residues (e.g., 16, 17, 18, 19, 20, 21, 22, 23, 24, 25, 26, 27, 28, 29, 30, or more amino acid residues) in length and identical to any portion of the sequence depicted in Figure 4, 9, 12, or 30. For example, an isolated nucleic acid is provided that contains a nucleic acid sequence that encodes a 25 amino acid sequence identical to any 25 amino acid sequence depicted in Figure 4, 9, 12, or 30 including, without limitation, the sequence starting at amino acid residue number 1 and ending at amino acid residue number 25, the sequence starting at amino acid residue number 2 and ending at amino acid residue number 26, the sequence starting at amino acid residue number 3 and ending at amino acid residue number 27, and so forth. Additional examples include, without limitation, isolated nucleic acids that contain a nucleic acid sequence that encodes an amino acid sequence that is 50 or more amino acid residues (e.g., 100, 150, 200, 250, 300, 350, or more amino acid residues) in length and identical to any portion of the sequence depicted in Figure 4,9,12, or 30. Such isolated nucleic acids can include, without limitation, those isolated nucleic acids containing a nucleic acid sequence that encodes an amino acid sequence represented in a single line of sequence depicted in Figure 4, 9, 12, or 30 since each line of sequence depicted in these figures, with the exception of the last line, provides a 50 amino acid sequence.

In addition, isolated nucleic acid that contains a nucleic acid sequence that encodes an amino acid sequence having a variation of the amino acid sequence depicted in Figure 4, 9, 12, or 30. For example, an isolated nucleic acid is provided containing a nucleic acid sequence encoding an amino acid sequence depicted in Figure 4, 9, 12, or 30 that contains a single insertion, a single deletion, a single substitution, multiple insertions, multiple deletions, multiple substitutions, or any combination thereof (e.g., single deletion together with multiple insertions). Multiple examples of isolated nucleic acid are provided containing a nucleic acid sequence encoding an amino acid sequence having a variation of an amino acid sequence depicted in Figure 4, 9, 12, or 30.

Figure 6 depicts the amino acid sequence depicted in Figure 4 (designated STdxsp) aligned with 20 other amino acid sequences. Examples of variations of the STdxsp sequence include, without limitation, any variation of the STdxsp sequence provided in Figure 6. Such variations are provided in Figure 6 in that a comparison of the amino acid residue (or lack thereof) at a particular position of the STdxsp sequence with the amino acid residue (or lack thereof) at the same position of any of the other 20 amino acid sequences depicted in Figure 6 provides a list of specific changes for the STdxsp sequence. For example, the "t" at position 1148 of the STdxsp sequence can be substituted with an "s" as indicated in Figure 6. As also indicated in Figure 6, the "f' at position 575 of the STdxsp sequence can be substituted with an "m", "a", "l", "i", "y", or "v". For Figure 6, the nucleic acid numbering of Figure 2 is used to number the amino acid residue positions of the STdxsp sequence. Thus, the first amino acid residue of the STdxsp sequence starts with number 182 and proceeds in increments of three. It will be appreciated that the STdxsp sequence can contain any number of variations as well as any combination of types of variations. For example, the STdxsp sequence can contain one variation provided in Figure 6 or more than one (e.g.. 2, 3, 4, 5, 6, 7, 8, 9, 10, 15, 20, 25, 50, 100, or more) of the variations provided in Figure 6. It is noted that the 21 full-length amino acid sequences depicted in Figure 6 can be polypeptides having DXS activity.

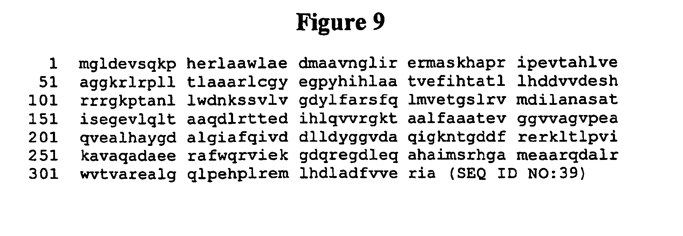

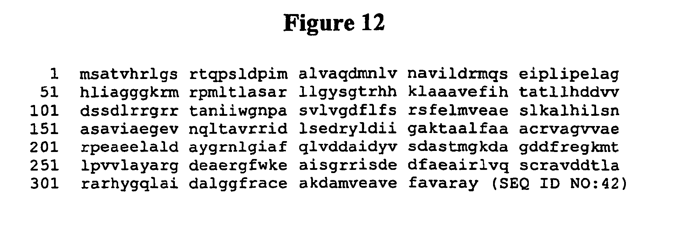

Figure 14 depicts the amino acid sequence depicted in Figure 9 (designated RSddsp) and the amino acid sequence depicted in Figure 12 (designated STddsp) aligned with each other as well as aligned with three other amino acid sequences. For Figure 14, the nucleic acid numbering of Figure 7 is used to number the amino acid residue positions of the RSddsp sequence, and the nucleic acid numbering of Figure 10 is used to number the amino acid residue positions of the STddsp sequence. Thus, the first amino acid residue of the RSddsp and STddsp sequences each start with a number other than 1 and proceed in increments of three. Examples of variations of the RSddsp sequence include, without limitation, any variation of the RSddsp sequence provided in Figure 14. Examples of variations of the STddsp sequence include, without limitation, any variation of the STddsp sequence provided in Figure 14. Such variations are provided in Figure 14 in that a comparison of the amino acid residue (or lack thereof) at a particular position of the RSddsp sequence or the STddsp sequence with the amino acid residue (or lack thereof) at the same position of any of the other amino acid sequences depicted in Figure 14 provides a list of specific changes for the RSddsp sequence and the STddsp sequence. For example, the "1" at position 762 of the RSddsp sequence or the "1" at position 1007 of the STddsp sequence can be substituted with an "a" as indicated in Figure 14. Again, it will be appreciated that the RSddsp sequence as well as the STddsp sequence can contain any number of variations as well as any combination of types of variations. For example, the RSddsp sequence can contain one variation provided in Figure 14 or more than one (e.g., 2, 3, 4, 5, 6, 7, 8, 9, 10, 15, 20, 25, 50, 100, or more) of the variations provided in Figure 14. Likewise, the STddsp sequence can contain one variation provided in Figure 14 or more than one (e.g., 2, 3, 4, 5, 6, 7, 8, 9, 10, 15, 20, 25, 50, 100, or more) of the variations provided in Figure 14. It is noted that the five full-length amino acid sequences depicted in Figure 14 can be polypeptides having DDS activity.

The amino acid sequence depicted in Figure 9 represents a *R. sphaeroides* (ATCC 17023) polypeptide having DDS activity. Another variant of this amino acid sequence is the amino acid sequence encoded by a clone isolated from *R. sphaeroides* (ATCC 35053). Briefly, a *R. sphaeroides* (ATCC 35053) clone was identified and found to encode an amino acid sequence identical to the amino acid sequence depicted in Figure 9 with the following exception. The *R. sphaeroides* (ATCC 35053) clone has a "y" at position 172 rather than an "h".

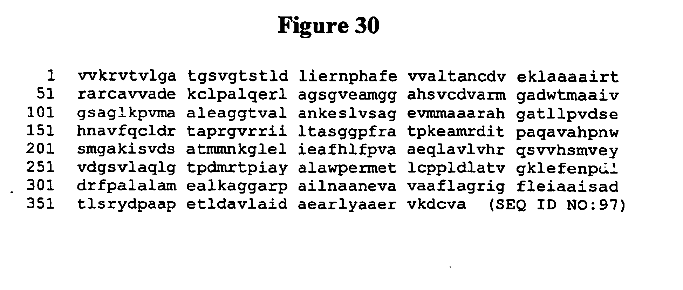

Figure 32 depicts the amino acid sequence depicted in Figure 30 (designated Stdxrp) aligned with 15 other amino acid sequences. Examples of variations of the Stdxrp sequence include, without limitation, any variation of the Stdxrp sequence provided in Figure 32. Such variations are provided in Figure 32 in that a comparison of the amino acid residue (or lack thereof) at a particular position of the Stdxrp sequence with the amino acid residue (or lack thereof) at the same position of any of the other 15 amino acid sequences depicted in Figure 32 provides a list of specific changes for the Stdxrp sequence. It will be appreciated that the Stdxrp sequence can contain any number of variations as well as any combination of types of variations. For example, the Stdxrp sequence can contain one variation provided in Figure 32 or more than one (e.g., 2, 3, 4, 5, 6, 7, 8, 9, 10, 15, 20, 25, 50, 100, or more) of the variations provided in Figure 32. It is noted that the full-length amino acid sequences depicted in Figure 32 can be polypeptides having DXR activity.

An isolated nucleic acid is also provided containing a nucleic acid sequence encoding an amino acid sequence that contains a variant of a portion of the amino acid sequence depicted in Figure 4, 9, 12, or 30 as described herein.

*2. Polypeptides*

Substantially pure polypeptides are provided. The term "substantially pure" as used herein with reference to a polypeptide means the polypeptide is substantially free of other polypeptides, lipids, carbohydrates, and nucleic acid with which it is naturally associated. Thus, a substantially pure polypeptide is any polypeptide that is removed from its natural environment and is at least 60 percent pure. A substantially pure polypeptide can be at least about 65, 70, 75, 80, 85, 90, 95, or 99 percent pure. Typically, a substantially pure polypeptide will yield a single major band on a non-reducing polyacrylamide gel.

Any substantially pure polypeptide having an amino acid sequence encoded by a nucleic acid described herein is contemplated. In addition, any substantially pure polypeptide containing an amino acid sequence having a length and a percent identity to the sequence set forth in SEQ ID NO:3 over that length as determined herein is contemplated provided the point defined by that length and percent identity is within the area defined by points A, B, C, and D of Figure 26; where point A has an X coordinate less than or equal to 641, and a Y coordinate less than or equal to 100; where point B has an X coordinate less than or equal to 641, and a Y coordinate greater than or equal to 50; where point C has an X coordinate greater than or equal to 25, and a Y coordinate greater than or equal to 50; and where point D has an X coordinate greater than or equal to 5, and a Y coordinate less than or equal to 100. For example, the X coordinate for point A can be 641, 635, 630, 625, 620, or less; and the Y coordinate for point A can be 100, 99, 95, 90, 85, 80, 75, or less. The X coordinate for point B can be 641, 635, 630, 625, 620, or less; and the Y coordinate for point B can be 50, 55, 60, 65, 70, 75, 80, 85, 90, 95, 99 or more. The X coordinate for point C can be 25, 30, 35, 40, 50, 60, 70, 80, 90, 100, 150, 200, or more; and the Y coordinate for point C can be 50, 55, 60, 65, 70, 75, 80, 85, 90, 95, 99 or more. The X coordinate for point D can be 5, 6, 7, 8, 9, 10, 15,20,25, 30, 40, 50,75, 100, or more; and the Y coordinate for point D can be 100, 99, 95, 90, 85, 80, 75, or less. In one example point A can be (641, 100), point B can be (641, 95), point C can be (400, 95), and point D can be (400, 100).

Any substantially pure polypeptide containing an amino acid sequence having a length and a percent identity to the sequence set forth in SEQ ID NO:39 over that length as determined herein is contemplated provided the point defined by that length and percent identity is within the area defined by points A, B, C, and D of Figure 26; where point A has an X coordinate less than or equal to 333, and a Y coordinate less than or equal to 100; where point B has an X coordinate less than or equal to 333, and a Y coordinate greater than or equal to 50; where point C has an X coordinate greater than or equal to 25, and a Y coordinate greater than or equal to 50; and where point D has an X coordinate greater than or equal to 5, and a Y coordinate less than or equal to 100. For example, the X coordinate for point A can be 333, 330, 325, 320, 315, or less; and the Y coordinate for point A can be 100, 99, 95, 90, 85, 80, 75, or less. The X coordinate for point B can be 333, 330, 325, 320, 315, or less; and the Y coordinate for point B can be 50, 55, 60, 65, 70, 75, 80, 85, 90, 95, 99 or more. The X coordinate for point C can be 25, 30, 35, 40, 50, 60, 70, 80, 90, 100,150, 200, or more; and the Y coordinate for point C can be 50, 55, 60, 65, 70, 75, 80, 85, 90, 95, 99 or more. The X coordinate for point D can be 5, 6, 7, 8, 9, 10, 15, 20, 25, 30, 40, 50, 75, 100, or more; and the Y coordinate for point D can be 100, 99, 95, 90, 85, 80, 75, or less. In one example point A can be (333,100), point B can be (333, 95), point C can be (150, 95), and point D can be (150, 100).

Any substantially pure polypeptide containing an amino acid sequence having a length and a percent identity to the sequence set forth in SEQ ID NO:42 over that length as determined herein is contemplated provided the point defined by that length and percent identity is within the area defined by points A, B, C, and D of Figure 26; where point A has an X coordinate less than or equal to 337, and a Y coordinate less than or equal to 100; where point B has an X coordinate less than or equal to 337, and a Y coordinate greater than or equal to 50; where point C has an X coordinate greater than or equal to 25, and a Y coordinate greater than or equal to 50; and where point D has an X coordinate greater than or equal to 5, and a Y coordinate less than or equal to 100. For example, the X coordinate for point A can be 337, 335, 330, 325, 320, 315, or less; and the Y coordinate for point A can be 100, 99, 95, 90, 85, 80, 75, or less. The X coordinate for point B can be 337, 335, 330, 325, 320, 315, or less; and the Y coordinate for point B can be 50, 55, 60, 65, 70, 75, 80, 85, 90, 95, 99 or more. The X coordinate for point C can be 25, 30, 35, 40, 50, 60, 70, 80, 90, 100, 150, 200, or more; and the Y coordinate for point C can be 50, 55, 60, 65, 70, 75, 80, 85, 90, 95, 99 or more. The X coordinate for point D can be 5, 6, 7, 8, 9, 10, 15, 20, 25, 30, 40, 50, 75, 100, or more; and the Y coordinate for point D can be 100, 99, 95, 90, 85, 80, 75, or less. In one example, point A can be (337, 100), point B can be (337, 95), point C can be (150, 95), and point D can be (150, 100).

Any substantially pure polypeptide containing an amino acid sequence having a length and a percent identity to the sequence set forth in SEQ ID NO:97 over that length as determined herein is contemplated provided the point defined by that length and percent identity is within the area defined by points A, B, C, and D of Figure 26; where point A has an X coordinate less than or equal to 386, and a Y coordinate less than or equal to 100; where point B has an X coordinate less than or equal to 386, and a Y coordinate greater than or equal to 50; where point C has an X coordinate greater than or equal to 25, and a Y coordinate greater than or equal to 50; and where point D has an X coordinate greater than or equal to 5, and a Y coordinate less than or equal to 100. For example, the X coordinate for point A can be 386, 380, 375, 370, 375, 360, 365, 350, 325, 300, or less; and the Y coordinate for point A can be 100, 99, 95, 90, 85, 80, 75, or less. The X coordinate for point B can be 386, 380, 375, 370, 375, 360, 365, 350, 325, 300, or less; and the Y coordinate for point B can be 50, 55, 60, 65, 70, 75, 80, 85, 90, 95, 99 or more. The X coordinate for point C can be 25, 30, 35, 40, 50, 60, 70, 80, 90, 100, 150, 200, 300, 350, or more; and the Y coordinate for point C can be 50, 55, 60, 65, 70, 75, 80, 85, 90, 95,99 or more. The X coordinate for point D can be 5, 6, 7, 8, 9, 10, 15, 20, 25, 30, 40, 50, 75, 100, 200, 300, 350, or more; and the Y coordinate for point D can be 100, 99, 95, 90, 85, 80, 75, or less. In one example, point A can be (386, 100), point B can be (386, 95), point C can be (350, 95), and point D can be (350, 100).

Any method can be used to obtain a substantially pure polypeptide. For example, common polypeptide purification techniques such as affinity chromotography and HPLC as well as polypeptide synthesis techniques can be used. In addition, any material can be used as a source to obtain a substantially pure polypeptide. For example, tissue from wild-type or transgenic animals can be used as a source material. In addition, tissue culture cells engineered to overexpress a particular polypeptide of interest can be used to obtain substantially pure polypeptide. Further, a polypeptide described herein can be "engineered" to contain an amino acid sequence that allows the polypeptide to be captured onto an affinity matrix. For example, a tag such as c-myc, hemagglutinin, polyhistidine, or Flag™ tag (Kodak) can be used to aid polypeptide purification. Such tags can be inserted anywhere within the polypeptide including at either the carboxyl or amino termini. Other fusions that could be useful include enzymes that aid in the detection of the polypeptide, such as alkaline phosphatase.

Polypeptides are provided that contain the entire amino acid sequence depicted in Figure 4, 9, 12, or 30. In addition, polypeptides are provided that contain a portion of the amino acid sequence depicted in Figure 4, 9, 12, or 30. For example, polypeptides are provided that contain a 15 amino acid sequence identical to any 15 amino acid sequence depicted in Figure 4, 9, 12, or 30 including, without limitation, the sequence starting at amino acid residue number 1 and ending at amino acid residue number 15, the sequence starting at amino acid residue number 2 and ending at amino acid residue number 16, the sequence starting at amino acid residue number 3 and ending at amino acid residue number 17, and so forth. It will be appreciated that also, polypeptides are provided that contain an amino acid sequence that is greater than 15 amino acid residues (e.g., 16, 17, 18, 19, 20, 21, 22, 23, 24, 25, 26, 27, 28, 29, 30, or more amino acid residues) in length and identical to any portion of the sequence depicted in Figure 4, 9, 12, or 30. For example, polypeptides are provided that contain a 25 amino acid sequence identical to any 25 amino acid sequence depicted in Figure 4, 9, 12, or 30 including, without limitation, the sequence starting at amino acid residue number 1 and ending at amino acid residue number 25, the sequence starting at amino acid residue number 2 and ending at amino acid residue number 26, the sequence starting at amino acid residue number 3 and ending at amino acid residue number 27, and so forth. Additional examples include, without limitation, polypeptides that contain an amino acid sequence that is 50 or more amino acid residues (e.g., 100, 150, 200, 250, 300, 350, or more amino acid residues) in length and identical to any portion of the sequence depicted in Figure 4, 9, 12, or 30. Such polypeptides can include, without limitation, those polypeptides containing a amino acid sequence represented in a single line of sequence depicted in Figure 4, 9,12, or 30 since each line of sequence depicted in these figures, with the possible exception of the last line, provides a 50 amino acid sequence.

In addition, polypeptides are provided that an amino acid sequence having a variation of the amino acid sequence depicted in Figure 4, 9, 12, or 30. For example, polypeptides are provided containing an amino acid sequence depicted in Figure 4, 9, 12, or 30 that contains a single insertion, a single deletion, a single substitution, multiple insertions, multiple deletions, multiple substitutions, or any combination thereof (e.g., single deletion together with multiple insertions). Multiple examples of polypeptides are provided containing an amino acid sequence having a variation of an amino acid sequence depicted in Figure 4, 9, 12, or 30.

Figure 6 depicts the amino acid sequence depicted in Figure 4 (designated STdxsp) aligned with 20 other amino acid sequences. Examples of variations of the STdxsp sequence include, without limitation, any variation of the STdxsp sequence provided in Figure 6. Such variations are provided in Figure 6 in that a comparison of the amino acid residue (or lack thereof) at a particular position of the STdxsp sequence with the amino acid residue (or lack thereof) at the same position of any of the other 20 amino acid sequences depicted in Figure 6 provides a list of specific changes for the STdxsp sequence. For example, the "t" at position 1148 of the STdxsp sequence can be substituted with an "s" as indicated in Figure 6. As also indicated in Figure 6, the "f" at position 575 of the STdxsp sequence can be substituted with an "m", "a", "l", "i", "y", or "v". For Figure 6, the nucleic acid numbering of Figure 2 is used to number the amino acid residue positions of the STdxsp sequence. Thus, the first amino acid residue of the STdxsp sequence starts with number 182 and proceeds in increments of three. It will be appreciated that the STdxsp sequence can contain any number of variations as well as any combination of types of variations. For example, the STdxsp sequence can contain one variation provided in Figure 6 or more than one (e.g., 2, 3, 4, 5, 6, 7, 8, 9, 10, 15, 20, 25, 50,100, or more) of the variations provided in Figure 6. It is noted that the 21 full-length amino acid sequences depicted in Figure 6 can be polypeptides having DXS activity.

Figure 14 depicts the amino acid sequence depicted in Figure 9 (designated RSddsp) and the amino acid sequence depicted in Figure 12 (designated STddsp) aligned with each other as well as aligned with three other amino acid sequences. For Figure 14, the nucleic acid numbering of Figure 7 is used to number the amino acid residue positions of the RSddsp sequence, and the nucleic acid numbering of Figure 10 is used to number the amino acid residue positions of the STddsp sequence. Thus, the first amino acid residue of the RSddsp and STddsp sequences each start with a number other than 1 and proceed in increments of three. Examples of variations of the RSddsp sequence include, without limitation, any variation of the RSddsp sequence provided in Figure 14. Examples of variations of the STddsp sequence include, without limitation, any variation of the STddsp sequence provided in Figure 14. Such variations are provided in Figure 14 in that a comparison of the amino acid residue (or lack thereof) at a particular position of the RSddsp sequence or the STddsp sequence with the amino acid residue (or lack thereof) at the same position of any of the other amino acid sequences depicted in Figure 14 provides a list of specific changes for the RSddsp sequence and the STddsp sequence. For example, the "1" at position 762 of the RSddsp sequence or the "1" at position 1007 of the STddsp sequence can be substituted with an "a" as indicated in Figure 14. Again, it will be appreciated that the RSddsp sequence as well as the STddsp sequence can contain any number of variations as well as any combination of types of variations. For example, the RSddsp sequence can contain one variation provided in Figure 14 or more than one (e.g., 2, 3, 4, 5, 6, 7, 8, 9, 10, 15, 20, 25, 50, 100, or more) of the variations provided in Figure 14. Likewise, the STddsp sequence can contain one variation provided in Figure 14 or more than one (e.g., 2, 3, 4, 5, 6, 7, 8, 9, 10, 15, 20, 25, 50, 100, or more) of the variations provided in Figure 14. It is noted that the five full-length amino acid sequences depicted in Figure 14 can be polypeptides having DDS activity.

Figure 32 depicts the amino acid sequence depicted in Figure 30 (designated Stdxrp) aligned with 15 other amino acid sequences. Examples of variations of the Stdxrp sequence include, without limitation, any variation of the Stdxrp sequence provided in Figure 32. Such variations are provided in Figure 32 in that a comparison of the amino acid residue (or lack thereof) at a particular position of the Stdxrp sequence with the amino acid residue (or lack thereof) at the same position of any of the other 15 amino acid sequences depicted in Figure 32 provides a list of specific changes for the Stdxrp sequence. It will be appreciated that the Stdxrp sequence can contain any number of variations as well as any combination of types of variations. For example, the Stdxrp sequence can contain one variation provided in Figure 32 or more than one (e.g., 2, 3, 4, 5, 6, 7, 8, 9, 10, 15, 20, 25, 50, 100, or more) of the variations provided in Figure 32. It is noted that the full-length amino acid sequences depicted in Figure 32 can be polypeptides having DXR activity.

Also, polypeptides are provided containing an amino acid sequence that contains a variant of a portion of the amino acid sequence depicted in Figure 4, 9, 12, or 30 as described herein.

*3. Genetically modified cells*

Any cell containing an isolated nucleic acid described herein is contemplated. This includes, without limitation, prokaryotic cells such as cells from the Rhodospirillaceae family (e.g., *Rhodobacter* cells) and eukaryotic cells such as plant and mammalian cells. It is noted that cells containing an isolated nucleic acid as described herein are not required to express the isolated nucleic acid. In addition, the isolated nucleic acid can be integrated into the genome of the cell or maintained in an episomal state. In other words, cells can be stably or transiently transformed with an isolated nucleic acid as described herein.

Any method can be used to introduce an isolated nucleic acid into a cell. In fact, many methods for introducing nucleic acid into a cell, whether *in vivo* or *in vitro,* are well known to those skilled in the art. For example, calcium phosphate precipitation, electroporation, heat shock, lipofection, microinjection, conjugation, and viral-mediated nucleic acid transfer are common methods that can be used to introduce nucleic acid into a cell. In addition, naked DNA can be delivered directly to cells *in vivo* as describe elsewhere (U.S. Patent Number 5,580,859 and U.S. Patent Number 5,589,466 including continuations thereof). Further, nucleic acid can be introduced into cells by generating transgenic animals.

Any method can be used to identify cells that contain an isolated nucleic acid described herein. For example, PCR and nucleic acid hybridization techniques such as Northern and Southern analysis can be used. In some cases, immunohistochemistry and biochemical techniques can be used to determine if a cell contains a particular nucleic acid by detecting the expression of a polypeptide encoded by that particular nucleic acid. For example, detection of polypeptide X-immunoreactivity after introduction of an isolated nucleic acid containing a cDNA that encodes polypeptide X into a cell that does not normally express polypeptide X can indicate that that cell not only contains the introduced nucleic acid but also expresses the encoded polypeptide X from that introduced nucleic acid. In this case, the detection of any enzymatic activities of polypeptide X also can indicate that that cell contains the introduced nucleic acid and expresses the encoded polypeptide X from that introduced nucleic acid.

Any method can be used to direct the expression of an amino acid sequence from a nucleic acid. Such methods are well known to those skilled in the art, and include, without limitation, constructing a nucleic acid such that a regulatory element drives the expression of a nucleic acid sequence that encodes a polypeptide. Typically, regulatory elements are DNA sequences that regulate the expression of other DNA sequences at the level of transcription. Such regulatory elements include, without limitation, promoters, enhancers, and the like. In addition, any method for expressing a polypeptide from an exogenous nucleic acid molecule in microorganisms such as bacteria and yeast can be used. For example, well-known methods for making and using nucleic acid constructs that are capable of expressing exogenous polypeptides within *Rhodobacter* species (e.g., *R. sphaeroides* and *R. capsulatus)* can be used. *See, e.g.,* Dryden and Dowhan, J. Bacteriol., 178(4):1030-1038 (1996); Vasilyeva et al., Applied Biochemistry and Biotechnology, 77-79:337-345 (1999); Graichen et al., J. Bacteriol., 181(14):4216-4222 (1999); Johnson et al., J Bacteriol., 167(2):604-610 (1986); and Duport et al., Gene, 145:103-108 (1994). Further, any methods can be used to identify cells that express an amino acid sequence from a nucleic acid. Such methods are well known to those skilled in the art, and include, without limitation, immunocytochemistry, Western analysis, Northern analysis, and RT-PCR

The cells described herein can contain a single copy, or multiple copies (e.g., about 5, 10, 20, 35, 50, 75, 100 or 150 copies), of a particular exogenous nucleic acid. For example, a bacterial cell can contain about 50 copies of exogenous nucleic acid X. In addition, the cells described herein can contain more than one particular exogenous nucleic acid. For example, a bacterial cell can contain about 50 copies of exogenous nucleic acid X as well as about 75 copies of exogenous nucleic acid Y. In these cases, each different nucleic acid can encode a different polypeptide having its own unique enzymatic activity. For example, a bacterial cell can contain two different exogenous nucleic acids such that a high level of CoQ(10) is produced. In this example, such a cell can contain a first exogenous nucleic acid that encodes a polypeptide having DXS activity and a second exogenous nucleic acid that encodes a polypeptide having DDS activity. In addition, a single exogenous nucleic acid can encode one or more than one polypeptide. For example, a single nucleic acid can contain sequences that encode three different polypeptides.

In addition to providing cells that contain an isolated nucleic acid of the invention cells (e.g., plant cells, animal cells, and microorganisms are provided that can be used to produce an isoprenoid compound such as CoQ(10). The term "microorganism" as used herein refers to all microscopic organisms including; without limitation, bacteria, algae, fungi, and protozoa. It is noted that bacteria cells can be membraneous bacteria or non-membraneous bacteria.

The term "non-membraneous bacteria" as used herein refers to any bacteria lacking intracytoplasmic membrane. The term "membraneous bacteria" as used herein refers to any naturally-occurring, genetically modified, or environmentally modified bacteria having an intracytoplasmic membrane. An intracytoplasmic membrane can be organized in a variety of ways including, without limitation, vesicles, tubules, thylakoid-like membrane sacs, and highly organized membrane stacks. Any method can be used to analyze bacteria for the presence of intracytoplasmic membranes including, without limitation, electron microscopy, light microscopy, and density gradients. *See, e.g.,* Chory et al., J. Bacteriol., 159:540-554 (1984); Niederman and Gibson, Isolation and Physiochemical Properties of Membranes from Purple Photosynthetic Bacteria. In: The Photosynthetic Bacteria, Ed. By Roderick K. Clayton and William R. Sistrom, Plenum Press, pp. 79-118 (1978); and Lueking et al., J. Biol. Chem., 253: 451-457 (1978). Examples of membraneous bacteria that can be used herein include, without limitation, bacteria of the Rhodospirillaceae family such as those in the genus Rhodobacter (e.g., *R. sphaeroides, R. capsulatus, R. sulfidophilus, R. adriaticus,* and *R. veldkampii),* the genus Rhodospirillum (e.g., *R. rubrum, R. photometricum, R. molischianum, R. fulvum,* and R. *salinarum),* the genus Rhodopseudomonas (e.g., *R. palustris, R. viridis,* and *R. sulfoviridis),* the genus Rhodomicrobium, the genus Rhodocyclus, and the genus Rhodopila; bacteria of the Chromatiaceae family such as those in the genus Chromatium, genus Thiocystis, the genus Thiospirillum, the genus Thiocapsa, the genus Lamprobacter, the genus Lalmprocystis, the genus Thiodictyon, the genus Amoebobacter, and the genus Thiopedia; green sulfur bacteria such as those in the genus Chlorobium and the genus Prosthecochloris; bacteria of the Methylococcaceae family such as those in the genus Methylococcus (e.g., *M. capsulatus*), and the genus Methylomonas (e.g., *M. methanica);* and particular bacteria of the Nitrobacteraceae family such as those in the genus Nitrobacter (e.g., *N. winogradsky* and *N. hamburgensis),* the genus Nitrococcus (e.g., *N. mobilis),* and the genus Nitrosomonas (e.g., *N. europaea).*

Membraneous bacteria can be highly membraneous bacteria. The term "highly membraneous bacteria" as used herein refers to any bacterium having more intracytoplasmic membrane than *R. sphaeroides* (ATCC 17023) cells have after the *R. sphaeroides* (ATCC 17023) cells have been (1) cultured chemoheterotrophically under aerobic conditions for four days, (2) cultured chemoheterotrophically under oxygen-limited conditions for four hours, and (3) harvested. The aerobic culture conditions involve culturing the cells in the dark at 30°C in the presence of 25 percent oxygen. The oxygen-limited conditions involve culturing the cells in the light at 30°C in the presence of 2 percent oxygen. After the four hour culturing step under oxygen-limited conditions, the *R. sphaeroides* (ATCC 17023) cells are harvested by centrifugation and analyzed.

Typically, any cell (e.g., membraneous bacteria) can be genetically modified such that a particular isoprenoid compound is produced. Such cells can contain exogenous nucleic acid that encodes a polypeptide having enzymatic activity. For example, a microorganism having endogenous DDS activity can be transformed with an exogenous nucleic acid that encodes a polypeptide having DDS activity. In this case, the microorganism can have increased DDS activity which can lead to an increased production of CoQ(10). Thus, a cell can be given an exogenous nucleic acid that encodes a polypeptide having an enzymatic activity that catalyzes the production of a compound normally produced by that cell. In this case, the genetically modified cell can produce more of the compound, or can produce the compound more efficiently, than a similar cell not having the genetic modification. Alternatively, a cell can be given an exogenous nucleic acid that encodes a polypeptide having an enzymatic activity that catalyzes the production of a compound that is not normally produced by that cell.

The invention provides cells containing exogenous nucleic acid that encodes a polypeptide having enzymatic activity that leads to an increased production of CoQ(10). Such cells can contain nucleic acid that encodes a polypeptide having DDS activity. Other examples include, without limitation, cells containing exogenous nucleic acid that encodes polypeptides having DXS and/or chorismate lyase (e.g., ubiC) activity. Nucleic acid molecules that encode polypeptides having such enzymatic activities can be obtained as described herein. For example, nucleic acid encoding a polypeptide having chorismate lyase can be cloned using the sequence information provided in Genbank® accession number X66619.

Typically, microorganisms of the invention produce CoQ(10) with the yield (mg of CoQ(10) per g of dry biomass) being at least about 5 (e.g., at least about 6, 7, 8, 9, 10, 11, 12, 13, 14, 15, 20, 25, 30, 35, or more) percent greater than that of a comparable wild-type strain grown under similar conditions. Bacteria can produce more CoQ(10) when grown under anaerobic conditions as compared to aerobic conditions. For example, anaerobically cultured bacteria can produce about 3 to 4 fold more CoQ(10) than aerobically cultured bacteria of the same species. When determining the yield of isoprenoid compound production for a particular cell (e.g., microorganism), any method can be used. *See, e.g.,* Cohen-Bazire et al., J. Cell Comp. Physiol., 49:25-68 (1957); Edlund, J. Chromatogr., 425:87-97 (1988); Rousseau and Varin, J Chromatogr. Sci., 36:247-52 (1998); and Leray et al., J. Lipid Res., 39:2099-2105 (1998).

A cell is provided containing an exogenous nucleic acid that encodes a polypeptide having DXS, DDS, ODS, SDS, DXR, 4-diphosphocytidyl-2C-methyl-D-erythritol synthase (e.g., ispD), 4-diphosphocytidyl-2C-methyl-D-erythritol kinase (e.g., ispE), and/or chorismate lyase (e.g., ubiC) activity. Nucleic acid molecules that encode polypeptides having such enzymatic activities can be obtained as described herein. Also, a cell is provided that contains more than one different exogenous nucleic acid molecule with each different exogenous nucleic acid molecule encoding a polypeptide having a different one of the following enzymatic activities: DXS, DDS, ODS, SDS, DXR, 4-diphosphocytidyl-2C-methyl-D-erythritol synthase (e.g., ispD), 4-diphosphocytidyl-2C-methyl-D-erythritol kinase (e.g., ispE), and/or chorismate lyase (e.g., ubiC) activity. For example, the invention provides a cell containing a first exogenous nucleic acid encoding a polypeptide having DXS activity and a second exogenous nucleic acid encoding a polypeptide having DDS activity.

A cell is provided containing an exogenous nucleic acid containing a dxs sequence (e.g., Stdxs sequence), dds sequence (e.g., Stdds or Rsdds sequence), dxr sequence (e.g., Stdxr sequence), ubiC sequence (e.g., EcUbiC sequence), or lytb sequence (e.g., RsLytB sequence). Such nucleic acids can be obtained as described herein. Also a cell is provided that contains more than one of the following sequences: a dxs sequence (e.g., Stdxs sequence), dds sequence (e.g., Stdds or Rsdds sequence), dxr sequence (e.g., Stdxr sequence), ubiC sequence (e.g., EcUbiC sequence), or lytB sequence (e.g., RsLytB sequence). For example, the invention provides a cell containing a first exogenous nucleic acid containing a dds sequence and a second exogenous nucleic acid containing a dxs sequence. Likewise, a cell is provided containing a single exogenous nucleic acid that contains a dds sequence and a dxs sequence.

Typically, a microorganism within the scope of the invention catabolizes a hexose carbon such as glucose. A microorganism, however, can catabolize a pentose carbon (e.g., ribose, arabinose, xylose, and lyxose). In other words, a microorganism within the scope of the invention can either utilize hexose or pentose carbon. In addition, a microorganism within the scope of the invention can use carbon sources such as methanol and/or organic acids (e.g., succinic acid or malic acid).

Any cells described herein can have reduced enzymatic activity such as reduced geranylgeranyl pyrophosphate synthase and/or magnesium protoporphyrin IX chelatase activity. Any cell described herein can have reduced biological activity such as reduced activity of aerobic repressor polypeptides (e.g., PPSR) or oxidation-reduction sensor polypeptides (e.g., CBB3). In the case of multi-subunit molecules such as CBB3, the activity of the oxidation-reduction sensor polypeptide can be reduced by inactivating one or more than one of the subunits. For example, CBB3 activity can be reduced by inactivating a single subunit of CBB3 such as the ccoN subunit

The term "reduced" as used herein with respect to a cell and a particular activity (e.g., particular enzymatic activity) refers to a lower level of activity than that measured in a comparable cell of the same species. Thus, a *R. sphaeroides* cell lacking geranylgeranyl pyrophosphate synthase activity is considered to have reduced geranylgeranyl pyrophosphate synthase activity since most, if not all, comparable *R. sphaeroides* cells have at least some geranylgeranyl pyrophosphate synthase activity. Such reduced enzymatic activities can be the result of lower enzyme concentration, lower specific activity of an enzyme, or combinations thereof.

Many different methods can be used to make a cell having reduced enzymatic and/or biological activity. For example, a *R. sphaeroides* cell can be engineered to have a disrupted enzyme-encoding locus using common mutagenesis or knock-out technology. Alternatively, antisense technology can be used to reduce enzymatic activity. For example, a *R. sphaeroides* cell can be engineered to contain a cDNA that encodes an antisense molecule that prevents an enzyme from being made. The term "antisense molecule" as used herein encompasses any nucleic acid that contains sequences that correspond to the coding strand of an endogenous polypeptide. An antisense molecule also can have flanking sequences (e.g., regulatory sequences). Thus, antisense molecules can be ribozymes or antisense oligonucleotides. A ribozyme can have any general structure including, without limitation, hairpin, hammerhead, or axhead structures, provided the molecule cleaves RNA.

Cells having a reduced enzymatic and/or biological activity can be identified using any method. For example, a *R. sphaeroides* cell having reduced geranylgeranyl pyrophosphate synthase activity can be easily identified using common biochemical methods that measure geranylgeranyl pyrophosphate synthase activity. *See, e.g.,* Math et al., Proc. Natl. Acad. Sci. USA, 89(15):6761-6764.(1992).

A cell is provided containing reduced geranylgeranyl diphosphate synthase, aerobic repressor, and/or cbb3-type cytochrome oxidase activity. Such cells can have reduced geranylgeranyl diphosphate synthase, aerobic repressor, and/or cbb3-type cytochrome oxidase activity as a result of disrupting the endogenous sequences that encode polypeptides having these activities. For example, a cell can have reduced geranylgeranyl diphosphate synthase activity as a result of knocking out a portion of the endogenous crtE sequence within a cell's genome; a cell can have reduced aerobic repressor activity as a result of knocking out a portion of the endogenous ppsR sequence within a cell's genome; and a cell can have reduced cbb3-type cytochrome oxidase activity as a result of knocking out a portion of the endogenous ccoN sequence within a cell's genome.

Also, a cell is provided containing non-functional crtE, ppsR, and/or ccoN nucleic acid sequences within its genome such that the encoded polypeptide is either mutated or not expressed. Such cells can be used to produce large amounts of CoQ(10). The sequence of crtE can be as set forth in Genbank® accession number AJ010302. The sequence of ppsR can be as set forth in Genbank® accession number AJ010302 or L19596. The sequence of ccoN can be as set forth in Genbank® accession number U58092. Knockout technology can be used to make cells containing non-functional crtE, ppsR, and/or ccoN nucleic acid sequences.

*4. Producing isoprenoid compounds*

The cells described herein can be used to produce isoprenoid compounds. For example, a microorganism having endogenous DDS activity can be transformed with nucleic acid that encodes a polypeptide having DDS activity such that the microorganism produces more CoQ(10) than had the microorganism not been given that nucleic acid. Once transformed, the microorganism can be used cultured under conditions optimal for CoQ(10) production.

In addition, substantially pure polypeptides having enzymatic activity can be used alone or in combination with cells to produce isoprenoid compounds. For example, a preparation containing a substantially pure polypeptide having DDS activity can be used to catalyze the formation of CoQ(10). Further, cell-free extracts containing a polypeptide having enzymatic activity can be used alone or in combination with substantially pure polypeptides and/or cells to produce isoprenoid compounds. For example, a cell-free extract containing a polypeptide having DXS activity can be used to form 1-deoxyxylulose-5-phosphate, while a microorganism containing polypeptides have the enzymatic activities necessary to catalyze the reactions needed to form CoQ(10) from 1-deoxyxylulose-5-phosphate can be used to produce CoQ(10). Any method can be used to produce a cell-free extract. For example, osmotic shock, sonication, and/or a repeated freeze-thaw cycle followed by filtration and/or centrifugation can be used to produce a cell-free extract from intact cells.

It is noted that a cell, substantially pure polypeptide, and/or cell-free extract can be used to produce a particular isoprenoid compound that is, in turn, treated chemically to produce another compound. For example, a microorganism can be used to produce CoQ(10), while a chemical process is used to modify CoQ(10) into a CoQ(10) derivative such as CoQ10 containing a polar group. Likewise, a chemical process can be used to produce a particular compound that is, in turn, converted into an isoprenoid compound using a cell, substantially pure polypeptide, and/or cell-free extract described herein. For example, a chemical process can be used to produce deoxyxylose-5-phosphate, while a microorganism can be used convert deoxyxylose-5-phosphate into CoQ(10).

Typically, a particular isoprenoid compound is produced by providing a microorganism and culturing the provided microorganism with culture medium such that that isoprenoid compound is produced. In general, the culture media and/or culture conditions can be such that the microorganisms grow to an adequate density and produce the desired compound efficiently. For large-scale production processes, the following methods can be used. First, a large tank (e.g., a 100 gallon, 200 gallon, 500 gallon, or more tank) containing appropriate culture medium with, for example, a glucose carbon source is inoculated with a particular microorganism. After inoculation, the microorganisms are incubated to allow biomass to be produced. Once a desired biomass is reached, the broth containing the microorganisms can be transferred to a second tank. This second tank can be any size. For example, the second tank can be larger, smaller, or the same size as the first tank. Typically, the second tank is larger than the first such that additional culture medium can be added to the broth from the first tank. In addition, the culture medium within this second tank can be the same as, or different from, that used in the first tank. For example, the first tank can contain medium with xylose, while the second tank contains medium with glucose.

Once transferred, the microorganisms can be incubated to allow for the production of the desired isopreniod compound. Once produced, any method can be used to isolate the desired compound. For example, if the microorganism releases the desired isoprenoid compound into the broth, then common separation techniques can be used to remove the biomass from the broth, and common isolation procedures (e.g., extraction, distillation, and ion-exchange procedures) can be used to obtain the isoprenoid compound from the microorganism-free broth. In addition, the desired isoprenoid compound can be isolated while it is being produced, or it can be isolated from the broth after the product production phase has been terminated. If the microorganism retains the desired isoprenoid compound, then the biomass can be collected and treated to release the isoprenoid compound, and the released isoprenoid compound can be isolated.

The invention will be further described in the following examples, which do not limit the scope of the invention described in the claims.

**EXAMPLES**

Example 1- Cloning nucleic acid that encodes a *Sphingomonas trueperi* polypeptide having DXS activity

*S. trueperi* cells were obtained from the American Type Culture Collection (ATCC Cat. No. 12417). To isolate bacterial genomic DNA, cells were grown in 100-200 mL cultures for 2-3 days at 30°C on a shaker rotating at 250 rpm. Cultured cells were centrifuged to form a cell pellet, washed by resuspending the pellet in a solution of 10 mM Tris/1 mM EDTA, and centrifuged again as before. The cell pellets were resuspended in 5 mL of GTE buffer per 100 mL of original culture. GTE buffer is 50 mM glucose/25 mM Tris-HCl (pH 8.0)/10 mM EDTA (pH 8.0). The bacterial cell walls were lysed by adding lysozyme (final concentration of 1 mg/mL), Proteinase K (final concentration of 1 mg/mL), and mutanolysin (final concentration of 5.5 μg/mL) to the resuspended cell solution to form a lysing mixture that was incubated for 90 minutes at 37°C. After this incubation, sodium dodecyl sulfate was added to the mixture to a final concentration of 1 percent, and additional Proteinase K was added until the concentration in the solution was 2 mg/mL. After a 1 hour incubation at 50°C, the solution containing the lysed cells was diluted 1:1 with fresh GTE buffer. Once diluted, sodium chloride was added to the solution to a final concentration of 0.15 M. Polypeptides and molecules other than nucleic acids were removed from the lysed bacterial cell solution by adding an equal volume of an organic mixture made up of phenol, chloroform, and isoamyl alcohol at a ratio of 25:24:1 (hereinafter referred to as PCIA). After adding PCIA, the solution was mixed. To separate the organic phase from the DNA-containing aqueous phase, the mixture was centrifuged at 12,000 x g for 10 minutes. The aqueous phase was transferred to a clean tube and re-extracted with an equal volume of chloroform alone. The aqueous and organic phases were separated by centrifugation at 3,000 x g for 10 minutes. The aqueous phase was again removed to a new tube and treated with 2.5 mg of RNase to degrade any bacterial RNA present. The purified DNA was recovered by adding 2.5 volumes of ethanol to the aqueous phase. After mixing the solution, the precipitated DNA was removed by spooling it on a glass rod. The spooled DNA was rinsed with 70 percent ethanol. Once rinsed, the ethanol was allowed to evaporate by leaving the DNA exposed to the air until dry. The dried DNA was resuspended in a solution of 10 mM Tris (pH 8.5). The resuspended DNA was re-extracted with PCIA followed by chloroform alone as before. The DNA was re-precipitated by adding one-tenth volume of 7.5 M ammonium acetate and 2.5 volumes ethanol, followed by spooling, rinsing, and air drying. The purified DNA was resuspended in 10 mM Tris (pH 8.5).

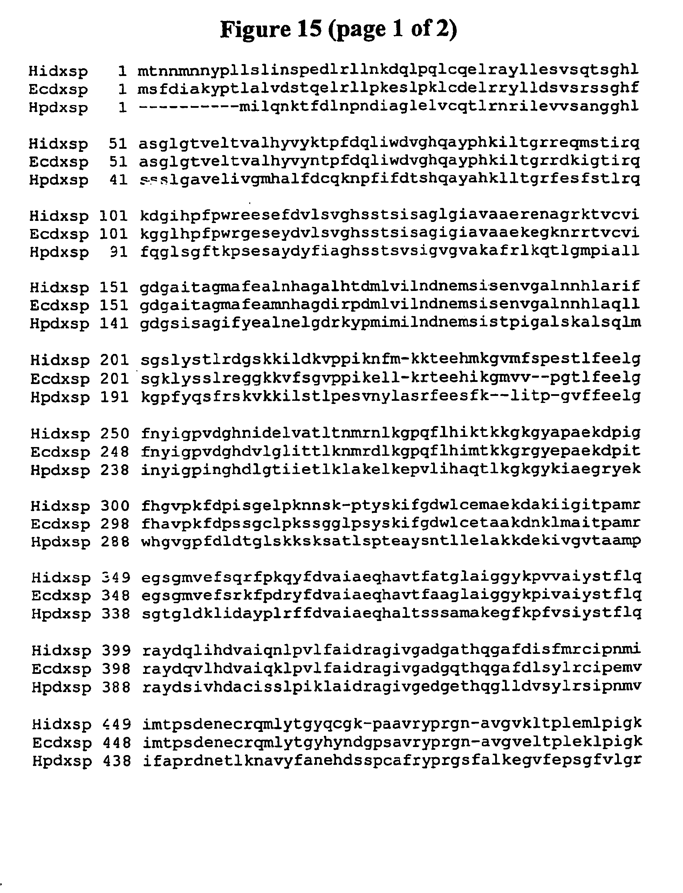

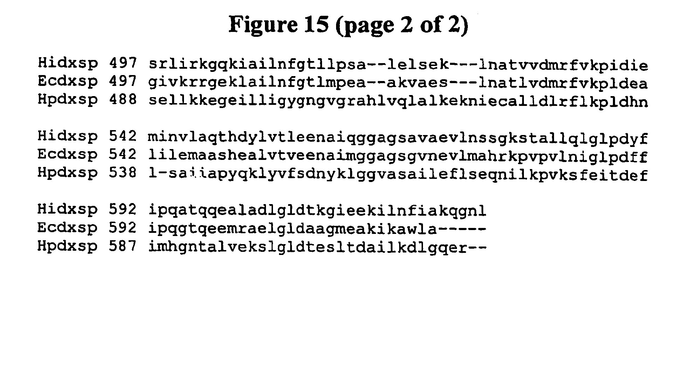

The following polymerase chain reaction (PCR) procedure was used to isolate nucleic acid that encodes a *S. trueperi* polypeptide having DXS activity. Three degenerate forward PCR primers (F1, F2, and F3) and three degenerate reverse PCR primers (R1, R2, and R3) were designed by comparing sequences of several clones that encode polypeptides have DXS activity (Figure 15). The sequence of each degenerate primer was as follows:

F1: 5'-RTKATTYTMAAYGAYAAYGAAATG-3' (SEQ ID NO:53)
F2: 5'-TTTGAAGARYTVGGYWTTAACTA-3' (SEQ ID NO:54)
F3: 5'-RCAYCARGCTTAYSCVCAYAA-3' (SEQ ID NO:55)
R1: 5'-CGTGYTGYTCDGCRATHGCBAC-3' (SEQ ID NO:56)
R2: 5'-TGYTCDGCRATHGCBACRTCRAA-3' (SEQ ID NO:57)
R3: 5'-GGSCCDATRTAGTTAAWRCC-3' (SEQ ID NO:58)

The primers were used in all logical combinations in PCR using Taq polymerase (Roche Molecular Biochemicals, Indianapolis, IN) and 1 ng of purified genomic DNA per microliter of reaction mix. Each PCR reaction was conducted using a touchdown PCR program with four cycles at each of the following annealing temperatures: 60°C, 58°C, 56°C, and 54°C, followed by 25 cycles at 52°C. Each cycle had an initial 30 second denaturing step at 94°C and a 90 second extension step at 72°C. The program had an initial denaturing step of 2 minutes at 94°C and final extension step of 5 minutes at 72°C.

Between about 2 μM and 12 μM of each PCR primer was used in each reaction, depending on the degree of degeneracy. After each PCR reaction was complete, a portion of each reaction was separated by gel electrophoresis using a 1.5 percent TAE (Tris-acetate-EDTA) agarose gel. The results from the gel electrophoresis indicated that the combination of degenerate primer F3 with degenerate primer R2 produced a nucleic acid molecule of 882 bp (referred to as the F3R2 fragment). The F3R2 fragment was purified away from the agarose gel matrix using the Qiagen Gel Extraction procedure according to the manufacturer's instructions (Qiagen Inc., Valencia, CA). A portion of the purified fragment was ligated into the pCRII-TOPO vector. The vector containing the F3R2 fragment was inserted into *E. coli* TOP10 cells using the TOPO cloning procedure (Invitrogen, Carlsbad, CA). The transformed TOP10 cells were plated onto LB agar plates containing 100 μg/mL of ampicillin (Amp) and 50 μg/mL of 5-Bromo-4-Chloro-3-Indolyl-β-D-Galactopyranoside (Xgal). Single white colonies were re-plated onto fresh LB-Amp-Xgal plates and screened by PCR with the F3 and R2 primers to confirm the presence of plasmids with the desired insert. Plasmid DNAs were obtained from bacterial colonies using the QiaPrep Spin Miniprep Kit (Qiagen, Inc). The plasmid DNAs were then quantified and sequenced with the M13 forward and reverse primers. Sequence analysis indicated that the sequence of the F3R2 fragment aligned with sequences from other nucleic acid molecules that encode polypeptides having DXS activity.

To obtain the complete coding sequence for the *S. trueperi* polypeptide having DXS activity, genome walking was performed as follows. Primers were designed based upon the sequence of the 882 bp F3R2 fragment for walking in both the upstream and downstream directions. These walking primers had the following sequences:

GSP1F: 5'-TCGTGACCAAGAAGGGCAAGGGCTATG-3'(SEQ ID NO:59)
GSP2F: 5'-GACAAGTATCACGGCGTCCAGAAGTTC-3' (SEQ ID NO:60)
GSP1R: 5'-ATAGCCCTTGCCCTTCTTGGTCACGAC-3' (SEQ ID NO:61)
GSP2R: 5'-CGAACGGATCATACTCGCTCTCGCTG-3' (SEQ ID NO:62)

The GSP1F and GSP2F primers are primers that face downstream of the DXS polypeptide start codon, while the GSP1R and GSP2R primers are primers that face in the opposite direction. In addition, GSP2F and GSP2R are nested inside of the GSP1F and GSP1R primers. Genome walking was conducted according to the manual of CLONTECH's Universal Genome Walking kit (CLONTECH Laboratories, Inc., Palo Alto, CA) with the exception that *Fsp* I and *Sma* I were used instead of *Dra* I and *EcoR* V. The genomic DNA used was from *S. trueperi.* DMSO was added to the PCR mixture until a final concentration of 5 percent was reached. The PCR reactions were performed using a Perkin Elmer 9700 Thermocycler. The first round of PCR consisted of 7 cycles of 2 seconds at 94°C and 3 minutes at 72°C, followed by 36 cycles of 2 seconds at 94°C and 3 minutes at 67°C, with a final extension at 67°C for 4 minutes. The second round of PCR consisted of 5 cycles of 2 seconds at 94°C and 3 minutes at 72°C, followed by 24 cycles of 2 seconds at 94°C and 3 minutes at 67°C, with a final extension at 67°C for 4 minutes. After the PCR was complete, a portion of the reaction mix from each round was separated by gel electrophoresis using a 1.5 percent TAE agarose gel. Good amplification products were obtained with the *Pvu* II and *Stu* I libraries using the GSP1F and GSP2F primers and with the *Fsp* I and *Pvu* II libraries using the GSP1R and GSP2R primers. The second round products from each of these libraries were gel purified, cloned using the TOPO cloning procedure (Invitrogen, Carlsbad, CA), and sequenced. A 1.7 kilobase (kb) fragment was subcloned from the *Pvu* IIF library, a 2.8 kb fragment was subcloned from the *Stu* IF library, a 400 bp fragment was subcloned from the *Fsp* IR library, and a 330 bp fragment was subcloned from the *Pvu* IIR library. Each of these subcloned fragments was sequenced. Sequence analysis indicated that each subcloned fragment contained a sequence that overlapped with that of the F3R2 fragment and was similar to other nucleic acid sequences that encode polypeptides having DXS activity.

Because the sequence information obtained by genome walking extended 13 bp upstream of the translational start codon, a second genome walk was conducted to gain additional sequence information. This second walk used GSPB2R, 5'-TGAGGATCTTGTGCGGATAGC-ATTGGTG-3' (SEQ ID NO:63) as the first round primer and GSPB3R, 5'-AGCGGCGTCTTG-GGTAGGTCAGCCAT-3' (SEQ ID NO:64) as the second round primer. The second walk was conducted using only the *Sma* I and *Stu* I libraries. CLONTECH's Advantage-GC Genomic Polymerase was used for PCR with a 1.0 mM GC Melt concentration according to the manufacturer's specifications. The first round of PCR was conducted using a Perkin Elmer 9700 Thermocycler with an initial denaturing step at 96°C for 5 seconds followed by 7 cycles consisting of 2 seconds at 94°C and 3 minutes at 72°C, followed by 36 cycles consisting of 2 seconds at 94°C and 3 minutes at 66°C, with a final extension at 66°C for 4 minutes. The second round of PCR had 5 cycles consisting of 2 seconds at 94°C and 3 minutes at 72°C, followed by 26 cycles consisting of 2 seconds at 94°C and 3 minutes at 66°C, with a final extension at 66°C for 4 minutes. Portions of the PCR products from each round were separated by gel electrophoresis using a 1.5 percent TAE agarose gel. The gel electrophoresis revealed the presence of a 250 bp amplification product obtained from the second round of PCR using the *Stu* I library. This fragment was gel purified, cloned using the TOPO cloning procedure (Invitrogen, Carlsbad, CA), and sequenced. An overlap with the previously obtained sequence was found, extending the length of the clone to 181 bp before the start codon. The full-length clone containing coding and non-coding sequence was 3626 bp in length (Figure 2). The open reading frame was 1926 bp in length (Figure 3), which encoded a polypeptide with 641 amino acid residues (Figure 4).

The coding sequence of the DXS polypeptide was amplified by PCR using *S. trueperi* genomic DNA as template. Primers were designed based on the sequence obtained above. The sequences of the primers were as follows:

SHDXF1: 5'-ATATGGTACCGTGTGACTGACCTGTCCAAC-3' (SEQ ID NO:65)
SHDXR1: 5'-AGTCTCTAGAATGTTGGAGATTCAAGGTGG-3' (SEQ ID NO:66)

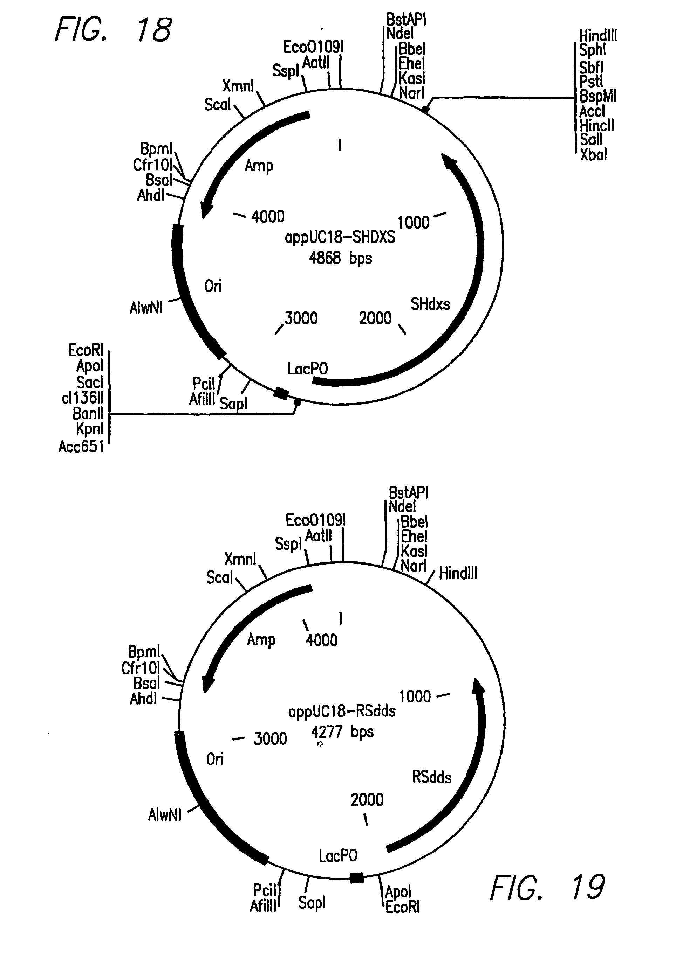

These primers were designed to introduce a *Kpn* I restriction site at the beginning of the amplified fragment and an *Xba* I restriction site at the end of the amplified fragment. The sequence of each restriction site is underlined. The PCR reaction mix contained the following: 100 ng genomic DNA, 2 μL of each primer (SHDXF1 and SHDXR1, each at 50 μM), 10 μL 10X *Pfu* Plus buffer, 5 μL DMSO, 8 μL dNTPs (10 μM each) and 5 units *Pfu* polymerase in a final volume of 100 μL. Each PCR reaction was performed in a Perkin Elmer Geneamp PCR system 2400 under the following conditions: an initial denaturation at 94°C for 5 minutes; 8 cycles of (1) 94°C for 45 seconds, (2) 55°C for 45 seconds, and (3) 72°C for 3 minutes; 21 cycles of (1) 94°C for 45 seconds, (2) 61°C for 45 seconds and (3) 72°C for 3 minutes; and a final extension of 72°C for 10 minutes. A portion of the PCR reaction was separated by gel electrophoresis using a 0.8 percent TAE gel. The gel electrophoresis revealed a 1.6 kb fragment. This fragment was (1) purified using a Qiagen Gel Extraction kit (Qiagen Inc., Valencia, CA), (2) treated with *Kpn* I and *Xba* I (New England BioLabs, Inc., Beverly, MA), and (3) subcloned into pUC18 that had also been treated with *Kpn* I and *Xba* I and gel purified. The resulting construct designated appUC18-SHDXS is depicted in Figure 18. The ligation was carried out with T4 DNA ligase at 16°C for 16 hours. Once ligated, 1 μL was used to electroporate *E. coli* ElectroMAX™ DH10B™ cells (Life Technologies, Inc., Rockville, MD). The electroporated cells were plated on LB-Amp plates (Amp concentration = 100 μg/mL). From these plates, eight individual colonies were chosen at random. The plasmid was isolated from each colony using a QiaPrep Spin Miniprep kit (Qiagen Inc., Valencia, CA). The extracted plasmid DNA was examined for the presence of the 1.6 kb fragment by digesting individual aliquots with one of three different restriction enzymes: *EcoR* I, *BamH* I, and *Nar* I. If the plasmids contained the correct 1.6 kb fragment, the *EcoR* I digest reaction would result in two fragments (0.77 and 4.13 kb), the *BamH* I digest reaction would result in one fragment (4.8 kb), and the *Nar* I digest reaction would result in two fragments (1.9 and 2.9 kb). After treating with the restriction enzymes, the digest reactions were separated by gel electrophoresis using a 0.8 percent TAE agarose gel. All 8 clones yielded digestion fragments consistent with a clone of 1.6 kb.

Example 2 - Introducing nucleic acid that encodes a polypeptide having DXS activity into cells

The nucleic acid molecule that encodes a polypeptide having DXS activity and was obtained as described in Example 1 is introduced into cells as follows. First, a construct is made to contain the nucleic acid molecule such that the encoded polypeptide having DXS activity is expressed in a desired host cell. When using prokaryotic cells, a construct functional in prokaryotic cells is used. When using eukaryotic cells, a construct functional in eukaryotic cells is used. Second, the construct is introduced into the desired host cell using appropriate methods. Once introduced, stable transformants are selected.

Example 3 - Cloning nucleic acid that encodes *a Rhodobacter sphaeroides* polypeptide having DDS activity

*R. sphaeroides* ATCC strain 17023 cells were grown in 550 R 8 A H media at 30°C and 100 rpm. The recipe for 550 R 8 A H media was provided by ATCC. Genomic DNA was isolated from *R. sphaeroides* cells as described in Example 1.

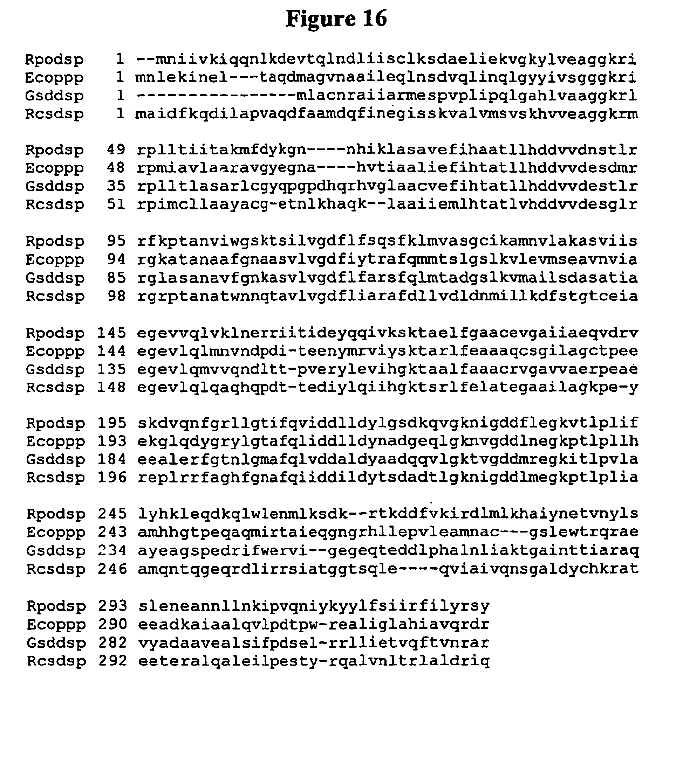

To isolate nucleic acid encoding an *R. sphaeroides* polypeptide having DDS activity, degenerate primers were designed and used as described in Example 1. Briefly, three degenerate forward primers (F4, F5, and F6) and four degenerate reverse primers (R4, R5, R6, and R7) were designed by comparing sequences of several clones that encode polypeptides have DDS, SDS, or ODS activity (Figure 16). The sequence of each degenerate primer was as follows:

F4: 5'-GGWGGHAARMGMMTKCGYCC-3' (SEQ ID NO:67)
F5: 5'-ACWYTGSTDCATGATGATGT-3' (SEQ ID NO:68)
F6: 5'-ACNYTNBTNCAYGAYGAYGT-3' (SEQ ID NO:69)
R4: 5'-TYRTCYACSACATCATCATG-3' (SEQ ID NO:70)
R5: 5'-TGHAVKACYTCACCYTCRGMAAT-3' (SEQ ID NO:71)
R6: 5'-TARTCNARDATRTCRTCDAT-3' (SEQ ID NO:72)
R7: 5'-TCRTCNCCNAYNKTYTINCC-3' (SEQ ID NO:73)

These primers were used in all logical combinations in PCR using Taq polymerase (Roche Molecular Biochemicals, Indianapolis, IN) and 1 ng of genomic DNA per microliter of reaction mix. PCR was conducted using the touchdown PCR program as described in Example 1. Between about 4 μM and 8 μM of each PCR primer was used in each reaction, depending on the degree of degeneracy. After each PCR reaction was complete, a portion of each reaction was separated by gel electrophoresis using a 1.5 percent TAE agarose gel. The results from the gel electrophoresis yielded no fragments of the expected size. A second amplification reaction was then performed using each sample from the first round of PCR. Briefly, one μL of reaction mixture from each first round of PCR was used in a 50 μL amplification reaction using the same primer pairs and thermocycling parameters used in the first round of PCR. A portion of each of the second round PCR reactions was separated by gel electrophoresis using a 1.5 percent TAE agarose gel. The combination of degenerate primers F6 and R5 produced a fragment of 209 bp (referred to as the F6R5 fragment). The F6R5 fragment was isolated from an agarose gel and purified using the Qiagen Gel Extraction procedure (Qiagen Inc., Valencia, CA). An aliquot of the purified fragment was ligated to pCRII-TOPO, and the product of the ligation reaction was inserted into TOP10 *E. coli* cells using a TOPO cloning procedure (Invitrogen, Carlsbad, CA). The products of the individual insertion reactions were plated onto LB media containing 100 μg/mL Amp and 50 μg/mL Xgal. Single white colonies that grew on the LB-Amp-Xgal plates were re-plated onto fresh LB-Amp plates and screened in a PCR reaction using the F6 and R5 primers to confirm the presence of the desired insert. Plasmid DNAs were obtained from several colonies using a QiaPrep Spin Miniprep kit (Qiagen, Inc). The obtained plasmid DNAs were quantified and sequenced with the M13 forward and reverse primers. Sequence analysis revealed that the F6R5 fragment contained sequences that aligned with sequences from other nucleic acid molecules that encode polypeptides having polyprenyl diphosphate synthase activity.

Genome walking was performed to obtain a complete coding sequence for the *R. sphaeroides* DDS polypeptide using procedures similar to those described in Example 1. Briefly, primers were designed based on the sequence of the F6R5 fragment for walking in both the upstream and downstream directions. These primers had the following sequences:

GSP3F: 5'-TGGAAGCTGCGGGCGAAGAGATAGTC-3' (SEQ ID NO:74)

GSP4F: 5'-CCCACCAGCACCGAGGATTTGTTGTC-3' (SEQ ID NO:75)
GSP3R: 5'-GAACCTGCTGTGGGACAACAAATCCTC-3' (SEQ ID NO:76)
GSP4R: 5'-TCGGTGCTGGTGGGCGACTATCTCTTC-3' (SEQ ID NO:77)

The GSP3F and GSP4F primers are primers that face downstream of the DDS polypeptide start codon, while the GSP3R and GSP4R primers are primers that face in the opposite direction. In addition, the GSP4F and GSP4R primers are nested inside the GSP3F and GSP3R primers.

The *Pvu* II, *Fsp* I, and *Stu* I libraries with the GSP3F and GSP4F primers and all four libraries with the GSP3R and GSP4R primers resulted in the production of amplified fragments. A 750 bp fragment from the *Pvu* I library, a 500 bp fragment from the *Fsp* I library, a 1.4 kb fragment from the *Stu* I library, and a 0.9 kb fragment from the *Sma* I library were all subcloned and sequenced. Sequence analysis indicated that each subcloned fragment contained a sequence that overlapped with the sequence of the F6R5 fragment and was similar to other nucleic acid sequences that encode polypeptides having polyprenyl diphosphate synthase activity. The full-length clone containing coding and non-coding sequence was 1990 bp in length (Figure 7). The open reading frame was 1002 bp in length (Figure 8), which encoded a polypeptide with 333 amino acid residues (Figure 9).

The coding sequence of the DDS polypeptide from *R. sphaeroides* was amplified by PCR using *R. sphaeroides* genomic DNA as template. PCR primers were designed based on the sequences obtained as described above. The sequences of the primers were as follows.

RDS18F: 5'-ACTAGAATTCCGCAACAGTTCCTTCATGTC-3' (SEQ ID NO:78)
RDS18R: 5'-ATAGAAGCTTACTTGCGGTCGGACTGATAG-3' (SEQ ID NO:79)

These primers were designed to introduce an *EcoR* I restriction site at the beginning of the amplified fragment and a *Hind* III restriction site at the end of the amplified fragment. The sequence of each restriction site is underlined. The PCR reaction mix contained the following: 100 ng genomic DNA, 2 μL of each primer (RDS18F and RDS18R, each at 50 μM), 10 μL 10X *Pfu* Plus buffer, 5 μL DMSO, 8 μL dNTPs (10 mM each) and 5 units *Pfu* polymerase in a final volume of 100 μL. Each PCR reaction was performed in a Perkin Elmer Geneamp PCR system 2400 under the following conditions: an initial denaturation at 94°C for 5 minutes; 8 cycles of (1) 94°C for 45 seconds, (2) 55°C for 45 seconds, and (3) 72°C for 3 minutes; 21 Cycles of (1) 94°C for 45 seconds, (2) 61°C for 45 seconds, and (3) 72°C for 3 minutes; and a final extension of 72°C for 10 minutes. After completing the PCR reactions, each PCR reaction was separated by gel electrophoresis using a 0.8 percent TAE agarose gel. The gel electrophoresis revealed a 1.6 kb fragment. This fragment was (1) purified from the agarose gel using a Qiagen Gel Extraction kit, (2) digested with *EcoR* I and *Hind* III (New England BioLabs, Beverly, MA), and (3) ligated to pUC18 that had also been digested with *EcoR* I and *Hind* III and gel purified. The resulting construct designated appUC18-RSdds is depicted in Figure 19. The ligation was carried out with T4 DNA ligase at 16°C for 16 hours. Once ligated, one μL of the ligation reaction was used to electroporate *E. coli* ElectroMAX™ DH1 OB™ cells (Life Technologies, Inc., Rockville, MD). The electroporated cells were plated onto LB-Amp plates (Amp concentration was 100 μg/mL). From these LB-Amp plates, eight individual colonies were selected at random, and the plasmids within these colonies were purified using a Qiaprep Spin Miniprep kit. These purified plasmids were evaluated for the presence of inserts by restriction enzyme analysis. If the plasmids contained the correct 1.6 kb fragment, then an *EcoR* I and *Hind* III digest reaction would result in two fragments (2.6 and 1.6 kb), and a *BamH*I digest reaction would result in one fragment (4.2 kb). After treating with the restriction enzymes, the digest reactions were separated by gel electrophoresis using a 0.8 percent TAE agarose gel. Of the eight clones tested, four contained the desired 1.6 kb fragment.

Example 4 - Cloning nucleic acid that encodes *a Sphingomonas trueperi* polypeptide having DDS activity

*S. trueperi* cells were grown as described in Example 1. In addition, genomic DNA was isolated from *S. trueperi* cells as described in Example 1.

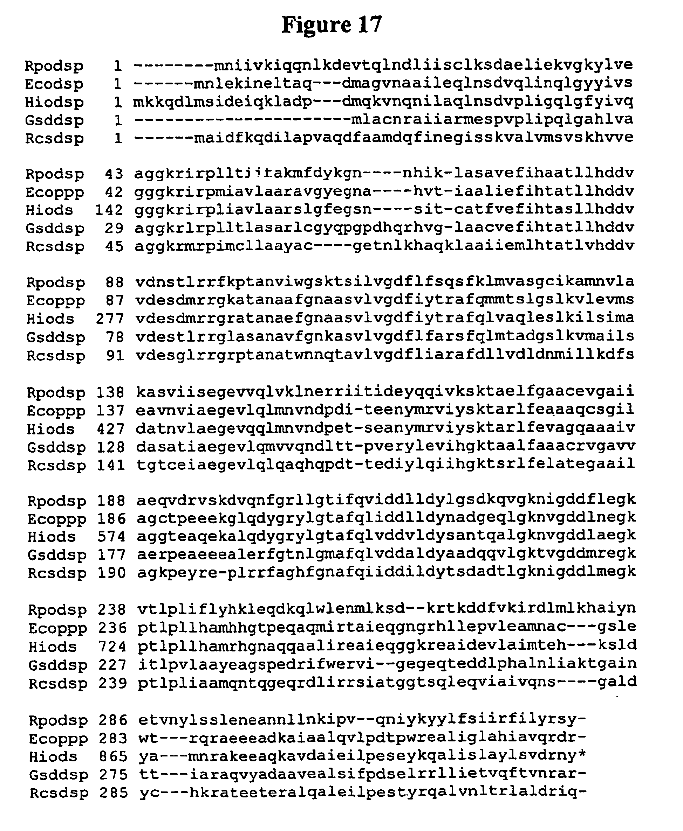

To isolate nucleic acid encoding a polypeptide having DDS activity from *S. trueperi,* a strategy similar to that described in Example 3 was employed. In this case, four degenerate forward primers (SF1, SF2, SF3, and SF4) and four degenerate reverse primers (SR1, SR2, SR3, and SR4) were designed comparing sequences of several clones that encode polypeptides having polyprenyl diphosphate synthase activity (Figure 17). Codon usage tables from twelve *Sphingomonas* species were used to develop an average preferred codon table that was used in primer design. The sequence of each degenerate primer was as follows:

SF1: 5'-CTSSTSCAYGAYGAYGTSGTSGA-3' (SEQ ID NO:80)
SF2: 5'-GTSGMVGSSGGSGGSAARC-3' (SEQ ID NO:81)
SF3: 5'-CTSMTSCAYGAYGAYGTS-3' (SEQ ID NO:82)

SF4: 5'-DSSRTBCTSGTSGGSGAYTT-3' (SEQ ID NO:83)
SR1: 5'-VAKRAARTCSCCSACSAGSAC-3' (SEQ ID NO:84)
SR2: 5'-SACYTCSCCYTCSGCRAT-3' (SEQ ID NO:85)
SR3: 5'-RTCRTCSCCVAYVKTYTTSCC-3' (SEQ ID NO:86)
SR4: 5'-SGGSAGSGTVRBYTTSCCYTC-3' (SEQ ID NO:87)

The primers were used in all logical combinations in PCR using Taq polymerase (Roche Molecular Biochemicals, Indianapolis, IN) and 1 ng of genomic DNA per microliter of reaction mix. PCR was conducted using the touchdown PCR program as described in Example 1. Between about 4μM and 20 μM of each PCR primer was used in each reaction depending on the degree of degeneracy. After each PCR reaction was complete, a portion of each reaction was separated by gel electrophoresis using a 1.5 percent TAE agarose gel. Each PCR reaction produced several amplified fragments of the expected sizes based on the coding sequences of other polyprenyl diphosphate synthase polypeptides. These fragments were isolated from TAE agarose gels and purified using the Qiagen Gel Extraction procedure (Qiagen Inc., Valencia, CA). An aliquot ot each purified fragment was ligated into pCRII-TOPO. The ligated plasmids were then inserted into TOP10 *E. coli* cells using a TOPO cloning procedure (Invitrogen, Carlsbad, CA). The products of each of the individual insertion reactions were plated on LB-Amp-Xgal plates as described in Examples 1 and 3. Single white colonies that grew on the LB-Amp-Xgal plates were re-plated onto fresh LB-Amp-Xgal plates and screened in a PCR reaction using the initial degenerate primers to confirm the presence of the desired insert. Plasmid DNAs having the desired insert were obtained from multiple colonies using a QiaPrep Spin Miniprep Kit (Qiagen, Inc). The obtained plasmid DNAs were then quantified and sequenced using the M13 forward and reverse primers. Sequence analysis revealed that a 201 bp fragment produced using the SF1 and SR2 degenerate primers, a 476 bp fragment produced using the SF1 and SR4 primers, and a 206 bp fragment produced using the SF3 and SR2 primers contained sequences similar to the coding sequences of other polyprenyl diphosphate synthases.

Genome walking was performed to obtain a complete coding sequence for the *S.* trueperi DDS polypeptide using procedures similar to those described in Example 1. Briefly, primers were designed based on the sequences of the obtained fragments. These primers had the following sequences:

GSP5F: 5'-GTGCTGGTCGGCGACTTCCTGTTCAG-3' (SEQ ID NO:88)
GSP6F: 5'-ATCGACCTGTCCGAGGATCGCTATCTC-3' (SEQ ID NO:89)
GSP5R: 5'-TCGAACGAGCGGCTGAACAGGAAGTC-3' (SEQ ID NO:90)
GSP6R: 5'-TGGCGGGATTGCCCCAGATGATGTTG-3' (SEQ ID NO:91)

The GSP5F and GSP6F primers are primers that face downstream of the DDS start codon, while the GSP5R and GSP6R primers are primers that face in the opposite direction. In addition, the GSP6F and GSP6R primers are nested inside the GSP5F and GSP5R primers.

Genome walking was conducted as described in Example 3 with the exception that the 36 cycles had 3 minute incubations at 66°C instead of 67°C and the final extension was performed at 66°C instead of 67°C for both the first and second rounds of PCR. Portions of the PCR reactions from each round were separated by gel electrophoresis using a 1.5 percent TAE agarose gel. PCR on the *Fsp* I and *Stu* I libraries with the forward primers and of all four libraries with the reverse primers resulted in the production of an amplified fragment. A 1.4 kb fragment from the *Fsp* I library, a 1.1 kb fragment from the *Stu* I library (forward primer), a 2.0 kb fragment from the *Pvu* II library (forward primer), and a 3.0 kb fragment from the *Stu* I library (reverse primer) were gel purified, cloned using the TOPO cloning procedure, and sequenced as described in Examples 1 and 3. The sequencing analysis revealed that these fragments contained sequences that overlapped with the sequence of the initially obtained fragments and were similar to the coding sequences of other polyprenyl diphosphate synthases. The full-length clone containing coding and non-coding sequence was 1833 bp in length (Figure 10). The open reading frame was 1014 bp in length (Figure 11), which encoded a polypeptide with 337 amino acid residues (Figure 12).

The coding sequence of the DDS polypeptide from *S. trueperi* was amplified by PCR using *S. trueperi* genomic DNA as template. PCR primers were designed based on the sequences obtained as described above. The sequences of the primers were as follows.

SHDDSF: 5'-ATTAGGTACCATCAGATAATCGTCGCTCAA-3' (SEQ ID NO:92)
SHDDSR: 5'-TATAGGATCCGACATGGACGAGGAAGACGC-3' (SEQ ID NO:93)

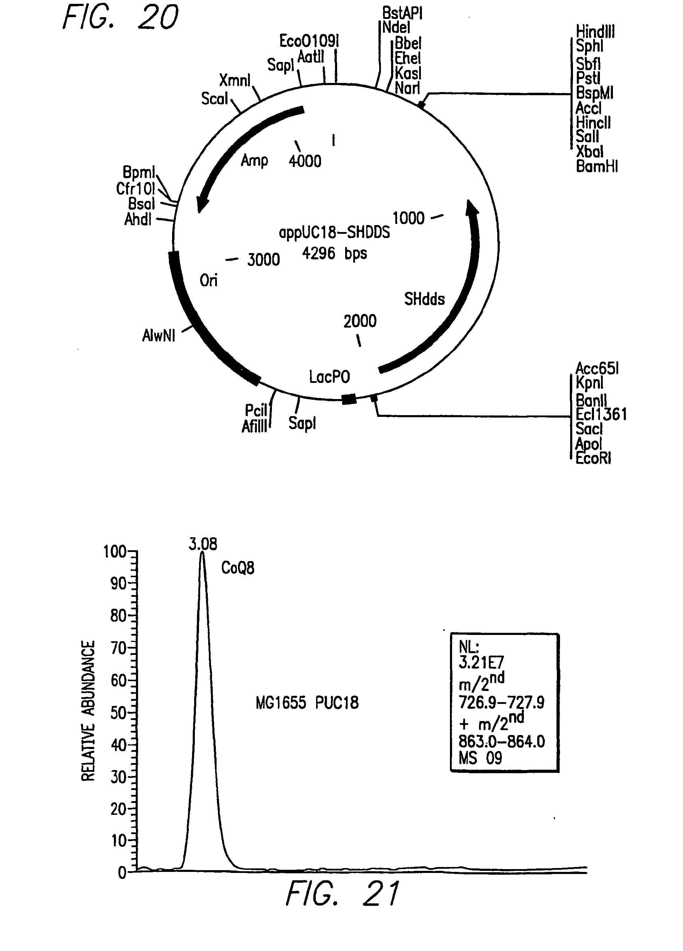

These primers were designed to introduce a *Kpn* I restriction site at the beginning of the amplified fragment and a *BamH*I restriction site at the end of amplified fragment. The sequence of each restriction site is underlined. The PCR reactions were performed as described in Example 3 with the exception that primers SHDDSF and SHDDSR were used instead of RDS 18F and RDS18R. Once the PCR was completed, the PCR reactions were separated by gel electrophoresis using a 0.8 percent TAE agarose gel. The gel electrophoresis revealed a 1.6 kb fragment. This 1.6 kb fragment was (1) purified using a Qiagen Gel Extraction kit, (2) digested with *Kpn* I and BamHI (New England BioLabs), and (3) ligated into pUC 18 that had also been digested with *Kpn* I and *BamH* I and gel purified using methods similar to those described in Example 3. The resulting construct designated appUC18-SHDDS is depicted in Figure 20. This construct was used to transform cells as described in Example 3. The transformed cells were plated onto LB-Amp plates, and eight individual colonies were selected at random. Plasmid DNA was isolated from each colony using a QiaPrep Spin Miniprep kit. The extracted plasmid DNA was tested for the presence of the 1.6 kb fragment using three different restriction digests. If the plasmids contained the 1.6 kb fragment, then a *BamH* I and *Kpn* I digest would yield two fragments (2.68 and 1.62 kb), an *EcoR* I digest would yield two fragments (1.45 and 2.85 kb), and a *Ban* II digest would yield two fragments (0.48 and 3.8 kb). All eight plasmids tested yielded digestion fragments consistent with a plasmid containing the desired 1.6 kb fragment.

Example 5 -Measuring CoQ(10)

Harvested cells were suspended in water to have about 0.1 gm dry weight per mL. The suspension was subjected to a French-press, and the resulting in suspension was frozen in 1 mL aliquots until used.

To measure CoQ(10) in a sample, two aliquots were repeatedly thawed and refrozen 4-5 times. Once transferred to a 50 mL centrifuge tube, 1 mL of 5% sodium dodecyl sulfate was added to the thawed material. The material was then flushed with nitrogen. After vortexing for one minute, six mL of ethanol was added to the material, and the resulting mixture was vortexed for one minute. Then, 15 mL of hexane was added to the mixture. After vortexing for five minutes, the mixture was centrifuged at 3000 rpm for ten minutes. Once centrifuged, the hexane layer was removed to a conical flask and flushed with nitrogen. This hexane extraction was repeated two times. The three extracts were pooled into a single tube that was evaporated on a vacuum evaporator until the residue was near dryness. The residue was dissolved in 2 mL of mobile phase by vortexing for 2-3 minutes. Once vortexed, the solution was transferred to a 5 mL volumetric flask. The tube that contained the residue was rinsed two additional times with 1 mL of mobile phase. Each time the rinse solution was transferred to the same 5 mL volumetric flask. After adjusting the total volume to 5 mL, the solution was mixed well and stored at -20°C until analyzed.

As a control, either water or a culture solution was spiked with standard CoQ(10), extracted as indicated above, and analyzed to determine the recovery of the spiked material. The CoQ(10) standard was a stock solution of CoQ(10), obtained from Sigma. The stock solution was made in HPLC grade ethanol at a concentration of 100 μg/mL, and then diluted to get CoQ(10) solutions ranging from 100 μg/mL to 1 μg/mL.

HPLC analysis was performed with the following parameters. The mobile phase was ethanol:methanol (7:3) or methanol:isopropylether (9:1). The flow rate was 0.75 mL/min. The column was Waters Nova-Pak C18 (3.9 x150 mm; 4Um). The detector was a PDA set from 200-300 nm with the resolution at 1.2 nm and the maximum absorbance at 275 nm. The run time was 15 minutes, and the injection volume was 50 μL. To calculate the amount of CoQ(10) present, 50 μL of each sample was injected, and the results compared to those obtained using the calibration curve. From these data points, the concentration per gm dry weight was calculated.

Example 6 - Introducing nucleic acid that encodes a Polypeptide having DDS activity into cells and measuring isoprenoid levels

The following procedures were followed individually for the *R. sphaeroides* and *S. trueperi* nucleic acid isolated as described in Examples 3 and 4, respectively.

Plasmid DNA encoding the polypeptide having DDS activity was electroporated into wild type *E. coli* strain MG1655. The electroporated cells were plated onto LB-Amp plates. A single individual bacterial colony was picked for each DDS coding sequence, and each colony was grown overnight in 2 mL of LB-Amp at 37°C with 200 rpm shaking. About 0.75 mL of these overnight cultures were used to inoculate flasks containing 75 mL LB-Amp medium (Amp concentration was 100 μg/mL). These second cultures were grown at 37°C at 200 rpm for 30 hours. Additional Amp (to a final concentration of 50μ g of fresh Amp per mL) was added to each flask after 12 hours of growth. After 30 hours, the bacteria were collected by centrifugation at 8,000 g for 10 minutes. The resulting bacterial cell pellets were washed by adding 20 mL of 10 mM Tris-HCL buffer (pH 8.0), resuspending the cells, and re-centrifuging as before. Each cell pellet was then resuspended in 10 mL of water. About 0.5 mL of each extract was used for dry mass analysis and the remaining cell suspensions (about 9.5 mL) were frozen at -20°C overnight.

The 9.5 mL cell suspensions were used as follows. First, the cells were thawed on ice and lysed by passing the cell suspensions through a French press three times (14,000 psi pressure). The resulting cell extracts were frozen at -20°C in 1 mL aliquots and maintained on ice prior to analysis.

High pressure liquid chromatography was performed using Waters' 2690 Alliance integrated system (Waters Corporation, Milford, Mass). Prior to analysis, all samples and standards were dissolved in HPLC-grade ethanol, loaded into the built-in auto-sampler, and kept at 5°-10°C in the dark. The separation was carried out using an isocratic elution program of 70:30 ethanol/methanol (v/v) at a flow rate of 1.0 mL/min. The column was a Waters Nova-Pak C18, 3.9-150 mm equipped with a guard column of the same stationary phase. The injection volume was typically 10-25 μL. Total run time was ten minutes.

Under these conditions, retention times were 3.1 and 4.9 minutes for CoQ(8) and CoQ(10), respectively. For quantification purposes, a four-point external calibration curve was calculated using freshly prepared CoQ(10) standards. Calibration levels were 1.0, 4.0, 10.0 and 100.0 μg/mL (ppm). Each standard was injected in triplicate, and the resulting calibration plot was linearly fitted with observed $r^2$'s of >0.999.

For UV and MS detection, a photodiode array (PDA, Model W6000LP, ThermoQuest Corp., San Jose, CA) and an ion trap mass analyzer (LCQ Classic, Finnigan/ThermoQuest Corp., San Jose, CA) were connected in series with the chromatograph and without splitting of the effluent. The PDA was operated in scanning mode from 220-300 nm. Effluent from the PDA was introduced into the mass analyzer via atmospheric-pressure chemical ionization (APCI) using the following parameters: capillary temperature, 150°C; capillary voltage, 3kV; vaporizer temperature, 400°C; sheath gas ($N_2$) flow, 80 arbitrary units; auxiliary gas ($N_2$) flow, 5 arbitrary units; and corona discharge needle, 5mA/6kV. Positive-ion detection was performed in full scan (250-1000 m/z), 2 mscans, 500 ms ion injection time.

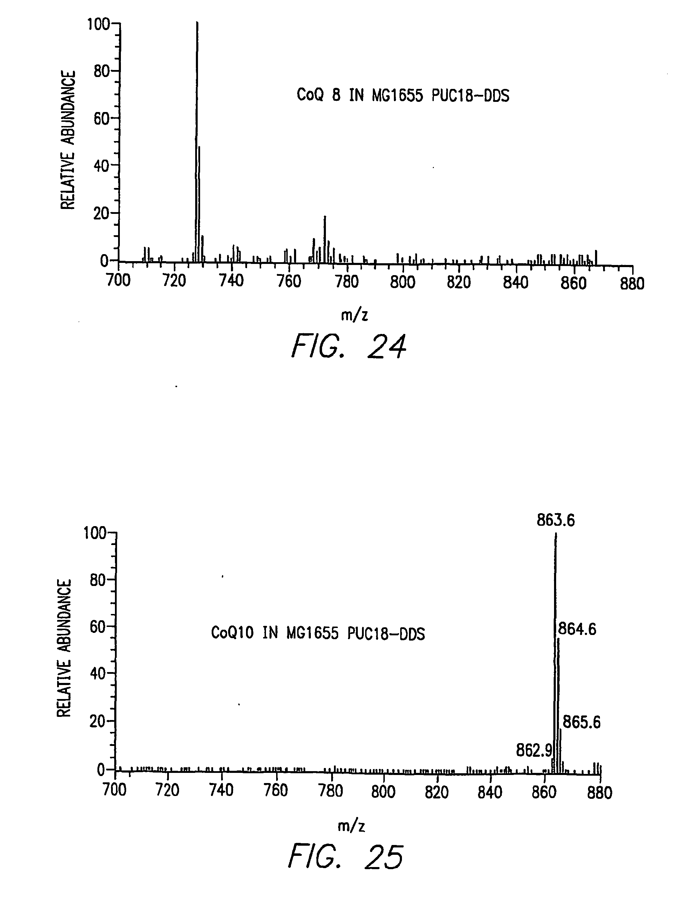

Under these conditions, CoQ(8) yielded a mass spectrum with a base peak at 727.5 m/z, corresponding to the protonated 'molecular ion' as well as several satellite ions from ethanol and/or methanol adducts (Figures 23 and 24). Similarly, CoQ(10) yielded a mass spectrum with a base peak at 863.6 m/z corresponding to its protonated 'molecular ion' (Figure 25). Several ethanol and/or methanol satellite adducts were observed as well. Both CoQ(8) and CoQ(10) yielded UV spectra with maxima at 274 nm.

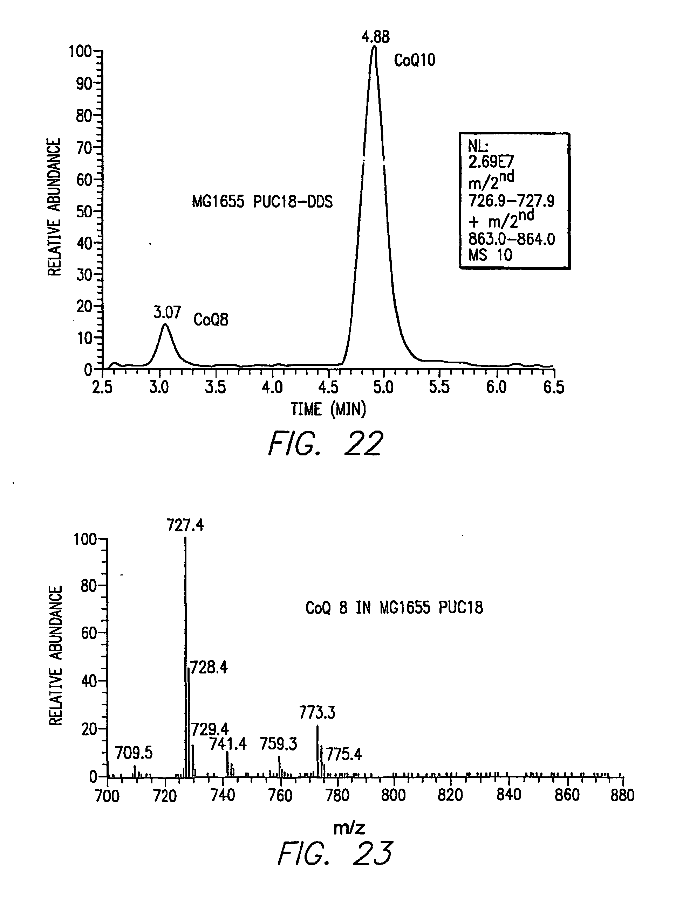

Two samples were analyzed: MG1655 PUC18 and MG1655 PUC18-DDS. MG1655 PUC18 is *E. coli* strain MG1655 transfected with the PUC18 vector only. MG1655 PUC18-DDS is *E. coli* strain MG1655 transfected with the PUC18 vector containing nucleic acid that encodes a *R. sphaeroides* polypeptide having DDS activity. The MG1655 PUC18 specimen contained only CoQ(8) (retention time 3.08 min, Figure 21) as confirmed by its mass spectrum (Figure 23), with a base peak at 727.4 m/z and a UV spectrum with a maximum at 274 nm. The MG1655 PUC18-DDS specimen, however, contained CoQ(8) and CoQ(10) (Figure 22), both of which were confirmed by matching mass spectra (Figures 24 and 25) and UV maxima.

Comparative Example 7 - Cloning nucleic acid that encodes a *Sphingomonas trueperi* polypeptide having DXR activity

*Sphingomonas trueperi* ATCC 12417 cultures (100-200 mL) were grown in nutrient broth at 30°C and 250 rpm for 2-3 days. The cells then were pelleted and washed with a 10 mM Tris:1.0 mM EDTA solution. The pellets were resuspended in 5 mL of GTE buffer (50 mM glucose, 25 mM Tris HCl (pH 8.0), 10 mM EDTA (pH 8.0)) per 100 mL of culture. Lysozyme and Proteinase K were added to a 1 mg/mL concentration and mutanolysin was added to 5.5 μg/mL. After a 1.5 hour incubation at 37°C, SDS was added to a final concentration of 1%, and the concentration of Proteinase K was brought to 2 mg/mL. After incubation at 50°C for one hour, an equal volume of GTE buffer was added, and NaCl was added to a 0.15 M concentration. The mixture was extracted with an equal volume ofphenol:chloroform:isoamyl alcohol (25:24:1) and centrifuged at 10,000 rpm for 10 minutes. The supernatant was removed to a clean tube, extracted with an equal volume of chloroform, and centrifuged at 5,000 rpm for 10 minutes. The supernatant was treated with RNAse and precipitated with 2.5 volumes of ethanol. The spooled DNA was washed with 70% ethanol, air dried, and resuspended in 10 mM Tris (pH 8.5). After resuspending, the resuspended DNA was further cleaned by re-extraction with phenol:chloroform:isoamyl alcohol and chloroform, and reprecipation with 1/10 volume 7.4 M $NH_4OAc$ and 2.5 volumes ethanol.

A conserved region of the 1-deoxy-D-xylulose 5-phosphate reductoisomerase (dxr) gene was cloned by PCR. Five degenerate forward and five degenerate reverse PCR primers were designed from conserved protein regions that were revealed by aligning known dxr genes (Figure 27). The degenerate sequences were designed from the conserved regions using the universal codon table. The primers were used in all logical combinations in PCR using Taq polymerase (Roche Molecular Biochemicals, Indianapolis, IN) and 1 ng of genomic DNA/μL reaction mix. PCR was conducted using a touchdown PCR program with 4 cycles at an annealing temperature of 59°C, 4 cycles at 57°C, 4 cycles at 55°C, and 24 cycles at 53°C. Each cycle used an initial 30 second denaturing step at 94°C and a 1.75 minute extension at 72°C, and the program had an initial denaturing step for 2 minutes at 94°C and final extension of 5 minutes at 72°C. The amounts of PCR primer used in the reaction were increased 3-12 fold above typical PCR amounts depending on the amount of degeneracy in the 3' end of the primer. In addition, separate PCR reactions containing each individual primer were made to identify PCR products resulting from single degenerate primers. Fifteen μL of each PCR product was separated on a 1.5% TAE (Tris-acetate-EDTA)-agarose gel. Degenerate primers F2 (5'-CCSGTSGAYWSSGARCAYAACGCS-3' (SEQ ID NO:132)) and R7 (5'-ATGATGAACAAGGGSCTSGAR-3' (SEQ ID NO:133)) produced a band of about 250 bp, which was the expected size based on dxr genes from other species. This band was not present in the individual F2 and R7 primer control reactions. Degenerate primers F3 (5'-CATCCVAACTGGWMVATGGG-3' (SEQ ID NO:134)) and R2 (5'-ATYGGYRWWCKCATATCMGG-3' (SEQ ID NO:135)) produced a band of about 200 bp, which also was the expected size. The F2-R7 and F3-R2 fragments were isolated and purified using a QIAquick Gel Extraction Kit (Qiagen Inc., Valencia, CA). Three μL of the purified band was ligated into pCR®II-TOPO vector, which was then transformed by a heat-shock method into TOP10 *E. coli* cells using a TOPO cloning procedure (Invitrogen, Carlsbad, CA). Transformations were plated on LB media containing 100 μg/mL of ampicillin and 50 μg/mL of 5-Bromo-4-Chloro-3-Indolyl-B-D-Galactopyranoside (X-gal). Individual, white colonies were resuspended in about 20 μL of 10 mM Tris and heated for 10 minutes at 95°C to break open the bacterial cells. To screen individual colonies, 2 μL of the heated cells was used in a 25 μL PCR reaction as described above using the appropriate degenerate primers. Plasmid DNA was obtained with a QIAprep Spin Miniprep Kit (Qiagen, Inc) from cultures of colonies having the desired insert and used for DNA sequencing with M13R and M13F primers. Sequence analysis revealed that the F2-R7 and F3-R2 fragments overlapped and were homologous to known dxr genes.

Genome walking was performed to obtain the complete coding sequence as follows. The overlapping of the F2-R7 and F3-R2 fragments resulted in a sequence 358. bp in length. The following four primers for conducting genome walking in both upstream and downstream directions were designed using the portion of this sequence that was internal to the degenerate primers:

GSP1F 5'-CGAATGGACGACGGATTGGCGATGGAC-3' (SEQ ID NO:136)
GSP2F 5'-TCAGTTCGAGCCCCTTGTTCATCATCGTC-3' (SEQ ID NO:137)
GSP1R 5'-CGAACTGATCGAAGCCTTCCACCTGTTC-3' (SEQ ID NO:138)
GSP2R 5'-GGTCCATCGCCAATCCGTCGTCCATTC-3' (SEQ ID NO:139)

The GSP1F and GSP2F primers faced upstream, the GSP1R and GSP2R primers faced downstream, and the GSP2F and GSP2R primers were nested inside the GSP1F and GSP1R primers. Genome walking was conducted according to the manual for CLONTECH's Universal Genome Walking Kit (CLONTECH Laboratories, Inc., Palo Alto, CA) with the exception that the enzymes FspI and SmaI were used in place of DraI and EcoRV. The DraI and EcoRV enzymes were replaced because they cut *S. trueperi* genomic DNA too infrequently to give fragment lengths amenable to PCR. The PCR mixture contained 5% DMSO. First round PCR was conducted in a Perkin Elmer 9700 Thermocycler with 7 cycles consisting of 2 seconds at 94°C and 3 minutes at 72°C, and 36 cycles consisting of 2 seconds at 94°C and 3 minutes at 66°C, with a final extension at 66°C for 4 minutes. Second round PCR used 5 cycles consisting of 2 seconds at 94°C and 3 minutes at 72°C, and 26 cycles consisting of 2 seconds at 94°C and 3 minutes at 66°C, with a final extension at 66°C for 4 minutes. Nine μL of the first round product and seven μL of the second round product were separated on a 1.5% TAE-agarose gel. A 1.3 Kb band was obtained from the second round product for the SmaI forward reaction, an 800 bp band for the StuI reverse reaction, and a 750 bp band for the PvuII reverse reaction. These fragments were gel purified, cloned, and sequenced. Internal primers were used to amplify and obtain additional sequence of the gene. Sequence analysis revealed that the sequence derived from genome walking overlapped with the original fragments and contained an entire coding sequence homologous to known dxr genes. The full-length clone containing coding and non-coding sequence was 2017 bp in length (Figure 28). The open reading frame starting with the first GTG site was 1161 bp in length (Figure 29), which encoded a polypeptide with 386 amino acid residues (Figure 30).

Example 8 - Making recombinant microorganisms

*Rhodobacter sphaeroides* (ATCC 35053) was routinely maintained on Luria Bretain (Miller) agar (Fisher scientific) plates. When needed, *R. sphaeroides* was cultured as follows. A 5 mL culture was grown in a 15 mL culture tube at 30°C in Innova 4230 Incubator, Shaker (New Brunswick Scientific, Edison, NJ) with a shaking speed of 250 rpm. Each 5 mL culture was started by inoculating liquid media (Sistrom media supplemented with 20% LB) with a single colony. The liquid media contained the following ingredients per liter: 2.72 g $KH_2PO_4$, 0.5 g $(NH_4)_2SO_4$, 0.5 g NaCl, 0.2 g EDTA disodium salt, 0.3 g $MgSO_4$. $7H_2O$, 0.033 g $CaCl_2$. $2H_2O$, 0.2 mg $FeSO_4 \cdot 7H_2O$, 0.02 mL $(NH_4)_6Mo_7O_{24}$. $4H_2O$ (1% solution), 1 mL Trace element solution, 0.2 mL Vitamin solution, 5 g Luria Bretain Broth Mix, and 8 mL Glucose (50%). The Trace element solution contained the following ingredients per liter: 1.765 g EDTA disodium salt, 10.95 g $ZnSO_4 \cdot 7H_2O$,5 g $FeSO_4 \cdot 7H_2O$, 1.54 g $MnSO_4 \cdot H_2O$, 0.392 g $CuSO_4 \cdot 5H_2O$, 0.284 g $Co(NO_3)_2 \cdot 6H_2O$, and 0.114 g $H_3BO_3$. The Vitamin solution contained the following ingredients per liter: 10 g Nicotinic acid, 5 g Thiamine HCl, and 0.01 g Biotin. The vitamins and glucose were added after the media cooled to room temperature after autoclaving. When necessary, the media was supplemented with one or more of the following antibiotics: Kanamycin (25 μg/mL; final concentration), Spectinomycin (25 μg/mL; final concentration), and/or Streptomycin (25 μg/mL; final concentration).

Electrocompetent *R. sphaeroides* cells

Electrocompetent *R. sphaeroides* cells were made as follows. A 5 mL culture of *R. sphaeroides* was grown overnight at 30°C in Sistrom's media supplemented with 20% LB. This culture was diluted 1/100 in 300 mL of the same media and grown to an $OD_{660}$ of 0.5-0.8. The cells were chilled on ice for 10 minutes and then centrifuged for 6 minutes at 7,500 g. The supernatant was discarded, and the cell pellet was resuspended in ice-cold 10% glycerol at half of the original volume. The cells were pelleted by centrifugation for 6 minutes at 7,500 g. The supernatant was again discarded, and cells resuspended in ice-cold 10% glycerol at one quarter of the original volume. The last centrifugation and resuspension steps were repeated, followed by centrifugation for 6 minutes at 7,500 g. The supernatant was decanted, and the cells resuspended in the small volume of glycerol that did not drain out. Additional ice-cold 10% glycerol was added to resuspend the cells, if necessary. Forty μL of the resuspended cells was used in a test electroporation to determine if the cells needed to be concentrated by centrifugation or diluted with 10% ice-cold glycerol. Time constants of 8.5-9.0 milliseconds resulted in good transformation efficiencies. If cells were too dilute, the time constant was greater than 9.0 and transformation efficiencies were low. If cells were too concentrated, the electroporation would spark. Once an acceptable time constant was achieved, cells were aliquoted into cold microfuge tubes and stored at -80°C. All water used for media and glycerol was 18.2 Mohm-cm or higher.

Electrocompetent *R. sphaeroides* cells were electroporated as follows. One μL of plasmid DNA was gently mixed into 40 μL of *R. sphaeroides* electrocompetent cells, which were then transferred to an electroporation cuvette with a 0.2 cm electrode gap. Electroporations were conducted using a Biorad Gene Pulser II (Biorad, Hercules, CA) with settings at 2.5 kV of energy, 400 ohms of resistance, and 25 μF of capacitance. Cells were recovered in 400 μL SOC media at 30°C for 6-16 hours. The cells were then plated (200 μL per plate) on the appropriate selective media. Transformation efficiencies averaged about 2,000 transformants/μg of DNA.

<u>Electrocompetent E. *coli* cells</u>

Electrocompetent *E. coli* strain S17-1 cells were made as follows. A 5 mL culture of *E. coli* strain S17-1 was grown overnight at 30°C in LB media supplemented with 25 μg/mL of streptomycin and 25 μg/mL of spectinomycin. This culture was diluted 1/100 in 300 mL of the same media and grown to an $OD_{660}$ of 0.5-0.8. The cells were chilled on ice for 10 minutes and then centrifuged for 6 minutes at 7,500 g. The supernatant was discarded, and the cell pellet was resuspended in ice-cold 10% glycerol at half of the original volume. The cells were pelleted by centrifugation for 6 minutes at 7,500 g. The supernatant was again discarded, and the cells were resuspended in ice-cold 10% glycerol at one quarter of the original volume. The last centrifugation and resuspension steps were repeated, followed by centrifugation for 6 minutes at 7,500 g. The supernatant was decanted, and the cells resuspended in the small volume of glycerol that did not drain out Additional ice-cold 10% glycerol was added to resuspend the cells, if necessary. Cells were aliquoted into cold microfuge tubes and stored at -80°C.

Electrocompetent *E. coli* strain S17-1 cells were electroporated as follows. Forty μL of competent cells was used per electroporation. Electroporation was conducted using a Biorad Gene Pulser II and a standard *E. coli* protocol: 2.5 kV of energy, 200 ohms of resistance, and 25 μF of capacitance. Electroporated cells were recovered in 250-1000 μL of SOC media for one hour, and 10-200 μL of culture was plated per plate of selective media. Transformation efficiencies averaged about 1.5 x $10^4$ transformants/μg of DNA.

<u>Constructs</u>

Various clones were overexpressed in *R. sphaeroides* using the broad-host-range vector pBBR1MCS2 (Kovach et al., Gene, 166:175-176 (1995)) that was engineered to have either an *R. sphaeroides* rrnB promoter, an *R. sphaeroides* glnB promoter, or a tet promoter. The pBBR1MCS2 vector is mobilizable and relatively small (5,144 bp), replicates in *R. sphaeroides,* has a multiple cloning site with lacZα color selection, and carries a kanamycin resistance gene. All restriction enzymes and T4 DNA ligase were obtained from New England Biolabs (Beverly, MA) unless otherwise indicated. All plasmid DNA preparations were done using QIAprep Spin Miniprep Kits or Qiagen Maxi Prep Kits, and all gel purifications were done using QIAquick Gel Extraction Kits (Qiagen, Valencia, CA).

<u>pMCS2rrnBP</u>

The vector designated pMCS2rrnBP, which contains an *R. sphaeroides* rrnB promoter, was constructed by inserting a copy of the *R. sphaeroides* rrnB promoter (rrnBP) into the pBBR1MCS2 vector. The rrnB promoter was isolated from the pTEX124 vector (obtained from S. Kaplan) by digestion with the restriction enzyme BamHI, which releases the promoter as a 363 bp fragment. Alternatively, the rrnB promoter can be obtained by PCR amplifying it from *R. sphaeroides* genomic DNA using primers based on published rrnB sequence (GenBank® accession number X53854). This fragment was gel purified from a 2% Tris-acetate-EDTA (TAE) agarose gel. The pBBRIMCS2 vector was also digested with BamHI, and the enzyme heat inactivated at 80°C for 20 minutes. The digested vector was then dephosphorylated with shrimp alkaline phosphatase (Roche Moelcular Biochemicals, Indianapolis, IN) and gel purified from a 1% TAE-agarose gel. The prepared vector and the rrnBP fragment were ligated using T4 DNA ligase at 16°C for 16 hours. One μL of ligation reaction was used to electroporate 40 μL of *E. coli* Electromax™ DH10B™ cells (Life Technologies, Inc., Rockville, MD). Electroporated cells were plated on LB media containing 25 μg/mL of kanamycin (LBK). Plasmid DNA was isolated from cultures of single colonies and was digested with HindIII restriction enzyme to confirm the presence of a single insertion of the rrnB promoter. The sequence of the rrnBP inserts for these colonies was also confirmed by DNA sequencing.

pMCS2glnBP

The vector designated pMCS2glnBP, which contains an *R. sphaeroides* glnB promoter, was constructed by inserting a copy of the *R. sphaeroides* glnB promoter (glnBP) into the pBBRIMCS2 vector. The glnB promoter was PCR amplified from genomic DNA obtained from *R. sphaeroides* strain 35053. The following primers were designed based on sequence information obtained from GenBank® accession number X71659:

glnBF 5'-ATTATCTAGAATCCGCCCCGCCTCCACCTC-3' (SEQ ID NO:140)
glnBR 5'-GATGGATCCTGGGTAGGGTCGCTGCTGTCC-3' (SEQ ID NO:141)

The primers introduced an XbaI restriction site at the 5' end and a BamHI restriction site at the 3' end. The following reaction mix and PCR program was used to amplify the promoter region of the glnB gene.

| Reaction Mix | | PCR program | |
|---|---|---|---|
| Pfu 10X buffer | 10 μL | 94°C | 2 minutes |
| DMSO | 5 μL | 7 cycles of: | |
| dNTP mix (10 mM) | 4 μL | | 94°C 30 seconds |
| glnBF (50 μM) | 2 μL | | 61°C 45 seconds |
| glnBP (50 μM) | 2 μL | | 72°C 3 minutes |
| Genomic DNA (50ng/μL) | 2 μL | 25 cycles of: | |
| Pfu enzyme (2.5 U/μL) | 2 μL | | 94°C 30 seconds |
| DI water | 73 μL | | 66°C 45 seconds |
| | | | 72°C 3 minutes |
| Total: | 100 μL | 72°C | 7 minutes |
| | | 4°C | Until used further |

The PCR product was separated on a 1.2% TAE-agarose gel. An about 500 bp fragment was excised and gel purified. The isolated DNA was restricted with XbaI and BamHI, and the resulting digested DNA column purified using a Qiagen gel isolation kit. Three μg of pBBR1MCS2 plasmid DNA was digested with BamHI and XbaI. The digestion was inactivated at 80°C for 20 minutes. The digested vector was then dephosphorylated with shrimp alkaline phosphatase and gel purified on a 1% TAE-agarose gel. Eighty-six ng of the prepared pBBR1MCS2 vector was ligated with 60 ng of the digested glnBP PCR product using T4 DNA ligase at 14°C for 14-16 hours. One μL of ligation reaction was used to electroporate 40 μL of *E. coli* Electromax™ DH10B™ cells. Electroporated cells were plated on LB media containing 25 μg/mL of kanamycin and 50 μg/mL of Xgal (LBKX). Eight individual, white colonies were selected, and their plasmid DNA isolated using a QIAprep Spin Miniprep Kit. Plasmid DNA isolated from each colony was digested in separation reaction mixtures with PstI and a combination of EcoRI/XbaI. All eight clones had a restriction pattern that indicated the presence of the insert. The sequence of three clones was verified.

pMCS2tetP

The vector designated pMCS2tetP, which contains a tet promoter, was constructed by cloning the promoter for the tetracycline resistance determinants from transposon. Tn1721 (Waters et al., Nucleic Acids Research, 11(17): 6089-6105 (1983)) into the pBBRIMCS2 vector. The tetA gene promoter (tetP) was amplified using plasmid pRK415 as template. The following primers were designed to introduce an XbaI restriction site at the beginning of the amplified fragment and a BamHI site at the end of the amplified fragment.

TETXBAF 5'-TTATCTAGAACCGTCTACGCCGACCTC-GTTCAAC-3' (SEQ ID NO:142)

TETBAMR 5'-TTAGGATCCCCTCCGCTGGTCCGATTG-AAC-3' (SEQ ID NO:143)

The PCR mix contained the following: 1X Native Plus Pfu buffer, 20 ng pRK415 plasmid DNA, 0.2 μM of each primer, 0.2 mM of each dNTP, 5% DMSO (v/v), and 10 units of native Pfu DNA polymerase in a final volume of 200 μL. The PCR reaction was performed in a Perkin Elmer Geneamp PCR System 2400 under the following conditions: an initial denaturation at 94°C for 1 minute; 8 cycles of 94°C for 30 seconds, 60°C for 45 seconds, and 72°C for 45 seconds; 24 cycles of 94°C for 30 seconds, 66°C for 45 seconds, and 72°C for 45 seconds; and a final extension for 7 minutes at 72°C. The amplification product was then separated by gel electrophoresis using a 2 %TAE-agarose gel. A 160 bp fragment was excised from the gel and purified. The purified fragment was digested simultaneously with XbaI and BamHI restriction enzymes, and purified with a QIAquick PCR Purification Kit. Three μg of pBBR1MCS2 plasmid DNA was digested with BamHI and XbaI, and the digest was inactivated at 80°C for 20 minutes. The digested vector was then dephosphorylated with shrimp alkaline phosphatase and gel purified on a 1% TAE-agarose gel.

100 ng of the prepared pBBRIMCS2 vector was ligated with 36 ng of the digested tetP PCR product using T4 DNA ligase at 16°C for 16 hours. One μL of ligation reaction was used to electroporate 40 μL of *E. coli* Electromax™ DH5α™ cells. Electroporated cells were plated on LB media containing 25 μg/mL of kanamycin and 50 μg/mL of Xgal (LBKX). Individual, white colonies were resuspended in about 25 μL of 10 mM Tris, and 2 μL of the resuspension was plated on LBKX. The remnant resuspension was heated for 10 minutes at 95°C to break open the bacterial cells. Two μL of the heated cells was used in a 25 μL PCR reaction using the following primers homologous to the vector and flanking the cloning site:

MCS2FS 5'-AGGCGATTAAGTTGGGTAAC-3' (SEQ ID NO: 144)
MCS2RS 5'-GACCATGATTACGCCAAG-3' (SEQ ID NO:145)

The PCR mix contained the following: 1X Taq PCR buffer, 0.2 μM each primer, 0.2 mM each dNTP, and 1 unit of Taq DNA polymerase per reaction. The PCR reaction was performed in a MJ Research PTC100 under the following conditions: an initial denaturation at 94°C for 2 minutes; 32 cycles of 94°C for 30 seconds, 55°C for 45 seconds, and 72°C for 1 minute; and a final extension for 7 minutes at 72°C. All colonies showed a single insertion event. Plasmid DNA was isolated from cultures of two individual colonies and sequenced to confirm the DNA sequence of the tet promoter in the construct.

pMCS2rrnBP/Stdxs

The nucleic acid encoding a *S. trueperi* polypeptide having DXS activity was cloned in the pMCS2rrnBP vector as follows. The *S. trueperi* dxs gene was amplified by PCR using primers homologous to sequence upstream and downstream of the gene. These primers, STDXSMCSF and STDXSMCSR, were designed to introduce a ClaI restriction site at the beginning of the amplified fragment and a KpnI site at the end of the amplified fragment.

STDXSMCSF 5'-GATAATCGATGTGTGACTGACCTGT-CCAAC-3' (SEQ ID NO:146)

STDXSMCSR 5'-CTTAGGTACCATGTTGGAGATTCAA-GGTGG-3'(SEQ ID NO:147)

The PCR mix contained the following: 1X Native Plus Pfu buffer, 200 ng *S. trueperi* genomic DNA, 0.2 μM of

each primer, 0.2 mM each dNTP, 5% DMSO (v/v), and 10 units of native Pfu DNA polymerase (Stratagene, La Jolla, CA) in a final volume of 200 μL. The PCR reaction was performed in a Perkin Elmer Geneamp PCR System 2400 under the following conditions: an initial denaturation at 94°C for 1 minute; 8 cycles of 94°C for 30 seconds, 54°C for 45 seconds, and 72°C for 3.5 minutes; 27 cycles of 94°C for 30 seconds, 60°C for 45 seconds, and 72°C for 3.5 minutes; and a final extension for 7 minutes at 72°C. The amplification product was then separated by gel electrophoresis using a 1% TAE-agarose gel. A 2.2 Kb fragment was excised from the gel and purified. The purified fragment was digested with ClaI restriction enzyme, purified with a QIAquick PCR Purification Kit, digested with KpnI restriction enzyme, purified again with a QIAquick PCR Purification Kit, and quantified on a minigel.

Three μg of the pMCS2rrnBP vector was digested with the restriction enzyme ClaI, gel purified on a 1% TAE-agarose gel, digested with KpnI, purified with a QIAquick PCR Purification Kit, dephosphorylated with shrimp alkaline phosphatase, and purified again with a QIAquick PCR Purification Kit 120 ng of the digested PCR product containing the *S. trueperi* dxs gene and the 50 ng of the prepared pMCS2rrnBP vector was ligated using T4 DNA ligase at 16°C for 16 hours. One μL of the ligation reaction was used to electroporate 40 μL of *E. coli* Electromax™ DH10B™ cells. The electroporated cells were plated onto media. Plasmid DNA was isolated from cultures of individual colonies and evaluated for the presence of the desired insert by restriction enzyme analysis with HindIII and SacI enzymes. The sequence of the Stdxs insert was confirmed by DNA sequencing. The resulting plasmid containing the Stdxs sequence under the control of the rmB promotor was designated pMCS2rrnBP/Stdxs.

Purified pMCS2rmBP/Stdxs plasmid DNA derived from a colony having the correct sequence was then electroporated into electrocompetent cells of *R. sphaeroides* strain 35053. Plasmid DNA was isolated from cultures of individual *R. sphaeroides* colonies. Restriction patterns of plasmid preparations from *R. sphaeroides* are difficult to analyze due to the presence of multiple native plasmids in this species. To check the plasmid integrity in *R. sphaeroides,* one μL of the plasmid preparation from a transformed *R. sphaeroides* colony was used to re-tranform *E. coli* Electromax™ DH10B™ cells by electroporation. Electroporated cells were plated on LBK media. Plasmid DNA was isolated from cultures of individual colonies and evaluated using SacI and HindIII restriction digests.

<u>pMCS2rrnBP/Stdxs2</u>

A second pMCS2rrnBP plasmid containing the nucleic acid encoding a *S. trueperi* polypeptide having DXS activity was constructed. This construct was made using the following forward primer designed to introduce the ribosomal binding site (rbs) from the *R. sphaeroides* dxs 1 gene along with a ClaI restriction site.

**SXSCLAF2 5'-ACTATCGATGAAGGAAGAGCATGGCTGACCT-ACCCAAGAC-3' (SEQ ID NO:146)**

*S. trueperi* genomic DNA was used as template in a PCR mixture using the primers SXSCLAF2 and STDXSMCSR. The PCR program and reaction mixture used were identical to those described for the pMCS2miBP/Stdxs construct. The PCR product was gel purified, digested with ClaI, purified with a QIAquick PCR Purification Kit, digested with restriction enzyme KpnI, and purified again with a QIAquick PCR Purification Kit 150 ng of digested PCR product was ligated into 50 ng of the prepared pMCS2rmBP vector using T4 DNA ligase at 16°C for 16 hours. One μL of the ligation reaction was transformed into *E. coli* Electromax™ DH10B™ cells, and the electroporated cells were plated onto LBK plates. Plasmid DNA was isolated from cultures of individual colonies and evaluated for the presence of the desired insert by restriction enzyme analysis with HindIII and SacI enzymes. The sequence of the dxs insert was confirmed by DNA sequencing. The resulting plasmid containing the Stdxs sequence under the control of the rrnB promotor and having an *R. sphaeroides* ribosomal binding site was designated pMCS2rrnBP/Stdxs2.

A confirmed construct was electroporated into *R. sphaeroides* strain 35053, and the electroporated cells were plated onto LBK media. Individual colonies were resuspended in about 25 μL of 10 mM Tris, and 2 μL of the resuspension was plated on LBK media. The remnant resuspension was heated for 10 minutes at 95°C to break open the bacterial cells, and two μL of the heated cells used in a 25 μL PCR reaction using the SXSCLAF2 and STDXSMCSR primers. The PCR mix contained the following: 1X Taq PCR buffer, 0.2 μM each primer, 0.2 mM each dNTP, 5% DMSO (v/v), and 1 unit of Taq DNA polymerase (Roche) per reaction. The PCR reaction was performed in a MJ Research PTC100 under the following conditions: an initial denaturation at 94°C for 2 minutes; 8 cycles of 94°C for 30 seconds, 54°C for 1 minute, and 72°C for 3.5 minutes; 24 cycles of 94°C for 30 seconds, 60°C 1 minute, and 72°C for 3.5 minutes; and a final extension for 7 minutes at 72°C.

pMCS2rrnBP/Rsdds

The nucleic acid encoding a *R. sphaeroides* polypeptide having DDS activity was cloned in the pMCS2rrnBP vector as follows. The *R. sphaeroides* dds gene was PCR amplified using the following primer pair:

RDS18F 5'-ACTAGAATTCCGCAACAGTTCCTTCATGTC-3' (SEQ ID NO:147)
RSDDSMCSR 5'-CTAGATCGATACTTGCGGTCGGACTGATAG-3' (SEQ ID NO:148)

The forward primer was located upstream of the start codon and introduced an EcoRI restriction site, while the reverse primer was located downstream of the stop codon and introduced a ClaI restriction site. Since the forward primer was located upstream, the *R. sphaeroides* dds maintained its native ribosomal binding site. The following reaction mix and PCR program were used to amplify the *R. sphaeroides* dds gene.

| Reaction Mix | | Program | |
|---|---|---|---|
| Pfu 10X buffer | 10 μL | 94°C 2 | minutes |
| DMSO | 5 μL | 8 cycles of: | |
| dNTP mix (10 mM) | 4 μL | 94°C | 30 seconds |
| RDS18F (50 μM) | 2 μL | 55°C | 45 seconds |
| RSDDSMCSR (50 μM) | 2 μL | 72°C | 3 minutes |
| Genomic DNA (50 ng/μL) | 2 μL | 21 cycles of: | |
| Pfu enzyme (2.5 U/μL) | 1 μL | 94°C | 30 seconds |
| DI water | 74 μL | 61°C | 45 seconds |
| | | 72°C | 3 minutes |
| Total: | 100 μL | 72°C | 7 minutes |
| | | 4°C | Until used further |

The PCR product was separated on a 1% TAE-agarose gel, and an about 1.8 Kb fragment was excised and gel purified. The isolated DNA was restricted with EcoRI and ClaI, and was column purified using a Qiagen gel isolation kit. Three μg of pMCS2rrnBP vector DNA was digested with EcoRI, and the linear DNA was gel isolated using a Qiagen gel isolation kit. The vector was further digested with ClaI, and the DNA was column purified. The double-digested vector was then dephosphorylated with shrimp alkaline phosphatase and purified using a QIAquick PCR Purification Kit. The EcoRI/ClaI-digested *R. sphaeroides* dds PCR product was ligated into the prepared vector using T4 DNA ligase for 14-16 hours at 16°C. One μL of the ligation reaction was transformed into *E. coli* Electromax™ DH10B™ cells, which were then plated on LBK (25 μg/mL) media. Individual colonies were resuspended in about 25 μL of DI water, and 2 μL of the resuspension was plated on LBK. The remnant resuspension was heated for 10 minutes at 95°C to break open the bacterial cells, and 2 μL of the heated cells was used in a 25 μL PCR reaction using the RDS 18F and RSDDSMCSR primers. The PCR mix contained the following: 1X Taq PCR buffer, 0.2 μM each primer, 0.2 mM each dNTP, 5% DMSO (v/v), and 1 unit of Taq DNA polymerase per reaction. The PCR reaction was performed under the following conditions: an initial denaturation at 94°C for 2 minutes; 6 cycles of 94°C for 30 seconds, 55°C for 45 seconds, and 72°C for 3 minutes; 25 cycles of 94°C for 30 seconds, 61°C 45 seconds, and 72°C for 3 minutes; and a final extension for 7 minutes at 72°C. The resulting plasmid containing the Rsdds sequence under the control of the rrnB promotor was designated pMCS2rmBP/Rsdds.

The pMCS2nnBP/Rsdds plasmid was electroporated into *E. coli* strain S 17-1. This strain contains a chromosomal copy of the trans-acting elements that mobilize oriT-containing plasmids during conjugation with a second bacterial strain. It also carries a gene conferring resistance to the antibiotics streptomycin and spectinomycin.

Using the S17-1 strain, the pMCS2rrnBP/Rsdds plasmid was transferred to *R. sphaeroides* 35053 by conjugation. Individual colonies were purified by restreaking on LBK plates. Single colonies were screened by PCR using the RDS18F and RSDDSMCSR primers to confirm the presence of the insert as described above.

pMCS2rmBP/Stdds

The nucleic acid encoding a *S. true peri* polypeptide having DDS activity was cloned in the pMCS2rrnBP vector as follows. The *S. trueperi* dds gene was PCR amplified using the following primer pair:

STDDSMCSF 5'-GTCGCTCGAGATCAGATAATCGTCGCTCAA-3' (SEQ ID NO:149)
STDDSMCSR 5'-ATATGGTACCGACATGGACGAGGAAGACGC-3' (SEQ ID NO:150)

The forward primer was located upstream of the start codon and introduced a XhoI restriction site, while the reverse primer was located downstream of the stop codon and introduced a KpnI restriction site. Since the forward primer was located upstream, the *S. trueperi* dds fragment maintained its native ribosomal binding site. The following reaction mix and PCR program were used to amplify the *S. trueperi* dds gene.

| Reaction Mix | | Program | | |
|---|---|---|---|---|
| Pfu 10X buffer | 10 µL | 94°C | 2 minutes | |
| DMSO | 5 µL | 8 cycles of: | | |
| dNTP mix (10 mM) | 4 µL | | 94°C | 30 seconds |
| SHDDSMCSF (50 µM) | 2 µL | | 55°C | 45 seconds |
| SHDDSMCSR (50 µM) | 2 µL | | 72°C | 3 minutes |
| Genomic DNA (50 ng/µL) | 2 µL | 21 cycles of: | | |
| Pfu enzyme (2.5 U/µL) | 1 µL | | 94°C | 30 seconds |
| DI water | 74 µL | | 61°C | 45 seconds |
| | | | | 72°C 3 minutes |
| Total: | | 100 µL | 72°C | 7 minutes |
| | | | 4°C | Until used further |

The PCR product was separated on a 1% TAE-agarose gel, and an about 1.6 Kb fragment was excised. The DNA was isolated using a Qiagen gel isolation kit. The isolated DNA was restricted with XhoI and KpnI, and was column purified using a Qiagen gel isolation kit. Two µg of pMCS2rmBP vector DNA was digested with KpnI, and the linear DNA was gel isolated using a Qiagen gel isolation kit. The vector was further digested with XhoI, and the DNA was column purified. The double-digested vector was then dephosphorylated with shrimp alkaline phosphatase and column purified using a Qiagen gel purification kit. The XhoI/KpnI-digested *S. trueperi* dds PCR product was ligated into the prepared vector using T4 DNA ligase for 14-16 hours at 16°C. One µL of the ligation reaction was transformed into *E. coli* Electromax™ DH10B™ cells, which were then plated on LBK (25 µg/mL) media. Individual colonies were resuspended in about 25 µL of DI water, and 2 µL of the resuspension was plated on LBK. The remnant resuspension was heated for 10 minutes at 95°C to break open the bacterial cells, and 2 µL of the heated cells was used in a 25 µL PCR reaction using the SHDDSMCSF and SHDDSMCSR primers. The PCR mix contained the following: 1X Taq PCR buffer, 0.2 µM each primer, 0.2 mM each dNTP, 5% DMSO (v/v), and 1 unit of Taq DNA polymerase per reaction. The PCR reaction was performed under the following conditions: an initial denaturation at 94°C for 2 minutes; 6 cycles of 94°C for 30 seconds, 55°C for 45 seconds, and 72°C for 3 minutes; 25 cycles of 94°C for 30 seconds, 61°C 45 seconds, and 72°C for 3 minutes; and a final extension for 7 minutes at 72°C. The resulting plasmid containing the Stdds sequence under the control of the rrnB promotor was designated pMCS2rrnBP/Stdds.

The pMCS2rrnBP/Stdds plasmid was electroporated into *E. coli* strain S 17-1. Using the S17-1 strain, the pMCS2rmBP/Stdds plasmid was transferred to *R. sphaeroides* 35053 by conjugation. Individual colonies were purified by restreaking on LBK plates. Single colonies were screened by PCR using the SHDDSMCSF and SHDDSMCSR primers to confirm the presence of the insert as described above.

pMCS2glnBP/Rsdds

The nucleic acid encoding a *R. sphaeroides* polypeptide having DDS activity was cloned in the pMCS2glnBP vector as follows. The *R. sphaeroides* dds gene was PCR amplified using the following primer pair.

RSDDSF 5'-TAGAGAATTCGAAGGAAGAGCATGGGATTGGACG-AGGTTTC-3' (SEQ ID NO:151)

RSDDSR 5'-TACTACTTGTATGTAGGTACCACTTGCGGTCGGAC-TGATAG-3' (SEQ ID NO:152)

The forward primer introduced an EcoRI restriction site and a ribosomal binding site that was designed based on *R. sphaeroides* dxs1 gene. The reverse primer introduced a KpnI restriction site. Following reaction mix and PCR program was used to amplify the *R. sphaeroides* dds gene.

| Reaction Mix | | Program | | |
|---|---|---|---|---|
| Pfu 10X buffer | 10 μL | 94°C | | 2 minutes |
| DMSO | 5 μL | 7 cycles of: | | |
| dNTP mix (10 mM) | 3 μL | | 94°C | 30 seconds |
| RSDDSF (100 μM) | 1 μL | | 55°C | 45 seconds |
| RSDDSR (100 μM) | I μL | | 72°C | 3 minutes |
| Genomic DNA (50 ng/μL) | 2 μL | 25 cycles of: | | |
| Pfu enzyme (2.5 U/μL) | 2 μL | | 94°C | 30 seconds |
| DI water | 76 μL | | 62°C | 45 seconds |
| | | | 72°C | 3 minutes |
| Total: | 100 μL | 72°C | | 7 minutes |
| | | 4°C | | Until used further |

The PCR product was separated on a 1% TAE-agarose gel, and a fragment about 1.6 Kb in size was excised. The excised DNA was isolated using a Qiagen gel isolation kit. The isolated DNA was restricted with EcoRI and KpnI and was column purified using a Qiagen gel isolation kit. Three μg of pMCS2glnBP vector DNA was digested with KpnI, and the linear DNA was gel isolated using a Qiagen gel isolation kit. The vector was further digested with EcoRI, and the DNA was column purified. The double-digested vector was then dephosphorylated with shrimp alkaline phosphatase and column purified using a Qiagen gel purification kit The KpnI/EcoRI-digested *R. sphaeroides* dds PCR product with the *R. sphaeroides* dxs1 ribosomal binding site described above was ligated into the prepared vector using T4 DNA ligase for 14-16 hours at 16°C. One μL of the ligation reaction was transformed into *E. coli* Electromax™ DH10B™ cells, which were then plated on LBK (25 μg/mL) media. Individual colonies were resuspended in about 25 μL of DI water, and 2 μL of the resuspension was plated on LBK. The remnant resuspension was heated for 10 minutes at 95°C to break open the bacterial cells, and 2 μL of the heated cells was used in a 25 μL PCR reaction using the glnBF and RSDDSR primers. The PCR mix contained the following: 1X Taq PCR buffer, 0.2 μM each primer, 0.2 mM each dNTP, 5% DMSO (v/v), and 1 unit of Taq DNA polymerase per reaction. The PCR reaction was performed under the following conditions: an initial denaturation at 94°C for 2 minutes; 6 cycles of 94°C for 30 seconds, 55°C for 45 seconds, and 72°C for 3 minutes; 25 cycles of 94°C for 30 seconds, 62°C 45 seconds, and 72°C for 3 minutes; and a final extension for 7 minutes at 72°C. A large scale plasmid preparation was done on a culture of a colony containing the Rsdds PCR product, and the glnBP/Rsdds region was sequenced to confirm the lack of nucleotide errors. The resulting plasmid containing the Rsdds sequence under the control of the glnB promotor was designated pMCS2glnBP/Rsdds.

The pMCS2glnBP/Rsdds plasmid DNA was electroporated into electrocompetent *R. sphaeroides* strain 35053 cells as well as electrocompetent carotenoid-deficient mutant cells of 35053 (ATCC 35053/ΔcrtE). Individual colonies of both strains were screened by PCR using the glnBF and RSDDSR primers to confirm the presence of the insert as described above.

pMCS2glnBP/Stdds

The nucleic acid encoding a *S. trueperi* polypeptide having DDS activity was cloned in the pMCS2glnBP vector as follows. The *S. trueperi* dds gene was PCR amplified using the following primer pair.

SHDDSECOVF 5'-GCGTGATATCGAAGGAAGAGCATGAGCGC-AACCGTCCACCG-3' (SEQ ID NO:153)

SHDDSKPNR 5'-ACTGCTAGGGTCCGAGGTACCGACATGGACGA-GGAAGACGC-3' (SEQ ID NO:154)

The forward primer introduced an EcoRV restriction site and a ribosomal binding site that was designed based on the *R. sphaeroides* dxs1 gene. The reverse primer introduced a KpnI restriction site. The following reaction mix and PCR program were used to amplify the *S. trueperi* dds gene.

| Reaction Mix | | Program | |
|---|---|---|---|
| Pfu 10X buffer | 10 μL | 94°C | 2 minutes |
| DMSO | 5 μL | 7 cycles of: | |
| dNTP mix (10 mM) | 3 μL | 94°C | 30 seconds |
| SHDDSECOVF (100 μM) | 1 μL | 58°C | 45 seconds |
| SHDDSKPNR (100 μM) | 1 μL | 72°C | 3 minutes |
| Genomic DNA (50 ng/μL) | 2 μL | 25 cycles of: | |
| Pfu enzyme (2.5 U/μL) | 2 μL | 94°C | 30 seconds |
| DI water | 76 μL | 65°C | 45 seconds |
| | | 72°C | 3 minutes |
| Total: | 100 μL | 72°C | 7 minutes |
| | | 4°C | Until used further |

The PCR product was separated on a 1% TAE-agarose gel, and a fragment about 1.2 Kb in size was excised. The excised DNA was isolated using a Qiagen gel isolation kit. The isolated DNA was restricted with EcoRV and KpnI and was column purified using a Qiagen gel isolation kit. Three μg of pMCS2glnBP vector DNA was digested with KpnI, and the linear DNA was gel isolated using a Qiagen gel isolation kit. The vector was further digested with EcoRV, and the DNA was column purified. The double-digested vector was then dephosphorylated with shrimp alkaline phosphatase and column purified using a Qiagen gel purification kit. The KpnI/EcoRV-digested *S. trueperi* dds PCR product with the *R. sphaeroides* dxs1 ribosomal binding site was ligated into the prepared vector using T4 DNA ligase for 14-16 hours at 16°C. One μL of the ligation reaction was transformed into *E. coli* Electromax™ DH10B™ cells, which were plated on LBK (25 μg/mL) media. Individual colonies were resuspended in about 25 μL of DI water, and 2 μL of the resuspension was plated on LBK. The remnant resuspension was heated for 10 minutes at 95°C to break open the bacterial cells, and 2 μL of the heated cells was used in a 25 μL PCR reaction using the glnBF and RSDDSR primers. The PCR mix contained the following: 1X Taq PCR buffer, 0.2 μM each primer, 0.2 mM each dNTP, 5% DMSO (v/v), and 1 unit of Taq DNA polymerase per reaction. The PCR reaction was performed under the following conditions: an initial denaturation at 94°C for 2 minutes; 6 cycles of 94°C for 30 seconds, 58°C for 45 seconds, and 72°C for 3 minutes; 25 cycles of 94°C for 30 seconds, 65°C 45 seconds, and 72°C for 3 minutes; and a final extension for 7 minutes at 72°C. A large scale plasmid preparation was done on a culture of a colony containing the Stdds PCR product, and the glnBP/Stdds region was sequenced to confirm the lack of nucleotide errors. The resulting plasmid containing the Stdds sequence under the control of the glnB promotor was designated pMCS2gInBP/Stdds.

The pMCS2glnBP/Stdds plasmid DNA was electroporated into electrocompetent cells of *R. sphaeroides* strain 35053 and a carotenoid-deficient mutant of 35053 (ATCC 35053/ΔcrtE). Individual colonies of both strains were screened by PCR using the gInBF and SHDDSKPNR primers to confirm the presence of the insert as described above.

<u>pMCS2tetP/Stdxs</u>

The nucleic acid encoding a *S. trueperi* polypeptide having DXS activity was cloned in the pMCS2tetP vector as follows. The pMCS2tetP plasmid DNA was digested with the restriction enzyme KpnI, cleaned with a QIAquick PCR Purification Kit, and digested with the restriction enzyme ClaI. The enzyme reactions were inactivated by heating at 65°C for 20 minutes. The digested vector DNA was then dephosphorylated with shrimp alkaline phosphatase and gel purified on a 1% TAE-agarose gel. The Kpn1/ClaI-digested *S. trueperi* dxs PCR product described above with the *R. sphaeroides* dxs1 ribosomal binding site was ligated into the prepared vector using T4 DNA ligase for 16 hours at 16°C. One μL of the ligation reaction was transformed into *E. coli* Electromax™ DH5α™ cells, which were plated on LBK media. Individual colonies were resuspended in about 25 μL of 10 mM Tris, and 2 μL of the resuspension was plated on LBK. The remnant resuspension was heated for 10 minutes at 95°C to break open the bacterial cells, and 2 μL of the heated cells was used in a 25 μL PCR reaction using the SXSCLAF2 and SHDXSMCSR primers. The PCR mix contained the following: 1X Taq PCR buffer, 0.2 μM each primer, 0.2 mM each dNTP, 5% DMSO (v/v), and 1 unit of Taq DNA polymerase per reaction. The PCR reaction was performed in a MJ Research PTC100 under the following conditions: an initial denaturation at 94°C for 2 minutes; 8 cycles of 94°C for 30 seconds, 54°C for 1 minute, and 72°C for 3.5 minutes; 24 cycles of 94°C for 30 seconds, 60°C 1 minute, and 72°C for 3.5 minutes; and a final extension for 7 minutes at 72°C. A large scale plasmid preparation was done on a culture of a colony containing the *S. trueperi* dxs PCR product, and the tetP/Stdxs region was sequenced to confirm the lack of nucleotide errors. The resulting plasmid containing the Stdxs sequence under the control of the tet promotor was designated pMCS2tetP/Stdxs.

Plasmid DNA (pMCS2tetP/Stdxs) was electroporated into electrocompetent cells of *R. sphaeroides* strain 35053 and a carotenoid-deficient mutant of 35053 (ATCC 35053/ΔcrtE). Individual colonies of both strains, along with an *E. coli* control, were screened by PCR using the TETXBAF and STDXSMCSR primers to confirm the presence of the insert as described above.

pMCS2tetP/Rsdds

The nucleic acid encoding a *R. sphaeroides* polypeptide having DDS activity was cloned in the pMCS2tetP vector as follows. Three μg of plasmid DNA of the pMCS2tetP vector was digested with the restriction enzyme KpnI. The digested DNA was cleaned with a QIAquick PCR Purification Kit and digested with the restriction enzyme EcoRI, after which the enzyme was inactivated by heating at 65°C for 20 minutes. The digested vector DNA was then dephosphorylated with shrimp alkaline phosphatase and gel purified. Sixty ng of vector DNA was ligated with 120 ng of the KpnI/EcoR I-digested *R. sphaeroides* dds PCR product described above using T4 DNA ligase at 16°C for 16 hours. One μL of the ligation reaction was transformed into *E. coli* Electromax™ DH5α™, which were then plated on LBK media. Individual colonies were resuspended in about 25 μL of 10 mM Tris, and 2 μL of the resuspension was plated on LBK. The remnant resuspension was heated for 10 minutes at 95°C to break open the bacterial cells, and 2 μL of the heated cells used in a 25 μL PCR reaction using the TETXBAF and RSDDSMCSR primers. The PCR mix contained the following: 1X Taq PCR buffer, 0.2 μM each primer, 0.2 mM each dNTP, 5% DMSO (v/v), and 1 unit of Taq DNA polymerase per reaction. The PCR reaction was performed in a MJ Research PTC100 under the following conditions: an initial denaturation at 94°C for 2 minutes; 8 cycles of 94°C for 30 seconds, 55°C for 1 minute, and 72°C for 3 minutes; 24 cycles of 94°C for 30 seconds, 64°C 1 minute, and 72°C for 3 minutes; and a final extension for 7 minutes at 72°C. Plasmid DNA was isolated for a colony having the desired insert, and the tetP/Rsdds region was sequenced to confirm the lack of nucleotide errors from PCR. The resulting plasmid containing the Rsdds sequence under the control of the tet promotor was designated pMCS2tetP/Rsdds.

Plasmid DNA (pMCS2tetP/Rsdds) was electroporated into electrocompetent cells of *R. sphaeroides* strain 35053 and the ATCC 35053/ΔcrtE strain. Individual colonies of both strains, along with an *E. coli* control, were screened by PCR using the TETXBAF and RSDDSMCSR primers to confirm the presence of the insert as described above.

pMCS2tetP/Stdds

The nucleic acid encoding a *S. trueperi* polypeptide having DDS activity was cloned in the pMCS2tetP vector as follows. Three μg of pMCS2tetP plasmid DNA was digested with the restriction enzyme KpnI. The digested DNA was gel purified and digested with the restriction enzyme EcoRV. The enzyme was then inactivated by heating at 80°C for 20 minutes, and the DNA dephosphorylated with shrimp alkaline phosphatase. The dephosphorylated DNA was purified using a QIAquick PCR purification kit. Fifty μg of digested vector DNA was ligated with 150 ng of the KpnI/EcoRV-digested *S. trueperi* dds PCR product described above using T4 DNA ligase at 16°C for 16 hours. One μL of the ligation reaction was transformed into *E. coli* Electromax™ DH10B™ cells, which were then plated on LBK media. Individual colonies were resuspended in about 25 μL of 10 mM Tris, and 2 μL of the resuspension was plated on LBK. The remnant resuspension was heated for 10 minutes at 95°C to break open the bacterial cells, and 2 μL of the heated cells used in a 25 μL PCR reaction using the TETXBAF and STDDSMCSR primers. The PCR mix contained the following: 1X Taq PCR buffer, 0.2 μM each primer, 0.2 mM each dNTP, 5% DMSO (v/v), and 1 unit of Taq DNA polymerase per reaction. The PCR reaction was performed in a MJ Research PTC100 under the following conditions: an initial denaturation at 94°C for 2 minutes; 8 cycles of 94°C for 30 seconds, 55°C for 1 minute, and 72°C for 3 minutes; 24 cycles of 94°C for 30 seconds, 64°C for 1 minute, and 72°C for 3 minutes; and a final extension for 7 minutes at 72°C. Plasmid DNA was isolated for a colony having the desired insert and was sequenced in the tetP/Stdds region to confirm the DNA sequence of the insert. The resulting plasmid containing the Stdds sequence under the control of the tet promotor was designated pMCS2tetP/Stdds.

Plasmid DNA (pMCS2tetP/Stdds) was electroporated into electrocompetent cells of *R. sphaeroides* strain 35053 and the ATCC 35053/ΔcrtE strain. Individual colonies of both strains, along with an *E. coli* control, were screened by PCR using the TETXBAF and STDDSMCSR primers to confirm the presence of the insert as described above.

pMCS2tetP/Stdxs/Rsdds

Nucleic acid encoding a *S. trueperi* polypeptide having DXS activity as well as nucleic acid encoding a *R. sphaeroides* polypeptide having DDS activity was cloned into the pMCS2tetP vector as follows. A vector containing both the *S. trueperi* dxs gene and the *R. sphaeroides* dds gene, each behind a tet promoter, was constructed using the pMCS2tetP/Stdxs construct described above as the starting vector. This vector was digested with restriction enzyme XbaI, cleaned with a QIAquick PCR Purification Kit, and digested with the restriction enzyme Bpu10I (Fermentas, Hanover, MD). The enzyme reaction was inactivated by heating for 20 minutes at 80°C. The digested vector DNA was then dephosphorylated using shrimp alkaline phosphatase and gel purified on a 1% TAE-agarose gel.

A PCR product containing a tet promoter region followed by a *R. sphaeroides* dds gene was amplified using the pMCS2tetP/Rsdds construct described above as template. The PCR mix contained the following: 1X Native Plus Pfu buffer, 5 ng plasmid template, 0.2 μM each primer, 0.2 mM each dNTP, 5% DMSO (v/v), and 10 units of native Pfu DNA polymerase in a final volume of 200 μL. The PCR reaction was performed in a MJ Research PTC 100 under the following conditions: an initial denaturation at 94°C for 2 minutes; 8 cycles of 94°C for 30 seconds, 55°C for 1 minute, and 72°C for 3 minutes; 24 cycles of 94°C for 30 seconds, 64°C 1 minute, and 72°C for 3 minutes; and a final extension for 7 minutes at 72°C. The amplification product was then separated by gel electrophoresis using a 1% TAE-agarose gel. A 1.6 Kb fragment was excised from the gel and purified. The purified fragment was digested with Bpu10I, cleaned with a QIAquick PCR Purification Kit, digested with Xba I restriction enzyme, purified again with a QIAquick PCR Purification Kit, and quantified on a minigel.

60 ng of the prepared pMCS2tetP/Stdxs vector was ligated with 70 ng of the digested tetP/Rsdds PCR product using T4 DNA ligase at 16°C for 16 hours. One μL of ligation reaction was used to electroporate 40 μL of *E. coli* Electromax™ DH5α™ cells. Electroporated cells were plated on LBK media. Individual colonies were screened by PCR using the RSDDSMCSF and STDXSMCSR primers, which produced a 4.1 Kb band. Individual colonies were resuspended in about 25 μL of 10 mM Tris, and 2 μL of the resuspension was plated on LBK. The remnant resuspension was heated for 10 minutes at 95°C to break open the bacterial cells, and 2 μL of the heated cells used in a 25 μL PCR reaction. The PCR reaction mix contained 0.2 μM each primer, 1X Genome Advantage (Clontech, Palo Alto, CA) reaction buffer, 1 M GCMelt, 1.1 mM $Mg(OAc)_2$, 0.2 mM each dNTP, and 1X Genome Advantage Polymerase. The PCR was conducted in a MJ Research PTC100 and consisted of an initial denaturation at 94°C for 1.5 minutes; 32 cycles of a 30 second denaturation at 94°C, a 1 minute annealing at 60°C, and a 6.5 minute extension at 72°C; followed by a final extension at 72°C for 5 minutes. A large-scale plasmid prep was done for a colony that had the desired insert, and plasmid DNA was sequenced through the tetP/Rsdds region to confirm the lack of nucleotide errors from PCR. The resulting plasmid containing the Stdxs sequence under the control of the tet promotor and the Rsdds sequence under the control of the tet promotor was designated pMCS2tetP/Stdxs/Rsdds.

Plasmid DNA (pMCS2tetP/Stdxs/Rsdds) was electroporated into electrocompetent cells of *R. sphaeroides* strains 35053 and the ATCC 35053/ΔcrtE. Individual colonies of both strains, along with an *E. coli* control, were screened by PCR using the RSDDSMCSF and STDDSMCSR primers to confirm the presence of the insert as described above.

<u>pMCS2tetP/Stdxr</u>

Nucleic acid encoding a *S. trueperi* polypeptide having DXR activity was cloned into the pMCS2tetP vector as follows. The *S. trueperi* dxr gene was amplified using genomic DNA as template. The following primers were designed to introduce an EcoRV restriction site and a ribosomal binding based on *R. sphaeroides* dxs1 gene at the beginning of the amplified fragment and a KpnI site at the end of the amplified fragment.

SXRRVF 5'-GATGATATCGAAGGAAGAGCATGGTGAAGCGCGT-CACGGTGT-3' (SEQ ID NO:155)

SXRKPNR 5'-CAAGAGTCAGAAGGTACCCGCCAGAATGGTGAGC-AGGATG-3' (SEQ ID NO:156)

The PCR mix contained the following: 1X Native Plus Pfu buffer, 200 ng genomic DNA, 0.2 μM of each primer, 0.2 mM of each dNTP, 5% DMSO (v/v), and 10 units of native Pfu DNA polymerase in a final volume of 200 μL. The PCR reaction was performed in a MJ Research PTC100 under the following conditions: an initial denaturation at 94°C for 2 minutes; 8 cycles of 94°C for 30 seconds, 59°C for 1 minute, and 72°C for 3 minutes; 24 cycles of 94°C for 30 seconds, 64°C 1 minute, and 72°C for 3 minutes; and a final extension for 7 minutes at 72°C. The amplification product was then separated by gel electrophoresis using a 1 % TAE-agarose gel. A 1.0 Kb fragment was excised from the gel and purified. The purified fragment was digested simultaneously with EcoRV and KpnI restriction enzymes, purified with a QIAquick PCR Purification Kit, and checked on a minigel.

Fifty ng of the EcoRV, KpnI-digested pMCS2tetP vector described above for the pMCS2tetP/Stdds construct was ligated with 75 ng of the digested *S. trueperi* dxr PCR product using T4 DNA ligase at 20°C for 4 hours. One μL of ligation reaction was used to electroporate 40 μL of *E. coli* Electromax™ DH10B™ cells, which were then plated on LBK media. Individual colonies were selected and screened by PCR using the TETXBAF and SXRKPNR primers. The PCR mix contained the following: 1X Taq PCR buffer, 200 ng genomic DNA, 0.2 μM of each primer, 0.2 mM of each dNTP, 5% DMSO (v/v), and 1 unit of Taq DNA polymerase per 25 μL reaction. The PCR reaction was performed in a MJ Research PTC100 under the following conditions: an initial denaturation at 94°C for 2 minutes; 32 cycles of 94°C for 30 seconds, 64°C 1 minute, and 72°C for 3 minutes; and a final extension for 7 minutes at 72°C. A large-scale plasmid preparation was done for a colony that had the desired insert, and the tetP/Stdxr region was sequenced to confirm the DNA sequence of the insert. The resulting plasmid containing the Stdxr sequence under the control of the tet promotor was designated pMCS2tetP/Stdxr.

Plasmid DNA (pMCS2tetP/Stdxr) was electroporated into electrocompetent cells of *R. sphaeroides* strains 35053 and ATCC 35053/ΔcrtE. Individual colonies of both strains, along with an *E. coli* control, were screened by PCR using the TETXBAF and SXRKPNR primers to confirm the presence of the insert as described above.

pMCS2tetP/Stdxr/Stdds

Nucleic acid encoding a *S. trueperi* polypeptide having DXR activity as well as nucleic acid encoding a *S. trueperi* polypeptide having DDS activity was cloned into the pMCS2tetP vector as follows. A vector containing both the *S. trueperi* dxr and dds genes, each behind a tet promoter, was constructed using the pMCS2tetP/Stdds construct described above as the starting vector. This vector was digested with restriction enzyme XbaI, cleaned with a QIAquick PCR Purification Kit, and digested with the restriction enzyme Bpu10I (Fermentas). The enzyme reaction was inactivated by heating for 20 minutes at 80°C. The digested vector DNA was then dephosphorylated with shrimp alkaline phosphatase and gel purified.

A PCR product containing a tet promoter region followed by a *S. trueperi* dxr gene was amplified using the pMCS2tetP/Stdxr construct described above as template and primers TETBPUF and SXRXBAR. The SXRXBAR primer, having the following sequence, was designed to introduce an XbaI restriction site on the end of the PCR product.

SXRXBAR 5'-CAAGAGTCAGAATCTAGACGCCAGAATGGTGA-
GCAGGATG-3' (SEQ ID NO:157)

The PCR mix contained the following: 1X Native Plus Pfu buffer, 5 ng plasmid template, 0.2 μM each primer, 0.2 mM each dNTP, 5% DMSO (v/v), and 10 units of native Pfu DNA polymerase in a final volume of 200 μL. The PCR reaction was performed in a MJ Research PTC 100 under the following conditions: an initial denaturation at 94°C for 2 minutes; 8 cycles of 94°C for 30 seconds, 59°C for 1 minute, and 72°C for 3.5 minutes; 24 cycles of 94°C for 30 seconds, 64°C 1 minute, and 72°C for 3.5 minutes; and a final extension for 7 minutes at 72°C. The amplification product was then separated by gel electrophoresis using a 1% TAE-agarose gel. A 1.4 Kb fragment was excised from the gel and purified. The purified fragment was digested with Bpu10I, cleaned with a QIAquick PCR Purification Kit, digested with XbaI restriction enzyme, purified again with a QIAquick PCR Purification Kit, and quantified on a minigel.

Sixty ng of the prepared pMCS2tetP/Stdds vector was ligated with 80 ng of the digested tetP/Stdxr PCR product using T4 DNA ligase at 16°C for 16 hours. One μL of ligation reaction was used to electroporate 40 μL of *E. coli* Electromax™ DH10B™ cells, which were then plated on LBK media. Individual colonies were screened by PCR using the SXREVF and SDSKPNR primers. Colonies were resuspended in about 25 μL of 10 mM Tris, and 2 μL of the resuspension was plated on LBK media. The remnant resuspension was heated for 10 minutes at 95°C to break open the bacterial cells, and 2 μL of the heated cells used in a 25 μL PCR reaction. The PCR mix contained the following: 1X Taq PCR buffer, 0.2 μM each primer, 0.2 mM each dNTP, 5% DMSO (v/v), and 1 unit of Taq DNA polymerase per reaction. The PCR reaction was performed in a MJ Research PTC100 under the following conditions: an initial denaturation at 94°C for 2 minutes; 8 cycles of 94°C for 30 seconds, 58°C for 1 minute, and 72°C for 4.5 minutes; 24 cycles of 94°C for 30 seconds, 64°C 1 minute, and 72°C for 4.5 minutes; and a final extension for 7 minutes at 72°C. A large-scale plasmid preparation was done for a colony that had the desired insert, and the tetP/Stdxr region was sequenced to confirm the lack of nucleotide errors from PCR. The resulting plasmid containing the Stdxr sequence under the control of the tet promotor and the Stdds sequence under the control of the tet promotor was designated pMCS2tetP/Stdxr/Stdds.

Plasmid DNA (pMCS2tetP/Stdxr/Stdds) was electroporated into electrocompetent cells of *R. sphaeroides* strains 35053 and ATCC 35053/ΔcrtE. Individual colonies of both strains, along with an *E. coli* control, were screened by PCR using the SXREVF and SDSKPNR primers to confirm the presence of the insert as described above.

pMCS2tetP/EcUbiC

Nucleic acid encoding a *E. coli* polypeptide having chorismate lyase activity was cloned into the pMCS2tetP vector as follows. The *E. coli* ubiC gene was amplified using genomic DNA from *E. coli* strain DH10B as template. The following primers were designed to introduce an EcoRV restriction site and a ribosomal binding site based on *R. sphaeroides* dxs 1 gene at the beginning of the amplified fragment, and a KpnI site at the end of the amplified fragment.

UBICRVF 5'-CTAGATATCGGAAGGAAGAGCATGTCACAC-CCCGCGTTA-3' (SEQ ID NO:158)

UBICKPNR 5'-TCAGGTACCGTGTCGCCACCCACAACGCC-CATAATG-3' (SEQ ID NO:159)

The PCR mix contained the following: 1X Native Plus Pfu buffer, 200 ng genomic DNA, 0.2 μM each primer, 0.2 mM each dNTP, and 10 units of native Pfu DNA polymerase in a final volume of 200 μL. The PCR reaction was performed in a MJ Research PTC100 under the following conditions: an initial denaturation at 94°C for 2 minutes; 8 cycles of 94°C for 30 seconds, 57°C for 1 minute, and 72°C for 2.5 minutes; 24 cycles of 94°C for 30 seconds, 64°C 1 minute, and 72°C for 2.5 minutes; and a final extension for 7 minutes at 72°C. The amplification product was then separated by gel electrophoresis using a 1.5 % TAE-agarose gel. A 650 bp fragment was excised from the gel and purified. The purified fragment was digested with EcoRV, cleaned with a QIAquick PCR Purification Kit, digested with KpnI restriction enzyme, purified again with a QIAquick PCR Purification Kit, and quantified on a minigel.

Seventy-five ng of the EcoRV, KpnI-digested pMCS2tetP vector described above for the pMCS2tetP/Stdds construct was ligated with 70 ng of the digested ubiC PCR product using T4 DNA ligase at 16°C for 16 hours. One μL of ligation reaction was used to electroporate 40 μL of *E. coli* Electromax™ DH5α™ cells, which were then plated on LBK media. Individual colonies were resuspended in about 25 μL of 10 mM Tris, and 2 μL of the resuspension was plated on LBK. The remnant resuspension was heated for 10 minutes at 95°C to break open the bacterial cells, and 2 μL of the heated cells used in a 25 μL PCR reaction using the TETXBAF and UBICKPNR primers. The PCR mix contained the following: 1X Taq PCR buffer, 0.2 μM each primer, 0.2 mM each dNTP, and 1 unit of Taq DNA polymerase per reaction. The PCR reaction was performed in a MJ Research PTC100 under the following conditions: an initial denaturation at 94°C for 2 minutes; 32 cycles of 94°C for 30 seconds, 62°C for 1 minute, and 72°C for 2 minutes; and a final extension for 7 minutes at 72°C. A large-scale plasmid preparation was done for a colony that had the desired insert and the tetP/ubiC region was sequenced to confirm the DNA sequence of the insert. The resulting plasmid containing the UbiC sequence under the control of the tet promotor was designated pMCS2tetP/EcUbiC.

Plasmid DNA (pMCS2tetP/EcUbiC) was electroporated into electrocompetent cells of *R. sphaeroides* strain 35053 and the ATCC 35053/ΔcrtE strain. Individual colonies of both strains, along with an *E. coli* control, were screened by PCR using the TETXBAF and UBICKPNR primers to confirm the presence of the insert as described above with the addition of 5% DMSO (v/v) to the PCR reaction.

pMCS2tetP/Stdxs/Rsdds/EcUbiC

Nucleic acid encoding an *S. trueperi* polypeptide having DXS activity, nucleic acid encoding an *R. sphaeroides* polypeptide having DDS activity, and nucleic acid encoding an *E. coli* polypeptide having chorismate lyase activity was cloned into the pMCS2tetP vector as follows. A vector containing the *S. trueperi* dxs gene, the *R sphaeroides* dds gene, and the *E. coli* ubiC gene, each behind a tet promoter, was constructed using the pMCS2tetP/Stdxs/Rsdds construct described above as the starting vector. This vector was digested with restriction enzyme KpnI, cleaned with a QIAquick PCR Purification Kit, and digested with the restriction enzyme NsiI. The enzyme reaction was inactivated by heating for 20 minutes at 65°C. The digested vector DNA was then dephosphorylated with shrimp alkaline phosphatase and gel purified.

A PCR product containing a tet promoter region followed by an *E. coli* ubiC gene was amplified using the pMCS2tetP/EcUbiC construct described above as template. The following primers were designed to introduce an KpnI restriction site at the beginning of the amplified fragment and an NsiI site at the end of the amplified fragment.

TETKPNF 5'-TAGGGTACCACCGTCTACGCCGACCT-CGTTCAAC-3' (SEQ ID NO:160)

UBICNSIR 5'-TGTATGCATGTCGCCACCCACAACGC-CCATAATG-3' (SEQ ID NO:161)

The PCR mix contained the following: 1X Native Plus Pfu buffer, 5 ng plasmid template, 0.2 μM each primer, 0.2 mM each dNTP, 5% DMSO (v/v), and 10 units of native Pfu DNA polymerase in a final volume of 200 μL. The PCR reaction was performed in a MJ Research PTC 100 under the following conditions: an initial denaturation at 94°C for 2 minutes; 8 cycles of 94°C for 30 seconds, 62°C for 1 minute, and 72°C for 2.5 minutes; 24 cycles of 94°C for 30 seconds, 66°C 1 minute, and 72°C for 2.5 minutes; and a final extension for 7 minutes at 72°C. The amplification product was then separated by gel electrophoresis using a 1% TAE-agarose gel. An 850 bp fragment was excised from the gel and purified. The purified fragment was digested with the restriction enzyme NsiI, cleaned with a QIAquick PCR Purification Kit, digested with the restriction enzyme KpnI, purified again with a QIAquick PCR Purification Kit, and quantified on a minigel.

Fifty ng of the prepared pMCS2tetP/Stdxs/Rsdds vector was ligated with 35 ng of the digested tetP/ubiC PCR product using T4 DNA ligase at 16°C for 16 hours. One μL of ligation reaction was used to electroporate 40 μL of *E. coli* Electromax™ DH10B™ cells, which were then plated on LBK media. Individual colonies were resuspended in about 25 μL of 10 mM Tris, and 2 μL of the resuspension was plated on LBK. The remnant resuspension was heated for 10 minutes at 95°C to break open the bacterial cells, and 2 μL of the heated cells used in a 25 μL PCR reaction using the SXSCLAF2 and UBICNSIR primers. The PCR reaction mix contained 1X GC-RICH PCR reaction buffer, 1.0 M GC-RICH resolution solution, 0.2 μM each primer, 0.2 mM each dNTP, and 1 unit of GC-RICH enzyme mix per reaction (Roche). The PCR reaction was performed in a MJ Research PTC100 under the following conditions: an initial denaturation at 94°C for 2 minutes; 8 cycles of 94°C for 30 seconds, 60°C for 1 minute, and 72°C for 5 minutes; 24 cycles of 94°C for 30 seconds, 64°C 1 minute, and 72°C for 5 minutes; and a final extension for 7 minutes at 72°C. A large-scale plasmid preparation was done for a colony that had the desired insert, and plasmid DNA was sequenced through the tetP/ubiC region to confirm the lack of nucleotide errors from PCR The resulting plasmid containing Stdxs sequence under the control of the tet promotor, the Rsdds sequence under the control of the tet promotor, and the UbiC sequence under the control of the tet promotor was designated pMCS2tetP/Stdns/Rsdds/EcUbiC.

Plasmid DNA (pMCS2tetP/Stdxs/Rsdds/EcUbiC) was electroporated into electrocompetent cells of *R. sphaeroides* strains 35053 and ATCC 35053/ΔcrtE. Individual colonies of both strains, along with an *E. coli* control, were screened by PCR using the SXSCLAF2 and UBICNSIR primers to confirm the presence of the insert as described above.

<u>pMCS2tetP/RsLytB</u>

Nucleic acid encoding a LytB *R. sphaeroides* polypeptide was cloned into the pMCS2tetP vector as follows. The *R. sphaeroides* lytB was identified by TBLASTN analysis of its genome using an *E. coli* lytB sequence as a query. Based on the identified sequence the following primers were designed to PCR amplify the gene:

LYTBHINDF 5'-GACGAAGCTTGAAGGAAGAGCATGCCTCCCCTCA-CCCTCTATC-3' (SEQ ID NO:162)

LYTBKPNR 5'-GTCACTGAATGAATGGTACCGCAGCCGAGAACCG-CCAGAAGCC-3' (SEQ ID NO:163)

The primers introduced a HindIII restriction site and ribosomal binding site at the 5' end, and a KpnI restriction site at the 3' end. The following reaction mix and PCR program were used to amplify the lytB gene.

| Reaction Mix | | Program | |
|---|---|---|---|
| Pfu 10X buffer | 10 μL | 94°C 2 minutes | |
| DMSO | 5 μL | 7 cycles of: | |
| dNTP mix (10 mM) | 3 μL | | 94°C 30 seconds |
| LYTBHINDF (100 μM) | 1 μL | | 59°C 45 seconds |
| LYTBKPNR (100 μM) | 1 μL | | 72°C 3 minutes |
| Genomic DNA (50 ng/μL) | 2 μL | 25 cycles of: | |
| Pfu enzyme (2.5 U/μL) | 2μL | | 94°C 30 seconds |
| DI water | 76 μL | | 66°C 45 seconds |
| | | | 72°C 3 minutes |
| Total: | 100 μL | 72°C | 7 minutes |
| | | 4°C | Until used further |

The PCR product was run on a 1% TAE agarose gel, and a fragment about 1.1 Kb in size was excised. The excised DNA was isolated using a Qiagen gel isolation kit The isolated DNA was restricted with HindIII and KpnI, and was column purified using a Qiagen gel isolation kit Two μg of pMCS2tetP vector DNA was digested with HindIII, and the linear DNA was gel isolated using a Qiagen gel isolation kit. The vector was further digested with KpnI, and the DNA was column purified. The double-digested vector was then dephosphorylated with shrimp alkaline phosphatase and column purified using a Qiagen gel purification kit. The KpnI/HindIII-digested *R. sphaeroides* lytB PCR product with the *R. sphaeroides* dxs1 ribosomal binding site described above was ligated into the prepared vector using T4 DNA ligase for 14-16 hours at 16°C. One μL of the ligation reaction was transformed into *E. coli* Electromax™ DH10B™ cells, which were then plated on LBK (25 μg/mL) media. Individual colonies were resuspended in about 25 μL of DI water, and 2 μL of the resuspension was plated on LBK. The remnant resuspension was heated for 10 minutes at 95°C to break open the bacterial cells, and 2 μL of the heated cells was used in a 25 μL PCR reaction using the LYTBHINDF and LYTBKPNR primers. The PCR mix contained the following: 1X Taq PCR buffer, 0.2 μM each primer, 0.2 mM each dNTP, 5% DMSO (v/v), and 1 unit of Taq DNA polymerase per reaction. The PCR reaction was performed under the following conditions: an initial denaturation at 94°C for 2 minutes; 8 cycles of 94°C for 30 seconds, 59°C for 1 minute, and 72°C for 3 minutes; 24 cycles of 94°C for 30 seconds, 66°C for 1 minute, and 72°C for 3 minutes; and a final extension for 7 minutes at 72°C. A large scale plasmid preparation was done on a culture of a colony containing the lytB PCR product, and the tetP/lytB region was sequenced to confirm the lack of nucleotide errors. The resulting plasmid containing the RsLytB sequence under the control of the tet promotor was designated pMCS2tetP/RsLytB.

Plasmid DNA (pMCS2tetP/RsLytB) was electroporated into electrocompetent cells of *R. sphaeroides* strain 35053 and a carotenoid-deficient mutant of 35053 (ATCC 35053/ΔcrtE). Individual colonies of both strains, along with an *E. coli* control, were screened by PCR using the TETXBAF and LYTBKPNR primers to confirm the presence of the insert as described above.

<u>pMCS2tetP/Stdxs/Rsdds/RsLvtB</u>

Nucleic acid encoding an *S. trueperi* polypeptide having DXS activity, nucleic acid encoding an *R. sphaeroides* polypeptide having DDS activity, and nucleic acid encoding LytB from *R. sphaeroides* were cloned into the pMCS2tetP vector as follows. The *R. sphaeroides* lytB gene was cloned and expressed along with the *R. sphaeroides* dds and *S. trueperi* dxs genes. In this triple expression system, each gene was expressed through its own tetP. The *R. sphaeroides* lytB gene was PCR amplified along with the tetP using the following primers.

TETKPNF 5'-TAGGGTACCACCGTCTACGCCGACCTC-
GTTGAAC-3' (SEQ ID NO:164)

LYTBNSIR 5'-AGGCAATGCATGCAGCCGAGAACCGCC-
AGAAGCC-3' (SEQ ID NO:165)

The following PCR mix and program were used to PCR amplify the lytB gene along with the tetP.

| Reaction Mix | | Program | | |
|---|---|---|---|---|
| Pfu 10X buffer | 10 μL | 94°C | 2 minutes | |
| DMSO | 5μL | 7 cycles of: | | |
| dNTP mix (10 mM) | 3 μL | | 94°C | 30 seconds |
| TETKPNF (100 μM) | 1 μL | | 63°C | 45 seconds |
| LYTBNSIR (100 μM) | 1 μL | | 72°C | 3 minutes |
| pMCS2tetP/lytB (10 ng/μL) | 1 μL | 25 cycles of: | | |
| Pfu enzyme (2.5 U/μL) | 2 μL | | 94°C | 30 seconds |
| DI water | 77 μL | | 69°C | 45 seconds |
| | | | 72°C 3 minutes | |
| Total: | 100 μL | 72°C | 7 minutes | |
| | | 4°C | Until used further | |

In this PCR reaction, pMCS2tetP/RsLytB plasmid DNA was used as a template. The PCR product was separated on a 1% TAE-agarose gel, and a fragment about 1.4 Kb in size was excised. The excised DNA was isolated using a Qiagen gel isolation kit. The isolated DNA was restricted with NsiI and KpnI, and was column purified using a Qiagen gel isolation kit. Two μg of pMCS2tetP/Stdxs/Rsdds plasmid DNA was digested with NsiI, and the linear DNA was gel isolated using a Qiagen gel isolation kit The vector was further digested with KpnI, and the DNA was column purified. The double-digested vector was then dephosphorylated with shrimp alkaline phosphatase and column purified using a Qiagen gel purification kit. The KpnI/NsiI-digested PCR product was ligated into the prepared plasmid using T4 DNA ligase for 14-16 hours at 16°C. One μL of the ligation reaction was transformed into *E. coli* Electromax™ DH10B™ cells, which were then plated on LBK (25 μg/mL) media. Individual colonies were resuspended in about 25 μL of DI water, and 2 μL of the resuspension was plated on LBK. The remnant resuspension was heated for 10 minutes at 95°C to break open the bacterial cells, and 2 μL of the heated cells was used in a 25 μL PCR reaction using the SXSCLAF2 and LYTBNSIR primers. The PCR mix contained the following: 1X Taq PCR buffer, 0.2 μM each primer, 0.2 mM each dNTP, 5% DMSO (v/v), and 1 unit of Taq DNA polymerase per reaction. The PCR reaction was performed under the following conditions: an initial denaturation at 94°C for 2 minutes; 6 cycles of 94°C for 30 seconds, 59°C for 45 sec, and 72°C for 4 minutes; 25 cycles of 94°C for 30 seconds, 65°C for 45 seconds, and 72°C for 4 minutes; and a final extension for 7 minutes at 72°C. A large scale plasmid preparation was done on a culture of a colony containing the correct insert, and the tetP/lytB region was sequenced to confirm the lack of nucleotide errors. The resulting plasmid containing Stdxs sequence under the control of the tet promotor, the Rsdds sequence under the control of the tet promotor, and the LytB sequence under the control of the tet promotor was designated pMCS2tetP/Stdxs/Rsdds/RsLytB.

Plasmid DNA (pMCS2tetP/Stdxs/Rsdds/RsLytB) was electroporated into electrocompetent cells of *R. sphaeroides* strain 35053 and a carotenoid-deficient mutant of 35053 (ATCC 35053/ΔcrtE). Individual colonies of both strains were screened by PCR using the SXSCLAF2 and LYTBNSIR primers to confirm the presence of the insert as described above.

Example 9 - Making recombinant microorganisms containing knock-outs

Various nucleic acid sequences within the *R. sphaeroides* genome were knocked out. All restriction enzymes and T4 DNA ligase were obtained from New England Biolabs (Beverly, MA) unless otherwise indicated. All plasmid DNA preparations were done using QIAprep Spin Miniprep Kits or Qiagen Maxi Prep Kits, and all gel purifications were done using QIAquick Gel Extraction Kits (Qiagen, Valencia, CA).

ATCC 35053/ΔertE(kan)

*R. sphaeroides* cells lacking crtE were made by inserting a kanamycin resistance gene into the crtE sequence as follows. In general, the crtE gene from *R. sphaeroides* was cloned into a pUC 19 vector, and a kanamycin gene (kan) was inserted into the gene to inactivate it. The crtE-kan insert was amplified by PCR and cloned into pSUP203, a mobilizable ColE1-based plasmid that is not maintained in *R. sphaeroides* unless it is integrated into a *R. sphaeroides* replicon. This plasmid was transformed into *E. coli* strain S 17-1, a strain that is able to mobilize oriT-containing plasmids in conjugations with a second bacterial strain. The S 17-1 strain was conjugated with *R. sphaeroides* strain 35053, and colonies were identified in which the crtE-kan insert had replaced the native crtE gene.

The crtE gene from *R. sphaeroides* strain 17023 was amplified by PCR using primers designed to introduce an SphI restriction site at the beginning of the amplified fragment and an XbaI restriction site at the end of the amplified fragment The sequences of the primers were as follows.

CRTESPHF 5'-AAGCATGCGAAAAAGTTGACACCTGTGGAGTC-3' (SEQ ID NO:166)
CRTEXBAR 5'-ACTCTAGAAGCACCTGCGAATGGACGAAG-3' (SEQ ID NO:167)

The fragment amplified included the crtE gene along with 85 nucleotides upstream of the translational start codon and 228 nucleotides downstream of the translational stop codon. The PCR reaction mix contained 0.2 μM each primer, 1X GC Genomic PCR Buffer (Clontech, Palo Alto, CA),1M GC-Melt, 1.1 mM $Mg(OAc)_2$, 0.2 mM each dNTP, 1X Advantage-GC Genomic Polymerase Mix, and 1 ng of genomic DNA per μL of reaction mix. The PCR was conducted in a Perkin Elmer Geneamp 2400 and consisted of an initial denaturation at 94°C for 30 seconds; 35 cycles of a 15 second denaturation at 94°C, a one minute annealing at 55°C, and a 3 minute extension at 72°C; followed by a final extension at 72°C for 5 minutes. Fifty μL of PCR product was separated on a 1% Tris-Acetate-EDTA (TAE)-agarose gel. A 1180 bp fragment was gel purified, and the purified DNA was digested with XbaI and SphI restriction enzymes (Promega, Madison, WI).

pUC19 vector was digested with the restriction enzymes SphI and XbaI, and gel purified on a 1% TAE- agarose gel. Fifty ng of purified vector was ligated with about 150 ng of digested crtE PCR product for 16 hours at 14°C using T4 DNA ligase (Roche Molecular Biochemicals, Indianapolis, IN). One μL of ligation reaction was transformed into ElectroMAX™ DH10B™ cells (Life Technologies, Gaithersburg, MD), which were then plated on LB media containing 100 μg/mL ampicillin and 50 μg/mL of 5-Bromo-4-Chloro-3-Indolyl-B-D-Galactopyranoside (LBKX). Individual, white colonies were resuspended in about 20 μL of 10 mM Tris, and 2 μL of the resuspension was plated on LBKX media. The remnant resuspension was heated for 10 minutes at 95°C to break open the bacterial cells, and 2 μL of the heated cells was used in a 25 μL PCR reaction using the CRTESPHF and CRTEXBAR primers. The PCR reaction mix contained 0.2 μM each primer, 1X GC Genomic PCR Buffer, 1 M GCMelt, 1.1 mM $Mg(OAc)_2$, 0.2 mM each dNTP, and 1X Advantage-GC Genomic Polymerase Mix. The PCR was conducted in a Perkin Elmer Geneamp 2400 and consisted of an initial denaturation at 94°C for 30 seconds; 35 cycles of a 15 second denaturation at 94°C, a one minute annealing at 55°C, and a 3 minute extension at 72°C; followed by a final extension at 72°C for 5 minutes. Plasmid DNA was isolated for colonies having a crtE gene insert and was digested with the restriction enzyme HindIII and with a mixture of SphI and XbaI to confirm vector structure.

One μg of the pUC19crtE construct was digested with XhoI and StuI restriction enzymes. These enzymes cut a 273 bp fragment of DNA from the center of the crtE gene. The digested DNA was separated on a 1% TAE-agarose gel. A 3.6 Kb fragment representing pUC 19 and the remaining ends of the crtE gene was excised and purified.

The kanamycin resistance gene was amplified by PCR from the PCRII vector (Invitrogen, Carlsbad, CA) using primers designed to introduce an StuI restriction site at the beginning of the amplified fragment and an XhoI restriction site at the end of the amplified fragment. The sequences of the primers were as follows.

KANSTUF 5'-ATAAAGGCCTTACATGGCGATAGCTAGACTG-3' (SEQ ID NO:168)
KANXHOR 5'-AAGGCTCGAGAAGGATCTTACCGCTGTTGAG-3' (SEQ ID NO:169)

The PCR reaction mix contained 0.2 μM each primer, 1X Pfu reaction buffer (Stratagene, La Jolla, CA), 0.2 mM each dNTP, 8 units Pfu, and 5 ng of the PCRII vector in a 200 μL reaction. The PCR was conducted in a Perkin Elmer Geneamp 2400 and consisted of an initial denaturation at 94°C for 2 minutes; 8 cycles of a 30 second denaturation at 94°C, a 1 minute annealing at 55°C, and a 2.5 minute extension at 72°C; 24 cycles of a 30 second denaturation at 94°C, a 1 minute annealing at 55°C, and a 2.5 minute extension at 72°C; followed by a final extension at 72°C for 5 minutes. The PCR product was separated on a 1% TAE- agarose gel, and a 1.2 Kb fragment was excised and purified. One μg of purified DNA was digested with XhoI and StuI restriction enzymes and cleaned using a QIAquick PCR Purification Kit.

Fifty ng of the digested pUC19crtE vector DNA was ligated with 75 ng of the digested kan PCR product for 16 hours at 14°C using T4 DNA ligase (Roche). One μL of ligation mix was electroporated into 40 μL of *E. coli* ElectroMAX™ DH10B™ electrocompetent cells, which were then plated on LB media containing 100 μg/mL ampicillin and 50 μg/mL kanamycin (LBAK). Plasmid DNA was isolated from cultures of individual colonies and was digested in separate reactions with the restriction enzymes PstI, SphI, and a StuI/XbaI mixture to confirm correct vector structure.

The crtE gene with the inserted kan gene was amplified by PCR using primers designed to have ScaI restriction sites on both ends of the fragment The sequences of the primers were as follows.

CRTESCAF 5'-ATAGTACTGAAAAAGTTGACACCTGTGGAGTC-3' (SEQ ID NO:170)
CRTESCAR 5'-ATAGTACTAGCACCTGCGAATGGACGAAG-3' (SEQ ID NO:171)

The PCR reaction mix contained 0.2 μM each primer, 1X GC Genomic PCR Buffer, 1 M GCMelt, 1.1 mM Mg(OAc)2,0.2 mM each dNTP, 1X Advantage-GC Genomic Polymerase Mix, and 1 ng of plasmid DNA per μL of reaction mix. The PCR was conducted in a Perkin Elmer Geneamp 9600 and consisted of an initial denaturation at 94°C for 1 minute; 8 cycles of a 30 second denaturation at 94°C, a 1 minute annealing at 55°C, and a 4 minute extension at 72°C; 25 cycles of a 30 second denaturation at 94°C, a 1 minute annealing at 60°C, and a 4 minute extension at 72°C; followed by a final extension at 72°C for 5 minutes. 200 μL of PCR product was separated on a 1% TAE-agarose gel. A 2.0 Kb fragment was excised and purified One μg of purified DNA was digested with ScaI restriction enzyme, and the digested DNA was purified using a QIAquick PCR Purification Kit.

2.3 μg of pSUP203 plasmid DNA was digested with ScaI restriction enzyme. The digested DNA was separated on a 1% TAE-agarose gel, and a 7.6 Kb fragment was excised and purified. The purified plasmid DNA was then dephosphorylated using calf intestinal alkaline phosphatase (Promega). 75 ng of dephosphorylated plasmid DNA was ligated with 60 ng and 120 ng of the ScaI-digested crtE-kan PCR product for 16 hours at 14°C using T4 DNA ligase (New England BioLabs). One μL of ligation mix was electroporated into 40 μL of *E. coli* ElectroMAX™ DH10B™ electrocompetent cells, which were then plated on LB media containing 10 μg/mL tetracycline, to which pSUP203 carries a resistance gene, and 25 μg/mL kanamycin. Plasmid DNA was isolated from cultures of individual colonies and digested with ScaI restriction enzyme to check insert size. 100 ng of plasmid DNA derived from a confirmed colony was electroporated into electrocompetent cells of the *E. coli* strain S17-1. This strain contains a chromosomal copy of the transacting elements that mobilize oriT-containing plasmids during conjugation with a second bacterial strain. It also carries a gene conferring resistance to the antibiotics streptomycin and spectinomycin. The transformation reaction was plated on LB media with 10 μg/mL tetracycline, 25 μg/mL kanamycin, and 25 μg/mL streptomycin. Individual colonies were resuspended in about 20 μL of 10 mM Tris and heated for 10 minutes at 95°C to break open the bacterial cells. Two μL of the heated cells was used in a 25 μL PCR reaction using the CRTESCAF and CRTESCAR primers to confirm the presence of the crtE-kan insert. The PCR reaction mix contained 0.2μM each primer, 1X GC Genomic PCR Buffer, 1.0 M GCMelt, 1.1 mM $Mg(OAc)_2$,0.2 mM each dNTP, and 1X Advantage-GC Genomic Polymerase Mix. The PCR was conducted in a Perkin Elmer Geneamp 9600 and consisted of an initial denaturation at 94°C for 1 minute; 30 cycles of a 30 second denaturation at 94°C, a 1 minute annealing at 56°C, and a 4 minute extension at 72°C; followed by a final extension at 72°C for 5 minutes.

The pSUP203crtE-kan construct was introduced into *R. sphaeroides* strain 35053 through conjugation with the *E. coli* S17-1 strain carrying this vector. The S17-1 donor was grown in LB media with 25 μg/mL kanamycin and 25 μg/mL streptomycin at 37°C for 16 hours. A growing culture of *R. sphaeroides* strain 35053 was used to inoculate Sistrom's media using 1/5 to 1/10 dilutions, and the subcultures were grown at 30°C for about 20 hours. For both the S17-1crtE-kan and 35053 genotypes, cells were pelleted from 1.5 mL of culture. Pellets were resuspended and pelleted four times in either 1X Sistrom's salts for the 35053 cells or LB media for the S17-1 cells. The pellets were each resuspended in 1.5 mL of LB, and 200 μL of the S17-1 cells was combined with 1.3 mL of the 35053 cells. This mixture was pelleted, the supernatant removed, and the pellet resuspended in 20 μL of LB media. The resuspended cells were spotted onto an LB plate and incubated at 30°C for 7.5 hours. The cells were then scraped off the plate, resuspended in 1.5 mL of 1X Sistrom's salts, and plated (200 μL/plate) on Sistrom's media supplemented with 25 μg/mL kanamycin and 10 μg/mL of telluride (SisKTell). The telluride retards the growth of *E. coli* cells but is detoxified by *R. sphaeroides.* After 7 days, small black colonies were picked off the plates and streaked to fresh plates of the same media. After 6 days of growth, grayish colonies were patched to LB plates containing 25 μg/mL kanamycin (LBK25) and also to LB plates containing 0.75 μg/mL tetracycline. Desirable double-crossover events, in which the crtE-kan gene was integrated and retained in the genome while the vector DNA was lost, exhibited kanamycin resistance but lacked tetracycline resistance. Colonies resulting from undesirable single-crossover events demonstrated both kanamycin and tetracycline resistance.

The mutants were confirmed using PCR and Southern hybridization as follows. Colonies that exhibited kanamycin resistance, lacked tetracycline resistance, and had a gray phenotype were screened by PCR for the crtE locus using the CRTESCAF and CRTESCAR primers as described above. To confirm that they were *R. sphaeroides* colonies with a truncated crtE gene rather than *E. coli* colonies carrying the vector, colonies were also screened using primers specific to the *R. sphaeroides* ppsR gene and the *E. coli* dxs gene. Individual colonies were resuspended in about 20 μL of 10 mM Tris, and heated for 10 minutes at 95°C to break open the bacterial cells. Two μL of the heated cells were used per 25 μL PCR reaction. The PCR reaction mix contained 0.2 μM each primer, 1X GC Genomic PCR Buffer, 1.0 M GCMelt, 1.1 mM $Mg(OAc)_2$, 0.2 mM each dNTP, and 1X Advantage-GC Genomic Polymerase Mix. The PCR was conducted in a Perkin Elmer Geneamp 9600 and consisted of an initial denaturation at 94°C for 1 minute; 8 cycles of a 30 second denaturation at 94°C, a 1 minute annealing at 55°C, and a 3.5 minute extension at 72°C; 22 cycles of a 30 second denaturation at 94°C, a 1 minute annealing at 61°C, and a 3.5 minute extension at 72°C; followed by a final extension at 72°C for 5 minutes. All suspected 35053crtE-kan colonies produced a crtE band the same size as the S17-1crtE-kan control. They all also produced a band of the expected size for the ppsR gene and did not produce a band for the *E. coli* dxs gene.

To further confirm the presence of double-crossover events, Southern hybridization was conducted on eight 35053crtE-kan colonies as well as *R. sphaeroides* strains 35053 and 17023. Sequence data for the photosynthetic operon of strain 17023 is available in Genbank and was used to determine restriction enzymes likely to have hybridization patterns that would distinguish mutants from non-mutants. Genomic DNA was isolated from each line using a Gentra Puregene DNA Isolation Kit (Gentra, Minneapolis, MN). Two μg of genomic DNA was used in digests with the restriction enzymes ApaI and XhoI. The digests were separated on a 0.8% TAE agarose gel, and the DNA transferred to a nylon membrane. DIG-labeled molecular weight markers II and III (Roche) were also included on the gel/membrane. DIG-labeled probes of the crtE locus were synthesized using a PCR DIG Probe Synthesis Kit (Roche). After baking, membranes were prehybridized in EasyHyb Buffer (Roche) for at least 2 hours and hybridized overnight using 400 nL of a 0.5 DIG labeling reaction per mL of hybridization solution. Detection was conducted using a Wash and Block Buffer Set (Roche). Membranes were washed two times for 5-10 minutes each at room temperature in 2X SSC/0.1% SDS and two times for 15-20 minutes each at 68°C in 0.1X SSC/0.1% SDS. They were then covered with blocking buffer and placed on a shaker for an hour at room temperature. The blocking buffer was replaced with fresh blocking buffer containing 150 mU of AP conjugate per mL of buffer, and the membranes shaken at room temperature for an additional 30 minutes. Membranes were then washed twice for 15 minutes each at room temperature with washing buffer, followed by a five minute wash with detection buffer. The detection buffer was replaced with fresh detection buffer containing 20 μL of NBT/BCIP solution per mL of buffer. This was placed in the dark at room temperature with no shaking until color developed, after which the buffer was replaced with 10 mM Tris-1 mM EDTA solution.

In the ApaI digest, the mutant lines exhibited a band of about 850 bp larger than the strain 35053 control, which is the size difference expected from the insertion of the kanamycin gene product in the StuI/XhoI sites. For the XhoI digest, strain 35053 exhibited a band of about 700 bp, strain 17023 had a band of about 1100 bp, mutant 7C had a band of 1550 bp, and the remaining mutants had a band of 2050 bp. The reason for the size difference in the XhoI bands for the mutants was unclear, but mutant 7C was used in further studies due to its possession of the expected band size relative to strain 35053. The resulting *R. sphaeroides* mutant containing a crtE knockout was designated ATCC 35053/ΔcrtE(kan).

ATCC 35053/ΔcrtE

*R. sphaeroides* cells lacking crtE were made using sacB selection as follows. A truncated crtE gene was cloned into the vector pL01, which is a suicide vector in *R. sphaeroides.* The pL01 vector carries a kanamycin resistance gene, a *B. subtilis* sacB gene, an oriT sequence, a ColEI replicon, and a multiple cloning site (Lenz et al., J. Bacteriol., 176 (14):4385-93 (1994)). The pL01crtE plasmid was introduced into *R. sphaeroides* strain 35053 through conjugation with an *E. coli* donor. The kanamycin resistance gene was used to select for single-crossover events between the truncated crtE gene and the genomic crtE gene that resulted in incorporation of the pL01 crtE DNA into the genome. The presence of the sacB gene on the vector allowed for subsequent selection for the loss of the vector DNA from the genome, as expression of this gene in the presence of sucrose is lethal to *E. coli* and to *R. sphaeroides* under certain growth conditions. A portion of the double-crossover events that led to loss of the sacB gene contained the truncated crtE allele. This method of gene knockout is useful because no residual antibiotic resistance gene is left in the genome.

A three-step PCR process was used to create a 249 bp in-frame deletion in the crtE gene. The crtE gene from *R. sphaeroides* strain 35053 was amplified by PCR using primers designed to introduce an SphI restriction site at the beginning of the amplified fragment and a SacI restriction site at the end of the amplified fragment. The sequences of the primers were as follows.

CRTESPHF 5'-CGTGGCATGCGTGTAAGAAAAAGTTGACA-CCTGTGGAGTC-3' (SEQ ID NO:172)

CRTESACR 5'- CTAAGAGCTCAGTTCGGGCTCGGTCTCGC-CTTTCAGGAAG -3' (SEQ ID NO:173)

The PCR reaction mix contained 0.2 μM each primer, 1X Genome Advantage reaction buffer, 1 M GCMelt, 1.1 mM $Mg(OAc)_2$, 0.2 mM each dNTP, 1X Genome Advantage Polymerase, and 1 ng of genomic DNA per μL of reaction mix. The PCR was conducted in a Perkin Elmer Geneamp 2400 and consisted of an initial denaturation at 94°C for 2 minutes; 32 cycles of a 30 second denaturation at 94°C, a 45 second annealing at 64°C, and a 3 minute extension at 72°C, followed by a final extension at 72°C for 7 minutes. 200 μL of PCR product was separated on a 1% TAE-agarose gel, and a 1.5 Kb fragment was excised and purified.

The second round of PCR consisted of two separate reactions: reaction A, which used primers CRTESPHF and CRTERI, and reaction B, which used primers CRTESACR and CRTEFI. The sequences of primers CRTEFI and CRTERI were as follows.

**CRTEFI 5'-GAGAGCGAGAGCCAGATCAAGAAGSGGCTG-AAGGACATCC-3' (SEQ ID NO:174)**

**CRTERI 5'-GGATGTCCTTCAGCCSCTTCTTGATCTGGCT-CTCGCTCTC-3' (SEQ ID NO:175)**

The 20 nucleotides on the 3' ends of this pair of primers are located near the center of the crtE gene, 249 bases apart from each other and facing towards the start (CRTERI) and end (CRTEFI) of the gene. The 20 bp on the 5' ends of these primers are the reverse complement of the 3' end of the other primer in the pair. PCR of the two separate reactions was conducted as in the first round, with the exception that 0.05 ng of first round product per μL of reaction mix was used as template. Also, the thermocycler program used a 2 minute initial denaturation at 94°C; eight cycles of a 30 second denaturation at 94°C, a 45 second annealing at 56°C, and a 3 minute extension at 72°C, followed by eight cycles of a 30 second denaturation at 94°C, a 45 second annealing at 60°C, and a 3 minute extension at 72°C; followed by 16 cycles of a 30 second denaturation at 94°C, a 45 second annealing at 64°C, and a 3 minute extension at 72°C; followed by a final extension at 72°C for 7 minutes. Both PCR products, about 590 and 650 bp in length, were separated on a 1% TAE-agarose gel, excised, and gel purified.

The third round of PCR used the same primers and reaction mixture as the first round of PCR with the exception that a mixture of 10 ng of each second round fragment was used as template rather than genomic DNA (200 μL reaction). The PCR program used was also the same as that used in the first round of PCR with the annealing time lengthened to 1.5 minutes. The 1.2 Kb third-round product was separated on a 1% TAE-agarose gel and purified. Three μg of purified DNA was digested with the restriction enzymes SacI and SphI. The digested DNA was cleaned using a QIAquick PCR Purification Kit and digested with the restriction enzyme StuI. StuI cut within the deleted region and ensured that there was little or no remaining full-length product. The digestion mixture was again cleaned using a QIAquick PCR Purification Kit.

Three μg of the vector pL0 1 was digested with the restriction enzymes SphI and SacI. The enzymes were inactivated by heating to 65°C for 20 minutes, and the vector was dephosphorylated using shrimp alkaline phosphatase (Roche). The dephosphorylated vector DNA was gel purified on a 1% TAE-agarose gel.

Sixty-six ng of digested vector DNA was ligated with 80 ng of the digested third-round PCR product at 16°C for 16 hours using T4 DNA ligase (Roche). One μL of ligation mix was electroporated into 40 μL of *E. coli* ElectroMAX™ DH5α™ electrocompetent cells (Life Technologies), which were then plated on LB media containing 50 μg/mL kanamycin (LBK50). Plasmid DNA was isolated from cultures of individual colonies and digested with the restriction enzyme SacI and with a mixture of SphI and SacI to confirm correct vector structure.

One μL of plasmid DNA was used to transform electrocompetent cells of the previously described *E. coli* strain S17-1. The electroporated cells were plated on LB media containing 25 μg/mL of kanamycin, 25 μg/mL of streptomycin, and 25 μg/mL of spectinomycin (LBKSMST). Single colonies were used to start cultures for plasmid DNA isolation and used in conjugation. These colonies were also plated on LB media containing 5% sucrose and 25 μg/mL of kanamycin to ensure that the sacB gene was still functional. Only colonies which exhibited lethality on the sucrose media were used in conjugation. The presence of the correct insert size was confirmed by digestion of plasmid DNA with the restriction enzymes SacI and SphI.

Growing cultures of *R. sphaeroides* strain 35053 were sub-cultured, using 1/5 and 1/10 volumes of inoculum, in 5 mL Sistrom's media supplemented with 20% LB and grown at 30°C for 12 hours. The S17-1 donor colonies were grown in LBKSMST media at 37°C for 12 hours. 1.5- 3.0 mL of each culture was pelleted, and the pellets were washed four times with LB media. Relative pellet size was estimated and about 2 volumes of 35053 cells were used to 1 volume of S17-1 cells. The cell mixture was pelleted, resuspended in 20 μL of LB media, spotted on an LB plate, and incubated at 30°C for 7- 15 hours. The cells were then scraped off the surface of the plate and resuspended in 1.5 mL of Sistrom's salts. 200 μL of resuspended cells were plated on each of seven plates of SisKTell media.

Colonies that grew on the plates after about 10 days, representing proposed single-crossover events, were streaked to new plates of the same media. Upon growth, single colonies were streaked out on LBK25 media. Purified colonies were patched to Sistrom's media supplemented with 1X LB, 15% sucrose, 0.5% DMSO (v/v), and 25 μg/mL

kanamycin (SisLBK15%SucDMSO). These were grown in an anaerobic chamber (Becton Dickinson, Sparks, MD) at 30°C for 5 days to check for lethality of the sacB gene in the proposed single-crossover events. Concurrently, the cultures were patched to SisLB media containing 15% sucrose and 0.5% DMSO (v/v) without kanamycin (SisLB15%SucDMSO). Several of the cultures exhibited both white and red colonies upon growth on this media. Whitish-gray colonies were purified from these cultures and tested by PCR to show that they contained the truncated crtE allele. These colonies were also screened using primers specific to the *R. sphaeroides* ppsR gene and the *E. coli* dxs gene as described above. Potential double crossovers were also streaked on LBK25 plates to confirm that they were now sensitive to kanamycin. The resulting *R. sphaeroides* mutant containing a crtE knockout was designated ATCC 35053/ΔcrtE.

Several discoveries were made using the sacB method to knockout nucleic acid sequenced within the *R. sphaeroides* genome. First, it was discovered that the cultures used in conjugations, particularly those of the recipient *R. sphaeroides* strain, should be in exponential growth. Second, it was discovered that when using the S17-1 strain as a vector donor, the use of telluride in the plating medium is unnecessary as this strain is a proline auxotroph and will not grow on Sistrom's media without LB supplementation. Third, it was discovered that potential single crossovers should be screened using two separate PCR reactions. The first reaction should use a primer within the gene of interest together with a primer homologous to upstream sequence. The second reaction should use a primer within the gene of interest together with a primer homologous to downstream sequence. One of these two reactions should produce a truncated fragment. Fourth, it was discovered that single crossovers that have been confirmed to have sacB lethality can be grown aerobically in Sistrom's media for 2 days and then plated on SisLB 15%SucDMSO media. The volume plated varies depending on the rate of growth of the strain, but is about one µL or less for strain 35053. This is then grown anaerobically for about 5 days. Fifth, it was discovered that the sacB gene may not completely kill cells with the gene, so there may be a background level of very small colonies. The desired double-crossover colonies, however, are typically larger. These colonies should be purified and screened by PCR to identify whether they contain the truncated or full-length allele. Sixth, it was discovered that using one primer homologous to sequence upstream of the knockout gene and one primer homologous to sequence downstream of the gene is useful in confirming the correct location of the insertion event in addition to determining the allele that is present.

ATCC 35053/ΔppsR(strep)

*R. sphaeroides* cells lacking PPSR were made by inserting a spectinomycin/streptomycin resistance gene into the ppsR sequence as follows. To PCR amplify the ppsR gene from *R. sphaeroides* strain 17023, the following primers were designed based on published sequence (GenBank Accession Number L19596).

PPSRF2 5'-AGTCAGTACTAACTGGTGAAGACGCTGAAG-3' (SEQ ID NO:176)
PPSRR2 5'-GATCAGTACTGTGAACGAATACGATACGCA-3' (SEQ ID NO:177)

Each primer contained a ScaI restriction site. The ppsR gene was amplified using following reaction mix and PCR amplification program.

| Reaction Mix | | Program | | |
|---|---|---|---|---|
| pfu 10X buffer | 10 µL | 94°C | 5 minutes | |
| DMSO | 5 µL | 8 cycles | of: | |
| dNTP mix (10 mM) | 8 µL | | 94°C | 45 seconds |
| PPSRF2 (50 µM) | 2 µL | | 54°C | 45 seconds |
| PPSRR2 (50 µM) | 2 µL | | 72°C | 3 minutes |
| Genomic DNA (50 ng/µL) | 2 µL | 25 cycles of: | | |
| pfu enzyme (2.5 U/µL) | 2 µL | | 94°C | 45 seconds |
| DI water | 69 µL | | 61°C | 45 seconds |
| | | | 72°C | 3 minutes |
| Total: | 100 µL | 72°C | 10 minutes | |
| | | 4°C | Until used further | |

The PCR product was separated on a 0.8% TAE agarose gel, and a band of about 1.8 Kb was cut and gel isolated using Qiagen Gel Isolation kit (Qiagen, Valencia, CA). The gel isolated DNA was digested with ScaI (New England BioLabs, Beverly, MA) for 5 hours. The digested DNA was column purified using Qiagen Gel Isolation kit. The cut DNA was ligated into vector pSUP203 that was also digested with ScaI enzyme.

2.3 µg of pSUP203 plasmid DNA was digested for 4 hours at 37°C with ScaI restriction enzyme. The digested DNA was separated on a 1% TAE agarose gel. A 7.6 Kb fragment was excised and purified. The purified plasmid DNA was then dephosphorylated using calf intestinal phosphatase (New England Biolabs). 100 ng of dephosphorylated plasmid DNA was ligated with 200 ng of the ScaI-digested PpsR DNA for 16 hours at 14°C using T4 DNA ligase (New England BioLabs). One μL of ligation mix was electroporated into 40 μL of *E. coli* ElectroMAX™ DH5α™ (Life Technologies, Gaithersburg, MD) electrocompetent cells, which were then recovered in 1 mL of SOC media for one hour at 37°C and plated on LB media containing 15 μg/mL tetracycline. Plasmid DNA was isolated from 8 individual colonies using Qiagen spin Mini prep kit and digested with ScaI restriction enzyme to check insert size. Four of the colonies had a correct insert. 1.5 μg of the plasmid DNA obtained from confirmed colony was digested with XhoI restriction enzyme (New England BioLabs, Beverly, MA). This enzyme has a single restriction site in the open reading frame of ppsR gene. A linear DNA band of about 8.4 Kb was gel isolated using a Qiagen Gel isolation kit. A spectinomycin/streptomycin resistance omega cassette was obtained by digesting plasmid pUI1638 (Obtained from Dr. Samuel Kaplan's laboratory) with XhoI enzyme. The digest was separated on a 0.8% TAE agarose gel, and a DNA band of about 2.1 Kb was gel isolated. This DNA which encoded for spectinomycin/streptomycin resistance gene was ligated to pSUP203/PpsR, which was also restricted with XhoI enzyme. One μL of ligation mix was electroporated into 40 μL of *E. coli* ElectroMAX™ DH5α™ (Life Technologies, Gaithersburg, MD) electrocompetent cells, which were then recovered in 1 mL of SOC media for one hour at 37°C and plated on LB media with 15 μg/mL tetracycline, 25 μg/mL spectionomycin, and 25 μg/mL streptomycin. Plasmid DNA was isolated from 10 individual colonies using Qiagen spin Mini prep kit and digested separately with ScaI and XhoI restriction enzyme to check insert size. Five of the colonies had a correct insert. 100 ng of plasmid DNA from a confirmed colony was electroporated into electrocompetent cells of the *E. coli* strain SM10. This strain contains a chromosomal copy of the trans-acting elements that mobilize oriT-containing plasmids during conjugation with a second bacterial strain. It also carries a gene conferring resistance to the antibiotic kanamycin. The transformation reaction was recovered in 1 mL of SOC media for one hour and plated on LB media with 10 μg/mL tetracycline, 25 μg/mL kanamycin, 25 μg/mL of streptomycin, and 25 μg/mL spectinomycin.

The pSUP203/ppsR-SM-ST construct was conjugated from the *E. coli* SM10 host into *R. sphaeroides* strain 35053. The SM10 donor was grown in LB media with 25 μg/mL kanamycin, 25 μg/mL streptomycin, and 25 μg/mL spectinomycin at 37°C for 16 hours. A growing culture of *R. sphaeroides* strain 35053 was used to inoculate Sistrom's media in 1/5 to 1/10 dilutions. These cultures were grown for about 20 hours. Cells were pelleted for 1.5 mL of culture of both the SM10 pSUP203/PpsR-SM-ST and 35053 genotypes. Pellets were washed four times in Sistrom's media without vitamins and glucose. The pellets were each resuspended in 1.5 mL of Sistrom's media without vitamins and glucose. 200 μL of the SM10 pSUP203/PpsR-SM-ST cells were combined with 1.3 mL of the 35053 cells. This mixture was pelleted, the supernatant was removed, and the pellet was resuspended in 20 μL of LB media. The resuspended cells were spotted onto a LB plate that was then incubated at 30°C for 7 hours. The cells were then scrapped off the LB plate, resuspended in 1.5 mL of 1X Sistrom's media without vitamins and glucose, and plated (200 μL/plate) on Sistrom's media supplemented with 25 μg/mL spectinomycin, 25 μg/mL streptomycin, and 10 μg/mL of telluride. The telluride retards the growth of *E. coli* cells but is detoxified by *R. sphaeroides.* After 7-10 days, small black colonies were picked off the plates and streaked to fresh plates of the same media. After 6 days of growth, colonies were patched to LB plates containing 25 μg/mL spectinomycin and 25 μg/mL streptomycin (LBSMST25), and also to LB plates containing 0.75 μg/mL tetracycline. Desirable double-crossover events, in which the PpsR-SM-ST gene is retained in the genome and the vector DNA is lost, would have spectinomycin/streptomycin resistance but lack tetracycline resistance. Colonies resulting from undesirable single-crossover events would demonstrate resistance to all of these antibiotic markers.

Colonies that exhibited only spectinomycin/streptomycin resistance and displayed deep red color were confirmed for double-crossover by Southern hybridization. Southern hybridization was conducted on nineteen potential 35053/PpsR-SM-ST colonies in addition to 35053 and *R. sphaeroides* strain 17023. Sequence data for the photosynthetic operon of 17023 is available in Genbank and was used to determine restriction enzymes likely to have hybridization patterns that would distinguish mutants from non-mutants. Genomic DNA was isolated from each line using a Gentra Puregene DNA Isolation Kit (Gentra, Minneapolis, MN). 2 μg of genomic DNA was used in digests using the restriction enzymes NcoI, ApaI, and XmaI in separate reactions. The digests were separated on a 1% TAE agarose gel, and the DNA was transferred to nylon membrane (Roche Molecular Biochemicals, Indianapolis, IN). DIG-labeled molecular weight markers II and III (Roche) were also included on the gel/membrane. DIG-labeled probes of the PpsR locus were made using a PCR DIG Probe Synthesis Kit (Roche). After baking, membranes were prehybridized in EasyHyb Buffer (Roche) for at least 2 hours and hybridized overnight using 400 nL of a 0.5 DIG labeling per mL of hybridization solution. Detection was done using a Roche Wash and Block Buffer Set (Roche). Membranes were washed two times for 5-10 minutes at room temperature in 2X SSC/0.1% SDS and two times for 15-20 minutes at 68°C in 0.1X SSC/0.1% SDS. They were then covered with blocking buffer and placed on a shaker for an hour at room temperature. The blocking buffer was replaced with fresh blocking buffer containing 150 mU of AP conjugate per mL of buffer, and the membranes shaken at room temperature for an additional 30 minutes. Membranes were then washed twice for 15 minutes at room temperature with washing buffer, followed by a five minutes wash with detection buffer. The detection buffer was replaced with fresh detection buffer containing 20 μL of NBT/BCIP solution per mL of buffer. This was placed in the dark at room temperature with no shaking until sufficient color was developed.

In the NcoI digest, the lanes of colony 9 and 10 exhibited a band about 2 Kb larger than the 35053 control, which is the size difference expected from the insertion of the spectinomycin/streptomycin resistance cassette into the XhoI site For the XmaI digest, 35053 exhibited a single band about 5.5 Kb, while colonies 9, 10, and 5 exhibited two bands whose summed size was about 2 Kb higher than that of 35053. Two bands were observed in colony 9, 10, and 5 because a XmaI was introduced along with the spectinomycin/streptomycin resistance cassette. For ApaI digest, the control 35053 sample exhibited two bands since ppsR gene harbors an ApaI site. Each of these bands was about 2.3 Kb in size. Colony 9, 10, and 5 exhibited three bands, whose summed size was about 2 Kb higher band that of 35053. An extra band was observed in colonies 9, 10, and 5 because an ApaI site was introduced along with the spectinomycin/streptomycin resistance cassette.

The resulting *R. sphaeroides* mutant containing the ppsR knockout was designated ATCC 35053/ΔppsR(strep).

ATCC 35053/ΔppsR

*R. sphaeroides* cells lacking ppsR were made using sacB selection as follows. A three-step PCR process was used to create a 255 bp in-frame deletion in the PpsR gene, so that there would be no residual antibiotic resistance gene in the genome. The PpsR gene from *R. sphaeroides* strain 35053 was amplified by PCR using primers designed to introduce an SacI restriction site at the beginning of the amplified fragment and a SphI restriction site at the end of the amplified fragment. The sequences of the primers were as follows.

**PPSRSACF2 5'-GTCAAATGAGCTCCAAACTGGTGAAGA-CGCTGAAGGACAT-3' (SEQ ID NO:178)**

**PPSRSPHR 5'-CAGTCGGGCATGCGTCCATTTCAGTTGAC-ATACTTCTGTG-3' (SEQ ID NO:179)**

The following PCR mix program was used to amplify the PpsR gene.

| Reaction Mix | | Program | | |
|---|---|---|---|---|
| pfu 10X buffer | 10 μL | 94°C | 2 minutes | |
| DMSO | 5 μL, | 8 cycles of: | | |
| dNTP mix (10 mM) | 3 μL | 94°C | 30 seconds | |
| PPSRSACF2 (100 μM | 1 μL | 58°C | 45 seconds | |
| PPSRSPHR (100 μM | 1 μL | 72°C | 3 minutes | |
| Genomic DNA (50 ng/μL) | 2 μL | 25 cycles of: | | |
| pfu enzyme (2.5 U/μL) | 2 μL | | 94°C | 30 seconds |
| DI water | 76 μL | | 64°C | 45 seconds |
| | | | 72°C | 3 minutes |
| Total: | 100 μL | 72°C | 7 minutes | |
| | | 4°C | Until used further | |

100 μL of PCR product was separated on a 1% TAE agarose gel, and a fragment about 1.8 Kb was excised and purified using Qiagen Gel isolation kit.

The second round of PCR consisted of two separate reactions: reaction A, which used primers PPSRSACF2 and PPSRMIDR, and reaction B, which used primers PPSRSPHR and PPSRMIDF. The sequences of primers PPSRMIDF and PPSRMIDR were as follows.

**PPSRMIDF 5'-CTCTTGCTCGGCGGCGTGCGGCTCTATCA-CGAGGGGGTGGA-3' (SEQ ID NO:180)**

PPSRMIDR 5'-TCCACCCCCTCGTGATAGAGCCGCACGCC-GCCGAGCAAGAG-3' (SEQ ID NO:181)

The 20 nucleotides on the 3' ends of this pair of primers are located near the center of the ppsR gene, 255 bases apart from each other, and facing towards the start (PPSRMmR) and end (PPSRMIDF) of the gene. The 20 bp on the 5' ends of these primers are the reverse complement of the 3' end of the other primer in the pair. The following reaction mix and program were used to conduct these PCR.

| Reaction Mix A | | Program | | |
|---|---|---|---|---|
| pfu 10X buffer | 10 µL | 94°C | 2 minutes | |
| DMSO | 5 µL | 8 cycles of: | | |
| dNTP mix (10 mM) | 3 µL | | 94°C | 30 seconds |
| PPSRSACF2 (100 µM) | 1 µL | | 58°C | 45 seconds |
| PPSRMIDR (100 µM) | 1 µL | | 72°C | 3 minutes |
| DNA from first round | 1 µL | 25 cycles of: | | |
| (10 ng/µL) | | | 94°C | 30 seconds |
| pfu enzyme (2.5 U/µL) | 2 µL | | 64°C 72°C | 45 seconds 3 minutes |
| DI water | 77 µL | 72°C | 7 minutes | |
| Total: | 100 µL | 4°C | Until further use | |

| Reaction Mix B | | Program | |
|---|---|---|---|
| pfu 10X buffer | 10 µL | 94°C 2 minutes | |
| DMSO | 5 µL | 8 cycles of: | |
| dNTP mix (10 mM) | 2 µL | 94°C | 30 seconds |
| PPSRSPHR (100 µM) | 1 µL | 58°C | 45 seconds |
| PPSRMIDF (100 µM | 1 µL | 72°C | 3 minutes |
| DNA from first round | 1 µL | 25 cycles of: | |
| (5ng/µL) | | 94°C | 30 seconds |
| pfu enzyme (2.5 U/µL) | 2 µL | 64°C | 45 seconds |
| $D_1$ water | 78 µL | 72°C | 3 minutes |
| | | 72°C | 7 minutes |
| Total: | 100 µL | 4°C Until | further use |

Both PCR products, about 800-700 bp in length, were separated on a 1% TAE agarose gel, excised, and gel purified using a Qiagen gel isolation kit.

The third round of PCR used primers PPSRSACF2 and PPSRSPHR but used both fragments derived in the second round of PCR as template. The PCR mixture used was the same as in the first round of PCR except that equal molar amounts of the round 2 fragments were used as template. The PCR program used was also the same as that used in the first round of PCR, with the annealing time lengthened to 1.5 minutes. The 1.5 Kb third-round product was separated on a 1% TAE agarose gel and purified using Qiagen gel isolation kit. The purified DNA was digested overnight at 37°C with the restriction enzymes SacI and SphI.

Three µg of the vector pL01 was digested with the restriction enzymes SphI and SacI at 37°C for 16 hours. The enzymes were inactivated by heating to 65°C for 20 minutes. Dephosphorylation of the vector was achieved by adding 4.7 µL of shrimp alkaline phosphatase 10X buffer (Roche) and 2 µL of shrimp alkaline phosphatase to the inactivated digest. This mixture was heated at 37°C for 10 minutes and then 65°C for 15 minutes. The dephosphorylated vector DNA was then gel purified on a 1.0% TAE agarose gel.

98 ng of vector DNA was ligated with 210 ng of the digested third round PCR at 14°C for 14 hours using T4 DNA ligase (Roche). One µL of ligation mix was electroporated into 40 µL of *E. coli* ElectroMAX™ DH5α™ electrocompetent cells (Life Technologies), which were then recovered in 1 mL of SOC media for one hour and plated on LB media with 25 µg/mL kanamycin (LBK25). Plasmid DNA was isolated from eight individual colonies. Plasmid DNA was checked for correct insert with a PCR screen using the PCR protocol from first round.

One μL of plasmid DNA was used to transform electrocompetent cells of *E. coli* strain S17-1. The electroporated cells were recovered in 1 mL of SOC media for one hour and plated on LB media with 25 μg/mL of kanamycin, 25 μg/mL of streptomycin, and 25 μg/mL of spectinomycin (LBKSMST). Single colonies were used to start cultures for plasmid DNA isolation and used in conjugation. These colonies were also plated on LB media containing 5% or 15% sucrose, and 25 μg/mL of kanamycin to ensure that the sacB gene was still functional. Only colonies that showed lethality on the sucrose media were used in conjugation. The presence of the correct insert size was confirmed by colony PCR.

Growing cultures of *R. sphaeroides* strain 35053 were subcultured, using 1/4 and 1/8 volumes of inoculum, in 5 mL Sistrom's media supplemented with 20% LB and grown at 30°C for 9 hours. The S17-1 donor colonies were grown in LBKSMST media at 37°C for 16 hours. 3.0 mL of 35053 and 0.5 mL of S17-1 donor cells were centrifuged and washed four times in Sistrom's media without glucose. Each cell pellet was resuspended into 20 μL LB, and the S17-1 donor suspension was mixed with 35053. The mixture was then spotted on LB, which was incubated at 30°C for 14-16 hours. The cells were then scraped off the surface of the plate and resuspended in 1.5 mL of Sistrom's salts. 200 μL of resuspended cells were plated on each of the seven Sistrom's media plates that were supplemented with 25 μg/mL of kanamycin.

Colonies that grew on the plates after about 10-14 days, representing proposed single crossover events, were streaked to new plates of the same media. Upon growth, single colonies were transferred to LBK25 media. These cultures were grown for 36 to 48 hours in Sistrom's media supplemented with 20% LB and no kanamycin at 30°C. 0.1 μL and 5 μL of this culture was plated on LB media that was supplemented with Sistrom's salts and 15% sucrose. The plates were placed in an anaerobic chamber (Becton Dickinson, Sparks, MD), and the chamber was placed in a 30°C incubator. After 4-5 days, several colonies showed up on the plates, indicating the occurrence of double-crossover events. Four colonies from each single-crossover strain were purified by streaking on LB agar plates. Single colonies of double-crossover strains were screen by PCR for integration of truncated version of the ppsR gene into the chromosome. For screening, the following primers were used, which were located upstream and downstream of the PpsR gene. The use of upstream and downstream primer confirms both the locus of integration as well as truncation of PpsR gene.

PPSRUPF 5'-GAGCAGCACACTCTGGGAGC-3' (SEQ ID NO:182)
PPSRDNR 5'-CCACACAGGTAGGACACCCAC-3' (SEQ ID NO:183)

The following reaction mix and PCR program was used.

| Reaction Mix | | Program | |
|---|---|---|---|
| Taq Mg+ 10X buffer | 2.5 μL | 94°C | 2 minutes |
| DMSO | 1.25 μL | 29 cycles of: | |
| dNTP mix (10 mM) | 0.5 μL | 94°C | 30 seconds |
| PPSRUPF (100 μM) | 0.125 μL | | 61°C 45 seconds |
| PPSRDNR (100 μM) | 0.125 μL | | 72°C 3 minutes |
| Cell boil mix | 2 μL | 72°C | 7 minutes |
| Taq enzyme (5 U/μL) | 0.2 μL | 4°C | Until further use |
| DI water | 18.3 μL | | |
| Total: | 25 μL | | |

The cell boil mix was prepared by resuspending a single colony in 20-25 μL of water. The suspension was heated at 95°C for 10 minutes in a PCR machine. The tube was given a quick spin to pellet the solids.

The colonies that exhibited the truncated version of the PpsR gene were further tested for kanamycin sensitivity by streaking them on LB plates that were supplemented with 25 μg/mL of kanamycin. Also, these colonies were PCR screened for the kanamycin resistance gene.

The resulting *R. sphaeroides* mutant containing the ppsR knockout was designated ATCC 35053/ΔppsR.

ATCC 35053/ΔccoN

*R. sphaeroides* cells lacking ccoN were made using sacB selection as follows. A mutant of *R. sphaeroides* strain 2.4.1 having a 546 bp deletion in the ccoN gene *(R. sphaeroides* 2.4.1/ΔccoN) was obtained from the laboratory of Samuel Kaplan at the University of Texas (Oh and Kaplan, Biochemistry, 38:2688-2696 (1999)). The mutated ccoN locus of this strain was amplified by PCR and cloned into pL01. This plasmid was transformed into *E. coli* strain S17-1. The S17-1 strain was conjugated with *R. sphaeroides* strain 35053, and colonies were identified in which the truncated locus had replaced the native ccoN gene.

The truncated ccoN gene from *R. sphaeroides* 2.4.1/ΔccoN was amplified by PCR using primers designed to introduce a SacI restriction site at the beginning of the amplified fragment and a SphI restriction site at the end of the amplified fragment. The sequences of the primers were as follows.

CCONSACF 5'-TCAGAGCTCGTGTGATCGAATGGGGCTTT-GTTCCTTGATG-3' (SEQ ID NO:184)

CCONSPHR 5'-GAAGCATGCAGGTGATCGACGTGCCACTC-GTCCGAATAG-3' (SEQ ID NO:185)

The PCR reaction mix contained 0.2 μM each primer, 1X Native Pfu reaction buffer, 0.2 mM each dNTP, 5% DMSO, and 10 units of Pfu DNA polymerase in a 200 μL reaction. Three μL of the glycerol stock was diluted in 20 μL of 10 mM Tris and heated at 94°C for 10 minutes, after which 4 μL was added to the PCR reaction. The PCR was conducted in a MJ Research PT100 and consisted of an initial denaturation at 94°C for 2 minutes; 32 cycles of a 30 second denaturation at 94°C, a 1 minute annealing at 66°C, and a 4 minute extension at 72°C, followed by a final extension at 72°C for 7 minutes. The PCR product was separated on a 1% TAE-agarose gel, and a 1.6 Kb fragment was excised and purified. Three μg of purified PCR product was digested with SacI restriction enzyme and separated on a 1% TAE gel. A 1.4 Kb band was excised and purified. A SacI restriction site exists about 200 bp from the CCONSPHR end of the original PCR product.

Three μg of the vector pL01 was digested with the restriction enzyme SacI. The enzyme was inactivated by heating to 65°C for 20 minutes, and the digested vector was dephosphorylated using shrimp alkaline phosphatase. The dephosphorylated vector DNA was gel purified on a 1% TAE-agarose gel.

50 ng of digested vector DNA was ligated with 65 ng of the digested ccoN PCR product at 16°C for 16 hours using T4 DNA ligase (Roche). One μL of ligation mix was electroporated into 40 μL of *E. coli* Electromax™ DH5α™ electrocompetent cells, which were then plated on LBK media. Plasmid DNA was isolated from cultures of individual colonies and digested with the restriction enzyme SacI to confirm correct insert size.

The *E. coli* strain S17-1 contains a chromosomal copy of the trans-acting elements that mobilize oriT-containing plasmids during conjugation with a second bacterial strain. It also carries genes conferring resistance to the antibiotics streptomycin and spectinomycin. In addition, S17-1 is a proline auxotroph and will not grow on unsupplemented Sistrom's media. One μL of DNA of the truncated ccoN construct was used to transform electrocompetent cells of *E. coli* strain S17-1. The electroporation was plated on LBKSMST. Single colonies were used to start cultures for plasmid DNA isolation and used in conjugation. These colonies were also plated on LB media containing 5% sucrose and 25 μg/mL of kanamycin to ensure that the sacB gene was still functional. Only colonies that exhibited lethality on the sucrose media were used in conjugation. The presence of the correct insert size was confirmed by digestion of plasmid DNA with the restriction enzyme SacI.

Growing-cultures of *R. sphaeroides* strain 35053 were subcultured in Sistrom's media supplemented with 20% LB to ensure that they were in exponential growth. The S17-1 donor colonies were grown in LBKSMST media at 37°C overnight or subcultured from growing colonies. 2-4 mL of each culture was centrifuged, and the pellets were washed four times in LB media. Relative pellet size was estimated, and about 2 volumes of 35053 cells were used to 1 volume of S17-1 cells. The cell mixture was then pelleted, resuspended in 20 μL of LB media, and spotted on an LB plate. This plate was incubated at 30°C for 7- 15 hours. The cells were then scraped off the surface of the plate and resuspended in 1.2 mL of Sistrom's salts. 200 μL of resuspended cells were plated on each of six plates of Sistrom's media containing 25 μg/mL of kanamycin (SisK).

Colonies that grew on the plates after about 10 days, representing potential single-crossover events, were streaked to new plates of SisK media. Upon growth, single colonies were transferred to LBK media. Purified colonies were streaked to Sistrom's media supplemented with 1X LB, 15% sucrose, 0.5% DMSO (v/v), and 25 μg/mL kanamycin (SisLBK15%SucDMSO). These were grown in an anaerobic chamber (Becton Dickinson, Sparks, MD) at 30°C for 5 days to check for lethality of the sacB gene in the single-crossover events. The purified colonies were also screened in two separate PCR reactions. The first reaction used a primer within the gene of interest (CCONR) together with a primer homologous to upstream sequence (CCONUPF2), and the second reaction used a primer within the gene of interest (CCONSACF) together with a primer homologous to downstream sequence (CCONDNR2). Single-crossover events exhibited a truncated fragment in one of the two reactions, depending on whether the crossover occurred upstream or downstream of the deletion. The primer sequences were as follows.

CCONUPF2 5'-CTCACAACCTCCAACCGATG-3' (SEQ ID NO:186)
CCONR 5'-CGATGGTGACCACGAAGAAG-3' (SEQ ID NO:94)
CCONDNR2 5'-CGTAACGCTCGGTCTCGTC-3' (SEQ ID NO:129)

Single-crossover colonies were grown in Sistrom's media supplemented with 20% LB. After 2 days of growth, 0.1-1 μL of the cultures was plated on Sistrom's media supplemented with 1X LB, 0.5% DMSO (v/v), and 15% sucrose (SisLB15%SucDMSO). These cultures were grown anaerobically for about 5 days. The sacB gene did not always completely kill cells with the gene, so there was often a background level of very small colonies. The larger colonies, which represented double-crossover events, were purified on LB media and screened by PCR to identify whether they contained the truncated or full-length allele. The CCONUPF2 and CCONDNR2 primers were used in this PCR screen to ensure that the truncated gene also was inserted in the correct location in the genome. Potential double-crossovers were also streaked on LBK plates to confirm that they were now sensitive to kanamycin.

The resulting *R. sphaeroides* mutant containing the ccoN knockout was designated ATCC 35053/ΔccoN.

ATCC 35053/ΔcrtE/ΔccoN

*R. sphaeroides* cells lacking crtE and ccoN were made as follows. The wildtype ccoN allele of a crtE knockout mutant (ATCC 35053/ΔcrtE) was replaced with a truncated ccoN allele as described above. Double-crossover colonies having the truncated ccoN allele were then re-screened by PCR for the crtE and ccoN loci. These colonies were plated on LBK25 and screened by PCR to confirm the loss of the vector from the genome. The resulting *R. sphaeroides* mutant containing the crtE knockout and ccoN knockout was designated ATCC 35053/ΔcrtE/AccoN.

ATCC 35053/ΔcrtE/ΔppsR/ΔccoN

*R. sphaeroides* cells lacking crtE, ppsR, and ccoN were made as follows. The wildtype ppsR allele of a crtE/ccoN knockout mutant (ATCC 35053/ΔcrtE/ΔccoN) was replaced with a truncated ppsR allele as described above with the following exceptions. After conjugation on an LB plate, the conjugated cells were plated on Sistrom's media containing 25 μg/mL of kanamycin and 0.5% DMSO (SisKDMSO) rather than on SisK. After purification on SisKDMSO and LBKDMSO, single-crossovers were grown aerobically in Sistrom's media supplemented with 1X LB and 0.5% DMSO. After 2 days of growth, the cultures were plated on Sistrom's media supplemented with 1X LB, 15% sucrose, and 0.5% DMSO, and grown anaerobically for 5 days. Potential double-crossover colonies were purified on LBDMSO and screened by PCR using the PPSRUPF and PPSRDNR primers. Colonies having the truncated ppsR allele were then rescreened by PCR for the crtE, ppsR, and ccoN loci. These colonies were also plated on LBKDMSO and screened by PCR to confirm the loss of the vector from the genome. The resulting *R. sphaeroides* mutant containing the crtE knockout, ppsR knockout, and ccoN knockout was designated ATCC 35053/ΔcrtE/ΔppsR/ΔccoN.

Example 10 - Making recombinant microorganisms that overexpress a particular sequence while a containing knock-out

Any construct developed for the overexpression of genes are transferred to any of the background genotypes developed by gene knockout techniques. For example, the pMCS2tetP/Stdxs/Rsdds/EcUbiC or the pMCS2tetP/Stdxs/Rsdds/RsLytB construct is transferred into the *R. sphaeroides* ATCC 35053/ΔcrtE/ΔppsR/ΔccoN mutant cells to combine the productive effects of gene overexpression and engineering of gene regulation or carbon flow. The construct is transfered to the desired genotype by electroporation or conjugation. Conjugation of a plasmid into an *R. sphaeroides* strain follows the procedure described for the isolation of single-crossover events except that, since the efficiency of plasmid transfer is much higher than that of chromosomal integration, a 0.1-1 μL plating volume from the ~400 μL conjugation recovery is ample to obtain transformed colonies. Single colony PCR is used to check the integrity of the construct in the new background, and evaluations of the productivity of the new strain are made. Genes that are productive are integrated, in one or more copies, into appropriate regions of the chromosome of a productive strain along with or downstream of a highly-expressing promoter.

Example 11 - Three liter fermentations

Cultures of *R. sphaeroides* ATCC 35053 with various inserted genes or knockouts were grown in 5 mL culture tubes containing Sistrom's media with 4 g/L glucose. After 48 hours of growth at 30°C with 250 rpm shaking, the entire contents of the tube were used to inoculate a 300 mL baffled shake flask containing Sistrom's media with 4 g/L glucose. After incubation at 30°C for 48 hours, the entire contents of the flask were added to 2.7 L of Sistrom's media containing 40 g/L glucose in a B. Braun Biotech International Model Biostat B fermenter.

The fermenter was maintained at 30°C, and the cascade was set to maintain the dissolved oxygen (DO) at 40%. The air inflow was maintained at 1 vvm, and the pH was maintained at 7.3 with an automatic feed of 2N $NH_4OH$. Foaming was controlled by addition of Sigma Antifoam 289. Kanamycin to a concentration of 50 μg/mL was added to fermentations with strains containing the broad host range vector pBBRIMCS2 either with or without an inserted gene. At 24 to 30 hours, when the agitation increase to maintain a DO of 40% had leveled off, the agitation and DO were decoupled, and the agitation was fixed at 240 rpm. The air inflow was lowered to 0.3 vvm. Kanamycin to 50 μg/mL was again added to fermentations containing the expression vector.

The fermentation samples for coenzyme Q10 and spheroidenone analysis were removed at 69 to 75 hours into the fermentation.

Example 12 - Three-hundred milliliter fermentations

Cultures of *R. sphaeroides* ATCC 35053 with various overexpressed genes or knockouts were grown in 5 mL culture tubes containing Sistrom's media with 4 g/L glucose. After 48 hours of growth at 30°C with 250 rpm shaking, the entire contents of the tube were used to inoculate a 300 mL baffled shake flask containing Sistrom's media with 4 g/L glucose. After incubation at 30°C for 48 hours, 30 mL of the flask were added to 270 mL of Sistrom's media containing 40 g/L glucose in a 500 mL Infors AG-CH-4103 fermenter.

The fermenter was maintained at 30°C, and the cascade was set to maintain the dissolved oxygen (DO) at 40%. The air inflow was maintained at 1 vvm, and the pH was maintained at 7.3 with an automatic feed of 2N $NH_4OH$. Foaming was controlled by addition of Sigma Antifoam 289. Kanamycin to a concentration of 50 μg/mL was added to fermentations with strains containing the broad host range vector pBBRIMCS2 either with or without an inserted gene. At 24 to 30 hours, when the agitation increase to maintain a DO of 40% had leveled off, the agitation and DO were decoupled, and the agitation was fixed at 400 rpm. The air inflow was lowered to 0.3 vvm. Kanamycin to 50 μg/mL was again added to fermentations containing the expression vector.

The fermentation samples for coenzyme Q 10 and spheroidenone analysis were removed at 69 to 75 hours into the fermentation.

Comparative Example 13 - Analysis of Spheroidenone

At various times during the fermentation, 15 mL of fermentation volume was withdrawn. The volume of sample needed to obtain 5 mg of dry cell weight (DCW) was used for spheroidenone analysis. The sample was washed one time in water and resuspended in an equal volume of water. The volume of sample calculated in step 1 was added to a 1.8 mL-microfuge tube and was centrifuged at 10,000 rpm for 3 minutes in an IEC MicroMax microfuge. The supernatant was removed, and the pellet was completely resuspended in 1.0 mL of Acetone:Methanol (7:2) and stored at room temperature away from light for 30 minutes. The sample was mixed once during this incubation. After incubation, the sample was centrifuged at 10,000 rpm for 3 minutes, and the extract (supernatant) collected. Samples were stored -20°C for analysis at a later time. The carotenoid extract was analyzed on a spectrophotometer scanning in the range of 350 nm to 800 nm, and the $OD_{480}$ was recorded. The amount of carotenoid in mg/100 mL of culture was calculated using the following equation:

$$\text{Spheroidenone (mg) / 100 mL culture} = ((OD_{480} - (0.0816 * OD_{770})) * 0.484) / \text{Vol. of sample from step 1}$$

From mg of Spheroidenone/100 mL of culture, the amount of Spheroidenone/mg of dry cell weight (DCW) was calculated using the DCW number as the conversion factor. Care was taken to correct for any dilution factor required while the sample was scanned on the spectrophotometer.

Example 14 - Analyzing CoQ(10) levels produced via fermentation

100 mL of fermentation broth was removed once per day and placed in a tared 250 mL centrifuge bottle. The samples were centrifuged at 15,000 X g for 5 minutes, the supernatant was poured off, and the samples were resuspended in 50 mL cold water. The samples were centrifuged again at 15,000 X g for 5 minutes, and the supernatant was poured off. The wet weight of the biomass was determined, and the biomass was resuspended in 1.5 times its weight in water. The samples were stored covered with foil at -80°C before analysis.

Before analysis, the samples were warmed at 21°C for 15 minutes. 1.0 mL was withdrawn. Sodium dodecyl sulfate was added to a final concentration of 1.67 %. The samples were extracted with 14 mL of a hexane:ethanol (5:

2) mixture. The samples were then evaporated to dryness and dissolved in 2 mL of a methanol:ethanol (9:2) mixture. The samples were then analyzed on a Waters Nova-Pak C18 (3.9 x 150 mm: 4 Um) column with a PDA detector set from 200-300 nm. Resolution was at 1.2 nm with a maximum absorbance at 275 nm. The run time was 15 minutes, and the injection volume was 20 μL.

The dry weight of the samples were determined drying an aliquot at 105°C in an aluminum weighing pan for at least four hours.

Example 15 - Production of CoQ(10)

The following seven experiments measured the amount of CoQ(10) produced by the indicated microorganisms in a 3 liter scale fermentation.

In experiment 1, the following data were collected after 96 hours of fermentation:

| Strain | Coenzyme Q10 (ppm) dry weight basis |
|---|---|
| ATCC 35053 | 2950 |
| ATCC 35053/ΔcrtE | 6508 |

These results demonstrated that the inactivation of crtE increased the production of CoQ(10).

In experiment 2, the following data were collected after 69 to 75 hours of fermentation:

| Strain | Coenzyme Q10 (ppm) dry weight basis |
|---|---|
| ATCC 35053 | 1655 |
| ATCC 35053/ΔppsR(strep) | 3812 |

These results demonstrated that the inactivation of ppsR increased the production of CoQ(10).

In experiment 3, the following data were collected after 69 to 75 hours of fermentation:

| Strain | Coenzyme Q10 (ppm) dry weight basis | Spheroidenone (ppm) dry weight basis |
|---|---|---|
| ATCC 35053 | 2951 | 1980 |
| ATCC 35053/ΔccoN | 3527 | 2959 |

These results demonstrated that the inactivation of ccoN increased the production of CoQ(10) and spheroidenone.

In experiment 4, the following data were collected after 69 to 75 hours of fermentation:

| Strain | Coenzyme Q10 (ppm) dry weight basis |
|---|---|
| ATCC 35053/ΔcrtE | 3255 |
| ATCC 35053/ΔcrfE/ΔccoN isolate 8-7 | 7951 |

These results demonstrated that the inactivation of crtE and ccoN increased the production of CoQ(10) as compared to inactivating crtE only.

In experiment 5, the following data were collected after 69 to 75 hours of fermentation:

| Strain | Coenzyme Q10 (ppm) dry weight basis |
|---|---|
| ATCC 35053/ΔcrtE | 3545 |
| ATCC 35053/ΔcrtE/ΔccoN isolate 111 | 4984 |
| ATCC 35053/ΔcrtE/ΔppsR/ΔccoN | 11,676 |

These results demonstrated that the inactivation of crtE and ccoN increased the production of CoQ(10) as compared to inactivating crtE only. In addition, these results demonstrated that the inactivation of crtE, ccoN, and ppsR increased the production of CoQ(10) as compared to inactivating only crtE and cooN.

In experiment 6, the following data were collected after 69 to 75 hours of fermentation:

| Strain | Coenzyme Q10 (ppm) dry weight basis |
|---|---|
| ATCC 35053/ΔcrtE | 3833 |
| ATCC 35053/ΔcrtE/pMCS2tetP/Stdxs | 4928 |
| ATCC 35053/ΔcrtE/pMCS2glnP/Stdxs | 5508 |
| ATCC 35053/ΔcrtE/pMCS2tetP/Stdds | 4652 |

These results demonstrated that the inactivation of crtE together with the addition of Stdxs increased the production of CoQ(10) as compared to inactivating crtE only. In addition, these results demonstrated that the use of the gln promoter with Stdxs resulted in more production of CoQ(10) when compared to the use of the tet promoter with Stdxs. Further, these results demonstrated that the inactivation of crtE together with the addition of Stdds increased the production of CoQ(10) as compared to inactivating crtE only.

In experiment 7, the following data were collected after 69 to 75 hours of fermentation:

| Strain | CoQ(10) (ppm) dry weight basis |
|---|---|
| ATCC 35053/pMCS2tetP | 3909 |
| ATCC 35053/pMCS2tetP/Stdxs/Rsdds | 5387 |
| ATCC 35053/pMCS2tetP/Stdxs/Rsdds/RsLytB | 5962 |
| ATCC 35053/pMCS2tetP/Stdxs/Rsdds/EcUbiC | 6439 |

These results demonstrated that the addition of Stdxs and Rsdds increased the production of CoQ(10) as compared to adding vector only. In addition, these results demonstrated that the addition of either RsLytB or EcUbiC together with the addition of Stdxs and Rsdds increased the production of CoQ(10) as compared to adding only Stdxs and Rsdds.

The following four experiments measured the amount of CoQ(10) produced by the indicated microorganisms in a 300 mL scale fermentation.

In experiment 1, the following data were collected after 69 to 75 hours of fermentation:

| Strain | CoQ(10) (ppm) dry weight basis |
|---|---|
| ATCC 35053/pMCS2tetP | 5250 |
| ATCC 35053/pMCS2tetP/Stdxs | 5758 |
| ATCC 35053/pMCS2tetP/Rsdds | 6944 |
| ATCC 35053/pMCS2tetP/Stdxs/Rsdds | 6875 |
| ATCC 35053/pMCS2tetP/Stdxs/Rsdds/EcUbiC | 7808 |

These results demonstrated that the addition of either Stdxs or Rsdds increased the production of CoQ(10) as compared to adding vector only. In addition, these results demonstrated that the addition of Stdxs, Rsdds, and EcUbiC increased the production of CoQ(10) as compared to adding only Stdxs and Rsdds.

In experiment 2, the following data were collected after 69 to 75 hours of fermentation:

| Strain | CoQ(10) (ppm) dry weight basis |
|---|---|
| ATCC 35053/pMCS2tetP | 5483 |
| ATCC 35053/pMCS2tetP/EcubiC | 6360 |
| ATCC 35053/pMCS2tetP/RsLytB | 5976 |
| ATCC 35053/pMCS2tetP/Stdxs/Rsdds/RsLytB | 6751 |

These results demonstrated that the addition of either EcUbiC or RsLytB increased the production of CoQ(10) as compared to adding vector only. In addition, these results demonstrated that the addition of Stdxs, Rsdds, and RsLytB increased the production of CoQ(10) as compared to adding only RsLytB.

In experiment 3, the following data were collected after 69 to 75 hours of fermentation:

| Strain | CoQ(10) (ppm) dry weight basis |
|---|---|
| ATCC 35053/pMCS2tetP | 5072 |
| ATCC 35053/pMCS2tetP/Stdxs/Rsdds/RsLytB | 8050 |

These results demonstrated that the addition of Stdxs, Rsdds, and RsLytB increased the production of CoQ(10) as compared to adding vector only.

In experiment 4, the following data were collected after 69 to 75 hours of fermentation:

| Strain | Coenzyme Q10 (ppm) dry weight basis |
|---|---|
| ATCC 35053/pMCS2tetP | 4503 |
| ATCC 35053/pMCS2tetP/Stdxs/Rsdds | 8833 |

These results demonstrated that the addition of Stdxs and Rsdds increased the production of CoQ(10) as compared to adding vector only.

It is to be understood that while the invention has been described in conjunction with the detailed description thereof, the foregoing description is intended to illustrate and not limit the scope of the invention, which is defined by the scope of the appended claims.

## Claims

1. A host cell comprising a genomic deletion and an exogenous nucleic acid, wherein said deletion comprises at least a portion of a ppsR sequence or cbb3 sequence,
wherein said host cell comprises a non-functional ppsR sequence or cbb3 sequence, and wherein said exogenous nucleic acid encodes a polypeptide having decaprenyl diphosphate synthase (DDS)-deoxyxylulose-5-phosphate synthase (DXS) or chorismate lyase activity.

2. The host cell of claim 1, wherein said host cell is *Rhodobacter.*

3. The host cell of claim 1, wherein said host cell comprises an exogenous nucleic acid comprising an UbiC sequence and/or LytB sequence.

4. The host cell of claim 1, wherein said cbb3 sequence is a ccoN sequence.

5. The host cell of claim 1, wherein said host cell comprises a non-functional crtE sequence and a non-functional cbb3 sequence, or a non-functional crtE sequence and a non-functional ppsR sequence, or a non-functional crtE sequence, ppsR sequence, and cbb3 sequence.

6. The host cell of any of claims 1-5, wherein said host cell is a membranous bacterium or a highly membranous bacterium.

7. The host cell of claim 6, wherein said membranous bacterium is from a genus selected from the group consisting of *Rhodobacter, Rhodospirillum, Rhodopseudomonas, Rhodomicrobium, Rhodocyclus, Rhodopila,* and *Methylomonas;* in particular wherein said membranous bacterium is *Rhodobacter sphaeroides, Rhodobacter capsulatus, Rhodobacter sulfidophilus, Rhodobacter adriaticus,* or *Rhodobacter veldkampii;* or wherein said membranous bacterium is *Rhodospirillum rubrum, Rhodospirillum photometricum, Rhodospirillum molischianum, Rhodospirillum fulvum,* or *Rhodospirillum salinarum.*

8. The host cell of any of claims 1-7, wherein said exogenous nucleic acid encodes a polypeptide having DXS activity.

9. The host cell of any of claims 1-7, wherein said exogenous nucleic acid encodes a polypeptide having DDS activity.

10. The host cell of any of claims 1-7, wherein said exogenous nucleic acid comprises at least one exogenous nucleic acid encoding a polypeptide having DXS activity and a polypeptide having DDS activity.

11. The host cell of any of claims 1-7, wherein said exogenous nucleic acid comprises at least one exogenous nucleic acid encoding a polypeptide having DXS activity, a polypeptide having DDS activity, and a polypeptide having ubiC activity.

12. A method for making CoQ(10), comprising culturing a host cell under conditions wherein said host cell produces CoQ(10),
wherein said host cell comprises an exogenous nucleic acid, wherein said exogenous nucleic acid encodes a polypeptide having DDS, DXS, or chorismate lyase activity; and comprises a genomic deletion, said deletion comprising at least a portion of a ppsR sequence or cbb3 sequence, and wherein said host cell comprises a non-functional ppsR sequence, or cbb3 sequence; and isolating CoQ(10).

13. The method of claim 12, wherein said cell is *Rhodobacter.*

14. The method of claim 12, wherein said polypeptide is a UbiC polypeptide or a LytB polypeptide.

15. The method of claim 12, wherein said cbb3 sequence is a ccoN sequence.

16. The method of any of claims 12-15, wherein said host cell comprises a non-functional crtE sequence and a non-functional cbb3 sequence, or a non-functional crtE sequence and a non-functional ppsR sequence, or a non-functional crtE sequence, ppsR sequence, and cbb3 sequence.

17. The method of any of claims 12-16, wherein said exogenous nucleic acid encodes a polypeptide having DXS activity.

**18.** The method of any of claims 12-16, wherein said exogenous nucleic acid encodes a polypeptide having DDS activity.

**19.** The method of any of claims 12-16, wherein said exogenous nucleic acid comprises at least one exogenous nucleic acid encoding a polypeptide having DXS activity and a polypeptide having DDS activity.

**20.** The method of any of claims 12-16, wherein said exogenous nucleic acid comprises at least one exogenous nucleic acid encoding a polypeptide having DXS activity, a polypeptide having DDS activity, and a polypeptide having ubiC activity.

**Patentansprüche**

**1.** Wirtszelle umfassend eine genomische Deletion und eine exogene Nukleinsäure, wobei die Deletion mindestens einen Teil einer ppsR-Sequenz oder cbb3-Sequenz umfasst, wobei die Wirtszelle eine nicht funktionelle ppsR-Sequenz oder cbb3-Sequenz umfasst und wobei die exogene Nukleinsäure ein Polypeptid kodiert, das Decaprenyldiphosphatsynthase- (DDS-), 1-Desoxyxylulose-5-phosphatsynthase-(DXS-) oder Chorismatlyase-Aktivität besitzt.

**2.** Wirtszelle nach Anspruch 1, wobei die Wirtszelle *Rhodobacter* ist.

**3.** Wirtszelle nach Anspruch 1, wobei die Wirtszelle eine exogene Nukleinsäure umfasst, die eine UbiC-Sequenz und/ oder LytB-Sequenz umfasst.

**4.** Wirtszelle nach Anspruch 1, wobei die cbb3-Sequenz eine ccoN-Sequenz ist.

**5.** Wirtszelle nach Anspruch 1, wobei die Wirtszelle eine nicht funktionelle crtE-Sequenz und eine nicht funktionelle cbb3-Sequenz; oder eine nicht funktionelle crtE-Sequenz und eine nicht funktionelle ppsR-Sequenz; oder eine nicht funktionelle crtE-Sequenz, ppsR-Sequenz und cbb3-Sequenz umfasst.

**6.** Wirtszelle nach einem beliebigen der Ansprüche 1 bis 5, wobei die Wirtszelle ein membranhaltiges Bakterium oder ein stark membranhaltiges Bakterium ist.

**7.** Wirtszelle nach Anspruch 6, wobei das membranhaltige Bakterium aus einer Gattung ausgewählt ist aus der Gruppe bestehend aus *Rhodobacter, Rhodospirillum, Rhodopseudomonas, Rhodomicrobium, Rhodocyclus, Rhodopila* und *Methylomonas;* insbesondere wobei das membranhaltige Bakterium *Rhodobacter sphaeroides, Rhodobacter capsulatus, Rhodobacter suljidophilus, Rhodobacter adriaticus* oder *Rhodobacter veldkampii* ist; oder wobei das membranhaltige Bakterium *Rhodospirillum rubrum, Rhodospirillum photometricum, Rhodospirillum molischianum, Rhodospirillum fulvum* oder *Rhodospirillum salinarum* ist.

**8.** Wirtszelle nach einem beliebigen der Ansprüche 1 bis 7, wobei die exogene Nukleinsäure ein Polypeptid mit DXS-Aktivität kodiert.

**9.** Wirtszelle nach einem beliebigen der Ansprüche 1 bis 7, wobei die exogene Nukleinsäure ein Polypeptid mit DDS-Aktivität kodiert.

**10.** Wirtszelle nach einem beliebigen der Ansprüche 1 bis 7, wobei die exogene Nukleinsäure mindestens eine exogene Nukleinsäure umfasst, die ein Polypeptid mit DXS-Aktivität und ein Polypeptid mit DDS-Aktivität kodiert.

**11.** Wirtszelle nach einem beliebigen der Ansprüche 1 bis 7, wobei die exogene Nukleinsäure mindestens eine exogene Nukleinsäure umfasst, die ein Polypeptid mit DXS-Aktivität, ein Polypeptid mit DDS-Aktivität und ein Polypeptid mit ubiC-Aktivität kodiert.

**12.** Verfahren zum Herstellen von CoQ(10), umfassend das Kultivieren einer Wirtszelle unter Bedingungen, unter denen die Wirtszelle CoQ(10) herstellt, wobei die Wirtszelle eine exogene Nukleinsäure umfasst, wobei die exogene Nukleinsäure ein Polypeptid mit DDS-, DXS- oder Chorismatlyase-Aktivität kodiert; und eine genomische Deletion umfasst, wobei die Deletion mindestens einen Teil einer ppsR-Sequenz oder cbb3-Sequenz umfasst, und wobei die Wirtszelle eine nicht funktionelle ppsR-Sequenz oder cbb3-Sequenz umfasst; und das Isolieren von CoQ(10).

13. Verfahren nach Anspruch 12, wobei die Zelle *Rhodobacter* ist.

14. Verfahren nach Anspruch 12, wobei das Polypeptid ein UbiC-Polypeptid oder ein LytB-Polypeptid ist.

15. Verfahren nach Anspruch 12, wobei die cbb3-Sequenz eine ccoN-Sequenz ist.

16. Verfahren nach einem beliebigen der Ansprüche 12 bis 15, wobei die Wirtszelle eine nicht funktionelle crtE-Sequenz und eine nicht funktionelle cbb3-Sequenz; oder eine nicht funktionelle crtE-Sequenz und eine nicht funktionelle ppsR-Sequenz; oder eine nicht funktionelle crtE-Sequenz, ppsR-Sequenz und cbb3-Sequenz umfasst.

17. Verfahren nach einem beliebigen der Ansprüche 12 bis 16, wobei die exogene Nukleinsäure ein Polypeptid mit DXS-Aktivität kodiert.

18. Verfahren nach einem beliebigen der Ansprüche 12 bis 16, wobei die exogene Nukleinsäure ein Polypeptid mit DDS-Aktivität kodiert.

19. Verfahren nach einem beliebigen der Ansprüche 12 bis 16, wobei die exogene Nukleinsäure mindestens eine exogene Nukleinsäure umfasst, die ein Polypeptid mit DXS-Aktivität und ein Polypeptid mit DDS-Aktivität kodiert.

20. Verfahren nach einem beliebigen der Ansprüche 12 bis 16, wobei die exogene Nukleinsäure mindestens eine exogene Nukleinsäure umfasst, die ein Polypeptid mit DXS-Aktivität, ein Polypeptid mit DDS-Aktivität und ein Polypeptid mit ubiC-Aktivität kodiert.

## Revendications

1. Cellule hôte comprenant une délétion génomique et un acide nucléique exogène, dans laquelle ladite délétion comprend au moins une portion d'une séquence ppsR ou d'une séquence cbb3, ladite cellule hôte comprenant une séquence ppsR ou une séquence cbb3 non fonctionnelle, et dans laquelle ledit acide nucléique exogène code pour un polypeptide ayant une activité décaprényle diphosphate synthase (DDS), 1-désoxyxylulose-5-phosphate synthase (DXS) ou chorismate lyase.

2. Cellule hôte selon la revendication 1, ladite cellule hôte étant Rhodobacter.

3. Cellule hôte selon la revendication 1, ladite cellule hôte comprenant un acide nucléique exogène comprenant une séquence UbiC et/ou une séquence LytB.

4. Cellule hôte selon la revendication 1, dans laquelle ladite séquence cbb3 est une séquence ccoN.

5. Cellule hôte selon la revendication 1, ladite cellule hôte comprenant une séquence crtE non fonctionnelle et une séquence cbb3 non fonctionnelle, ou une séquence crtE non fonctionnelle et une séquence ppsR non fonctionnelle, ou une séquence crtE, une séquence ppsR et une séquence cbb3 non fonctionnelles.

6. Cellule hôte selon l'une quelconque des revendications 1 à 5, ladite cellule hôte étant une bactérie membraneuse ou une bactérie hautement membraneuse.

7. Cellule hôte selon la revendication 6, dans laquelle ladite bactérie membraneuse est d'un genre choisi dans le groupe constitué par *Rhodobacter, Rhodospirillum, Rhodopseudomonas, Rhodomicrobium, Rhodocyclus, Rhodopila* et *Methylomonas ;* en particulier dans laquelle ladite bactérie membraneuse est *Rhodobacter sphaeroides, Rhodobacter capsulatus, Rhodobacter sulfidophilus, Rhodobacter adriaticus* ou *Rhodobacter veldkampii ;* ou dans laquelle ladite bactérie membraneuse est *Rhodospirillum rubrum, Rhodospirillum photometricum, Rhodospirillum molischianum, Rhodospirillum fulvum* ou *Rhodospirillum salinarum.*

8. Cellule hôte selon l'une quelconque des revendications 1 à 7, dans laquelle ledit acide nucléique exogène code pour un polypeptide ayant une activité DXS.

9. Cellule hôte selon l'une quelconque des revendications 1 à 7, dans laquelle ledit acide nucléique exogène code pour un polypeptide ayant une activité DDS.

**10.** Cellule hôte selon l'une quelconque des revendications 1 à 7, dans laquelle ledit acide nucléique exogène comprend au moins un acide nucléique exogène codant pour un polypeptide ayant une activité DXS et un polypeptide ayant une activité DDS.

**11.** Cellule hôte selon l'une quelconque des revendications 1 à 7, dans laquelle ledit acide nucléique exogène comprend au moins un acide nucléique exogène codant pour un polypeptide ayant une activité DXS, un polypeptide ayant une activité DDS, et un polypeptide ayant une activité ubiC.

**12.** Procédé de préparation de CoQ(10), comprenant la culture d'une cellule hôte dans des conditions dans lesquelles ladite cellule hôte produit CoQ(10),
dans lequel ladite cellule hôte comprend un acide nucléique exogène, ledit acide nucléique exogène codant pour un polypeptide ayant une activité DDS, DXS ou chorismate lyase ; et comprend une délétion génomique, ladite délétion comprenant au moins une portion d'une séquence ppsR ou d'une séquence cbb3, et dans lequel ladite cellule hôte comprend une séquence ppsR ou une séquence cbb3 non fonctionnelle ; et l'isolement de CoQ (10).

**13.** Procédé selon la revendication 12, dans lequel ladite cellule est *Rhodobacter.*

**14.** Procédé selon la revendication 12, dans lequel ledit polypeptide est un polypeptide UbiC ou un polypeptide LytB.

**15.** Procédé selon la revendication 12, dans lequel ladite séquence cbb3 est une séquence ccoN.

**16.** Procédé selon l'une quelconque des revendications 12 à 15, dans lequel ladite cellule hôte comprend une séquence crtE non fonctionnelle et une séquence cbb3 non fonctionnelle, ou une séquence crtE non fonctionnelle et une séquence ppsR non fonctionnelle, ou une séquence crtE, une séquence ppsR et une séquence cbb3 non fonctionnelles.

**17.** Procédé selon l'une quelconque des revendications 12 à 16, dans lequel ledit acide nucléique exogène code pour un polypeptide ayant une activité DXS.

**18.** Procédé selon l'une quelconque des revendications 12 à 16, dans lequel ledit acide nucléique exogène code pour un polypeptide ayant une activité DDS.

**19.** Procédé selon l'une quelconque des revendications 12 à 16, dans lequel ledit acide nucléique exogène comprend au moins un acide nucléique exogène codant pour un polypeptide ayant une activité DXS et un polypeptide ayant une activité DDS.

**20.** Procédé selon l'une quelconque des revendications 12 à 16, dans lequel ledit acide nucléique exogène comprend au moins un acide nucléique exogène codant pour un polypeptide ayant une activité DXS, un polypeptide ayant une activité DDS, et un polypeptide ayant une activité ubiC.

Figure 1

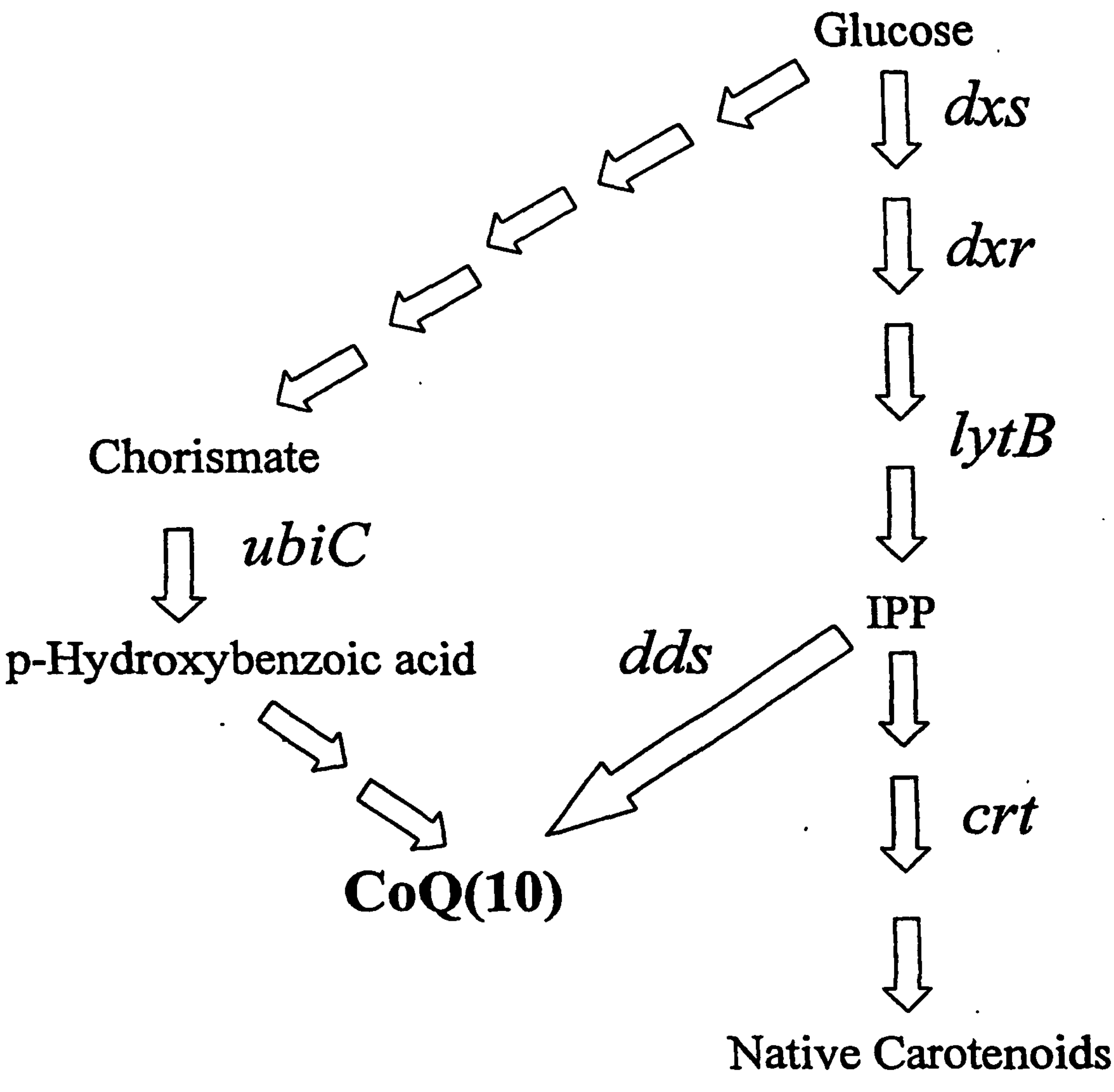
Glucose
dxs
dxr
lytB
IPP
crt
Native Carotenoids
Chorismate
ubiC
p-Hydroxybenzoic acid
dds
CoQ(10)

# Figure 2 (page 1 of 2)

```
   1 ctgcggccag accacgcata tcgacgacga ttcgatcacg aaaaacgtac
  51 ggtccgcagc ccagcacgcc ggtttttcgc cggtccggcc ggtgatcgag
 101 gtgcgcggca agtgcggcaa gtgtgactga cctgtccaac agaccgttcg
 151 acttgagact aacgttgcgc taacaaagcc catggctgac ctacccaaga
 201 cgccgctgct cgacacggtc gacacgccgc aggacctccg gaagctcgcc
 251 cccgcccagc tgcgccagct ggccgacgag cttcgtgccg aaaccatcag
 301 tgcggtgggc tccaccggcg ggcatctagg ctccggcctg ggcgtcgtcg
 351 aactgacggt ggcgatccac tatgtattca acacccccga cgaccggctg
 401 atctgggacg tcgggcacca atgctatccg cacaagatcc tcaccggtcg
 451 gcgcgatcgg atccgcacga ttcgtcaggg tggaggcctc tccggcttca
 501 ccaagcgcag cgagagcgag tatgatccgt tcggtgccgc gcactcgtcg
 551 acctcgatct cggccgcact cggctttgcg atcgccaaca agctcaacga
 601 ggcgccgggc aaggcgatcg cggtgatcgg cgacggcgcg atgagcgcgg
 651 gcatggccta tgaggcgatg aacaacgccg aggccgccgg caaccggctg
 701 gtggtgatcc tcaacgacaa cgacatgtcg atcgccccgc cggtgggcgg
 751 gctttcggcc tatcttgcgc gcctcatttc ctcgtccgaa tatctcggcc
 801 tgcgcgagct cgccaagcgc ttcacccgca agctttcgcg ccgcctcacc
 851 gcggcagccg gcaaggcgga ggaattcgcc cgcggcatgg cgaccggcgg
 901 cacgctgttc gaggaacttg gcttctatta tgtcggcccg atcgacggcc
 951 acaatctcga gcatctgatc ccggtgctgg agaatgtccg cgacagcgag
1001 cagggcccga tcctgatcca tgtcgtgacc aagaagggca agggctatgc
1051 cccggccgaa gcggcggcgg acaagtatca cggcgtccag aagttcgacg
1101 tgatcaccgg ggcacaggcc aaggcacccc cgggcccgcc cgcctatacc
1151 aaggtgttcg ccgatgcgct gctcgccgaa gcggagcgtg atgcgtcggt
1201 ctgcgcgatc accgcggcga tgccctcggg caccgggctc gacaagttcc
1251 aggcgacgtt ccccgatcgc accttcgacg tgggcattgc cgaacagcac
1301 gcggtcacct tcgcagcggg ccttgccgcg caggggatgc ggccgttctg
1351 cgcgatctac tcgaccttcc tgcagcgcgc ctacgaccag gtcgtccacg
1401 acgtcgcgat ccagaacctg ccggtccgct tcgcgatcga ccgcgcgggc
1451 ctggtcggtg ccgacggcgc gacccatgcc ggcagcttcg acgtgaccta
1501 tctcgccagc ctgcccaatt tcgtggtgat ggcggccgcg gacgaggtcg
1551 agctcgtcca catgacccac acggcggcga tgcacgacag cggcccgatc
1601 gcgctgcgct atccacgcgg caacggcgtc ggactggcgc tgcccaaggt
1651 tccggagcgg ctggaaatcg gcaagggtcg cgtggtccga gagggcaaga
1701 aggtagcgat cctgtcgctc ggcacgcgcc ttgcggaagc actaaaggcc
1751 gccgacacgc tcgaggccaa gggcctctcg accaccgtcg ccgacctgcg
1801 cttcgccaaa ccgctcgacg aggatctgat ccgccgcctg ctcaccaccc
1851 acgaagtggc ggtgacgatc gaggaaggcg cgatcggcgg ccccggtgcg
1901 catgtgctga cgctcgccag cgataccggc ctgatcgacg ccggcctcaa
1951 gctgcgcacc atgcgcctgc cggacatatt ccaggaccag gacaagcccg
2001 agaagcagta tgacgaagcg gggctgaacg ccgccaacat cgtcgacacg
2051 gtgctgaagg cgctccgcta caacgaggcc gagctggccg acggggtgcg
2101 ggcgtaaacg acgccagatc ctccccggaa cggggagggg aaccgccgcc
2151 gaaggcggtg gtggaggggc cgctgcggca cgcancggtt tcccaggctg
```

# Figure 2 (page 2 of 2)

```
2201  agagcgatcc gcgccttgcg gcgcgccccc cccaccattc gctggcgcgg
2251  atggtccccc tccccgttcc ggggaggatc tgggtcctgc cccaccttga
2301  atctccaaca tgcacatgcc atgtacatgc acatggctac gcagcttccc
2351  cagactcgct ccagccgcgt tgtcgtgctg gtatcgcccg aggaaaaacg
2401  gcgcatttcc gccaatgcgg aagcggcgga catgacggtc agcgacttca
2451  tgcgcaccgc cgccgaacgc tataccgagc cgaccgacgc cgagatggcg
2501  ctgatgcgcg acctgctcgc ccagctcgaa caggccaatg cccgcacgga
2551  cgcggccttt gcccagctcg aagctgcgcg cgccgccgcc accgcgttcg
2601  acgaggaggc gtatcgcgcc gaggtccgcg aacagctgct gaccgatacc
2651  tcaatcgact gggatgcgct gtccactgcc ctttccggct gggcgcgcca
2701  gtgagcttct ggaccgatgc gctacgcgcg ctccagcagg tcgcgctgct
2751  ccagcacaag gtcgagcagg cgctgaccac cgccgaggaa gcccgccgcc
2801  attcaatcga gacgcgcgag cgggtgatcc ggcttgagac gctgatcgac
2851  atcgcgatga gacgccagcc cgcagcaccg cctacgccgc ctgcgcttcc
2901  cgaaagtcca caaaccggca gctagcgccc gcttccccga gcgcgtacat
2951  cgcggtacgt gctgaaaatg accatccttc ccctcaccgc ccgcccccgc
3001  gcgctcgcgc actggctgtt cgtcgtcgcc gcgatgatcg tcgcgatggt
3051  cgtggtcggg ggcattaccc ggctcaccga atcgggcctg tcgatcaccg
3101  aatggaagcc aatctccggc atcgtgcccc cgctcaacga cgcgcagtgg
3151  caggccgagt tcgaccacta caagcagatc ggccagtatg agcagctcaa
3201  ccagggcatg acgctcggcg ggttcaagag catcttcttc tgggaatata
3251  tccaccgcct gctcggccgg ctgatcggca tggtgttcgc gctgccgctg
3301  ctgtggttcg ccgtccgcaa gcagatcccg cagggctatg gctggcggct
3351  ggtcgcgctg ctcgcgctag gcgggctgca gggcgcgttc ggctggtgga
3401  tggtgaagtc ggggctcaac cacacccgca cctcggttag ccatttctgg
3451  ctggcgaccc acctgatgac cgcactgttc acgctgggcg gcatcgtctg
3501  gacgatgctc gacctgcgcg cgcttgccgc caaccatgcc gagcgccctg
3551  cccgactgac cgggctcggc gcgggcgtgc tggtactgct ggcggtccag
3601  ctcttctacg gggcgctggt agcagg  (SEQ ID NO:1)
```

# Figure 3

| | | | | | |
|---|---|---|---|---|---|
| 1 | atggctgacc | tacccaagac | gccgctgctc | gacacggtcg | acacgccgca |
| 51 | ggacctccgg | aagctcgccc | ccgcccagct | gcgccagctg | gccgacgagc |
| 101 | ttcgtgccga | aaccatcagt | gcggtgggct | ccaccggcgg | gcatctaggc |
| 151 | tccggcctgg | gcgtcgtcga | actgacggtg | gcgatccact | atgtattcaa |
| 201 | caccccccgac | gaccggctga | tctgggacgt | cgggcaccaa | tgctatccgc |
| 251 | acaagatcct | caccggtcgg | cgcgatcgga | tccgcacgat | tcgtcagggt |
| 301 | ggaggcctct | ccggcttcac | caagcgcagc | gagagcgagt | atgatccgtt |
| 351 | cggtgccgcg | cactcgtcga | cctcgatctc | ggccgcactc | ggctttgcga |
| 401 | tcgccaacaa | gctcaacgag | gcgccgggca | aggcgatcgc | ggtgatcggc |
| 451 | gacggcgcga | tgagcgcggg | catggcctat | gaggcgatga | acaacgccga |
| 501 | ggccgccggc | aaccggctgg | tggtgatcct | caacgacaac | gacatgtcga |
| 551 | tcgccccgcc | ggtgggcggg | ctttcggcct | atcttgcgcg | cctcatttcc |
| 601 | tcgtccgaat | atctcggcct | gcgcgagctc | gccaagcgct | tcacccgcaa |
| 651 | gctttcgcgc | cgcctcaccg | cggcagccgg | caaggcggag | gaattcgccc |
| 701 | gcggcatggc | gaccggcggc | acgctgttcg | aggaacttgg | cttctattat |
| 751 | gtcggcccga | tcgacggcca | caatctcgag | catctgatcc | cggtgctgga |
| 801 | gaatgtccgc | gacagcgagc | agggcccgat | cctgatccat | gtcgtgacca |
| 851 | agaagggcaa | gggctatgcc | ccggccgaag | cggcggcgga | caagtatcac |
| 901 | ggcgtccaga | agttcgacgt | gatcaccggg | gcacaggcca | aggcaccccc |
| 951 | gggcccgccc | gcctatacca | aggtgttcgc | cgatgcgctg | ctcgccgaag |
| 1001 | cggagcgtga | tgcgtcggtc | tgcgcgatca | ccgcggcgat | gccctcgggc |
| 1051 | accgggctcg | acaagttcca | ggcgacgttc | cccgatcgca | ccttcgacgt |
| 1101 | gggcattgcc | gaacagcacg | cggtcacctt | cgcagcgggc | cttgccgcgc |
| 1151 | aggggatgcg | gccgttctgc | gcgatctact | cgaccttcct | gcagcgcgcc |
| 1201 | tacgaccagg | tcgtccacga | cgtcgcgatc | cagaacctgc | cggtccgctt |
| 1251 | cgcgatcgac | cgcgcgggcc | tggtcggtgc | cgacggcgcg | acccatgccg |
| 1301 | gcagcttcga | cgtgacctat | ctcgccagcc | tgcccaattt | cgtggtgatg |
| 1351 | gcggccgcgg | acgaggtcga | gctcgtccac | atgacccaca | cggcggcgat |
| 1401 | gcacgacagc | ggcccgatcg | cgctgcgcta | tccacgcggc | aacggcgtcg |
| 1451 | gactggcgct | gcccaaggtt | ccggagcggc | tggaaatcgg | caagggtcgc |
| 1501 | gtggtccgag | agggcaagaa | ggtagcgatc | ctgtcgctcg | gcacgcgcct |
| 1551 | tgcggaagca | ctaaaggccg | ccgacacgct | cgaggccaag | ggcctctcga |
| 1601 | ccaccgtcgc | cgacctgcgc | ttcgccaaac | cgctcgacga | ggatctgatc |
| 1651 | cgccgcctgc | tcaccaccca | cgaagtggcg | gtgacgatcg | aggaaggcgc |
| 1701 | gatcggcggc | cccggtgcgc | atgtgctgac | gctcgccagc | gataccggcc |
| 1751 | tgatcgacgc | cggcctcaag | ctgcgcacca | tgcgcctgcc | ggacatattc |
| 1801 | caggaccagg | acaagcccga | gaagcagtat | gacgaagcgg | ggctgaacgc |
| 1851 | cgccaacatc | gtcgacacgg | tgctgaaggc | gctccgctac | aacgaggccg |
| 1901 | agctggccga | cggggtgcgg | gcgtaa | (SEQ ID NO:2) | |

# Figure 4

1 madlpktpll dtvdtpqdlr klapaqlrql adelraetis avgstgghlg
51 sglgvveltv aihyvfntpd drliwdvghq cyphkiltgr rdrirtirqg
101 gglsgftkrs eseydpfgaa hsstsisaal gfaianklne apgkaiavig
151 dgamsagmay eamnnaeaag nrlvvilndn dmsiappvgg lsaylarlis
201 sseylglrel akrftrklsr rltaaagkae efargmatgg tlfeelgfyy
251 vgpidghnle hlipvlenvr dseqgpilih vvtkkgkgya paeaaadkyh
301 gvqkfdvitq aqakappgpp aytkvfadal laeaerdasv caitaampsg
351 tgldkfqatf pdrtfdvgia eqhavtfaag laaqgmrpfc aiystflqra
401 ydqvvhdvai qnlpvrfaid raglvgadga thagsfdvty laslpnfvvm
451 aaadevelvh mthtaamhds gpialryprg ngvglalpkv perleigkgr
501 vvregkkvai lslgtrlaea lkaadtleak glsttvadlr fakpldedli
551 rrlltthevа vtieegaigg pgahvltlas dtglidaglk lrtmrlpdif
601 qdqdkpekqy deaglnaani vdtvlkalry neaeladgvr a (SEQ ID NO:3)

# Figure 5 (page 1 of 25)

```
STdxsdna     182 atg-------------------------------------
CRdxsdna       1 atgctgcgtggtgctgtttctcacggccctgcggtcgccg
CJdxsdna       1 ----------------------------------------
PAdxsdna       1 atg-------------------------------------
LEdxsdna       1 atg-------------------------------------
MTdxsdna       1 ----------------------------------------
RSdxs1dna      1 atg-------------------------------------
RSdxs2dna      1 atg-------------------------------------
SPCCdxsdna     1 ----------------------------------------
ECdxsdna       1 atg-------------------------------------
NMdxsdna       1 ----------------------------------------
HIdxsdna       1 atg-------------------------------------
SSdxsdna       1 ----------------------------------------
HPdxsdna       1 ----------------------------------------

STdxsdna     185 -----------gct--------------------------
CRdxsdna      41 accgggctgccgct--------------------------
CJdxsdna       1 ------------at--------------------------
PAdxsdna       4 -----------cccaagacgctccatgagattccccgc--
LEdxsdna       4 -----------gctttgtgtgcttatgcatttcctgggat
MTdxsdna       1 ----------------------------------------
RSdxs1dna      4 -----------acc--------------------------
RSdxs2dna      4 -----------acc--------------------------
SPCCdxsdna     1 ----------------------------------------
ECdxsdna       4 -----------agtttt-----------------------
NMdxsdna       1 ----------------------------------------
HIdxsdna       4 -----------act--------------------------
SSdxsdna       1 ----------------------------------------
HPdxsdna       1 ------------gt--------------------------

STdxsdna     188 ----------------------------------------
CRdxsdna      55 ----------------------------------------
CJdxsdna       3 ----------------------------------------
PAdxsdna      31 ----------------------------------------
LEdxsdna      33 tttgaacaggactggtgtggtttcagattcttctaaggca
MTdxsdna       1 ----------------------------------------
RSdxs1dna      7 ----------------------------------------
RSdxs2dna      7 ----------------------------------------
SPCCdxsdna     1 ----------------------------------------
ECdxsdna      10 ----------------------------------------
NMdxsdna       1 ----------------------------------------
HIdxsdna       7 ----------------------------------------
SSdxsdna       1 ----------------------------------------
HPdxsdna       3 ----------------------------------------
```

# Figure 5 (page 2 of 25)

```
STdxsdna      188 ---------------------------------gacc---
CRdxsdna       55 ---------------------------------ggcc---
CJdxsdna        3 ---------------------------------ga-g---
PAdxsdna       31 ---------------------------------gagc---
LEdxsdna       73 acccctttgttctctggatggattcatggaacagatc---
MTdxsdna        1 ----------------------------------------
RSdxs1dna       7 ---------------------------------gaca---
RSdxs2dna       7 ---------------------------------aatc---
SPCCdxsdna      1 ----------------------------------------
ECdxsdna       10 ---------------------------------gata---
NMdxsdna        1 ----------------------------------atg---
HIdxsdna        7 ---------------------------------aacaata
SSdxsdna        1 ----------------------------------------
HPdxsdna        3 ---------------------------------gatt---

STdxsdna      192 t---------ac----------cc----------------
CRdxsdna       59 c---------cg----------cccgctgcgctgctcccg
CJdxsdna        6 t---------aa----------aa----------------
PAdxsdna       35 g---------cc----------cc----------------
LEdxsdna      110 t---------gcagtttttgttcc----------------
MTdxsdna        1 ----------------------------------------
RSdxs1dna      11 g---------ac----------cc----------------
RSdxs2dna      11 ccaccccgcgac----------cc----------------
SPCCdxsdna      1 ----------------------------------------
ECdxsdna       14 t---------tg----------cc----------------
NMdxsdna        4 a---------ac----------cc----------------
HIdxsdna       14 t---------ga----------ac----------------
SSdxsdna        1 ----------------------------------------
HPdxsdna        7 t---------tg----------ca----------------

STdxsdna      197 ----------------------------------------
CRdxsdna       80 tcgcccgtggtgtgcgcagcgcagcgcccacgcgtcagcg
CJdxsdna       11 ----------------------------------------
PAdxsdna       40 ----------------------------------------
LEdxsdna      125 ----------------------------------------
MTdxsdna        1 ----------------------------------------
RSdxs1dna      16 ----------------------------------------
RSdxs2dna      25 ----------------------------------------
SPCCdxsdna      1 ----------------------------------------
ECdxsdna       19 ----------------------------------------
NMdxsdna        9 ----------------------------------------
HIdxsdna       19 ----------------------------------------
SSdxsdna        1 ----------------------------------------
HPdxsdna       12 ----------------------------------------
```

# Figure 5 (page 3 of 25)

```
STdxsdna      197 ----------------------------------------
CRdxsdna      120 tcgcgcggaggcttcggtcaatgccccgcgggcgggcccg
CJdxsdna       11 ----------------------------------------
PAdxsdna       40 ----------------------------------------
LEdxsdna      125 ----------------------------------------
MTdxsdna        1 ----------------------------------------
RSdxs1dna      16 ----------------------------------------
RSdxs2dna      25 ----------------------------------------
SPCCdxsdna      1 ----------------------------------------
ECdxsdna       19 ----------------------------------------
NMdxsdna        9 ----------------------------------------
HIdxsdna       19 ----------------------------------------
SSdxsdna        1 ----------------------------------------
HPdxsdna       12 ----------------------------------------

STdxsdna      197 ----------------------------------------
CRdxsdna      160 gccggtagctactcgggcgagtgggataagctttcagtgg
CJdxsdna       11 ----------------------------------------
PAdxsdna       40 ----------------------------------------
LEdxsdna      125 ----------------------------------------
MTdxsdna        1 ----------------------------------------
RSdxs1dna      16 ----------------------------------------
RSdxs2dna      25 ----------------------------------------
SPCCdxsdna      1 ----------------------------------------
ECdxsdna       19 ----------------------------------------
NMdxsdna        9 ----------------------------------------
HIdxsdna       19 ----------------------------------------
SSdxsdna        1 ----------------------------------------
HPdxsdna       12 ----------------------------------------

STdxsdna      197 --------------------------------aag--acg
CRdxsdna      200 aggagattgatgagtggcgcgatgtgggcccgaag--acg
CJdxsdna       11 --------------------------------aat--ttg
PAdxsdna       40 --------------------------------gcc--acg
LEdxsdna      125 --------------------------------aac--aca
MTdxsdna        1 ----------------------------------------
RSdxs1dna      16 --------------------------------tgc--acg
RSdxs2dna      25 --------------------------------gaa--acc
SPCCdxsdna      1 -------------------------------------atg
ECdxsdna       19 --------------------------------aaa--tac
NMdxsdna        9 --------------------------------aag----c
HIdxsdna       19 --------------------------------aat--tat
SSdxsdna        1 -------------------------------------gtg
HPdxsdna       12 --------------------------------aaataaaa
```

# Figure 5 (page 4 of 25)

```
STdxsdna     203 ccgctgctc----------gacacggtcga--------ca
CRdxsdna     238 cccctgctg----------gacactgtcaa--------tt
CJdxsdna      17 cccatactc----------aa-------------------
PAdxsdna      46 cccctgctc----------gaccgcgcctc--------tt
LEdxsdna     131 agcttactcatgaggtcaagaaaggtcacgtgtggttca
MTdxsdna       1 ---atgctg----------caacagatccg--------cg
RSdxs1dna     22 ccgacgctc----------gac-cgggtga--------cg
RSdxs2dna     31 ccgcttttg----------gatcgcgtctg--------ct
SPCCdxsdna     4 catctcagc----------gaaa---ttac--------cc
ECdxsdna      25 ccgaccctg----------gcactggtcga--------ct
NMdxsdna      13 cccctactc----------gacctgattga--------ca
HIdxsdna      25 cctctttta----------tctttaattaa--------tt
SSdxsdna       4 acgattctg----------gagaacatccg--------gc
HPdxsdna      20 cttttgatt----------taaaccctaac--------ga

STdxsdna     225 cgcc-gcaggacc----tccgga---------------ag
CRdxsdna     260 accc-ggtgcacc----tgaaga---------------ac
CJdxsdna      28 ------gaagagt----tagaaa---------------ag
PAdxsdna      68 cgcc-ggccgaac----tgcgcc---------------gg
LEdxsdna     171 ggct-tccttatcagaatctggagaatactacacacagag
MTdxsdna      20 ggcc-cgctgatc----tgcagc---------------ac
RSdxs1dna     43 ctcccggtggaca----taaagg---------------gc
RSdxs2dna     53 gccc-ggccgaca----tgaagg---------------cg
SPCCdxsdna    23 atcc-caaccagc----tccacg---------------gg
ECdxsdna      47 ccac-ccaggagt----tacgac---------------tg
NMdxsdna      35 gccc-gcaagatt----tgcgcc---------------gt
HIdxsdna      47 ctcc-agaagatt----tgcgtc---------------tt
SSdxsdna      26 aacc-acgcgacc----tgaagg---------------cg
HPdxsdna      42 tatt-gcagg--------cttgg---------------ag

STdxsdna     245 ctcgcccccgcccagctgcgccag----------------
CRdxsdna     280 ttcaacaatgagcagctgaagcag----------------
CJdxsdna      43 ctaagtttaaaagaattagaaaat----------------
PAdxsdna      88 ctgggcgaggcggacctggaaacc----------------
LEdxsdna     210 accgccaacgcctatttggacactgtgaactatcccatt
MTdxsdna      40 ctttcccaggcgcagcttcgggag----------------
RSdxs1dna     64 ctcacggaccgtgagttgcgctcg----------------
RSdxs2dna     73 ctgagtgacgccgaactggagcgg----------------
SPCCdxsdna    43 ttgtcggttgctcagcttgagcaa----------------
ECdxsdna      67 ttgccgaaagagagtttaccgaaa----------------
NMdxsdna      55 ctggacaaaaaacagctgccgcgc----------------
HIdxsdna      67 ttaaataaagatcagctaccacaa----------------
SSdxsdna      46 ctgcccgaggagcagctgcacgaa----------------
HPdxsdna      58 tt------------ggtgtgtcaa----------------
```

# Figure 5 (page 5 of 25)

```
STdxsdna      269 ------------------------------------ctgg
CRdxsdna      304 ------------------------------------ctct
CJdxsdna       67 ------------------------------------ttag
PAdxsdna      112 ------------------------------------ctgg
LEdxsdna      250 catatgaaaaatctgtctctgaaggaacttaaacaactag
MTdxsdna       64 ------------------------------------ctgg
RSdxs1dna      88 ------------------------------------ctgg
RSdxs2dna      97 ------------------------------------ctgg
SPCCdxsdna     67 ------------------------------------attg
ECdxsdna       91 ------------------------------------ctct
NMdxsdna       79 ------------------------------------cttg
HIdxsdna       91 ------------------------------------ctct
SSdxsdna       70 ------------------------------------ctgt
HPdxsdna       70 ------------------------------------acg-

STdxsdna      273 ccgacgagcttcgtgccgaaacca-tcagtg--cggtggg
CRdxsdna      308 gcaaggagctgcgcagtgacatcg-tgcaca--ccgtctc
CJdxsdna       71 cagcatctatgcgtgaaaaatca-tacaag--ttgtgag
PAdxsdna      116 ccgacgagct--gcgccagtacct-gctgtataccgtcgg
LEdxsdna      290 cagatgaactaaggtcagatacaa-ttttca--atgtatc
MTdxsdna       68 ccgccgagatccgtgagttcctga-tccaca--aggttgc
RSdxs1dna      92 ccgacgagctgcgggccgaaacga-tctcgg--ccgtgtc
RSdxs2dna     101 ccgacgaagtgcgttccgaggtga-tttcgg--tcgttgc
SPCCdxsdna     71 gccaccagattcgtgagaagcacc-tgcaga--cggttgc
ECdxsdna       95 gcgacgaactgcgccgctatttac-tcgaca--gcgtgag
NMdxsdna       83 ccggcgagttgcgcacctttctgc-tggaat--ctgtcgg
HIdxsdna       95 gtcaagaattacgtgcttatcttt-tagaat--ctgttag
SSdxsdna       74 ccgaggaga-tcaggcagttcctggtgcacg--cggtcac
HPdxsdna       73 -ctacg-gaatcgt------attt-tagaag--tggtgag

STdxsdna      310 ctccaccggcgggcatctaggctccggcctgggcgtcgtc
CRdxsdna      345 tcgcaccggtggacaccttagcagcagcctgggcgtggtg
CJdxsdna      108 taaaaatggtgggcatttaagttcaaatttgggtgctgta
PAdxsdna      153 ccagaccggcggtcatttcggcgccggcctcggcgtggtc
LEdxsdna      327 aaagactggggtcaccttggctcaagtcttggtgttgtt
MTdxsdna      105 cgccacgggggggcatctggggccgaacctgggagtggtg
RSdxs1dna     129 ggtgacgggcgggcatctgggcgcaggcctcggcgtggtg
RSdxs2dna     138 cgagacgggaggacatctggggtcctcgctgggggtggtt
SPCCdxsdna    108 agcgaccggtgggcacctcgggccgggcttgggcgtggtg
ECdxsdna      132 ccgttccagcgggcacttcgcctccgggctgggcacggtc
NMdxsdna      120 gcagaccggcgggcatttcgccagcaatttgggcgcggtc
HIdxsdna      132 tcaaactagcggacatttagcgtcaggtttaggcactgta
SSdxsdna      111 cagaaccggcggtcatctgggacccaacctgggggtggtg
HPdxsdna      102 cgctaatgggggcatttaagctcttctttaggggctgtg
```

# Figure 5 (page 6 of 25)

| | | |
|---|---|---|
| STdxsdna | 350 | gaactgacggtggcgatccactatgtattcaacaccccg |
| CRdxsdna | 385 | gagctgacggtggctatgcactatgtattcaacaccccgg |
| CJdxsdna | 148 | gaacttagtatagcaatgcatttggtttttgatgcaaaaa |
| PAdxsdna | 193 | gagctgaccattgccctgcactacgtcttcgacactccgg |
| LEdxsdna | 367 | gagctgactgttgctcttcattatgtcttcaatgcaccgc |
| MTdxsdna | 145 | gaactcaccttggcgctgcaccgggtattcgactcgccgc |
| RSdxs1dna | 169 | gagttgacggttgcgctgcatgcgatcttcgatgcgcccc |
| RSdxs2dna | 178 | gagctgactgtcgcgctgcatgcggtcttcaacacgccca |
| SPCCdxsdna | 148 | gaattgaccctagcgctttaccaaacgctcgatctcgatc |
| ECdxsdna | 172 | gaactgaccgtggcgctgcactatgtctacaacaccccgt |
| NMdxsdna | 160 | gagctgacggttgcgctgcactacgtttacaacacgcccg |
| HIdxsdna | 172 | gagctaaccgttgcgctgcattatgtatataagacgccat |
| SSdxsdna | 151 | gagctgaccatcgccctgcaccgggtcttcgagtcgcccg |
| HPdxsdna | 142 | gagctgattgtgggcatgcatgccttatttgattgccaaa |

| | | |
|---|---|---|
| STdxsdna | 390 | acgaccggctgatctgggacgtcgggcaccaatgctatcc |
| CRdxsdna | 425 | aggacaagattatttgggacgtgggccaccaggcgtatgg |
| CJdxsdna | 188 | aagatccttttatttttgatgtgtcgcatcagtcttatac |
| PAdxsdna | 233 | acgaccgcctggtctgggacgtcggccaccaggcctatcc |
| LEdxsdna | 407 | aagataggattctctgggatgttggtcatcagtcttatcc |
| MTdxsdna | 185 | acgatccgatcatcttcgacaccggtcaccaggcctacgt |
| RSdxs1dna | 209 | gcgacaagatcatctgggacgtgggccaccagtgctaccc |
| RSdxs2dna | 218 | ccgacaagctcgtctgggacgtgggccaccagtgctaccc |
| SPCCdxsdna | 188 | gcgacaaagtggtttgggacgttggccaccaagcctatcc |
| ECdxsdna | 212 | ttgaccaattgatttgggatgtggggcatcaggcttatcc |
| NMdxsdna | 200 | aagacaagctggtgtgggatgtcggacaccaaagctatcc |
| HIdxsdna | 212 | ttgatcagttaatttgggatgtgggacatcaagcttatcc |
| SSdxsdna | 191 | tcgaccgcatcctgtgggacaccggccaccagagctacgt |
| HPdxsdna | 182 | aaaaccctttcattttgacacttcgcaccaagcttacgc |

| | | |
|---|---|---|
| STdxsdna | 430 | gcacaagatcctcaccggtcggcgcgatcgga---tccgc |
| CRdxsdna | 465 | ccacaagatcctgactggccgtcgcaagggta---tggcc |
| CJdxsdna | 228 | acacaagcttttaagcggaaaagaagaaatat---ttgat |
| PAdxsdna | 273 | gcacaagatcctcaccgagcgccgcgagctga---tgggc |
| LEdxsdna | 447 | tcacaaaatcttgactggtagaagggacaaga---tgtcg |
| MTdxsdna | 225 | ccacaagatgttgaccggacgcagccaggact---tcgca |
| RSdxs1dna | 249 | ccacaagatcctgaccgggcggcgcgaccgca---tccgc |
| RSdxs2dna | 258 | ccacaagatcctcaccggccggcgcgagcaga---tgcgc |
| SPCCdxsdna | 228 | ccacaagctgctgacag---ggcgctatcacaacttccat |
| ECdxsdna | 252 | gcataaaatttgaccggacgccgcgacaaaa---tcggc |
| NMdxsdna | 240 | gcacaaaattcttaccggacgtaaaaaccaga---tgcac |
| HIdxsdna | 252 | acataaaatcctaacgggtcgccgagagcaaa---tgtcc |
| SSdxsdna | 231 | acacaagctgctgacgggacgtcagga---ct---tctcc |
| HPdxsdna | 222 | ccacaagcttttaaccgggcgctttgaaagct---ttagc |

# Figure 5 (page 7 of 25)

```
STdxsdna     467 acgattcgtcagggtggaggcctctccggcttcaccaag-
CRdxsdna     502 acgattcgccagaccaacggcctttcgggcttcacgaag-
CJdxsdna     265 actttaagacaaatcaatggtttaagtggttatacaaaa-
PAdxsdna     310 accctgcgccagaagaacggcctggcggccttcccgcgc-
LEdxsdna     484 acattaaggcagacagatggtcttgcaggatttactaag-
MTdxsdna     262 accctgcgtaagaagggcgggttgtcggggtatccgtct-
RSdxs1dna    286 accctgcggcagggcgggggtctctcgggcttcaccaag-
RSdxs2dna    295 accctgcgccagaagggcggcctctcgggcttcaccaag-
SPCCdxsdna   265 accttgcggcaaaaggatggcattgcgggctacccgaag-
ECdxsdna     289 accatccgtcagaaaggcggtctgcacccgttcccgtgg-
NMdxsdna     277 accatgcgccaatatggcggtttggcgggttttccgaaa-
HIdxsdna     289 acaattcgccaaaaagacggtat-tcatccttttccttgg
SSdxsdna     265 aagctgcgcggcaagggcggcctgtccggctacccctcg-
HPdxsdna     259 actttaaggcaattcaagggtttgagcggctttactaaa-

STdxsdna     506 cgcagcgagagcgagtatgatccgttcggtgccgcgc-ac
CRdxsdna     541 cgcgacgagagcgagtacgaccctttcggcgctggcc-ac
CJdxsdna     304 cctagcgagggagattat------tttgtagcagggc-at
PAdxsdna     349 cgcgcagagagcgagtacgacaccttcggcgtcggcc-ac
LEdxsdna     523 cgatcggagagtgaatatgattgctttggcaccggcc-ac
MTdxsdna     301 cgtgccgagagcgagcacga-ctgggtggagtcgagccac
RSdxs1dna    325 cgctccgagagcccctatgactgtttcggcgcgggcc-at
RSdxs2dna    334 cgctcggaatccgcctacgacccgttcggcgcggctc-at
SPCCdxsdna   304 cgcacggaaaaccgcttcgatcatttcggtgccggtc-ac
ECdxsdna     328 cgcggcgaaagcgaatatgacgtattaagcgtcgggc-at
NMdxsdna     316 cgttgcgagtccgagtacgacgcgttcggcgtggggc-at
HIdxsdna     328 cgtgaagaaagtgaatttgatgtattaagtgttggtc-ac
SSdxsdna     304 cgcgaggagtccgagcacgacgtcatcgagaacagcc-ac
HPdxsdna     298 cccagcgagagcgcatacgattatttcatcgccgggc-at

STdxsdna     545 tcgtcgacctcgatctcggccgcact--cggctttgcgat
CRdxsdna     580 agctccacctcgatttcggcggctct--gggtatggcggt
CJdxsdna     337 tctagtacctctatttctttggcagt--aggtgcttgtaa
PAdxsdna     388 tccagcacctccatcagcgccgccct--gggcatggccat
LEdxsdna     562 agttccaccaccatctcagcaggcct--agggatggctgt
MTdxsdna     340 gccagcgcggcgctgtcgtacgcgga--cgggttggccaa
RSdxs1dna    364 tcctcgacctcgatctcggccgcggt--gggctttgccgc
RSdxs2dna    373 tcctcgacctcgatctcggccgcgct--cggctttgccat
SPCCdxsdna   343 gcttccaccagtatttctgctggcct--cggtatggctct
ECdxsdna     367 tcatcaacctccatcagtgccggaat--tggtattgcggt
NMdxsdna     355 tcctccacctccatcggcgcggcgtt--gggcatggcggc
HIdxsdna     367 tcctctacgtctattagtgcgggatt--aggcattgccgt
SSdxsdna     343 gcctccac--cgccctcggctgggccgacggactcgccaa
HPdxsdna     337 agttccacttcggtgt------ctat--aggcgttggggt
```

# Figure 5 (page 8 of 25)

```
STdxsdna     583 cgc-c-------aacaagctc--------aacgag-gc--
CRdxsdna     618 ggg-c-------cgcgacgtt--------aagggc-aa--
CJdxsdna     375 ggc-t-------attgcttta--------aagggt-ga--
PAdxsdna     426 cgc-c-------gcccgcctg--------caaggc-aa--
LEdxsdna     600 tgg-t-------agagatcta--------aaagga-ag--
MTdxsdna     378 ggc-g-------ttcgagttg--------accg-g-ac--
RSdxs1dna    402 ggc-a-------cgcgagatg--------ggcggc-ga--
RSdxs2dna    411 ggg-t-------cgcgagctg--------ggccag-cc--
SPCCdxsdna   381 agcac-------gggatgccc--------agggcg-aa--
ECdxsdna     405 tgc-tgccgaaaaagaaggca--------aa--aa-tc--
NMdxsdna     393 ggc-g-------gacaaacag--------ttgggcagc--
HIdxsdna     405 tgc-c------------gcag--------aacgag-aaaa
SSdxsdna     381 ggc-c-------cgccgggtg--------caggggg-ga--
HPdxsdna     369 ggc-t-------a--aagcttttgtttgaaacaa-gc--

STdxsdna     604 -gccgg--gcaaggc----gatcgcggtgatcggcgacgg
CRdxsdna     639 -gaaga--acagtgt----gatcgctgtcatcggcgacgg
CJdxsdna     396 -aaagc--gtattcc----tgttgctttgattggagatgg
PAdxsdna     447 -ggagc--gtaagtc----ggtggccgtgatcggcgacgg
LEdxsdna     621 -aaaca--acaatgt----tattgccgtaataggtgatgg
MTdxsdna     398 -accgc--aaccggcatgtggtcgcggtggtcggtgacgg
RSdxs1dna    423 -cacgg--gcgacgc----ggtggcggtgatcggcgacgg
RSdxs2dna    432 -cgtgg--gcgacac----gatcgccgtgatcggcgacgg
SPCCdxsdna   403 -gacta--ccgatgt----g-tcgctgtgattggtgatgg
ECdxsdna     431 -gcc----gca---c----cgtctgtgtcattggcgatgg
NMdxsdna     415 -gaccg--ccgcagc----g-tcgccatcatcggcgacgg
HIdxsdna     423 tgcaggtagaaaaac----agtatgcgtaatcggtgatgg
SSdxsdna     402 -gaagg--gccatgt----cgtcgccgtcatcggcggacg
HPdxsdna     396 -gctag--gcatgcc----catagctttattaggcgatgg

STdxsdna     637 cgcgatgagcgcgggcatggcctatgaggcgatgaacaac
CRdxsdna     672 cgccatcaccgggggtatggcctatgaggccatgaaccat
CJdxsdna     429 tgctttaagtgcgggtatggcctatgaggctttaaatgaa
PAdxsdna     480 tgcgctgaccgccggcatggccttcgaggcactcaaccac
LEdxsdna     654 tgccatgacagcaggtcaagcttatgaagccatgaataat
MTdxsdna     435 tgcgctcaccggcggtatgtgctgggaggcgctgaacaat
RSdxs1dna    456 ctcgatgtcggccggcatggccttcgaggcgctgaaccac
RSdxs2dna    465 ctccatcaccgcgggcatggcctacgaggcactgaaccac
SPCCdxsdna   435 atcgctcaccggtggcatggccttggaagccatcaaccac
ECdxsdna     459 cgcgattaccgcaggcatggcgtttgaagcgatgaatcac
NMdxsdna     447 cgcgatgacggcgggtcaggcgtttgaagccttgaactgc
HIdxsdna     459 cgcaattactgcgggaatggcatttgaggcattaaatcac
SSdxsdna     435 ggcgctgaccggcggcatggcctgggaggccctgaacaac
HPdxsdna     429 gagcattagtgcagggatttttatgaagccttaaacga-
```

# Figure 5 (page 9 of 25)

```
STdxsdna      677 gccgaggcc--gccgg--caa--c-cggc------t--gg
CRdxsdna      712 gcgggcttc--ctgga--caa--g-aaca------t--ga
CJdxsdna      469 ttgggtgat--tctaa--att--t-cctt------g--cg
PAdxsdna      520 gcctcggaa--gtcga--cgc--c-gaca------t--gc
LEdxsdna      694 gc--tggtt--acctg--gac--t-ctgaca----t--ga
MTdxsdna      475 atc---gcc--gcatc--ccg--c-cggc------c--gg
RSdxs1dna     496 ggcgggcac--ctgaa--gaa--c-cggg------t--ga
RSdxs2dna     505 gc--gggcc--atctgaacaa--g-cgcc------t--gt
SPCCdxsdna    475 gctggtcacttgccca--aaa--cacggc------t--gt
ECdxsdna      499 ------gcg--ggcga--tat--c-cgtcctgatat--gc
NMdxsdna      487 gc--gggcg--atatg--gat--g-tgga------tttgc
HIdxsdna      499 gcgggggc----attg--cat--a-caga------tatgt
SSdxsdna      475 atcgcggcc--gccaa--gga--c-cagc------c--gc
HPdxsdna      468 -actgggcg--atagg--aaatac-ccca------t--ga

STdxsdna      702 tggtgatcct---c---aacgacaac-gaca---tgtcga
CRdxsdna      737 ttgtgattct---g---aacgacaac-cagcaggtgtcgc
CJdxsdna      494 taatactttt---a---aatgataat-gaaa---tgagta
PAdxsdna      545 tggtgatcct---c---aacgacaac-gaca---tgtcga
LEdxsdna      719 ttgttatctt---a---aacgacaatagaca---agtttc
MTdxsdna      497 tgattatcgtggtc---aacgacaat-gggc---gcagct
RSdxs1dna     521 tcgtgatcct---g---aacgacaac-gaga---tgagca
RSdxs2dna     530 tcgtgatcct---g---aacgacaat-gaca---tgagca
SPCCdxsdna    503 tggtcgtgct---c---aacgacaat-gaca---tgtcga
ECdxsdna      524 tggtgattct---c---aacgacaat-gaaa---tgtcga
NMdxsdna      512 tggtcgtcct---c---aacgacaac-gaaa---tgtcga
HIdxsdna      524 tagttatttt---a---aatgataac-gaaa---tgtcta
SSdxsdna      500 tgatcatcgt---cgtcaacgacaac-gagc---gctcct
HPdxsdna      494 tcatgatttt---a---aacgataat-gaaa---tgagta

STdxsdna      732 tcgccccgccg-----------------------gt---
CRdxsdna      770 tgcccacgcagtacaacaacaagaaccaggaccccgt---
CJdxsdna      524 tttcaaaacca-----------------------at---
PAdxsdna      575 tctcgcacaac-----------------------gt---
LEdxsdna      750 tttacctactg-----------------------ctact
MTdxsdna      530 acgcgcccaca-----------------------at---
RSdxs1dna     551 tcgcgccgccg-----------------------gt---
RSdxs2dna     560 tcgcgccgccc-----------------------gt---
SPCCdxsdna    533 tctcgcccaac-----------------------gt---
ECdxsdna      554 tttccgaaaat-----------------------gt---
NMdxsdna      542 tttcccccaac-----------------------gt---
HIdxsdna      554 tttcagaaaac-----------------------gt---
SSdxsdna      533 acgcgcccacc-----------------------at---
HPdxsdna      524 tcagcacgcct-----------------------at---
```

# Figure 5 (page 10 of 25)

```
STdxsdna     745 --gggcgggctttcggcctatcttgcgcgcctcatttcct
CRdxsdna     807 --gggcgccctgtccagcgccctggcgcgcctgcaggcca
CJdxsdna     537 --tggagcaatttcaaagtatctttctcaggctatggcaa
PAdxsdna     588 --cggcgggctctccaactacctggcgaagatcctctcca
LEdxsdna     766 ctggatggccagttgctcctgttggagctctaagtagtg
MTdxsdna     543 --cgggggcgtcgccgaccatctggccacgctg-------
RSdxs1dna    564 --gggggcgctgtcgtcctatctctcgcggctc-tatgcg
RSdxs2dna    573 --gggggcgcttgcgcgctatctcgtgaatctc---tcct
SPCCdxsdna   546 --gggtgcgctctctcgctatct-----gaataagattcg
ECdxsdna     567 --cggcgcgctcaacaaccatctggcacagctgctttcc-
NMdxsdna     555 --cggtgcgttgcccaaataccttgccagc-----aacgt
HIdxsdna     567 --tggtgcattaaataatcatcttgcgcg---tatttct
SSdxsdna     546 --cggcggcctcgccaaccacctggccaccctgcgcacca
HPdxsdna     537 --tggagccttatccaaagcccttagccagctga--tgaa

STdxsdna     783 c--gtc--------------cga-ata----t--------
CRdxsdna     845 a--ccg--------------gcc-cct----g--------
CJdxsdna     575 c--gca--------------gtt-tta----t--------
PAdxsdna     626 g--ccg--------------cac-cta----t--------
LEdxsdna     806 c--tttgagcaggttacagtcta-ataggcct--------
MTdxsdna     574 c--ggc--------------tgc-a---------------
RSdxs1dna    601 g--gcg--------------cgc-cgt----t--------
RSdxs2dna    608 c--gaa--------------ggc-gcc----c--------
SPCCdxsdna   579 g--gtt--------------ag------------------
ECdxsdna     604 g--gta--------------agc-ttt----a--------
NMdxsdna     588 c--gtg--------------cgcgata----tg-------
HIdxsdna     602 ctggct--------------ctc-ttt----a--------
SSdxsdna     584 c--cga--------------cgg-cta----cgagaaggt
HPdxsdna     573 a--ggc--------------ccg-ttt----t--------

STdxsdna     794 -ctcggc---c--tgc-gcga-gc---tcgcc--------
CRdxsdna     856 -cgcgag---c--tgc-gcga-ga---ttgcc--------
CJdxsdna     586 -caaagt---t--tta-aaaa-gcgtattgct--------
PAdxsdna     637 -agcagc---a--tgc-gcga-gg---gcagc--------
LEdxsdna     835 -ctcagagaac--taa-gaga-ag---tcgca--------
MTdxsdna     582 -gccggc---c--tac-gag-----------c--------
RSdxs1dna    612 -ccagga---c--ttc-aaggcgg---ccgcc--------
RSdxs2dna    619 -ttcgccacgc--tgc-gcgc-gg---ccgcc--------
SPCCdxsdna   585 --tgagc---cgatgc-agtt-gc---tcacc--------
ECdxsdna     615 -ctcttca--c--tgc-gcga-------------------
NMdxsdna     601 -cacgga---c--tgttgagt-ac---cgtca--------
HIdxsdna     615 -ctctacg--c--ttc-gtga-tg---gcagt--------
SSdxsdna     603 cctcgcc---t--ggg-gcaa-gg---acgtc--------
HPdxsdna     584 -accagt---c--ttt-ccgc-tc---taaagttaaaaa
```

# Figure 5 (page 11 of 25)

```
STdxsdna      815 -----aagcg----------cttcac-----cc-------
CRdxsdna      877 -----aaggg----------cgtgac-----ca-------
CJdxsdna      610 -----aaaat----------gtt-----------------
PAdxsdna      658 -----aagaa----------ggt-----------------
LEdxsdna      859 -----aaggg----------agttac-----ta-------
MTdxsdna      596 -----aggcg----------ctggagacgggcc-------
RSdxs1dna     634 -----aagggagcgctcgggcttctg-----cc-------
RSdxs2dna     643 -----gacgg----------gctcga-----gg-------
SPCCdxsdna    607 -----gatgg----------tttgac-----ccaggggat
ECdxsdna      630 -----aggcg----------ggaaaa-----aa-------
NMdxsdna      623 -----aagcg----------c-aaac-----gg-------
HIdxsdna      637 -----aaaaa----------aatc----------------
SSdxsdna      625 -----ctgct----------gcgtac-----cc-------
HPdxsdna      613 atcttaagca----------ccttac-----ct-------

STdxsdna      828 gcaag------ctttcg----cgccgc---c--tcaccgc
CRdxsdna      890 agcag------ctgcct----gacgtt---g--tccagaa
CJdxsdna      618 ggata------tatt----------gc---c--tgatagt
PAdxsdna      666 ----g------ctctcg----cgcctg---c--ccggggc
LEdxsdna      872 agcag------attggt----ggtcct---a--tgcatga
MTdxsdna      614 gcgac------ctggtg----cgc-gc---g--gtgccgc
RSdxs1dna     657 cgaac------cgttcc----aggagggcgc--gcgccgc
RSdxs2dna     656 cctcg------ctgccg----gggccg---c--tccgcga
SPCCdxsdna    627 gcaacaaattcccttcgtcggcggcgc---cattaccca a
ECdxsdna      643 gtttt------ctctgg----cgtgcc---g--ccaatta
NMdxsdna      635 gcaag------gtatta----gacaaa---a--tacccgg
HIdxsdna      646 -cttg------ataaag----ttcctc---caatcaaaaa
SSdxsdna      638 ccatc------gtcggc----cacccc---c--tctacga
HPdxsdna      631 gaaag------cgt----------ga---a--ttactta

STdxsdna      853 --g-gc----agccggcaaggcg----g-----aggaa--
CRdxsdna      915 --g-gc----aactgctaagatt----g-----acgag--
CJdxsdna      637 ----gc----tacttatatggcc----a-----agcgt--
PAdxsdna      687 ctg-gg----agatcgcccggcgcaccg-----aggaa--
LEdxsdna      897 --g-ct----tgctgcaaaagtt----g-----atgaa--
MTdxsdna      638 --ttgt----cggcggtctgtgg----t-----ttcga--
RSdxs1dna     685 --g-cc----a--aggagatgct----g-----aaga---
RSdxs2dna     681 --c-gg----ggcgcgccgggcg----c-----gccag--
SPCCdxsdna    664 --g-gctttgagccggttaag-g----a-----aggca--
ECdxsdna      668 --a-ag----agctgctcaaacgcaccg-----aagaa--
NMdxsdna      660 --c-gc----gatggagtttgcc----c-----aaaaa--
HIdxsdna      672 --t-tt----tatgaaaaaacc----g-----aagaaca
SSdxsdna      663 --g-gc------cctgcacggcg----ccaagaagggc--
HPdxsdna      649 --g-cg----agtcg----tttt----g-----aagaa--
```

# Figure 5 (page 12 of 25)

```
STdxsdna     875 t--------tcgcccgcggcatg----g----cg------
CRdxsdna     937 t--------atgctcgcggcatgatcag----cggc----
CJdxsdna     658 t--------ttgaagagagtttt----a----aacttatt
PAdxsdna     715 t--------acgccaagggcatg----c----tg------
LEdxsdna     919 t--------atgctcgtggcatg----a----tt------
MTdxsdna     661 t--------tcctgcacagcgtc----a----ag------
RSdxs1dna    704 ------------------gcgtc----a----cc------
RSdxs2dna    703 c--------tcgtgaccgggatg----c----cg------
SPCCdxsdna   689 tgaagcgcctctcctacagcaag-----------------
ECdxsdna     694 c--------atattaaaggcatg----g----ta------
NMdxsdna     682 g--------tcgaacataaaatc----a----aa------
HIdxsdna     696 t--------atgaaaggtgtaat----gttttcg------
SSdxsdna     688 t--------tcaaggacgccttc----g----cc------
HPdxsdna     667 t--------ctttcaagctcat-----------c------

STdxsdna     893 a------ccg-----------gcggcacg-----------
CRdxsdna     961 a------ctg-----------gctccacg-----------
CJdxsdna     682 a------ccc-----------ctgggctt-----------
PAdxsdna     733 g------tcc-----------ccggcacc-----------
LEdxsdna     937 agtggttctg-----------gatcaaca-----------
MTdxsdna     679 g------ccg-----------gcatcaaggactcgctgtc
RSdxs1dna    712 g------tcg-----------gcggcacg-----------
RSdxs2dna    721 g------gcg-----------ggggcacg-----------
SPCCdxsdna   712 ---------a-----------ttggggcg-----------
ECdxsdna     712 g------tgc-----------ctggcacg-----------
NMdxsdna     700 a------cccttgccgaagaagccgaaca-----------
HIdxsdna     718 c------cag-----------aaagtaca-----------
SSdxsdna     706 c------cgc-----------agggca-------------
HPdxsdna     682 a------ccc-----------cgggcgtg-----------

STdxsdna     905 ----------c-------------------tgttcgagga
CRdxsdna     973 ----------c-------------------tgtttgagga
CJdxsdna     694 ----------t-------------------tgtttgaaga
PAdxsdna     745 ----------c-------------------tgttcgagga
LEdxsdna     955 ----------t-------------------tgtttgaaga
MTdxsdna     702 gccgcagttgc-------------------tgttcaccga
RSdxs1dna    724 ----------c-------------------tcttcgagga
RSdxs2dna    733 ----------c-------------------tcttcgagga
SPCCdxsdna   721 ----------g-------------------tctttgaaga
ECdxsdna     724 ----------t-------------------tgtttgaaga
NMdxsdna     723 ----------cgccaaacagtcactgtctttgtttgaaaa
HIdxsdna     730 ----------t-------------------tatttgaaga
SSdxsdna     716 ------------------------------tgttcgagga
HPdxsdna     694 ----------t-------------------tttttgaaga
```

# Figure 5 (page 13 of 25)

```
STdxsdna      916 acttggcttctattatgtcggcccgatcgacggccacaat
CRdxsdna      984 gctgggcctgtactacatcggccctgtggacggccacaac
CJdxsdna      705 attagggcttgaatatatagggcctattgatggacataat
PAdxsdna      756 gctcggctggaattacatcggcccgatcgacggccacgac
LEdxsdna      966 acttggactttactatattggtcctgtggatggtcacaac
MTdxsdna      723 cctcgggttgaagtacgtcggcccggtcgacggcca---t
RSdxs1dna     735 gctgggtttctcctatgtcggcccgatcgacgggcacgat
RSdxs2dna     744 gctgggcttcacctatgtcggccccatcgacggccacgac
SPCCdxsdna    732 gctgggcttcacctacatggggccagtggatggtcacaac
ECdxsdna      735 gctgggctttaactacatcggcccggtggacggtcacgat
NMdxsdna      753 cttcggcttccgctataccggccccgtggacggacacaac
HIdxsdna      741 actcggttttaactatattggcccagtggatgggcataac
SSdxsdna      726 cctgggcctgaagtacgtcggccccatcgacgggcacgac
HPdxsdna      705 attaggcattaactatatagggcctattaatgggc-----

STdxsdna      956 ctcgagcatctgatcccggtgctggagaatgtcc-g----
CRdxsdna     1024 ctggacgacctcatcgccgtgctcagcgaggtgc-g----
CJdxsdna      745 ttaggtgaaattat-----ttctgcattaaaacaag----
PAdxsdna      796 ctgccgaccctggtggctaccctgcgcaacatgc-g----
LEdxsdna     1006 attgatgatctaattgcgattctcaaagaggtta-gaagt
MTdxsdna      760 gacgag---------cgggcggtggaggtcgcgc-t----
RSdxs1dna     775 ctcgaccagcttctgccggtgctgcggaccgtca-a----
RSdxs2dna     784 atggaggcgctcctccagacgctgcgcgcggcgc-g----
SPCCdxsdna    772 cttgaagaactgatc---------gccaccttcc-g----
ECdxsdna      775 gtgctggggcttatcaccacgctaaagaacatgc-g----
NMdxsdna      793 gtcgaaaatctggtcgatgtattggaagacctgc-g----
HIdxsdna      781 attgatgaattagtggctacgcttacgaatatgc-g----
SSdxsdna      766 atcggcgcggtcgagtccgcgctgc------gcc-g----
HPdxsdna      740 ----atgatttgagcgcgattattgaaaccttaa-a----

STdxsdna      991 -c---gaca---gcga-gc---a---g-------ggc---
CRdxsdna     1059 -c---agcg---ccga-ga---ccgtg-------ggc---
CJdxsdna      776 -c---aaaa---gctatgc---a---a-------aag---
PAdxsdna      831 -c---gaca------t-ga---a---g-------ggc---
LEdxsdna     1045 ac---taaa---ac-a-ac---a---g-------gtc---
MTdxsdna      786 -g---cgca---gcgc-gc---g---g-------cgcttc
RSdxs1dna     810 -g---cagc---gggc-gc---a---t-------gcg---
RSdxs2dna     819 -g---gccc---g-ga-cc---ac--g-------ggg---
SPCCdxsdna    798 -c---ga-a---gcgc-ac---a---aacacaccgga---
ECdxsdna      810 -c---ga------cct-ga---a---a-------ggc---
NMdxsdna      828 -c---ggac---gc----a---a---a-------ggc---
HIdxsdna      816 -------ta---atct-ga---a---a-------ggc---
SSdxsdna      795 -c---gccaagcgctt-cc---a---c-------ggg---
HPdxsdna      771 -attagcca---aaga-gcttaa---a-------gag---
```

# Figure 5 (page 14 of 25)

```
STdxsdna     1007 ------ccgatcctgatccatgtcgtgaccaagaagggca
CRdxsdna     1078 ------ccggtgctggtgcacgtggtaacggagaagggcc
CJdxsdna      793 ------ccttgtgtgatacatgctcaaaccataaagggta
PAdxsdna      844 ------ccgcagttcctccatgtggtgaccaagaaaggca
LEdxsdna     1061 ------cag-tactgatccatgttgtcactgagaaaggca
MTdxsdna      805 ggtgcaccggtgatcgtgcacgtcgtcacccgcaagggca
RSdxs1dna     826 ------ccggtgctgatccatgtcatcaccaagaagggca
RSdxs2dna     835 ------ccggtgctcatccatgtggtcacgaagaagggca
SPCCdxsdna    820 ------ccagtactcgtccacgttgccacaaccaagggta
ECdxsdna      823 ------ccgcagttcctgcatatcatgaccaaaaaaggtc
NMdxsdna      841 ------ccgcagcttctgcacgtcatcaccaaaaagggca
HIdxsdna      829 ------ccacaattttgcatataaaaacgaaaaaaggta
SSdxsdna      814 ------ccggtgctggtgcactgcctcaccgtcaagggcc
HPdxsdna      793 ------ccggtgctaatccatgcgcaaaccttaaagggca

STdxsdna     1041 agggctatgccccggccgaagcg---gcggcggacaagta
CRdxsdna     1112 gcggctacctgcccgccgagacg---gcgcaggacaagat
CJdxsdna      827 aaggctatgctttagctgaagga---aaacatgctaaatg
PAdxsdna      878 agggcttcgccccggccgaactg---gatccgatcggcta
LEdxsdna     1094 gaggttatccatatgctgagaga---gctgcagataagta
MTdxsdna      845 tgggctacccgccggccga---------ggccgac-----
RSdxs1dna     860 ggggctatgctccggccgaggcc---gcgcgcgaccgtgg
RSdxs2dna     869 agggttacgcccccgccgagaat---gcccccgacaagta
SPCCdxsdna    854 agggctatccctacgctgaagaa---gatcaggttggcta
ECdxsdna      857 gtggttatgaaccggcagaaaaa---gacccgatcacttt
NMdxsdna      875 acggctacaaactcgccgaaaac---gatcccgtcaaata
HIdxsdna      863 aaggatacgcacccgcagaaaaa---gatccgattggttt
SSdxsdna      848 gcggctacgaacccgccctcgcccacgaggaggaccactt
HPdxsdna      827 aaggctataagatcgctgaaggg---cgctatgaaaaatg

STdxsdna     1078 tcacggcgtccagaag--tt--cgacgt----gatc-acc
CRdxsdna     1149 gcacggtgtggtcaag--tt--cgaccc----ccgc-acc
CJdxsdna      864 gcacggggtgggagcc--tt--tgatat----agat-agt
PAdxsdna      915 ccacgcgatcaccaag--ct--gga--------agc-tcc
LEdxsdna     1131 tcatggagttgccaag--tt--tgatcc----agca-aca
MTdxsdna      871 -caggccgagcagatgcatt--ccacggtcccgatcgatc
RSdxs1dna     897 ccatgccacgaacaag--tt--caacgt----cctg-acc
RSdxs2dna     906 tcacggggtgaacaag--tt--cgaccc----cgtc-acg
SPCCdxsdna    891 tcatgcccaaaatccc--tt--tgatct----ggcg-aca
ECdxsdna      894 ccacgccgtgcctaaa--tt--tgatcc----ctcc-agc
NMdxsdna      912 ccacgccgtcgccaac--ctgcctaaag----aaag-cgc
HIdxsdna      900 ccacggtgtacctaaa--tt--tgatcc----aatc-agt
SSdxsdna      888 ccacaccgtcggcgtg--at--ggaccc----gctc-acc
HPdxsdna      864 gcatggggtggggcct--tt--tgattt----ggat-acc
```

# Figure 5 (page 15 of 25)

```
STdxsdna     1109 ggggcacaggcc-----aaggcaccc------ccgggcc-
CRdxsdna     1180 ggcaagcaggtg-----caggccaag------acgaagg-
CJdxsdna      895 ggagagagtgtt-----aaaaaaagt------gatacta-
PAdxsdna      942 cggcagtgcgcc-----gaagaagac------c--ggcg-
LEdxsdna     1162 ggaaagcaattc-----aaag----c------cagtgcca
MTdxsdna      908 cggccaccggac-----aagccacca------aggtggc-
RSdxs1dna     928 ggcgcgcaggtg-----aagccggtc------tcgaacg-
RSdxs2dna     937 ggcgagcagaag-----aagtcggtg------gccaacg-
SPCCdxsdna    922 ggg---aaggct-----aaaccagcttcaaaaccgaagc-
ECdxsdna      925 ggttgtttgccg-----aaaagtagc------ggcggtt-
NMdxsdna      945 ggcgcaaatgccgtctgaaaaagaac------ccaagcc-
HIdxsdna      931 ggcgaattgccc-----aa---aaac------aatagta-
SSdxsdna      919 tgtg---agccc-----ctctcgccc------accgacg-
HPdxsdna      895 gg---cttgtct-----aaaaaatcc------aaaag---

STdxsdna     1137 ---cgccc--gc--------ctat-----accaaggtgtt
CRdxsdna     1208 ---ccatg--tc--------gtac-----acgaactactt
CJdxsdna      923 ---aaaaa--tc--------tgct-----actgaaatttt
PAdxsdna      968 ---gaccc--aa--------gtat-----tccagcgtctt
LEdxsdna     1187 agacacag--tc--------ctat-----acaacatattt
MTdxsdna      936 ---cggcccagg--------ctgg-----acggcgacctt
RSdxs1dna     956 ---ccccc--tc--------ctat-----accaaggtctt
RSdxs2dna     965 ---cgccg--aa--------ctac-----accaaggtctt
SPCCdxsdna    953 ---cgcct--ag--------ctat-----tccaaagtgtt
ECdxsdna      953 ---tgccg--ag--------ctat-----tcaaaaatctt
NMdxsdna      978 ---cgccg--ccaaaccgacctat-----acccaagtgtt
HIdxsdna      956 ---aacca--ac--------ttat-----tcgaaaatttt
SSdxsdna      944 ---gcccg--tc--------ctgg-----acctcggtgtt
HPdxsdna      918 ---cgcaa--tc--------ttatcgcccactgaagcgta

STdxsdna     1159 cgccgatgcgctgctcgc-cgaagcgg-agcgtgatgcgt
CRdxsdna     1230 cgcggacgcgctgacggc-ggaggcgg-agcgcgacagcc
CJdxsdna      945 ttctaagaatttgcttga-tttagcct-caaaatatgaaa
PAdxsdna      990 cggccagtggctgtgcga-catggccg-cccaggacgcg-
LEdxsdna     1212 tgccgaggctttaattgc-agaagcag-aagcagataaag
MTdxsdna      960 ctctgatgcacttatcgg-ctacgc-----ccagaaacgc
RSdxs1dna     978 cgcccagagcctcatcaa-ggaggccg-aggtcgacgagc
RSdxs2dna     987 cggctccaccctgaccga-ggaggccg-cgcgcgatccgc
SPCCdxsdna    975 tggccaaaccctgacgac-cttggcca-agagcgat-cgc
ECdxsdna      975 tggcga----ctggttgtgcgaaacggcagcgaaagacaa
NMdxsdna     1008 cggcaaatggctgtgcga-ccgggcgg-cggcagattc--
HIdxsdna      978 tggcgattggctatgtga-aatggcag-aaaaagatgcca
SSdxsdna      966 cggcgacgagatcgt--a-cggatcgg-cgcggagcgcga
HPdxsdna      945 ttctaacaccctttaga-attagcta-aaaaagatgaaa
```

# Figure 5 (page 16 of 25)

```
STdxsdna     1197 cgg----tctg-c--gcg--atcaccgcggcgatgccctc
CRdxsdna     1268 gca----tcgt-g--gcg--gtgcacgcggccatggcggg
CJdxsdna      983 ata----ttgt-t--ggg--gttacggcggctatgccaag
PAdxsdna     1027 -cg----cctg-c--tcggcatcaccccggcgatgaagga
LEdxsdna     1250 aca----ttgt-t--gca--atccatgctgccatgggggg
MTdxsdna      994 cgtgacatcgt-g--gcc--attaccgcggccatgccggg
RSdxs1dna    1016 gga----tctg-c--gcg--gtgacggccgccatgccgga
RSdxs2dna    1025 gca----tcgt-g--gcg--atcaccgccgctatgccctc
SPCCdxsdna   1012 cgc----attgtc--ggg--attacggctgcgatggcgac
ECdxsdna     1011 caa----gctg-atggcg--attactccggcgatgcgtga
NMdxsdna     1044 ccg----actg-gttgcg--attaccccgccatgcgcga
HIdxsdna     1016 aaa----ttat-a--ggt--atcacacctgcaatgcgtga
SSdxsdna     1002 ggaca--tcgt-c--gcg--atcaccgccgcgatgctc--
HPdxsdna      983 aaa----tcgt-a--ggc--gtaaccgcggcgatgcctag

STdxsdna     1228 gggcacc-----gggctcg-acaagttccaggcgacg--t
CRdxsdna     1299 cggcacc-----ggcctgt-accggttcgagaagaag--t
CJdxsdna     1014 tggaaca-----ggtcttg-ataagcttatagaaaaa--t
PAdxsdna     1059 aggttcc-----gacctgg-tggcctt-cagcgaacg--t
LEdxsdna     1281 tgggacc-----ggaatga-acctttcca-tcgtcg--c
MTdxsdna     1029 ccccacc-----gggctga-ccgcgttcgggcagcgc--t
RSdxs1dna    1047 cgggacg-----gggctca-acctcttcggcgagcgg--t
RSdxs2dna    1056 gggcacc-----ggcgtcg-acatcatgcagaagcgt--t
SPCCdxsdna   1044 aggcacc-----ggcttgg-acattctccagaaggcg--c
ECdxsdna     1044 aggttcc-----ggcatgg-tcgagttttcacgtaaa--t
NMdxsdna     1077 gggcagc-----ggcttgg-ttgagtttga-acaacga-t
HIdxsdna     1047 gggttca-----ggtatggtagaatttc--ccaacgc-t
SSdxsdna     1033 ---cacccggtggggctcg-ccaggttc--gccgaccgct
HPdxsdna     1014 cggcaca-----ggattag-acaaactcattgacgct--t

STdxsdna     1260 tccccg-atc-gcaccttcgacgtcgctatcgccgagcag
CRdxsdna     1331 tcccgg-acc-gcacctttgacgtgggcattgcggagcag
CJdxsdna     1046 atccaa-atc-gtttttgggatgtggctattgcagaacag
PAdxsdna     1090 tatccggaac-gctacttcgacgtcgccatcgccgaacag
LEdxsdna     1312 ttccca-acaaggtgttttgatgttggaatagcagaacaa
MTdxsdna     1061 tcccgg-atc-gattgttcgacgtcgggatcgccgagcaa
RSdxs1dna    1079 ttccga-agc-gcaccttcgatgtgggcatcgcggaacag
RSdxs2dna    1088 tcccga-acc-gcgtcttcgacgtgggcatcgccgagcag
SPCCdxsdna   1076 tgccga-agc-aatacatcgatgttggcattgccgaacag
ECdxsdna     1076 tcccgg-atc-gctacttcgacgtggcaattgccgagcaa
NMdxsdna     1109 tccccg-acc-gctatttcgatgtcggcatcgccgagcag
HIdxsdna     1079 tcccaa-aac-aatattttgacgtagcgattgcagaacag
SSdxsdna     1067 tcccgg-acc-gggtctgggacgtcggcatcgccgagcag
HPdxsdna     1046 accctt-tgc-gctttttgatgtcgctatcgctgagcaa
```

# Figure 5 (page 17 of 25)

```
STdxsdna     1298 cacgcggtcacct-tcgcagcgggccttgccgcgcagggg
CRdxsdna     1369 cacgccgtgacct-ttgctgccggcctggcgtgcgagggc
CJdxsdna     1084 catgcagtaactt-ctatggccgctatggcaaaagaagga
PAdxsdna     1129 catgccgtgaccc-tggccgccggcatggcctgcgagggc
LEdxsdna     1351 catgcagtaacct-ttgctgctggattggcttgtgaaggc
MTdxsdna     1099 cacgcgatgacgt-cggcggccgggttggcgatgggtggg
RSdxs1dna    1117 catgcggtgacct-tctcggcggcgcttgcggcaggcggc
RSdxs2dna    1126 catgccgtgacct-tcgcggccggcctcgccggggccggg
SPCCdxsdna   1114 cacgccgtggtgc-tagctgccggtatggcctgcgatggc
ECdxsdna     1114 cacgcggtgacct-ttgctgcgggtctggcgattggtggg
NMdxsdna     1147 cacgccgttacct-ttgccggcggtttggcttgcgaaggg
HIdxsdna     1117 cacgctgtcacgt-ttgccacaggacttgcaattggcgga
SSdxsdna     1105 cacgcggccgtgt-ccgcggccgggctcgccaccggcgga
HPdxsdna     1084 cacgctttaacttctagcagc--gctatggctaaagaggg

STdxsdna     1337 atgcggccgttctgcgcg-atctactcgaccttcctgcag
CRdxsdna     1408 ctggtgcccttctgcacc-atctacagtaccttcatgcag
CJdxsdna     1123 tttaaacctttattgca-atatatagcaccttttttgcag
PAdxsdna     1168 atgaagccggtggtagcg-atctactcgaccttcctccag
LEdxsdna     1390 attaaacctttctgtgca-atctattcgtctttcatgcag
MTdxsdna     1138 ctgcaccccgtggtggcg-atctactcgacgttcctgaac
RSdxs1dna    1156 atgcggcccttctgcgcc-atctattccaccttcctccag
RSdxs2dna    1165 atgaagcccttctgcgcg-atctattcctcgttcctgcaa
SPCCdxsdna   1153 atgcgtccggtggtggca-atctattccaccttcctgcag
ECdxsdna     1153 tacaaacccattgtcgcg-atttactccactttcctgcaa
NMdxsdna     1186 atgaagcccgtcgtggcg-atttattccacctttttacaa
HIdxsdna     1156 tataaacctgtcgtcgca-atttactcgacatttttacaa
SSdxsdna     1144 ctgcacccggtcgtcgcc-gtctacgccaccttcctcaac
HPdxsdna     1122 gtttaaaccttttgtgagcatctattctactttttgcag

STdxsdna     1376 cgcgcctacgaccaggtcgtccacgacgtcgcgatccaga
CRdxsdna     1447 cgcggttacgaccagatcgtgcacgacgtgtccctgcaga
CJdxsdna     1162 cgtgcttatgatcaagtgatccatgattgtgcgattatga
PAdxsdna     1207 cgcgcctacgaccagttgatccatgacgtcgccgtgcagc
LEdxsdna     1429 agggcttatgaccaggtagtgcatgacgttgatttgcaaa
MTdxsdna     1177 cgggcgttcgaccagatcatgatggatgtggcgctgcaca
RSdxs1dna    1195 cgcggctacgaccagatcgtgcatgacgtggcaatccagc
RSdxs2dna    1204 cggggttacgaccagatcgcccatgacgtggcgctgcaga
SPCCdxsdna   1192 cgggcctttgatcaagtcatccacgacgtttgtatccaaa
ECdxsdna     1192 cgcgcctatgatcaggtgctgcatgacgtggcgattcaaa
NMdxsdna     1225 cgcgcctacgaccaactggtgcacgacatcgccctgcaaa
HIdxsdna     1195 cgtgcttacgatcaattaattcacgatgttgccattcaaa
SSdxsdna     1183 cgcgccttcgaccagctcctgatggacgtcgc---cctgc
HPdxsdna     1162 agggcttatgattctattgtgcatgacgcttgtatttcta
```

# Figure 5 (page 18 of 25)

```
STdxsdna      1416 acc--tgccg-gtccgcttcgcgatcgaccgcgcgggcct
CRdxsdna      1487 agc--tgcct-gtgcgcttcgctatggaccgcgctggcct
CJdxsdna      1202 att--taaat-gtggtttttgctatggatagggcagggat
PAdxsdna      1247 acc--tcgac-gtgctgttcgccatcgaccgcgccggcct
LEdxsdna      1469 agc--tgccc-gtgaggtttgcaatggacagagcaggtct
MTdxsdna      1217 agc--tgccg-gtcaccatggtgctggaccgtgccgggat
RSdxs1dna     1235 gcc--tgccg-gtgcgctttgccatcgaccgcgccggcct
RSdxs2dna     1244 acc--ttccc-gtccgcttcgtgatcgaccgggcggggct
SPCCdxsdna    1232 agc--tgccc-gtcttcttctgcctcgatcgcgcggggat
ECdxsdna      1232 agc--ttccg-gtcctgttcgccatcgaccgcgcgggcat
NMdxsdna      1265 acc--tgccc-gttttgtttgccgtcgaccgcgcgggcat
HIdxsdna      1235 atc--tccct-gtgctatttgcaattgatcgagcagggat
SSdxsdna      1220 accgctgcggtgtgaccttcgtcctggaccgggccggcgt
HPdxsdna      1202 gct--tgccg-attaaattagccattgacagggctgggat

STdxsdna      1453 ggtcggtgccgacggcgcgacccatgccggcagcttcgac
CRdxsdna      1524 ggtgggcgctgacggctccacgcactgcggcgccttcgac
CJdxsdna      1239 agtaggcgaagatggggagacgcatcaaggtgtttttgat
PAdxsdna      1284 ggtcggcgaggacggcccgacccacgccggtagcttcgac
LEdxsdna      1506 tgttggagcagatggtccaacacattgtggtgcatttgat
MTdxsdna      1254 caccggtagcgacggcgccagccacaacggaatgtgggac
RSdxs1dna     1272 cgtggggggcggacggcgccacccatgcgggctcgttcgat
RSdxs2dna     1281 cgtggggggccgatggcgcgacccatgcgggggccttcgac
SPCCdxsdna    1269 agttggcgcggatggcccgactcaccaaggcatgtacgac
ECdxsdna      1269 tgttggtgctgacggtcaaacccatcagggtgcttttgat
NMdxsdna      1302 cgtcggcgcggacggcccgacccatgccggtttgtacgat
HIdxsdna      1272 agttggtgcagatggggctacacatcaaggtgcattcgat
SSdxsdna      1260 cacgggcgtcgacggcgcctcgcacaacggcatgtgggac
HPdxsdna      1239 tgtgggcgaagatggcgagacgcaccaagggcttttagac

STdxsdna      1493 gtgacctatctcgccagcctgcccaatttcgtggtgatgg
CRdxsdna      1564 gtgacgttcatggcgtcgctgccgcacatgatcaccatgg
CJdxsdna      1279 cttagtttttagctcctttgccaaatttcactcttttag
PAdxsdna      1324 atctcctacctgcgctgcatccccggcatgctggtgatga
LEdxsdna      1546 gttacttacatggcatgtcttcctaacatggttgtaatgg
MTdxsdna      1294 ttgtcgatgctgggtatcgtgcccggcatccgggtggcag
RSdxs1dna     1312 gtggccttcctgtcgaacctgcccggcatcgtggtgatgg
RSdxs2dna     1321 gttggcttcatcacttcgctgcccaacatgaccgtgatgg
SPCCdxsdna    1309 attgcttacctgcggctgattcccaacatggtgctgatgg
ECdxsdna      1309 ctctcttacctgcgctgcataccggaaatggtcattatga
NMdxsdna      1342 ttaagctttttgcgctgcattccgaat---atgattgtcg
HIdxsdna      1312 attagctttatgcgttgcattccaaatatgatcattatga
SSdxsdna      1300 atgtccgtcctccaggtcgtgccc---ggcctcaggatcg
HPdxsdna      1279 gtgtcgtatttgcgctctatccctaa---catggtcatt
```

# Figure 5 (page 19 of 25)

```
STdxsdna      1533 cggccgcggacgaggtcgag-ctcgtccacatg--accca
CRdxsdna      1604 ctccctcgaacgaggcggag-ctcatcaacatg--gtggc
CJdxsdna      1319 c-----cccaagagat--------gaacaaatg--atgca
PAdxsdna      1364 cccccagcgacgaggacgag-ctgcgcaagctg--ctcac
LEdxsdna      1586 ctccttctgatgaagcggag-ctatttcacatggtagcaa
MTdxsdna      1334 cgcccagagacg-----cca-cccggttgcgtg--aagaa
RSdxs1dna     1352 ccgccgccgacgaggccgag-ctcgtccatatg--gtagc
RSdxs2dna     1361 ccgcggccgacgaggccgag-ctcatccacatg--atcgc
SPCCdxsdna    1349 caccgaaagatgaggccgaa-c---tgcagcgg--atgct
ECdxsdna      1349 ccccgagcgatgaaaacgaa-tgtcgccagatg--ctcta
NMdxsdna      1379 ccgcgccgagcgatgaaaat-gaatgccgcctg--ctgct
HIdxsdna      1352 cgccgagtgatgaaaatgaa-tgccgtcaaatg--ctcta
SSdxsdna      1337 ccgccccgcgcgacgccgac-cacgtgcgcgcc--cagct
HPdxsdna      1316 ttgccccacgagacaatgagactttaaaaaacg--ccgtg

STdxsdna      1570 ca-cg---gcg--g--cga--tg--cacg-------acag
CRdxsdna      1641 ca-cctgcgcc--g--cca--tc--gacg-------ac--
CJdxsdna      1344 aa-at---ata--a--tgg--ag--tatgcttatttacat
PAdxsdna      1401 ca-c-----cg--g--ctacctg--ttcg-------a---
LEdxsdna      1625 ctgct---gcc--g--cca--tt--gatg-------aca-
MTdxsdna      1366 ct-cg---gcgagg--cgc--tc--gacgtcg----acga
RSdxs1dna     1389 ca-cc---gcc--g--ccg--cc--catg-------acga
RSdxs2dna     1398 ca-cc---gcc--g--tgg--cc--ttcg-------gcga
SPCCdxsdna    1383 ag-tg---acg--g--gta--tt--gaat-------acga
ECdxsdna      1386 ta-c-----cg--g--cta--t---cact-------ataa
NMdxsdna      1416 tt-cg---acc--t--gct--at--cagg-------caga
HIdxsdna      1389 ta-ca---ggt--tatcaa--tg--tgga-------aaac
SSdxsdna      1374 gc-gg---gag--g--cgg--tc--gccg-------tgga
HPdxsdna      1354 cg-tt---ttg--c--caa--tgaacacg-------attc

STdxsdna      1591 c--g---gcccgatcgcgctgc-gctatccacgcggcaac
CRdxsdna      1663 -------gcgccctcgtgcttccgcttcccccgcggcaac
CJdxsdna      1372 caag---gacctattgctttgc-gttatcctag-----ag
PAdxsdna      1419 t--g---gcccggccgcggtgc-gctatccgcgcggcagc
LEdxsdna      1646 -------gaccaagttgtttta-gatacccaagaggaaat
MTdxsdna      1392 c--g---gcccgacggcgttac-ggttccc------caaa
RSdxs1dna     1410 a--g---ggcccatcgccttcc-gctatccgcgcggcgac
RSdxs2dna     1419 g--g---gccccatcgccttcc-gcttcccgcggggcgag
SPCCdxsdna    1404 c--g---gcccgatcgccatgc-gtttcccgcgcgggaat
ECdxsdna      1404 c--gatggcccgtcagcggtgc-gctacccgcgtggcaac
NMdxsdna      1437 c--g---cgcccgccgccgtcc-gctatccgcgcggcacg
HIdxsdna      1412 c--t---gc-----ggcagtgc-gctaccctcgcggaaat
SSdxsdna      1395 c--g---acgcgccgacgctg-----atccgcttcccgaa
HPdxsdna      1377 a--a---gcccttgcgcgttcc-gatacc-------ctag
```

# Figure 5 (page 20 of 25)

```
STdxsdna     1625 ggcg---tcggactggc-gctgccc---------------
CRdxsdna     1696 ggcc---tgggcctgga-cctggccgcctacggcatcagc
CJdxsdna     1403 ggag---ttttattttg-gataaag---------------
PAdxsdna     1453 ggcc---ccaaccatcc-gatcgat---------------
LEdxsdna     1678 ggga---tcggtgtaga-gcttccg---------------
MTdxsdna     1420 ggtgatgtgggagaaga-tatttc----------------
RSdxs1dna    1444 ggcg---tgggggtcga-ggtgccg---------------
RSdxs2dna    1453 gggg---tgggcgtcga-gatgccc---------------
SPCCdxsdna   1438 ggta---ttggcgtacc-cctgccggaag-----------
ECdxsdna     1441 gcgg---tcggcgtgga-actg------------------
NMdxsdna     1471 ggta---cgggcgtgcc-ggtttca---------------
HIdxsdna     1441 gccg---ttggtgtaaa-act---t---------------
SSdxsdna     1425 ggag---tccgtcggcccgcggatc---------------
HPdxsdna     1404 gggg---tcg--tttgc-gttaaaa---------------

STdxsdna     1646 aa-gg---t--tccggag-----c------ggctg-----
CRdxsdna     1732 aa-gg---a--cctgaag-----g------gtgtgcccct
CJdxsdna     1424 aa-tt---taatccttgt-----g------agata-----
PAdxsdna     1474 cc-gg---a--cctgcaa-----c------cggtg-----
LEdxsdna     1699 gctgg---a--aacaaaggaattc------ctctt-----
MTdxsdna     1443 ---gg---c--tttggag-----c------ggcgt-----
RSdxs1dna    1465 gt-ga---a--gggcgtg-----c------cgctc-----
RSdxs2dna    1474 ga-gc---g--cgggacg-----g------tgctg-----
SPCCdxsdna   1463 aa-gg---c--tg-ggag-----t------cgctc-----
ECdxsdna     1459 ac-gc---c--gctggaa-----a------aacta-----
NMdxsdna     1492 ga-cg---g--catggaa-----a------ccgtg-----
HIdxsdna     1459 ac-tc---c--tttagaa-----a------tgctt-----
SSdxsdna     1447 cc-gg---c--cctcgac-----c------gggtc-----
HPdxsdna     1423 ga-gggggt--ttttgag-----cctagcggtttt-----

STdxsdna     1664 -gaaatcggcaagggtc--gcgtggtccga----gag---
CRdxsdna     1755 cgaggtgggcaagggtg--ttgtccgccgc----cag---
CJdxsdna     1444 -aaacttggtaagg------------cac----aat---
PAdxsdna     1492 -gagatcggcaagg-----gcgtggtccgt----cggcgc
LEdxsdna     1723 -gaggttggtaaaggta--ggatattgatt----gag---
MTdxsdna     1459 -ggaggcgtggatgtgctggcggcgcccgcc---gat---
RSdxs1dna    1483 -cagatcggccgtggcc--gggtggtgagc----gag---
RSdxs2dna    1492 -gagcccggccggggcc--gcgtggtgcgc----gaa---
SPCCdxsdna   1480 -ccgattgggaaagcag--agcaactgcgc----caa---
ECdxsdna     1477 -ccaattggcaaaggca--ttgtgaagcgt----cgt---
NMdxsdna     1510 -gaaatcggcaagggca--ttatccgccgc----gaa---
HIdxsdna     1477 -cctattggtaaatcac--gtttaattcga----aaa---
SSdxsdna     1465 -g---gcggcctcgatg--tgctgcaccgc----ga----
HPdxsdna     1450 -gttttaggccaaag-c--gaattgttgaaaaagag---
```

# Figure 5 (page 21 of 25)

```
STdxsdna      1694 ggcaagaaggtagcgatcctgtcgctcggcacg-cgcctt
CRdxsdna      1786 ggcaaggacgtgtgcctggtggcgtacggcagc-agtgtg
CJdxsdna      1463 ggcttgtaaaaaataatagtgaaatt------g-cttttt
PAdxsdna      1522 ggcggcagggtcgcactgctggtcttcggcgtg-cagttg
LEdxsdna      1753 ggggagagagtggctctattgggatatggctc--agcagt
MTdxsdna      1492 ggtttgaaccacgacgtcctgttggtggccatc-ggc---
RSdxs1dna     1513 ggcacgcgaatcgcgctcctgtccttcggcacc-cgtctg
RSdxs2dna     1522 gggacggatgtcgcgatcctctccttcggcgcg-catctg
SPCCdxsdna    1510 ggcgatgatttgctgatgttggcttacggctcg-atggtc
ECdxsdna      1507 ggcgagaaactggcgatccttaactttggtacg-ctgatg
NMdxsdna      1540 ggtgagaaaaccgcattcattgccttcggcagt-atggtc
HIdxsdna      1507 ggtcaaaaaattgcgattttaaatttggtact-ctatta
SSdxsdna      1491 --cgagcggcccgaggtgctgctggtcgccgtg-ggcgtc
HPdxsdna      1483 ggcgaaattttactcat--aggctatggtaatggcgtggg

STdxsdna      1733 gcgg--aagca-------ctaa-aggcc---------gcc
CRdxsdna      1825 aacg--aggcg-------ctgg-ccgcg---------gcg
CJdxsdna      1496 taggttatgga-------caag-gtgtg---------gca
PAdxsdna      1561 gcgg--aggcg-------atga-aggtc---------gcc
LEdxsdna      1791 gcag--aactg-------tttggatgct---------gct
MTdxsdna      1528 gcgt--tcgca-------ccga-tggcgttggcggtggcc
RSdxs1dna     1552 gccg--aggtg-------cagg-tggcc---------gcc
RSdxs2dna     1561 cacg--aggcc-------ttgc-aggcg---------gcg
SPCCdxsdna    1549 tatc--cggcc-------ctgc-agacg---------gca
ECdxsdna      1546 ccag--aagcg-------gcga-aagtc---------gcc
NMdxsdna      1579 gccc--ctgca-------ttgg-cggtc---------gcc
HIdxsdna      1546 ccat--ccgct-------ttag-agtta---------tca
SSdxsdna      1528 atgg--ca-caggtctgcctcc-agacc---------gcc
HPdxsdna      1521 gcgg--gcgca-------ttta-----g---------tcc

STdxsdna      1754 gacacgctcgaggcc--aagggcctctcgaccaccg----
CRdxsdna      1846 gacatgctggagcgc--gatggcgtgtccaccaccg----
CJdxsdna      1519 aaagcgtggcaagtcttaagagccttgcaagaaatgaata
PAdxsdna      1582 gaaagcctcgacg----------------ccacgg----
LEdxsdna      1813 attgtgctagaatcc--cgcggcttacaagtaacag----
MTdxsdna      1558 aagcggctgcacaac--caggggatcggtgtgacgg----
RSdxs1dna     1573 gaggcgctggctgcg--cgcgggatctctcccacgg----
RSdxs2dna     1582 aaacttctcgaggcc--gaggggtgagcgtgaccg----
SPCCdxsdna    1570 gaactgctgaatgag--cacggcatctcagctactg----
ECdxsdna      1567 gaatcgctgaacg-----------------ccacgc----
NMdxsdna      1600 ggaaaactgaacg-----------------ccaccg----
HIdxsdna      1567 gaaaaactcaatg-----------------caacgg----
SSdxsdna      1555 gagctgctccgggcc--cgcggcatcggatgcacgg----
HPdxsdna      1538 aactggctttaaaag--aaaaaaacatagaatgcgc----
```

# Figure 5 (page 22 of 25)

```
STdxsdna     1788 ------------tcgcc--gacctgcgcttcgccaaaccg
CRdxsdna     1880 ------------tcatt--gacgcgcgcttctgcaagcct
CJdxsdna     1559 ataatgctaatttgatt--gatttaattttgctaaacct
PAdxsdna     1601 ------------tcgtc--gacatgcgtttcgtcaaaccc
LEdxsdna     1847 ------------ttgca--gatgcacgtttctgcaaacca
MTdxsdna     1592 ------------tgatc--gacccgcgctgggtgttgccg
RSdxs1dna    1607 ------------ttgcg--gatgcgcgctttgcaaagccg
RSdxs2dna    1616 ------------tggcc--gacgcccgcttctcgcgcccg
SPCCdxsdna   1604 ------------tgatc--aatgcccgcttcgccaagccc
ECdxsdna     1586 ------------tggtc--gatatgcgttttgtgaaaccg
NMdxsdna     1619 ------------tcgcc--gatatgcgcttcgtcaaaccg
HIdxsdna     1586 ------------ttgtc--gatatgcgttttgtgaaaccg
SSdxsdna     1589 ------------tcgtc--gacccgcgctgggtcaagccc
HPdxsdna     1572 ------------tctcttggatctcaggtttttaaagcct

STdxsdna     1814 ctcgacgaggatctgatcc-gc-c-gcctgctcaccaccc
CRdxsdna     1906 ctggacaccaagctgatcc-gctc-ggctgc-caaggagc
CJdxsdna     1597 ttagatgaagagcttttgt-gt-gagcttgctaaaaaaag
PAdxsdna     1627 ctcgacgaagccctggtac-gc-g-aattggcgggcagcc
LEdxsdna     1873 ctggaccatgccctcataa-gg-a-gccttgcaaaatcac
MTdxsdna     1618 gtgtctgacggtgtg---c-gc-g-aactggcggtgcagc
RSdxs1dna    1633 ctcgaccgggatctgat----c-c-tgcagctcgcggccc
RSdxs2dna    1642 ctcgacacggggcacatcg-ac-c-agctcgtgcgccatc
SPCCdxsdna   1630 ttagatgaggaactgattgtgc-c-gctggcgcgccagat
ECdxsdna     1612 cttgatgaagcgttaattc-tg-g-aaatggccgccagcc
NMdxsdna     1645 atagacgaagagttgattg-tc-c-gccttgcccgaagcc
HIdxsdna     1612 attgatattgaaatgatta-at-gtgcttgcacaa-actc
SSdxsdna     1615 gtcgaccccgtgctg--------c-ccccactcgccgccg
HPdxsdna     1600 ttagatccaaatttaagcg-cg-a-tcgttgccccttatc

STdxsdna     1851 acgaagtggcggtga---cgatcgaggaa--ggcgc---g
CRdxsdna     1943 accctgtcatgatca---ccatcgaggag--ggctc---c
CJdxsdna     1635 taaaatttggtttat---ttttagtgaaaatgttaa---a
PAdxsdna     1664 acgaactgctggtga---ccatcgaggaa--aacgccgtg
LEdxsdna     1910 atgaagtgctaatca---ctgtcgaagaa--ggatc---a
MTdxsdna     1652 acaagctgctcgtca---cgctagaggac--aacgg---g
RSdxs1dna    1667 atcacgaggcgctcattaccatcgaggag--ggcgc---c
RSdxs2dna    1679 acgcggcgctggtaa---cggtggagcag--ggggc---c
SPCCdxsdna   1668 cggcaaagtcg-tca---cctttgaggaa--ggctg---c
ECdxsdna     1649 atgaagcgctggtca---ccgtagaagaa--aacgc---c
NMdxsdna     1682 acgaccgcatcgtta---cccttgaagaa--aacgcc--g
HIdxsdna     1649 acgattatttggtca---cattggaagaa--aatgc---a
SSdxsdna     1646 agcaccggctcgtcg---ccgtcgtggag--gac------
HPdxsdna     1637 aaaagctctatgttt---ttagcgataat--tacaa---g
```

# Figure 5 (page 23 of 25)

```
STdxsdna      1883 atc--ggcggccccggt-gcgc-atgtgctgacg------
CRdxsdna      1975 gtg--ggtggcttcgct-gcgc-acgtgatgcag------
CJdxsdna      1669 att--ggcggtatagaaagttt-aattaataatt------
PAdxsdna      1699 atg--ggcggcgccggc-tcg------gcggtcggcgagt
LEdxsdna      1942 att--ggaggttttgga-tctc-atgttgttcag------
MTdxsdna      1684 gtc--aacggtggggcg-gggt-cagcggtg---------
RSdxs1dna     1702 atc--ggcggcttcggc-agcc-atgtggcgcag------
RSdxs2dna     1711 atg--ggcggcttcggc-gcct-atgtcatgcactgt---
SPCCdxsdna    1699 cta--cccggcggcttt-ggct-ccgcgattatg------
ECdxsdna      1681 att--atgggc-----g-gcgc-agg--------------
NMdxsdna      1715 aacagggcggcgcaggc-agcg-cggtgctggaa------
HIdxsdna      1681 att--caagg---tgga-gcgggatctgctgttg------
SSdxsdna      1675 aac--agccgggccgcc-gggg-tcggttcggcg------
HPdxsdna      1669 ctt--ggagg----ggt-g--------------g------

STdxsdna      1913 --ctc---gccagcgatac-cggcc--t----gatcgacg
CRdxsdna      2005 --ttc---ctcgcactgga-gggcc--t----gctggacg
CJdxsdna      1700 --ttt---tacaaaaata---------t----gat-----
PAdxsdna      1730 tcctc---gccagcga----gggcc--t------------
LEdxsdna      1972 --ttcatggccttagat----gggc--t----tcttgatg
MTdxsdna      1711 ---tc---ggccgcgctgc-ggcgc--gcggagatcgacg
RSdxs1dna     1732 --ctt---ctggccgaggc-cgggg--t----cttcgacc
RSdxs2dna     1744 --ctc---gcca---attc-cggcg--g----cttcgacg
SPCCdxsdna    1729 --gag---tcc----ttgc-aggcccat----gatc--tg
ECdxsdna      1698 ----c---agcggcgtgaa-cgaag--t----gctgatgg
NMdxsdna      1747 --gt----gttggcgaaacacggca--t----ctgcaaac
HIdxsdna      1709 ----c---ggaagtactaa-attca--t----caggaaaa
SSdxsdna      1705 --gtc---gccctggcgct-cgggg--a----cgccgatg
HPdxsdna      1682 --cta---gc--gcgattt-tagag--t----ttttga--

STdxsdna      1941 ---ccggcctc---aagc---------tgcgcaccatgcg
CRdxsdna      2033 ---gcgggctc---aagt---------tccggcccatgac
CJdxsdna      1717 ----ttgcatgtaaaagt---------tgttagctttgaa
PAdxsdna      1749 ----cgaagtc---ccgc---------tgctgcaactggg
LEdxsdna      2000 ---gcaagttg---aagt---------ggagaccaatagt
MTdxsdna      1742 tgccctgccgc---gatg---------t------cgggtt
RSdxs1dna     1760 ---gcggcttc---cggt---------atcgctcgatggt
RSdxs2dna     1769 ---ggggcctc---gcgc---------tccgggtcatgac
SPCCdxsdna    1753 ---cagg--tt---ccgg---------tgttgccgatcgg
ECdxsdna      1724 ---cccatcgt---aaaccagtacccgtgctgaacattgg
NMdxsdna      1775 ---ccg---tc---ttgc---------t------tttggg
HIdxsdna      1735 ---tcaaccgc---a-ct---------tttacaacttg-g
SSdxsdna      1733 ---tcgacgta---ccgg---------tgcgccgcttcgg
HPdxsdna      1706 ---------------------------gcgaac------
```

# Figure 5 (page 24 of 25)

```
STdxsdna     1966 cctgccggaca----tattccaggaccaggacaagcccga
CRdxsdna     2058 gctgccggacc----gctacatcgaccacggcgactaccg
CJdxsdna     1744 tatga-agaca----aatttattgaacatggaaa------
PAdxsdna     1773 cctgcccgact----actacgtcgaacacgccaagcccag
LEdxsdna     2025 tcttcctgatc----gatacattgaccatggatctcctgt
MTdxsdna     1764 gccgcaggagt----tctacgagcacgcgtctcgaagcga
RSdxs1dna    1785 gctgcccgaca----cgttcatcgaccacaacagcgccga
RSdxs2dna    1794 gctgcccgacc----gcttcatcgagcaggcgagccccga
SPCCdxsdna   1776 tgttcccgatc----tcttggtggaacatgccagccctga
ECdxsdna     1758 cctgccggact----tctt------------tattccgc
NMdxsdna     1791 cgttgccgata----ccgtaaccggacacggcgatccgaa
HIdxsdna     1758 cttgccagattatttattccacaagcgacaca---gcaa
SSdxsdna     1758 catccccgagc----agttcctcgcgcacgccaggcgcgg
HPdxsdna     1712 -------aaaa----tattttaaagcctgttaaaagcttt

STdxsdna     2002 g------aagcagt-a--------tgacgaa--------g
CRdxsdna     2094 c------gaccagc-t--------ggccatg--------g
CJdxsdna     1773 -------aacaagt--------------gag--------g
PAdxsdna     1809 c------gagatgc-t--------cgccgaa--------t
LEdxsdna     2061 t------gatcagt-t--------ggcggaa--------g
MTdxsdna     1800 g------gtgctg------------gccgat--------c
RSdxs1dna    1821 a------gtgatgt-a--------tgccacc--------g
RSdxs2dna    1830 g------gacatgt-a--------tgccgat--------g
SPCCdxsdna   1812 tgaatctaaacagg-agttgggcctgacg----------c
ECdxsdna     1781 a------aggaactca--------ggaagaa--------a
NMdxsdna     1827 a------aaacttt-t--------agacgat--------t
HIdxsdna     1795 g------aagca-t-t--------ggcagat--------t
SSdxsdna     1794 t------gaggtgc-t--------cgccgac--------a
HPdxsdna     1741 g------aaatcat----------tgatgaatttatcatg

STdxsdna     2019 cggg-gctgaacgccgcc--------aacatcgtc-----
CRdxsdna     2111 ccgg-cctcaccagccag--------cacatcgcc-----
CJdxsdna     1784 tgga-aaaaaatctagaa--------aaagatgtc-----
PAdxsdna     1826 gcgg-cctggatgccgcg--------ggcatcg-------
LEdxsdna     2078 ctgg-cctaacaccatct--------cacattgca-----
MTdxsdna     1814 tggg-gctta---ccgac--------caggacgt------
RSdxs1dna    1838 ccgg-gctgaatgcggcc--------gacatagag-----
RSdxs2dna    1847 cggg-gctgcgggccgag--------gatatcgcg-----
SPCCdxsdna   1841 cgcg-tcagatggccgat--------cgcatcctc-----
ECdxsdna     1799 tgcgcgccgaactcggcc------------tcgat-----
NMdxsdna     1844 tggg-cttgagtgc--------------------c-----
HIdxsdna     1811 tagg-attggatacaaaa--------ggcattgaa-----
SSdxsdna     1811 tcgg-gctgaccccggtg--------gagatcgcc-----
HPdxsdna     1765 catg-g--gaacaccgctttagtggaaaaatccttaggat
```

# Figure 5 (page 25 of 25)

```
STdxsdna      2045 --gacacggtgc---tgaagg-cgctc---cgctacaacg
CRdxsdna      2137 --tccaccgcgc---tcacca-ccctggggcgcgccaagg
CJdxsdna      1810 --aatagtttgt---tgacg----------------aaag
PAdxsdna      1850 ---------------aaaagg-cagta---cg--------
LEdxsdna      2104 --gcaacagtat---ttaaca-tactt---gg-----aca
MTdxsdna      1836 --ggcccggcg------------gatc---accggctggg
RSdxs1dna     1864 --cggaaggcgc---tggaga-cgct--------------
RSdxs2dna     1873 --gccaccgcgc---ggggcg-cgctcg--cccgggggcg
SPCCdxsdna    1867 --gaaaagtt-----tggaag-c---------cgtcaacg
ECdxsdna      1822 --gccgctggta---tggaag-c---------caaaatca
NMdxsdna      1858 --gaagcggtg-----gaacg-gcgtg---tgcg------
HIdxsdna      1837 --gaaaaaattc---tcaa-----ctt---tattgcaa--
SSdxsdna      1837 --g-ggcggatc---gg--cg-cgagc---ctgcccgtgc
HPdxsdna      1802 tagacacagagagtttgactgacgcta---ttttaaaaga

STdxsdna      2076 ---ag----gccgag-----ctggccga-cgg----gg-t
CRdxsdna      2171 ---ac----gccgccaagttctcactgt-cag----cgct
CJdxsdna      1829 ---tt----ttaaaa-----ttttatca------------
PAdxsdna      1863 -----------ccag-----cgtctcga-c----------
LEdxsdna      2130 ---aa----ccagag-----a-ggctct-aga----gg-t
MTdxsdna      1859 ---tc----gccgcg-----ctgggtac-cgg----gg-t
RSdxs1dna     1884 ----g----ggggtg-----gaggtcct-cgc----cc-g
RSdxs2dna     1905 ---cgtgatgccgct-----ccggcaga-cggcaaagc-c
SPCCdxsdna    1890 ---ga----ttggtg-----ctg--ctt-cgg----ct-t
ECdxsdna      1847 ---ag----gcctgg-----ct------------------
NMdxsdna      1881 ----c----gcgtgg-----ctgtcggatcgg----ga-t
HIdxsdna      1862 --------------------------aa-caa----gg-t
SSdxsdna      1865 ---gg----gaggaa-----ccggccga-gga----gc-a
HPdxsdna      1839 tttag----gacaag-----agagatga------------

STdxsdna      2098 gcgggcg--taa-------------------------
CRdxsdna      2199 gcaagcg--taa-------------------------
CJdxsdna      1845 ------t--taa-------------------------
PAdxsdna      1876 -cggcag--tag-------------------------
LEdxsdna      2151 catgaca--taa-------------------------
MTdxsdna      1881 gtgtgcg--tccgacgcgattccagaacatctcgactaa
RSdxs1dna     1905 ccgcgcc--tga-------------------------
RSdxs2dna     1935 gcgggcg--gtctga----------------------
SPCCdxsdna    1910 ga-----------------------------------
ECdxsdna      1857 ---ggca--taa-------------------------
NMdxsdna      1903 gcggcaaattaa-------------------------
HIdxsdna      1870 a-attta--taa-------------------------
SSdxsdna      1887 gcccgca--tga-------------------------
HPdxsdna      1858 -------------------------------------
```

# Figure 6 (page 1 of 18)

```
STdxsp      182 ----------------------------------------
AAdxsp        1 ----------------------------------------
BSdxsp        1 ----------------------------------------
CRdxsp        1 mlrgavshgpa-----------------------------
CJdxsp        1 ----------------------------------------
PAdxsp        1 mpkt------------------------------------
LEdxsp        1 ----------------------------------------
MLdxsp        1 ----------------------------------------
MTdxsp        1 ----------------------------------------
RCdxsp        1 ----------------------------------------
RSdxs1p       1 ----------------------------------------
RSdxs2p       1 mtn-------------------------------------
SPCCdxsp      1 ----------------------------------------
SPdxsp        1 ----------------------------------------
TMdxsp        1 ----------------------------------------
ECdxsp        1 m---------------------------------------
NMdxsp        1 ----------------------------------------
HIdxsp        1 m---------------------------------------
PFdxsp        1 mifnyvffknfvpvvlyilliiyinlngmnnknqikteki
SSdxsp        1 ----------------------------------------
HPdxsp        1 ----------------------------------------

STdxsp      182 ----------------------------------------
AAdxsp        1 ----------------------------------------
BSdxsp        1 ----------------------------------------
CRdxsp       12 ----------------------------------------
CJdxsp        1 ----------------------------------------
PAdxsp        5 ----------------------------------------
LEdxsp        1 ----------------------------------------
MLdxsp        1 ----------------------------------------
MTdxsp        1 ----------------------------------------
RCdxsp        1 ----------------------------------------
RSdxs1p       1 ----------------------------------------
RSdxs2p       4 ----------------------------------------
SPCCdxsp      1 ----------------------------------------
SPdxsp        1 ----------------------------------------
TMdxsp        1 ----------------------------------------
ECdxsp        2 ----------------------------------------
NMdxsp        1 ----------------------------------------
HIdxsp        2 ----------------------------------------
PFdxsp       41 yikklnrlsrknslcssknkiaclfdignddnrnttygyn
SSdxsp        1 ----------------------------------------
HPdxsp        1 ----------------------------------------
```

# Figure 6 (page 2 of 18)

```
STdxsp      182 ----------------------------------------
AAdxsp        1 ----------------------------------------
BSdxsp        1 ----------------------------------------
CRdxsp       12 ----------------------------------------
CJdxsp        1 ----------------------------------------
PAdxsp        5 ----------------------------------------
LEdxsp        1 ----------------------------------------
MLdxsp        1 ----------------------------------------
MTdxsp        1 ----------------------------------------
RCdxsp        1 ----------------------------------------
RSdxs1p       1 ----------------------------------------
RSdxs2p       4 ----------------------------------------
SPCCdxsp      1 ----------------------------------------
SPdxsp        1 ----------------------------------------
TMdxsp        1 ----------------------------------------
ECdxsp        2 ----------------------------------------
NMdxsp        1 ----------------------------------------
HIdxsp        2 ----------------------------------------
PFdxsp       81 vnvknddinsllknnysnklymdkrkninnvistnkisgs
SSdxsp        1 ----------------------------------------
HPdxsp        1 ----------------------------------------

STdxsp      182 ----------------------------------------
AAdxsp        1 ----------------------------------------
BSdxsp        1 ----------------------------------------
CRdxsp       12 ----------------------------------------
CJdxsp        1 ----------------------------------------
PAdxsp        5 ----------------------------------------
LEdxsp        1 ----------------------------------------
MLdxsp        1 ----------------------------------------
MTdxsp        1 ----------------------------------------
RCdxsp        1 ----------------------------------------
RSdxs1p       1 ----------------------------------------
RSdxs2p       4 ----------------------------------------
SPCCdxsp      1 ----------------------------------------
SPdxsp        1 ----------------------------------------
TMdxsp        1 ----------------------------------------
ECdxsp        2 ----------------------------------------
NMdxsp        1 ----------------------------------------
HIdxsp        2 ----------------------------------------
PFdxsp      121 isnicsrnqkeneqkrnkqrcltqchtynmsheqdkland
SSdxsp        1 ----------------------------------------
HPdxsp        1 ----------------------------------------
```

# Figure 6 (page 3 of 18)

```
STdxsp       182 ----------------------------------------
AAdxsp         1 ----------------------------------------
BSdxsp         1 ----------------------------------------
CRdxsp        12 ----------------------------------------
CJdxsp         1 ----------------------------------------
PAdxsp         5 ----------------------------------------
LEdxsp         1 ----------------------------------------
MLdxsp         1 ----------------------------------------
MTdxsp         1 ----------------------------------------
RCdxsp         1 ----------------------------------------
RSdxs1p        1 ----------------------------------------
RSdxs2p        4 ----------------------------------------
SPCCdxsp       1 ----------------------------------------
SPdxsp         1 ----------------------------------------
TMdxsp         1 ----------------------------------------
ECdxsp         2 ----------------------------------------
NMdxsp         1 ----------------------------------------
HIdxsp         2 ----------------------------------------
PFdxsp       161 nnrnnkknfnllfinyfnlkrmknsllnkdnffyckekkl
SSdxsp         1 ----------------------------------------
HPdxsp         1 ----------------------------------------

STdxsp       182 ----------------------------------------
AAdxsp         1 ----------------------------------------
BSdxsp         1 ----------------------------------------
CRdxsp        12 ----------------------------------------
CJdxsp         1 ----------------------------------------
PAdxsp         5 ----------------------------------------
LEdxsp         1 ----------------------------------------
MLdxsp         1 ----------------------------------------
MTdxsp         1 ----------------------------------------
RCdxsp         1 ----------------------------------------
RSdxs1p        1 ----------------------------------------
RSdxs2p        4 ----------------------------------------
SPCCdxsp       1 ----------------------------------------
SPdxsp         1 ----------------------------------------
TMdxsp         1 ----------------------------------------
ECdxsp         2 ----------------------------------------
NMdxsp         1 ----------------------------------------
HIdxsp         2 ----------------------------------------
PFdxsp       201 sflhkaykkknctfqnyslkrksnrdshklfsgefddytn
SSdxsp         1 ----------------------------------------
HPdxsp         1 ----------------------------------------
```

# Figure 6 (page 4 of 18)

```
STdxsp      182 ----------------------------------------
AAdxsp        1 ----------------------------------------
BSdxsp        1 ----------------------------------------
CRdxsp       12 ----------------------------------------
CJdxsp        1 ----------------------------------------
PAdxsp        5 ----------------------------------------
LEdxsp        1 ----------------------------------------
MLdxsp        1 ----------------------------------------
MTdxsp        1 ----------------------------------------
RCdxsp        1 ----------------------------------------
RSdxs1p       1 ----------------------------------------
RSdxs2p       4 ----------------------------------------
SPCCdxsp      1 ----------------------------------------
SPdxsp        1 ----------------------------------------
TMdxsp        1 ----------------------------------------
ECdxsp        2 ----------------------------------------
NMdxsp        1 ----------------------------------------
HIdxsp        2 ----------------------------------------
PFdxsp      241 nnalyesekkeyitlnnnnknnnnknndnknndnndynnn
SSdxsp        1 ----------------------------------------
HPdxsp        1 ----------------------------------------

STdxsp      182 ----------------------------------------
AAdxsp        1 ----------------------------------------
BSdxsp        1 ----------------------------------------
CRdxsp       12 ----------------------------------------
CJdxsp        1 ----------------------------------------
PAdxsp        5 ----------------------------------------
LEdxsp        1 ----------------------------------------
MLdxsp        1 ----------------------------------------
MTdxsp        1 ----------------------------------------
RCdxsp        1 ----------------------------------------
RSdxs1p       1 ----------------------------------------
RSdxs2p       4 ----------------------------------------
SPCCdxsp      1 ----------------------------------------
SPdxsp        1 ----------------------------------------
TMdxsp        1 ----------------------------------------
ECdxsp        2 ----------------------------------------
NMdxsp        1 ----------------------------------------
HIdxsp        2 ----------------------------------------
PFdxsp      281 nscnnlgersnhydnyggdnnnpcnnnndkydigkyfkqi
SSdxsp        1 ----------------------------------------
HPdxsp        1 ----------------------------------------
```

# Figure 6 (page 5 of 18)

```
STdxsp     182 ---------------------------------------m
AAdxsp       1 ----------------------------------------
BSdxsp       1 ----------------------------------------
CRdxsp      12 ---------------------------------------v
CJdxsp       1 ----------------------------------------
PAdxsp       5 ---------------------------------------l
LEdxsp       1 ---------------------------------------m
MLdxsp       1 ----------------------------------------
MTdxsp       1 ----------------------------------------
RCdxsp       1 ---------------------------------------m
RSdxs1p      1 ---------------------------------------m
RSdxs2p      4 ---------------------------------------p
SPCCdxsp     1 ----------------------------------------
SPdxsp       1 ----------------------------------------
TMdxsp       1 ----------------------------------------
ECdxsp       2 ---------------------------------------s
NMdxsp       1 ----------------------------------------
HIdxsp       2 ---------------------------------------t
PFdxsp     321 ntfinideyktiygdeiykeiyelyvernipeyyerkyfs
SSdxsp       1 ----------------------------------------
HPdxsp       1 ---------------------------------------m

STdxsp     185 a---------------------------------dl----
AAdxsp       1 ----------------------------------ml----
BSdxsp       1 ----------------------------------------
CRdxsp      13 a---------------------------------draaag
CJdxsp       1 ----------------------------------m-----
PAdxsp       6 h---------------------------------ei----
LEdxsp       2 alcayafpgilnrtgvvsdsskatplfsgwihgtdlqflf
MLdxsp       1 ----------------------------------------
MTdxsp       1 ----------------------------------------
RCdxsp       2 s---------------------------------at----
RSdxs1p      2 t---------------------------------dr----
RSdxs2p      5 t---------------------------------pr----
SPCCdxsp     1 ----------------------------------------
SPdxsp       1 ----------------------------------------
TMdxsp       1 ----------------------------------------
ECdxsp       3 f---------------------------------di----
NMdxsp       1 ----------------------------------m-----
HIdxsp       3 n---------------------------------nm----
PFdxsp     361 e---------------------------------di----
SSdxsp       1 ----------------------------------------
HPdxsp       2 i---------------------------------lq----
```

# Figure 6 (page 6 of 18)

```
STdxsp      194 ----------------------------------------
AAdxsp        3 ----------------------------------------
BSdxsp        1 ----------------------------------------
CRdxsp       20 parcaapvargvrsaaptrqrraeasvnapragpagsysg
CJdxsp        2 ----------------------------------------
PAdxsp        9 ----------------------------------------
LEdxsp       42 qhklthevkkrsrvvqaslsesgeyytqr-----------
MLdxsp        1 ----------------------------------------
MTdxsp        1 ----------------------------------------
RCdxsp        5 ----------------------------------------
RSdxs1p       5 ----------------------------------------
RSdxs2p       8 ----------------------------------------
SPCCdxsp      1 ----------------------------------------
SPdxsp        1 ----------------------------------------
TMdxsp        1 ----------------------------------------
ECdxsp        6 ----------------------------------------
NMdxsp        2 ----------------------------------------
HIdxsp        6 ----------------------------------------
PFdxsp      364 ----------------------------------------
SSdxsp        1 ----------------------------------------
HPdxsp        5 ----------------------------------------

STdxsp      194 -----------------p-k----t---------------
AAdxsp        3 -----------------e-k----y---------------
BSdxsp        1 ------------------------m---------------
CRdxsp       60 ewdklsveeidewrdvgp-k----t---------------
CJdxsp        2 -----------------s-k--------------------
PAdxsp        9 -----------------p-rerpat---------------
LEdxsp       71 -----------------p-p----t---------------
MLdxsp        1 ----------------------------------------
MTdxsp        1 ----------------------------------------
RCdxsp        5 -----------------psr----t---------------
RSdxs1p       5 -----------------p-c----t---------------
RSdxs2p       8 -----------------p-e----t---------------
SPCCdxsp      1 ----------------------------------------
SPdxsp        1 ----------------------------------------
TMdxsp        1 ----------------------------------------
ECdxsp        6 -----------------a-k----y---------------
NMdxsp        2 -----------------n-p----s---------------
HIdxsp        6 -----------------n-n----y---------------
PFdxsp      364 -----------------k-k----svlfdidkyndvefek
SSdxsp        1 ------------------------m---------------
HPdxsp        5 -----------------n-k----t---------------
```

# Figure 6 (page 7 of 18)

```
STdxsp     203 ------------------------------------plld
AAdxsp       6 ------------------------------------eilk
BSdxsp       2 ------------------------------------dll-
CRdxsp      80 ------------------------------------plld
CJdxsp       4 ---------------------------------------k
PAdxsp      16 ------------------------------------plld
LEdxsp      74 ------------------------------------pild
MLdxsp       1 -------------------------------------mle
MTdxsp       1 -------------------------------------mlq
RCdxsp       9 ------------------------------------phld
RSdxs1p      8 ------------------------------------ptld
RSdxs2p     11 ------------------------------------plld
SPCCdxsp     1 ------------------------------------mhls
SPdxsp       1 ------------------------------------mhis
TMdxsp       1 ------------------------------------mlld
ECdxsp       9 ------------------------------------ptla
NMdxsp       5 ------------------------------------plld
HIdxsp       9 ------------------------------------plls
PFdxsp     382 aikeefinngvyinnidntyykkenilimkkilhyfpllk
SSdxsp       2 ------------------------------------tile
HPdxsp       8 ------------------------------------fdln

STdxsp     215 tvdtpqdlrklapaqlrqladelraetisavgstgghlgs
AAdxsp      10 dykgpfdiknydyetlqklaqevrdyiinvtsknggghvgp
BSdxsp       5 siqdpsflknmsideleklsdeirqflitslsasgghigp
CRdxsp      84 tvnypvhlknfnneqlkqlckelrsdivhtvsrtgghlss
CJdxsp       5 fahtqeeleklslkelenlaasmrekiiqvvskngghlss
PAdxsp      20 rasspaelrrlgeadletladelrqyllytvgqtgghfga
LEdxsp      78 tvnypihmknlslkelkqladelrsdtifnvsktgghlgs
MLdxsp       4 qirrpadlqhlsqqqlrdlaaeirellvhkvaatgghlgp
MTdxsp       4 qirgpadlqhlsqaqlrelaaeireflihkvaatgghlgp
RCdxsp      13 rvtgpadlkamsiadltalasevrreivevvsqtgghlgs
RSdxs1p     12 rvtlpvdikgltdrelrsladelraetisavsvtgghlga
RSdxs2p     15 rvccpadmkalsdaelerladevrsevisvvaetgghlgs
SPCCdxsp     5 eithpnqlhglsvaqleqighqirekhlqtvaatgghlgp
SPdxsp       5 elthpnelkglsireleevsrqirekhlqtvatsgghlgp
TMdxsp       5 ------eikrmsydelkrlaedirkritevvlkngghlas
ECdxsp      13 lvdstqelrllpkeslpklcdelrrylldsvsrssghfas
NMdxsp       9 lidspqdlrrldkkqlprlagelrtfllesvgqtgghfas
HIdxsp      13 linspedlrllnkdqlpqlcqelrayllesvsqtsghlas
PFdxsp     422 linnpsdlkklkkqylpllahelkiflffivnitgghfss
SSdxsp       6 nirqprdlkalpeeqlhelseeirqflvhavtrtgghlgp
HPdxsp      12 ----pndi-----aglelvcqtlrnrilevvsangghlss
```

# Figure 6 (page 8 of 18)

```
STdxsp      335 glgvveltvaihyvfntpddrliwdvghqcyphkiltgrr
AAdxsp       50 slgvveltiallrvfnppedvivwdighqgypwkiltdrk
BSdxsp       45 nlgvveltvalhkefnspkdkflwdvghqsyvhklltgrg
CRdxsp      124 slgvveltvamhyvfntpedkiiwdvghqayghkiltgrr
CJdxsp       45 nlgavelsiamhlvfdakkdpfifdvshqsythkllsgke
PAdxsp       60 glgvveltialhyvfdtpddrlvwdvghqayphkilterr
LEdxsp      118 slgvveltvalhyvfnapqdrilwdvghqsyphkiltgrr
MLdxsp       44 nlgvveltlalhrvfdsphdpiifdtghqayvhkmltgrc
MTdxsp       44 nlgvveltlalhrvfdsphdpiifdtghqayvhkmltgrs
RCdxsp       53 slgvveltvalhavfnspgdkliwdvghqcyphkiltgrr
RSdxs1p      52 glgvveltvalhaifdaprdkiiwdvghqcyphkiltgrr
RSdxs2p      55 slgvveltvalhavfntptdklvwdvghqcyphkiltgrr
SPCCdxsp     45 glgvveltlalyqtldldrdkvvwdvghqayphklltgry
SPdxsp       45 glgvveltvalystldldkdrviwdvghqayphkmltgry
TMdxsp       39 nlgtieltlalyrvfdpredaiiwdtghqaythkiltgrd
ECdxsp       53 glgtveltvalhyvyntpfdqliwdvghqayphkiltgrr
NMdxsp       49 nlgaveltvalhyvyntpedklvwdvghqsyphkiltgrk
HIdxsp       53 glgtveltvalhyvyktpfdqliwdvghqayphkiltgrr
PFdxsp      462 vlssleiqllllyifnqpydnviydighqayvhkiltgrk
SSdxsp       46 nlgvveltialhrvfespvdrilwdtghqsyvhklltgrq
HPdxsp       43 slgavelivgmhalfdcqknpfifdtshqayahklltgrf

STdxsp      455 drirtirqggglsgftkrseseydpfgaahsstsisaalg
AAdxsp       90 eqfptlrqykgisgflrreesiydafgaghsstsisaalg
BSdxsp       85 kefatlrqykglcgfpkrsesehdvwetghsstslsgamg
CRdxsp      164 kgmatirqtnglsgftkrdeseydpfgaghsstsisaalg
CJdxsp       85 eifdtlrqinglsgytkpsegdy--fvaghsstsislavg
PAdxsp      100 elmgtlrqknglaafprraeseydtfgvghsstsisaalg
LEdxsp      158 dkmstlrqtdglagftkrseseydcfgtghssttisaglg
MLdxsp       84 qdfdslrkkaglsgypsraesehdwvesshastalsyadg
MTdxsp       84 qdfatlrkkgglsgypsraesehdwvesshasaalsyadg
RCdxsp       93 srmltlrqaggisgfpkrsesphdafgaghsstsisaalg
RSdxs1p      92 drirtlrqggglsgftkrsespydcfgaghsstsisaavg
RSdxs2p      95 eqmrtlrqkgglsgftkrsesaydpfgaahsstsisaalg
SPCCdxsp     85 hnfhtlrqkdgiagypkrtenrfdhfgaghastsisaglg
SPdxsp       85 hdfhtlrqkdgvagylkrsesrfdhfgaghastsisaglg
TMdxsp       79 dlfhtirtfgglsgfvtrrespldwfgtghagtsiaaglg
ECdxsp       93 dkigtirqkgglhpfpwrgeseydvlsvghsstsisagig
NMdxsp       89 nqmhtmrqygglagfpkrceseydafgvghsstsigaalg
HIdxsp       93 eqmstirqkdgihpfpwreesefdvlsvghsstsisaglg
PFdxsp      502 llflslrnkkgisgflnifesiydkfgaghsstslsaiqg
SSdxsp       86 d-fsklrgkgglsgypsreesehdvienshastalgwadg
HPdxsp       83 esfstlrqfkglsgftkpsesaydyfiaghsstsvsigvg
```

# Figure 6 (page 9 of 18)

```
STdxsp      575 fa-------------------------------------
AAdxsp      130 fr-------------------------------------
BSdxsp      125 ma-------------------------------------
CRdxsp      204 ma-------------------------------------
CJdxsp      123 ac-------------------------------------
PAdxsp      140 ma-------------------------------------
LEdxsp      198 ma-------------------------------------
MLdxsp      124 la-------------------------------------
MTdxsp      124 la-------------------------------------
RCdxsp      133 fa-------------------------------------
RSdxs1p     132 fa-------------------------------------
RSdxs2p     135 fa-------------------------------------
SPCCdxsp    125 ma-------------------------------------
SPdxsp      125 ma-------------------------------------
TMdxsp      119 fe-------------------------------------
ECdxsp      133 ia-------------------------------------
NMdxsp      129 ma-------------------------------------
HIdxsp      133 ia-------------------------------------
PFdxsp      542 yyeaewqvknkekygngdieisdnanvtnnerifqkgihn
SSdxsp      125 la-------------------------------------
HPdxsp      123 va-------------------------------------

STdxsp      581 ---iankln--------eapgk-a----------------
AAdxsp      132 ---igkdlkg-------ekedy-v----------------
BSdxsp      127 ---aardik--------gtdey-i----------------
CRdxsp      206 ---vgrdvk--------gkkns-v----------------
CJdxsp      125 ---kaialk--------gekri-p----------------
PAdxsp      142 ---iaarlq--------gkerk-s----------------
LEdxsp      200 ---vgrdlk--------grnnn-v----------------
MLdxsp      126 ---kafela--------gnrnrhv----------------
MTdxsp      126 ---kafelt--------ghrnrhv----------------
RCdxsp      135 ---vgrelg--------qpvgd-t----------------
RSdxs1p     134 ---aaremg--------gdtgd-a----------------
RSdxs2p     137 ---mgrelg--------qpvgd-t----------------
SPCCdxsp    127 ---lardaq--------gedyr-c----------------
SPdxsp      127 ---lardak--------gedfk-v----------------
TMdxsp      121 ---kafell--------gekrh-v----------------
ECdxsp      135 ---vaaeke--------gknrr-t----------------
NMdxsp      131 ---aadkql--------gsdrr-s----------------
HIdxsp      135 ---vaaere--------nagrk-t----------------
PFdxsp      582 dnninnninnnnyinpsdvvgr-entnvpnvrndnhnvdk
SSdxsp      127 ---karrvq--------gekgh-v----------------
HPdxsp      125 ---kafclk--------qalgm-p----------------
```

# Figure 6 (page 10 of 18)

```
STdxsp      617 --iavigdgamsagmayeamnna-eaagnr-lvvilndnd
AAdxsp      145 --iavigdgaltagmayealnnaghirpdr-fivilndne
BSdxsp      139 --ipiigdgaltggmalealnhi-gdekkd-mivilndne
CRdxsp      218 --iavigdgaitggmayeamnha-gfldkn-mivilndnq
CJdxsp      137 --valigdgalsagmayealnel-gdskfp-cvillndne
PAdxsp      154 --vavigdgaltagmafealnha-sevdad-mlvilndnd
LEdxsp      212 --iavigdgamtagqayeamnna-gyldsd-mivilndnr
MLdxsp      139 --vavvgdgaltggmcwealnni-aatprp-vvivvndng
MTdxsp      139 --vavvgdgaltggmcwealnni-aasrrp-viivvndng
RCdxsp      147 --iaiigdgsitagmayealnha-ghlksr-mfvilndnd
RSdxs1p     146 --vavigdgsmsagmafealnhg-ghlknr-vivilndne
RSdxs2p     149 --iavigdgsitagmayealnha-ghlnkr-lfvilndnd
SPCCdxsp    139 --vavigdgsltggmaleainhaghlpktr-llvvlndnd
SPdxsp      139 --vsiigdgaltggmaleainhaghlphtr-lmvilndne
TMdxsp      133 --vvvigdgaltsgmalealnql-knlnsk-mkiilndng
ECdxsp      147 --vcvigdgaitagmafeamnha-gdirpd-mlvilndne
NMdxsp      143 --vaiigdgamtagqafealnca-gdmdvd-llvvlndne
HIdxsp      147 --vcvigdgaitagmafealnha-galhtd-mlvilndne
PFdxsp      621 vhiaiigdggltggmalealnyi-sflnsk-iliiyndng
SSdxsp      139 --vaviggraltggmawealnni-aaakdqpliivvndne
HPdxsp      137 --iallgdgsisagifyealnel-gdrkyp-mimilndne

STdxsp      725 msiap----------pvgglsayl--arlissseyl--gl
AAdxsp      182 msisp----------nvgaistyl--nriisghfvq--et
BSdxsp      175 msiap----------nvgaihsml--grlrtagkyq--wv
CRdxsp      254 qvslptqynnknqd-pvgalssal--arlqanrplr--el
CJdxsp      173 msisk----------pigaiskyl--sqamatqfyq--sf
PAdxsp      190 msish----------nvgglsnyl--akilssrtys--sm
LEdxsp      248 qvslptatldgpva-pvgalssal--srlqsnrplr--el
MLdxsp      175 rsyap----------tiggvadhl--atlrlqpaye--rl
MTdxsp      175 rsyap----------tiggvadhl--atlrlqpay-----
RCdxsp      183 msiap----------pvgalqhyl--ntiarqapfa--al
RSdxs1p     182 msiap----------pvgalssyl--srlyagapfq--df
RSdxs2p     185 msiap----------pvgalaryl--vnlsskapfa--tl
SPCCdxsp    176 msisp----------nvgalsryl--nk-irvsepm--ql
SPdxsp      176 msisp----------nvgaisrylnkvrlsspmqfltdnl
TMdxsp      169 msisp----------nvgglayhl--sklrtspiyl--kg
ECdxsp      183 msise----------nvgalnnhl--aqllsgklys--sl
NMdxsp      179 msisp----------nvgalpkyl--asnvvrdmh---gl
HIdxsp      183 msise----------nvgalnnhl--arifsgslys--tl
PFdxsp      659 qvslptnavsisgnrpigsisdhl--hyfvsnie------
SSdxsp      176 rsyap----------tigglanhl--atlrttdgye--kv
HPdxsp      173 msist----------pigalskal--sqlmkgpfyq--sf
```

# Figure 6 (page 11 of 18)

```
STdxsp      803 relakrf---trk--lsr------rltaa---a-gkaeef
AAdxsp      208 rqkiknf---lqh--fge------tplri---m-klteef
BSdxsp      201 kdeleyl---fkk--ipavgg---klaat---a-ervkds
CRdxsp      289 reiakgv---tkq--lpd------vvqka---t-akidey
CJdxsp      199 kkriakm---ldi--lpd------satym---a-krfees
PAdxsp      216 regsk-------k--vls------rlpgaweia-rrteey
LEdxsp      283 revakgv---tkq--igg------pmhel---a-akvdey
MLdxsp      201 lekg-------rd--alh------slpli---g-qiayrf
MTdxsp      198 -eqalet---grd--lvr------avplv---g-glwfrf
RCdxsp      209 kaaaegi---emh--lpg------pvrdg---a-rrarqm
RSdxs1p     208 kaaakga---lgl--lpe------pfqeg---a-rrakem
RSdxs2p     211 raaadgl---eas--lpg------plrdg---a-rrarql
SPCCdxsp    201 --ltdgl---tqg--mqqipfvggaitqg---f-epvkeg
SPdxsp      206 eeqikhl---pf---vgd------sltpe---m-ervkeg
TMdxsp      195 kkvlkkv---lekteigf------eveee---m-kylrds
ECdxsp      209 reggkkv---fsg--vp-------pikel---l-krteeh
NMdxsp      204 lstvkaq---tgk--vld------kipgamefa-qkvehk
HIdxsp      209 rdgskki---ldk--vp-------piknf---m-kkteeh
PFdxsp      691 ---anag---dnk--lsk------n---------------
SSdxsp      202 lawgkdvllrtpi--vgh------plyea---lhgakkgf
HPdxsp      199 rskvkki---lst--lpe------svnyl---a-srfees

STdxsp      878 argm--atg-------g-------tlfeelgfyyvgpidg
AAdxsp      233 lkgl--isp-------g-------vifeelgfnyigpidg
BSdxsp      229 lkym--lvs-------g-------mffeelgftylgpvdg
CRdxsp      314 argmisgtg-------s-------tlfeelglyyigpvdg
CJdxsp      224 fk-l--itp-------g-------llfeelgleyigpidg
PAdxsp      240 akgm--lvp-------g-------tlfeelgwnyigpidg
LEdxsp      308 argmisgsg-------s-------tlfeelglyyigpvdg
MLdxsp      222 mhsv--kagikdslspq-------llftdlglkyvgpvdg
MTdxsp      222 lhsv--kagikdslspq-------llftdlglkyvgpvdg
RCdxsp      234 vtam--pgg-------a-------tlfeelgfdyigpvdg
RSdxs1p     233 lksv--tvg-------g-------tlfeelgfsyvgpidg
RSdxs2p     236 vtgm--pgg-------g-------tlfeelgftyvgpidg
SPCCdxsp    230 mkrl--syski-----g-------avfeelgftymgpvdg
SPdxsp      230 mkrl--vvpkv-----g-------avieelgfkyfgpidg
TMdxsp      222 lkgm--iqg-------t-------nffeslglkyfgpfdg
ECdxsp      233 ikgm--vvp-------g-------tlfeelgfnyigpvdg
NMdxsp      232 iktl--aee-------aehakqslslfenfgfrytgpvdg
HIdxsp      233 mkgvmfspe-------s-------tlfeelgfnyigpvdg
PFdxsp      702 ------ake-------n-------nifenlnydyigvvng
SSdxsp      231 kdaf--apq-------g--------mfedlglkyvgpidg
HPdxsp      224 fk-l--itp-------g-------vffeelginyigping
```

# Figure 6 (page 12 of 18)

```
STdxsp     950  hnlehlipvlenvrdse-q-gpilihvvtkkgkgyapaea
AAdxsp     257  hdikaledtlnnvkdi--k-gpvllhvytkkgkgykpaee
BSdxsp     253  hsyhelienlqyakkt--k-gpvllhvitkkgkgykpaet
CRdxsp     340  hnlddliavlsevrsae-tvgpvlvhvvtekgrgylpaet
CJdxsp     247  hnlgeiisalkqak-am-q-kpcvihaqtikgkgyalaeg
PAdxsp     264  hdlptlvatlrnmrdm--k-gpqflhvvtkkgkgfapael
LEdxsp     334  hniddliailkevrstk-ttgpvlihvvtekgrgypyaer
MLdxsp     253  hd-ehavevalrkargf-g-gpvivhvvtrkgmgyppaea
MTdxsp     253  hd-eravevalrsarrf-g-apvivhvvtrkgmgyppaea
RCdxsp     258  hdmaelvetlrvtrara-s-gpvlihvcttkgkgyapaeg
RSdxs1p    257  hdldqllpvlrtvkqra-h-apvlihvitkkgrgyapaea
RSdxs2p    260  hdmeallqtlraarart-t-gpvlihvvtkkgkgyapaen
SPCCdxsp   256  hnleeliatfreah-kh-t-gpvlvhvattkgkgypyaee
SPdxsp     256  hslqelidtfkqa-ekv-p-gpvfvhvsttkgkgydlaek
TMdxsp     246  hniellekvfkrirdyd-y-ssv-vhvvtkkgkgftaaee
ECdxsp     257  hdvlglittlknmrdl--k-gpqflhimtkkgrgyepaek
NMdxsp     263  hnvenlvdvledlr-gr-k-gpqllhvitkkgngyklaen
HIdxsp     259  hnidelvatltnmrnl--k-gpqflhiktkkgkgyapaek
PFdxsp     722  nnteelfkvlnnikenklk-ratvlhvrtkksndfinsks
SSdxsp     254  hdigavesalrrak-rf-h-gpvlvhcltvkgrgyepala
HPdxsp     247  hdlsaiietlklakelk-e--pvlihaqtlkgkgykiaeg

STdxsp    1064  -aadkyhgvqk-----fd--vitg-aqaka-----pp---
AAdxsp     294  -npvkwhgvap-----yk--vesg-eiik------ks---
BSdxsp     290  dtigtwhgtgp-----yk--intg-dfvkp-----ka---
CRdxsp     379  -aqdkmhgvvk-----fd--prtg-kqvqa-----kt---
CJdxsp     284  -khakwhgvga-----fd--idsg-esvkk-----sd---
PAdxsp     301  -dpigyhaitk-----le--apgs-apkkt----------
LEdxsp     373  -aadkyhgvak-----fd--patg-kqfka-----sa---
MLdxsp     290  dqaeqmhtcgv-----md--pttg-qptki----------
MTdxsp     290  dqaeqmhstvp-----id--patg-qatkv----------
RCdxsp     296  -aedklhgvsk-----fd--ietg-kqkks-----ip---
RSdxs1p    295  -ardrghatnk-----fn--vltg-aqvkp-----vs---
RSdxs2p    298  -apdkyhgvnk-----fd--pvtg-eqkks-----va---
SPCCdxsp   293  -dqvgyhaqnp-----fd--latgkakpas-----kp---
SPdxsp     293  -dqvgyhaqsp-----fn--lstgkaypss-----kp---
TMdxsp     283  -nptkyh-------------------sas-----ps---
ECdxsp     294  -dpitfhavpk-----fd--pssg-clpks-----sg---
NMdxsp     300  -dpvkyhavan-----lp--kesa-aqmpsekepkpa---
HIdxsp     296  -dpigfhgvpk-----fd--pisg-elpk------nn---
PFdxsp     761  -pisilhsikkneifpfdttilng-nihke-----nkiee
SSdxsp     291  heedhfhtvgv-----md--plt--cepls-----pt---
HPdxsp     284  -ryekwhgvgp-----fd--ldtg-lskks-----ks---
```

# Figure 6 (page 13 of 18)

```
STdxsp      1133 ---------------------------------gpp---ay
AAdxsp       316 ---------------------------------spp---tw
BSdxsp       314 ---------------------------------aap---sw
CRdxsp       402 ---------------------------------kam---sy
CJdxsp       307 ---------------------------------tkk---sa
PAdxsp       322 ---------------------------------ggp---ky
LEdxsp       396 ---------------------------------ktq---sy
MLdxsp       312 ---------------------------------aap---dw
MTdxsp       312 ---------------------------------agp---gw
RCdxsp       319 ---------------------------------nap---ny
RSdxs1p      318 ---------------------------------nap---sy
RSdxs2p      321 ---------------------------------nap---ny
SPCCdxsp     317 ---------------------------------kpp---sy
SPdxsp       317 ---------------------------------kpp---sy
TMdxsp       294 ---------------------------------gkpkmlsy
ECdxsp       317 ---------------------------------glp---sy
NMdxsp       328 ---------------------------------akp---ty
HIdxsp       318 ---------------------------------skp---ty
PFdxsp       794 eknvssstkydvnnknnknndnseiikyedmfske---tf
SSdxsp       314 ---------------------------------dgp---sw
HPdxsp       307 ---------------------------------ail---sp

STdxsp      1148 tkvfadallaeaerdasvcaitaampsgtgldkfqatfpd
AAdxsp       321 tsvfgkalvelaerdekivaitpamregsglvefakrfpd
BSdxsp       319 sglvsgtvqrmaredgrivaitpampvgsklegfakefpd
CRdxsp       407 tnyfadaltaeaerdsrivavhaamaggtglyrfekkfpd
CJdxsp       312 teifsknlldlaskyenivgvtaampsgtgldkliekypn
PAdxsp       327 ssvfgqwlcdmaaqdarllgitpamkegsdlvafserype
LEdxsp       401 ttyfaealiaeaeadkdivaihaamgggtgmnlfhrrfpt
MLdxsp       317 taifsdaligyamkrrdivaitaampgptgltafgqcfpd
MTdxsp       317 tatfsdaligyaqkrrdivaitaampgptgltafgqrfpd
RCdxsp       324 tavfgerlteeaardqaivavtaamptgtgldimqkrfpr
RSdxs1p      323 tkvfaqslikeaevdericavtaampdgtglnlfgerfpk
RSdxs2p      326 tkvfgstlteeaardprivaitaampsgtgvdimqkrfpn
SPCCdxsp     322 skvfgqtlttlaksdrrivgitaamatgtgldilqkalpk
SPdxsp       322 skvfahtlttlakenpnivgitaamatgtgldklqaklpk
TMdxsp       302 sellghtlsrvaredkkivaitaamadgtglsifqkehpd
ECdxsp       322 skifgdwlcetaakdnklmaitpamregsgmvefsrkfpd
NMdxsp       333 tqvfgkwlcdraaadsrlvaitpamregsglvefeqrfpd
HIdxsp       323 skifgdwlcemaekdakiigitpamregsgmvefsqrfpk
PFdxsp       831 tdiytnemlkylkkdrniiflspamlggsglvkiserypn
SSdxsp       319 tsvfgdeivrigaeredivaitaamlhpvglarfadrfpd
HPdxsp       312 teaysntllelakkdekivgvtaampsgtgldklidaypl
```

# Figure 6 (page 14 of 18)

```
STdxsp      1268 rtfdvaiaeqhavtfaaglaa-qgmrpfcaiystflqray
AAdxsp       361 rffdvgiaeqhactfaaglaa-eglrpvaayystflqray
BSdxsp       359 rmfdvgiaeqhaatmaaamam-qgmkpflaiystflqray
CRdxsp       447 rtfdvgiaeqhavtfaaglac-eglvpfctiystfmqrgy
CJdxsp       352 rfwdvaiaeqhavtsmaamak-egfkpfiaiystflqray
PAdxsp       367 ryfdvaiaeqhavtlaagmac-egmkpvvaiystflqray
LEdxsp       441 rcfdvgiaeqhavtfaaglac-egikpfcaiyssfmqray
MLdxsp       357 rlfdvgiaeqhamtsaaglam-grmhpvvaiystflnraf
MTdxsp       357 rlfdvgiaeqhamtsaaglam-gglhpvvaiystflnraf
RCdxsp       364 rvfdvgiaeqhavtfaagmaa-aglkpflalyssfvqrgy
RSdxs1p      363 rtfdvgiaeqhavtfsaalaa-ggmrpfcaiystflqrgy
RSdxs2p      366 rvfdvgiaeqhavtfaaglag-agmkpfcaiyssflqrgy
SPCCdxsp     362 qyidvgiaeqhavvlaagmac-dgmrpvvaiystflqraf
SPdxsp       362 qyvdvgiaeqhavtlaagmac-egirpvvaiystflqrgy
TMdxsp       342 rffdlgiteqtcvtfgaalgl-hgmkpvvaiystflqray
ECdxsp       362 ryfdvaiaeqhavtfaaglai-ggykpivaiystflqray
NMdxsp       373 ryfdvgiaeqhavtfagglac-egmkpvvaiystflqray
HIdxsp       363 qyfdvaiaeqhavtfatglai-ggykpvvaiystflqray
PFdxsp       871 nvydvgiaeqhsvtfaaamamnkklkiqlciystflqray
SSdxsp       359 rvwdvgiaeqhaavsaaglat-gglhpvvavyatflnraf
HPdxsp       352 rffdvaiaeqhaltsssamak-egfkpfvsiystflqray

STdxsp      1385 dqvvhdvaiqnlpvrfaidraglvgadgathagsfdvtyl
AAdxsp       400 dqvihdvalqnlpvtfaidraglvgddgpthhgvfdlsyl
BSdxsp       398 dqvvhdicrqnanvfigidraglvgadgethqgvfdiafm
CRdxsp       486 dqivhdvslqklpvrfamdraglvgadgsthcgafdvtfm
CJdxsp       391 dqvihdcaimnlnvvfamdragivgedgethqgvfdlsfl
PAdxsp       406 dqlihdvavqhldvlfaidraglvgedgpthagsfdisyl
LEdxsp       480 dqvvhdvdlqklpvrfamdraglvgadgpthcgafdvtym
MLdxsp       396 dqimmdvalhklpvtmvidragitgsdgpshngmwdlsml
MTdxsp       396 dqimmdvalhklpvtmvldragitgsdgashngmwdlsml
RCdxsp       403 dqlvhdvalqnlpvrlmidraglvgqdgathagafdvsml
RSdxs1p      402 dqivhdvaiqrlpvrfaidraglvgadgathagsfdvafl
RSdxs2p      405 dqiahdvalqnlpvrfvidraglvgadgathagafdvgfi
SPCCdxsp     401 dqvihdvciqklpvffcldragivgadgpthqgmydiayl
SPdxsp       401 dqiihdvciqklpvffcldragivgadgpthqgmydiayl
TMdxsp       381 dqiihdvalqnapvlfaidrsgvvgedgpthhglfdinyl
ECdxsp       401 dqvlhdvaiqklpvlfaidragivgadgqthqgafdlsyl
NMdxsp       412 dqlvhdialqnlpvlfavdragivgadgpthaglydlsfl
HIdxsp       402 dqlihdvaiqnlpvlfaidragivgadgathqgafdisfm
PFdxsp       911 dqiihdlnlqniplkviigrsglvgedgathqgiydlsyl
SSdxsp       398 dqllmdvalhrcgvtfvldragvtgvdgashngmwdmsvl
HPdxsp       391 dsivhdacisslpiklaidragivgedgethqglldvsyl
```

# Figure 6 (page 15 of 18)

```
STdxsp      1505 aslpnfvvmaaadevelvhmthtaamhdsg-pialryprg
AAdxsp       440 rcvpnmvvcapkdeqelrdllytg-iysgk-pfalryprg
BSdxsp       438 rhipnmvlmmpkdenegqhmvhtalsydeg-piamrfprg
CRdxsp       526 aslphmitmapsneaelinmvatcaaidda-pscfrfprg
CJdxsp       431 aplpnftllaprdeqmmqnimeyaylh-qg-pialryprg
PAdxsp       446 rcipgmlvmtpsdedelrklltgylfd-g-paavryprg
LEdxsp       520 aclpnmvvmapsdeaelfhmvataaaiddr-pscfryprg
MLdxsp       436 givpgmrvaaprdairlreelgealdvddg-ptairfpkg
MTdxsp       436 givpgirvaaprdatrlreelgealdvddg-ptalrfpkg
RCdxsp       443 anlpnftvmaaadeaelchmvvtaaahdsg-pialryprg
RSdxs1p      442 snlpgivvmaaadeaelvhmvataaahdeg-piafryprg
RSdxs2p      445 tslpnmtvmaaadeaelihmiatavafgeg-piafrfprg
SPCCdxsp     441 rlipnmvlmapkdeaelqrmlvtgieyd-g-piamrfprg
SPdxsp       441 rcipnlvlmapkdeaelqqmlvtgvnytgg-aiamryprg
TMdxsp       421 lpvpnmkiispsspeefvnslytvlkhldg-pvairypke
ECdxsp       441 rcipemvimtpsdenecrqmlytgyhyndg-psavryprg
NMdxsp       452 rcipnmivaapsdenecrlllstcyqada--paavryprg
HIdxsp       442 rcipnmiimtpsdenecrqmlytg--yqcgkpaavryprg
PFdxsp       951 gtlnnayiispsnqvdlkralrfayldkdh-svyiriprm
SSdxsp       438 qvvpglriaaprdadhvraqlreavavdda-ptlirfpk-
HPdxsp       431 rsipnmvifaprdnetlknavrfanehdss-pcafryprg

STdxsp      1622 n---------gvglalpk---------vp-erle------
AAdxsp       478 a---------aygvpteg---------f--kkie------
BSdxsp       477 n---------glgvkmde---------ql-ktip------
CRdxsp       565 n---------glgldlaaygiskdlkgvp---le------
CJdxsp       469 s---------fi-ldkef---------np-ceik------
PAdxsp       484 s---------gpnhpidp---------dl-qpve------
LEdxsp       559 n---------gigvelpagnkg-----ip---le------
MLdxsp       475 d----------vcedipa---------lk-rrsg------
MTdxsp       475 d----------vgedisa---------le-rrgg------
RCdxsp       482 e---------grgvempe---------rg-evle------
RSdxs1p      481 d---------gvgvevpv---------kg-vplq------
RSdxs2p      484 e---------gvgvempe---------rg-tvle------
SPCCdxsp     479 n---------gigvplpe---------egweslp------
SPdxsp       480 n---------gigvplme---------egweple------
TMdxsp       460 s---------fygevesl---------le-nmke------
ECdxsp       480 n---------avgveltp---------l--eklp------
NMdxsp       490 t---------gtgvpvsd---------gm-etve------
HIdxsp       480 n---------avgvkltp---------l--emlp------
PFdxsp       990 nilsdkymkgylnihmkn---------es-knidvnvdin
SSdxsp       476 e---------svg---pr---------ip-aldr------
HPdxsp       470 s------------falkegvf------ep-sgfv------
```

# Figure 6 (page 16 of 18)

| | | |
|---|---|---|
| STdxsp | 1667 | --------------------igkg-r-vvr---------- |
| AAdxsp | 492 | --------------------igtw-e-ell---------- |
| BSdxsp | 492 | --------------------igtw-e-vlr---------- |
| CRdxsp | 587 | --------------------vgkg-v-vrr---------- |
| CJdxsp | 483 | --------------------lgka-qwlvk---------- |
| PAdxsp | 499 | --------------------igkg-v-vrr---------- |
| LEdxsp | 576 | --------------------vgkg-r-ili---------- |
| MLdxsp | 489 | --------------------vdvl-a-vpa---------- |
| MTdxsp | 489 | --------------------vdvl-a-apa---------- |
| RCdxsp | 497 | --------------------igkg-r-vmt---------- |
| RSdxs1p | 496 | --------------------igrg-r-vvs---------- |
| RSdxs2p | 499 | --------------------pgrg-r-vvr---------- |
| SPCCdxsp | 495 | --------------------igka-e-qlr---------- |
| SPdxsp | 496 | --------------------igka-e-ilr---------- |
| TMdxsp | 475 | --------------------idlgwk-ilk---------- |
| ECdxsp | 494 | --------------------igkg-i-vkr---------- |
| NMdxsp | 505 | --------------------igkg-i-irr---------- |
| HIdxsp | 494 | --------------------igks-r-lir---------- |
| PFdxsp | 1020 | ddvdkyseeymdddnfiksfigks-r-iikmdnennntne |
| SSdxsp | 488 | --------------------vggl-d-vlhrd-------- |
| HPdxsp | 485 | --------------------lgqs-e-llk---------- |

| | | |
|---|---|---|
| STdxsp | 1691 | ----------eg--kk--vailslgtrlaealkaadtlea |
| AAdxsp | 500 | ----------eg--ed--cvilavgypvyqalraaeklyk |
| BSdxsp | 500 | ----------pg--nd--aviltfgttiemaieaaeelqk |
| CRdxsp | 595 | ----------qg--kd--vclvaygssvnealaaadmler |
| CJdxsp | 492 | ----------nn--se--iaflgygqgvakawqvlralqe |
| PAdxsp | 507 | ----------rg--gr--vallvfgvqlaeamkvaeslda |
| LEdxsp | 584 | ----------eg--er--vallgygsavqncldaaivles |
| MLdxsp | 497 | ----------tglaqd--vllvgvgvfasmalavakrlhn |
| MTdxsp | 497 | ----------dg--lnhdvllvaigafapmalavakrlhn |
| RCdxsp | 505 | ----------eg--te--vailsfgahlaqalkaaemlea |
| RSdxs1p | 504 | ----------eg--tr--iallsfgtrlaevqvaaealaa |
| RSdxs2p | 507 | ----------eg--td--vailsfgahlhealqaakllea |
| SPCCdxsp | 503 | ----------qg--dd--llmlaygsmvypalqtaellne |
| SPdxsp | 504 | ----------sg--dd--vlllgygsmvypalqtaellhe |
| TMdxsp | 484 | ----------rg--re--aaiiatgtilnevlkip----- |
| ECdxsp | 502 | ----------rg--ek--lailnfgtlmpeaakvaeslna |
| NMdxsp | 513 | ----------eg--ek--tafiafgsmvapalavagklna |
| HIdxsp | 502 | ----------kg--qk--iailnfgtllpsalelseklna |
| PFdxsp | 1058 | hyssrgdtqtkk--kk--vcifnmgsmlfnvinaikeiek |
| SSdxsp | 498 | ----------er--pe--vllvavgvmaqvclqtaellra |
| HPdxsp | 493 | ----------ke--ge--illigygngvgrahlvqlalke |

# Figure 6 (page 17 of 18)

```
STdxsp       1769 k-----glsttvadlrfakpldedlirrll--tthevavt
AAdxsp        526 e-----girvgvvnarfvkpmdekmlrdla--nrydtfit
BSdxsp        526 e-----glsvrvvnarfikpidekmmksil--keglpilt
CRdxsp        621 d-----gvsttvidarfckpldtklirsaa--kehpvmit
CJdxsp        518 m-----nnnanlidlifakpldeellcela--kkskiwfi
PAdxsp        533 ----------tvvdmrfvkpldealvrela--gshellvt
LEdxsp        610 r-----glqvtvadarfckpldhalirsla--kshevlit
MLdxsp        525 q-----gigvtvidprwvlpvcdgvl-ela--hthklivt
MTdxsp        525 q-----gigvtvidprwvlpv-sdgvrela--vqhkllvt
RCdxsp        531 e-----gvsttvadarfcrpldtdlidrli--eghaalit
RSdxs1p       530 r-----gisptvadarfakpldrdlilqla--ahhealit
RSdxs2p       533 e-----gvsvtvadarfsrpldtghidqlv--rhhaalvt
SPCCdxsp      529 h-----gisatvinarfakpldeelivpla--rqigkvvt
SPdxsp        530 h-----gieatvvnarfvkpldtelilpla--erigkvvt
TMdxsp        505 -------ldvtvvnaltvkpldtavlkeia--rdhdliit
ECdxsp        528 ----------tlvdmrfvkpldealilema--ashealvt
NMdxsp        539 ----------tvadmrfvkpideelivrla--rshdrivt
HIdxsp        528 ----------tvvdmrfvkpidieminvla--qthdylvt
PFdxsp       1094 eqyishnysfsivdmiflnpldknmidhvikqnkhqylit
SSdxsp        524 r-----gigctvvdprwvkpv-dpvlppla--aehrlvav
HPdxsp        519 k-----niecalldlrflkpldpnlsaiva--pyqklyvf

STdxsp       1868 ieega-i-ggpgahv----ltlasdtglida-glklrtmr
AAdxsp        559 vednt-vvggfgsgv----leffaregimk----rvinlg
BSdxsp        559 ieeav-leggfgssi----lefahdqg--ey-htpidrmg
CRdxsp        654 ieegs-v-ggfaahv----mqflaleglldg-glkfrpmt
CJdxsp        551 fsenvki-ggiesli----nnflqk---ydl-hvkvvsfe
PAdxsp        561 ieena-vmggagsav----geflasegl----evpllqlg
LEdxsp        643 veegs-i-ggfgshv----vqfmaldglldg-klkwrpiv
MLdxsp        557 ledng-vnggvgaav----stalrq---vei-dtpcrdvg
MTdxsp        557 ledng-v-nggagsa----vsaalrraeid---vpcrdvg
RCdxsp        564 leqga-m-ggfgamv----lhylartgqlek-grairtmt
RSdxs1p       563 ieega-i-ggfgshv----aqllaeagvfdr-gfryrsmv
RSdxs2p       566 veqga-m-ggfgayv----mhclansggfdg-glalrvmt
SPCCdxsp      562 feegc-l---pggfg----saimeslqahdl-qvpvlpig
SPdxsp        563 meegc-lmggfgsav----aealmdnnvl----vplkrlg
TMdxsp        536 veeamki-ggfgsfv----aqrlqemgwqg----kivnlg
ECdxsp        556 veena-imggagsgvnevlmahrkpvpvlni-g-------
NMdxsp        567 leena-eqggagsav----levlakhgickp-vlll---g
HIdxsp        556 leena-iqggagsav----aevlnssgksta-llql---g
PFdxsp       1134 yednt-i-ggfsthf----nnyliennyitkhnlyvhniy
SSdxsp        556 vednsra-agvgsav----alalgda---dv-dvpvrrfg
HPdxsp        552 sdnyk-l-ggvasai----leflseqnilk----pvksfe
```

# Figure 6 (page 18 of 18)

```
STdxsdna     1967 lpdifqdqdkpekqydeaglnaanivdtvl-k-al-ryne
AAdxsp        590 vpdrfiehgkqdilrnlvgidaegiekavr-d-al-kggr
BSdxsp        591 ipdrfiehgsvtalleeigltkqqvanrir-l-lm----p
CRdxsp        687 lpdryidhgdyrdqlamagltsqhiastal-t-tlgrakd
CJdxsp        582 yedkfiehgkts----eveknlekdvnslltk-vl-kfyh
PAdxsp        592 lpdyyvehakpsemlaecgldaagiekavr-q-rl-drq-
LEdxsp        676 lpdryidhgspvdqlaeagltpshiaatvf-n-il-gqtr
MLdxsp        588 lpqefydhasrsevladlgltdqdvarrit-gwvv-afgh
MTdxsp        588 lpqefyehasrsevladlgltdqdvarrit-g-wv-----
RCdxsp        597 lpdcyidhgspeemyawagltandirdtal-a-aa-rpsk
RSdxs1p       596 lpdtfidhnsaevmyataglnaadierkal-e-tl---gv
RSdxs2p       599 lpdrfieqaspedmyadaglraediaatar-g-al-argr
SPCCdxsp      593 vpdllvehaspdeskqelgltprqmadril-e----kfgs
SPdxsp        594 vpdilvdhatpeqstvdlgltpaqmaqnim-a-sl-fkte
TMdxsp        567 vedlfvphggrkellsmlgldsegltktv-----l-tyik
ECdxsp        587 lpdffipqgtqeemraelgldaagmeaki----------k
NMdxsp        598 vadtvtghgdpkklldlglsaeaverrvr-a-wl---sd
HIdxsp        587 lpdyfipqatqqealadlgldtkgieekil-n-fi-a-kq
PFdxsp       1168 lsnepiehasfkdqqevvkmdkcslvnrik-n-yl-knnp
SSdxsp        587 ipeqflaharrgevladigltpveiagrig-a-sl-pvre
HPdxsp        582 iidefimhgntalvekslgldtesltdail-k-dl-gqer

STdxsdna     2078 a----e--l--ad-----gvra*--------
AAdxsp        627 l----i------------------------
BSdxsp        625 p----k--t--hk-----gigs---------
CRdxsp        725 a----a--kfsls-----alqa---------
CJdxsp        616 ------------------------------
PAdxsp        628 ------------------------------
LEdxsp        713 e----a--l--ev-----mt-----------
MLdxsp        626 c----g--s--gddagqygprssqtm-----
MTdxsp        621 a----a--l--gt-----gvcasdaipehld
RCdxsp        634 sv---r--i--vh-----sa-----------
RSdxs1p       631 e----v--l--ar-------ra---------
RSdxs2p       636 vmplrq--t--ak-----prav---------
SPCCdxsp      628 r----q--r--ig-----aasa---------
SPdxsp        631 t----esvv--ap-----gvs----------
TMdxsp        601 a----r--s--re-----gkv----------
ECdxsp        617 a----w--l--a-------------------
NMdxsp        633 r----d--a--an------------------
HIdxsp        623 g----n--l----------------------
PFdxsp       1205 t------------------------------
SSdxsp        624 -----e--p--ae-----eqpa---------
HPdxsp        619 ------------------------------
```

# Figure 7

1 cgacggcccg gtagccccgg cgcggctgca gcaccgtcag acgtccgccg
51 agaaagccgt cggaagtcaa ttcgtccggg gcgaacatca gggggtcgtc
101 gggatgccgt tgtcggacat cacccggcag gcgcgatccc agtcttcttc
151 cgggacaaac agacgccgcg gcaatatgcc gatggagcct tcgaggacgc
201 tcatgtggac gtccaccgga aaggcgtcta tatcctcgcc ctgaaggagc
251 gcggtggcga aggcgatgat cgtcgggtcg gtcgtgcgca acagttcctt
301 catgtcgggg acattgtcgg caacgcctcg gtttgtcgag gccggttcgt
351 cgaccgggtg gcaggatcgg gatgggattg gacgaggttt cgcaaaagcc
401 gcatgaacgg ctcgccgcgt ggctggccga ggacatggcc gccgtcaacg
451 ggctgatccg cgagcggatg gcctcgaaac acgcgccccg cattcccgag
501 gtcacggcgc atctggtcga ggccggcggc aagcggctgc ggccgctcct
551 gacgctcgcc gcggcgcggc tgtgcggcta cgagggggccc tatcacatcc
601 atctggccgc gacggtggag ttcatccaca cggcgacgct gcttcacgac
651 gatgtggtgg acgaaagcca ccgccgccgc ggcaaaccca cggcgaacct
701 gctgtgggac aacaaatcct cggtgctggt gggcgactat ctcttcgccc
751 gcagcttcca gctgatggtc gagaccggct cgcttcgcgt gatggacatc
801 ctcgccaatg cctcggccac catctccgag ggcgaggtgc tgcagctgac
851 cgcggcccag gatctgcgca cgaccgagga catccacctg caggtggtgc
901 gcggcaagac ggccgcgctc tttgccgcgg caaccgaggt gggcggcgtg
951 gtcgcgggcg tgcccgaggc gcaggtcgag gcgctccacg cctacgggga
1001 cgcgctgggg atcgccttcc agatcgtcga cgacctcctc gattatggcg
1051 gcgtggatgc ccagatcggc aagaacaccg gcgacgactt ccgcgaacgc
1101 aagctgacgc tgccggtcat caaggcggtg gcccaggccg atgccgagga
1151 gcgcgccttc tggcagcggg tgatcgagaa gggcgaccag cgcgagggtg
1201 acctcgagca agcccatgcg atcatgtccc gccacggcgc catggaggcc
1251 gcccggcagg atgcgctccg ctgggtcacg gtggcgcgcg aggcactcgg
1301 ccagctgccg gagcacccgc tgcgcgagat gctgcacgat ctggccgatt
1351 tcgtggtcga acgcatcgcc tgatcccttc cgggcgctct gccccggcgc
1401 agcgcaggat cccgcgctgc gcccctttcg gccttccgac agtccctctg
1451 ccgcgggagg ccggcctcgc ctgagaagcc gcactggccg ccggtcttcc
1501 cccgaaccgc tcccgggcct gctcggaagg cgtccgccgc aaaagccccc
1551 gcggggggc cccaccggcg gccatcagga agagaccgtt gaagcggccc
1601 gctcgaatcc tgtcgcgccc ccccccgacc gggcggctct ccgatccgtg
1651 ttcgctcggc gatggacagc cgttccctgt ccgttcatga tggcgccatg
1701 cagaccctta ccgttcccga ttccggcctc gccccctcct gcccggccaa
1751 aggctcgccc gcggcgtctg ccgccatctg cgcagccatg atttcgtctc
1801 ggtggtcgaa ctcgtgcccg cgcccggcct caggtcgac gtgatggcgc
1851 tggggcccaa gggcgagatc tgggtggtgg aatgcaaatc ctcgcgcgcg
1901 gactatcagt ccgaccgcaa gtggcagggc tatctcgact ggtgcgaccg
1951 cttcttcttc gcggtggacg aggaccagcc cgggccgtcg (SEQ ID NO:37)

# Figure 8

```
   1  atgggattgg acgaggtttc gcaaaagccg catgaacggc tcgccgcgtg
  51  gctggccgag gacatggccg ccgtcaacgg gctgatccgc gagcggatgg
 101  cctcgaaaca cgcgccccgc attcccgagg tcacggcgca tctggtcgag
 151  gccggcggca agcggctgcg gccgctcctg acgctcgccg cggcgcggct
 201  gtgcggctac gaggggccct atcacatcca tctggccgcg acggtggagt
 251  tcatccacac ggcgacgctg cttcacgacg atgtggtgga cgaaagccac
 301  cgccgccgcg gcaaacccac ggcgaacctg ctgtgggaca acaaatcctc
 351  ggtgctggtg ggcgactatc tcttcgcccg cagcttccag ctgatggtcg
 401  agaccggctc gcttcgcgtg atggacatcc tcgccaatgc ctcggccacc
 451  atctccgagg gcgaggtgct gcagctgacc gcggcccagg atctgcgcac
 501  gaccgaggac atccacctgc aggtggtgcg cggcaagacg gccgcgctct
 551  ttgccgcggc aaccgaggtg ggcggcgtgg tcgcgggcgt gcccgaggcg
 601  caggtcgagg cgctccacgc ctacggggac gcgctgggga tcgccttcca
 651  gatcgtcgac gacctcctcg attatggcgg cgtggatgcc cagatcggca
 701  agaacaccgg cgacgacttc cgcgaacgca agctgacgct gccggtcatc
 751  aaggcggtgg cccaggccga tgccgaggag cgcgccttct ggcagcgggt
 801  gatcgagaag ggcgaccagc gcgagggtga cctcgagcaa gcccatgcga
 851  tcatgtcccg ccacggcgcc atggaggccg cccggcagga tgcgctccgc
 901  tgggtcacgg tggcgcgcga ggcactcggc cagctgccgg agcacccgct
 951  gcgcgagatg ctgcacgatc tggccgattt cgtggtcgaa cgcatcgcct
1001  ga   (SEQ ID NO:38)
```

# Figure 9

```
  1  mgldevsqkp herlaawlae dmaavnglir ermaskhapr ipevtahlve
 51  aggkrlrpll tlaaarlcgy egpyhihlaa tvefihtatl lhddvvdesh
101  rrrgkptanl lwdnkssvlv gdylfarsfq lmvetgslrv mdilanasat
151  isegevlqlt aaqdlrtted ihlqvvrgkt aalfaaatev ggvvagvpea
201  qvealhaygd algiafqivd dlldyggvda qigkntgddf rerkltlpvi
251  kavaqadaee rafwqrviek gdqregdleq ahaimsrhga meaarqdalr
301  wvtvarealg qlpehplrem lhdladfvve ria (SEQ ID NO:39)
```

# Figure 10

```
   1 ggatcgcgca gcgcctcggc cacgcgcacc atcagcagca gattgccgtt
  51 cggcagccgc gcgaagccgg ggttgaaggc gccaaggaca taggtcgcgt
 101 cgtccacccc ctcgcgcagc ggtgagcggg tcaggtcgac attgtcgggc
 151 cggaagatca gataatcgtc gctcaagcgc ttgcccccctc gggtttcacg
 201 cccagcaacg gggtcaggcc ccgggggttc cggcttcagc gccggcttcc
 251 tgggcctggc ggtggtgccg gatcacctcg tcgatgatga agcgcaggaa
 301 tttctcggaa aattcggggt cgagatcggc atcctgcgcc agcgcgcgca
 351 gccgggcgat ctgcgcctcc tcgcggccgg gatcggcggg cggcagcccg
 401 gattcggcct tgtagcgccc caccgcctgg gtcaccttga accgctcggc
 451 gagcatgaag acgagcgccg catcgatatt gtcgatgctc tggcgatagc
 501 gggtcagcgt cgcgtcggtc atgcgaatct cctttgccgc tgcggcacgg
 551 ccatgcaagc acctcttgcc tttgcaatgc acaaaggcca gaggctcgtt
 601 gcatatgagc gcaaccgtcc accgcctggg ctcgcgaacc cagccttcgc
 651 tcgatccgat catggcgctg gtcgcccagg acatgaacct ggtgaacgcg
 701 gtgatcctcg atcgcatgca gtccgagatc ccgctgatcc ccgaactcgc
 751 cggccatctg atcgctggcg gcggcaagcg gatgcggccg atgctgacgc
 801 tcgccagcgc ccggctgctc ggctattcgg gcacgcgcca ccacaagctg
 851 gcggcggcag tggagttcat ccacaccgcg acgctgctgc atgacgacgt
 901 ggtcgacagc tcggacctgc gccgcggccg ccgcaccgcc aacatcatct
 951 ggggcaatcc cgccagcgtg ctggtcggcg acttcctgtt cagccgctcg
1001 ttcgagctga tggtcgaggc cgaaagcctc aaggcgctgc acatcctgtc
1051 gaacgccagc gcggtgatcg ccgagggcga agtcaaccag ctgaccgcgg
1101 tgcgccggat cgacctgtcc gaggatcgct atctcgacat catcggcgcc
1151 aagactgcgg cgctgttcgc cgccgcctgc cgggtggcgg gcgtggtcgc
1201 cgagcgtccc gaggcggagg aactcgcgct cgacgcctat ggccgcaacc
1251 tcggcatcgc tttccagctg gtcgacgacg cgatcgacta tgtctcggac
1301 gcgtcgacga tgggcaagga tgccggcgac gatttccgcg aaggcaagat
1351 gacgctgccg gtggtcctgg cgtacgcgcg cggcgacgag gcggaacgcg
1401 gcttctggaa ggaagcgatt tcgggccgcc gcatctcgga cgaggatttc
1451 gccgaggcga tccggctggt gcagagctgc cgcgcggtgg acgacacgct
1501 cgcccgtgcc cgccattacg gccagctcgc gatcgatgcg ctgggcggct
1551 tccgcgcctg cgaggcgaag gacgcgatgg tcgaggcggt cgaattcgcg
1601 gtggcgcgcg cctactgacg cgcgccgacc ggagcatttc cgggtggatc
1651 gcttgcgatc caaggctcgg gaaatgcgac catcaaaaag cttccgggga
1701 ttacgcctcg gtcgactttt cttcgccctc gtcctcgtcg acttcgagcg
1751 cgtcttcctc gtccatgtcg agcactacct cgatgccctc gacgatcagg
1801 tcgagctgct cgtagctcgc cgtcatctcg atc  (SEQ ID NO:40)
```

# Figure 11

```
   1  atgagcgcaa ccgtccaccg cctgggctcg cgaacccagc cttcgctcga
  51  tccgatcatg gcgctggtcg cccaggacat gaacctggtg aacgcggtga
 101  tcctcgatcg catgcagtcc gagatcccgc tgatccccga actcgccggc
 151  catctgatcg ctggcggcgg caagcggatg cggccgatgc tgacgctcgc
 201  cagcgcccgg ctgctcggct attcgggcac gcgccaccac aagctggcgg
 251  cggcagtgga gttcatccac accgcgacgc tgctgcatga cgacgtggtc
 301  gacagctcgg acctgcgccg cggccgccgc accgccaaca tcatctgggg
 351  caatcccgcc agcgtgctgg tcggcgactt cctgttcagc cgctcgttcg
 401  agctgatggt cgaggccgaa agcctcaagg cgctgcacat cctgtcgaac
 451  gccagcgcgg tgatcgccga gggcgaagtc aaccagctga ccgcggtgcg
 501  ccggatcgac ctgtccgagg atcgctatct cgacatcatc ggcgccaaga
 551  ctgcggcgct gttcgccgcc gcctgccggg tggcgggcgt ggtcgccgag
 601  cgtcccgagg cggaggaact cgcgctcgac gcctatggcc gcaacctcgg
 651  catcgctttc cagctggtcg acgacgcgat cgactatgtc tcggacgcgt
 701  cgacgatggg caaggatgcc ggcgacgatt tccgcgaagg caagatgacg
 751  ctgccggtgg tcctggcgta cgcgcgcggc gacgaggcgg aacgcggctt
 801  ctggaaggaa gcgatttcgg gccgccgcat ctcggacgag gatttcgccg
 851  aggcgatccg gctggtgcag agctgccgcg cggtggacga cacgctcgcc
 901  cgtgcccgcc attacggcca gctcgcgatc gatgcgctgg gcggcttccg
 951  cgcctgcgag gcgaaggacg cgatggtcga ggcggtcgaa ttcgcggtgg
1001  cgcgcgccta ctga  (SEQ ID NO:41)
```

# Figure 12

```
  1  msatvhrlgs rtqpsldpim alvaqdmnlv navildrmqs eiplipelag
 51  hliagggkrm rpmltlasar llgysgtrhh klaaavefih tatllhddvv
101  dssdlrrgrr taniiwgnpa svlvgdflfs rsfelmveae slkalhilsn
151  asaviaegev nqltavrrid lsedryldii gaktaalfaa acrvagvvae
201  rpeaeelald aygrnlgiaf qlvddaidyv sdastmgkda gddfregkmt
251  lpvvlayarg deaergfwke aisgrrisde dfaeairlvq scravddtla
301  rarhygqlai dalggfrace akdamveave favaray (SEQ ID NO:42)
```

# Figure 13 (page 1 of 5)

```
RSddsdna        372 atg----------------------------ggattggac
STddsdna        605 atg----------------------------agcgcaacc
SPddsdna          1 atgattcagtatgtatatttaaaacatatgaggaaattat
GSddsdna          1 ----------------------------------------
RCddsdna          1 atg----------------------------gccatcga-

RSddsdna        384 ga---------ggtttcgcaaaagccgcat------gaac
STddsdna        617 gtccaccgcctgggctcgcgaacccagccttcgctcgatc
SPddsdna         41 gg---------agtcttggaaaagtccgtt------cgac
GSddsdna          1 ----------------------------------------
RCddsdna         12 -------------tttc---aa----gcaa------gata

RSddsdna        409 ggctcgccgcgtggctggccgaggacatggccgccgtca-
STddsdna        657 cgatcatggcgctggtcgcccaggacatgaacctggtga-
SPddsdna         66 tgttcttcggttttct--actacgaaccgcaatgcttcac
GSddsdna          1 --------------------------atgctggcctgca-
RCddsdna         26 ttctcg-ctcctg--ttgctcaagatttgcagcgatgg-

RSddsdna        448 acgggctgatccgcgagcggatggcctcgaaaca---cgc
STddsdna        696 acgcggtgatcctcgatcgcatgcagtccgagat---c--
SPddsdna        104 atttaattaaaaacgag---------ttggaacaaatctc
GSddsdna         14 accgggcgatcatcgcccggatg-----gaaagt---ccg
RCddsdna         62 accagtttattaatgaaggaatcagctccaaggt---cgc

RSddsdna        485 g--ccccgcattc--------ccgaggtca----cggcgc
STddsdna        731 ---ccgctgatcc--------ccgaactcg----ccggcc
SPddsdna        135 a--ccagggattcgtcaaatgctgaattcaaattcagaat
GSddsdna         46 gttcccctgatcc--------cgcagcttg----gcgccc
RCddsdna         99 a--ctggtcatgt--------c---agtca----gcaagc

RSddsdna        511 atctggtcgag---------------------gccggcgg
STddsdna        756 atctgatcgct---------------------ggcggcgg
SPddsdna        173 ttcttgaagagtgttctaaatattataccattgctcaagg
GSddsdna         74 atcttgtcgcg---------------------gcgggagg
RCddsdna        122 atgtcgttgaa---------------------gcaggtgg

RSddsdna        530 caagcggctgcggccgc---------tcctgacgctcgcc
STddsdna        775 caagcggatgcggccga---------tgctgacgctcgcc
SPddsdna        213 aaaacaaatgcgtccttctcttgttttgctgatgtccaaa
GSddsdna         93 caagcgccttcgcccgc---------tgctgacgctggcc
RCddsdna        141 aaagcgcatgcgtccga---------ttatg-tgcttgct
```

# Figure 13 (page 2 of 5)

```
RSddsdna        561 gcggcgcggctgtgc---ggctacgag--gggccc-----
STddsdna        806 agcgcccggctgctc---ggctattcg--ggcacg-----
SPddsdna        253 gctacaagcttgtgccatggtattgat--cggtccgtagt
GSddsdna        124 tccgcacgtctgtgc---ggttatcagccgggtcc-----
RCddsdna        171 g----gccgct-tat---gcctgtggt--gaaacc-----

RSddsdna        591 ---------------------------t----atcacatc
STddsdna        836 ---------------------------c----gccaccac
SPddsdna        291 gggcgacaaatatattgatgatgatgat----ttaagatc
GSddsdna        156 ---------------------------ggaccatcagcgt
RCddsdna        196 ---------------------------a----atttaaag

RSddsdna        600 cat---------ctggccgcgacggtg-------------
STddsdna        845 aag---------ctggcggcggcagtg-------------
SPddsdna        327 att---------ttcgacgggtcaaattcttccttctcaa
GSddsdna        169 catgtcggg---ctcgccgcctgcgtt-------------
RCddsdna        205 catgcacagaagctggcggccattatt-------------

RSddsdna        618 ---------------------gagttcatccacacggcga
STddsdna        863 ---------------------gagttcatccacaccgcga
SPddsdna        358 ttgagattagcacaaataaccgagatgatccatatagcaa
GSddsdna        193 ---------------------gagttcattcataccgcca
RCddsdna        232 ---------------------gaaatgctgcatacggcga

RSddsdna        637 cgctgcttcacgacgatgtggtggacgaaagccaccgccg
STddsdna        882 cgctgctgcatgacgacgtggtcgacagctcggacctgcg
SPddsdna        398 gtttgctgcatgacgatgtgattgatcacgctaatgtccg
GSddsdna        212 cactgctgcatgatgatgtcgtggatgagagcacgttgcg
RCddsdna        251 ctctggtacatgatgatgatgtagatgagtctggcttacg

RSddsdna        677 ccgcggcaaacccacg-gcgaacctgctgtgggacaacaa
STddsdna        922 ccgcggccgccgcacc-gccaacatcatctggggcaatcc
SPddsdna        438 tagaggctcaccttcaagcaatgttgctttcgg-----ta
GSddsdna        252 tcgggggctggcttcg-gccaatgccgtgttcggcaacaa
RCddsdna        291 ccgtggcagaccaaca-gcaaatgcgacatggaataacca

RSddsdna        716 atcctcggtg----ctggtgggcgactatctcttcgcccg
STddsdna        961 cgccagcgtg----ctggtcggcgacttcctgttcagccg
SPddsdna        473 atcgacggtcaatccttgcgggtaatttcatccttgcacg
GSddsdna        291 ggcgtccgtg----ctggtaggtgacttcctgttcgcccg
RCddsdna        330 gactgcggta----ctggtgggggatttctgattgcccg
```

# Figure 13 (page 3 of 5)

```
RSddsdna    752 cagcttccagctgatggtcgagaccggctcg-----cttc
STddsdna    997 ctcgttcgagctgatggtcgaggccgaaagc-----ctca
SPddsdna    513 g-gcttcga----ctgctatggcccgccttcgaaatcccc
GSddsdna    327 ctcgttccagcttatgacagcagacggctcc-----ctga
RCddsdna    366 ggcatttgatctgctggttgatctggacaat-----atga

RSddsdna    787 gcgtgatggacatcctcgccaatgcctcggccaccatctc
STddsdna   1032 aggcgctgcacatcctgtcgaacgccagcgcggtgatcgc
SPddsdna    548 aagttacggagttgttagctacagtgatagcagacttggt
GSddsdna    362 aggtcatggcgatcctgtcggatgcatcggcgacaattgc
RCddsdna    401 tcctgttaaaggacttctctacaggaacctgtgagattgc

RSddsdna    827 cgagggcgaggtgctgcagctgaccgcgg--cccaggatc
STddsdna   1072 cgagggcgaagtcaaccagctgaccgcggtgcgccggatc
SPddsdna    588 tcgaggtgagtttttgcagctaaaaaata--ctatggat-
GSddsdna    402 tgaaggtgaagtccttcagatggtcgtgc--agaacgacc
RCddsdna    441 tgagggtgaagtattgcagttgc---agg--cacagcatc

RSddsdna    865 tgcgc---acgaccgaggacatccacc-------------
STddsdna   1112 --gac---ctgtccgaggatcgctatc-------------
SPddsdna    625 --cct---tcatctttggaaataaaacaatcaaattttga
GSddsdna    440 ttacg---acgcctgtagaacgctatc-------------
RCddsdna    476 agccagatacaacagaagatatttatt-------------

RSddsdna    889 ---------tgcaggtggtgcgcggcaagacggccgcgct
STddsdna   1134 ---------tcgacatcatcggcgccaagactgcggcgct
SPddsdna    660 ctattatattgaaaaaagtttttg-aaaacagccagttt
GSddsdna    464 ---------ttgaagtcattcacggcaagacggctgcgct
RCddsdna    503 ---------tacagattattcacggtaaaacctcacggtt

RSddsdna    920 ctttgccgcggcaaccgaggtgggcggcgtggtcg-----
STddsdna   1165 gttcgccgccgcctgccgggtggcgggcgtggtcg-----
SPddsdna    699 aatttcca-------aaagctgcaaggcttctacaatcct
GSddsdna    495 gtttgcggctgcctgccgtgtcggcgctgtcgtgg-----
RCddsdna    534 gttcgaactggcgaccgaaggcgctgcaatactgg-----

RSddsdna    955 cgggc---gtgcccgaggcgcaggtcgaggcgctccacgc
STddsdna   1200 ccgag---cgtcccgaggcggaggaactcgcgctcgacgc
SPddsdna    732 cggacaatgttctcctactgtagcaacagctgctgga-ga
GSddsdna    530 ccgag---cgtccggaagcagaagaggaagctctggagcg
RCddsdna    569 caggc---aaacctga-----ataccgtgaacctttacgt
```

# Figure 13 (page 4 of 5)

```
RSddsdna     992 c---tacggggacgcgctggggatcgccttccagatcgtc
STddsdna    1237 c---tatggccgcaacctcggcatcgctttccagctggtc
SPddsdna     771 a---tacggtcgatgcattggtactgcttttcaactaatg
GSddsdna     567 g---tttggcaccaatctgggtatggcgttccagcttgtt
RCddsdna     601 cgttttgccggacactttggcaat-gcttttcagattatt

RSddsdna    1029 gacgacctcctcgattatggcggcgtg-gatgcccagatc
STddsdna    1274 gacgacgcgatcgactatgtctcggac-gcgtcgacgatg
SPddsdna     808 gatgacgtgttggactat-acgtcgaaagatgatacttta
GSddsdna     604 gatgatgccctggattatgccgcagac-cagcaggttttg
RCddsdna     640 gatgatattctggattacacttcagat-gctgatacgctc

RSddsdna    1068 ggcaagaacaccggcgacgacttcc-gcgaacgcaagctg
STddsdna    1313 ggcaaggatgccggcgacgatttcc-gcgaaggcaagatg
SPddsdna     847 ggaaaggcggctggtgcagatttgaagctagggttggcta
GSddsdna     643 ggcaagaccgttggtgatgacatgc-gtgaaggcaagatc
RCddsdna     679 ggcaaaaatattggcgatgacttga-tggaaggcaaaccc

RSddsdna    1107 acgctgccggtcatcaaggcggtggcccaggccgatgcc-
STddsdna    1352 acgctgccggtggtcctggcgtacgcgcgcggcgacgag-
SPddsdna     887 cagct-cccgtcctctttgc-atggaaaaagt--atcca-
GSddsdna     682 accctgccggtcct------ggccgcctatgaggctggct
RCddsdna     718 accctgccgctgattgcagcaatgcaaaatactcaaggt-

RSddsdna    1146 -----gaggagcgcgccttctggcagcgggtgatcgagaa
STddsdna    1391 -----gcggaacgcggcttctggaaggaagcgatttcg--
SPddsdna     922 -----ga--------acttggtgca----atgattgtgaa
GSddsdna     716 cgccggaagatcgtattttctgggagcgcgtcattggaga
RCddsdna     757 -----gaacagcgcgacctgatccgtcgc-------agca

RSddsdna    1181 gggcgaccagcgcgagggtgac--ctcgagcaagcccatg
STddsdna    1424 ----ggccgccgcatctcggac--gaggatttcgccgagg
SPddsdna     945 tagattcaatcatccttctgat--atccaacgggctcgtt
GSddsdna     756 aggggagcagactgaggacgat--ctgcctcatgctctga
RCddsdna     785 ttgccactggcg-gtacttcacagcttgaacaagttattg

RSddsdna    1219 cgatca------tgtcccgccacggcgccatggaggc--c
STddsdna    1458 cgatccggctggtgcagagctgccgcgcggtggacga--c
SPddsdna     983 ctttgg------ttgagtgcactgatgctatcgagca--a
GSddsdna     794 acctga------ttgcaaagacgggtgcgatcaatacgac
RCddsdna     824 cgattg------tacaaaattcgggagcgctgga------
```

# Figure 13 (page 5 of 5)

```
RSddsdna    1251 gcccggcaggatgcgctccgctgggtcacggtggcgcgcg
STddsdna    1496 acgctcgcccgtgcccgccattacggccagctcgcgatcg
SPddsdna    1015 accatcacttgggcaaaagaatatatcaaaaaagccaaag
GSddsdna     828 gatcgcccg--cgcgcaggtctatgccgacgcagctgttg
RCddsdna     852 ttattgccataagcgtgctactgaagaaaccgagcgagca

RSddsdna    1291 ----aggcactcggccagctgccggagcacccgctgcgcg
STddsdna    1536 ----atgcgct-gggcggcttcc-gcgcctgcgaggcgaa
SPddsdna    1055 ----attcccttctgtgtctccctgattcacctgcaagga
GSddsdna     866 ----aagccctgtccattttcccggatagcgaactgcgcc
RCddsdna     892 ttacaggcactagaaatattacctgagagtacttaccggc

RSddsdna    1327 agatgc--tgcacgatctggccgatttcgtggtcgaacgc
STddsdna    1570 ggacgcgatggtcgaggcggtcgaattcgcggtggcgcgc
SPddsdna    1091 aggcac--tttttgcgttggctgataaagtaataacgaga
GSddsdna     902 gccttc--tgatcgaaacggttcagttcacggtgaatcgg
RCddsdna     932 aggcgc--tggttaacttgacccgcttagctttagaccga

RSddsdna    1365 atcgcctga
STddsdna    1610 gcctactga
SPddsdna    1129 aagaagtga
GSddsdna     940 gcccgctaa
RCddsdna     970 atccaataa
```

# Figure 14 (page 1 of 2)

```
RSddsp      372 -------------mgldevsq-----kphe
STddsp      605 msatv--------hrlgsrtq-----psld
SPddsp        1 miqyvylkhmrklwslgkvrstvlrfsttn
GSddsp        1 ------------------------------
RCddsp        1 -------------maidf----------kq

RSddsp      408 rlaawlae-dmaavnglirermaskhapri
STddsp      656 pimalvaq-dmnlvnavildrmqse-ipli
SPddsp       31 rnashlikneleqispgirq-mlnsnsefl
GSddsp        1 ----------mlacnraiiarmesp-vpli
RCddsp        8 dilapvaq-dfaamdqfinegisskva-lv

RSddsp      495 pevtahlveaggkrlrplltla---aarlc
STddsp      740 pelaghliagggkrmrpmltla---sarll
SPddsp       60 eecskyytiaqgkqmrpslvllmskatslc
GSddsp       20 pqlgahlvaaggkrlrplltla---sarlc
RCddsp       36 msvskhvveaggkrmrpimcll---aayac

RSddsp      576 -----------------------gye-gp-
STddsp      821 -----------------------gys-gt-
SPddsp       90 hgidrsvvgdkyiddddlrsfstgqi-lp-
GSddsp       47 -----------------------gyqpgpd
RCddsp       63 -----------------------get-nl-

RSddsp      591 --yhih-laatvefihtatllhddvvdesh
STddsp      836 --rhhk-laaavefihtatllhddvvdssd
SPddsp      118 --sqlr-laqitemihiasllhddvidhan
GSddsp       54 hqrhvg-laacvefihtatllhddvvdest
RCddsp       68 --khaqklaaiiemlhtatlvhdddvdesg

RSddsp      672 rrrgkptanllwdnkssvlvgdylfarsfq
STddsp      917 lrrgrrtaniiwgnpasvlvgdflfsrsfe
SPddsp      145 vrrgspssnvafgnrrsilagnfilarast
GSddsp       83 lrrglasanavfgnkasvlvgdflfarsfq
RCddsp       96 lrrgrptanatwnnqtavlvgdfliarafd

RSddsp      762 lmvetgslrvmdilanasatisegevlqlt
STddsp     1007 lmveaeslkalhilsnasaviaegevnqlt
SPddsp      175 amarlrnpqvtellatviadlvrgeflqlk
GSddsp      113 lmtadgslkvmailsdasatiaegevlqmv
RCddsp      126 llvdldnmillkdfstgtceiaegevlqlq
```

# Figure 14 (page 2 of 2)

```
RSddsp     852 aaqdlrtte------dihlqvvrgktaalf
STddsp    1097 avrridlse------dryldiigaktaalf
SPddsp     205 ntmdpssleikqsnfdyyieksflktasli
GSddsp     143 vqndlttpv------erylevihgktaalf
RCddsp     156 aqhqpdtte------diylqiihgktsrlf

RSddsp     924 aaatevggvvagvpeaqvealhaygdalgi
STddsp    1169 aaacrvagvvaerpeaeelaldaygrnlgi
SPddsp     235 sksckastilgqcsptvataageygrcigt
GSddsp     167 aaacrvgavvaerpeaeeealerfgtnlgm
RCddsp     180 elategaailagkpeyr-eplrrfaghfgn

RSddsp    1014 afqivddlldyggvdaqigkntgddfrerk
STddsp    1259 afqlvddaidyvsdastmgkdagddfregk
SPddsp     265 afqlmddvldytskddtlgkaagadlklgl
GSddsp     197 afqlvddaldyaadqqvlgktvgddmregk
RCddsp     209 afqiiddildytsdadtlgknigddlmegk

RSddsp    1104 ltlpvikavaqadaeerafwqrviekgdq-
STddsp    1349 mtlpvvlayargdeaergfwkeaisgrri-
SPddsp     295 atapvlfa-----------wkkypelgami
GSddsp     227 itlpvlaayeagspedrifwervigegeq-
RCddsp     239 ptlpliaamqntqgeqrdlirrsiatggt-

RSddsp    1191 -----regdleqahaimsrhgameaarqda
STddsp    1436 -----sdedfaeairlvqscravddtlara
SPddsp     314 vnrfnhpsdiqrarslvectdaieqtitwa
GSddsp     256 -----teddlphalnliaktgainttiara
RCddsp     268 -----sq--leqviaivqnsgaldychkra

RSddsp    1266 lrwvtvarealgqlpehplremlhdladfv
STddsp    1511 rhygqlaidalggfraceakdamveavefa
SPddsp     344 keyikkakdsllclpdsparkalfaladkv
GSddsp     281 qvyadaavealsifpdselrrllietvqft
RCddsp     291 teeteralqaleilpestyrqalvnltrla

RSddsp    1356 veria*
STddsp    1601 varay*
SPddsp     374 itrkk-
GSddsp     311 vnrar-
RCddsp     321 ldriq-
```

# Figure 15 (page 1 of 2)

```
Hidxsp    1 mtnnmnnypllslinspedlrllnkdqlpqlcqelrayllesvsqtsghl
Ecdxsp    1 msfdiakyptlalvdstqelrllpkeslpklcdelrrylldsvsrssghf
Hpdxsp    1 ----------milqnktfdlnpndiaglelvcqtlrnrilevvsangghl

Hidxsp   51 asglgtveltvalhyvyktpfdqliwdvghqayphkiltgrreqmstirq
Ecdxsp   51 asglgtveltvalhyvyntpfdqliwdvghqayphkiltgrrdkigtirq
Hpdxsp   41 ssslgavelivgmhalfdcqknpfifdtshqayahklltgrfesfstlrq

Hidxsp  101 kdgihpfpwreesefdvlsvghsstsisaglgiavaaerenagrktvcvi
Ecdxsp  101 kgglhpfpwrgeseydvlsvghsstsisagigiavaaekegknrrtvcvi
Hpdxsp   91 fqglsgftkpsesaydyfiaghsstsvsigvgvakafrlkqtlgmpiall

Hidxsp  151 gdgaitagmafealnhagalhtdmlvilndnemsisenvgalnnhlarif
Ecdxsp  151 gdgaitagmafeamnhagdirpdmlvilndnemsisenvgalnnhlaqll
Hpdxsp  141 gdgsisagifyealnelgdrkypmimilndnemsistpigalskalsqlm

Hidxsp  201 sgslystlrdgskkildkvppiknfm-kkteehmkgvmfspestlfeelg
Ecdxsp  201 sgklysslreggkkvfsgvppikell-krteehikgmvv--pgtlfeelg
Hpdxsp  191 kgpfyqsfrskvkkilstlpesvnylasrfeesfk--litp-gvffeelg

Hidxsp  250 fnyigpvdghnidelvatltnmrnlkgpqflhiktkkgkgyapaekdpig
Ecdxsp  248 fnyigpvdghdvlglittlknmrdlkgpqflhimtkkgrgyepaekdpit
Hpdxsp  238 inyigpinghdlgtiietlklakelkepvlihaqtlkgkgykiaegryek

Hidxsp  300 fhgvpkfdpisgelpknnsk-ptyskifgdwlcemaekdakiigitpamr
Ecdxsp  298 fhavpkfdpssgclpkssgglpsyskifgdwlcetaakdnklmaitpamr
Hpdxsp  288 whgvgpfdldtglskksksatlspteaysntllelakkdekivgvtaamp

Hidxsp  349 egsgmvefsqrfpkqyfdvaiaeqhavtfatglaiggykpvvaiystflq
Ecdxsp  348 egsgmvefsrkfpdryfdvaiaeqhavtfaaglaiggykpivaiystflq
Hpdxsp  338 sgtgldklidayplrffdvaiaeqhaltsssamakegfkpfvsiystflq

Hidxsp  399 raydqlihdvaiqnlpvlfaidragivgadgathqgafdisfmrcipnmi
Ecdxsp  398 raydqvlhdvaiqklpvlfaidragivgadgqthqgafdlsylrcipemv
Hpdxsp  388 raydsivhdacisslpiklaidragivgedgethqglldvsylrsipnmv

Hidxsp  449 imtpsdenecrqmlytgyqcgk-paavryprgn-avgvkltplemlpigk
Ecdxsp  448 imtpsdenecrqmlytgyhyndgpsavryprgn-avgveltpleklpigk
Hpdxsp  438 ifaprdnetlknavyfanehdsspcafryprgsfalkegvfepsgfvlgr
```

# Figure 15 (page 2 of 2)

```
Hidxsp 497 srlirkgqkiailnfgtllpsa--lelsek---lnatvvdmrfvkpidie
Ecdxsp 497 givkrrgeklailnfgtlmpea--akvaes---lnatlvdmrfvkpldea
Hpdxsp 488 sellkkegeilligygngvgrahlvqlalkekniecalldlrflkpldhn

Hidxsp 542 minvlaqthdylvtleenaiqggagsavaevlnssgkstallqlglpdyf
Ecdxsp 542 lilemaashealvtveenaimggagsgvnevlmahrkpvpvlniglpdff
Hpdxsp 538 l-saiiapyqklyvfsdnyklggvasaileflseqnilkpvksfeitdef

Hidxsp 592 ipqatqqealadlgldtkgieekilnfiakqgnl
Ecdxsp 592 ipqgtqeemraelgldaagmeakikawla-----
Hpdxsp 587 imhgntalvekslgldtesltdailkdlgqer--
```

# Figure 16

```
Rpodsp   1 --mniivkiqqnlkdevtqlndliisclksdaeliekvgkylveaggkri
Ecoppp   1 mnlekinel---taqdmagvnaaileqlnsdvqlinqlgyyivsgggkri
Gsddsp   1 ---------------mlacnraiiarmespvplipqlgahlvaaggkrl
Rcsdsp   1 maidfkqdilapvaqdfaamdqfinegisskvalvmsvskhvveaggkrm

Rpodsp  49 rplltiitakmfdykgn----nhiklasavefihaatllhddvvdnstlr
Ecoppp  48 rpmiavlaaravgyegna----hvtiaaliefihtatllhddvvdesdmr
Gsddsp  35 rplltlasarlcgyqpgpdhqrhvglaacvefihtatllhddvvdestlr
Rcsdsp  51 rpimcllaayacg-etnlkhaqk--laaiiemlhtatlvhddvvdesglr

Rpodsp  95 rfkptanviwgsktsilvgdflfsqsfklmvasgcikamnvlakasviis
Ecoppp  94 rgkatanaafgnaasvlvgdfiytrafqmmtslgslkvlevmseavnvia
Gsddsp  85 rglasanavfgnkasvlvgdflfarsfqlmtadgslkvmailsdasatia
Rcsdsp  98 rgrptanatwnnqtavlvgdfliarafdllvdldnmillkdfstgtceia

Rpodsp 145 egevvqlvklnerriitideyqqivksktaelfgaacevgaiiaeqvdrv
Ecoppp 144 egevlqlmnvndpdi-teenymrviysktarlfeaaaqcsgilagctpee
Gsddsp 135 egevlqmvvqndltt-pverylevihgktaalfaaacrvgavvaerpeae
Rcsdsp 148 egevlqlqaqhqpdt-tediylqiihgktsrlfelategaailagkpe-y

Rpodsp 195 skdvqnfgrllgtifqviddlldylgsdkqvgknigddflegkvtlplif
Ecoppp 193 ekglqdygrylgtafqliddlldynadgeqlgknvgddlnegkptlpllh
Gsddsp 184 eealerfgtnlgmafqlvddaldyaadqqvlgktvgddmregkitlpvla
Rcsdsp 196 replrrfaghfgnafqiiddildytsdadtlgknigddlmegkptlplia

Rpodsp 245 lyhkleqdkqlwlenmlksdk--rtkddfvkirdlmlkhaiynetvnyls
Ecoppp 243 amhhgtpeqaqmirtaieqgngrhllepvleamnac---gslewtrqrae
Gsddsp 234 ayeagspedrifwervi--gegeqteddlphalnliaktgainttiaraq
Rcsdsp 246 amqntqgeqrdlirrsiatggtsqle----qviaivqnsgaldychkrat

Rpodsp 293 sleneannllnkipvqniykyylfsiirfilyrsy
Ecoppp 290 eeadkaiaalqvlpdtpw-realiglahiavqrdr
Gsddsp 282 vyadaavealsifpdsel-rrllietvqftvnrar
Rcsdsp 292 eeteralqaleilpesty-rqalvnltrlaldriq
```

# Figure 17

```
Rpodsp    1 --------mniivkiqqnlkdevtqlndliisclksdaeliekvgkylve
Ecodsp    1 ------mnlekineltaq---dmagvnaaileqlnsdvqlinqlgyyivs
Hiodsp    1 mkkqdlmsideiqkladp---dmqkvnqnilaqlnsdvpligqlgfyivq
Gsddsp    1 ---------------------mlacnraiiarmespvplipqlgahlva
Rcsdsp    1 ------maidfkqdilapvaqdfaamdqfinegisskvalvmsvskhvve

Rpodsp   43 aggkrirplltiitakmfdykgn----nhik-lasavefihaatllhddv
Ecoppp   42 gggkrirpmiavlaaravgyegna----hvt-iaaliefihtatllhddv
Hiods   142 gggkrirpliavlaarslgfegsn----sit-catfvefihtasllhddv
Gsddsp   29 aggkrlrplltlasarlcgyqpgpdhqrhvg-laacvefihtatllhddv
Rcsdsp   45 aggkrmrpimcllaayac----getnlkhaqklaaiiemlhtatlvhddv

Rpodsp   88 vdnstlrrfkptanviwgsktsilvgdflfsqsfklmvasgcikamnvla
Ecoppp   87 vdesdmrrgkatanaafgnaasvlvgdfiytrafqmmtslgslkvlevms
Hiods   277 vdesdmrrgratanaefgnaasvlvgdfiytrafqlvaqleslkilsima
Gsddsp   78 vdestlrrglasanavfgnkasvlvgdflfarsfqlmtadgslkvmails
Rcsdsp   91 vdesglrrgrptanatwnnqtavlvgdfliarafdllvdldnmillkdfs

Rpodsp  138 kasviisegevvqlvklnerriitideyqqivksktaelfgaacevgaii
Ecoppp  137 eavnviaegevlqlmnvndpdi-teenymrviysktarlfeaaaqcsgil
Hiods   427 datnvlaegevqqlmnvndpet-seanymrviysktarlfevagqaaaiv
Gsddsp  128 dasatiaegevlqmvvqndltt-pverylevihgktaalfaaacrvgavv
Rcsdsp  141 tgtceiaegevlqlqaqhqpdt-tediylqiihgktsrlfelategaail

Rpodsp  188 aeqvdrvskdvqnfgrllgtifqviddlldylgsdkqvgknigddflegk
Ecoppp  186 agctpeeekglqdygrylgtafqliddlldynadgeqlgknvgddlnegk
Hiods   574 aggteaqekalqdygrylgtafqlvddvldysantqalgknvgddlaegk
Gsddsp  177 aerpeaeeealerfgtnlgmafqlvddaldyaadqqvlgktvgddmregk
Rcsdsp  190 agkpeyre-plrrfaghfgnafqiiddildytsdadtlgknigddlmegk

Rpodsp  238 vtlpliflyhkleqdkqlwlenmlksd--krtkddfvkirdlmlkhaiyn
Ecoppp  236 ptlpllhamhhgtpeqaqmirtaieqgngrhllepvleamnac---gsle
Hiods   724 ptlpllhamrhgnaqqaalireaieqggkreaidevlaimteh---ksld
Gsddsp  227 itlpvlaayeagspedrifwervi--gegeqteddlphalnliaktgain
Rcsdsp  239 ptlpliaamqntqgeqrdlirrsiatggtsqleqviaivqns----gald

Rpodsp  286 etvnylssleneannllnkipv--qniykyylfsiirfilyrsy-
Ecoppp  283 wt---rqraeeeadkaiaalqvlpdtpwrealiglahiavqrdr-
Hiods   865 ya---mnrakeeaqkavdaieilpeseykqalislaylsvdrny*
Gsddsp  275 tt---iaraqvyadaavealsifpdselrrllietvqftvnrar-
Rcsdsp  285 yc---hkrateeteralqaleilpestyrqalvnltrlaldriq-
```

FIG. 18

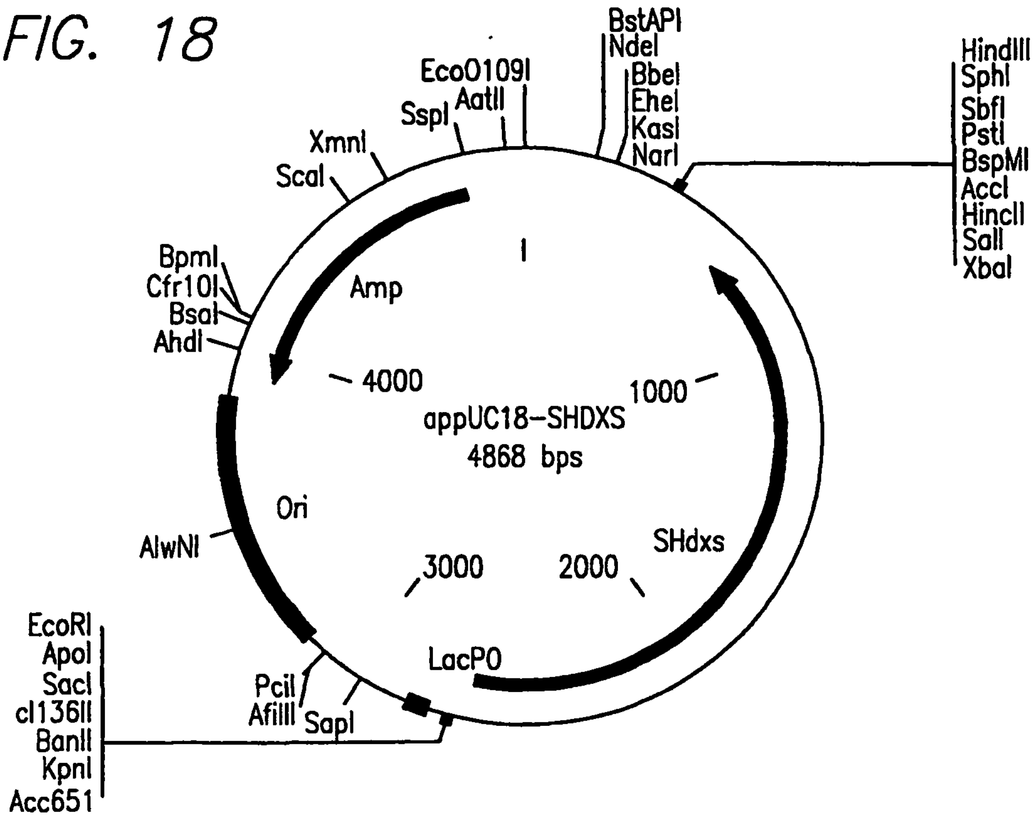
BstAPI
NdeI
BbeI
EheI
KasI
NarI
Eco0109I
AatII
SspI
XmnI
ScaI
HindIII
SphI
SbfI
PstI
BspMI
AccI
HincII
SalI
XbaI
BpmI
Cfr10I
BsaI
AhdI
Amp
4000
1000
appUC18-SHDXS
4868 bps
Ori
AlwNI
3000
2000
SHdxs
LacPO
EcoRI
ApoI
SacI
cl136II
BanII
KpnI
Acc651
PciI
AflIII
SapI

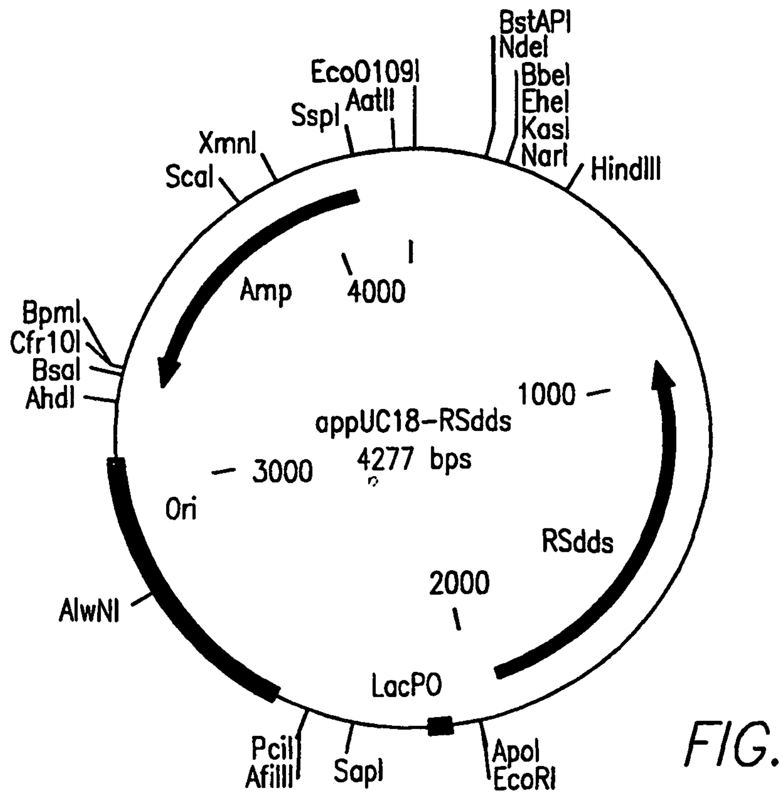
BstAPI
NdeI
BbeI
EheI
KasI
NarI
HindIII
Eco0109I
AatII
SspI
XmnI
ScaI
Amp
4000
BpmI
Cfr10I
BsaI
AhdI
1000
appUC18-RSdds
4277 bps
3000
Ori
RSdds
2000
AlwNI
LacPO
PciI
AflIII
SapI
ApoI
EcoRI

FIG. 19

FIG. 20

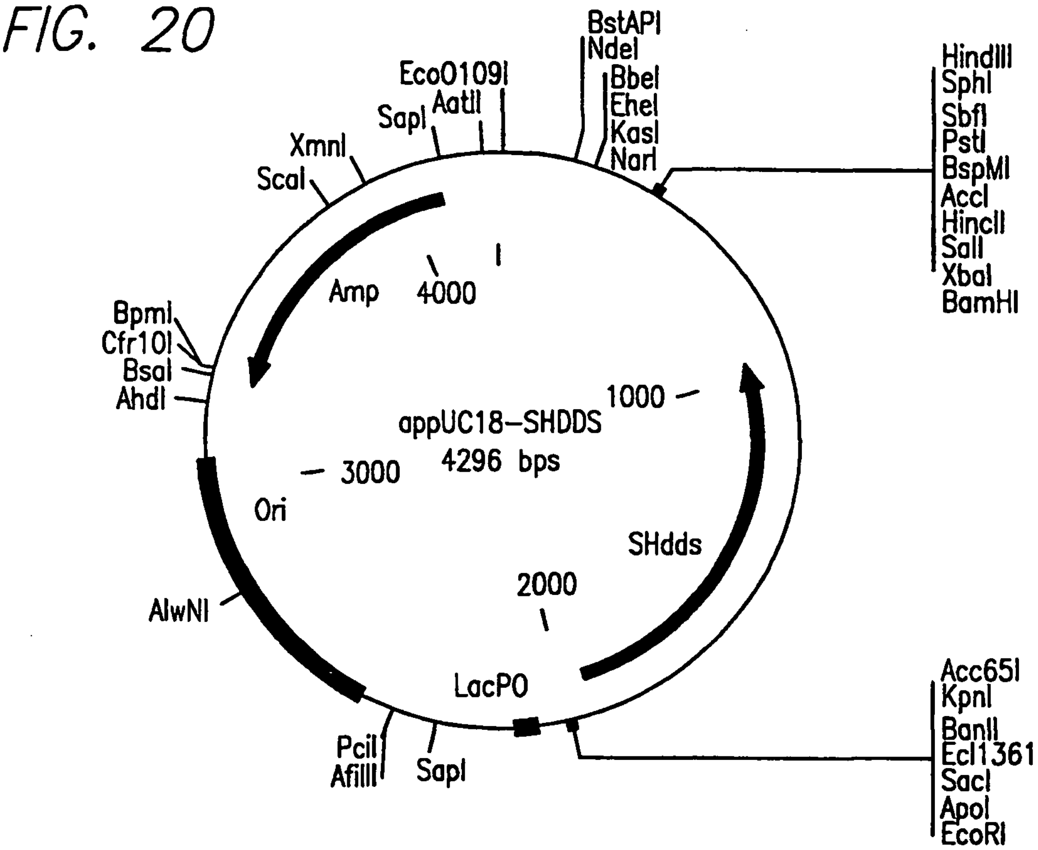
BstAPI
NdeI
BbeI
EheI
KasI
NarI
Eco0109I
AatII
SapI
XmnI
ScaI
HindIII
SphI
SbfI
PstI
BspMI
AccI
HincII
SalI
XbaI
BamHI
Amp
4000
BpmI
Cfr10I
BsaI
AhdI
1000
appUC18-SHDDS
4296 bps
3000
Ori
SHdds
2000
AlwNI
LacPO
PciI
AflIII
SapI
Acc65I
KpnI
BanII
Ecl136I
SacI
ApoI
EcoRI

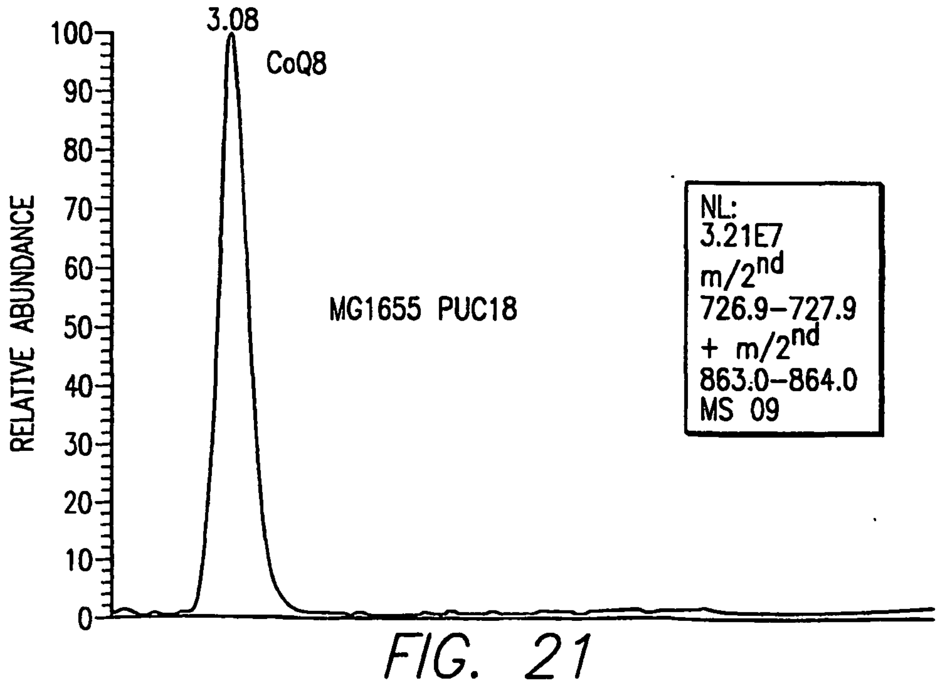
3.08
CoQ8
RELATIVE ABUNDANCE
100
90
80
70
60
50
40
30
20
10
0
MG1655 PUC18
NL:
3.21E7
m/2nd
726.9-727.9
+ m/2nd
863.0-864.0
MS 09

FIG. 21

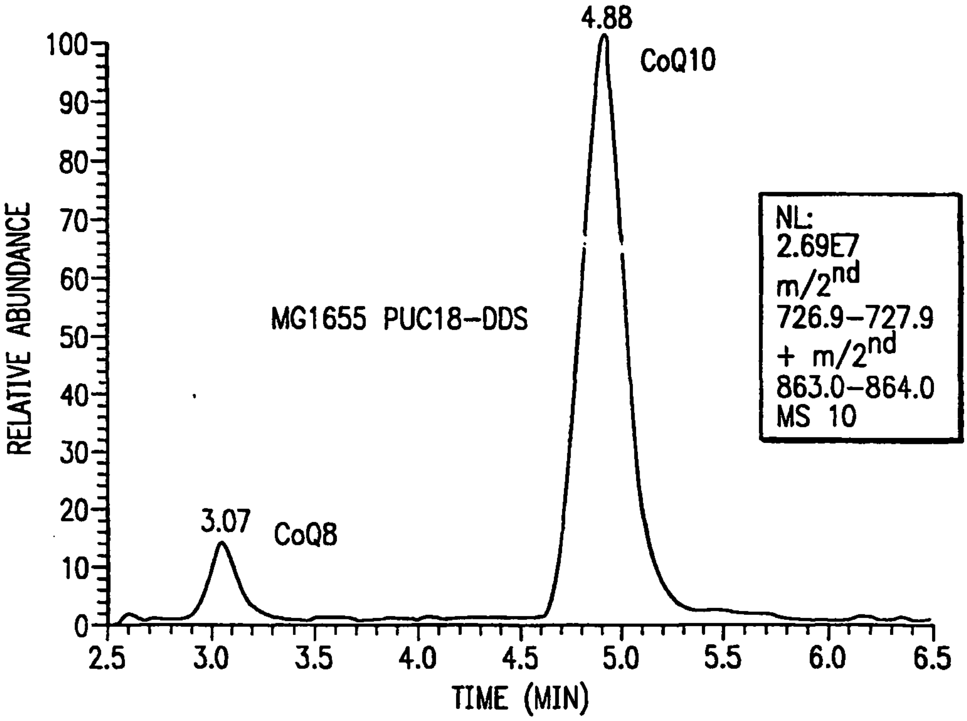
4.88
CoQ10
NL:
2.69E7
m/2nd
726.9-727.9
+ m/2nd
863.0-864.0
MS 10
MG1655 PUC18-DDS
3.07 CoQ8
RELATIVE ABUNDANCE
100
90
80
70
60
50
40
30
20
10
0
2.5
3.0
3.5
4.0
4.5
5.0
5.5
6.0
6.5
TIME (MIN)

FIG. 22

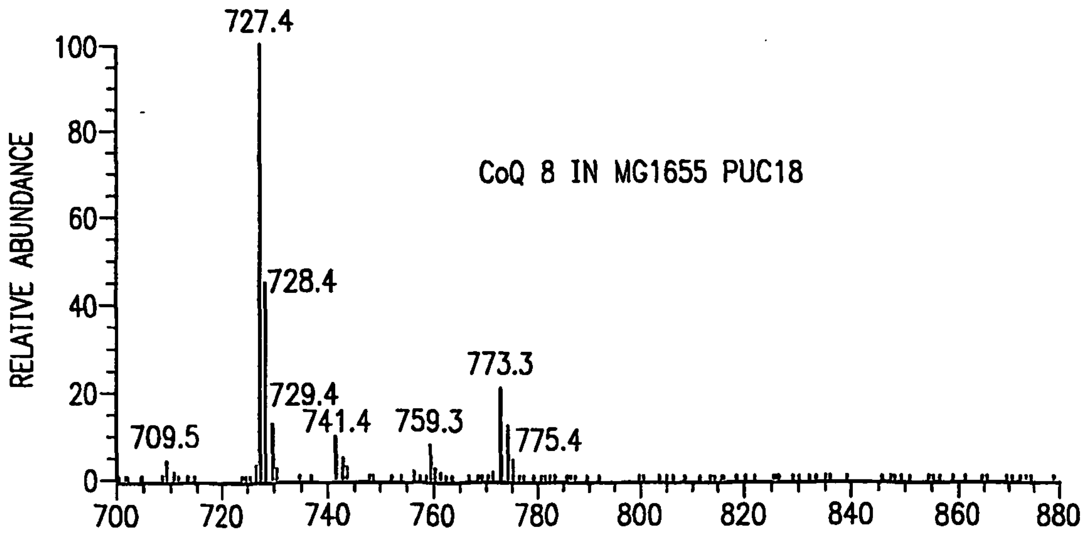
727.4
CoQ 8 IN MG1655 PUC18
728.4
773.3
729.4
709.5
741.4
759.3
775.4
RELATIVE ABUNDANCE
100
80
60
40
20
0
700
720
740
760
780
800
820
840
860
880
m/z

FIG. 23

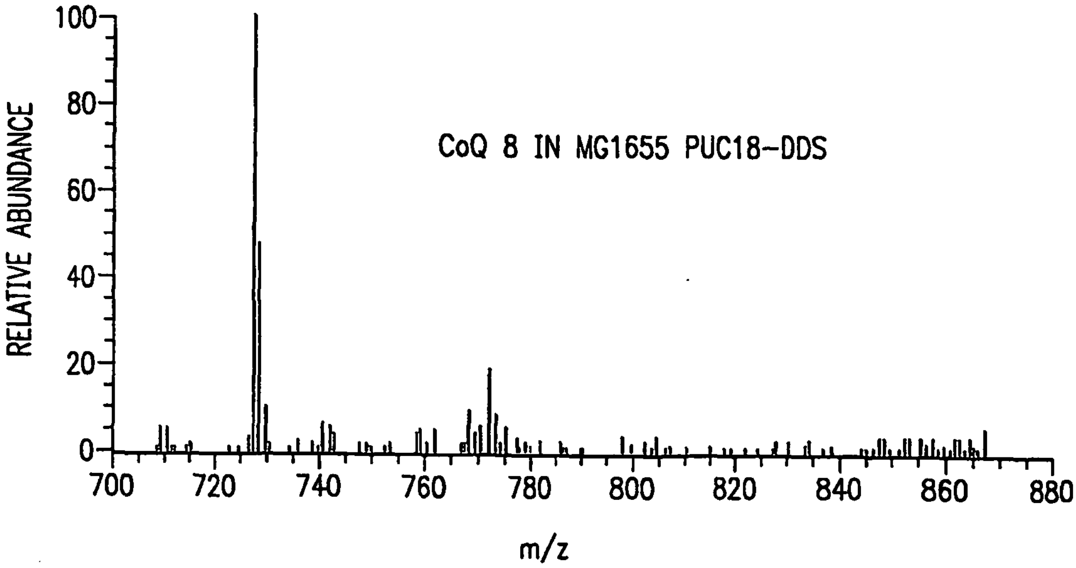
RELATIVE ABUNDANCE
100
80
60
40
20
0
CoQ 8 IN MG1655 PUC18-DDS
700
720
740
760
780
800
820
840
860
880
m/z

FIG. 24

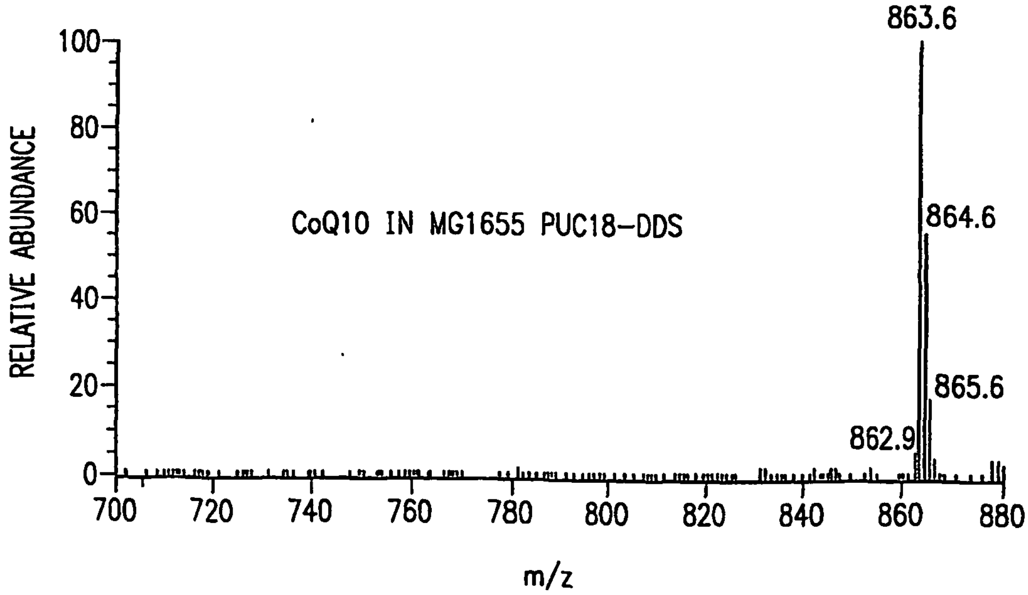
RELATIVE ABUNDANCE
100
80
60
40
20
0
CoQ10 IN MG1655 PUC18-DDS
863.6
864.6
865.6
862.9
700
720
740
760
780
800
820
840
860
880
m/z

FIG. 25

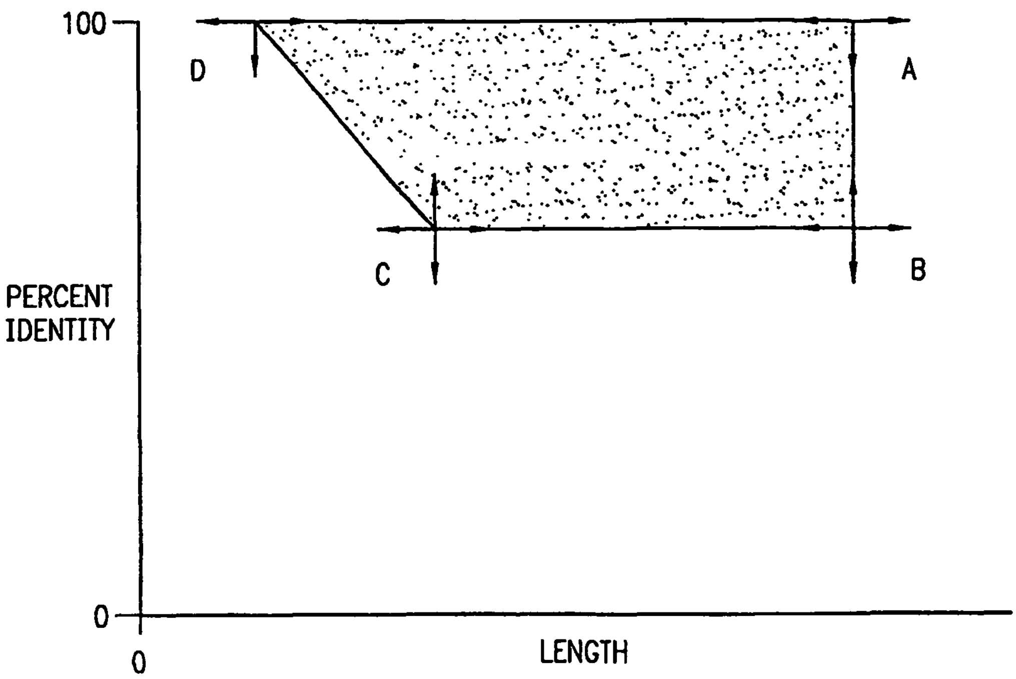


FIG. 26

# Figure 27 (page 1 of 2)

```
Bsdxrp   1 ----------------------------------mknicllgatgsigeq
Hmdxrp   1 --------------------------------mqkqnivilgstgsigks
Ecdxrp   1 ----------------------------------mkqltilgstgsigcs
Zmdxrp   1 -------------------------------msqprtvtvlgatgsighs
Sldxrp   1 ----------------------------------mkavtllgstgsigtq
Ssdxrp   1 ---------------------------------mvkrisilgstgsigtq
Mtdxrp   1 matggrvvirrrgdnevvahndevtnstdgradgrlrvvvlgstgsigtq

Bsdxrp  17 tldvlrahqdqfqlvsmsfg-rnidkavpmievfqpkfvsvgdldtyhkl
Hmdxrp  19 tlsviennpqkyhafalvgg-knveamfeqcikfrphfaalddvnaakil
Ecdxrp  17 tldvvrhnpehfrvvalvag-knvtrmveqclefspryavmddeasakll
Zmdxrp  20 tldliernldryqvialtan-rnvkdladaakrtnakraviadpslyndl
Sldxrp  17 tldileqypdrfrlvglaag-rnvallseqirrhrpeivaiqdaaqlsel
Ssdxrp  18 tldivthhpdafqvvglaag-gnvallaqqvaefrpeivairqaekledl
Mtdxrp  51 alqviadnpdrfevvglaaggahldtllrqraqtgvtniavadehaaq--

Bsdxrp  66 kqmsfsfec---qiglgeeglieaavmeevdivvnallgsvgliptlkai
Hmdxrp  68 rekli-ahhiptevlagrraicelaahpdadqimasivgaagllptlsav
Ecdxrp  66 ktmlq-qqgsrtevlsgqqaacdmaaledvdqvmaaivgaagllptlaai
Zmdxrp  69 keala---gssveaaagadalve-aammgadwtmaaiigcaglkatlaai
Sldxrp  66 qaaiadl-dnpplilgeagvtevarygdaeivvtgivgcagllptiaai
Ssdxrp  67 kaavaeltdyqpmyvvgeegvvevarygdaesvvtgivgcagllptmaai
Mtdxrp  99 -----rvgdip---yhgsdaatrlveqteadvvlnalvgalglrptlaal

Bsdxrp 113 eqkktialanketlvtaghivkehakkydvpllpvdsehsaifqalqg--
Hmdxrp 117 kagkrvllankeslvtcgqlfidavknygskllpvdsehnaifq---s-l
Ecdxrp 115 ragktillankeslvtcgrlfmdavkqskaqllpvdsehnaifq---s-l
Zmdxrp 115 rkgktvalankeslvsagglmidavrehgttllpvdsehnaifq---c-f
Sldxrp 115 eagkdialanketliaagpvvlpllqkhgvtitpadsehsaifqciqg-l
Ssdxrp 117 aagkdialanketliagapvvlplvekmgvkllpadsehsaifqclqg-v
Mtdxrp 141 ktgarlalankeslvaggslvlraarpg--qivpvdsehsalaqclrggt

Bsdxrp 161 -eqak------------nierliitasggsfrdktreelesvtvedalkh
Hmdxrp 163 ppeaqekigfcplsel-gvskiiltgsggpfrytpleqftnitpeqavah
Ecdxrp 161 pqpiqhnlgyadleqn-gvvsilltgsggpfretplrdlatmtpdqacrh
Zmdxrp 161 phhnrdy-----------vrriiitasggpfrttslaematvtperavqh
Sldxrp 164 sthad----frpaqvvaglrrilltasggafrdwpverlsqvtvadalkh
Ssdxrp 166 pe--------------gglrriiltasggafrdlpverlpfvtvqdalkh
Mtdxrp 189 pde---------------vaklvltasggpfrgwsaadlehvtpeqagah
```

# Figure 27 (page 2 of 2)

```
Bsdxrp 198 pnwsmgakitidsatmmnkglevieahwlfdipyeqidvvlhkesiihsm
Hmdxrp 212 pnwsmgkkisvdsatmmnkgleyiearwlfnasaeemeviihpqsiihsm
Ecdxrp 210 pnwsmgrkisvdsatmmnkgleyiearwlfnasasqmevlihpqsvihsm
Zmdxrp 200 pnwsmgakisidsatmmnkglelieayhlfqiplekfeilvhpqsvihsm
Sldxrp 210 pnwsmgrkitvdsatlmnkglevieahylfgldydyidivihpqsiihsl
Ssdxrp 202 pnwsmgqkitidsatlmnkglevieahylfgldydhidivihpqsiihsl
Mtdxrp 224 ptwsmgpmntlnsaslvnkglevievethllfgipydridvvvhpqsiihsm

Bsdxrp 248 vefhdksviaqlgtpdmrvpiqyaltypdrlplpdakrlelweigslhfe
Hmdxrp 262 vryvdgsvitqmgnpdmrtpiaetmayphrtfa-gvepldffkikeltfi
Ecdxrp 260 vryqdgsvlaqlgepdmrtpiahtmawpnrvns-gvkpldfcklsaltfa
Zmdxrp 250 veyldgsilaqigspdmrtpightlawpkrmet-paesldftklrqmdfe
Sldxrp 260 ieledtsvlaqlgwpdmrlpllyalswpdrlst-qwsaldlvkagslefr
Ssdxrp 252 ievqdtsvlaqlgwpdmrlpllyalswperiyt-dwepldlvkagslsfr
Mtdxrp 274 vtfidgstiaqasppdmklpislalgwprrv-sgaaaacdfhtasswefe

Bsdxrp 298 kadfdrfrclqfafesgkiggtmptvlnaanevavaaflagkipflaied
Hmdxrp 311 epdfnrypnlklaidafaagqyattamnaaneiavqafldrqigfmdiak
Ecdxrp 309 apdydrypclklameafeqggaattalnaaneitvaaflaqqirftdiaa
Zmdxrp 299 apdyerfpaltlamesiksggarpavmnaaneiavaafldkkigfldiak
Sldxrp 309 epdhakypcmdlayaagrkggtmpavlnaaneqavalfleeqihfsdipr
Ssdxrp 301 epdhdkypcmqlaygagraggampavlnaaneqavalflqekisfldipr
Mtdxrp 323 pldtdvfpavelarqagvaggcmtavynaaneeaaaaflagrigfpaivg

Bsdxrp 348 cieka--ltrhqllkkpswr---tfkkwtk---------ipgdtsiqysh
Hmdxrp 361 inskt--ierispytiqniddvleidaqare--------ia-ktllre--
Ecdxrp 359 lnlsv--lekmdmrepqcvddvlsvdanare--------varkevmrlas
Zmdxrp 349 ivekt--ldhytpatpssledvfaidnear---------iqaaalmeslp
Sldxrp 359 lieracdrhqtewqqqpslddilaydawarqfv------qasyqslesvv
Ssdxrp 351 liektcdlyvgqntaspdletilaadqwarrtv------lensacvatrp
Mtdxrp 373 iiadvlhaadqwavepatvddvldaqrwareraqravsgmasvaiastak

Bsdxrp 384 kvvcs---------
Hmdxrp 398 --------------
Ecdxrp 399 --------------
Zmdxrp 388 a-------------
Sldxrp 403 --------------
Ssdxrp 395 --------------
Mtdxrp 423 pgaagrhastlers
```

# Figure 28

ggcccgggctggtggggtttctggcgctggggctggtgttcggcgcgttcttcttcgtcg
cgatcgtgacgcggaacgccaagctggcggcggggcaggtctatgtcgggctgccggtgc
tcgcgctgctgctgctccgcgaccatccgcagggctttgccgcgacgctgtggacgatgg
cgatcgtctgggtgtgcgacagcggcgcctatttgccggtcgcgcgatcggtgggccca
agctcgcgccctcgatcagcccgaacaagacctgggcggggctgatcggcgggttggttg
ccgcgatcctgttctccgccggctatgtcgcgctggcgccggggagcgcgatcggctggt
ggctggtcgcggtgtcgccgctggtagccttcgcctcgcagatcggcgacctgtacgaga
gccatctcaagcgggtcgcgggcgtgaaggattcgagcaacctgctgcccggccatggcg
gcattctcgaccggctcgacggccttgtcttcgcagccccggttgcagctttgtttttg
cgatccatcatcaggtggtcgtgggaggatactggtggtgaagcgcgtcacggtgttggg
ggcgaccggctcggtcggcacctcgacgctggatctgatcgaacgaaatccgcacgcctt
cgaagtcgtggcgctgaccgcaaattgcgatgtcgagaagctggctgccgcggcgatccg
cacgcgcgcgcgctgcgccgtggtcgccgacgagaaatgcctgccggcgctacaggagcg
gctggccggcagcggtgtcgaggcgatgggcggggcgcattcggtgtgcgacgtggcgcg
gatgggtgctgactggacgatggctgcgatcgtcggcagcgcagggctcaagccggtgat
ggccgcgctggaggccggtggcaccgtcgcgctcgcgaacaaggagtcgctcgtctcggc
gggtgaggtgatgatggcggcggcccgcgcgcatggcgcgacgctgctgccggtcgattc
ggagcacaatgcggtgttccagtgcctcgatcgcaccgcgcccaggggcgtccgccggat
catccttaccgccagcggtggtccgttccgcgcgacgccgaaggaagcgatgcgcgacat
cacccccgcacaggcggtggcgcatcccaactggtcgatgggcgccaagatctcggtcga
ctccgcgacgatgatgaacaaggggctcgaactgatcgaagccttccacctgttcccggt
cgccgccgagcaactggccgtgctggtccatcgccaatccgtcgtccattcgatggtgga
atatgtcgacggatcggtgctggcccagctcggcacgcccgacatgcgcacgccgatcgc
ctatgcgctggcttggcccgagcggatggagacgctgtgcccgccgctcgaccttgccac
ggtgggtaagctcgagttcgaaaatcccgatctcgatcgcttcccggcgctcgcgctggc
gatggaggcattgaaggcgggcggggcgcgtccggccattctcaatgccgccaacgaagt
cgccgtcgcggcctttctcgccgggcggatcggattccttgaaattgccgcaatctctgc
cgatacgctgtctcgctatgacccggccgcgccggaaacgctcgatgccgtgctggcgat
cgacgcggaggcgcggctttacgcggctgagcgagtgaaggactgcgtcgcttgatccaa
tcccccggcatcctgctcaccattctggcgttcgcgctggtgatcggccgctcgtgttc
ctgcacgagctgggacattatctggcggccgcctcttcggggtgaaggccgaggaattc
tcgatcggcttcggccgcgagatcgccggcaccaccgatcgccgcggcacgcgctggaag
ttcagcctgttgccgctgggcggctatgtccgcttcgccggcgacatgaacccggcgagc
cagccttcgcccgaatggctgcagaccagcccgggcc (SEQ ID NO:95)

# Figure 29

gtggtgaagcgcgtcacggtgttgggggcgaccggctcggtcggcacctcgacgctggat
ctgatcgaacgaaatccgcacgccttcgaagtcgtggcgctgaccgcaaattgcgatgtc
gagaagctggctgccgcggcgatccgcacgcgcgcgcgctgcgccgtggtcgccgacgag
aaatgcctgccggcgctacaggagcggctggccggcagcggtgtcgaggcgatgggcggg
gcgcattcggtgtgcgacgtggcgcggatgggtgctgactggacgatggctgcgatcgtc
ggcagcgcagggctcaagccggtgatggccgcgctggaggccggtggcaccgtcgcgctc
gcgaacaaggagtcgctcgtctcggcgggtgaggtgatgatggcggcggcccgcgcgcat
ggcgcgacgctgctgccggtcgattcggagcacaatgcggtgttccagtgcctcgatcgc
accgcgcccaggggcgtccgccggatcatccttaccgccagcggtggtccgttccgcgcg
acgccgaaggaagcgatgcgcgacatcaccccgcacaggcggtggcgcatcccaactgg
tcgatgggcgccaagatctcggtcgactccgcgacgatgatgaacaaggggctcgaactg
atcgaagccttccacctgttcccggtcgccgccgagcaactggccgtgctggtccatcgc
caatccgtcgtccattcgatggtggaatatgtcgacggatcggtgctggcccagctcggc
acgcccgacatgcgcacgccgatcgcctatgcgctggcttggcccgagcggatggagacg
ctgtgcccgccgctcgaccttgccacggtgggtaagctcgagttcgaaaatcccgatctc
gatcgcttcccggcgctcgcgctggcgatggaggcattgaaggcgggcggggcgcgtccg
gccattctcaatgccgccaacgaagtcgccgtcgcggcctttctcgccgggcggatcgga
ttccttgaaattgccgcaatctctgccgatacgctgtctcgctatgacccggccgcgccg
gaaacgctcgatgccgtgctggcgatcgacgcggaggcgcggctttacgcggctgagcga
gtgaaggactgcgtcgcttga (SEQ ID NO:96)

# Figure 30

```
  1  vvkrvtvlga tgsvgtstld liernphafe vvaltancdv eklaaaairt
 51  rarcavvade kclpalqerl agsgveamgg ahsvcdvarm gadwtmaaiv
101  gsaglkpvma aleaggtval ankeslvsag evmmaaarah gatllpvdse
151  hnavfqcldr taprgvrrii ltasggpfra tpkeamrdit paqavahpnw
201  smgakisvds atmmnkglel ieafhlfpva aeqlavlvhr qsvvhsmvey
251  vdgsvlaqlg tpdmrtpiay alawpermet lcppldlatv gklefenpdl
301  drfpalalam ealkaggarp ailnaaneva vaaflagrig fleiaaisad
351  tlsrydpaap etldavlaid aearlyaaer vkdcva  (SEQ ID NO:97)
```

# Figure 31 (page 1 of 15)

```
Stdxrcds     1 --------------------------------------------------
Padxrd       1 at------------------------------------------------
Zmdxrd       1 --------------------------------------------------
Sgdxrd       1 --------------------------------------------------
Nmdxrd       1 --------------------------------------------------
Ecdxrd       1 --------------------------------------------------
Sldxrd       1 --------------------------------------------------
Mldxrd       1 --------------------------------------------------
Pmdxrp       1 atgagtattagttat-----------------------------------
Atdxrd       1 atgatgacattaaactcactatctccagctgaatccaaagctatttcttt
Cjdxrd       1 --------------------------------------------------
Pfdxrd       1 --------------------------------------------------

Stdxrcds     1 -----------------------------------------gtgg-----
Padxrd       3 -----------------------------------------gagt-----
Zmdxrd       1 -----------------------------------------atga-----
Sgdxrd       1 -----------------------------------------ttgg-----
Nmdxrd       1 --------------------------------------------a-----
Ecdxrd       1 --------------------------------------------a-----
Sldxrd       1 --------------------------------------------g-----
Mldxrd       1 --------------------------------------------g-----
Pmdxrp      16 -----------------------------------------ttta-----
Atdxrd      51 cttggatacctccaggttcaatccaatccctaaactctcaggtgggttta
Cjdxrd       1 --------------------------------------------------
Pfdxrd       1 --------------------------------------------a-----

Stdxrcds     5 --------------------------------------------------
Padxrd       7 --------------------------------------------------
Zmdxrd       5 --------------------------------------------------
Sgdxrd       5 --------------------------------------------------
Nmdxrd       2 --------------------------------------------------
Ecdxrd       2 --------------------------------------------------
Sldxrd       2 --------------------------------------------------
Mldxrd       2 --------------------------------------------------
Pmdxrp      20 --------------------------------------------------
Atdxrd     101 gtttgaggaggaggaatcaagggagaggttttggaaaaggtgttaagtgt
Cjdxrd       1 --------------------------------------------------
Pfdxrd       2 --------------------------------------------------
```

# Figure 31 (page 2 of 15)

```
Stdxrcds    5 --------------------------------------------------
Padxrd      7 --------------------------------------------------
Zmdxrd      5 --------------------------------------------------
Sgdxrd      5 --------------------------------------------------
Nmdxrd      2 --------------------------------------------------
Ecdxrd      2 --------------------------------------------------
Sldxrd      2 --------------------------------------------------
Mldxrd      2 --------------------------------------------------
Pmdxrp     20 --------------------------------------------------
Atdxrd    151 tcagtgaaagtgcagcagcaacaacaacctcctccagcatggcctgggag
Cjdxrd      1 --------------------------------------------------
Pfdxrd      2 --------------------------------------------------

Stdxrcds    5 ---------tga----ag--------------------------------
Padxrd      7 ---------cgaccgcag--------------------------------
Zmdxrd      5 ---------gtc----ag--------------------------------
Sgdxrd      5 ---------tca--------------------------------------
Nmdxrd      2 ---------tga----ca--------------------------------
Ecdxrd      2 ---------tga----ag--------------------------------
Sldxrd      2 ---------tga----aa--------------------------------
Mldxrd      2 ---------tga----acaatccgatcgaggggcacgctggcggccgcct
Pmdxrp     20 ---------tga----aa--------------------------------
Atdxrd    201 agctgtccctga---gg--------------------------------
Cjdxrd      1 --------------------------------------------------
Pfdxrd      2 ---------tga----ag--------------------------------

Stdxrcds   10 -cg------c------gtca-cggtgttgggggcgacc------------
Padxrd     16 -cg------g------atca-gcgtgctcggcgcgacc------------
Zmdxrd     10 -cc------aagaacagtca-ctgttttaggggcgacc------------
Sgdxrd      8 ------------------------ttctcggctcgacc------------
Nmdxrd      7 -ccacaagtc------ctga-ccatattaggcagtacc------------
Ecdxrd      7 -ca------a------ctca-ccattctgggctcgacc------------
Sldxrd      7 -gc------a------gtga-cactgctcggttcaacc------------
Mldxrd     39 ccg------c------gtgc-tggtgttgggaagtact------------
Pmdxrp     25 -aa------g------atcg-ttattttaggttcaact------------
Atdxrd    215 -cg------c------ctcgtcaatcttgggatggaccaaaacccatctc
Cjdxrd      1 ----------------atga-tactttttggaagtacg------------
Pfdxrd      7 ------------------aa-atatatttatatatatt------------
```

# Figure 31 (page 3 of 15)

```
Stdxrcds   34 ----------------ggctcggtcggcacctcgacgctggatc------
Padxrd     40 ----------------ggctcgatcggcctgagcaccctggacg------
Zmdxrd     40 ----------------ggatccattggtcattcaacactggatt------
Sgdxrd     22 ----------------ggctcgatcggcacccaggccatcgacg------
Nmdxrd     37 ----------------ggcagcataggcgaaagcacgctggacg------
Ecdxrd     31 ----------------ggctcgattggttgcagcacgctggacg------
Sldxrd     31 ----------------ggctcgatcgggacacaaaccctagaca------
Mldxrd     64 ----------------ggctcaattggcacccaggcgctggaag------
Pmdxrp     49 ----------------ggatcgattggtaccagtactttatccg------
Atdxrd    252 tatcgttggatctactggttctattggcactcagacattggata------
Cjdxrd     22 ----------------ggc---------------agtataggag------
Pfdxrd     26 ----------------ttttct-tcatcacaataactattaatgatttag

Stdxrcds   62 -------tgatcgaacgaaatccgcacgccttcgaagtcg------tggc
Padxrd     68 -------tcgtccagcgtcatcccgatcgttacgaagcct------tcgc
Zmdxrd     68 -------taatcgaacggaatttagatcggtatcaggtca------tcgc
Sgdxrd     50 -------tggtgctccgcaaccccggccggttcaaggtgg------tcgc
Nmdxrd     65 -------ttgtctcccgccaccccgaaaaattccgcgtat------tcgc
Ecdxrd     59 -------tggtgcgccataatcccgaacacttccgcgtag------ttgc
Sldxrd     59 -------ttcttgagcagtatcccgatcgctttcgcctcg------tagg
Mldxrd     92 -------ttatcgccgccaatccggaccgtttcgaggtag------tcgg
Pmdxrp     77 -------tgattacacataatcctgataagtaccaagtgt------ttgc
Atdxrd    296 -------ttgtggctgagaatcctgacaaattcagagttg------tggc
Cjdxrd     35 -------taaatgctcttaaacttgctgctttaaaaaaca------ttcc
Pfdxrd     59 taataaataatacatcaaaatgtgtttccattgaaagaagaaaaaataac

Stdxrcds   99 gct----------------------------gaccgca------aattgc
Padxrd    105 cct----------------------------gactggc------ttcagc
Zmdxrd    105 ttt----------------------------gaccgcc------aaccgc
Sgdxrd     87 gct----------------------------gtccgcg------gccggc
Nmdxrd    102 gct----------------------------ggcaggg------cataag
Ecdxrd     96 gct----------------------------ggtggca------ggcaaa
Sldxrd     96 gct----------------------------ggcggct------ggtcgt
Mldxrd    129 gct----------------------------ggccgc------------c
Pmdxrp    114 gtt----------------------------agttggt------ggacgt
Atdxrd    333 tct----------------------------agctgct------ggttcg
Cjdxrd     72 cat----------------------------ttctgct------ttagct
Pfdxrd    109 gcatatataaattatggtataggatataatggaccagataataaaataac
```

# Figure 31 (page 4 of 15)

```
Stdxrcds  115 ---------gatgtcgag--aagctgg-----c--------------tgc
Padxrd    121 cgcctggccgaactcgag--gcgctg--------------------tgc
Zmdxrd    121 ---------aatgtcaaa--gatctgg-----c--------------cga
Sgdxrd    103 ---------ggcgcggtg--gagctgc-----t--------------cgc
Nmdxrd    118 ---------caggtcgag--aaattgg-----c--------------ggc
Ecdxrd    112 ---------aatgtc-ac--tcgcatg-----g--------------tag
Sldxrd    112 ---------aatgtggcg--ctgtt-------------------------
Mldxrd    139 ---------gggggcgcg--cagctggacacgc--------------tgc
Pmdxrp    130 ---------aatgtagagctaatgttt-----c--------------aac
Atdxrd    349 ---------aatgttact--ctacttg-----c--------------t--
Cjdxrd     88 ---------tgtggggat--aacatcg-----c--------------t--
Pfdxrd    159 ---------aaagagtag--aagatgt-----aaaagaataaagttatgc

Stdxrcds  135 -cgcg---gcgatc--cgcac-g-cgcgcgc-gctgc--g-c-------c
Padxrd    148 -ctca---ggcacc--gcccc-g-tctatgc-ggtggt-g-c-------c
Zmdxrd    141 -tgcg---gcgaaa--agaac-g-aatgcca-agcgg--g-c-------g
Sgdxrd    123 -cgag---caggccgtcgcactg-ggcgtgc-acacc--g-t-------c
Nmdxrd    138 tcaat---gtcaaa--cgttc---caccccg-aatat--g-c-------c
Ecdxrd    131 -aaca---gtgcct--ggaat-t-ctctccccgctat--g-c-------c
Sldxrd    126 ----g---tcggag--caaat-t-cggcggc-accga--c-c-------a
Mldxrd    164 -tgag---gc----------------agcgc-gccgc--gac-------c
Pmdxrp    152 -aatgtttgacatt--ccaac-c-gtcgttt-gctgc--g-ttagatgac
Atdxrd    367 ----------gatc--aggta-a-ggagatt-taagcctg-c-------a
Cjdxrd    106 -cttt---taaatg--agcaa-atcgcaagg-tttaa--a-c-------c
Pfdxrd    193 -aaaa---aggat----ttaa-t-agatatt-ggtgc--a-a-------t

Stdxrcds  166 gtggtcgc--cg------ac---------ga---------gaaatgc---
Padxrd    180 ggagcagg--cc------gc---------gg---------cgattgc---
Zmdxrd    172 gttatcgc--tg------ac---------cc---------gtcgctt---
Sgdxrd    157 gcggtggc--cg------acccggccgccga---------ggaagccg--
Nmdxrd    169 gtcgttgc--cg------at---------gc---------cgaa--c---
Ecdxrd    163 gtaatgga--cg------at---------gaagcgagtgcgaaactt---
Sldxrd    154 gagattgtggcg------at---------tc---------aagatgcagc
Mldxrd    184 ggcgtcac--ca------at---------atc--------gccatcg---
Pmdxrp    193 gatgtcgc--ag------cc-------------------aaaatgt---
Atdxrd    394 ttggttgc--tgttagaaac---------ga---------gtcactg---
Cjdxrd    138 caaatttg--tt------tc---------ca---------taaaaga---
Pfdxrd    222 aaagaaac--ca------at---------taatgta----gcaattt---
```

# Figure 31 (page 5 of 15)

```
Stdxrcds  187 ----ctg------c---cg---gc--gctacagg------agcggctg--
Padxrd    201 ----ctt------g---ca---gg--gct-cgct------cgccgc-g--
Zmdxrd    193 ----tat------a---at---ga--tctgaaag------aggctttg--
Sgdxrd    188 ----ctg------c---gc---ga--ggccctggcggccaaggcgcag--
Nmdxrd    188 ----acg------c---cg---cccggcttgaag------ccctgttgaa
Ecdxrd    193 ----ctt------aaaacg---at--gctacagc------aacag-----
Sldxrd    180 tcagctg------t---cg---ga--actgcaag------cggcgatc--
Mldxrd    206 ----ctg------a---cgatcgc--gc----gg------ctcagctg--
Pmdxrp    212 ----tgg------c--------------agaga------aactgaaa--
Atdxrd    421 ----att------a---at---ga--gcttaaag------aggcttta--
Cjdxrd    159 ----tt-----------------c--aaaaaata------agcattta--
Pfdxrd    248 ----ttggaagtac---tg---gt--agtatagg------tacgaatg--

Stdxrcds  211 ----gcc------------ggcagcgg----------------------
Padxrd    223 ----gcg------------ggtatccg----------------------
Zmdxrd    217 ----gcc------------ggaagctc----------------------
Sgdxrd    218 ----ggc------------gcccgctg----------------------
Nmdxrd    216 acgcgac------------ggca-cgg----------------------
Ecdxrd    217 -------------------ggtagccg----------------------
Sldxrd    208 ----gca------------gaccttga----------------------
Mldxrd    229 ----gcc------------ggc---------------------------
Pmdxrp    229 ----gcc--------------caccaa----------------------
Atdxrd    445 ----gct------------gatttgga----------------------
Cjdxrd    178 ----gtt------------aaacacga----------------------
Pfdxrd    278 ----ctttaaatataataagggagtgtaataaaattgaaaatgtttttaa

Stdxrcds  222 tg------tcg-ag-----gcgat-gggcggggc-------------gca
Padxrd    234 ca------ccc-gg-----gtgct-gttcggcga-------------gca
Zmdxrd    228 tg------ttg-ag-----gcagc-cgcgggtgc-------------tga
Sgdxrd    229 cc------gcg--g-----gtgct-ggcgggccc-------------gga
Nmdxrd    230 cg------actcag-----gtttt-acacggcgc-------------gca
Ecdxrd    225 ca------ccg-aa-----gtctt-aagtgggca-------------aca
Sldxrd    219 ta------atc-cg-----ccgct-catcctgac----------------
Mldxrd    235 -g------aca-tc-----cctta-ccacgggac-------------cga
Pmdxrp    238 ag------cca-aacaacagtctt-agcaggaca-------------gca
Atdxrd    456 ctataaactcg-ag-----attat-tccaggaga-------------gca
Cjdxrd    189 ta------gag-tt-----tttatagggcaagaa-------------ggt
Pfdxrd    324 tg------tta-aa-----gcatt-gtatgtgaataagagtgtgaatgaa
```

# Figure 31 (page 6 of 15)

```
Stdxrcds  246 ttcgg------tgtgcgacgtggc----g---------cgga--------
Padxrd    258 ggcgt------tgtgcgaagtggc----c---------ag----------
Zmdxrd    252 tgcct------tggtcgaagccgc----c---------atga--------
Sgdxrd    252 cgcgg------cgaccgagctggcc---g---------cggc--------
Nmdxrd    255 ggcat------tggttgacgttgcctctg---------ccga--------
Ecdxrd    249 agccg------cttgcgatatggca---g---------cgct--------
Sldxrd    240 --cgg------tgaggcaggtgtc----a---------cgga--------
Mldxrd    258 tgcg---------------gtcac----c---------cggc--------
Pmdxrp    267 agcca------tttgtgagttagc----gg--------caca--------
Atdxrd    486 aggag------tgattgaggttgc----c---------cgac--------
Cjdxrd    214 ttagagcaaattttaacagaatgt----c---------aaga--------
Pfdxrd    361 ttata------tgaacaagctaga----gaatttttaccagaatatttgt

Stdxrcds  269 --------------------------------------------------
Padxrd    279 --------------------------------------------------
Zmdxrd    275 --------------------------------------------------
Sgdxrd    276 --------------------------------------------------
Nmdxrd    282 --------------------------------------------------
Ecdxrd    273 --------------------------------------------------
Sldxrd    261 --------------------------------------------------
Mldxrd    272 --------------------------------------------------
Pmdxrp    291 --------------------------------------------------
Atdxrd    509 --------------------------------------------------
Cjdxrd    243 --------------------------------------------------
Pfdxrd    401 gtatacatgataaaagtgtatatgaagaattaaaagaactggtaaaaaat

Stdxrcds  269 -------------------------tg-----------gg--tgctga--
Padxrd    279 -------------------------cg-----------cg--cccgaa--
Zmdxrd    275 -------------------------tg-----------gg--tgccga--
Sgdxrd    276 -------------------------gg-----------ag--tgcc-a--
Nmdxrd    282 -------------------------cg-----------aa--gtcag---
Ecdxrd    273 -------------------------tg-----------aggatgttga--
Sldxrd    261 -------------------------agtggctcgctacgg--tgatgc--
Mldxrd    272 -------------------------tg-----------gt--tgaggaga
Pmdxrp    291 -------------------------tcct---------ga--agcaga--
Atdxrd    509 -------------------------at-----------cc--tgaagc--
Cjdxrd    243 -------------------------ta-----------ag--cttta--
Pfdxrd    451 ataaaagattataaacctataatattg-----------tg--tggtga--
```

# Figure 31 (page 7 of 15)

```
Stdxrcds  279 ctg-----gacg-----atgg---c-----tgcg---atc----gtcggc
Padxrd    289 gtg-----gacatggtaatgg---c-----ggcc---atc----gtcggc
Zmdxrd    285 ttg-----gaca-----atgg---c-----agcc---att----atcggt
Sgdxrd    285 ctc-----ggtg-----ctga---a-----cggc---atc----accggt
Nmdxrd    291 cgg-----tgtc-----atgt---g-----cgcc---atc----gtcggg
Ecdxrd    285 tca-----ggtg-----atgg---c-----agcc---att----gttggc
Sldxrd    282 cga-----gatt-----gtggtcac-----tggc---att----gtcggt
Mldxrd    284 ctgaggctgacg-----ttgt---cctcaatgcg---ctg----gtcggg
Pmdxrp    303 tat-----ggta-----atgg---c-----tgcg---att----gtgggg
Atdxrd    519 tgt-----aacc-----gttg---t-----taccggaata----gtaggt
Cjdxrd    253 ctc-----aa------------------tgcc---att----gtaggt
Pfdxrd    486 tga-----aggg-----atga---a-----agaa---atatgtagtagta

Stdxrcds  304 agcgcagggctcaagccggtgatgg-------------------------
Padxrd    319 gccgccgggctgccgtcgaccctgg-------------------------
Zmdxrd    310 tgcgccggtctaaaagcgacgcttg-------------------------
Sgdxrd    310 tcgatcggcctggccccgacgctgg-------------------------
Nmdxrd    316 gcggtggggctgccttccgcgctcg-------------------------
Ecdxrd    310 gctgctgggctgttacctacgcttg-------------------------
Sldxrd    310 tgcgctggtctgctacccacgatcg-------------------------
Mldxrd    319 gcattgggtctgcgacccacactgg-------------------------
Pmdxrp    328 gcggcgggattattgcctactttgt-------------------------
Atdxrd    547 tgtgcgggactaaagcctacggttg-------------------------
Cjdxrd    271 tttgcaggacttaaaagcactttaa-------------------------
Pfdxrd    515 atagtatagataaaatagttattggtattgattcttttcaaggattatat

Stdxrcds  329 -ccgcgctggaggccggtggcacc---------gtcgcgctcgcgaacaa
Padxrd    344 -cggccgtcgaggccggcaagcgc---------gtactgctggccaacaa
Zmdxrd    335 -cagctattcgcaagggcaaaacg---------gtcgctttagcgaataa
Sgdxrd    335 -ccgcgctgcgggccggccgggtg---------ctggtgctggcgaacaa
Nmdxrd    341 -cagcggcgcaaaaggcaaaacc---------atttatctggcgaacaa
Ecdxrd    335 -ctgcgatccgcgcgggtaaaacc---------atttgctggccaataa
Sldxrd    335 -ccgcgatcgaagccggcaaggat---------atcgcccttgccaacaa
Mldxrd    344 -ctgcactgcacacgggcgcgcga---------ttggcgttggccaacaa
Pmdxrp    353 -ctgcggtgaaagctggaaaacgt---------gtactattagcaaataa
Atdxrd    572 -ctgcaattgaagcaggaaaggac---------attgctcttgcaaacaa
Cjdxrd    296 -aggctaaagagcttggcaaaaac---------atagctttagctaacaa
Pfdxrd    565 tctactatgtatgcaattatgaataataaaatagttgcgttagctaataa
```

# Figure 31 (page 8 of 15)

```
Stdxrcds  369 ggagtcgctcgtctcggcgggtgaggtgatgatgg-cggcggcccgc-gc
Padxrd    384 ggaggcgctggtgatgtccggcgcgctgttcatgc-aggcggt-caa-gc
Zmdxrd    375 ggaatccttagtttcagctggcggattgatgatcg-atgccgtgcgg-ga
Sgdxrd    375 ggagtcgctgatcgtcggcggtccgctggtgaagg-cggtg-------gc
Nmdxrd    381 agagacgctggtggtttccggcgcgttgtttatgg-aaaccgcccgt-gc
Ecdxrd    375 agaatcactggttacctgcggacgtctgtttatggacgccgtaaagcaga
Sldxrd    375 agaaaccctgattgcagcaggcccagtggtcctgc-cactcctgcaa-aa
Mldxrd    384 ggaatcgctggtagctggcggttcgctggtgttgg-ccgcggcgc----a
Pmdxrp    393 agaagccttggtaacttgcgggcaattatttattg-atgcagtgcgt-ga
Atdxrd    612 agagacattaatcgcaggtggtcctttcgtgcttc-cgcttgccaac-aa
Cjdxrd    336 agaaagtcttgtagtagctgg-gagttttt--------------------
Pfdxrd    615 agaatccattgtctctgctggtttctttttaaaga-aattattaaat-at

Stdxrcds  417 gcat-ggc---gcgacgctgctgccggtcgattcggagcacaatgcggtg
Padxrd    431 gcagcggc---gcggtgctcctgccgatcgacagcgagcacaacgcgatc
Zmdxrd    423 acat-ggc---acgacgcttctccccgtcgattccgagcataacgctatt
Sgdxrd    417 gcag-ccc---ggccagatcgtgccggtggactccgagcacgccgcgctg
Nmdxrd    429 aaac-ggc---gcggcagtgctgcccgtcgacagcgaacacaacgccgtt
Ecdxrd    425 gcaa-agc---gcaat--tgttaccggtcgatagcgaacataacgccatt
Sldxrd    423 gcac-ggt---gtcaccattacgcctgccgactccgagcactccgcgatc
Mldxrd    429 gcca-ggc---caga---tcgtgcccgtagactcggaacactccgcgctg
Pmdxrp    441 atct-caa---gcacaattgttaccagtagatagtgaacataatgcgatt
Atdxrd    660 acat-aat---gtaaagattcttccggcagattcagaacattctgccata
Cjdxrd    366 gaaa-ggg---gctaaattttacccgttgatagtgagc---atgcagct
Pfdxrd    663 tcat-aaaaatgcaaagataatacctgttgattcagaacatagtgctata

Stdxrcds  463 ttccag----t-------gc---ct---------------cg--at----
Padxrd    478 ttccag----t-------cg---ctgccgcgcaattatgccg--at----
Zmdxrd    469 ttccaa----t-------gc---tt---------------c----c----
Sgdxrd    463 ttccag----g-------cg---ct---------------gg--cc----
Nmdxrd    475 ttccaagtttt-------gc---cg---------------cgcgat----
Ecdxrd    469 tttcag----a-------g-----t---------------tt--ac----
Sldxrd    469 tttcag----t-------gc---at---------------cc--aa----
Mldxrd    472 gcgcaa----t-------gc---ctgcg------------cg--gt----
Pmdxrp    487 ttccaa----tcccttccgc---ct---------------ga--ag----
Atdxrd    706 tttcag----t-------gt---at--------------------t----
Cjdxrd    409 ttaaaa----t-------ttttact---------------cg--aa----
Pfdxrd    712 tttcaa----t-------gt---tt---------------ag-ataata
```

# Figure 31 (page 9 of 15)

```
Stdxrcds  478 ---------------cg-----ca-------------------ccgc---
Padxrd    508 ---------------gg-----cc-------------------tgga---
Zmdxrd    482 ---------------cg-----catcataa-------------ccgc---
Sgdxrd    478 ---------------gg-----cg-------------------gcgc---
Nmdxrd    496 ---------------ta-----ca-------------------cagg---
Ecdxrd    482 ---------------cg-----ca-------------------acct---
Sldxrd    484 ---------------gggctttca-------------------acccatg
Mldxrd    490 ---------------gg-----ta-------------------cc-----
Pmdxrp    509 ---------------cg-----caaagacaaattgggttttgcccgc---
Atdxrd    718 ---------------ca-----ag-------------------gttt---
Cjdxrd    427 ---------------gg-----ta-------------------aaaa---
Pfdxrd    731 ataaggtattaaaaaca-----aa-------------------atgt---

Stdxrcds  486 --------------gcccagg--------ggcg---tccgccg-----ga
Padxrd    516 --------------gcgggtc--------ggcg---tgcgccg-----ga
Zmdxrd    496 --------------gacta------------tg---ttcgccg-----ga
Sgdxrd    486 --------------ccgcgcg--------gagg---tccgcaa-----gc
Nmdxrd    504 --------------tcgcctg--------aacg---aacacgg-----ca
Ecdxrd    490 --------------atccagcataatct-ggga---tacgctgaccttga
Sldxrd    500 ctgattttcggcctgctcaagtcgtggcagggc---tgcgacg-----ga
Mldxrd    496 ---------------cccgac--------gaag---ttgctaa-----gt
Pmdxrp    536 --------------tttctgaatta----ggga---tcagtaa-----ga
Atdxrd    726 --------------gcctgaa--------ggcgctctgcgcaa-----ga
Cjdxrd    435 --------------aaatata--------gcaa---aacttta-----ta
Pfdxrd    754 --------------ttacaag--------acaa---tttttct-----aa

Stdxrcds  506 tc-------------------a--------------------tccttacc
Padxrd    536 tc-------------------c--------------------tcttgacc
Zmdxrd    512 tt-------------------a--------------------ttattacg
Sgdxrd    506 tg-------------------g--------------------tggtgacc
Nmdxrd    524 tcgcttcgatt----------a--------------------tcctgacc
Ecdxrd    522 gc--------------------aaaatggcgtggtgtccatttacttacc
Sldxrd    542 tt-------------------c--------------------tcctgact
Mldxrd    515 ta-------------------g--------------------tgctaacc
Pmdxrp    560 tt-------------------g--------------------tgttaacg
Atdxrd    749 ta-------------------a--------------------tcttgact
Cjdxrd    455 tc---------------------------------------------aca
Pfdxrd    774 aattaacaatataaataaaata--------------------tttttatg
```

# Figure 31 (page 10 of 15)

```
Stdxrcds  517 gccagc-ggtggtccgttccgcg--cg----acgccgaaggaagcgatgc
Padxrd    547 gcctcc-ggcggcccgttccgcg--ag----acgccgctgga-gcaactc
Zmdxrd    523 gccagc-ggaggtcccttcagaa--ca----acgtctcttgccgaaatg-
Sgdxrd    517 gccagc-ggcggcccgttccgcaaccg----cacccgtgagcagc--tgg
Nmdxrd    544 gcttcc-ggcggcccgtttctga--c------cgccgatttaaac-acgt
Ecdxrd    553 gggtct-ggtggccctttccgtg--ag----acgcc--attgcgcgattt
Sldxrd    553 gccagt-ggcggcgcttttcggg--ac----tggccggtcgaacggctgt
Mldxrd    526 gcctcc-ggcgggccgtttcgtg--gctggaacgccg-gcgacttggagc
Pmdxrp    571 ggatcc-ggtggtccattccgtt--at----acccctctgga-gcaattt
Atdxrd    760 gcatct-ggtggagcttttaggg--at----tggcctgtcgaaaagctaa
Cjdxrd    460 gcaagt-ggtggagctttttata--gg----tataaaatcaaagatttaa
Pfdxrd    804 ttcatctggaggtccatttcaaa--at----ttaactatggacgaattaa

Stdxrcds  560 gcg-ac--a-tca---cccccgcacaggcggtggcg-catcccaactggt
Padxrd    589 gct-tc--ggtga---cgccggagcaggcttgtgcg-cacccgaactggt
Zmdxrd    565 gca-ac--ggtca---cgccagaacgcgcggttcag-catcccaactggt
Sgdxrd    560 cgg-cc--g-tca---cgccggccgacgcgctggcg-cacccgacctggg
Nmdxrd    584 tcg-ac--a-gcattacgcccgaccaagcggtcaaa-caccccaattggc
Ecdxrd    594 ggc-aacaa-tga---cgccggatcaagc-ctgccgtcatccgaactggt
Sldxrd    596 cgc-aa--g-taa---ctgtcgcagatgcgctcaag-catcccaactggt
Mldxrd    572 gcg-------tta---cacccgagcaggcgggcgtc-catccgacttggt
Pmdxrp    613 gaacag--a-tca---ccccagcacaagcagttgcg-catcctaattggt
Atdxrd    803 agg-aa--g-tta---aagtagcggatgcgttgaag-catccaaactgga
Cjdxrd    503 atc-aa--g-tca---gtgtcaaagatgctttaaaa-catcctaattgga
Pfdxrd    848 aaa-at--g-taa---catcagaaaatgctttaaag-catcctaaatgga

Stdxrcds  602 cgatgggcgccaagatctcggtcgactccgcgacgatgatgaacaagggg
Padxrd    632 cgatggggcgtaagatttccgtcgactccgccagcatgatgaacaagggg
Zmdxrd    608 caatgggtgccaagatttctatcgattctgctacaatgatgaataagggg
Sgdxrd    602 cgatgggcccggtggtgacgatcaactcggcgaccctggtgaacaagggc
Nmdxrd    629 gtatgggacgcaaaatctccgtcgattccgccaccatgatgaacaaaggt
Ecdxrd    638 cgatggggcgtaaaatttctgtcgattcggctaccatgatgaacaaaggt
Sldxrd    638 cgatggggcgcaagattaccgtcgactccgccaccttgatgaataaaggc
Mldxrd    611 caatggggacgatgaacacgctgaactcagcgtctctggttaacaagggg
Pmdxrp    656 caatggggaaaaagatctctgtcgattccgctaccatgatgaataaaggg
Atdxrd    845 acatgggaaagaaaatcactgtggactctgctacgcttttcaacaagggt
Cjdxrd    545 acatgggagcaaagatcactatagatagtgcgactatggcaaataagctt
Pfdxrd    890 aaatgggtaagaaaataactatagattctgcaactatgatgaataaaggt
```

# Figure 31 (page 11 of 15)

```
Stdxrcds   652 ctcgaactgatcgaagccttccacctgttcccggtcgcc--gccgagcaa
Padxrd     682 ctcgaactgatcgaggcgtgctggctgttc---gacgcccagccgagcca
Zmdxrd     658 cttgaattgatagaagcctatcatctcttccagattcca--ttagaaaaa
Sgdxrd     652 ctggaggtgatcgaggcgcacctgctgtacgacgtgccg--ttcgaccgg
Nmdxrd     679 ttggagctgattgaagcgcattggctgttcaactgtccg--cccgacaaa
Ecdxrd     688 ctggaatacattgaagcgcgttggctgtttaacgccagc--gccagccag
Sldxrd     688 ctcgaggtgatcgaagcccactatctcttcggcttggat--tacgactac
Mldxrd     661 ctcgagctcatcgaagccaacctgttgttcggcattccc--tacgaccgc
Pmdxrp     706 ttggaatatattgaagcacgctggttatttaatgcctcg--gcagaagaa
Atdxrd     895 cttgaggtcattgaagcgcattatttgtttggagctgag--tatgacgat
Cjdxrd     595 tttgagattatagaggcttatcatttat------atgat--tttaaagaa
Pfdxrd     940 ttagaggttatagaaacccatttttatttgatgtagat--tataatgat

Stdxrcds   700 c-tggccgtgctggtccatcgccaatccgtcgtccattcgatggtggaat
Padxrd     729 ggtcgaggtggtgatccacccgcagagcgtgatccactcgatggtggact
Zmdxrd     706 t-ttgaaattttggttcatcctcagtcagttattcactccatggtggaat
Sgdxrd     700 a-tcgaggtggtggtccatccgcagtcggtcgttcattcgatggtggaat
Nmdxrd     727 c-tcgaagtcgtcatccatccgcaatctgtgatacacagcatggtgcgct
Ecdxrd     736 a-tggaagtgctgattcacccgcagtcagtgattcactcaatggtgcgct
Sldxrd     736 a-tcgacatcgtcatccatccccagagcatcatccactcgctgattgagc
Mldxrd     709 a-ttgaggtggttgtgcaccctcagtcaattgttcattcgatggtgacat
Pmdxrp     754 a-tggaagttattattcatcctcaatccattattcattctatggtacgtt
Atdxrd     943 a-tagagattgtcattcatccgcaaagtatcatacattccatgattgaaa
Cjdxrd     637 a-ttgatgctttaatagaaccaagatctttagtgcatgcaatgtgtgaat
Pfdxrd     988 a-tagaagttatagtacataaagaatgcattatacattcttgtgttgaat

Stdxrcds   749 atgtcgacggatcggtgctggcccagctcggcacgcccgacatgcgcacg
Padxrd     779 acgtcgacggttcggtgatcgcccagctcggcaatccggacatgcgcacg
Zmdxrd     755 atttggatggttctatccttgcccagatcggtagtcctgatatgagaaca
Sgdxrd     749 tcgtggacggttcgacgatggcccaggccagcccgccggacatgcgcatg
Nmdxrd     776 accgcgacggctccgtgttggcgcaactgggcaatcccgatatgcgaacg
Ecdxrd     785 atcaggacggcagtgttctggcgcagctgggggaaccggatatgcgtacg
Sldxrd     785 tagaagatacctccgtcttggcgcaattgggctggccggatatgcgactg
Mldxrd     758 tcatcgacggctcgacgatcgcccaagccagccctccggacatgaagcta
Pmdxrp     803 acatcgatgggtccgtgattgctcaaatggggaatcctgatatgcgtaca
Atdxrd     992 cacaggattcatctgtgcttgctcaattgggttggcctgatatgcgttta
Cjdxrd     686 ttaaaaatggagctagcacggcgtatttttcaaaagcagatatgaaacta
Pfdxrd    1037 ttatagacaaatcagtaataagtcaaatgtattatccagatatgcaaata
```

# Figure 31 (page 12 of 15)

```
Stdxrcds   799 ccgatcgcctatgcgctggcttggcccgagcgga----t----------g
Padxrd     829 ccgatttcctatgccatggcctggccggagcgaa----t----------c
Zmdxrd     805 ccgatcggtcatactttggcttggccaaagcgga----t----------g
Sgdxrd     799 ccgatcgcgctgggcctcggctggccggaccggg----t----------g
Nmdxrd     826 cctatcgcttattgtttgggtttgcccgagcgca----t----------c
Ecdxrd     835 ccaattgcccacaccatggcatggccgaatcgcg----t----------g
Sldxrd     835 cccttgctctacgccctctcctggcccgatcgcc----t----------c
Mldxrd     808 cctatttctttggcgttgggctggccacagcggg----t----------g
Pmdxrp     853 ccgattgcggaaaccatggcatatccaagtcggaccgtt----------g
Atdxrd    1042 ccgattctctacaccatgtcatggcccgatagag----ttccttgttctg
Cjdxrd     736 gctatttcagatgctatattt------gaaaac----a----------a
Pfdxrd    1087 cccatattatattctttaacatggcctgatagaa----t----------a

Stdxrcds   835 gag-acgc---tgtgccc----gccgc-t-cgaccttg--------ccac
Padxrd     865 gat-tccg---gcgtttc----gccgc-t-ggatatgt--------tcgc
Zmdxrd     841 gaa-acac---cagccga----atcgt-t-ggatttta--------ccaa
Sgdxrd     835 ccggacgc-----cgccc----ccggc-tgcgactgga--------ccaa
Nmdxrd     862 gat-tcgg----gtgtcg----gcgacct-ggatttcg--------acgc
Ecdxrd     871 aa-----c---tctggcgtgaagccgc-t-cgattttt--------gcaa
Sldxrd     871 tct-actc---aatggtc----ggcgc-t-cgatctgg--------tcaa
Mldxrd     844 g-----------gtg-gc----gctgc-t-cgagcctgtgctttcactac
Pmdxrp     893 ctg-gcgt---tgagccc--------t-t-ggatttt--------acca
Atdxrd    1088 aag-taac---t-tggcc----aagac-t-tgaccttt--------gcaa
Cjdxrd     766 gat-acgcctattttaga----ggctg-t-tgatttta--------gca-
Pfdxrd    1123 aaa-acaa---atttaaa----acctt-t-agatttgg--------ctca

Stdxrcds   867 ggtgggtaagctcgagttcgaaaatcccgatctcgatcgcttc-------
Padxrd     897 cgtcggtcgcctggatttccagcgccccgacgagcagcgcttc-------
Zmdxrd     873 attgcgccagatggattttgaagcaccagattatgaacgtttt-------
Sgdxrd     867 ggccgcgacctgggagttcttcccgctggacaacgaggcgttc-------
Nmdxrd     894 attgtccgcgctgaccttccaaaagcccgactttgaccgcttc-------
Ecdxrd     903 actaagtgcgttgacatttgccgcaccggattatgatcgttat-------
Sldxrd     903 agcgggcagcttggagttccgggaaccggatcacgccaaatac-------
Mldxrd     876 cgcatctacctgggaattcgagccgctggacatcgatgttttt-------
Pmdxrp     921 actgaatggattaacctttattgagccagactatcaacgttat-------
Atdxrd    1119 actcggttcattgactttcaagaaaccagacaatgtgaaatac-------
Cjdxrd     800 -------aaatgcctgctttaaatttc-atc-caatcagcacaaaaaaa
Pfdxrd    1155 ggtttcaactcttacatttcataaccttctttagaacatttc-------
```

# Figure 31 (page 13 of 15)

```
Stdxrcds  910 ---ccggcgctcgcgctggcgatggaggcattgaag-gcgggcggggcgc
Padxrd    940 ---ccctgcctgcgcctggcgagccaggccgcggaa-accggcggcagcg
Zmdxrd    916 ---ccggcattaactttggcaatggaatccatcaaa-tcaggtggggctc
Sgdxrd    910 ---ccggcggtcgagctggcccgcgaggtgggtacg-ctcggcgggaccg
Nmdxrd    937 ---ccctgcctgaagctcgcctatgaagccatgaac-gcaggcggagccg
Ecdxrd    946 ---ccatgcctgaaactggcgatggaggcgttcgaa-caaggccaggcag
Sldxrd    946 ---ccctgcatggacttggcctacgccgccggtcgc-aaaggcggcacaa
Mldxrd    919 ---cccgcagtcgagctggcccggcacgctggacag-atcggcggctgta
Pmdxrp    964 ---ccttgtttaaaattagctattgacgcattttca-gccggacaatatg
Atdxrd   1162 ---ccatccatggatcttgcttatgctgc-tggacgagctggaggcacaa
Cjdxrd    841 tatcctatttttaagcttaaaaatacattttaaaa-gagccaaatttag
Pfdxrd   1198 ---ccgtgtattaaattagcttatcaagcaggtata-aaaggaaactttt

Stdxrcds  956 gtccggccattctcaatgccgccaacgaagtcgccgtcgcggcctttctc
Padxrd    986 ccccggccatgctgaatgccgcgaacgaggtggccgtggccgcatttctc
Zmdxrd    962 gtcctgctgtaatgaatgccgctaatgaaatagctgtggcggccttcctt
Sgdxrd    956 ccccggcggtcttcaatgccgccaacgaggaatgtgtggacg-ctttcct
Nmdxrd    983 cgccctgcgtattgaacgccgccaacgaagccgccgtcgccgcctttttg
Ecdxrd    992 cgacgacagcattgaatgccgcaaacgaaatcaccgttgctgctttttctt
Sldxrd    992 tgccagccgtcttgaatgcggcgaatgagcaagccgtcgccctcttccta
Mldxrd    965 tgaccgccatttacgatgctgctaatgaggaggctgcagaggccttcctc
Pmdxrp   1010 ccacgacagcaatgaatgcagcgaatgaaatcgcggtagcgtctttctta
Atdxrd   1208 tgactggagttctcagcgccgccaatgagaaagctgttgaaatgttcatt
Cjdxrd    890 gt---gttatcatcaatgctgctaatgaagttggtgtttataatttttta
Pfdxrd   1244 atccaactgtactaaatgcgtcaaatgaaatagctaacaacttattttg

Stdxrcds 1006 gccgggcggat----------c--------ggattccttgaaa-ttgccg
Padxrd   1036 gagcggcacat----------c--------cgcttcagcgaca-tcgcgg
Zmdxrd   1012 gataagaaaat----------c--------ggttttcttgata-tcgcta
Sgdxrd   1005 gaagggcgcactgcccttcacc--------ggaatcgtggaca-ctgtgg
Nmdxrd   1033 gacggacagat----------t--------aagtttaccgaca-ttgcca
Ecdxrd   1042 gcgcaacaaat----------c--------cgctttacggata-tcgctg
Sldxrd   1042 gaggagcaaat----------t--------cacttctcggata-ttccgc
Mldxrd   1015 caaggtcggat----------c--------ggcttccccgcca-tcgtcg
Pmdxrp   1060 gacaataagat----------t--------aaattcacagata-ttg---
Atdxrd   1258 gatgaaaagat----------aagctatttggatatcttcaaggttgtgg
Cjdxrd    937 gaaaataaaag----------t--------ggattttagaca-ttgcta
Pfdxrd   1294 aataataaaat----------t--------aaatatttgata-tttcct
```

# Figure 31 (page 14 of 15)

```
Stdxrcds 1037 c----aatctctgccg----atacgctgtctcgctatgac----ccgg--
Padxrd   1067 t----tatcatcgagg----acgtgctgaaccgcgaggcg----gtga--
Zmdxrd   1043 a----aattgtcgaga----aaacattagatcattataca----cccg--
Sgdxrd   1046 c----gaaggtggtcgccgaacacggcacaccgcaat--------cgg--
Nmdxrd   1064 a----aaccgtcgccc----attgtctttcac---aagacttttcaga--
Ecdxrd   1073 cgttgaatttatccgt----a----ctggaaaaaatggat----atgc--
Sldxrd   1073 g----cctgattgaac----gtgcctgcgatcgccaccaa----acgg--
Mldxrd   1046 c----aacaatcgcgg----atgtgttgcagcgtgccgac----caat--
Pmdxrp   1088 --------cgcgacta----aatcagttagtcgtgagcaa----attg--
Atdxrd   1298 a----attaacatgcg----ataaac---atcgaaacgag----ttggta
Cjdxrd    968 a----atgcattttta----aagcccttgatcattttgga----gtac--
Pfdxrd   1325 ctat-aatatcgcaag----ttcttgaatctttcaattct----caaa--

Stdxrcds 1073 -ccgcgcc---g------gaaacgc-------tc---g------atg---
Padxrd   1103 -ccgcagt---c------gaatcgc-------tc---g------atc---
Zmdxrd   1079 -caacccc---g------tcttctt-------tg---g------aag---
Sgdxrd   1082 -gaacttc---g------ctcacgg-------tg---g------agg---
Nmdxrd   1101 -cggcata---g------gcgac-a-------ta---g------ggg---
Ecdxrd   1109 -gcgaacc---a------caatgtg-------tg---g------acg---
Sldxrd   1109 -agtggcaacag------caaccga-------gcttgg------atg---
Mldxrd   1082 -gggctcc---c------caatggg-------gt---g------agggac
Pmdxrp   1120 -caaccac---a------aaaaattcattgcata---g------aag---
Atdxrd   1333 acatcacc---gtctcttgaagaga-------tt---gttcactatg---
Cjdxrd   1004 -ctaaaat---t------tcaagca-------ta---g------aag---
Pfdxrd   1364 -aggtttc---g------gaaaata-------gt---g------aag---

Stdxrcds 1094 ccg----tgctggc----g------------atcga------cgc--gga
Padxrd   1124 agg----tcctggctgccg------------atcgc------cgc-----
Zmdxrd   1100 atg----tctttgc----g------------atcga------caa--tga
Sgdxrd   1103 acg----tac---------------------tcca------cgc--gga
Nmdxrd   1121 ggc----tcttggc----g------------caaga------tgcccgga
Ecdxrd   1130 atg----tgttatc----t------------gttga------tgc--gaa
Sldxrd   1136 aca----ttttggc----c------------tacga------cgc--ttg
Mldxrd   1106 ccgctactgtggat----g------------atgta------ctc---ga
Pmdxrp   1148 atg----tacttga----g------------gtaga------taa--aaa
Atdxrd   1367 act----tgtgggc----a------------cgtgaatatgccgc--gaa
Cjdxrd   1025 aag----tttttga----g------------tatga--------------
Pfdxrd   1385 att----taatgaa----gcaaattctacaaataca------ttc--ttg
```

# Figure 31 (page 15 of 15)

```
Stdxrcds 1116 g-gcgcggc--tttacgcggctgagcg-agtg------------------
Padxrd   1147 --gcgcg-----ttcggtcgccgggca-atgg------------------
Zmdxrd   1122 a-gcgcgga--tacaagccgctgcttt-aatg------------------
Sgdxrd   1119 gagctgggc-----ccgggcccgggcc-cgcg------------------
Nmdxrd   1145 c-acgcgca--caagcgcgg----gca-ttta------------------
Ecdxrd   1152 c-gcgcg----tgaagtcgccaga----aaag------------------
Sldxrd   11[illegible] g-gcacggcagtttgtgcaagctagct-atca------------------
Mldxrd   1131 c-gcgcagc--gctgggcccgtgagcg-agcgttgtgtgcggtagcaaca
Pmdxrp   1170 g-gcaagggaattatctcagtcaatca-tttt------------------
Atdxrd   1395 t-gtgcagc--tttcttctg--gtgct-aggc------------------
Cjdxrd   1041 -----------ttttaaaacaagagagtattt------------------
Pfdxrd   1419 g-gccaaag--ataaagctaccgatat-atac------------------

Stdxrcds 1144 ------------aag----gactgc--gtcg----cttga----------
Padxrd   1171 ------------ttg----acccgg--cacg----ccggctag-------
Zmdxrd   1150 ------------gag----agtttg--cccg----cgtga----------
Sgdxrd   1145 ------------a-------gctggcggccg----gctga----------
Nmdxrd   1169 ------------tcg----gcacac--tgcg----c-tga----------
Ecdxrd   1175 ------------agg----tgatgc--gtct----cgcaagctga-----
Sldxrd   1188 ------------aagtctggaatcc--gtcg----tttag----------
Mldxrd   1177 gcgagttctggaaag----gtctct--gacatggtcttagaaaggtccta
Pmdxrp   1200 ------------aag----tttttc--acat----ccgtaa---------
Atdxrd   1421 ------------cag-------ttc--at-g----catga----------
Cjdxrd   1062 ------------aag----ga--------------gttaa----------
Pfdxrd   1447 ------------aac----aaacat--aatt----cttcatag-------

Stdxrcds 1162 -
Padxrd   1192 -
Zmdxrd   1168 -
Sgdxrd   1162 -
Nmdxrd   1186 -
Ecdxrd   1198 -
Sldxrd   1210 -
Mldxrd   1221 a
Pmdxrp   1219 -
Atdxrd   1435 -
Cjdxrd   1072 -
Pfdxrd   1468 -
```

# Figure 32 (page 1 of 7)

```
Stdxrp     1 --------------------------------------------------
Zmdxrp     1 ms------------------------------------------------
Padxrp     1 ms------------------------------------------------
Ecdxrp     1 --------------------------------------------------
Nmdxrp     1 m-------------------------------------------------
Hidxrp     1 m-------------------------------------------------
Ssdxrp     1 --------------------------------------------------
Pmdxrp     1 msisy---------------------------------------------
Sldxrp     1 --------------------------------------------------
Sgdxrp     1 --------------------------------------------------
Bsdxrp     1 --------------------------------------------------
Mldxrp     1 mnn-----------------------------------------------
Mtdxrp     1 matggrv-------------------------------------------
Atdxrp     1 mmtlnslspaeskaisfldtsrfnpipklsggfslrrrnqgrgfgkgvkc
Cjdxrp     1 --------------------------------------------------
Pfdxrp     1 mkkyiyiyfffititindlvinntskcvsierrknnayinygigyngpdn

Stdxrp     1 ----------------------------vvk-------r-----------
Zmdxrp     3 ----------------------------qpr-------t-----------
Padxrp     3 ----------------------------rpq-------r-----------
Ecdxrp     1 -----------------------------mk-------q-----------
Nmdxrp     2 ----------------------------tpq-------v-----------
Hidxrp     2 ----------------------------qkq-------n-----------
Ssdxrp     1 ----------------------------mvk-------r-----------
Pmdxrp     6 ----------------------------fmk-------k-----------
Sldxrp     1 -----------------------------mk-------a-----------
Sgdxrp     1 --------------------------------------------------
Bsdxrp     1 -----------------------------mk-------n-----------
Mldxrp     4 ----------------------------pieghaggrlr-----------
Mtdxrp     8 ----------------------------vir-------rrgdnevvahnd
Atdxrp    51 svkvqqqqqpppawpgravpeaprqswdgpk-------p-----------
Cjdxrp     1 --------------------------------------------------
Pfdxrp    51 kitksrrckriklckkdlidig------aik-------kpin--------
```

# Figure 32 (page 2 of 7)

```
Stdxrp     5 ---------------vtvlgatgsvgtstldlie---rnphafevvalta
Zmdxrp     7 ---------------vtvlgatgsighstldlie---rnldryqvialta
Padxrp     7 ---------------isvlgatgsiglstldvvq---rhpdryeafaltg
Ecdxrp     4 ---------------ltilgstgsigcstldvvr---hnpehfrvvalva
Nmdxrp     6 ---------------ltilgstgsigestldvvs---rhpekfrvfalag
Hidxrp     6 ---------------ivilgstgsigkstlsvie---nnpqkyhafalvg
Ssdxrp     5 ---------------isilgstgsigtqtldivt---hhpdafqvvglaa
Pmdxrp    10 ---------------ivilgstgsigtstlsvit---hnpdkyqvfalvg
Sldxrp     4 ---------------vtllgstgsigtqtldile---qypdrfrlvglaa
Sgdxrp     1 ---------------mvilgstgsigtqaidvvl---rnpgrfkvvalsa
Bsdxrp     4 ---------------icllgatgsigeqtldvlr---ahqdqfqlvsmsf
Mldxrp    15 ---------------vlvlgstgsigtqalevia---anpdrfevvglaa
Mtdxrp    23 evtnstdgradgrlrvvvlgstgsigtqalqvia---dnpdrfevvglaa
Atdxrp    83 ---------------isivgstgsigtqtldiva---enpdkfrvvalaa
Cjdxrp     1 ---------------milfgstgsigvnalklaa---lk--nipisalac
Pfdxrp    80 ---------------vaifgstgsigtnalniirecnkienvfnvkalyv

Stdxrp    37 -n-cdveklaaairtrarcavvadekclpalqerla--g----s----g
Zmdxrp    39 -n-rnvkdladaakrtnakraviadpslyndlkeala--g----s----s
Padxrp    39 -f-srlaelealclrhrpvyavvpeqaaaialqgsla--a----a----g
Ecdxrp    36 -g-knvtrmveqclefspryavmddeasakllktmlqqqg----s----r
Nmdxrp    38 -h-kqveklaaqcqtfhpeyavvadaehaarleallkrdg----t----a
Hidxrp    38 -g-knveamfeqcikfrphfaalddvnaakilrekli--a----h----h
Ssdxrp    37 -g-gnvallaqqvaefrpeivairqaekledlkaava--el---t----d
Pmdxrp    42 -g-rnvelmfqqcltfqpsfaalddvaakmlaeklk--ahq--s----q
Sldxrp    36 -g-rnvallseqirrhrpeivaiqdaaqlselqaaia--dld--n----p
Sgdxrp    33 ag-gavellaeqavalgvhtvavad----paaeeaaar-g----p----g
Bsdxrp    36 -g-rnidkavpmievfqpkfvsvgdldtyhklkqmsf--s----f----e
Mldxrp    47 -ggaqldtllrqraatgvtniaiaddra-----aqla--g----dipyhg
Mtdxrp    70 -ggahldtllrqraqtgvtniavadehaaqrvgd---------------
Atdxrp   115 -g-snvtlladqvrrfkpalvavrneslinelkeala--d----l----d
Cjdxrp    31 -g-dniallneqiarfkpkfvsikdsknkhlvkhdrv--f----i----g
Pfdxrp   115 -n-ksvnelyeqareflpeylcihdksvyeelkelvk--nikdyk----p
```

# Figure 32 (page 3 of 7)

```
Stdxrp    75 --v--ea--mg--------gahsvcdva-rm-g-adwtmaa-ivgsaglk
Zmdxrp    77 --v--ea--aa--------gadalveaa-mm-g-adwtmaa-iigcaglk
Padxrp    77 --i--rtrvlf--------geqalceva-sa-pevdmvmaa-ivgaaglp
Ecdxrp    76 --t--ev--ls--------gqqaacdma-aled-vdqvmaa-ivgaagll
Nmdxrp    78 --t--qv--lh--------gaqalvdva-sa-devsgvmca-ivgavglp
Hidxrp    76 --iptev--la--------grraicelaahp-d-adqimas-ivgaagll
Ssdxrp    76 --y--qp--myvv------geegvveva-ry-gdaesvvtg-ivgcagll
Pmdxrp    82 --t--tv--la--------gqqaicelaahp-e-admvmaa-ivgaagll
Sldxrp    76 --p--li--lt--------geagvteva-ry-gdaeivvtg-ivgcagll
Sgdxrp    69 --g--qg--agrplprvlagpdaatela-aa-e-chsvlng-itgsigla
Bsdxrp    74 --c--qi--gl--------geeglieaa-vm-eevdivvna-llgsvgli
Mldxrp    85 --t--da--vt--------rl-----ve-et-e-advvlna-lvgalglr
Mtdxrp   103 --i--py--hg--------sdaatrlve-qt-e-advvlna-lvgalglr
Atdxrp   153 ykl--ei--ip--------geqgvieva-rh-p-eavtvvtgivgcaglk
Cjdxrp    69 --q--eg--le--------qiltecqdk-ll-------lna-ivgfaglk
Pfdxrp   157 --i--il--cgde------gmkeic--s-sn-s-idkivig-idsfqgly

Stdxrp   107 pvmaaleaggtvalankeslvsagevmmaaarah-gatllpvdsehnavf
Zmdxrp   109 atlaairkgktvalankeslvsagglmidavreh-gttllpvdsehnaif
Padxrp   112 stlaaveagkrvllankealvmsgalfmqavkrs-gavllpidsehnaif
Ecdxrp   109 ptlaairagktillankeslvtcgrlfmdavkqs-kaqllpvdsehnaif
Nmdxrp   111 salaaaqkgktiylanketlvvsgalfmetaran-gaavlpvdsehnavf
Hidxrp   111 ptlsavkagkrvllankeslvtcgqlfidavkny-gskllpvdsehnaif
Ssdxrp   111 ptmaaiaagkdialanketliagapvvlplvekm-gvkllpadsehsaif
Pmdxrp   115 ptlsavkagkrvllankealvtcgqlfidavres-qaqllpvdsehnaif
Sldxrp   109 ptiaaieagkdialanketliaagpvvlpllqkh-gvtitpadsehsaif
Sgdxrp   109 ptlaalragrvlvlankeslivggplvkavaqp---gqivpvdsehaalf
Bsdxrp   107 ptlkaieqkktialanketlvtaghivkehakky-dvpllpvdsehsaif
Mldxrp   112 ptlaalhtgarlalankeslvaggslvlaaaqp---gqivpvdsehsala
Mtdxrp   135 ptlaalktgarlalankeslvaggslvlraarp---gqivpvdsehsala
Atdxrp   188 ptvaaieagkdialanketliaggpfvlplankh-nvkilpadsehsaif
Cjdxrp    96 stlkakelgknialankeslvvagsfl------k-gakflpvdsehaalk
Pfdxrp   189 stmyaimnnkivalankesivsagfflkkllnihknakiipvdsehsaif
```

# Figure 32 (page 4 of 7)

```
Stdxrp    156 qcldrtap-------------r----------g-----vrriiltasggp
Zmdxrp    158 qcfphhnr-------------d----------y-----vrriiitasggp
Padxrp    161 qslprnya-------------d----------glervgvrrilltasggp
Ecdxrp    158 qslpqpiq-------------hnlgyadleqng-----vvsilltgsggp
Nmdxrp    160 qvlprdytgrlne--------h----------g-----iasiiltasggp
Hidxrp    160 qslppeaq-------------ekigfcplselg-----vskiiltgsggp
Ssdxrp    160 qclqgvpe-------------g----------g-----lrriiltasgga
Pmdxrp    164 qslppeaq-------------rqigfcplselg-----iskivltgsggp
Sldxrp    158 qciqglst-------------hadfrpaqvvag-----lrrilltasgga
Sgdxrp    156 qalaggar-------------a----------e-----vrklvvtasggp
Bsdxrp    156 qalqgeqa-------------k----------n-----ierliitasggs
Mldxrp    159 qclrggtp-------------d----------e-----vaklvltasggp
Mtdxrp    182 qclrggtp-------------d----------e-----vaklvltasggp
Atdxrp    237 qciqglpe-------------g----------a-----lrkiiltasgga
Cjdxrp    139 flle--gk-------------k----------n-----iaklyitasgga
Pfdxrp    239 qcldnnkvlktkclqdnfskin----------n-----inkiflcssggp

Stdxrp    178 fratpkeamrditpaqavahpnwsmgakisvdsatmmnkglelieafhlf
Zmdxrp    180 frttslaematvtperavqhpnwsmgakisidsatmmnkglelieayhlf
Padxrp    188 fretpleqlasvtpeqacahpnwsmgrkisvdsasmmnkglelieacwlf
Ecdxrp    190 fretplrdlatmtpdqacrhpnwsmgrkisvdsatmmnkgleyiearwlf
Nmdxrp    187 fltadlntfdsitpdqavkhpnwrmgrkisvdsatmmnkglelieahwlf
Hidxrp    192 frytpleqftnitpeqavahpnwsmgkkisvdsatmmnkgleyiearwlf
Ssdxrp    182 frdlpverlpfvtvqdalkhpnwsmgqkitidsatlmnkglevieahylf
Pmdxrp    196 frytpleqfeqitpaqavahpnwsmgkkisvdsatmmnkgleyiearwlf
Sldxrp    190 frdwpverlsqvtvadalkhpnwsmgrkitvdsatlmnkglevieahylf
Sgdxrp    178 frnrtreqlaavtpadalahptwamgpvvtinsatlvnkglevieahlly
Bsdxrp    178 frdktreelesvtvedalkhpnwsmgakitidsatmmnkglevieahwlf
Mldxrp    181 frgwnagdlervtpeqagvhptwsmgtmntlnsaslvnkglelieanllf
Mtdxrp    204 frgwsaadlehvtpeqagahptwsmgpmntlnsaslvnkglevieth llf
Atdxrp    259 frdwpveklkevkvadalkhpnwnmgkkitvdsatlfnkglevieahylf
Cjdxrp    159 fyrykikdlnqvsvkdalkhpnwnmgakitidsatmanklfeiieayhly
Pfdxrp    274 fqnltmdelknvtsenalkhpkwkmgkkitidsatmmnkglevieth flf
```

# Figure 32 (page 5 of 7)

```
Stdxrp    228 pvaaeqlavlvhrqsvvhsmveyvdgsvlaqlgtpdmrtpiayalawper
Zmdxrp    230 qiplekfeilvhpqsvihsmveyldgsilaqigspdmrtpightlawpkr
Padxrp    238 daqpsqvevvihpqsvihsmvdyvdgsviaqlgnpdmrtpisyamawper
Ecdxrp    240 nasasqmevlihpqsvihsmvryqdgsvlaqlgepdmrtpiahtmawpnr
Nmdxrp    237 ncppdklevvihpqsvihsmvryrdgsvlaqlgnpdmrtpiayclglper
Hidxrp    242 nasaeemeviihpqsiihsmvryvdgsvitqmgnpdmrtpiaetmayphr
Ssdxrp    232 gldydhidivihpqsiihslievqdtsvlaqlgwpdmrlpllyalswper
Pmdxrp    246 nasaeemeviihpqsiihsmvryidgsviaqmgnpdmrtpiaetmaypsr
Sldxrp    240 gldydyidivihpqsiihslieledtsvlaqlgwpdmrlpllyalswpdr
Sgdxrp    228 dvpfdrievvvhpqsvvhsmvefvdgstmaqasppdmrmpialglgwpdr
Bsdxrp    228 dipyeqidvvlhkesiihsmvefhdksviaqlgtpdmrvpiqyaltypdr
Mldxrp    231 gipydrievvvhpqsivhsmvtfidgstiaqasppdmklpislalgwpqr
Mtdxrp    254 gipydridvvvhpqsiihsmvtfidgstiaqasppdmklpislalgwprr
Atdxrp    309 gaeyddieivihpqsiihsmietqdssvlaqlgwpdmrlpilytmswpdr
Cjdxrp    209 df--keidalieprslvhamcefkngastayfskadmklaisdaif--ek
Pfdxrp    324 dvdyndievivhkeciihscvefidksvisqmyypdmqipilysltwpdr

Stdxrp    278 m---et-l-cppldlatvgklefenpdldrfpalalamealkaggarpai
Zmdxrp    280 m---et-p-aesldftklrqmdfeapdyerfpaltlamesiksggarpav
Padxrp    288 i---ds-g-vspldmfavgrldfqrpdeqrfpclrlasqaaetggsapam
Ecdxrp    290 v---ns-g-vkpldfcklsaltfaapdydrypclklameafeqgqaatta
Nmdxrp    287 i---ds-g-vgdldfdalsaltfqkpdfdrfpclklayeamnaggaapcv
Hidxrp    292 t---fa-g-vepldffkikeltfiepdfnrypnlklaidafaagqyatta
Ssdxrp    282 i---yt-d-wepldlvkagslsfrepdhdkypcmqlaygagraggampav
Pmdxrp    296 t---va-g-vepldfyqlngltfiepdyqrypclklaidafsagqyatta
Sldxrp    290 l---st-q-wsaldlvkagslefrepdhakypcmdlayaagrkggtmpav
Sgdxrp    278 v---pd-a-apgcdwtkaatweffpldneafpavelarevgtlggtapav
Bsdxrp    278 l---pl-pdakrlelweigslhfekadfdrfrclqfafesgkiggtmptv
Mldxrp    281 v---gg-a-aracafttastwefepldidvfpavelarhagqiggcmtai
Mtdxrp    304 v---sg-a-aaacdfhtasswefepldtdvfpavelarqagvaggcmtav
Atdxrp    359 vpcsev-t-wprldlcklgsltfkkpdnvkypsmdlayaagraggtmtgv
Cjdxrp    255 q---dtpi-leavdfskmpalkfhpistkkypifklkntflkepnl-gvi
Pfdxrp    374 i---kt-n-lkpldlaqvstltfhkpslehfpciklayqagikgnfyptv
```

# Figure 32 (page 6 of 7)

```
Stdxrp   323 lnaanevavaaflagrigfleiaaisadtlsry---d---pa-a--pe--
Zmdxrp   325 mnaaneiavaafldkkigfldiakivektldhy---t---pa-t--ps--
Padxrp   333 lnaanevavaaflerhirfsdiaviiedvlnre---a---vt-a--ve--
Ecdxrp   335 lnaaneitvaaflaqqirftdiaalnlsvlekm---d---mr-e--pq--
Nmdxrp   332 lnaaneaavaafldgqikftdiaktvahclsqd---f---sd-g--ig--
Hidxrp   337 mnaaneiavqafldrqigfmdiakinsktieri---s---py-t--iq--
Ssdxrp   327 lnaaneqavalflqekisfldiprliektcdlyvgqn---ta-s--pd--
Pmdxrp   341 mnaaneiavasfldnkikftdiarlnqlvvskl---q---pq-k--ih--
Sldxrp   335 lnaaneqavalfleeqihfsdiprlieracdrh---q---te-w--qqqp
Sgdxrp   323 fnaaneecvdaflkgalpftgivdtvakvvaeh---gt--pq-s--gt--
Bsdxrp   324 lnaanevavaaflagkipflaiedciekaltrh---qllkkp-s--wr--
Mldxrp   326 ydaaneeaaeaflqgrigfpaivatiadvlqra---d---qw-a--pq--
Mtdxrp   349 ynaaneeaaaaflagrigfpaivgiiadvlhaa---d---qw-avepa--
Atdxrp   407 lsaanekavemfidekisyldifkvveltcdkhrn-e---lv-t--sp--
Cjdxrp   300 inaanevgvynflenksgfldiakcifkaldhf---g---vp-k--is--
Pfdxrp   419 lnasneiannlflnnkikyfdissiisqvlesf---n---sqkv--se--

Stdxrp   362 tldavlaid--aearlyaaervkdcva-----------------------
Zmdxrp   364 sledvfaid--neariqaaalmeslpa-----------------------
Padxrp   372 sldqvlaad--rrarsvagqwltrhag-----------------------
Ecdxrp   374 cvddvlsvd--anarevarkevmrlas-----------------------
Nmdxrp   371 diggllaqd--artraqarafigtlr------------------------
Hidxrp   376 niddvleid--aqareiaktllre--------------------------
Ssdxrp   369 -letilaad--qwarrtvlen-sacvatrp--------------------
Pmdxrp   380 ciedvlevd--kkarelsqsiilsfshp----------------------
Sldxrp   376 slddilayd--awarqfvqasyqslesvv---------------------
Sgdxrp   363 sltvedvlh--aes--warararelaag----------------------
Bsdxrp   366 tfkkwtkip--gdtsiqyshkvv-cs------------------------
Mldxrp   365 wgegpatvddvldaqrwareralcavatassgkvsdmvlers--------
Mtdxrp   390 tvddvl------daqrwareraqravsgmasvaiastakpgaagrhastl
Atdxrp   448 sleeivhyd--lwareyaanvqlssgarpvha------------------
Cjdxrp   339 sieevfeyd--fktreylrs------------------------------
Pfdxrp   459 nsedlmkqi--lqihswakdkatdiynkhn--------------------
```

# Figure 32 (page 7 of 7)

```
Stdxrp   387 ---
Zmdxrp   389 ---
Padxrp   397 ---
Ecdxrp   399 ---
Nmdxrp   395 ---
Hidxrp   398 ---
Ssdxrp   395 ---
Pmdxrp   406 ---
Sldxrp   403 ---
Sgdxrp   387 ---
Bsdxrp   389 ---
Mldxrp   407 ---
Mtdxrp   434 ers
Atdxrp   478 ---
Cjdxrp   357 ---
Pfdxrp   487 ---
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6103488 A
- US 4683195 A
- US 5580859 A
- US 5589466 A
- US 0130328 W
- US 60236580 B


### Non-patent literature cited in the description


- **SAMBROOK et al.** Molecular Cloning. Cold Spring harbor Laboratory, 1989
- **DRYDEN ; DOWHAN.** *J. Bacteriol.,* 1996, vol. 178 (4), 1030-1038
- **VASILYEVA et al.** *Applied Biochemistry and Biotechnology,* 1999, vol. 77 (79), 337-345
- **GRAICHEN et al.** *J. Bacteriol.,* 1999, vol. 181 (14), 4216-4222
- **JOHNSON et al.** *J Bacteriol.,* 1986, vol. 167 (2), 604-610
- **DUPORT et al.** *Gene,* 1994, vol. 145, 103-108
- **CHORY et al.** *J. Bacteriol,* 1984, vol. 159, 540-554
- The Photosynthetic Bacteria. Plenum Press, 1978, 79-118
- **LUEKING et al.** *J. Biol. Chem.,* 1978, vol. 253, 451-457
- **COHEN-BAZIRE et al.** *J. Cell Comp. Physiol.,* 1957, vol. 49, 25-68
- **EDLUND.** *J. Chromatogr.,* 1988, vol. 425, 87-97
- **ROUSSEAU ; VARIN.** *J Chromatogr. Sci.,* 1998, vol. 36, 247-52
- **LERAY et al.** *J. Lipid Res.,* 1998, vol. 39, 2099-2105
- **MATH et al.** *Proc. Natl. Acad. Sci. USA,* 1992, vol. 89 (15), 6761-6764
- **KOVACH et al.** *Gene,* 1995, vol. 166, 175-176
- **WATERS et al.** *Nucleic Acids Research,* 1983, vol. 11 (17), 6089-6105
- **LENZ et al.** *J. Bacteriol.,* 1994, vol. 176 (14), 4385-93
- **OH ; KAPLAN.** *Biochemistry,* 1999, vol. 38, 2688-2696